# EUROPEAN PATENT SPECIFICATION

(11) **EP 2 894 169 B1**
(45) Date of publication and mention of the grant of the patent: **23.05.2018**
(21) Application number: 13836076.3
(22) Date of filing: 06.09.2013
(51) Int. Cl.: C07K 19/00, C12N 15/62, C12Q 1/68, A61P 35/00

(54) **FUSION PROTEIN COMPRISING AXL AND COMPOSITION FOR TREATING CANCER COMPRISING SAME**
FUSIONSPROTEIN MIT AXL SOWIE ZUSAMMENSETZUNG ZUR KREBSBEHANDLUNG DAMIT
PROTÉINE DE FUSION COMPRENANT AXL ET COMPOSITION POUR LE TRAITEMENT DU CANCER LA COMPRENANT

(30) Priority: 07.09.2012 KR 20120099616
(43) Date of publication of application: 15.07.2015
(73) Proprietor: Macrogen, Inc., Geumcheon-gu Seoul, 08511 (KR)
(72) Inventor: SEO, Jeong-Sun, Seoul 153-781 (KR); KIM, Young-Tae, Seoul 110-799 (KR); JU, Young-Seok, Seoul 153-781 (KR); KIM, Eun-Hee, Seoul 153-781 (KR); KANG, Jin-Hyoung, Seoul 137-040 (KR)
(74) Representative: Krauns, Christian
(86) International application number: PCT/KR2013/008075
(87) International publication number: WO 2014/038890

(56) References cited:
- EP-A1- 1 897 940
- DATABASE Geneseq [Online] 18 November 2004 (2004-11-18), "Ax1 GH2_420_G3F1 nucleotide sequence.", XP002752953, retrieved from EBI accession no. GSN:ADN60235 Database accession no. ADN60235 & WO 2004/039955 A2 (RIGEL PHARMACEUTICALS INC [US]; LORENS JAMES B [NO]; ATCHISON ROBERT E) 13 May 2004 (2004-05-13)
- "Beyond ALK-RET, ROS1 and other oncogene fusions in lungcancer", Transl Lung Cancer Res, 1 January 2015 (2015-01-01), XP055240876, DOI: 10.3978/j.issn.2218-6751.2014.11.11 Retrieved from the Internet: URL:http://www.ncbi.nlm.nih.gov/pmc/articl es/PMC4384213/pdf/tlcr-04-02-156.pdf
- SHIEH, YI-SHING ET AL.: 'Expression of AXL in lung adenocarcinoma and correlation with tumor progression' NEOPLASIA vol. 7, no. 12, December 2005, pages 1058 - 1064, XP055137005
- LIN, JULES ET AL.: 'Molecular predictors of prognosis in lung cancer' ANNALS OF SURGICAL ONCOLOGY vol. 19, no. 2, February 2012, pages 669 - 676, XP035005728
- JU, YOUNG SEOK ET AL.: 'A transforming KIF5B and RET gene fusion in lung adenocarcinoma revealed from whole-genome and transcriptome sequencing' GENOME RESEARCH vol. 22, no. 3, March 2012, pages 436 - 445, XP055145939
- SOUNG, YOUNG HWA ET AL.: 'Mutational analysis of EGFR and K-RAS genes in lung adenocarcinomas' VIRCHOWS ARCHIV vol. 446, no. 5, May 2005, pages 483 - 488, XP019344735
- SEO, JEONG-SUN ET AL.: 'The transcriptional landscape and mutational profile of lung adenocarcinoma' GENOME RESEARCH vol. 22, no. 11, November 2012, pages 2109 - 2119, XP055210571

<110> MACROGEN INC.
<120> Fusion Protein containing AXL and composition for diagnosing cancer
<130> OPP20133976KR
<150> KR 10-2012-0099616
   <151> 2012-09-07
<160> 205
<170> KopatentIn 1.71
<210> 1
   <211> 1425
   <212> DNA
   <213> Artificial Sequence
<220>
   <223> CDS of CCDC6 gene (NM_005436)
<400> 1
   atggcggaca gcgccagcga gagcgacacg gacggggcgg ggggcaacag cagcagctcg      60
   gccgccatgc agtcgtcctg ctcgtcgacc tcgggcggcg gcggtggcgg cgggggaggc     120
   ggcggcggtg ggaagtcggg gggcattgtc atctcgccgt tccgcctgga ggagctcacc     180
   aaccgcctgg cctcgctgca gcaagagaac aaggtgctga agatagagct ggagacctac     240
   aaactgaagt gcaaggcact gcaggaggag aaccgcgacc tgcgcaaagc cagcgtgacc     300
   atccaagcca gggctgagca ggaagaagaa ttcattagta acactttatt caagaaaatt     360
   caggctttgc agaaggagaa agaaaccctt gctgtaaatt atgagaaaga agaagaattc     420
   ctcactaatg agctctccag aaaattgatg cagttgcagc atgagaaagc cgaactagaa     480
   cagcatcttg aacaagagca ggaatttcag gtcaacaaac tgatgaagaa aattaaaaaa     540
   ctggagaatg acaccatttc taagcaactt acattagaac agttgagacg ggagaagatt     600
   gaccttgaaa atacattgga acaagaacaa gaagcactag ttaatcgcct ctggaaaagg     660
   atggataagc ttgaagctga aaagcgaatc ctgcaggaaa aattagacca gcccgtctct     720
   gctccaccat cgcctagaga tatctccatg gagattgatt ctccagaaaa tatgatgcgt     780
   cacatcaggt ttttaaagaa tgaagtggaa cggctgaaga agcaactgag agctgctcag     840
   ttacagcatt cagagaaaat ggcacagtat ctggaggagg aacgtcacat gagagaagag     900
   aacttgaggc tccagaggaa gctgcagagg gagatggaga gaagagaagc cctctgtcga     960
   cagctctccg agagtgagtc cagcttagaa atggacgacg aaaggtattt taatgagatg    1020
   tctgcacaag gattaagacc tcgcactgtg tccagcccga tcccttacac accttctccg    1080
   agttcaagca ggcctatatc acctggtcta tcatatgcaa gtcacacggt tggtttcacg    1140
   ccaccaactt cactgactag agctggaatg tcttattaca attccccggg tcttcacgtg    1200
   cagcacatgg gaacatccca tggtatcaca aggccttcac cacggagaag caacagtcct    1260
   gacaaattca aacggcccac gccgcctcca tctcccaaca cacagacccc agtccagcca    1320
   cctccgcctc cacctccgcc acccatgcag cccacggtcc cctcagcagc cacctcgcag    1380
   cctactcctt cgcaacattc ggcgcacccc tcctcccagc cttaa                    1425
<210> 2
   <211> 847
   <212> DNA
   <213> Artificial Sequence
<220>
   <223> CCDC6 gene fragment
<400> 2
   atggcggaca gcgccagcga gagcgacacg gacggggcgg ggggcaacag cagcagctcg    60
   gccgccatgc agtcgtcctg ctcgtcgacc tcgggcggcg gcggtggcgg cgggggaggc   120
   ggcggcggtg ggaagtcggg gggcattgtc atctcgccgt tccgcctgga ggagctcacc   180
   aaccgcctgg cctcgctgca gcaagagaac aaggtgctga agatagagct ggagacctac   240
   aaactgaagt gcaaggcact gcaggaggag aaccgcgacc tgcgcaaagc cagcgtgacc   300
   atccaagcca gggctgagca ggaagaagaa ttcattagta acactttatt caagaaaatt   360
   caggctttgc agaaggagaa agaaaccctt gctgtaaatt atgagaaaga agaagaattc   420
   ctcactaatg agctctccag aaaattgatg cagttgcagc atgagaaagc cgaactagaa   480
   cagcatcttg aacaagagca ggaatttcag gtcaacaaac tgatgaagaa aattaaaaaa   540
   ctggagaatg acaccatttc taagcaactt acattagaac agttgagacg ggagaagatt   600
   gaccttgaaa atacattgga acaagaacaa gaagcactag ttaatcgcct ctggaaaagg   660
   atggataagc ttgaagctga aaagcgaatc ctgcaggaaa aattagacca gcccgtctct   720
   gctccaccat cgcctagaga tatctccatg gagattgatt ctccagaaaa tatgatgcgt   780
   cacatcaggt ttttaaagaa tgaagtggaa cggctgaaga agcaactgag agctgctcag    840
   ttacagc                                                             847
<210> 3
   <211> 16
   <212> DNA
   <213> Artificial Sequence
<220>
   <223> Break-point sequence of CCDC6 gene fragment
<400> 3
   gctgctcagt tacagc 16
<210> 4
   <211> 474
   <212> PRT
   <213> Artificial Sequence
<220>
   <223> CCDC6 protein
<400> 4
   Met Ala Asp Ser Ala Ser Glu Ser Asp Thr Asp Gly Ala Gly Gly Asn
   1               5                   10                  15
   Ser Ser Ser Ser Ala Ala Met Gln Ser Ser Cys Ser Ser Thr Ser Gly
               20                  25                  30
   Gly Gly Gly Gly Gly Gly Gly Gly Gly Gly Gly Gly Lys Ser Gly Gly
           35                  40                  45
   Ile Val Ile Ser Pro Phe Arg Leu Glu Glu Leu Thr Asn Arg Leu Ala
       50                  55                  60
   Ser Leu Gln Gln Glu Asn Lys Val Leu Lys Ile Glu Leu Glu Thr Tyr
   65                  70                  75                  80
   Lys Leu Lys Cys Lys Ala Leu Gln Glu Glu Asn Arg Asp Leu Arg Lys
                   85                  90                  95
   Ala Ser Val Thr Ile Gln Ala Arg Ala Glu Gln Glu Glu Glu Phe Ile
               100                 105                 110
   Ser Asn Thr Leu Phe Lys Lys Ile Gln Ala Leu Gln Lys Glu Lys Glu
           115                 120                 125
   Thr Leu Ala Val Asn Tyr Glu Lys Glu Glu Glu Phe Leu Thr Asn Glu
       130                 135                 140
   Leu Ser Arg Lys Leu Met Gln Leu Gln His Glu Lys Ala Glu Leu Glu
   145                 150                 155                 160
   Gln His Leu Glu Gln Glu Gln Glu Phe Gln Val Asn Lys Leu Met Lys
                   165                 170                 175
   Lys Ile Lys Lys Leu Glu Asn Asp Thr Ile Ser Lys Gln Leu Thr Leu
               180                 185                 190
   Glu Gln Leu Arg Arg Glu Lys Ile Asp Leu Glu Asn Thr Leu Glu Gln
           195                 200                 205
   Glu Gln Glu Ala Leu Val Asn Arg Leu Trp Lys Arg Met Asp Lys Leu
       210                 215                 220
   Glu Ala Glu Lys Arg Ile Leu Gln Glu Lys Leu Asp Gln Pro Val Ser
   225                 230                 235                 240
   Ala Pro Pro Ser Pro Arg Asp Ile Ser Met Glu Ile Asp Ser Pro Glu
                   245                 250                 255
   Asn Met Met Arg His Ile Arg Phe Leu Lys Asn Glu Val Glu Arg Leu
               260                 265                 270
   Lys Lys Gln Leu Arg Ala Ala Gln Leu Gln His Ser Glu Lys Met Ala
   275 280 285
   Gln Tyr Leu Glu Glu Glu Arg His Met Arg Glu Glu Asn Leu Arg Leu
   290 295 300
   Gln Arg Lys Leu Gln Arg Glu Met Glu Arg Arg Glu Ala Leu Cys Arg
   305 310 315 320
   Gln Leu Ser Glu Ser Glu Ser Ser Leu Glu Met Asp Asp Glu Arg Tyr
   325 330 335
   Phe Asn Glu Met Ser Ala Gln Gly Leu Arg Pro Arg Thr Val Ser Ser
   340 345 350
   Pro Ile Pro Tyr Thr Pro Ser Pro Ser Ser Ser Arg Pro Ile Ser Pro
   355 360 365
   Gly Leu Ser Tyr Ala Ser His Thr Val Gly Phe Thr Pro Pro Thr Ser
   370 375 380
   Leu Thr Arg Ala Gly Met Ser Tyr Tyr Asn Ser Pro Gly Leu His Val
   385 390 395 400
   Gln His Met Gly Thr Ser His Gly Ile Thr Arg Pro Ser Pro Arg Arg
   405 410 415
   Ser Asn Ser Pro Asp Lys Phe Lys Arg Pro Thr Pro Pro Pro Ser Pro
   420 425 430
   Asn Thr Gln Thr Pro Val Gln Pro Pro Pro Pro Pro Pro Pro Pro Pro
   435 440 445
   Met Gln Pro Thr Val Pro Ser Ala Ala Thr Ser Gln Pro Thr Pro Ser
   450 455 460
   Gln His Ser Ala His Pro Ser Ser Gln Pro
   465 470
<210> 5
   <211> 282
   <212> PRT
   <213> Artificial Sequence
<220>
   <223> CCDC6 protein fragment
<400> 5
   Met Ala Asp Ser Ala Ser Glu Ser Asp Thr Asp Gly Ala Gly Gly Asn
   1               5                   10                  15
   Ser Ser Ser Ser Ala Ala Met Gln Ser Ser Cys Ser Ser Thr Ser Gly
               20                  25                  30
   Gly Gly Gly Gly Gly Gly Gly Gly Gly Gly Gly Gly Lys Ser Gly Gly
           35                  40                  45
   Ile Val Ile Ser Pro Phe Arg Leu Glu Glu Leu Thr Asn Arg Leu Ala
       50                  55                  60
   Ser Leu Gln Gln Glu Asn Lys Val Leu Lys Ile Glu Leu Glu Thr Tyr
   65                  70                  75                  80
   Lys Leu Lys Cys Lys Ala Leu Gln Glu Glu Asn Arg Asp Leu Arg Lys
                   85                  90                  95
   Ala Ser Val Thr Ile Gln Ala Arg Ala Glu Gln Glu Glu Glu Phe Ile
               100                 105                 110
   Ser Asn Thr Leu Phe Lys Lys Ile Gln Ala Leu Gln Lys Glu Lys Glu
           115                 120                 125
   Thr Leu Ala Val Asn Tyr Glu Lys Glu Glu Glu Phe Leu Thr Asn Glu
       130                 135                 140
   Leu Ser Arg Lys Leu Met Gln Leu Gln His Glu Lys Ala Glu Leu Glu
   145                 150                 155                 160
   Gln His Leu Glu Gln Glu Gln Glu Phe Gln Val Asn Lys Leu Met Lys
                   165                 170                 175
   Lys Ile Lys Lys Leu Glu Asn Asp Thr Ile Ser Lys Gln Leu Thr Leu
               180                 185                 190
   Glu Gln Leu Arg Arg Glu Lys Ile Asp Leu Glu Asn Thr Leu Glu Gln
           195                 200                 205
   Glu Gln Glu Ala Leu Val Asn Arg Leu Trp Lys Arg Met Asp Lys Leu
       210                 215                 220
   Glu Ala Glu Lys Arg Ile Leu Gln Glu Lys Leu Asp Gln Pro Val Ser
   225                 230                 235                 240
   Ala Pro Pro Ser Pro Arg Asp Ile Ser Met Glu Ile Asp Ser Pro Glu
                   245                 250                 255
   Asn Met Met Arg His Ile Arg Phe Leu Lys Asn Glu Val Glu Arg Leu
               260                 265                 270
   Lys Lys Gln Leu Arg Ala Ala Gln Leu Gln
           275                 280
<210> 6
   <211> 5
   <212> PRT
   <213> Artificial Sequence
<220>
   <223> Break-point of CCDC6 protein fragment
<400> 6
   Ala Ala Gln Leu Gln
   1 5
<210> 7
   <211> 7044
   <212> DNA
   <213> Artificial Sequence
<220>
   <223> CDS fo ROS1 gene(NM_002944)
<400> 7
   atgaagaaca tttactgtct tattccgaag cttgtcaatt ttgcaactct tggctgccta        60
   tggatttctg tggtgcagtg tacagtttta aatagctgcc taaagtcgtg tgtaactaat       120
   ctgggccagc agcttgacct tggcacacca cataatctga gtgaaccgtg tatccaagga       180
   tgtcactttt ggaactctgt agatcagaaa aactgtgctt taaagtgtcg ggagtcgtgt       240
   gaggttggct gtagcagcgc ggaaggtgca tatgaagagg aagtactgga aaatgcagac       300
   ctaccaactg ctccctttgc ttcttccatt ggaagccaca atatgacatt acgatggaaa       360
   tctgcaaact tctctggagt aaaatacatc attcagtgga aatatgcaca acttctggga       420
   agctggactt atactaagac tgtgtccaga ccgtcctatg tggtcaagcc cctgcacccc       480
   ttcactgagt acattttccg agtggtttgg atcttcacag cgcagctgca gctctactcc       540
   cctccaagtc ccagttacag gactcatcct catggagttc ctgaaactgc acctttgatt       600
   aggaatattg agagctcaag tcccgacact gtggaagtca gctgggatcc acctcaattc       660
   ccaggtggac ctattttggg ttataactta aggctgatca gcaaaaatca aaaattagat       720
   gcagggacac agagaaccag tttccagttt tactccactt taccaaatac tatctacagg       780
   ttttctattg cagcagtaaa tgaagttggt gagggtccag aagcagaatc tagtattacc       840
   acttcatctt cagcagttca acaagaggaa cagtggctct ttttatccag aaaaacttct        900
   ctaagaaaga gatctttaaa acatttagta gatgaagcac attgccttcg gttggatgct       960
   atataccata atattacagg aatatctgtt gatgtccacc agcaaattgt ttatttctct      1020
   gaaggaactc tcatatgggc gaagaaggct gccaacatgt ctgatgtatc tgacctgaga      1080
   attttttaca gaggttcagg attaatttct tctatctcca tagattggct ttatcaaaga       1140
   atgtatttca tcatggatga actggtatgt gtctgtgatt tagagaactg ctcaaacatc      1200
   gaggaaatta ctccaccctc tattagtgca cctcaaaaaa ttgtggctga ttcatacaat      1260
   gggtatgtct tttacctcct gagagatggc atttatagag cagaccttcc tgtaccatct      1320
   ggccggtgtg cagaagctgt gcgtattgtg gagagttgca cgttaaagga ctttgcaatc      1380
   aagccacaag ccaagcgaat catttacttc aatgacactg cccaagtctt catgtcaaca      1440
   tttctggatg gctctgcttc ccatctcatc ctacctcgca tcccctttgc tgatgtgaaa      1500
   agttttgctt gtgaaaacaa tgactttctt gtcacagatg gcaaggtcat tttccaacag      1560
   gatgctttgt cttttaatga attcatcgtg ggatgtgacc tgagtcacat agaagaattt      1620
   gggtttggta acttggtcat ctttggctca tcctcccagc tgcaccctct gccaggccgc      1680
   ccgcaggagc tttcggtgct gtttggctct caccaggctc ttgttcaatg gaagcctcct      1740
   gcccttgcca taggagccaa tgtcatcctg atcagtgata ttattgaact ctttgaatta      1800
   ggcccttctg cctggcagaa ctggacctat gaggtgaaag tatccaccca agaccctcct      1860
   gaagtcactc atattttctt gaacataagt ggaaccatgc tgaatgtacc tgagctgcag 1920
   agtgctatga aatacaaggt ttctgtgaga gcaagttctc caaagaggcc aggcccctgg 1980
   tcagagccct cagtgggtac taccctggtg ccagctagtg aaccaccatt tatcatggct 2040
   gtgaaagaag atgggctttg gagtaaacca ttaaatagct ttggcccagg agagttctta 2100
   tcctctgata taggaaatgt gtcagacatg gattggtata acaacagcct ctactacagt 2160
   gacacgaaag gcgacgtttt tgtgtggctg ctgaatggga cggatatctc agagaattat 2220
   cacctaccca gcattgcagg agcaggggct ttagcttttg agtggctggg tcactttctc 2280
   tactgggctg gaaagacata tgtgatacaa aggcagtctg tgttgacggg acacacagac 2340
   attgttaccc acgtgaagct attggtgaat gacatggtgg tggattcagt tggtggatat 2400
   ctctactgga ccacactcta ttcagtggaa agcaccagac taaatgggga aagttccctt 2460
   gtactacaga cacagccttg gttttctggg aaaaaggtaa ttgctctaac tttagacctc 2520
   agtgatgggc tcctgtattg gttggttcaa gacagtcaat gtattcacct gtacacagct 2580
   gttcttcggg gacagagcac tggggatacc accatcacag aatttgcagc ctggagtact 2640
   tctgaaattt cccagaatgc actgatgtac tatagtggtc ggctgttctg gatcaatggc 2700
   tttaggatta tcacaactca agaaataggt cagaaaacca gtgtctctgt tttggaacca 2760
   gccagattta atcagttcac aattattcag acatccctta agcccctgcc agggaacttt 2820
   tcctttaccc ctaaggttat tccagattct gttcaagagt cttcatttag gattgaagga 2880
   aatgcttcaa gttttcaaat cctgtggaat ggtccccctg cggtagactg gggtgtagtt 2940
   ttctacagtg tagaatttag tgctcattct aagttcttgg ctagtgaaca acactcttta 3000
   cctgtattta ctgtggaagg actggaacct tatgccttat ttaatctttc tgtcactcct 3060
   tatacctact ggggaaaggg ccccaaaaca tctctgtcac ttcgagcacc tgaaacagtt 3120
   ccatcagcac cagagaaccc cagaatattt atattaccaa gtggaaaatg ctgcaacaag 3180
   aatgaagttg tggtggaatt taggtggaac aaacctaagc atgaaaatgg ggtgttaaca 3240
   aaatttgaaa ttttctacaa tatatccaat caaagtatta caaacaaaac atgtgaagac 3300
   tggattgctg tcaatgtcac tccctcagtg atgtcttttc aacttgaagg catgagtccc 3360
   agatgcttta ttgccttcca ggttagggcc tttacatcta aggggccagg accatatgct 3420
   gacgttgtaa agtctacaac atcagaaatc aacccatttc ctcacctcat aactcttctt 3480
   ggtaacaaga tagttttttt agatatggat caaaatcaag ttgtgtggac gttttcagca 3540
   gaaagagtta tcagtgccgt ttgctacaca gctgataatg agatgggata ttatgctgaa 3600
   ggggactcac tctttcttct gcacttgcac aatcgctcta gctctgagct tttccaagat 3660
   tcactggttt ttgatatcac agttattaca attgactgga tttcaaggca cctctacttt 3720
   gcactgaaag aatcacaaaa tggaatgcaa gtatttgatg ttgatcttga acacaaggtg 3780
   aaatatccca gagaggtgaa gattcacaat aggaattcaa caataatttc tttttctgta 3840
   tatcctcttt taagtcgctt gtattggaca gaagtttcca attttggcta ccagatgttc 3900
   tactacagta ttatcagtca caccttgcac cgaattctgc aacccacagc tacaaaccaa 3960
   caaaacaaaa ggaatcaatg ttcttgtaat gtgactgaat ttgagttaag tggagcaatg 4020
   gctattgata cctctaacct agagaaacca ttgatatact ttgccaaagc acaagagatc 4080
   tgggcaatgg atctggaagg ctgtcagtgt tggagagtta tcacagtacc tgctatgctc 4140
   gcaggaaaaa cccttgttag cttaactgtg gatggagatc ttatatactg gatcatcaca 4200
   gcaaaggaca gcacacagat ttatcaggca aagaaaggaa atggggccat cgtttcccag 4260
   gtgaaggccc taaggagtag gcatatcttg gcttacagtt cagttatgca gccttttcca 4320
   gataaagcgt ttctgtctct agcttcagac actgtggaac caactatact taatgccact 4380
   aacactagcc tcacaatcag attacctctg gccaagacaa acctcacatg gtatggcatc 4440
   accagcccta ctccaacata cctggtttat tatgcagaag ttaatgacag gaaaaacagc 4500
   tctgacttga aatatagaat tctggaattt caggacagta tagctcttat tgaagattta 4560
   caaccatttt caacatacat gatacagata gctgtaaaaa attattattc agatcctttg 4620
   gaacatttac caccaggaaa agagatttgg ggaaaaacta aaaatggagt accagaggca 4680
   gtgcagctca ttaatacaac tgtgcggtca gacaccagcc tcattatatc ttggagagaa 4740
   tctcacaagc caaatggacc taaagaatca gtccgttatc agttggcaat ctcacacctg 4800
   gccctaattc ctgaaactcc tctaagacaa agtgaatttc caaatggaag gctcactctc 4860
   cttgttacta gactgtctgg tggaaatatt tatgtgttaa aggttcttgc ctgccactct 4920
   gaggaaatgt ggtgtacaga gagtcatcct gtcactgtgg aaatgtttaa cacaccagag 4980
   aaaccttatt ccttggttcc agagaacact agtttgcaat ttaattggaa ggctccattg 5040
   aatgttaacc tcatcagatt ttgggttgag ctacagaagt ggaaatacaa tgagttttac 5100
   catgttaaaa cttcatgcag ccaaggtcct gcttatgtct gtaatatcac aaatctacaa 5160
   ccttatactt catataatgt cagagtagtg gtggtttata agacgggaga aaatagcacc 5220
   tcacttccag aaagctttaa gacaaaagct ggagtcccaa ataaaccagg cattcccaaa 5280
   ttactagaag ggagtaaaaa ttcaatacag tgggagaaag ctgaagataa tggatgtaga 5340
   attacatact atatccttga gataagaaag agcacttcaa ataatttaca gaaccagaat 5400
   ttaaggtgga agatgacatt taatggatcc tgcagtagtg tttgcacatg gaagtccaaa 5460
   aacctgaaag gaatatttca gttcagagta gtagctgcaa ataatctagg gtttggtgaa 5520
   tatagtggaa tcagtgagaa tattatatta gttggagatg atttttggat accagaaaca 5580
   agtttcatac ttactattat agttggaata tttctggttg ttacaatccc actgaccttt 5640
   gtctggcata gaagattaaa gaatcaaaaa agtgccaagg aaggggtgac agtgcttata 5700
   aacgaagaca aagagttggc tgagctgcga ggtctggcag ccggagtagg cctggctaat 5760
   gcctgctatg caatacatac tcttccaacc caagaggaga ttgaaaatct tcctgccttc 5820
   cctcgggaaa aactgactct gcgtctcttg ctgggaagtg gagcctttgg agaagtgtat 5880
   gaaggaacag cagtggacat cttaggagtt ggaagtggag aaatcaaagt agcagtgaag 5940
   actttgaaga agggttccac agaccaggag aagattgaat tcctgaagga ggcacatctg 6000
   atgagcaaat ttaatcatcc caacattctg aagcagcttg gagtttgtct gctgaatgaa 6060
   ccccaataca ttatcctgga actgatggag ggaggagacc ttcttactta tttgcgtaaa 6120
   gcccggatgg caacgtttta tggtccttta ctcaccttgg ttgaccttgt agacctgtgt 6180
   gtagatattt caaaaggctg tgtctacttg gaacggatgc atttcattca cagggatctg 6240
   gcagctagaa attgccttgt ttccgtgaaa gactatacca gtccacggat agtgaagatt 6300
   ggagactttg gactcgccag agacatctat aaaaatgatt actatagaaa gagaggggaa 6360
   ggcctgctcc cagttcggtg gatggctcca gaaagtttga tggatggaat cttcactact 6420
   caatctgatg tatggtcttt tggaattctg atttgggaga ttttaactct tggtcatcag 6480
   ccttatccag ctcattccaa ccttgatgtg ttaaactatg tgcaaacagg agggagactg 6540
   gagccaccaa gaaattgtcc tgatgatctg tggaatttaa tgacccagtg ctgggctcaa 6600
   gaacccgacc aaagacctac ttttcataga attcaggacc aacttcagtt attcagaaat 6660
   tttttcttaa atagcattta taagtccaga gatgaagcaa acaacagtgg agtcataaat 6720
   gaaagctttg aaggtgaaga tggcgatgtg atttgtttga attcagatga cattatgcca 6780
   gttgctttaa tggaaacgaa gaaccgagaa gggttaaact atatggtact tgctacagaa 6840
   tgtggccaag gtgaagaaaa gtctgagggt cctctaggct cccaggaatc tgaatcttgt 6900
   ggtctgagga aagaagagaa ggaaccacat gcagacaaag atttctgcca agaaaaacaa 6960
   gtggcttact gcccttctgg caagcctgaa ggcctgaact atgcctgtct cactcacagt 7020
   ggatatggag atgggtctga ttaa 7044
<210> 8
   <211> 1403
   <212> DNA
   <213> Artificial Sequence
<220>
   <223> ROS1 gene fragment
<400> 8
   tctggcatag aagattaaag aatcaaaaaa gtgccaagga aggggtgaca gtgcttataa        60
   acgaagacaa agagttggct gagctgcgag gtctggcagc cggagtaggc ctggctaatg       120
   cctgctatgc aatacatact cttccaaccc aagaggagat tgaaaatctt cctgccttcc       180
   ctcgggaaaa actgactctg cgtctcttgc tgggaagtgg agcctttgga gaagtgtatg       240
   aaggaacagc agtggacatc ttaggagttg gaagtggaga aatcaaagta gcagtgaaga       300
   ctttgaagaa gggttccaca gaccaggaga agattgaatt cctgaaggag gcacatctga       360
   tgagcaaatt taatcatccc aacattctga agcagcttgg agtttgtctg ctgaatgaac       420
   cccaatacat tatcctggaa ctgatggagg gaggagacct tcttacttat ttgcgtaaag       480
   cccggatggc aacgttttat ggtcctttac tcaccttggt tgaccttgta gacctgtgtg       540
   tagatatttc aaaaggctgt gtctacttgg aacggatgca tttcattcac agggatctgg       600
   cagctagaaa ttgccttgtt tccgtgaaag actataccag tccacggata gtgaagattg       660
   gagactttgg actcgccaga gacatctata aaaatgatta ctatagaaag agaggggaag       720
   gcctgctccc agttcggtgg atggctccag aaagtttgat ggatggaatc ttcactactc       780
   aatctgatgt atggtctttt ggaattctga tttgggagat tttaactctt ggtcatcagc       840
   cttatccagc tcattccaac cttgatgtgt taaactatgt gcaaacagga gggagactgg       900
   agccaccaag aaattgtcct gatgatctgt ggaatttaat gacccagtgc tgggctcaag       960
   aacccgacca aagacctact tttcatagaa ttcaggacca acttcagtta ttcagaaatt      1020
   ttttcttaaa tagcatttat aagtccagag atgaagcaaa caacagtgga gtcataaatg      1080
   aaagctttga aggtgaagat ggcgatgtga tttgtttgaa ttcagatgac attatgccag      1140
   ttgctttaat ggaaacgaag aaccgagaag ggttaaacta tatggtactt gctacagaat      1200
   gtggccaagg tgaagaaaag tctgagggtc ctctaggctc ccaggaatct gaatcttgtg      1260
   gtctgaggaa agaagagaag gaaccacatg cagacaaaga tttctgccaa gaaaaacaag      1320
   tggcttactg cccttctggc aagcctgaag gcctgaacta tgcctgtctc actcacagtg      1380
   gatatggaga tgggtctgat taa                                               1403
<210> 9
   <211> 17
   <212> DNA
   <213> Artificial Sequence
<220>
   <223> Break-point oof ROS1 gene fragment
<400> 9
   tctggcatag aagatta 17
<210> 10
   <211> 2347
   <212> PRT
   <213> Artificial Sequence
<220>
   <223> ROS1 protein
<400> 10
   Met Lys Asn Ile Tyr Cys Leu Ile Pro Lys Leu Val Asn Phe Ala Thr
    1               5                  10                  15
   Leu Gly Cys Leu Trp Ile Ser Val Val Gln Cys Thr Val Leu Asn Ser
               20                  25                  30
   Cys Leu Lys Ser Cys Val Thr Asn Leu Gly Gln Gln Leu Asp Leu Gly
           35                  40                  45
   Thr Pro His Asn Leu Ser Glu Pro Cys Ile Gln Gly Cys His Phe Trp
       50                  55                  60
   Asn Ser Val Asp Gln Lys Asn Cys Ala Leu Lys Cys Arg Glu Ser Cys
   65                  70                  75                  80
   Glu Val Gly Cys Ser Ser Ala Glu Gly Ala Tyr Glu Glu Glu Val Leu
                   85                  90                  95
   Glu Asn Ala Asp Leu Pro Thr Ala Pro Phe Ala Ser Ser Ile Gly Ser
               100                 105                 110
   His Asn Met Thr Leu Arg Trp Lys Ser Ala Asn Phe Ser Gly Val Lys
           115                 120                 125
   Tyr Ile Ile Gln Trp Lys Tyr Ala Gln Leu Leu Gly Ser Trp Thr Tyr
       130                 135                 140
   Thr Lys Thr Val Ser Arg Pro Ser Tyr Val Val Lys Pro Leu His Pro
   145                 150                 155                 160
   Phe Thr Glu Tyr Ile Phe Arg Val Val Trp Ile Phe Thr Ala Gln Leu
                   165                 170                 175
   Gln Leu Tyr Ser Pro Pro Ser Pro Ser Tyr Arg Thr His Pro His Gly
               180                 185                 190
   Val Pro Glu Thr Ala Pro Leu Ile Arg Asn Ile Glu Ser Ser Ser Pro
           195                 200                 205
   Asp Thr Val Glu Val Ser Trp Asp Pro Pro Gln Phe Pro Gly Gly Pro
       210                 215                 220
   Ile Leu Gly Tyr Asn Leu Arg Leu Ile Ser Lys Asn Gln Lys Leu Asp
   225                 230                 235                 240
   Ala Gly Thr Gln Arg Thr Ser Phe Gln Phe Tyr Ser Thr Leu Pro Asn
                   245                 250                 255
   Thr Ile Tyr Arg Phe Ser Ile Ala Ala Val Asn Glu Val Gly Glu Gly
               260                 265                 270
   Pro Glu Ala Glu Ser Ser Ile Thr Thr Ser Ser Ser Ala Val Gln Gln
           275                 280                 285
   Glu Glu Gln Trp Leu Phe Leu Ser Arg Lys Thr Ser Leu Arg Lys Arg
       290                 295                 300
   Ser Leu Lys His Leu Val Asp Glu Ala His Cys Leu Arg Leu Asp Ala
   305                 310                 315                 320
   Ile Tyr His Asn Ile Thr Gly Ile Ser Val Asp Val His Gln Gln Ile
                   325                 330                 335
   Val Tyr Phe Ser Glu Gly Thr Leu Ile Trp Ala Lys Lys Ala Ala Asn
               340                 345                 350
   Met Ser Asp Val Ser Asp Leu Arg Ile Phe Tyr Arg Gly Ser Gly Leu
           355                 360                 365
   Ile Ser Ser Ile Ser Ile Asp Trp Leu Tyr Gln Arg Met Tyr Phe Ile
       370                 375                 380
   Met Asp Glu Leu Val Cys Val Cys Asp Leu Glu Asn Cys Ser Asn Ile
   385                 390                 395                 400
   Glu Glu Ile Thr Pro Pro Ser Ile Ser Ala Pro Gln Lys Ile Val Ala
                   405                 410                 415
   Asp Ser Tyr Asn Gly Tyr Val Phe Tyr Leu Leu Arg Asp Gly Ile Tyr
               420                 425                 430
   Arg Ala Asp Leu Pro Val Pro Ser Gly Arg Cys Ala Glu Ala Val Arg
           435                 440                 445
   Ile Val Glu Ser Cys Thr Leu Lys Asp Phe Ala Ile Lys Pro Gln Ala
       450                 455                 460
   Lys Arg Ile Ile Tyr Phe Asn Asp Thr Ala Gln Val Phe Met Ser Thr
   465                 470                 475                 480
   Phe Leu Asp Gly Ser Ala Ser His Leu Ile Leu Pro Arg Ile Pro Phe
                   485                 490                 495
   Ala Asp Val Lys Ser Phe Ala Cys Glu Asn Asn Asp Phe Leu Val Thr
               500                 505                 510
   Asp Gly Lys Val Ile Phe Gln Gln Asp Ala Leu Ser Phe Asn Glu Phe
           515                 520                 525
   Ile Val Gly Cys Asp Leu Ser His Ile Glu Glu Phe Gly Phe Gly Asn
       530                 535                 540
   Leu Val Ile Phe Gly Ser Ser Ser Gln Leu His Pro Leu Pro Gly Arg
   545                 550                 555                 560
   Pro Gln Glu Leu Ser Val Leu Phe Gly Ser His Gln Ala Leu Val Gln
                   565                 570                 575
   Trp Lys Pro Pro Ala Leu Ala Ile Gly Ala Asn Val Ile Leu Ile Ser
               580                 585                 590
   Asp Ile Ile Glu Leu Phe Glu Leu Gly Pro Ser Ala Trp Gln Asn Trp
           595                 600                 605
   Thr Tyr Glu Val Lys Val Ser Thr Gln Asp Pro Pro Glu Val Thr His
       610                 615                 620
   Ile Phe Leu Asn Ile Ser Gly Thr Met Leu Asn Val Pro Glu Leu Gln
   625                 630                 635                 640
   Ser Ala Met Lys Tyr Lys Val Ser Val Arg Ala Ser Ser Pro Lys Arg
   645 650 655
   Pro Gly Pro Trp Ser Glu Pro Ser Val Gly Thr Thr Leu Val Pro Ala
   660 665 670
   Ser Glu Pro Pro Phe Ile Met Ala Val Lys Glu Asp Gly Leu Trp Ser
   675 680 685
   Lys Pro Leu Asn Ser Phe Gly Pro Gly Glu Phe Leu Ser Ser Asp Ile
   690 695 700
   Gly Asn Val Ser Asp Met Asp Trp Tyr Asn Asn Ser Leu Tyr Tyr Ser
   705 710 715 720
   Asp Thr Lys Gly Asp Val Phe Val Trp Leu Leu Asn Gly Thr Asp Ile
   725 730 735
   Ser Glu Asn Tyr His Leu Pro Ser Ile Ala Gly Ala Gly Ala Leu Ala
   740 745 750
   Phe Glu Trp Leu Gly His Phe Leu Tyr Trp Ala Gly Lys Thr Tyr Val
   755 760 765
   Ile Gln Arg Gln Ser Val Leu Thr Gly His Thr Asp Ile Val Thr His
   770 775 780
   Val Lys Leu Leu Val Asn Asp Met Val Val Asp Ser Val Gly Gly Tyr
   785 790 795 800
   Leu Tyr Trp Thr Thr Leu Tyr Ser Val Glu Ser Thr Arg Leu Asn Gly
   805 810 815
   Glu Ser Ser Leu Val Leu Gln Thr Gln Pro Trp Phe Ser Gly Lys Lys
   820 825 830
   Val Ile Ala Leu Thr Leu Asp Leu Ser Asp Gly Leu Leu Tyr Trp Leu
   835 840 845
   Val Gln Asp Ser Gln Cys Ile His Leu Tyr Thr Ala Val Leu Arg Gly
   850 855 860
   Gln Ser Thr Gly Asp Thr Thr Ile Thr Glu Phe Ala Ala Trp Ser Thr
   865 870 875 880
   Ser Glu Ile Ser Gln Asn Ala Leu Met Tyr Tyr Ser Gly Arg Leu Phe
   885 890 895
   Trp Ile Asn Gly Phe Arg Ile Ile Thr Thr Gln Glu Ile Gly Gln Lys
   900 905 910
   Thr Ser Val Ser Val Leu Glu Pro Ala Arg Phe Asn Gln Phe Thr Ile
   915 920 925
   Ile Gln Thr Ser Leu Lys Pro Leu Pro Gly Asn Phe Ser Phe Thr Pro
   930 935 940
   Lys Val Ile Pro Asp Ser Val Gln Glu Ser Ser Phe Arg Ile Glu Gly
   945 950 955 960
   Asn Ala Ser Ser Phe Gln Ile Leu Trp Asn Gly Pro Pro Ala Val Asp
   965 970 975
   Trp Gly Val Val Phe Tyr Ser Val Glu Phe Ser Ala His Ser Lys Phe
               980                 985                 990
   Leu Ala Ser Glu Gln His Ser Leu Pro Val Phe Thr Val Glu Gly Leu
           995                 1000                1005
   Glu Pro Tyr Ala Leu Phe Asn Leu Ser Val Thr Pro Tyr Thr Tyr Trp
       1010                1015                1020
   Gly Lys Gly Pro Lys Thr Ser Leu Ser Leu Arg Ala Pro Glu Thr Val
   1025                1030                1035                1040
   Pro Ser Ala Pro Glu Asn Pro Arg Ile Phe Ile Leu Pro Ser Gly Lys
                   1045                1050                1055
   Cys Cys Asn Lys Asn Glu Val Val Val Glu Phe Arg Trp Asn Lys Pro
               1060                1065                1070
   Lys His Glu Asn Gly Val Leu Thr Lys Phe Glu Ile Phe Tyr Asn Ile
           1075                1080                1085
   Ser Asn Gln Ser Ile Thr Asn Lys Thr Cys Glu Asp Trp Ile Ala Val
       1090                1095                1100
   Asn Val Thr Pro Ser Val Met Ser Phe Gln Leu Glu Gly Met Ser Pro
   1105                1110                1115                1120
   Arg Cys Phe Ile Ala Phe Gln Val Arg Ala Phe Thr Ser Lys Gly Pro
                   1125                1130                1135
   Gly Pro Tyr Ala Asp Val Val Lys Ser Thr Thr Ser Glu Ile Asn Pro
               1140                1145                1150
   Phe Pro His Leu Ile Thr Leu Leu Gly Asn Lys Ile Val Phe Leu Asp
           1155                1160                1165
   Met Asp Gln Asn Gln Val Val Trp Thr Phe Ser Ala Glu Arg Val Ile
       1170                1175                1180
   Ser Ala Val Cys Tyr Thr Ala Asp Asn Glu Met Gly Tyr Tyr Ala Glu
   1185                1190                1195                1200
   Gly Asp Ser Leu Phe Leu Leu His Leu His Asn Arg Ser Ser Ser Glu
                   1205                1210                1215
   Leu Phe Gln Asp Ser Leu Val Phe Asp Ile Thr Val Ile Thr Ile Asp
               1220                1225                1230
   Trp Ile Ser Arg His Leu Tyr Phe Ala Leu Lys Glu Ser Gln Asn Gly
           1235                1240                1245
   Met Gln Val Phe Asp Val Asp Leu Glu His Lys Val Lys Tyr Pro Arg
       1250                1255                1260
   Glu Val Lys Ile His Asn Arg Asn Ser Thr Ile Ile Ser Phe Ser Val
   1265                1270                1275                1280
   Tyr Pro Leu Leu Ser Arg Leu Tyr Trp Thr Glu Val Ser Asn Phe Gly
                   1285                1290                1295
   Tyr Gln Met Phe Tyr Tyr Ser Ile Ile Ser His Thr Leu His Arg Ile
               1300                1305                1310
   Leu Gln Pro Thr Ala Thr Asn Gln Gln Asn Lys Arg Asn Gln Cys Ser
          1315                1320                1325
   Cys Asn Val Thr Glu Phe Glu Leu Ser Gly Ala Met Ala Ile Asp Thr
      1330                1335                1340
   Ser Asn Leu Glu Lys Pro Leu Ile Tyr Phe Ala Lys Ala Gln Glu Ile
   1345                1350                1355                1360
   Trp Ala Met Asp Leu Glu Gly Cys Gln Cys Trp Arg Val Ile Thr Val
                  1365                1370                1375
   Pro Ala Met Leu Ala Gly Lys Thr Leu Val Ser Leu Thr Val Asp Gly
              1380                1385                1390
   Asp Leu Ile Tyr Trp Ile Ile Thr Ala Lys Asp Ser Thr Gln Ile Tyr
          1395                1400                1405
   Gln Ala Lys Lys Gly Asn Gly Ala Ile Val Ser Gln Val Lys Ala Leu
      1410                1415                1420
   Arg Ser Arg His Ile Leu Ala Tyr Ser Ser Val Met Gln Pro Phe Pro
   1425                1430                1435                1440
   Asp Lys Ala Phe Leu Ser Leu Ala Ser Asp Thr Val Glu Pro Thr Ile
                  1445                1450                1455
   Leu Asn Ala Thr Asn Thr Ser Leu Thr Ile Arg Leu Pro Leu Ala Lys
              1460                1465                1470
   Thr Asn Leu Thr Trp Tyr Gly Ile Thr Ser Pro Thr Pro Thr Tyr Leu
          1475                1480                1485
   Val Tyr Tyr Ala Glu Val Asn Asp Arg Lys Asn Ser Ser Asp Leu Lys
      1490                1495                1500
   Tyr Arg Ile Leu Glu Phe Gln Asp Ser Ile Ala Leu Ile Glu Asp Leu
   1505                1510                1515                1520
   Gln Pro Phe Ser Thr Tyr Met Ile Gln Ile Ala Val Lys Asn Tyr Tyr
                  1525                1530                1535
   Ser Asp Pro Leu Glu His Leu Pro Pro Gly Lys Glu Ile Trp Gly Lys
              1540                1545                1550
   Thr Lys Asn Gly Val Pro Glu Ala Val Gln Leu Ile Asn Thr Thr Val
          1555                1560                1565
   Arg Ser Asp Thr Ser Leu Ile Ile Ser Trp Arg Glu Ser His Lys Pro
      1570                1575                1580
   Asn Gly Pro Lys Glu Ser Val Arg Tyr Gln Leu Ala Ile Ser His Leu
   1585                1590                1595                1600
   Ala Leu Ile Pro Glu Thr Pro Leu Arg Gln Ser Glu Phe Pro Asn Gly
                  1605                1610                1615
   Arg Leu Thr Leu Leu Val Thr Arg Leu Ser Gly Gly Asn Ile Tyr Val
              1620                1625                1630
   Leu Lys Val Leu Ala Cys His Ser Glu Glu Met Trp Cys Thr Glu Ser
          1635                1640                1645
   His Pro Val Thr Val Glu Met Phe Asn Thr Pro Glu Lys Pro Tyr Ser
      1650                1655                1660
   Leu Val Pro Glu Asn Thr Ser Leu Gln Phe Asn Trp Lys Ala Pro Leu
   1665                1670                1675                1680
   Asn Val Asn Leu Ile Arg Phe Trp Val Glu Leu Gln Lys Trp Lys Tyr
                  1685                1690                1695
   Asn Glu Phe Tyr His Val Lys Thr Ser Cys Ser Gln Gly Pro Ala Tyr
              1700                1705                1710
   Val Cys Asn Ile Thr Asn Leu Gln Pro Tyr Thr Ser Tyr Asn Val Arg
          1715                1720                1725
   Val Val Val Val Tyr Lys Thr Gly Glu Asn Ser Thr Ser Leu Pro Glu
      1730                1735                1740
   Ser Phe Lys Thr Lys Ala Gly Val Pro Asn Lys Pro Gly Ile Pro Lys
   1745                1750                1755                1760
   Leu Leu Glu Gly Ser Lys Asn Ser Ile Gln Trp Glu Lys Ala Glu Asp
                  1765                1770                1775
   Asn Gly Cys Arg Ile Thr Tyr Tyr Ile Leu Glu Ile Arg Lys Ser Thr
              1780                1785                1790
   Ser Asn Asn Leu Gln Asn Gln Asn Leu Arg Trp Lys Met Thr Phe Asn
          1795                1800                1805
   Gly Ser Cys Ser Ser Val Cys Thr Trp Lys Ser Lys Asn Leu Lys Gly
      1810                1815                1820
   Ile Phe Gln Phe Arg Val Val Ala Ala Asn Asn Leu Gly Phe Gly Glu
   1825                1830                1835                1840
   Tyr Ser Gly Ile Ser Glu Asn Ile Ile Leu Val Gly Asp Asp Phe Trp
                  1845                1850                1855
   Ile Pro Glu Thr Ser Phe Ile Leu Thr Ile Ile Val Gly Ile Phe Leu
              1860                1865                1870
   Val Val Thr Ile Pro Leu Thr Phe Val Trp His Arg Arg Leu Lys Asn
          1875                1880                1885
   Gln Lys Ser Ala Lys Glu Gly Val Thr Val Leu Ile Asn Glu Asp Lys
      1890                1895                1900
   Glu Leu Ala Glu Leu Arg Gly Leu Ala Ala Gly Val Gly Leu Ala Asn
   1905                1910                1915                1920
   Ala Cys Tyr Ala Ile His Thr Leu Pro Thr Gln Glu Glu Ile Glu Asn
                  1925                1930                1935
   Leu Pro Ala Phe Pro Arg Glu Lys Leu Thr Leu Arg Leu Leu Leu Gly
              1940                1945                1950
   Ser Gly Ala Phe Gly Glu Val Tyr Glu Gly Thr Ala Val Asp Ile Leu
          1955                1960                1965
   Gly Val Gly Ser Gly Glu Ile Lys Val Ala Val Lys Thr Leu Lys Lys
      1970                1975                1980
   Gly Ser Thr Asp Gln Glu Lys Ile Glu Phe Leu Lys Glu Ala His Leu
   1985 1990 1995 2000
   Met Ser Lys Phe Asn His Pro Asn Ile Leu Lys Gln Leu Gly Val Cys
   2005 2010 2015
   Leu Leu Asn Glu Pro Gln Tyr Ile Ile Leu Glu Leu Met Glu Gly Gly
   2020 2025 2030
   Asp Leu Leu Thr Tyr Leu Arg Lys Ala Arg Met Ala Thr Phe Tyr Gly
   2035 2040 2045
   Pro Leu Leu Thr Leu Val Asp Leu Val Asp Leu Cys Val Asp Ile Ser
   2050 2055 2060
   Lys Gly Cys Val Tyr Leu Glu Arg Met His Phe Ile His Arg Asp Leu
   2065 2070 2075 2080
   Ala Ala Arg Asn Cys Leu Val Ser Val Lys Asp Tyr Thr Ser Pro Arg
   2085 2090 2095
   Ile Val Lys Ile Gly Asp Phe Gly Leu Ala Arg Asp Ile Tyr Lys Asn
   2100 2105 2110
   Asp Tyr Tyr Arg Lys Arg Gly Glu Gly Leu Leu Pro Val Arg Trp Met
   2115 2120 2125
   Ala Pro Glu Ser Leu Met Asp Gly Ile Phe Thr Thr Gln Ser Asp Val
   2130 2135 2140
   Trp Ser Phe Gly Ile Leu Ile Trp Glu Ile Leu Thr Leu Gly His Gln
   2145 2150 2155 2160
   Pro Tyr Pro Ala His Ser Asn Leu Asp Val Leu Asn Tyr Val Gln Thr
   2165 2170 2175
   Gly Gly Arg Leu Glu Pro Pro Arg Asn Cys Pro Asp Asp Leu Trp Asn
   2180 2185 2190
   Leu Met Thr Gln Cys Trp Ala Gln Glu Pro Asp Gln Arg Pro Thr Phe
   2195 2200 2205
   His Arg Ile Gln Asp Gln Leu Gln Leu Phe Arg Asn Phe Phe Leu Asn
   2210 2215 2220
   Ser Ile Tyr Lys Ser Arg Asp Glu Ala Asn Asn Ser Gly Val Ile Asn
   2225 2230 2235 2240
   Glu Ser Phe Glu Gly Glu Asp Gly Asp Val Ile Cys Leu Asn Ser Asp
   2245 2250 2255
   Asp Ile Met Pro Val Ala Leu Met Glu Thr Lys Asn Arg Glu Gly Leu
   2260 2265 2270
   Asn Tyr Met Val Leu Ala Thr Glu Cys Gly Gln Gly Glu Glu Lys Ser
   2275 2280 2285
   Glu Gly Pro Leu Gly Ser Gln Glu Ser Glu Ser Cys Gly Leu Arg Lys
   2290 2295 2300
   Glu Glu Lys Glu Pro His Ala Asp Lys Asp Phe Cys Gln Glu Lys Gln
   2305 2310 2315 2320
   Val Ala Tyr Cys Pro Ser Gly Lys Pro Glu Gly Leu Asn Tyr Ala Cys
                   2325                2330                2335
   Leu Thr His Ser Gly Tyr Gly Asp Gly Ser Asp
               2340                2345
<210> 11
   <211> 466
   <212> PRT
   <213> Artificial Sequence
<220>
   <223> ROS1 protein fragment
<400> 11
   Trp His Arg Arg Leu Lys Asn Gln Lys Ser Ala Lys Glu Gly Val Thr
   1               5                   10                  15
   Val Leu Ile Asn Glu Asp Lys Glu Leu Ala Glu Leu Arg Gly Leu Ala
               20                  25                  30
   Ala Gly Val Gly Leu Ala Asn Ala Cys Tyr Ala Ile His Thr Leu Pro
           35                  40                  45
   Thr Gln Glu Glu Ile Glu Asn Leu Pro Ala Phe Pro Arg Glu Lys Leu
       50                  55                  60
   Thr Leu Arg Leu Leu Leu Gly Ser Gly Ala Phe Gly Glu Val Tyr Glu
   65                  70                  75                  80
   Gly Thr Ala Val Asp Ile Leu Gly Val Gly Ser Gly Glu Ile Lys Val
                   85                  90                  95
   Ala Val Lys Thr Leu Lys Lys Gly Ser Thr Asp Gln Glu Lys Ile Glu
               100                 105                 110
   Phe Leu Lys Glu Ala His Leu Met Ser Lys Phe Asn His Pro Asn Ile
           115                 120                 125
   Leu Lys Gln Leu Gly Val Cys Leu Leu Asn Glu Pro Gln Tyr Ile Ile
       130                 135                 140
   Leu Glu Leu Met Glu Gly Gly Asp Leu Leu Thr Tyr Leu Arg Lys Ala
   145                 150                 155                 160
   Arg Met Ala Thr Phe Tyr Gly Pro Leu Leu Thr Leu Val Asp Leu Val
                   165                 170                 175
   Asp Leu Cys Val Asp Ile Ser Lys Gly Cys Val Tyr Leu Glu Arg Met
               180                 185                 190
   His Phe Ile His Arg Asp Leu Ala Ala Arg Asn Cys Leu Val Ser Val
           195                 200                 205
   Lys Asp Tyr Thr Ser Pro Arg Ile Val Lys Ile Gly Asp Phe Gly Leu
       210                 215                 220
   Ala Arg Asp Ile Tyr Lys Asn Asp Tyr Tyr Arg Lys Arg Gly Glu Gly
   225                 230                 235                 240
   Leu Leu Pro Val Arg Trp Met Ala Pro Glu Ser Leu Met Asp Gly Ile
                   245                 250                 255
   Phe Thr Thr Gln Ser Asp Val Trp Ser Phe Gly Ile Leu Ile Trp Glu
               260                 265                 270
   Ile Leu Thr Leu Gly His Gln Pro Tyr Pro Ala His Ser Asn Leu Asp
           275                 280                 285
   Val Leu Asn Tyr Val Gln Thr Gly Gly Arg Leu Glu Pro Pro Arg Asn
       290                 295                 300
   Cys Pro Asp Asp Leu Trp Asn Leu Met Thr Gln Cys Trp Ala Gln Glu
   305                 310                 315                 320
   Pro Asp Gln Arg Pro Thr Phe His Arg Ile Gln Asp Gln Leu Gln Leu
                   325                 330                 335
   Phe Arg Asn Phe Phe Leu Asn Ser Ile Tyr Lys Ser Arg Asp Glu Ala
               340                 345                 350
   Asn Asn Ser Gly Val Ile Asn Glu Ser Phe Glu Gly Glu Asp Gly Asp
           355                 360                 365
   Val Ile Cys Leu Asn Ser Asp Asp Ile Met Pro Val Ala Leu Met Glu
       370                 375                 380
   Thr Lys Asn Arg Glu Gly Leu Asn Tyr Met Val Leu Ala Thr Glu Cys
   385                 390                 395                 400
   Gly Gln Gly Glu Glu Lys Ser Glu Gly Pro Leu Gly Ser Gln Glu Ser
                   405                 410                 415
   Glu Ser Cys Gly Leu Arg Lys Glu Glu Lys Glu Pro His Ala Asp Lys
               420                 425                 430
   Asp Phe Cys Gln Glu Lys Gln Val Ala Tyr Cys Pro Ser Gly Lys Pro
           435                 440                 445
   Glu Gly Leu Asn Tyr Ala Cys Leu Thr His Ser Gly Tyr Gly Asp Gly
       450                 455                 460
   Ser Asp
   465
<210> 12
   <211> 5
   <212> PRT
   <213> Artificial Sequence
<220>
   <223> Break-point of ROS1 protein fragment
<400> 12
   Trp His Arg Arg Leu
   1               5
<210> 13
   <211> 2250
   <212> DNA
   <213> Artificial Sequence
<220>
   <223> CCDC6-ROS1 fusion gene
<400> 13
   atggcggaca gcgccagcga gagcgacacg gacggggcgg ggggcaacag cagcagctcg 60
   gccgccatgc agtcgtcctg ctcgtcgacc tcgggcggcg gcggtggcgg cgggggaggc 120
   ggcggcggtg ggaagtcggg gggcattgtc atctcgccgt tccgcctgga ggagctcacc 180
   aaccgcctgg cctcgctgca gcaagagaac aaggtgctga agatagagct ggagacctac 240
   aaactgaagt gcaaggcact gcaggaggag aaccgcgacc tgcgcaaagc cagcgtgacc 300
   atccaagcca gggctgagca ggaagaagaa ttcattagta acactttatt caagaaaatt 360
   caggctttgc agaaggagaa agaaaccctt gctgtaaatt atgagaaaga agaagaattc 420
   ctcactaatg agctctccag aaaattgatg cagttgcagc atgagaaagc cgaactagaa 480
   cagcatcttg aacaagagca ggaatttcag gtcaacaaac tgatgaagaa aattaaaaaa 540
   ctggagaatg acaccatttc taagcaactt acattagaac agttgagacg ggagaagatt 600
   gaccttgaaa atacattgga acaagaacaa gaagcactag ttaatcgcct ctggaaaagg 660
   atggataagc ttgaagctga aaagcgaatc ctgcaggaaa aattagacca gcccgtctct 720
   gctccaccat cgcctagaga tatctccatg gagattgatt ctccagaaaa tatgatgcgt 780
   cacatcaggt ttttaaagaa tgaagtggaa cggctgaaga agcaactgag agctgctcag 840
   ttacagctct ggcatagaag attaaagaat caaaaaagtg ccaaggaagg ggtgacagtg 900
   cttataaacg aagacaaaga gttggctgag ctgcgaggtc tggcagccgg agtaggcctg 960
   gctaatgcct gctatgcaat acatactctt ccaacccaag aggagattga aaatcttcct 1020
   gccttccctc gggaaaaact gactctgcgt ctcttgctgg gaagtggagc ctttggagaa 1080
   gtgtatgaag gaacagcagt ggacatctta ggagttggaa gtggagaaat caaagtagca 1140
   gtgaagactt tgaagaaggg ttccacagac caggagaaga ttgaattcct gaaggaggca 1200
   catctgatga gcaaatttaa tcatcccaac attctgaagc agcttggagt ttgtctgctg 1260
   aatgaacccc aatacattat cctggaactg atggagggag gagaccttct tacttatttg 1320
   cgtaaagccc ggatggcaac gttttatggt cctttactca ccttggttga ccttgtagac 1380
   ctgtgtgtag atatttcaaa aggctgtgtc tacttggaac ggatgcattt cattcacagg 1440
   gatctggcag ctagaaattg ccttgtttcc gtgaaagact ataccagtcc acggatagtg 1500
   aagattggag actttggact cgccagagac atctataaaa atgattacta tagaaagaga 1560
   ggggaaggcc tgctcccagt tcggtggatg gctccagaaa gtttgatgga tggaatcttc 1620
   actactcaat ctgatgtatg gtcttttgga attctgattt gggagatttt aactcttggt 1680
   catcagcctt atccagctca ttccaacctt gatgtgttaa actatgtgca aacaggaggg 1740
   agactggagc caccaagaaa ttgtcctgat gatctgtgga atttaatgac ccagtgctgg 1800
   gctcaagaac ccgaccaaag acctactttt catagaattc aggaccaact tcagttattc 1860
   agaaattttt tcttaaatag catttataag tccagagatg aagcaaacaa cagtggagtc 1920
   ataaatgaaa gctttgaagg tgaagatggc gatgtgattt gtttgaattc agatgacatt 1980
   atgccagttg ctttaatgga aacgaagaac cgagaagggt taaactatat ggtacttgct 2040
   acagaatgtg gccaaggtga agaaaagtct gagggtcctc taggctccca ggaatctgaa 2100
   tcttgtggtc tgaggaaaga agagaaggaa ccacatgcag acaaagattt ctgccaagaa 2160
   aaacaagtgg cttactgccc ttctggcaag cctgaaggcc tgaactatgc ctgtctcact 2220
   cacagtggat atggagatgg gtctgattaa 2250
<210> 14
   <211> 33
   <212> DNA
   <213> Artificial Sequence
<220>
   <223> fused resion of CCDC6-ROS1 fusion gene
<400> 14
   gctgctcagt tacagctctg gcatagaaga tta 33
<210> 15
   <211> 749
   <212> PRT
   <213> Artificial Sequence
<220>
   <223> CCDC6-ROS1 fusion protein
<400> 15
   Met Ala Asp Ser Ala Ser Glu Ser Asp Thr Asp Gly Ala Gly Gly Asn
   1 5 10 15
   Ser Ser Ser Ser Ala Ala Met Gln Ser Ser Cys Ser Ser Thr Ser Gly
   20 25 30
   Gly Gly Gly Gly Gly Gly Gly Gly Gly Gly Gly Gly Lys Ser Gly Gly
   35 40 45
   Ile Val Ile Ser Pro Phe Arg Leu Glu Glu Leu Thr Asn Arg Leu Ala
   50 55 60
   Ser Leu Gln Gln Glu Asn Lys Val Leu Lys Ile Glu Leu Glu Thr Tyr
   65 70 75 80
   Lys Leu Lys Cys Lys Ala Leu Gln Glu Glu Asn Arg Asp Leu Arg Lys
   85 90 95
   Ala Ser Val Thr Ile Gln Ala Arg Ala Glu Gln Glu Glu Glu Phe Ile
   100 105 110
   Ser Asn Thr Leu Phe Lys Lys Ile Gln Ala Leu Gln Lys Glu Lys Glu
           115                 120                 125
   Thr Leu Ala Val Asn Tyr Glu Lys Glu Glu Glu Phe Leu Thr Asn Glu
       130                 135                 140
   Leu Ser Arg Lys Leu Met Gln Leu Gln His Glu Lys Ala Glu Leu Glu
   145                 150                 155                 160
   Gln His Leu Glu Gln Glu Gln Glu Phe Gln Val Asn Lys Leu Met Lys
                   165                 170                 175
   Lys Ile Lys Lys Leu Glu Asn Asp Thr Ile Ser Lys Gln Leu Thr Leu
               180                 185                 190
   Glu Gln Leu Arg Arg Glu Lys Ile Asp Leu Glu Asn Thr Leu Glu Gln
           195                 200                 205
   Glu Gln Glu Ala Leu Val Asn Arg Leu Trp Lys Arg Met Asp Lys Leu
       210                 215                 220
   Glu Ala Glu Lys Arg Ile Leu Gln Glu Lys Leu Asp Gln Pro Val Ser
   225                 230                 235                 240
   Ala Pro Pro Ser Pro Arg Asp Ile Ser Met Glu Ile Asp Ser Pro Glu
                   245                 250                 255
   Asn Met Met Arg His Ile Arg Phe Leu Lys Asn Glu Val Glu Arg Leu
               260                 265                 270
   Lys Lys Gln Leu Arg Ala Ala Gln Leu Gln Leu Trp His Arg Arg Leu
           275                 280                 285
   Lys Asn Gln Lys Ser Ala Lys Glu Gly Val Thr Val Leu Ile Asn Glu
       290                 295                 300
   Asp Lys Glu Leu Ala Glu Leu Arg Gly Leu Ala Ala Gly Val Gly Leu
   305                 310                 315                 320
   Ala Asn Ala Cys Tyr Ala Ile His Thr Leu Pro Thr Gln Glu Glu Ile
                   325                 330                 335
   Glu Asn Leu Pro Ala Phe Pro Arg Glu Lys Leu Thr Leu Arg Leu Leu
               340                 345                 350
   Leu Gly Ser Gly Ala Phe Gly Glu Val Tyr Glu Gly Thr Ala Val Asp
           355                 360                 365
   Ile Leu Gly Val Gly Ser Gly Glu Ile Lys Val Ala Val Lys Thr Leu
       370                 375                 380
   Lys Lys Gly Ser Thr Asp Gln Glu Lys Ile Glu Phe Leu Lys Glu Ala
   385                 390                 395                 400
   His Leu Met Ser Lys Phe Asn His Pro Asn Ile Leu Lys Gln Leu Gly
                   405                 410                 415
   Val Cys Leu Leu Asn Glu Pro Gln Tyr Ile Ile Leu Glu Leu Met Glu
               420                 425                 430
   Gly Gly Asp Leu Leu Thr Tyr Leu Arg Lys Ala Arg Met Ala Thr Phe
           435                 440                 445
   Tyr Gly Pro Leu Leu Thr Leu Val Asp Leu Val Asp Leu Cys Val Asp
       450                 455                 460
   Ile Ser Lys Gly Cys Val Tyr Leu Glu Arg Met His Phe Ile His Arg
   465                 470                 475                 480
   Asp Leu Ala Ala Arg Asn Cys Leu Val Ser Val Lys Asp Tyr Thr Ser
                   485                 490                 495
   Pro Arg Ile Val Lys Ile Gly Asp Phe Gly Leu Ala Arg Asp Ile Tyr
               500                 505                 510
   Lys Asn Asp Tyr Tyr Arg Lys Arg Gly Glu Gly Leu Leu Pro Val Arg
           515                 520                 525
   Trp Met Ala Pro Glu Ser Leu Met Asp Gly Ile Phe Thr Thr Gln Ser
       530                 535                 540
   Asp Val Trp Ser Phe Gly Ile Leu Ile Trp Glu Ile Leu Thr Leu Gly
   545                 550                 555                 560
   His Gln Pro Tyr Pro Ala His Ser Asn Leu Asp Val Leu Asn Tyr Val
                   565                 570                 575
   Gln Thr Gly Gly Arg Leu Glu Pro Pro Arg Asn Cys Pro Asp Asp Leu
               580                 585                 590
   Trp Asn Leu Met Thr Gln Cys Trp Ala Gln Glu Pro Asp Gln Arg Pro
           595                 600                 605
   Thr Phe His Arg Ile Gln Asp Gln Leu Gln Leu Phe Arg Asn Phe Phe
       610                 615                 620
   Leu Asn Ser Ile Tyr Lys Ser Arg Asp Glu Ala Asn Asn Ser Gly Val
   625                 630                 635                 640
   Ile Asn Glu Ser Phe Glu Gly Glu Asp Gly Asp Val Ile Cys Leu Asn
                   645                 650                 655
   Ser Asp Asp Ile Met Pro Val Ala Leu Met Glu Thr Lys Asn Arg Glu
               660                 665                 670
   Gly Leu Asn Tyr Met Val Leu Ala Thr Glu Cys Gly Gln Gly Glu Glu
           675                 680                 685
   Lys Ser Glu Gly Pro Leu Gly Ser Gln Glu Ser Glu Ser Cys Gly Leu
       690                 695                 700
   Arg Lys Glu Glu Lys Glu Pro His Ala Asp Lys Asp Phe Cys Gln Glu
   705                 710                 715                 720
   Lys Gln Val Ala Tyr Cys Pro Ser Gly Lys Pro Glu Gly Leu Asn Tyr
                   725                 730                 735
   Ala Cys Leu Thr His Ser Gly Tyr Gly Asp Gly Ser Asp
               740                 745
<210> 16
   <211> 11
   <212> PRT
   <213> Artificial Sequence
<220>
   <223> Fused region of CCDC6-ROS1 fusion protein
<400> 16
   Ala Ala Gln Leu Gln Leu Trp His Arg Arg Leu
   1               5                   10
<210> 17
   <211> 2130
   <212> DNA
   <213> Artificial Sequence
<220>
   <223> CDS of FGFR2 gene (NM_001144914)
<400> 17
   atggtcagct ggggtcgttt catctgcctg gtcgtggtca ccatggcaac cttgtccctg      60
   gcccggccct ccttcagttt agttgaggat accacattag agccagaaga gccaccaacc     120
   aaataccaaa tctctcaacc agaagtgtac gtggctgcgc caggggagtc gctagaggtg     180
   cgctgcctgt tgaaagatgc cgccgtgatc agttggacta aggatggggt gcacttgggg     240
   cccaacaata ggacagtgct tattggggag tacttgcaga taaagggcgc cacgcctaga     300
   gactccggcc tctatgcttg tactgccagt aggactgtag acagtgaaac ttggtacttc     360
   atggtgaatg tcacagatgc catctcatcc ggagatgatg aggatgacac cgatggtgcg     420
   gaagattttg tcagtgagaa cagtaacaac aagagagcac catactggac caacacagaa     480
   aagatggaaa agcggctcca tgctgtgcct gcggccaaca ctgtcaagtt tcgctgccca     540
   gccgggggga acccaatgcc aaccatgcgg tggctgaaaa acgggaagga gtttaagcag     600
   gagcatcgca ttggaggcta caaggtacga aaccagcact ggagcctcat tatggaaagt     660
   gtggtcccat ctgacaaggg aaattatacc tgtgtagtgg agaatgaata cgggtccatc     720
   aatcacacgt accacctgga tgttgtggcg cctggaagag aaaaggagat tacagcttcc     780
   ccagactacc tggagatagc catttactgc ataggggtct tcttaatcgc ctgtatggtg     840
   gtaacagtca tcctgtgccg aatgaagaac acgaccaaga agccagactt cagcagccag     900
   ccggctgtgc acaagctgac caaacgtatc cccctgcgga gacaggtaac agtttcggct     960
   gagtccagct cctccatgaa ctccaacacc ccgctggtga ggataacaac acgcctctct    1020
   tcaacggcag acacccccat gctggcaggg gtctccgagt atgaacttcc agaggaccca    1080
   aaatgggagt ttccaagaga taagctgaca ctgggcaagc cctgggagaa aggttgcttt    1140
   gggcaagtgg tcatggcgga agcagtggga attgacaaag acaagcccaa ggaggcggtc    1200
   accgtggccg tgaagatgtt gaaagatgat gccacagaga aagacctttc tgatctggtg    1260
   tcagagatgg agatgatgaa gatgattggg aaacacaaga atatcataaa tcttcttgga    1320
   gcctgcacac aggatgggcc tctctatgtc atagttgagt atgcctctaa aggcaacctc 1380
   cgagaatacc tccgagcccg gaggccaccc gggatggagt actcctatga cattaaccgt 1440
   gttcctgagg agcagatgac cttcaaggac ttggtgtcat gcacctacca gctggccaga 1500
   ggcatggagt acttggcttc ccaaaaatgt attcatcgag atttagcagc cagaaatgtt 1560
   ttggtaacag aaaacaatgt gatgaaaata gcagactttg gactcgccag agatatcaac 1620
   aatatagact attacaaaaa gaccaccaat gggcggcttc cagtcaagtg gatggctcca 1680
   gaagccctgt ttgatagagt atacactcat cagagtgatg tctggtcctt cggggtgtta 1740
   atgtgggaga tcttcacttt agggggctcg ccctacccag ggattcccgt ggaggaactt 1800
   tttaagctgc tgaaggaagg acacagaatg gataagccag ccaactgcac caacgaactg 1860
   tacatgatga tgagggactg ttggcatgca gtgccctccc agagaccaac gttcaagcag 1920
   ttggtagaag acttggatcg aattctcact ctcacaacca atgaggaata cttggacctc 1980
   agccaacctc tcgaacagta ttcacctagt taccctgaca caagaagttc ttgttcttca 2040
   ggagatgatt ctgttttttc tccagacccc atgccttacg aaccatgcct tcctcagtat 2100
   ccacacataa acggcagtgt taaaacatga 2130
<210> 18
   <211> 1965
   <212> DNA
   <213> Artificial Sequence
<220>
   <223> FGFR2 gene fragment
<400> 18
   atggtcagct ggggtcgttt catctgcctg gtcgtggtca ccatggcaac cttgtccctg      60
   gcccggccct ccttcagttt agttgaggat accacattag agccagaaga gccaccaacc     120
   aaataccaaa tctctcaacc agaagtgtac gtggctgcgc caggggagtc gctagaggtg     180
   cgctgcctgt tgaaagatgc cgccgtgatc agttggacta aggatggggt gcacttgggg     240
   cccaacaata ggacagtgct tattggggag tacttgcaga taaagggcgc cacgcctaga     300
   gactccggcc tctatgcttg tactgccagt aggactgtag acagtgaaac ttggtacttc     360
   atggtgaatg tcacagatgc catctcatcc ggagatgatg aggatgacac cgatggtgcg     420
   gaagattttg tcagtgagaa cagtaacaac aagagagcac catactggac caacacagaa     480
   aagatggaaa agcggctcca tgctgtgcct gcggccaaca ctgtcaagtt tcgctgccca     540
   gccgggggga acccaatgcc aaccatgcgg tggctgaaaa acgggaagga gtttaagcag     600
   gagcatcgca ttggaggcta caaggtacga aaccagcact ggagcctcat tatggaaagt     660
   gtggtcccat ctgacaaggg aaattatacc tgtgtagtgg agaatgaata cgggtccatc     720
   aatcacacgt accacctgga tgttgtggcg cctggaagag aaaaggagat tacagcttcc        780
   ccagactacc tggagatagc catttactgc ataggggtct tcttaatcgc ctgtatggtg        840
   gtaacagtca tcctgtgccg aatgaagaac acgaccaaga agccagactt cagcagccag        900
   ccggctgtgc acaagctgac caaacgtatc cccctgcgga gacaggtaac agtttcggct        960
   gagtccagct cctccatgaa ctccaacacc ccgctggtga ggataacaac acgcctctct       1020
   tcaacggcag acacccccat gctggcaggg gtctccgagt atgaacttcc agaggaccca       1080
   aaatgggagt ttccaagaga taagctgaca ctgggcaagc ccctgggaga aggttgcttt       1140
   gggcaagtgg tcatggcgga agcagtggga attgacaaag acaagcccaa ggaggcggtc       1200
   accgtggccg tgaagatgtt gaaagatgat gccacagaga aagacctttc tgatctggtg       1260
   tcagagatgg agatgatgaa gatgattggg aaacacaaga atatcataaa tcttcttgga       1320
   gcctgcacac aggatgggcc tctctatgtc atagttgagt atgcctctaa aggcaacctc       1380
   cgagaatacc tccgagcccg gaggccaccc gggatggagt actcctatga cattaaccgt       1440
   gttcctgagg agcagatgac cttcaaggac ttggtgtcat gcacctacca gctggccaga       1500
   ggcatggagt acttggcttc ccaaaaatgt attcatcgag atttagcagc cagaaatgtt       1560
   ttggtaacag aaaacaatgt gatgaaaata gcagactttg gactcgccag agatatcaac       1620
   aatatagact attacaaaaa gaccaccaat gggcggcttc cagtcaagtg gatggctcca       1680
   gaagccctgt ttgatagagt atacactcat cagagtgatg tctggtcctt cggggtgtta       1740
   atgtgggaga tcttcacttt agggggctcg ccctacccag ggattcccgt ggaggaactt       1800
   tttaagctgc tgaaggaagg acacagaatg gataagccag ccaactgcac caacgaactg       1860
   tacatgatga tgagggactg ttggcatgca gtgccctccc agagaccaac gttcaagcag       1920
   ttggtagaag acttggatcg aattctcact ctcacaacca atgag                       1965
<210> 19
   <211> 21
   <212> DNA
   <213> Artificial Sequence
<220>
   <223> Break-point of FGFR2 gene fragment
<400> 19
   ctcactctca caaccaatga g 21
<210> 20
   <211> 709
   <212> PRT
   <213> Artificial Sequence
<220>
   <223> FGFR2 protein
<400> 20
   Met Val Ser Trp Gly Arg Phe Ile Cys Leu Val Val Val Thr Met Ala
    1               5                  10                  15
   Thr Leu Ser Leu Ala Arg Pro Ser Phe Ser Leu Val Glu Asp Thr Thr
               20                  25                  30
   Leu Glu Pro Glu Glu Pro Pro Thr Lys Tyr Gln Ile Ser Gln Pro Glu
           35                  40                  45
   Val Tyr Val Ala Ala Pro Gly Glu Ser Leu Glu Val Arg Cys Leu Leu
       50                  55                  60
   Lys Asp Ala Ala Val Ile Ser Trp Thr Lys Asp Gly Val His Leu Gly
   65                  70                  75                  80
   Pro Asn Asn Arg Thr Val Leu Ile Gly Glu Tyr Leu Gln Ile Lys Gly
                   85                  90                  95
   Ala Thr Pro Arg Asp Ser Gly Leu Tyr Ala Cys Thr Ala Ser Arg Thr
               100                 105                 110
   Val Asp Ser Glu Thr Trp Tyr Phe Met Val Asn Val Thr Asp Ala Ile
           115                 120                 125
   Ser Ser Gly Asp Asp Glu Asp Asp Thr Asp Gly Ala Glu Asp Phe Val
       130                 135                 140
   Ser Glu Asn Ser Asn Asn Lys Arg Ala Pro Tyr Trp Thr Asn Thr Glu
   145                 150                 155                 160
   Lys Met Glu Lys Arg Leu His Ala Val Pro Ala Ala Asn Thr Val Lys
                   165                 170                 175
   Phe Arg Cys Pro Ala Gly Gly Asn Pro Met Pro Thr Met Arg Trp Leu
               180                 185                 190
   Lys Asn Gly Lys Glu Phe Lys Gln Glu His Arg Ile Gly Gly Tyr Lys
           195                 200                 205
   Val Arg Asn Gln His Trp Ser Leu Ile Met Glu Ser Val Val Pro Ser
       210                 215                 220
   Asp Lys Gly Asn Tyr Thr Cys Val Val Glu Asn Glu Tyr Gly Ser Ile
   225                 230                 235                 240
   Asn His Thr Tyr His Leu Asp Val Val Ala Pro Gly Arg Glu Lys Glu
                   245                 250                 255
   Ile Thr Ala Ser Pro Asp Tyr Leu Glu Ile Ala Ile Tyr Cys Ile Gly
               260                 265                 270
   Val Phe Leu Ile Ala Cys Met Val Val Thr Val Ile Leu Cys Arg Met
           275                 280                 285
   Lys Asn Thr Thr Lys Lys Pro Asp Phe Ser Ser Gln Pro Ala Val His
       290                 295                 300
   Lys Leu Thr Lys Arg Ile Pro Leu Arg Arg Gln Val Thr Val Ser Ala
   305                 310                 315                 320
   Glu Ser Ser Ser Ser Met Asn Ser Asn Thr Pro Leu Val Arg Ile Thr
                   325                 330                 335
   Thr Arg Leu Ser Ser Thr Ala Asp Thr Pro Met Leu Ala Gly Val Ser
               340                 345                 350
   Glu Tyr Glu Leu Pro Glu Asp Pro Lys Trp Glu Phe Pro Arg Asp Lys
           355                 360                 365
   Leu Thr Leu Gly Lys Pro Leu Gly Glu Gly Cys Phe Gly Gln Val Val
       370                 375                 380
   Met Ala Glu Ala Val Gly Ile Asp Lys Asp Lys Pro Lys Glu Ala Val
   385                 390                 395                 400
   Thr Val Ala Val Lys Met Leu Lys Asp Asp Ala Thr Glu Lys Asp Leu
                   405                 410                 415
   Ser Asp Leu Val Ser Glu Met Glu Met Met Lys Met Ile Gly Lys His
               420                 425                 430
   Lys Asn Ile Ile Asn Leu Leu Gly Ala Cys Thr Gln Asp Gly Pro Leu
           435                 440                 445
   Tyr Val Ile Val Glu Tyr Ala Ser Lys Gly Asn Leu Arg Glu Tyr Leu
       450                 455                 460
   Arg Ala Arg Arg Pro Pro Gly Met Glu Tyr Ser Tyr Asp Ile Asn Arg
   465                 470                 475                 480
   Val Pro Glu Glu Gln Met Thr Phe Lys Asp Leu Val Ser Cys Thr Tyr
                   485                 490                 495
   Gln Leu Ala Arg Gly Met Glu Tyr Leu Ala Ser Gln Lys Cys Ile His
               500                 505                 510
   Arg Asp Leu Ala Ala Arg Asn Val Leu Val Thr Glu Asn Asn Val Met
           515                 520                 525
   Lys Ile Ala Asp Phe Gly Leu Ala Arg Asp Ile Asn Asn Ile Asp Tyr
       530                 535                 540
   Tyr Lys Lys Thr Thr Asn Gly Arg Leu Pro Val Lys Trp Met Ala Pro
   545                 550                 555                 560
   Glu Ala Leu Phe Asp Arg Val Tyr Thr His Gln Ser Asp Val Trp Ser
                   565                 570                 575
   Phe Gly Val Leu Met Trp Glu Ile Phe Thr Leu Gly Gly Ser Pro Tyr
               580                 585                 590
   Pro Gly Ile Pro Val Glu Glu Leu Phe Lys Leu Leu Lys Glu Gly His
           595                 600                 605
   Arg Met Asp Lys Pro Ala Asn Cys Thr Asn Glu Leu Tyr Met Met Met
       610                 615                 620
   Arg Asp Cys Trp His Ala Val Pro Ser Gln Arg Pro Thr Phe Lys Gln
   625                 630                 635                 640
   Leu Val Glu Asp Leu Asp Arg Ile Leu Thr Leu Thr Thr Asn Glu Glu
                   645                 650                 655
   Tyr Leu Asp Leu Ser Gln Pro Leu Glu Gln Tyr Ser Pro Ser Tyr Pro
   660 665 670
   Asp Thr Arg Ser Ser Cys Ser Ser Gly Asp Asp Ser Val Phe Ser Pro
   675 680 685
   Asp Pro Met Pro Tyr Glu Pro Cys Leu Pro Gln Tyr Pro His Ile Asn
   690 695 700
   Gly Ser Val Lys Thr
   705
<210> 21
   <211> 655
   <212> PRT
   <213> Artificial Sequence
<220>
   <223> FGFR2 protein fragment
<400> 21
   Met Val Ser Trp Gly Arg Phe Ile Cys Leu Val Val Val Thr Met Ala
   1 5 10 15
   Thr Leu Ser Leu Ala Arg Pro Ser Phe Ser Leu Val Glu Asp Thr Thr
   20 25 30
   Leu Glu Pro Glu Glu Pro Pro Thr Lys Tyr Gln Ile Ser Gln Pro Glu
   35 40 45
   Val Tyr Val Ala Ala Pro Gly Glu Ser Leu Glu Val Arg Cys Leu Leu
   50 55 60
   Lys Asp Ala Ala Val Ile Ser Trp Thr Lys Asp Gly Val His Leu Gly
   65 70 75 80
   Pro Asn Asn Arg Thr Val Leu Ile Gly Glu Tyr Leu Gln Ile Lys Gly
   85 90 95
   Ala Thr Pro Arg Asp Ser Gly Leu Tyr Ala Cys Thr Ala Ser Arg Thr
   100 105 110
   Val Asp Ser Glu Thr Trp Tyr Phe Met Val Asn Val Thr Asp Ala Ile
   115 120 125
   Ser Ser Gly Asp Asp Glu Asp Asp Thr Asp Gly Ala Glu Asp Phe Val
   130 135 140
   Ser Glu Asn Ser Asn Asn Lys Arg Ala Pro Tyr Trp Thr Asn Thr Glu
   145 150 155 160
   Lys Met Glu Lys Arg Leu His Ala Val Pro Ala Ala Asn Thr Val Lys
   165 170 175
   Phe Arg Cys Pro Ala Gly Gly Asn Pro Met Pro Thr Met Arg Trp Leu
   180 185 190
   Lys Asn Gly Lys Glu Phe Lys Gln Glu His Arg Ile Gly Gly Tyr Lys
   195 200 205
   Val Arg Asn Gln His Trp Ser Leu Ile Met Glu Ser Val Val Pro Ser
       210                 215                 220
   Asp Lys Gly Asn Tyr Thr Cys Val Val Glu Asn Glu Tyr Gly Ser Ile
   225                 230                 235                 240
   Asn His Thr Tyr His Leu Asp Val Val Ala Pro Gly Arg Glu Lys Glu
                   245                 250                 255
   Ile Thr Ala Ser Pro Asp Tyr Leu Glu Ile Ala Ile Tyr Cys Ile Gly
               260                 265                 270
   Val Phe Leu Ile Ala Cys Met Val Val Thr Val Ile Leu Cys Arg Met
           275                 280                 285
   Lys Asn Thr Thr Lys Lys Pro Asp Phe Ser Ser Gln Pro Ala Val His
       290                 295                 300
   Lys Leu Thr Lys Arg Ile Pro Leu Arg Arg Gln Val Thr Val Ser Ala
   305                 310                 315                 320
   Glu Ser Ser Ser Ser Met Asn Ser Asn Thr Pro Leu Val Arg Ile Thr
                   325                 330                 335
   Thr Arg Leu Ser Ser Thr Ala Asp Thr Pro Met Leu Ala Gly Val Ser
               340                 345                 350
   Glu Tyr Glu Leu Pro Glu Asp Pro Lys Trp Glu Phe Pro Arg Asp Lys
           355                 360                 365
   Leu Thr Leu Gly Lys Pro Leu Gly Glu Gly Cys Phe Gly Gln Val Val
       370                 375                 380
   Met Ala Glu Ala Val Gly Ile Asp Lys Asp Lys Pro Lys Glu Ala Val
   385                 390                 395                 400
   Thr Val Ala Val Lys Met Leu Lys Asp Asp Ala Thr Glu Lys Asp Leu
                   405                 410                 415
   Ser Asp Leu Val Ser Glu Met Glu Met Met Lys Met Ile Gly Lys His
               420                 425                 430
   Lys Asn Ile Ile Asn Leu Leu Gly Ala Cys Thr Gln Asp Gly Pro Leu
           435                 440                 445
   Tyr Val Ile Val Glu Tyr Ala Ser Lys Gly Asn Leu Arg Glu Tyr Leu
       450                 455                 460
   Arg Ala Arg Arg Pro Pro Gly Met Glu Tyr Ser Tyr Asp Ile Asn Arg
   465                 470                 475                 480
   Val Pro Glu Glu Gln Met Thr Phe Lys Asp Leu Val Ser Cys Thr Tyr
                   485                 490                 495
   Gln Leu Ala Arg Gly Met Glu Tyr Leu Ala Ser Gln Lys Cys Ile His
               500                 505                 510
   Arg Asp Leu Ala Ala Arg Asn Val Leu Val Thr Glu Asn Asn Val Met
           515                 520                 525
   Lys Ile Ala Asp Phe Gly Leu Ala Arg Asp Ile Asn Asn Ile Asp Tyr
       530                 535                 540
   Tyr Lys Lys Thr Thr Asn Gly Arg Leu Pro Val Lys Trp Met Ala Pro
   545                 550                 555                 560
   Glu Ala Leu Phe Asp Arg Val Tyr Thr His Gln Ser Asp Val Trp Ser
                   565                 570                 575
   Phe Gly Val Leu Met Trp Glu Ile Phe Thr Leu Gly Gly Ser Pro Tyr
               580                 585                 590
   Pro Gly Ile Pro Val Glu Glu Leu Phe Lys Leu Leu Lys Glu Gly His
           595                 600                 605
   Arg Met Asp Lys Pro Ala Asn Cys Thr Asn Glu Leu Tyr Met Met Met
       610                 615                 620
   Arg Asp Cys Trp His Ala Val Pro Ser Gln Arg Pro Thr Phe Lys Gln
   625                 630                 635                 640
   Leu Val Glu Asp Leu Asp Arg Ile Leu Thr Leu Thr Thr Asn Glu
                   645                 650                 655
<210> 22
   <211> 7
   <212> PRT
   <213> Artificial Sequence
<220>
   <223> Break-point of FGFR2 protein fragment
<400> 22
   Leu Thr Leu Thr Thr Asn Glu
   1               5
<210> 23
   <211> 6084
   <212> DNA
   <213> Artificial Sequence
<220>
   <223> CDS of CIT gene (NM_007174)
<400> 23
   atgttgaagt tcaaatatgg agcgcggaat cctttggatg ctggtgctgc tgaacccatt        60
   gccagccggg cctccaggct gaatctgttc ttccagggga aaccaccctt tatgactcaa       120
   cagcagatgt ctcctctttc ccgagaaggg atattagatg ccctctttgt tctctttgaa       180
   gaatgcagtc agcctgctct gatgaagatt aagcacgtga gcaactttgt ccggaagtat       240
   tccgacacca tagctgagtt acaggagctc cagccttcgg caaaggactt cgaagtcaga       300
   agtcttgtag gttgtggtca ctttgctgaa gtgcaggtgg taagagagaa agcaaccggg       360
   gacatctatg ctatgaaagt gatgaagaag aaggctttat tggcccagga gcaggtttca       420
   ttttttgagg aagagcggaa catattatct cgaagcacaa gcccgtggat cccccaatta       480
   cagtatgcct ttcaggacaa aaatcacctt tatctggtca tggaatatca gcctggaggg       540
   gacttgctgt cacttttgaa tagatatgag gaccagttag atgaaaacct gatacagttt 600
   tacctagctg agctgatttt ggctgttcac agcgttcatc tgatgggata cgtgcatcga 660
   gacatcaagc ctgagaacat tctcgttgac cgcacaggac acatcaagct ggtggatttt 720
   ggatctgccg cgaaaatgaa ttcaaacaag atggtgaatg ccaaactccc gattgggacc 780
   ccagattaca tggctcctga agtgctgact gtgatgaacg gggatggaaa aggcacctac 840
   ggcctggact gtgactggtg gtcagtgggc gtgattgcct atgagatgat ttatgggaga 900
   tcccccttcg cagagggaac ctctgccaga accttcaata acattatgaa tttccagcgg 960
   tttttgaaat ttccagatga ccccaaagtg agcagtgact ttcttgatct gattcaaagc 1020
   ttgttgtgcg gccagaaaga gagactgaag tttgaaggtc tttgctgcca tcctttcttc 1080
   tctaaaattg actggaacaa cattcgtaac tctcctcccc ccttcgttcc caccctcaag 1140
   tctgacgatg acacctccaa ttttgatgaa ccagagaaga attcgtgggt ttcatcctct 1200
   ccgtgccagc tgagcccctc aggcttctcg ggtgaagaac tgccgtttgt ggggttttcg 1260
   tacagcaagg cactggggat tcttggtaga tctgagtctg ttgtgtcggg tctggactcc 1320
   cctgccaaga ctagctccat ggaaaagaaa cttctcatca aaagcaaaga gctacaagac 1380
   tctcaggaca agtgtcacaa gatggagcag gaaatgaccc ggttacatcg gagagtgtca 1440
   gaggtggagg ctgtgcttag tcagaaggag gtggagctga aggcctctga gactcagaga 1500
   tccctcctgg agcaggacct tgctacctac atcacagaat gcagtagctt aaagcgaagt 1560
   ttggagcaag cacggatgga ggtgtcccag gaggatgaca aagcactgca gcttctccat 1620
   gatatcagag agcagagccg gaagctccaa gaaatcaaag agcaggagta ccaggctcaa 1680
   gtggaagaaa tgaggttgat gatgaatcag ttggaagagg atcttgtctc agcaagaaga 1740
   cggagtgatc tctacgaatc tgagctgaga gagtctcggc ttgctgctga agaattcaag 1800
   cggaaagcga cagaatgtca gcataaactg ttgaaggcta aggatcaagg gaagcctgaa 1860
   gtgggagaat atgcgaaact ggagaagatc aatgctgagc agcagctcaa aattcaggag 1920
   ctccaagaga aactggagaa ggctgtaaaa gccagcacgg aggccaccga gctgctgcag 1980
   aatatccgcc aggcaaagga gcgagccgag agggagctgg agaagctgca gaaccgagag 2040
   gattcttctg aaggcatcag aaagaagctg gtggaagctg aggagctcga agagaaacat 2100
   cgggaggccc aagtctcagc ccagcaccta gaagtgcacc tgaaacagaa agagcagcac 2160
   tatgaggaaa agattaaagt gttggacaat cagataaaga aagacctggc tgacaaggag 2220
   acactggaga acatgatgca gagacacgag gaggaggccc atgagaaggg caaaattctc 2280
   agcgaacaga aggcgatgat caatgctatg gattccaaga tcagatccct ggaacagagg 2340
   attgtggaac tgtctgaagc caataaactt gcagcaaata gcagtctttt tacccaaagg 2400
   aacatgaagg cccaagaaga gatgatttct gaactcaggc aacagaaatt ttacctggag 2460
   acacaggctg ggaagttgga ggcccagaac cgaaaactgg aggagcagct ggagaagatc 2520
   agccaccaag accacagtga caagaatcgg ctgctggaac tggagacaag attgcgggag 2580
   gtcagtctag agcacgagga gcagaaactg gagctcaagc gccagctcac agagctacag 2640
   ctctccctgc aggagcgcga gtcacagttg acagccctgc aggctgcacg ggcggccctg 2700
   gagagccagc ttcgccaggc gaagacagag ctggaagaga ccacagcaga agctgaagag 2760
   gagatccagg cactcacggc acatagagat gaaatccagc gcaaatttga tgctcttcgt 2820
   aacagctgta ctgtaatcac agacctggag gagcagctaa accagctgac cgaggacaac 2880
   gctgaactca acaaccaaaa cttctacttg tccaaacaac tcgatgaggc ttctggcgcc 2940
   aacgacgaga ttgtacaact gcgaagtgaa gtggaccatc tccgccggga gatcacggaa 3000
   cgagagatgc agcttaccag ccagaagcaa acgatggagg ctctgaagac cacgtgcacc 3060
   atgctggagg aacaggtcat ggatttggag gccctaaacg atgagctgct agaaaaagag 3120
   cggcagtggg aggcctggag gagcgtcctg ggtgatgaga aatcccagtt tgagtgtcgg 3180
   gttcgagagc tgcagagaat gctggacacc gagaaacaga gcagggcgag agccgatcag 3240
   cggatcaccg agtctcgcca ggtggtggag ctggcagtga aggagcacaa ggctgagatt 3300
   ctcgctctgc agcaggctct caaagagcag aagctgaagg ccgagagcct ctctgacaag 3360
   ctcaatgacc tggagaagaa gcatgctatg cttgaaatga atgcccgaag cttacagcag 3420
   aagctggaga ctgaacgaga gctcaaacag aggcttctgg aagagcaagc caaattacag 3480
   cagcagatgg acctgcagaa aaatcacatt ttccgtctga ctcaaggact gcaagaagct 3540
   ctagatcggg ctgatctact gaagacagaa agaagtgact tggagtatca gctggaaaac 3600
   attcaggttc tctattctca tgaaaaggtg aaaatggaag gcactatttc tcaacaaacc 3660
   aaactcattg attttctgca agccaaaatg gaccaacctg ctaaaaagaa aaagggttta 3720
   tttagtcgac ggaaagagga ccctgcttta cccacacagg ttcctctgca gtacaatgag 3780
   ctgaagctgg ccctggagaa ggagaaagct cgctgtgcag agctagagga agcccttcag 3840
   aagacccgca tcgagctccg gtccgcccgg gaggaagctg cccaccgcaa agcaacggac 3900
   cacccacacc catccacgcc agccaccgcg aggcagcaga tcgccatgtc cgccatcgtg 3960
   cggtcgccag agcaccagcc cagtgccatg agcctgctgg ccccgccatc cagccgcaga 4020
   aaggagtctt caactccaga ggaatttagt cggcgtctta aggaacgcat gcaccacaat 4080
   attcctcacc gattcaacgt aggactgaac atgcgagcca caaagtgtgc tgtgtgtctg 4140
   gataccgtgc actttggacg ccaggcatcc aaatgtctcg aatgtcaggt gatgtgtcac 4200
   cccaagtgct ccacgtgctt gccagccacc tgcggcttgc ctgctgaata tgccacacac 4260
   ttcaccgagg ccttctgccg tgacaaaatg aactccccag gtctccagac caaggagccc 4320
   agcagcagct tgcacctgga agggtggatg aaggtgccca ggaataacaa acgaggacag 4380
   caaggctggg acaggaagta cattgtcctg gagggatcaa aagtcctcat ttatgacaat 4440
   gaagccagag aagctggaca gaggccggtg gaagaatttg agctgtgcct tcccgacggg 4500
   gatgtatcta ttcatggtgc cgttggtgct tccgaactcg caaatacagc caaagcagat 4560
   gtcccataca tactgaagat ggaatctcac ccgcacacca cctgctggcc cgggagaacc 4620
   ctctacttgc tagctcccag cttccctgac aaacagcgct gggtcaccgc cttagaatca 4680
   gttgtcgcag gtgggagagt ttctagggaa aaagcagaag ctgatgctaa actgcttgga 4740
   aactccctgc tgaaactgga aggtgatgac cgtctagaca tgaactgcac gctgcccttc 4800
   agtgaccagg tggtgttggt gggcaccgag gaagggctct acgccctgaa tgtcttgaaa 4860
   aactccctaa cccatgtccc aggaattgga gcagtcttcc aaatttatat tatcaaggac 4920
   ctggagaagc tactcatgat agcaggagaa gagcgggcac tgtgtcttgt ggacgtgaag 4980
   aaagtgaaac agtccctggc ccagtcccac ctgcctgccc agcccgacat ctcacccaac 5040
   atttttgaag ctgtcaaggg ctgccacttg tttggggcag gcaagattga gaacgggctc 5100
   tgcatctgtg cagccatgcc cagcaaagtc gtcattctcc gctacaacga aaacctcagc 5160
   aaatactgca tccggaaaga gatagagacc tcagagccct gcagctgtat ccacttcacc 5220
   aattacagta tcctcattgg aaccaataaa ttctacgaaa tcgacatgaa gcagtacacg 5280
   ctcgaggaat tcctggataa gaatgaccat tccttggcac ctgctgtgtt tgccgcctct 5340
   tccaacagct tccctgtctc aatcgtgcag gtgaacagcg cagggcagcg agaggagtac 5400
   ttgctgtgtt tccacgaatt tggagtgttc gtggattctt acggaagacg tagccgcaca 5460
   gacgatctca agtggagtcg cttacctttg gcctttgcct acagagaacc ctatctgttt 5520
   gtgacccact tcaactcact cgaagtaatt gagatccagg cacgctcctc agcagggacc 5580
   cctgcccgag cgtacctgga catcccgaac ccgcgctacc tgggccctgc catttcctca 5640
   ggagcgattt acttggcgtc ctcataccag gataaattaa gggtcatttg ctgcaaggga 5700
   aacctcgtga aggagtccgg cactgaacac caccggggcc cgtccacctc ccgcagcagc 5760
   cccaacaagc gaggcccacc cacgtacaac gagcacatca ccaagcgcgt ggcctccagc 5820
   ccagcgccgc ccgaaggccc cagccacccg cgagagccaa gcacacccca ccgctaccgc 5880
   gaggggcgga ccgagctgcg cagggacaag tctcctggcc gccccctgga gcgagagaag 5940
   tcccccggcc ggatgctcag cacgcggaga gagcggtccc ccgggaggct gtttgaagac 6000
   agcagcaggg gccggctgcc tgcgggagcc gtgaggaccc cgctgtccca ggtgaacaag 6060
   gtctgggacc agtcttcagt ataa 6084
<210> 24
   <211> 3306
   <212> DNA
   <213> Artificial Sequence
<220>
   <223> CIT gene fragment
<400> 24
   gcacatagag atgaaatcca gcgcaaattt gatgctcttc gtaacagctg tactgtaatc      60
   acagacctgg aggagcagct aaaccagctg accgaggaca acgctgaact caacaaccaa     120
   aacttctact tgtccaaaca actcgatgag gcttctggcg ccaacgacga gattgtacaa     180
   ctgcgaagtg aagtggacca tctccgccgg gagatcacgg aacgagagat gcagcttacc     240
   agccagaagc aaacgatgga ggctctgaag accacgtgca ccatgctgga ggaacaggtc     300
   atggatttgg aggccctaaa cgatgagctg ctagaaaaag agcggcagtg ggaggcctgg     360
   aggagcgtcc tgggtgatga gaaatcccag tttgagtgtc gggttcgaga gctgcagaga     420
   atgctggaca ccgagaaaca gagcagggcg agagccgatc agcggatcac cgagtctcgc     480
   caggtggtgg agctggcagt gaaggagcac aaggctgaga ttctcgctct gcagcaggct     540
   ctcaaagagc agaagctgaa ggccgagagc ctctctgaca agctcaatga cctggagaag     600
   aagcatgcta tgcttgaaat gaatgcccga agcttacagc agaagctgga gactgaacga     660
   gagctcaaac agaggcttct ggaagagcaa gccaaattac agcagcagat ggacctgcag     720
   aaaaatcaca ttttccgtct gactcaagga ctgcaagaag ctctagatcg ggctgatcta     780
   ctgaagacag aaagaagtga cttggagtat cagctggaaa acattcaggt tctctattct     840
   catgaaaagg tgaaaatgga aggcactatt tctcaacaaa ccaaactcat tgattttctg     900
   caagccaaaa tggaccaacc tgctaaaaag aaaaagggtt tatttagtcg acggaaagag     960
   gaccctgctt tacccacaca ggttcctctg cagtacaatg agctgaagct ggccctggag    1020
   aaggagaaag ctcgctgtgc agagctagag gaagcccttc agaagacccg catcgagctc    1080
   cggtccgccc gggaggaagc tgcccaccgc aaagcaacgg accacccaca cccatccacg    1140
   ccagccaccg cgaggcagca gatcgccatg tccgccatcg tgcggtcgcc agagcaccag    1200
   cccagtgcca tgagcctgct ggccccgcca tccagccgca gaaaggagtc ttcaactcca    1260
   gaggaattta gtcggcgtct taaggaacgc atgcaccaca atattcctca ccgattcaac    1320
   gtaggactga acatgcgagc cacaaagtgt gctgtgtgtc tggataccgt gcactttgga    1380
   cgccaggcat ccaaatgtct cgaatgtcag gtgatgtgtc accccaagtg ctccacgtgc    1440
   ttgccagcca cctgcggctt gcctgctgaa tatgccacac acttcaccga ggccttctgc    1500
   cgtgacaaaa tgaactcccc aggtctccag accaaggagc ccagcagcag cttgcacctg    1560
   gaagggtgga tgaaggtgcc caggaataac aaacgaggac agcaaggctg ggacaggaag    1620
   tacattgtcc tggagggatc aaaagtcctc atttatgaca atgaagccag agaagctgga 1680
   cagaggccgg tggaagaatt tgagctgtgc cttcccgacg gggatgtatc tattcatggt 1740
   gccgttggtg cttccgaact cgcaaataca gccaaagcag atgtcccata catactgaag 1800
   atggaatctc acccgcacac cacctgctgg cccgggagaa ccctctactt gctagctccc 1860
   agcttccctg acaaacagcg ctgggtcacc gccttagaat cagttgtcgc aggtgggaga 1920
   gtttctaggg aaaaagcaga agctgatgct aaactgcttg gaaactccct gctgaaactg 1980
   gaaggtgatg accgtctaga catgaactgc acgctgccct tcagtgacca ggtggtgttg 2040
   gtgggcaccg aggaagggct ctacgccctg aatgtcttga aaaactccct aacccatgtc 2100
   ccaggaattg gagcagtctt ccaaatttat attatcaagg acctggagaa gctactcatg 2160
   atagcaggag aagagcgggc actgtgtctt gtggacgtga agaaagtgaa acagtccctg 2220
   gcccagtccc acctgcctgc ccagcccgac atctcaccca acatttttga agctgtcaag 2280
   ggctgccact tgtttggggc aggcaagatt gagaacgggc tctgcatctg tgcagccatg 2340
   cccagcaaag tcgtcattct ccgctacaac gaaaacctca gcaaatactg catccggaaa 2400
   gagatagaga cctcagagcc ctgcagctgt atccacttca ccaattacag tatcctcatt 2460
   ggaaccaata aattctacga aatcgacatg aagcagtaca cgctcgagga attcctggat 2520
   aagaatgacc attccttggc acctgctgtg tttgccgcct cttccaacag cttccctgtc 2580
   tcaatcgtgc aggtgaacag cgcagggcag cgagaggagt acttgctgtg tttccacgaa 2640
   tttggagtgt tcgtggattc ttacggaaga cgtagccgca cagacgatct caagtggagt 2700
   cgcttacctt tggcctttgc ctacagagaa ccctatctgt ttgtgaccca cttcaactca 2760
   ctcgaagtaa ttgagatcca ggcacgctcc tcagcaggga cccctgcccg agcgtacctg 2820
   gacatcccga acccgcgcta cctgggccct gccatttcct caggagcgat ttacttggcg 2880
   tcctcatacc aggataaatt aagggtcatt tgctgcaagg gaaacctcgt gaaggagtcc 2940
   ggcactgaac accaccgggg cccgtccacc tcccgcagca gccccaacaa gcgaggccca 3000
   cccacgtaca acgagcacat caccaagcgc gtggcctcca gcccagcgcc gcccgaaggc 3060
   cccagccacc cgcgagagcc aagcacaccc caccgctacc gcgaggggcg gaccgagctg 3120
   cgcagggaca agtctcctgg ccgccccctg gagcgagaga agtcccccgg ccggatgctc 3180
   agcacgcgga gagagcggtc ccccgggagg ctgtttgaag acagcagcag gggccggctg 3240
   cctgcgggag ccgtgaggac cccgctgtcc caggtgaaca aggtctggga ccagtcttca 3300
   gtataa 3306
<210> 25
   <211> 21
   <212> DNA
   <213> Artificial Sequence
<220>
   <223> Break-point of CIT gene fragment
<400> 25
   gcacatagag atgaaatcca g 21
<210> 26
   <211> 2027
   <212> PRT
   <213> Artificial Sequence
<220>
   <223> CIT protein
<400> 26
   Met Leu Lys Phe Lys Tyr Gly Ala Arg Asn Pro Leu Asp Ala Gly Ala
   1               5                   10                  15
   Ala Glu Pro Ile Ala Ser Arg Ala Ser Arg Leu Asn Leu Phe Phe Gln
               20                  25                  30
   Gly Lys Pro Pro Phe Met Thr Gln Gln Gln Met Ser Pro Leu Ser Arg
           35                  40                  45
   Glu Gly Ile Leu Asp Ala Leu Phe Val Leu Phe Glu Glu Cys Ser Gln
       50                  55                  60
   Pro Ala Leu Met Lys Ile Lys His Val Ser Asn Phe Val Arg Lys Tyr
   65                  70                  75                  80
   Ser Asp Thr Ile Ala Glu Leu Gln Glu Leu Gln Pro Ser Ala Lys Asp
                   85                  90                  95
   Phe Glu Val Arg Ser Leu Val Gly Cys Gly His Phe Ala Glu Val Gln
               100                 105                 110
   Val Val Arg Glu Lys Ala Thr Gly Asp Ile Tyr Ala Met Lys Val Met
           115                 120                 125
   Lys Lys Lys Ala Leu Leu Ala Gln Glu Gln Val Ser Phe Phe Glu Glu
       130                 135                 140
   Glu Arg Asn Ile Leu Ser Arg Ser Thr Ser Pro Trp Ile Pro Gln Leu
   145                 150                 155                 160
   Gln Tyr Ala Phe Gln Asp Lys Asn His Leu Tyr Leu Val Met Glu Tyr
                   165                 170                 175
   Gln Pro Gly Gly Asp Leu Leu Ser Leu Leu Asn Arg Tyr Glu Asp Gln
               180                 185                 190
   Leu Asp Glu Asn Leu Ile Gln Phe Tyr Leu Ala Glu Leu Ile Leu Ala
           195                 200                 205
   Val His Ser Val His Leu Met Gly Tyr Val His Arg Asp Ile Lys Pro
       210                 215                 220
   Glu Asn Ile Leu Val Asp Arg Thr Gly His Ile Lys Leu Val Asp Phe
   225                 230                 235                 240
   Gly Ser Ala Ala Lys Met Asn Ser Asn Lys Met Val Asn Ala Lys Leu
                   245                 250                 255
   Pro Ile Gly Thr Pro Asp Tyr Met Ala Pro Glu Val Leu Thr Val Met
               260                 265                 270
   Asn Gly Asp Gly Lys Gly Thr Tyr Gly Leu Asp Cys Asp Trp Trp Ser
           275                 280                 285
   Val Gly Val Ile Ala Tyr Glu Met Ile Tyr Gly Arg Ser Pro Phe Ala
       290                 295                 300
   Glu Gly Thr Ser Ala Arg Thr Phe Asn Asn Ile Met Asn Phe Gln Arg
   305                 310                 315                 320
   Phe Leu Lys Phe Pro Asp Asp Pro Lys Val Ser Ser Asp Phe Leu Asp
                   325                 330                 335
   Leu Ile Gln Ser Leu Leu Cys Gly Gln Lys Glu Arg Leu Lys Phe Glu
               340                 345                 350
   Gly Leu Cys Cys His Pro Phe Phe Ser Lys Ile Asp Trp Asn Asn Ile
           355                 360                 365
   Arg Asn Ser Pro Pro Pro Phe Val Pro Thr Leu Lys Ser Asp Asp Asp
       370                 375                 380
   Thr Ser Asn Phe Asp Glu Pro Glu Lys Asn Ser Trp Val Ser Ser Ser
   385                 390                 395                 400
   Pro Cys Gln Leu Ser Pro Ser Gly Phe Ser Gly Glu Glu Leu Pro Phe
                   405                 410                 415
   Val Gly Phe Ser Tyr Ser Lys Ala Leu Gly Ile Leu Gly Arg Ser Glu
               420                 425                 430
   Ser Val Val Ser Gly Leu Asp Ser Pro Ala Lys Thr Ser Ser Met Glu
           435                 440                 445
   Lys Lys Leu Leu Ile Lys Ser Lys Glu Leu Gln Asp Ser Gln Asp Lys
       450                 455                 460
   Cys His Lys Met Glu Gln Glu Met Thr Arg Leu His Arg Arg Val Ser
   465                 470                 475                 480
   Glu Val Glu Ala Val Leu Ser Gln Lys Glu Val Glu Leu Lys Ala Ser
                   485                 490                 495
   Glu Thr Gln Arg Ser Leu Leu Glu Gln Asp Leu Ala Thr Tyr Ile Thr
               500                 505                 510
   Glu Cys Ser Ser Leu Lys Arg Ser Leu Glu Gln Ala Arg Met Glu Val
           515                 520                 525
   Ser Gln Glu Asp Asp Lys Ala Leu Gln Leu Leu His Asp Ile Arg Glu
       530                 535                 540
   Gln Ser Arg Lys Leu Gln Glu Ile Lys Glu Gln Glu Tyr Gln Ala Gln
   545                 550                 555                 560
   Val Glu Glu Met Arg Leu Met Met Asn Gln Leu Glu Glu Asp Leu Val
                   565                 570                 575
   Ser Ala Arg Arg Arg Ser Asp Leu Tyr Glu Ser Glu Leu Arg Glu Ser
   580 585 590
   Arg Leu Ala Ala Glu Glu Phe Lys Arg Lys Ala Thr Glu Cys Gln His
   595 600 605
   Lys Leu Leu Lys Ala Lys Asp Gln Gly Lys Pro Glu Val Gly Glu Tyr
   610 615 620
   Ala Lys Leu Glu Lys Ile Asn Ala Glu Gln Gln Leu Lys Ile Gln Glu
   625 630 635 640
   Leu Gln Glu Lys Leu Glu Lys Ala Val Lys Ala Ser Thr Glu Ala Thr
   645 650 655
   Glu Leu Leu Gln Asn Ile Arg Gln Ala Lys Glu Arg Ala Glu Arg Glu
   660 665 670
   Leu Glu Lys Leu Gln Asn Arg Glu Asp Ser Ser Glu Gly Ile Arg Lys
   675 680 685
   Lys Leu Val Glu Ala Glu Glu Leu Glu Glu Lys His Arg Glu Ala Gln
   690 695 700
   Val Ser Ala Gln His Leu Glu Val His Leu Lys Gln Lys Glu Gln His
   705 710 715 720
   Tyr Glu Glu Lys Ile Lys Val Leu Asp Asn Gln Ile Lys Lys Asp Leu
   725 730 735
   Ala Asp Lys Glu Thr Leu Glu Asn Met Met Gln Arg His Glu Glu Glu
   740 745 750
   Ala His Glu Lys Gly Lys Ile Leu Ser Glu Gln Lys Ala Met Ile Asn
   755 760 765
   Ala Met Asp Ser Lys Ile Arg Ser Leu Glu Gln Arg Ile Val Glu Leu
   770 775 780
   Ser Glu Ala Asn Lys Leu Ala Ala Asn Ser Ser Leu Phe Thr Gln Arg
   785 790 795 800
   Asn Met Lys Ala Gln Glu Glu Met Ile Ser Glu Leu Arg Gln Gln Lys
   805 810 815
   Phe Tyr Leu Glu Thr Gln Ala Gly Lys Leu Glu Ala Gln Asn Arg Lys
   820 825 830
   Leu Glu Glu Gln Leu Glu Lys Ile Ser His Gln Asp His Ser Asp Lys
   835 840 845
   Asn Arg Leu Leu Glu Leu Glu Thr Arg Leu Arg Glu Val Ser Leu Glu
   850 855 860
   His Glu Glu Gln Lys Leu Glu Leu Lys Arg Gln Leu Thr Glu Leu Gln
   865 870 875 880
   Leu Ser Leu Gln Glu Arg Glu Ser Gln Leu Thr Ala Leu Gln Ala Ala
   885 890 895
   Arg Ala Ala Leu Glu Ser Gln Leu Arg Gln Ala Lys Thr Glu Leu Glu
   900 905 910
   Glu Thr Thr Ala Glu Ala Glu Glu Glu Ile Gln Ala Leu Thr Ala His
   915 920 925
   Arg Asp Glu Ile Gln Arg Lys Phe Asp Ala Leu Arg Asn Ser Cys Thr
   930 935 940
   Val Ile Thr Asp Leu Glu Glu Gln Leu Asn Gln Leu Thr Glu Asp Asn
   945 950 955 960
   Ala Glu Leu Asn Asn Gln Asn Phe Tyr Leu Ser Lys Gln Leu Asp Glu
   965 970 975
   Ala Ser Gly Ala Asn Asp Glu Ile Val Gln Leu Arg Ser Glu Val Asp
   980 985 990
   His Leu Arg Arg Glu Ile Thr Glu Arg Glu Met Gln Leu Thr Ser Gln
   995 1000 1005
   Lys Gln Thr Met Glu Ala Leu Lys Thr Thr Cys Thr Met Leu Glu Glu
   1010 1015 1020
   Gln Val Met Asp Leu Glu Ala Leu Asn Asp Glu Leu Leu Glu Lys Glu
   1025 1030 1035 1040
   Arg Gln Trp Glu Ala Trp Arg Ser Val Leu Gly Asp Glu Lys Ser Gln
   1045 1050 1055
   Phe Glu Cys Arg Val Arg Glu Leu Gln Arg Met Leu Asp Thr Glu Lys
   1060 1065 1070
   Gln Ser Arg Ala Arg Ala Asp Gln Arg Ile Thr Glu Ser Arg Gln Val
   1075 1080 1085
   Val Glu Leu Ala Val Lys Glu His Lys Ala Glu Ile Leu Ala Leu Gln
   1090 1095 1100
   Gln Ala Leu Lys Glu Gln Lys Leu Lys Ala Glu Ser Leu Ser Asp Lys
   1105 1110 1115 1120
   Leu Asn Asp Leu Glu Lys Lys His Ala Met Leu Glu Met Asn Ala Arg
   1125 1130 1135
   Ser Leu Gln Gln Lys Leu Glu Thr Glu Arg Glu Leu Lys Gln Arg Leu
   1140 1145 1150
   Leu Glu Glu Gln Ala Lys Leu Gln Gln Gln Met Asp Leu Gln Lys Asn
   1155 1160 1165
   His Ile Phe Arg Leu Thr Gln Gly Leu Gln Glu Ala Leu Asp Arg Ala
   1170 1175 1180
   Asp Leu Leu Lys Thr Glu Arg Ser Asp Leu Glu Tyr Gln Leu Glu Asn
   1185 1190 1195 1200
   Ile Gln Val Leu Tyr Ser His Glu Lys Val Lys Met Glu Gly Thr Ile
   1205 1210 1215
   Ser Gln Gln Thr Lys Leu Ile Asp Phe Leu Gln Ala Lys Met Asp Gln
   1220 1225 1230
   Pro Ala Lys Lys Lys Lys Gly Leu Phe Ser Arg Arg Lys Glu Asp Pro
   1235 1240 1245
   Ala Leu Pro Thr Gln Val Pro Leu Gln Tyr Asn Glu Leu Lys Leu Ala
      1250                1255                1260
   Leu Glu Lys Glu Lys Ala Arg Cys Ala Glu Leu Glu Glu Ala Leu Gln
   1265                1270                1275                1280
   Lys Thr Arg Ile Glu Leu Arg Ser Ala Arg Glu Glu Ala Ala His Arg
                  1285                1290                1295
   Lys Ala Thr Asp His Pro His Pro Ser Thr Pro Ala Thr Ala Arg Gln
              1300                1305                1310
   Gln Ile Ala Met Ser Ala Ile Val Arg Ser Pro Glu His Gln Pro Ser
          1315                1320                1325
   Ala Met Ser Leu Leu Ala Pro Pro Ser Ser Arg Arg Lys Glu Ser Ser
      1330                1335                1340
   Thr Pro Glu Glu Phe Ser Arg Arg Leu Lys Glu Arg Met His His Asn
   1345                1350                1355                1360
   Ile Pro His Arg Phe Asn Val Gly Leu Asn Met Arg Ala Thr Lys Cys
                  1365                1370                1375
   Ala Val Cys Leu Asp Thr Val His Phe Gly Arg Gln Ala Ser Lys Cys
              1380                1385                1390
   Leu Glu Cys Gln Val Met Cys His Pro Lys Cys Ser Thr Cys Leu Pro
          1395                1400                1405
   Ala Thr Cys Gly Leu Pro Ala Glu Tyr Ala Thr His Phe Thr Glu Ala
      1410                1415                1420
   Phe Cys Arg Asp Lys Met Asn Ser Pro Gly Leu Gln Thr Lys Glu Pro
   1425                1430                1435                1440
   Ser Ser Ser Leu His Leu Glu Gly Trp Met Lys Val Pro Arg Asn Asn
                  1445                1450                1455
   Lys Arg Gly Gln Gln Gly Trp Asp Arg Lys Tyr Ile Val Leu Glu Gly
              1460                1465                1470
   Ser Lys Val Leu Ile Tyr Asp Asn Glu Ala Arg Glu Ala Gly Gln Arg
          1475                1480                1485
   Pro Val Glu Glu Phe Glu Leu Cys Leu Pro Asp Gly Asp Val Ser Ile
      1490                1495                1500
   His Gly Ala Val Gly Ala Ser Glu Leu Ala Asn Thr Ala Lys Ala Asp
   1505                1510                1515                1520
   Val Pro Tyr Ile Leu Lys Met Glu Ser His Pro His Thr Thr Cys Trp
                  1525                1530                1535
   Pro Gly Arg Thr Leu Tyr Leu Leu Ala Pro Ser Phe Pro Asp Lys Gln
              1540                1545                1550
   Arg Trp Val Thr Ala Leu Glu Ser Val Val Ala Gly Gly Arg Val Ser
          1555                1560                1565
   Arg Glu Lys Ala Glu Ala Asp Ala Lys Leu Leu Gly Asn Ser Leu Leu
      1570                1575                1580
   Lys Leu Glu Gly Asp Asp Arg Leu Asp Met Asn Cys Thr Leu Pro Phe
   1585 1590 1595 1600
   Ser Asp Gln Val Val Leu Val Gly Thr Glu Glu Gly Leu Tyr Ala Leu
   1605 1610 1615
   Asn Val Leu Lys Asn Ser Leu Thr His Val Pro Gly Ile Gly Ala Val
   1620 1625 1630
   Phe Gln Ile Tyr Ile Ile Lys Asp Leu Glu Lys Leu Leu Met Ile Ala
   1635 1640 1645
   Gly Glu Glu Arg Ala Leu Cys Leu Val Asp Val Lys Lys Val Lys Gln
   1650 1655 1660
   Ser Leu Ala Gln Ser His Leu Pro Ala Gln Pro Asp Ile Ser Pro Asn
   1665 1670 1675 1680
   Ile Phe Glu Ala Val Lys Gly Cys His Leu Phe Gly Ala Gly Lys Ile
   1685 1690 1695
   Glu Asn Gly Leu Cys Ile Cys Ala Ala Met Pro Ser Lys Val Val Ile
   1700 1705 1710
   Leu Arg Tyr Asn Glu Asn Leu Ser Lys Tyr Cys Ile Arg Lys Glu Ile
   1715 1720 1725
   Glu Thr Ser Glu Pro Cys Ser Cys Ile His Phe Thr Asn Tyr Ser Ile
   1730 1735 1740
   Leu Ile Gly Thr Asn Lys Phe Tyr Glu Ile Asp Met Lys Gln Tyr Thr
   1745 1750 1755 1760
   Leu Glu Glu Phe Leu Asp Lys Asn Asp His Ser Leu Ala Pro Ala Val
   1765 1770 1775
   Phe Ala Ala Ser Ser Asn Ser Phe Pro Val Ser Ile Val Gln Val Asn
   1780 1785 1790
   Ser Ala Gly Gln Arg Glu Glu Tyr Leu Leu Cys Phe His Glu Phe Gly
   1795 1800 1805
   Val Phe Val Asp Ser Tyr Gly Arg Arg Ser Arg Thr Asp Asp Leu Lys
   1810 1815 1820
   Trp Ser Arg Leu Pro Leu Ala Phe Ala Tyr Arg Glu Pro Tyr Leu Phe
   1825 1830 1835 1840
   Val Thr His Phe Asn Ser Leu Glu Val Ile Glu Ile Gln Ala Arg Ser
   1845 1850 1855
   Ser Ala Gly Thr Pro Ala Arg Ala Tyr Leu Asp Ile Pro Asn Pro Arg
   1860 1865 1870
   Tyr Leu Gly Pro Ala Ile Ser Ser Gly Ala Ile Tyr Leu Ala Ser Ser
   1875 1880 1885
   Tyr Gln Asp Lys Leu Arg Val Ile Cys Cys Lys Gly Asn Leu Val Lys
   1890 1895 1900
   Glu Ser Gly Thr Glu His His Arg Gly Pro Ser Thr Ser Arg Ser Ser
   1905 1910 1915 1920
   Pro Asn Lys Arg Gly Pro Pro Thr Tyr Asn Glu His Ile Thr Lys Arg
                   1925                1930                1935
   Val Ala Ser Ser Pro Ala Pro Pro Glu Gly Pro Ser His Pro Arg Glu
               1940                1945                1950
   Pro Ser Thr Pro His Arg Tyr Arg Glu Gly Arg Thr Glu Leu Arg Arg
           1955                1960                1965
   Asp Lys Ser Pro Gly Arg Pro Leu Glu Arg Glu Lys Ser Pro Gly Arg
       1970                1975                1980
   Met Leu Ser Thr Arg Arg Glu Arg Ser Pro Gly Arg Leu Phe Glu Asp
   1985                1990                1995                2000
   Ser Ser Arg Gly Arg Leu Pro Ala Gly Ala Val Arg Thr Pro Leu Ser
                   2005                2010                2015
   Gln Val Asn Lys Val Trp Asp Gln Ser Ser Val
               2020                2025
<210> 27
   <211> 1101
   <212> PRT
   <213> Artificial Sequence
<220>
   <223> CIT protein fragment
<400> 27
   Ala His Arg Asp Glu Ile Gln Arg Lys Phe Asp Ala Leu Arg Asn Ser
   1               5                   10                  15
   Cys Thr Val Ile Thr Asp Leu Glu Glu Gln Leu Asn Gln Leu Thr Glu
               20                  25                  30
   Asp Asn Ala Glu Leu Asn Asn Gln Asn Phe Tyr Leu Ser Lys Gln Leu
           35                  40                  45
   Asp Glu Ala Ser Gly Ala Asn Asp Glu Ile Val Gln Leu Arg Ser Glu
       50                  55                  60
   Val Asp His Leu Arg Arg Glu Ile Thr Glu Arg Glu Met Gln Leu Thr
   65                  70                  75                  80
   Ser Gln Lys Gln Thr Met Glu Ala Leu Lys Thr Thr Cys Thr Met Leu
                   85                  90                  95
   Glu Glu Gln Val Met Asp Leu Glu Ala Leu Asn Asp Glu Leu Leu Glu
               100                 105                 110
   Lys Glu Arg Gln Trp Glu Ala Trp Arg Ser Val Leu Gly Asp Glu Lys
           115                 120                 125
   Ser Gln Phe Glu Cys Arg Val Arg Glu Leu Gln Arg Met Leu Asp Thr
       130                 135                 140
   Glu Lys Gln Ser Arg Ala Arg Ala Asp Gln Arg Ile Thr Glu Ser Arg
   145                 150                 155                 160
   Gln Val Val Glu Leu Ala Val Lys Glu His Lys Ala Glu Ile Leu Ala
                   165                 170                 175
   Leu Gln Gln Ala Leu Lys Glu Gln Lys Leu Lys Ala Glu Ser Leu Ser
               180                 185                 190
   Asp Lys Leu Asn Asp Leu Glu Lys Lys His Ala Met Leu Glu Met Asn
           195                 200                 205
   Ala Arg Ser Leu Gln Gln Lys Leu Glu Thr Glu Arg Glu Leu Lys Gln
       210                 215                 220
   Arg Leu Leu Glu Glu Gln Ala Lys Leu Gln Gln Gln Met Asp Leu Gln
   225                 230                 235                 240
   Lys Asn His Ile Phe Arg Leu Thr Gln Gly Leu Gln Glu Ala Leu Asp
                   245                 250                 255
   Arg Ala Asp Leu Leu Lys Thr Glu Arg Ser Asp Leu Glu Tyr Gln Leu
               260                 265                 270
   Glu Asn Ile Gln Val Leu Tyr Ser His Glu Lys Val Lys Met Glu Gly
           275                 280                 285
   Thr Ile Ser Gln Gln Thr Lys Leu Ile Asp Phe Leu Gln Ala Lys Met
       290                 295                 300
   Asp Gln Pro Ala Lys Lys Lys Lys Gly Leu Phe Ser Arg Arg Lys Glu
   305                 310                 315                 320
   Asp Pro Ala Leu Pro Thr Gln Val Pro Leu Gln Tyr Asn Glu Leu Lys
                   325                 330                 335
   Leu Ala Leu Glu Lys Glu Lys Ala Arg Cys Ala Glu Leu Glu Glu Ala
               340                 345                 350
   Leu Gln Lys Thr Arg Ile Glu Leu Arg Ser Ala Arg Glu Glu Ala Ala
           355                 360                 365
   His Arg Lys Ala Thr Asp His Pro His Pro Ser Thr Pro Ala Thr Ala
       370                 375                 380
   Arg Gln Gln Ile Ala Met Ser Ala Ile Val Arg Ser Pro Glu His Gln
   385                 390                 395                 400
   Pro Ser Ala Met Ser Leu Leu Ala Pro Pro Ser Ser Arg Arg Lys Glu
                   405                 410                 415
   Ser Ser Thr Pro Glu Glu Phe Ser Arg Arg Leu Lys Glu Arg Met His
               420                 425                 430
   His Asn Ile Pro His Arg Phe Asn Val Gly Leu Asn Met Arg Ala Thr
           435                 440                 445
   Lys Cys Ala Val Cys Leu Asp Thr Val His Phe Gly Arg Gln Ala Ser
       450                 455                 460
   Lys Cys Leu Glu Cys Gln Val Met Cys His Pro Lys Cys Ser Thr Cys
   465                 470                 475                 480
   Leu Pro Ala Thr Cys Gly Leu Pro Ala Glu Tyr Ala Thr His Phe Thr
                   485                 490                 495
   Glu Ala Phe Cys Arg Asp Lys Met Asn Ser Pro Gly Leu Gln Thr Lys
               500                 505                 510
   Glu Pro Ser Ser Ser Leu His Leu Glu Gly Trp Met Lys Val Pro Arg
           515                 520                 525
   Asn Asn Lys Arg Gly Gln Gln Gly Trp Asp Arg Lys Tyr Ile Val Leu
       530                 535                 540
   Glu Gly Ser Lys Val Leu Ile Tyr Asp Asn Glu Ala Arg Glu Ala Gly
   545                 550                 555                 560
   Gln Arg Pro Val Glu Glu Phe Glu Leu Cys Leu Pro Asp Gly Asp Val
                   565                 570                 575
   Ser Ile His Gly Ala Val Gly Ala Ser Glu Leu Ala Asn Thr Ala Lys
               580                 585                 590
   Ala Asp Val Pro Tyr Ile Leu Lys Met Glu Ser His Pro His Thr Thr
           595                 600                 605
   Cys Trp Pro Gly Arg Thr Leu Tyr Leu Leu Ala Pro Ser Phe Pro Asp
       610                 615                 620
   Lys Gln Arg Trp Val Thr Ala Leu Glu Ser Val Val Ala Gly Gly Arg
   625                 630                 635                 640
   Val Ser Arg Glu Lys Ala Glu Ala Asp Ala Lys Leu Leu Gly Asn Ser
                   645                 650                 655
   Leu Leu Lys Leu Glu Gly Asp Asp Arg Leu Asp Met Asn Cys Thr Leu
               660                 665                 670
   Pro Phe Ser Asp Gln Val Val Leu Val Gly Thr Glu Glu Gly Leu Tyr
           675                 680                 685
   Ala Leu Asn Val Leu Lys Asn Ser Leu Thr His Val Pro Gly Ile Gly
       690                 695                 700
   Ala Val Phe Gln Ile Tyr Ile Ile Lys Asp Leu Glu Lys Leu Leu Met
   705                 710                 715                 720
   Ile Ala Gly Glu Glu Arg Ala Leu Cys Leu Val Asp Val Lys Lys Val
                   725                 730                 735
   Lys Gln Ser Leu Ala Gln Ser His Leu Pro Ala Gln Pro Asp Ile Ser
               740                 745                 750
   Pro Asn Ile Phe Glu Ala Val Lys Gly Cys His Leu Phe Gly Ala Gly
           755                 760                 765
   Lys Ile Glu Asn Gly Leu Cys Ile Cys Ala Ala Met Pro Ser Lys Val
       770                 775                 780
   Val Ile Leu Arg Tyr Asn Glu Asn Leu Ser Lys Tyr Cys Ile Arg Lys
   785                 790                 795                 800
   Glu Ile Glu Thr Ser Glu Pro Cys Ser Cys Ile His Phe Thr Asn Tyr
                   805                 810                 815
   Ser Ile Leu Ile Gly Thr Asn Lys Phe Tyr Glu Ile Asp Met Lys Gln
               820                 825                 830
   Tyr Thr Leu Glu Glu Phe Leu Asp Lys Asn Asp His Ser Leu Ala Pro
           835                 840                 845
   Ala Val Phe Ala Ala Ser Ser Asn Ser Phe Pro Val Ser Ile Val Gln
       850                 855                 860
   Val Asn Ser Ala Gly Gln Arg Glu Glu Tyr Leu Leu Cys Phe His Glu
   865                 870                 875                 880
   Phe Gly Val Phe Val Asp Ser Tyr Gly Arg Arg Ser Arg Thr Asp Asp
                   885                 890                 895
   Leu Lys Trp Ser Arg Leu Pro Leu Ala Phe Ala Tyr Arg Glu Pro Tyr
               900                 905                 910
   Leu Phe Val Thr His Phe Asn Ser Leu Glu Val Ile Glu Ile Gln Ala
           915                 920                 925
   Arg Ser Ser Ala Gly Thr Pro Ala Arg Ala Tyr Leu Asp Ile Pro Asn
       930                 935                 940
   Pro Arg Tyr Leu Gly Pro Ala Ile Ser Ser Gly Ala Ile Tyr Leu Ala
   945                 950                 955                 960
   Ser Ser Tyr Gln Asp Lys Leu Arg Val Ile Cys Cys Lys Gly Asn Leu
                   965                 970                 975
   Val Lys Glu Ser Gly Thr Glu His His Arg Gly Pro Ser Thr Ser Arg
               980                 985                 990
   Ser Ser Pro Asn Lys Arg Gly Pro Pro Thr Tyr Asn Glu His Ile Thr
           995                 1000                1005
   Lys Arg Val Ala Ser Ser Pro Ala Pro Pro Glu Gly Pro Ser His Pro
      1010                1015                1020
   Arg Glu Pro Ser Thr Pro His Arg Tyr Arg Glu Gly Arg Thr Glu Leu
   1025               1030                1035                1040
   Arg Arg Asp Lys Ser Pro Gly Arg Pro Leu Glu Arg Glu Lys Ser Pro
                  1045                1050                1055
   Gly Arg Met Leu Ser Thr Arg Arg Glu Arg Ser Pro Gly Arg Leu Phe
              1060                1065                1070
   Glu Asp Ser Ser Arg Gly Arg Leu Pro Ala Gly Ala Val Arg Thr Pro
          1075                1080                1085
   Leu Ser Gln Val Asn Lys Val Trp Asp Gln Ser Ser Val
      1090                1095                1100
<210> 28
   <211> 7
   <212> PRT
   <213> Artificial Sequence
<220>
   <223> Break-point of CIT protein fragment
<400> 28
   Ala His Arg Asp Glu Ile Gln
   1 5
<210> 29
   <211> 5271
   <212> DNA
   <213> Artificial Sequence
<220>
   <223> FGFR2-CIT fusion gene
<400> 29
   atggtcagct ggggtcgttt catctgcctg gtcgtggtca ccatggcaac cttgtccctg      60
   gcccggccct ccttcagttt agttgaggat accacattag agccagaaga gccaccaacc     120
   aaataccaaa tctctcaacc agaagtgtac gtggctgcgc caggggagtc gctagaggtg     180
   cgctgcctgt tgaaagatgc cgccgtgatc agttggacta aggatggggt gcacttgggg     240
   cccaacaata ggacagtgct tattggggag tacttgcaga taaagggcgc cacgcctaga     300
   gactccggcc tctatgcttg tactgccagt aggactgtag acagtgaaac ttggtacttc     360
   atggtgaatg tcacagatgc catctcatcc ggagatgatg aggatgacac cgatggtgcg     420
   gaagattttg tcagtgagaa cagtaacaac aagagagcac catactggac caacacagaa     480
   aagatggaaa agcggctcca tgctgtgcct gcggccaaca ctgtcaagtt tcgctgccca     540
   gccgggggga acccaatgcc aaccatgcgg tggctgaaaa acgggaagga gtttaagcag     600
   gagcatcgca ttggaggcta caaggtacga aaccagcact ggagcctcat tatggaaagt     660
   gtggtcccat ctgacaaggg aaattatacc tgtgtagtgg agaatgaata cgggtccatc     720
   aatcacacgt accacctgga tgttgtggcg cctggaagag aaaaggagat tacagcttcc     780
   ccagactacc tggagatagc catttactgc ataggggtct tcttaatcgc ctgtatggtg     840
   gtaacagtca tcctgtgccg aatgaagaac acgaccaaga agccagactt cagcagccag     900
   ccggctgtgc acaagctgac caaacgtatc cccctgcgga gacaggtaac agtttcggct     960
   gagtccagct cctccatgaa ctccaacacc ccgctggtga ggataacaac acgcctctct    1020
   tcaacggcag acacccccat gctggcaggg gtctccgagt atgaacttcc agaggaccca    1080
   aaatgggagt ttccaagaga taagctgaca ctgggcaagc ccctgggaga aggttgcttt    1140
   gggcaagtgg tcatggcgga agcagtggga attgacaaag acaagcccaa ggaggcggtc    1200
   accgtggccg tgaagatgtt gaaagatgat gccacagaga aagacctttc tgatctggtg    1260
   tcagagatgg agatgatgaa gatgattggg aaacacaaga atatcataaa tcttcttgga    1320
   gcctgcacac aggatgggcc tctctatgtc atagttgagt atgcctctaa aggcaacctc    1380
   cgagaatacc tccgagcccg gaggccaccc gggatggagt actcctatga cattaaccgt    1440
   gttcctgagg agcagatgac cttcaaggac ttggtgtcat gcacctacca gctggccaga 1500
   ggcatggagt acttggcttc ccaaaaatgt attcatcgag atttagcagc cagaaatgtt 1560
   ttggtaacag aaaacaatgt gatgaaaata gcagactttg gactcgccag agatatcaac 1620
   aatatagact attacaaaaa gaccaccaat gggcggcttc cagtcaagtg gatggctcca 1680
   gaagccctgt ttgatagagt atacactcat cagagtgatg tctggtcctt cggggtgtta 1740
   atgtgggaga tcttcacttt agggggctcg ccctacccag ggattcccgt ggaggaactt 1800
   tttaagctgc tgaaggaagg acacagaatg gataagccag ccaactgcac caacgaactg 1860
   tacatgatga tgagggactg ttggcatgca gtgccctccc agagaccaac gttcaagcag 1920
   ttggtagaag acttggatcg aattctcact ctcacaacca atgaggcaca tagagatgaa 1980
   atccagcgca aatttgatgc tcttcgtaac agctgtactg taatcacaga cctggaggag 2040
   cagctaaacc agctgaccga ggacaacgct gaactcaaca accaaaactt ctacttgtcc 2100
   aaacaactcg atgaggcttc tggcgccaac gacgagattg tacaactgcg aagtgaagtg 2160
   gaccatctcc gccgggagat cacggaacga gagatgcagc ttaccagcca gaagcaaacg 2220
   atggaggctc tgaagaccac gtgcaccatg ctggaggaac aggtcatgga tttggaggcc 2280
   ctaaacgatg agctgctaga aaaagagcgg cagtgggagg cctggaggag cgtcctgggt 2340
   gatgagaaat cccagtttga gtgtcgggtt cgagagctgc agagaatgct ggacaccgag 2400
   aaacagagca gggcgagagc cgatcagcgg atcaccgagt ctcgccaggt ggtggagctg 2460
   gcagtgaagg agcacaaggc tgagattctc gctctgcagc aggctctcaa agagcagaag 2520
   ctgaaggccg agagcctctc tgacaagctc aatgacctgg agaagaagca tgctatgctt 2580
   gaaatgaatg cccgaagctt acagcagaag ctggagactg aacgagagct caaacagagg 2640
   cttctggaag agcaagccaa attacagcag cagatggacc tgcagaaaaa tcacattttc 2700
   cgtctgactc aaggactgca agaagctcta gatcgggctg atctactgaa gacagaaaga 2760
   agtgacttgg agtatcagct ggaaaacatt caggttctct attctcatga aaaggtgaaa 2820
   atggaaggca ctatttctca acaaaccaaa ctcattgatt ttctgcaagc caaaatggac 2880
   caacctgcta aaaagaaaaa gggtttattt agtcgacgga aagaggaccc tgctttaccc 2940
   acacaggttc ctctgcagta caatgagctg aagctggccc tggagaagga gaaagctcgc 3000
   tgtgcagagc tagaggaagc ccttcagaag acccgcatcg agctccggtc cgcccgggag 3060
   gaagctgccc accgcaaagc aacggaccac ccacacccat ccacgccagc caccgcgagg 3120
   cagcagatcg ccatgtccgc catcgtgcgg tcgccagagc accagcccag tgccatgagc 3180
   ctgctggccc cgccatccag ccgcagaaag gagtcttcaa ctccagagga atttagtcgg 3240
   cgtcttaagg aacgcatgca ccacaatatt cctcaccgat tcaacgtagg actgaacatg 3300
   cgagccacaa agtgtgctgt gtgtctggat accgtgcact ttggacgcca ggcatccaaa 3360
   tgtctcgaat gtcaggtgat gtgtcacccc aagtgctcca cgtgcttgcc agccacctgc 3420
   ggcttgcctg ctgaatatgc cacacacttc accgaggcct tctgccgtga caaaatgaac 3480
   tccccaggtc tccagaccaa ggagcccagc agcagcttgc acctggaagg gtggatgaag 3540
   gtgcccagga ataacaaacg aggacagcaa ggctgggaca ggaagtacat tgtcctggag 3600
   ggatcaaaag tcctcattta tgacaatgaa gccagagaag ctggacagag gccggtggaa 3660
   gaatttgagc tgtgccttcc cgacggggat gtatctattc atggtgccgt tggtgcttcc 3720
   gaactcgcaa atacagccaa agcagatgtc ccatacatac tgaagatgga atctcacccg 3780
   cacaccacct gctggcccgg gagaaccctc tacttgctag ctcccagctt ccctgacaaa 3840
   cagcgctggg tcaccgcctt agaatcagtt gtcgcaggtg ggagagtttc tagggaaaaa 3900
   gcagaagctg atgctaaact gcttggaaac tccctgctga aactggaagg tgatgaccgt 3960
   ctagacatga actgcacgct gcccttcagt gaccaggtgg tgttggtggg caccgaggaa 4020
   gggctctacg ccctgaatgt cttgaaaaac tccctaaccc atgtcccagg aattggagca 4080
   gtcttccaaa tttatattat caaggacctg gagaagctac tcatgatagc aggagaagag 4140
   cgggcactgt gtcttgtgga cgtgaagaaa gtgaaacagt ccctggccca gtcccacctg 4200
   cctgcccagc ccgacatctc acccaacatt tttgaagctg tcaagggctg ccacttgttt 4260
   ggggcaggca agattgagaa cgggctctgc atctgtgcag ccatgcccag caaagtcgtc 4320
   attctccgct acaacgaaaa cctcagcaaa tactgcatcc ggaaagagat agagacctca 4380
   gagccctgca gctgtatcca cttcaccaat tacagtatcc tcattggaac caataaattc 4440
   tacgaaatcg acatgaagca gtacacgctc gaggaattcc tggataagaa tgaccattcc 4500
   ttggcacctg ctgtgtttgc cgcctcttcc aacagcttcc ctgtctcaat cgtgcaggtg 4560
   aacagcgcag ggcagcgaga ggagtacttg ctgtgtttcc acgaatttgg agtgttcgtg 4620
   gattcttacg gaagacgtag ccgcacagac gatctcaagt ggagtcgctt acctttggcc 4680
   tttgcctaca gagaacccta tctgtttgtg acccacttca actcactcga agtaattgag 4740
   atccaggcac gctcctcagc agggacccct gcccgagcgt acctggacat cccgaacccg 4800
   cgctacctgg gccctgccat ttcctcagga gcgatttact tggcgtcctc ataccaggat 4860
   aaattaaggg tcatttgctg caagggaaac ctcgtgaagg agtccggcac tgaacaccac 4920
   cggggcccgt ccacctcccg cagcagcccc aacaagcgag gcccacccac gtacaacgag 4980
   cacatcacca agcgcgtggc ctccagccca gcgccgcccg aaggccccag ccacccgcga 5040
   gagccaagca caccccaccg ctaccgcgag ggcggaccgg agctgcgcag ggacaagtct 5100
   cctggccgcc ccctggagcg agagaagtcc cccggccgga tgctcagcac gcggagagag 5160
   cggtcccccg ggaggctgtt tgaagacagc agcaggggcc ggctgcctgc gggagccgtg 5220
   aggaccccgc tgtcccaggt gaacaaggtc tgggaccagt cttcagtata a 5271
<210> 30
   <211> 42
   <212> DNA
   <213> Artificial Sequence
<220>
   <223> Fused region of FGFR2-CIT fusion gene
<400> 30
   ctcactctca caaccaatga ggcacataga gatgaaatcc ag 42
<210> 31
   <211> 1756
   <212> PRT
   <213> Artificial Sequence
<220>
   <223> FGFR2-CIT fusion protein
<400> 31
   Met Val Ser Trp Gly Arg Phe Ile Cys Leu Val Val Val Thr Met Ala
   1               5                   10                  15
   Thr Leu Ser Leu Ala Arg Pro Ser Phe Ser Leu Val Glu Asp Thr Thr
               20                  25                  30
   Leu Glu Pro Glu Glu Pro Pro Thr Lys Tyr Gln Ile Ser Gln Pro Glu
           35                  40                  45
   Val Tyr Val Ala Ala Pro Gly Glu Ser Leu Glu Val Arg Cys Leu Leu
       50                  55                  60
   Lys Asp Ala Ala Val Ile Ser Trp Thr Lys Asp Gly Val His Leu Gly
   65                  70                  75                  80
   Pro Asn Asn Arg Thr Val Leu Ile Gly Glu Tyr Leu Gln Ile Lys Gly
                   85                  90                  95
   Ala Thr Pro Arg Asp Ser Gly Leu Tyr Ala Cys Thr Ala Ser Arg Thr
               100                 105                 110
   Val Asp Ser Glu Thr Trp Tyr Phe Met Val Asn Val Thr Asp Ala Ile
           115                 120                 125
   Ser Ser Gly Asp Asp Glu Asp Asp Thr Asp Gly Ala Glu Asp Phe Val
       130                 135                 140
   Ser Glu Asn Ser Asn Asn Lys Arg Ala Pro Tyr Trp Thr Asn Thr Glu
   145                 150                 155                 160
   Lys Met Glu Lys Arg Leu His Ala Val Pro Ala Ala Asn Thr Val Lys
                   165                 170                 175
   Phe Arg Cys Pro Ala Gly Gly Asn Pro Met Pro Thr Met Arg Trp Leu
               180                 185                 190
   Lys Asn Gly Lys Glu Phe Lys Gln Glu His Arg Ile Gly Gly Tyr Lys
           195                 200                 205
   Val Arg Asn Gln His Trp Ser Leu Ile Met Glu Ser Val Val Pro Ser
       210                 215                 220
   Asp Lys Gly Asn Tyr Thr Cys Val Val Glu Asn Glu Tyr Gly Ser Ile
   225                 230                 235                 240
   Asn His Thr Tyr His Leu Asp Val Val Ala Pro Gly Arg Glu Lys Glu
                   245                 250                 255
   Ile Thr Ala Ser Pro Asp Tyr Leu Glu Ile Ala Ile Tyr Cys Ile Gly
               260                 265                 270
   Val Phe Leu Ile Ala Cys Met Val Val Thr Val Ile Leu Cys Arg Met
           275                 280                 285
   Lys Asn Thr Thr Lys Lys Pro Asp Phe Ser Ser Gln Pro Ala Val His
       290                 295                 300
   Lys Leu Thr Lys Arg Ile Pro Leu Arg Arg Gln Val Thr Val Ser Ala
   305                 310                 315                 320
   Glu Ser Ser Ser Ser Met Asn Ser Asn Thr Pro Leu Val Arg Ile Thr
                   325                 330                 335
   Thr Arg Leu Ser Ser Thr Ala Asp Thr Pro Met Leu Ala Gly Val Ser
               340                 345                 350
   Glu Tyr Glu Leu Pro Glu Asp Pro Lys Trp Glu Phe Pro Arg Asp Lys
           355                 360                 365
   Leu Thr Leu Gly Lys Pro Leu Gly Glu Gly Cys Phe Gly Gln Val Val
       370                 375                 380
   Met Ala Glu Ala Val Gly Ile Asp Lys Asp Lys Pro Lys Glu Ala Val
   385                 390                 395                 400
   Thr Val Ala Val Lys Met Leu Lys Asp Asp Ala Thr Glu Lys Asp Leu
                   405                 410                 415
   Ser Asp Leu Val Ser Glu Met Glu Met Met Lys Met Ile Gly Lys His
               420                 425                 430
   Lys Asn Ile Ile Asn Leu Leu Gly Ala Cys Thr Gln Asp Gly Pro Leu
           435                 440                 445
   Tyr Val Ile Val Glu Tyr Ala Ser Lys Gly Asn Leu Arg Glu Tyr Leu
       450                 455                 460
   Arg Ala Arg Arg Pro Pro Gly Met Glu Tyr Ser Tyr Asp Ile Asn Arg
   465                 470                 475                 480
   Val Pro Glu Glu Gln Met Thr Phe Lys Asp Leu Val Ser Cys Thr Tyr
                   485                 490                 495
   Gln Leu Ala Arg Gly Met Glu Tyr Leu Ala Ser Gln Lys Cys Ile His
               500                 505                 510
   Arg Asp Leu Ala Ala Arg Asn Val Leu Val Thr Glu Asn Asn Val Met
           515                 520                 525
   Lys Ile Ala Asp Phe Gly Leu Ala Arg Asp Ile Asn Asn Ile Asp Tyr
   530 535 540
   Tyr Lys Lys Thr Thr Asn Gly Arg Leu Pro Val Lys Trp Met Ala Pro
   545 550 555 560
   Glu Ala Leu Phe Asp Arg Val Tyr Thr His Gln Ser Asp Val Trp Ser
   565 570 575
   Phe Gly Val Leu Met Trp Glu Ile Phe Thr Leu Gly Gly Ser Pro Tyr
   580 585 590
   Pro Gly Ile Pro Val Glu Glu Leu Phe Lys Leu Leu Lys Glu Gly His
   595 600 605
   Arg Met Asp Lys Pro Ala Asn Cys Thr Asn Glu Leu Tyr Met Met Met
   610 615 620
   Arg Asp Cys Trp His Ala Val Pro Ser Gln Arg Pro Thr Phe Lys Gln
   625 630 635 640
   Leu Val Glu Asp Leu Asp Arg Ile Leu Thr Leu Thr Thr Asn Glu Ala
   645 650 655
   His Arg Asp Glu Ile Gln Arg Lys Phe Asp Ala Leu Arg Asn Ser Cys
   660 665 670
   Thr Val Ile Thr Asp Leu Glu Glu Gln Leu Asn Gln Leu Thr Glu Asp
   675 680 685
   Asn Ala Glu Leu Asn Asn Gln Asn Phe Tyr Leu Ser Lys Gln Leu Asp
   690 695 700
   Glu Ala Ser Gly Ala Asn Asp Glu Ile Val Gln Leu Arg Ser Glu Val
   705 710 715 720
   Asp His Leu Arg Arg Glu Ile Thr Glu Arg Glu Met Gln Leu Thr Ser
   725 730 735
   Gln Lys Gln Thr Met Glu Ala Leu Lys Thr Thr Cys Thr Met Leu Glu
   740 745 750
   Glu Gln Val Met Asp Leu Glu Ala Leu Asn Asp Glu Leu Leu Glu Lys
   755 760 765
   Glu Arg Gln Trp Glu Ala Trp Arg Ser Val Leu Gly Asp Glu Lys Ser
   770 775 780
   Gln Phe Glu Cys Arg Val Arg Glu Leu Gln Arg Met Leu Asp Thr Glu
   785 790 795 800
   Lys Gln Ser Arg Ala Arg Ala Asp Gln Arg Ile Thr Glu Ser Arg Gln
   805 810 815
   Val Val Glu Leu Ala Val Lys Glu His Lys Ala Glu Ile Leu Ala Leu
   820 825 830
   Gln Gln Ala Leu Lys Glu Gln Lys Leu Lys Ala Glu Ser Leu Ser Asp
   835 840 845
   Lys Leu Asn Asp Leu Glu Lys Lys His Ala Met Leu Glu Met Asn Ala
   850 855 860
   Arg Ser Leu Gln Gln Lys Leu Glu Thr Glu Arg Glu Leu Lys Gln Arg
   865 870 875 880
   Leu Leu Glu Glu Gln Ala Lys Leu Gln Gln Gln Met Asp Leu Gln Lys
   885 890 895
   Asn His Ile Phe Arg Leu Thr Gln Gly Leu Gln Glu Ala Leu Asp Arg
   900 905 910
   Ala Asp Leu Leu Lys Thr Glu Arg Ser Asp Leu Glu Tyr Gln Leu Glu
   915 920 925
   Asn Ile Gln Val Leu Tyr Ser His Glu Lys Val Lys Met Glu Gly Thr
   930 935 940
   Ile Ser Gln Gln Thr Lys Leu Ile Asp Phe Leu Gln Ala Lys Met Asp
   945 950 955 960
   Gln Pro Ala Lys Lys Lys Lys Gly Leu Phe Ser Arg Arg Lys Glu Asp
   965 970 975
   Pro Ala Leu Pro Thr Gln Val Pro Leu Gln Tyr Asn Glu Leu Lys Leu
   980 985 990
   Ala Leu Glu Lys Glu Lys Ala Arg Cys Ala Glu Leu Glu Glu Ala Leu
   995 1000 1005
   Gln Lys Thr Arg Ile Glu Leu Arg Ser Ala Arg Glu Glu Ala Ala His
   1010 1015 1020
   Arg Lys Ala Thr Asp His Pro His Pro Ser Thr Pro Ala Thr Ala Arg
   1025 1030 1035 1040
   Gln Gln Ile Ala Met Ser Ala Ile Val Arg Ser Pro Glu His Gln Pro
   1045 1050 1055
   Ser Ala Met Ser Leu Leu Ala Pro Pro Ser Ser Arg Arg Lys Glu Ser
   1060 1065 1070
   Ser Thr Pro Glu Glu Phe Ser Arg Arg Leu Lys Glu Arg Met His His
   1075 1080 1085
   Asn Ile Pro His Arg Phe Asn Val Gly Leu Asn Met Arg Ala Thr Lys
   1090 1095 1100
   Cys Ala Val Cys Leu Asp Thr Val His Phe Gly Arg Gln Ala Ser Lys
   1105 1110 1115 1120
   Cys Leu Glu Cys Gln Val Met Cys His Pro Lys Cys Ser Thr Cys Leu
   1125 1130 1135
   Pro Ala Thr Cys Gly Leu Pro Ala Glu Tyr Ala Thr His Phe Thr Glu
   1140 1145 1150
   Ala Phe Cys Arg Asp Lys Met Asn Ser Pro Gly Leu Gln Thr Lys Glu
   1155 1160 1165
   Pro Ser Ser Ser Leu His Leu Glu Gly Trp Met Lys Val Pro Arg Asn
   1170 1175 1180
   Asn Lys Arg Gly Gln Gln Gly Trp Asp Arg Lys Tyr Ile Val Leu Glu
   1185 1190 1195 1200
   Gly Ser Lys Val Leu Ile Tyr Asp Asn Glu Ala Arg Glu Ala Gly Gln
   1205 1210 1215
   Arg Pro Val Glu Glu Phe Glu Leu Cys Leu Pro Asp Gly Asp Val Ser
   1220 1225 1230
   Ile His Gly Ala Val Gly Ala Ser Glu Leu Ala Asn Thr Ala Lys Ala
   1235 1240 1245
   Asp Val Pro Tyr Ile Leu Lys Met Glu Ser His Pro His Thr Thr Cys
   1250 1255 1260
   Trp Pro Gly Arg Thr Leu Tyr Leu Leu Ala Pro Ser Phe Pro Asp Lys
   1265 1270 1275 1280
   Gln Arg Trp Val Thr Ala Leu Glu Ser Val Val Ala Gly Gly Arg Val
   1285 1290 1295
   Ser Arg Glu Lys Ala Glu Ala Asp Ala Lys Leu Leu Gly Asn Ser Leu
   1300 1305 1310
   Leu Lys Leu Glu Gly Asp Asp Arg Leu Asp Met Asn Cys Thr Leu Pro
   1315 1320 1325
   Phe Ser Asp Gln Val Val Leu Val Gly Thr Glu Glu Gly Leu Tyr Ala
   1330 1335 1340
   Leu Asn Val Leu Lys Asn Ser Leu Thr His Val Pro Gly Ile Gly Ala
   1345 1350 1355 1360
   Val Phe Gln Ile Tyr Ile Ile Lys Asp Leu Glu Lys Leu Leu Met Ile
   1365 1370 1375
   Ala Gly Glu Glu Arg Ala Leu Cys Leu Val Asp Val Lys Lys Val Lys
   1380 1385 1390
   Gln Ser Leu Ala Gln Ser His Leu Pro Ala Gln Pro Asp Ile Ser Pro
   1395 1400 1405
   Asn Ile Phe Glu Ala Val Lys Gly Cys His Leu Phe Gly Ala Gly Lys
   1410 1415 1420
   Ile Glu Asn Gly Leu Cys Ile Cys Ala Ala Met Pro Ser Lys Val Val
   1425 1430 1435 1440
   Ile Leu Arg Tyr Asn Glu Asn Leu Ser Lys Tyr Cys Ile Arg Lys Glu
   1445 1450 1455
   Ile Glu Thr Ser Glu Pro Cys Ser Cys Ile His Phe Thr Asn Tyr Ser
   1460 1465 1470
   Ile Leu Ile Gly Thr Asn Lys Phe Tyr Glu Ile Asp Met Lys Gln Tyr
   1475 1480 1485
   Thr Leu Glu Glu Phe Leu Asp Lys Asn Asp His Ser Leu Ala Pro Ala
   1490 1495 1500
   Val Phe Ala Ala Ser Ser Asn Ser Phe Pro Val Ser Ile Val Gln Val
   1505 1510 1515 1520
   Asn Ser Ala Gly Gln Arg Glu Glu Tyr Leu Leu Cys Phe His Glu Phe
   1525 1530 1535
   Gly Val Phe Val Asp Ser Tyr Gly Arg Arg Ser Arg Thr Asp Asp Leu
               1540                1545                1550
   Lys Trp Ser Arg Leu Pro Leu Ala Phe Ala Tyr Arg Glu Pro Tyr Leu
           1555                1560                1565
   Phe Val Thr His Phe Asn Ser Leu Glu Val Ile Glu Ile Gln Ala Arg
       1570                1575                1580
   Ser Ser Ala Gly Thr Pro Ala Arg Ala Tyr Leu Asp Ile Pro Asn Pro
   1585                1590                1595                1600
   Arg Tyr Leu Gly Pro Ala Ile Ser Ser Gly Ala Ile Tyr Leu Ala Ser
                   1605                1610                1615
   Ser Tyr Gln Asp Lys Leu Arg Val Ile Cys Cys Lys Gly Asn Leu Val
               1620                1625                1630
   Lys Glu Ser Gly Thr Glu His His Arg Gly Pro Ser Thr Ser Arg Ser
           1635                1640                1645
   Ser Pro Asn Lys Arg Gly Pro Pro Thr Tyr Asn Glu His Ile Thr Lys
       1650                1655                1660
   Arg Val Ala Ser Ser Pro Ala Pro Pro Glu Gly Pro Ser His Pro Arg
   1665                1670                1675                1680
   Glu Pro Ser Thr Pro His Arg Tyr Arg Glu Gly Arg Thr Glu Leu Arg
                   1685                1690                1695
   Arg Asp Lys Ser Pro Gly Arg Pro Leu Glu Arg Glu Lys Ser Pro Gly
               1700                1705                1710
   Arg Met Leu Ser Thr Arg Arg Glu Arg Ser Pro Gly Arg Leu Phe Glu
           1715                1720                1725
   Asp Ser Ser Arg Gly Arg Leu Pro Ala Gly Ala Val Arg Thr Pro Leu
       1730                1735                1740
   Ser Gln Val Asn Lys Val Trp Asp Gln Ser Ser Val
   1745                1750                1755
<210> 32
   <211> 14
   <212> PRT
   <213> Artificial Sequence
<220>
   <223> Fused region of FGFR2-CIT fusion protein
<400> 32
   Leu Thr Leu Thr Thr Asn Glu Ala His Arg Asp Glu Ile Gln
   1               5                   10
<210> 33
   <211> 2685
   <212> DNA
   <213> Artificial Sequence
<220>
   <223> CDS of AXL gene (NM_021913)
<400> 33
   atggcgtggc ggtgccccag gatgggcagg gtcccgctgg cctggtgctt ggcgctgtgc 60
   ggctgggcgt gcatggcccc caggggcacg caggctgaag aaagtccctt cgtgggcaac 120
   ccagggaata tcacaggtgc ccggggactc acgggcaccc ttcggtgtca gctccaggtt 180
   cagggagagc cccccgaggt acattggctt cgggatggac agatcctgga gctcgcggac 240
   agcacccaga cccaggtgcc cctgggtgag gatgaacagg atgactggat agtggtcagc 300
   cagctcagaa tcacctccct gcagctttcc gacacgggac agtaccagtg tttggtgttt 360
   ctgggacatc agaccttcgt gtcccagcct ggctatgttg ggctggaggg cttgccttac 420
   ttcctggagg agcccgaaga caggactgtg gccgccaaca cccccttcaa cctgagctgc 480
   caagctcagg gacccccaga gcccgtggac ctactctggc tccaggatgc tgtccccctg 540
   gccacggctc caggtcacgg cccccagcgc agcctgcatg ttccagggct gaacaagaca 600
   tcctctttct cctgcgaagc ccataacgcc aagggggtca ccacatcccg cacagccacc 660
   atcacagtgc tcccccagca gccccgtaac ctccacctgg tctcccgcca acccacggag 720
   ctggaggtgg cttggactcc aggcctgagc ggcatctacc ccctgaccca ctgcaccctg 780
   caggctgtgc tgtcagacga tgggatgggc atccaggcgg gagaaccaga ccccccagag 840
   gagcccctca cctcgcaagc atccgtgccc ccccatcagc ttcggctagg cagcctccat 900
   cctcacaccc cttatcacat ccgcgtggca tgcaccagca gccagggccc ctcatcctgg 960
   acccactggc ttcctgtgga gacgccggag ggagtgcccc tgggcccccc tgagaacatt 1020
   agtgctacgc ggaatgggag ccaggccttc gtgcattggc aagagccccg ggcgcccctg 1080
   cagggtaccc tgttagggta ccggctggcg tatcaaggcc aggacacccc agaggtgcta 1140
   atggacatag ggctaaggca agaggtgacc ctggagctgc agggggacgg gtctgtgtcc 1200
   aatctgacag tgtgtgtggc agcctacact gctgctgggg atggaccctg gagcctccca 1260
   gtacccctgg aggcctggcg cccagggcaa gcacagccag tccaccagct ggtgaaggaa 1320
   ccttcaactc ctgccttctc gtggccctgg tggtatgtac tgctaggagc agtcgtggcc 1380
   gctgcctgtg tcctcatctt ggctctcttc cttgtccacc ggcgaaagaa ggagacccgt 1440
   tatggagaag tgtttgaacc aacagtggaa agaggtgaac tggtagtcag gtaccgcgtg 1500
   cgcaagtcct acagtcgtcg gaccactgaa gctaccttga acagcctggg catcagtgaa 1560
   gagctgaagg agaagctgcg ggatgtgatg gtggaccggc acaaggtggc cctggggaag 1620
   actctgggag agggagagtt tggagctgtg atggaaggcc agctcaacca ggacgactcc 1680
   atcctcaagg tggctgtgaa gacgatgaag attgccatct gcacgaggtc agagctggag 1740
   gatttcctga gtgaagcggt ctgcatgaag gaatttgacc atcccaacgt catgaggctc 1800
   atcggtgtct gtttccaggg ttctgaacga gagagcttcc cagcacctgt ggtcatctta 1860
   cctttcatga aacatggaga cctacacagc ttcctcctct attcccggct cggggaccag 1920
   ccagtgtacc tgcccactca gatgctagtg aagttcatgg cagacatcgc cagtggcatg 1980
   gagtatctga gtaccaagag attcatacac cgggacctgg cggccaggaa ctgcatgctg 2040
   aatgagaaca tgtccgtgtg tgtggcggac ttcgggctct ccaagaagat ctacaatggg 2100
   gactactacc gccagggacg tatcgccaag atgccagtca agtggattgc cattgagagt 2160
   ctagctgacc gtgtctacac cagcaagagc gatgtgtggt ccttcggggt gacaatgtgg 2220
   gagattgcca caagaggcca aaccccatat ccgggcgtgg agaacagcga gatttatgac 2280
   tatctgcgcc agggaaatcg cctgaagcag cctgcggact gtctggatgg actgtatgcc 2340
   ttgatgtcgc ggtgctggga gctaaatccc caggaccggc caagttttac agagctgcgg 2400
   gaagatttgg agaacacact gaaggccttg cctcctgccc aggagcctga cgaaatcctc 2460
   tatgtcaaca tggatgaggg tggaggttat cctgaacccc ctggagctgc aggaggagct 2520
   gaccccccaa cccagccaga ccctaaggat tcctgtagct gcctcactgc ggctgaggtc 2580
   catcctgctg gacgctatgt cctctgccct tccacaaccc ctagccccgc tcagcctgct 2640
   gataggggct ccccagcagc cccagggcag gaggatggtg cctga 2685
<210> 34
   <211> 2577
   <212> DNA
   <213> Artificial Sequence
<220>
   <223> AXL gene fragment
<400> 34
   atggcgtggc ggtgccccag gatgggcagg gtcccgctgg cctggtgctt ggcgctgtgc        60
   ggctgggcgt gcatggcccc caggggcacg caggctgaag aaagtccctt cgtgggcaac       120
   ccagggaata tcacaggtgc ccggggactc acgggcaccc ttcggtgtca gctccaggtt       180
   cagggagagc cccccgaggt acattggctt cgggatggac agatcctgga gctcgcggac       240
   agcacccaga cccaggtgcc cctgggtgag gatgaacagg atgactggat agtggtcagc       300
   cagctcagaa tcacctccct gcagctttcc gacacgggac agtaccagtg tttggtgttt       360
   ctgggacatc agaccttcgt gtcccagcct ggctatgttg ggctggaggg cttgccttac       420
   ttcctggagg agcccgaaga caggactgtg gccgccaaca cccccttcaa cctgagctgc      480
   caagctcagg gacccccaga gcccgtggac ctactctggc tccaggatgc tgtccccctg      540
   gccacggctc caggtcacgg cccccagcgc agcctgcatg ttccagggct gaacaagaca       600
   tcctctttct cctgcgaagc ccataacgcc aagggggtca ccacatcccg cacagccacc      660
   atcacagtgc tcccccagca gccccgtaac ctccacctgg tctcccgcca acccacggag 720
   ctggaggtgg cttggactcc aggcctgagc ggcatctacc ccctgaccca ctgcaccctg 780
   caggctgtgc tgtcagacga tgggatgggc atccaggcgg gagaaccaga ccccccagag 840
   gagcccctca cctcgcaagc atccgtgccc ccccatcagc ttcggctagg cagcctccat 900
   cctcacaccc cttatcacat ccgcgtggca tgcaccagca gccagggccc ctcatcctgg 960
   acccactggc ttcctgtgga gacgccggag ggagtgcccc tgggcccccc tgagaacatt 1020
   agtgctacgc ggaatgggag ccaggccttc gtgcattggc aagagccccg ggcgcccctg 1080
   cagggtaccc tgttagggta ccggctggcg tatcaaggcc aggacacccc agaggtgcta 1140
   atggacatag ggctaaggca agaggtgacc ctggagctgc agggggacgg gtctgtgtcc 1200
   aatctgacag tgtgtgtggc agcctacact gctgctgggg atggaccctg gagcctccca 1260
   gtacccctgg aggcctggcg cccagggcaa gcacagccag tccaccagct ggtgaaggaa 1320
   ccttcaactc ctgccttctc gtggccctgg tggtatgtac tgctaggagc agtcgtggcc 1380
   gctgcctgtg tcctcatctt ggctctcttc cttgtccacc ggcgaaagaa ggagacccgt 1440
   tatggagaag tgtttgaacc aacagtggaa agaggtgaac tggtagtcag gtaccgcgtg 1500
   cgcaagtcct acagtcgtcg gaccactgaa gctaccttga acagcctggg catcagtgaa 1560
   gagctgaagg agaagctgcg ggatgtgatg gtggaccggc acaaggtggc cctggggaag 1620
   actctgggag agggagagtt tggagctgtg atggaaggcc agctcaacca ggacgactcc 1680
   atcctcaagg tggctgtgaa gacgatgaag attgccatct gcacgaggtc agagctggag 1740
   gatttcctga gtgaagcggt ctgcatgaag gaatttgacc atcccaacgt catgaggctc 1800
   atcggtgtct gtttccaggg ttctgaacga gagagcttcc cagcacctgt ggtcatctta 1860
   cctttcatga aacatggaga cctacacagc ttcctcctct attcccggct cggggaccag 1920
   ccagtgtacc tgcccactca gatgctagtg aagttcatgg cagacatcgc cagtggcatg 1980
   gagtatctga gtaccaagag attcatacac cgggacctgg cggccaggaa ctgcatgctg 2040
   aatgagaaca tgtccgtgtg tgtggcggac ttcgggctct ccaagaagat ctacaatggg 2100
   gactactacc gccagggacg tatcgccaag atgccagtca agtggattgc cattgagagt 2160
   ctagctgacc gtgtctacac cagcaagagc gatgtgtggt ccttcggggt gacaatgtgg 2220
   gagattgcca caagaggcca aaccccatat ccgggcgtgg agaacagcga gatttatgac 2280
   tatctgcgcc agggaaatcg cctgaagcag cctgcggact gtctggatgg actgtatgcc 2340
   ttgatgtcgc ggtgctggga gctaaatccc caggaccggc caagttttac agagctgcgg 2400
   gaagatttgg agaacacact gaaggccttg cctcctgccc aggagcctga cgaaatcctc 2460
   tatgtcaaca tggatgaggg tggaggttat cctgaacccc ctggagctgc aggaggagct 2520
   gaccccccaa cccagccaga ccctaaggat tcctgtagct gcctcactgc ggctgag 2577
<210> 35
   <211> 15
   <212> DNA
   <213> Artificial Sequence
<220>
   <223> Break-point of AXL gene fragment
<400> 35
   ctcactgcgg ctgag 15
<210> 36
   <211> 894
   <212> PRT
   <213> Artificial Sequence
<220>
   <223> AXL protein
<400> 36
   Met Ala Trp Arg Cys Pro Arg Met Gly Arg Val Pro Leu Ala Trp Cys
   1               5                   10                  15
   Leu Ala Leu Cys Gly Trp Ala Cys Met Ala Pro Arg Gly Thr Gln Ala
               20                  25                  30
   Glu Glu Ser Pro Phe Val Gly Asn Pro Gly Asn Ile Thr Gly Ala Arg
           35                  40                  45
   Gly Leu Thr Gly Thr Leu Arg Cys Gln Leu Gln Val Gln Gly Glu Pro
       50                  55                  60
   Pro Glu Val His Trp Leu Arg Asp Gly Gln Ile Leu Glu Leu Ala Asp
   65                  70                  75                  80
   Ser Thr Gln Thr Gln Val Pro Leu Gly Glu Asp Glu Gln Asp Asp Trp
                   85                  90                  95
   Ile Val Val Ser Gln Leu Arg Ile Thr Ser Leu Gln Leu Ser Asp Thr
               100                 105                 110
   Gly Gln Tyr Gln Cys Leu Val Phe Leu Gly His Gln Thr Phe Val Ser
           115                 120                 125
   Gln Pro Gly Tyr Val Gly Leu Glu Gly Leu Pro Tyr Phe Leu Glu Glu
       130                 135                 140
   Pro Glu Asp Arg Thr Val Ala Ala Asn Thr Pro Phe Asn Leu Ser Cys
   145                 150                 155                 160
   Gln Ala Gln Gly Pro Pro Glu Pro Val Asp Leu Leu Trp Leu Gln Asp
                   165                 170                 175
   Ala Val Pro Leu Ala Thr Ala Pro Gly His Gly Pro Gln Arg Ser Leu
               180                 185                 190
   His Val Pro Gly Leu Asn Lys Thr Ser Ser Phe Ser Cys Glu Ala His
           195                 200                 205
   Asn Ala Lys Gly Val Thr Thr Ser Arg Thr Ala Thr Ile Thr Val Leu
       210                 215                 220
   Pro Gln Gln Pro Arg Asn Leu His Leu Val Ser Arg Gln Pro Thr Glu
   225                 230                 235                 240
   Leu Glu Val Ala Trp Thr Pro Gly Leu Ser Gly Ile Tyr Pro Leu Thr
                   245                 250                 255
   His Cys Thr Leu Gln Ala Val Leu Ser Asp Asp Gly Met Gly Ile Gln
               260                 265                 270
   Ala Gly Glu Pro Asp Pro Pro Glu Glu Pro Leu Thr Ser Gln Ala Ser
           275                 280                 285
   Val Pro Pro His Gln Leu Arg Leu Gly Ser Leu His Pro His Thr Pro
       290                 295                 300
   Tyr His Ile Arg Val Ala Cys Thr Ser Ser Gln Gly Pro Ser Ser Trp
   305                 310                 315                 320
   Thr His Trp Leu Pro Val Glu Thr Pro Glu Gly Val Pro Leu Gly Pro
                   325                 330                 335
   Pro Glu Asn Ile Ser Ala Thr Arg Asn Gly Ser Gln Ala Phe Val His
               340                 345                 350
   Trp Gln Glu Pro Arg Ala Pro Leu Gln Gly Thr Leu Leu Gly Tyr Arg
           355                 360                 365
   Leu Ala Tyr Gln Gly Gln Asp Thr Pro Glu Val Leu Met Asp Ile Gly
       370                 375                 380
   Leu Arg Gln Glu Val Thr Leu Glu Leu Gln Gly Asp Gly Ser Val Ser
   385                 390                 395                 400
   Asn Leu Thr Val Cys Val Ala Ala Tyr Thr Ala Ala Gly Asp Gly Pro
                   405                 410                 415
   Trp Ser Leu Pro Val Pro Leu Glu Ala Trp Arg Pro Gly Gln Ala Gln
               420                 425                 430
   Pro Val His Gln Leu Val Lys Glu Pro Ser Thr Pro Ala Phe Ser Trp
           435                 440                 445
   Pro Trp Trp Tyr Val Leu Leu Gly Ala Val Val Ala Ala Ala Cys Val
       450                 455                 460
   Leu Ile Leu Ala Leu Phe Leu Val His Arg Arg Lys Lys Glu Thr Arg
   465                 470                 475                 480
   Tyr Gly Glu Val Phe Glu Pro Thr Val Glu Arg Gly Glu Leu Val Val
                   485                 490                 495
   Arg Tyr Arg Val Arg Lys Ser Tyr Ser Arg Arg Thr Thr Glu Ala Thr
               500                 505                 510
   Leu Asn Ser Leu Gly Ile Ser Glu Glu Leu Lys Glu Lys Leu Arg Asp
           515                 520                 525
   Val Met Val Asp Arg His Lys Val Ala Leu Gly Lys Thr Leu Gly Glu
       530                 535                 540
   Gly Glu Phe Gly Ala Val Met Glu Gly Gln Leu Asn Gln Asp Asp Ser
   545 550 555 560
   Ile Leu Lys Val Ala Val Lys Thr Met Lys Ile Ala Ile Cys Thr Arg
   565 570 575
   Ser Glu Leu Glu Asp Phe Leu Ser Glu Ala Val Cys Met Lys Glu Phe
   580 585 590
   Asp His Pro Asn Val Met Arg Leu Ile Gly Val Cys Phe Gln Gly Ser
   595 600 605
   Glu Arg Glu Ser Phe Pro Ala Pro Val Val Ile Leu Pro Phe Met Lys
   610 615 620
   His Gly Asp Leu His Ser Phe Leu Leu Tyr Ser Arg Leu Gly Asp Gln
   625 630 635 640
   Pro Val Tyr Leu Pro Thr Gln Met Leu Val Lys Phe Met Ala Asp Ile
   645 650 655
   Ala Ser Gly Met Glu Tyr Leu Ser Thr Lys Arg Phe Ile His Arg Asp
   660 665 670
   Leu Ala Ala Arg Asn Cys Met Leu Asn Glu Asn Met Ser Val Cys Val
   675 680 685
   Ala Asp Phe Gly Leu Ser Lys Lys Ile Tyr Asn Gly Asp Tyr Tyr Arg
   690 695 700
   Gln Gly Arg Ile Ala Lys Met Pro Val Lys Trp Ile Ala Ile Glu Ser
   705 710 715 720
   Leu Ala Asp Arg Val Tyr Thr Ser Lys Ser Asp Val Trp Ser Phe Gly
   725 730 735
   Val Thr Met Trp Glu Ile Ala Thr Arg Gly Gln Thr Pro Tyr Pro Gly
   740 745 750
   Val Glu Asn Ser Glu Ile Tyr Asp Tyr Leu Arg Gln Gly Asn Arg Leu
   755 760 765
   Lys Gln Pro Ala Asp Cys Leu Asp Gly Leu Tyr Ala Leu Met Ser Arg
   770 775 780
   Cys Trp Glu Leu Asn Pro Gln Asp Arg Pro Ser Phe Thr Glu Leu Arg
   785 790 795 800
   Glu Asp Leu Glu Asn Thr Leu Lys Ala Leu Pro Pro Ala Gln Glu Pro
   805 810 815
   Asp Glu Ile Leu Tyr Val Asn Met Asp Glu Gly Gly Gly Tyr Pro Glu
   820 825 830
   Pro Pro Gly Ala Ala Gly Gly Ala Asp Pro Pro Thr Gln Pro Asp Pro
   835 840 845
   Lys Asp Ser Cys Ser Cys Leu Thr Ala Ala Glu Val His Pro Ala Gly
   850 855 860
   Arg Tyr Val Leu Cys Pro Ser Thr Thr Pro Ser Pro Ala Gln Pro Ala
   865 870 875 880
   Asp Arg Gly Ser Pro Ala Ala Pro Gly Gln Glu Asp Gly Ala
   885 890
<210> 37
   <211> 859
   <212> PRT
   <213> Artificial Sequence
<220>
   <223> AXL protein fragment
<400> 37
   Met Ala Trp Arg Cys Pro Arg Met Gly Arg Val Pro Leu Ala Trp Cys
   1 5 10 15
   Leu Ala Leu Cys Gly Trp Ala Cys Met Ala Pro Arg Gly Thr Gln Ala
   20 25 30
   Glu Glu Ser Pro Phe Val Gly Asn Pro Gly Asn Ile Thr Gly Ala Arg
   35 40 45
   Gly Leu Thr Gly Thr Leu Arg Cys Gln Leu Gln Val Gln Gly Glu Pro
   50 55 60
   Pro Glu Val His Trp Leu Arg Asp Gly Gln Ile Leu Glu Leu Ala Asp
   65 70 75 80
   Ser Thr Gln Thr Gln Val Pro Leu Gly Glu Asp Glu Gln Asp Asp Trp
   85 90 95
   Ile Val Val Ser Gln Leu Arg Ile Thr Ser Leu Gln Leu Ser Asp Thr
   100 105 110
   Gly Gln Tyr Gln Cys Leu Val Phe Leu Gly His Gln Thr Phe Val Ser
   115 120 125
   Gln Pro Gly Tyr Val Gly Leu Glu Gly Leu Pro Tyr Phe Leu Glu Glu
   130 135 140
   Pro Glu Asp Arg Thr Val Ala Ala Asn Thr Pro Phe Asn Leu Ser Cys
   145 150 155 160
   Gln Ala Gln Gly Pro Pro Glu Pro Val Asp Leu Leu Trp Leu Gln Asp
   165 170 175
   Ala Val Pro Leu Ala Thr Ala Pro Gly His Gly Pro Gln Arg Ser Leu
   180 185 190
   His Val Pro Gly Leu Asn Lys Thr Ser Ser Phe Ser Cys Glu Ala His
   195 200 205
   Asn Ala Lys Gly Val Thr Thr Ser Arg Thr Ala Thr Ile Thr Val Leu
   210 215 220
   Pro Gln Gln Pro Arg Asn Leu His Leu Val Ser Arg Gln Pro Thr Glu
   225 230 235 240
   Leu Glu Val Ala Trp Thr Pro Gly Leu Ser Gly Ile Tyr Pro Leu Thr
   245 250 255
   His Cys Thr Leu Gln Ala Val Leu Ser Asp Asp Gly Met Gly Ile Gln
               260                 265                 270
   Ala Gly Glu Pro Asp Pro Pro Glu Glu Pro Leu Thr Ser Gln Ala Ser
           275                 280                 285
   Val Pro Pro His Gln Leu Arg Leu Gly Ser Leu His Pro His Thr Pro
       290                 295                 300
   Tyr His Ile Arg Val Ala Cys Thr Ser Ser Gln Gly Pro Ser Ser Trp
   305                 310                 315                 320
   Thr His Trp Leu Pro Val Glu Thr Pro Glu Gly Val Pro Leu Gly Pro
                   325                 330                 335
   Pro Glu Asn Ile Ser Ala Thr Arg Asn Gly Ser Gln Ala Phe Val His
               340                 345                 350
   Trp Gln Glu Pro Arg Ala Pro Leu Gln Gly Thr Leu Leu Gly Tyr Arg
           355                 360                 365
   Leu Ala Tyr Gln Gly Gln Asp Thr Pro Glu Val Leu Met Asp Ile Gly
       370                 375                 380
   Leu Arg Gln Glu Val Thr Leu Glu Leu Gln Gly Asp Gly Ser Val Ser
   385                 390                 395                 400
   Asn Leu Thr Val Cys Val Ala Ala Tyr Thr Ala Ala Gly Asp Gly Pro
                   405                 410                 415
   Trp Ser Leu Pro Val Pro Leu Glu Ala Trp Arg Pro Gly Gln Ala Gln
               420                 425                 430
   Pro Val His Gln Leu Val Lys Glu Pro Ser Thr Pro Ala Phe Ser Trp
           435                 440                 445
   Pro Trp Trp Tyr Val Leu Leu Gly Ala Val Val Ala Ala Ala Cys Val
       450                 455                 460
   Leu Ile Leu Ala Leu Phe Leu Val His Arg Arg Lys Lys Glu Thr Arg
   465                 470                 475                 480
   Tyr Gly Glu Val Phe Glu Pro Thr Val Glu Arg Gly Glu Leu Val Val
                   485                 490                 495
   Arg Tyr Arg Val Arg Lys Ser Tyr Ser Arg Arg Thr Thr Glu Ala Thr
               500                 505                 510
   Leu Asn Ser Leu Gly Ile Ser Glu Glu Leu Lys Glu Lys Leu Arg Asp
           515                 520                 525
   Val Met Val Asp Arg His Lys Val Ala Leu Gly Lys Thr Leu Gly Glu
       530                 535                 540
   Gly Glu Phe Gly Ala Val Met Glu Gly Gln Leu Asn Gln Asp Asp Ser
   545                 550                 555                 560
   Ile Leu Lys Val Ala Val Lys Thr Met Lys Ile Ala Ile Cys Thr Arg
                   565                 570                 575
   Ser Glu Leu Glu Asp Phe Leu Ser Glu Ala Val Cys Met Lys Glu Phe
               580                 585                 590
   Asp His Pro Asn Val Met Arg Leu Ile Gly Val Cys Phe Gln Gly Ser
           595                 600                 605
   Glu Arg Glu Ser Phe Pro Ala Pro Val Val Ile Leu Pro Phe Met Lys
       610                 615                 620
   His Gly Asp Leu His Ser Phe Leu Leu Tyr Ser Arg Leu Gly Asp Gln
   625                 630                 635                 640
   Pro Val Tyr Leu Pro Thr Gln Met Leu Val Lys Phe Met Ala Asp Ile
                   645                 650                 655
   Ala Ser Gly Met Glu Tyr Leu Ser Thr Lys Arg Phe Ile His Arg Asp
               660                 665                 670
   Leu Ala Ala Arg Asn Cys Met Leu Asn Glu Asn Met Ser Val Cys Val
           675                 680                 685
   Ala Asp Phe Gly Leu Ser Lys Lys Ile Tyr Asn Gly Asp Tyr Tyr Arg
       690                 695                 700
   Gln Gly Arg Ile Ala Lys Met Pro Val Lys Trp Ile Ala Ile Glu Ser
   705                 710                 715                 720
   Leu Ala Asp Arg Val Tyr Thr Ser Lys Ser Asp Val Trp Ser Phe Gly
                   725                 730                 735
   Val Thr Met Trp Glu Ile Ala Thr Arg Gly Gln Thr Pro Tyr Pro Gly
               740                 745                 750
   Val Glu Asn Ser Glu Ile Tyr Asp Tyr Leu Arg Gln Gly Asn Arg Leu
           755                 760                 765
   Lys Gln Pro Ala Asp Cys Leu Asp Gly Leu Tyr Ala Leu Met Ser Arg
       770                 775                 780
   Cys Trp Glu Leu Asn Pro Gln Asp Arg Pro Ser Phe Thr Glu Leu Arg
   785                 790                 795                 800
   Glu Asp Leu Glu Asn Thr Leu Lys Ala Leu Pro Pro Ala Gln Glu Pro
                   805                 810                 815
   Asp Glu Ile Leu Tyr Val Asn Met Asp Glu Gly Gly Gly Tyr Pro Glu
               820                 825                 830
   Pro Pro Gly Ala Ala Gly Gly Ala Asp Pro Pro Thr Gln Pro Asp Pro
           835                 840                 845
   Lys Asp Ser Cys Ser Cys Leu Thr Ala Ala Glu
       850                 855
<210> 38
   <211> 5
   <212> PRT
   <213> Artificial Sequence
<220>
   <223> Break-point of AXL protein fragment
<400> 38
   Leu Thr Ala Ala Glu
   1               5
<210> 39
   <211> 1035
   <212> DNA
   <213> Artificial Sequence
<220>
   <223> CDS of MBIP gene (NM_016586)
<400> 39
   atggctgctg ccacggagct taatcgcccg agcagcggtg acaggaacct ggagcgaaga      60
   tgcagaccca acctctcccg agaggtgctc tacgaaatct ttcgctccct acacaccctg     120
   gttggacagc ttgacctcag agatgatgtg gtgaaaatta caatcgattg gaacaagctc     180
   cagagcctct cggcattcca gcctgcattg ctctttagtg cacttgaaca acacatttta     240
   tatttacagc cttttttagc aaaacttcag tctccgatta aagaggagaa tacaactgct     300
   gttgaagaga taggaagaac agaaatgggg aacaaaaatg aagtaaatga caaattttcc     360
   attggcgacc tacaagagga agaaaagcac aaagaaagtg atttaagaga tgtgaaaaag     420
   acacagatcc attttgatcc agaagtagtt cagataaagg ctggaaaagc agaaattgac     480
   agacgaatat ctgcatttat tgaaagaaag caagctgaaa tcaatgaaaa caacgtcagg     540
   gaattttgca atgttattga ttgtaatcaa gaaaatagtt gtgcaagaac tgatgcgatt     600
   tttacccctt accccggatt taaaagtcac gtaaaagttt ctagagttgt gaatacatac     660
   ggaccacaga ctagacctga aggaattcca gggtcaggtc ataaacctaa cagcatgctt     720
   cgagactgtg gtaatcaggc tgtagaagaa cgactacaaa atattgaggc ccacttgcgg     780
   ttacagacag gtggtccagt gccaagagac atttatcaga gaattaaaaa acttgaggat     840
   aaaatccttg aattggaagg catctctcct gaatattttc agtctgtaag cttttctgga     900
   aaaagaagaa aagttcaacc acctcaacag aactattcac tggctgaact tgatgagaaa     960
   attagtgccc tcaaacaagc cctcctcaga aaatcaagag aagcagaatc catggcaacc    1020
   caccaccttc catga                                                     1035
<210> 40
   <211> 561
   <212> DNA
   <213> Artificial Sequence
<220>
   <223> MBIP gene fragment
<400> 40
   attgacagac gaatatctgc atttattgaa agaaagcaag ctgaaatcaa tgaaaacaac      60
   gtcagggaat tttgcaatgt tattgattgt aatcaagaaa atagttgtgc aagaactgat     120
   gcgattttta ccccttaccc cggatttaaa agtcacgtaa aagtttctag agttgtgaat     180
   acatacggac cacagactag acctgaagga attccagggt caggtcataa acctaacagc     240
   atgcttcgag actgtggtaa tcaggctgta gaagaacgac tacaaaatat tgaggcccac     300
   ttgcggttac agacaggtgg tccagtgcca agagacattt atcagagaat taaaaaactt     360
   gaggataaaa tccttgaatt ggaaggcatc tctcctgaat attttcagtc tgtaagcttt     420
   tctggaaaaa gaagaaaagt tcaaccacct caacagaact attcactggc tgaacttgat     480
   gagaaaatta gtgccctcaa acaagccctc ctcagaaaat caagagaagc agaatccatg     540
   gcaacccacc accttccatg a                                               561
<210> 41
   <211> 15
   <212> DNA
   <213> Artificial Sequence
<220>
   <223> Break-point of MBIP gene fragment
<400> 41
   attgacagac gaata 15
<210> 42
   <211> 344
   <212> PRT
   <213> Artificial Sequence
<220>
   <223> MBIP protein
<400> 42
   Met Ala Ala Ala Thr Glu Leu Asn Arg Pro Ser Ser Gly Asp Arg Asn
    1               5                  10                  15
   Leu Glu Arg Arg Cys Arg Pro Asn Leu Ser Arg Glu Val Leu Tyr Glu
               20                  25                  30
   Ile Phe Arg Ser Leu His Thr Leu Val Gly Gln Leu Asp Leu Arg Asp
           35                  40                  45
   Asp Val Val Lys Ile Thr Ile Asp Trp Asn Lys Leu Gln Ser Leu Ser
       50                  55                  60
   Ala Phe Gln Pro Ala Leu Leu Phe Ser Ala Leu Glu Gln His Ile Leu
   65                  70                  75                  80
   Tyr Leu Gln Pro Phe Leu Ala Lys Leu Gln Ser Pro Ile Lys Glu Glu
                   85                  90                  95
   Asn Thr Thr Ala Val Glu Glu Ile Gly Arg Thr Glu Met Gly Asn Lys
               100                 105                 110
   Asn Glu Val Asn Asp Lys Phe Ser Ile Gly Asp Leu Gln Glu Glu Glu
           115                 120                 125
   Lys His Lys Glu Ser Asp Leu Arg Asp Val Lys Lys Thr Gln Ile His
       130                 135                 140
   Phe Asp Pro Glu Val Val Gln Ile Lys Ala Gly Lys Ala Glu Ile Asp
   145                 150                 155                 160
   Arg Arg Ile Ser Ala Phe Ile Glu Arg Lys Gln Ala Glu Ile Asn Glu
                   165                 170                 175
   Asn Asn Val Arg Glu Phe Cys Asn Val Ile Asp Cys Asn Gln Glu Asn
               180                 185                 190
   Ser Cys Ala Arg Thr Asp Ala Ile Phe Thr Pro Tyr Pro Gly Phe Lys
           195                 200                 205
   Ser His Val Lys Val Ser Arg Val Val Asn Thr Tyr Gly Pro Gln Thr
       210                 215                 220
   Arg Pro Glu Gly Ile Pro Gly Ser Gly His Lys Pro Asn Ser Met Leu
   225                 230                 235                 240
   Arg Asp Cys Gly Asn Gln Ala Val Glu Glu Arg Leu Gln Asn Ile Glu
                   245                 250                 255
   Ala His Leu Arg Leu Gln Thr Gly Gly Pro Val Pro Arg Asp Ile Tyr
               260                 265                 270
   Gln Arg Ile Lys Lys Leu Glu Asp Lys Ile Leu Glu Leu Glu Gly Ile
           275                 280                 285
   Ser Pro Glu Tyr Phe Gln Ser Val Ser Phe Ser Gly Lys Arg Arg Lys
       290                 295                 300
   Val Gln Pro Pro Gln Gln Asn Tyr Ser Leu Ala Glu Leu Asp Glu Lys
   305                 310                 315                 320
   Ile Ser Ala Leu Lys Gln Ala Leu Leu Arg Lys Ser Arg Glu Ala Glu
                   325                 330                 335
   Ser Met Ala Thr His His Leu Pro
               340
<210> 43
   <211> 186
   <212> PRT
   <213> Unknown
<220>
   <223> MBIP protein fragment
<400> 43
   Ile Asp Arg Arg Ile Ser Ala Phe Ile Glu Arg Lys Gln Ala Glu Ile
   1               5                   10                  15
   Asn Glu Asn Asn Val Arg Glu Phe Cys Asn Val Ile Asp Cys Asn Gln
               20                  25                  30
   Glu Asn Ser Cys Ala Arg Thr Asp Ala Ile Phe Thr Pro Tyr Pro Gly
           35                  40                  45
   Phe Lys Ser His Val Lys Val Ser Arg Val Val Asn Thr Tyr Gly Pro
       50                  55                  60
   Gln Thr Arg Pro Glu Gly Ile Pro Gly Ser Gly His Lys Pro Asn Ser
   65                  70                  75                  80
   Met Leu Arg Asp Cys Gly Asn Gln Ala Val Glu Glu Arg Leu Gln Asn
                   85                  90                  95
   Ile Glu Ala His Leu Arg Leu Gln Thr Gly Gly Pro Val Pro Arg Asp
               100                 105                 110
   Ile Tyr Gln Arg Ile Lys Lys Leu Glu Asp Lys Ile Leu Glu Leu Glu
           115                 120                 125
   Gly Ile Ser Pro Glu Tyr Phe Gln Ser Val Ser Phe Ser Gly Lys Arg
       130                 135                 140
   Arg Lys Val Gln Pro Pro Gln Gln Asn Tyr Ser Leu Ala Glu Leu Asp
   145                 150                 155                 160
   Glu Lys Ile Ser Ala Leu Lys Gln Ala Leu Leu Arg Lys Ser Arg Glu
                   165                 170                 175
   Ala Glu Ser Met Ala Thr His His Leu Pro
               180                 185
<210> 44
   <211> 5
   <212> PRT
   <213> Artificial Sequence
<220>
   <223> Break-point of MBIP protein fragment
<400> 44
   Ile Asp Arg Arg Ile
   1               5
<210> 45
   <211> 3138
   <212> DNA
   <213> Artificial Sequence
<220>
   <223> AXL-MBIPCIT fusion gene
<400> 45
   atggcgtggc ggtgccccag gatgggcagg gtcccgctgg cctggtgctt ggcgctgtgc          60
   ggctgggcgt gcatggcccc caggggcacg caggctgaag aaagtccctt cgtgggcaac         120
   ccagggaata tcacaggtgc ccggggactc acgggcaccc ttcggtgtca gctccaggtt         180
   cagggagagc cccccgaggt acattggctt cgggatggac agatcctgga gctcgcggac        240
   agcacccaga cccaggtgcc cctgggtgag gatgaacagg atgactggat agtggtcagc         300
   cagctcagaa tcacctccct gcagctttcc gacacgggac agtaccagtg tttggtgttt 360
   ctgggacatc agaccttcgt gtcccagcct ggctatgttg ggctggaggg cttgccttac 420
   ttcctggagg agcccgaaga caggactgtg gccgccaaca cccccttcaa cctgagctgc 480
   caagctcagg gacccccaga gcccgtggac ctactctggc tccaggatgc tgtccccctg 540
   gccacggctc caggtcacgg cccccagcgc agcctgcatg ttccagggct gaacaagaca 600
   tcctctttct cctgcgaagc ccataacgcc aagggggtca ccacatcccg cacagccacc 660
   atcacagtgc tcccccagca gccccgtaac ctccacctgg tctcccgcca acccacggag 720
   ctggaggtgg cttggactcc aggcctgagc ggcatctacc ccctgaccca ctgcaccctg 780
   caggctgtgc tgtcagacga tgggatgggc atccaggcgg gagaaccaga ccccccagag 840
   gagcccctca cctcgcaagc atccgtgccc ccccatcagc ttcggctagg cagcctccat 900
   cctcacaccc cttatcacat ccgcgtggca tgcaccagca gccagggccc ctcatcctgg 960
   acccactggc ttcctgtgga gacgccggag ggagtgcccc tgggcccccc tgagaacatt 1020
   agtgctacgc ggaatgggag ccaggccttc gtgcattggc aagagccccg ggcgcccctg 1080
   cagggtaccc tgttagggta ccggctggcg tatcaaggcc aggacacccc agaggtgcta 1140
   atggacatag ggctaaggca agaggtgacc ctggagctgc agggggacgg gtctgtgtcc 1200
   aatctgacag tgtgtgtggc agcctacact gctgctgggg atggaccctg gagcctccca 1260
   gtacccctgg aggcctggcg cccagggcaa gcacagccag tccaccagct ggtgaaggaa 1320
   ccttcaactc ctgccttctc gtggccctgg tggtatgtac tgctaggagc agtcgtggcc 1380
   gctgcctgtg tcctcatctt ggctctcttc cttgtccacc ggcgaaagaa ggagacccgt 1440
   tatggagaag tgtttgaacc aacagtggaa agaggtgaac tggtagtcag gtaccgcgtg 1500
   cgcaagtcct acagtcgtcg gaccactgaa gctaccttga acagcctggg catcagtgaa 1560
   gagctgaagg agaagctgcg ggatgtgatg gtggaccggc acaaggtggc cctggggaag 1620
   actctgggag agggagagtt tggagctgtg atggaaggcc agctcaacca ggacgactcc 1680
   atcctcaagg tggctgtgaa gacgatgaag attgccatct gcacgaggtc agagctggag 1740
   gatttcctga gtgaagcggt ctgcatgaag gaatttgacc atcccaacgt catgaggctc 1800
   atcggtgtct gtttccaggg ttctgaacga gagagcttcc cagcacctgt ggtcatctta 1860
   cctttcatga aacatggaga cctacacagc ttcctcctct attcccggct cggggaccag 1920
   ccagtgtacc tgcccactca gatgctagtg aagttcatgg cagacatcgc cagtggcatg 1980
   gagtatctga gtaccaagag attcatacac cgggacctgg cggccaggaa ctgcatgctg 2040
   aatgagaaca tgtccgtgtg tgtggcggac ttcgggctct ccaagaagat ctacaatggg 2100
   gactactacc gccagggacg tatcgccaag atgccagtca agtggattgc cattgagagt 2160
   ctagctgacc gtgtctacac cagcaagagc gatgtgtggt ccttcggggt gacaatgtgg 2220
   gagattgcca caagaggcca aaccccatat ccgggcgtgg agaacagcga gatttatgac 2280
   tatctgcgcc agggaaatcg cctgaagcag cctgcggact gtctggatgg actgtatgcc 2340
   ttgatgtcgc ggtgctggga gctaaatccc caggaccggc caagttttac agagctgcgg 2400
   gaagatttgg agaacacact gaaggccttg cctcctgccc aggagcctga cgaaatcctc 2460
   tatgtcaaca tggatgaggg tggaggttat cctgaacccc ctggagctgc aggaggagct      2520
   gaccccccaa cccagccaga ccctaaggat tcctgtagct gcctcactgc ggctgagatt      2580
   gacagacgaa tatctgcatt tattgaaaga aagcaagctg aaatcaatga aaacaacgtc 2640
   agggaatttt gcaatgttat tgattgtaat caagaaaata gttgtgcaag aactgatgcg 2700
   atttttaccc cttaccccgg atttaaaagt cacgtaaaag tttctagagt tgtgaataca 2760
   tacggaccac agactagacc tgaaggaatt ccagggtcag gtcataaacc taacagcatg 2820
   cttcgagact gtggtaatca ggctgtagaa gaacgactac aaaatattga ggcccacttg 2880
   cggttacaga caggtggtcc agtgccaaga gacatttatc agagaattaa aaaacttgag 2940
   gataaaatcc ttgaattgga aggcatctct cctgaatatt ttcagtctgt aagcttttct 3000
   ggaaaaagaa gaaaagttca accacctcaa cagaactatt cactggctga acttgatgag 3060
   aaaattagtg ccctcaaaca agccctcctc agaaaatcaa gagaagcaga atccatggca 3120
   acccaccacc ttccatga 3138
<210> 46
   <211> 30
   <212> DNA
   <213> Artificial Sequence
<220>
   <223> Fused region of AXL-MBIPCIT fusion gene
<400> 46
   ctcactgcgg ctgagattga cagacgaata 30
<210> 47
   <211> 1045
   <212> PRT
   <213> Artificial Sequence
<220>
   <223> AXL-MBIPCIT fusion protein
<400> 47
   Met Ala Trp Arg Cys Pro Arg Met Gly Arg Val Pro Leu Ala Trp Cys
   1 5 10 15
   Leu Ala Leu Cys Gly Trp Ala Cys Met Ala Pro Arg Gly Thr Gln Ala
   20 25 30
   Glu Glu Ser Pro Phe Val Gly Asn Pro Gly Asn Ile Thr Gly Ala Arg
           35                  40                  45
   Gly Leu Thr Gly Thr Leu Arg Cys Gln Leu Gln Val Gln Gly Glu Pro
       50                  55                  60
   Pro Glu Val His Trp Leu Arg Asp Gly Gln Ile Leu Glu Leu Ala Asp
   65                  70                  75                  80
   Ser Thr Gln Thr Gln Val Pro Leu Gly Glu Asp Glu Gln Asp Asp Trp
                   85                  90                  95
   Ile Val Val Ser Gln Leu Arg Ile Thr Ser Leu Gln Leu Ser Asp Thr
               100                 105                 110
   Gly Gln Tyr Gln Cys Leu Val Phe Leu Gly His Gln Thr Phe Val Ser
           115                 120                 125
   Gln Pro Gly Tyr Val Gly Leu Glu Gly Leu Pro Tyr Phe Leu Glu Glu
       130                 135                 140
   Pro Glu Asp Arg Thr Val Ala Ala Asn Thr Pro Phe Asn Leu Ser Cys
   145                 150                 155                 160
   Gln Ala Gln Gly Pro Pro Glu Pro Val Asp Leu Leu Trp Leu Gln Asp
                   165                 170                 175
   Ala Val Pro Leu Ala Thr Ala Pro Gly His Gly Pro Gln Arg Ser Leu
               180                 185                 190
   His Val Pro Gly Leu Asn Lys Thr Ser Ser Phe Ser Cys Glu Ala His
           195                 200                 205
   Asn Ala Lys Gly Val Thr Thr Ser Arg Thr Ala Thr Ile Thr Val Leu
       210                 215                 220
   Pro Gln Gln Pro Arg Asn Leu His Leu Val Ser Arg Gln Pro Thr Glu
   225                 230                 235                 240
   Leu Glu Val Ala Trp Thr Pro Gly Leu Ser Gly Ile Tyr Pro Leu Thr
                   245                 250                 255
   His Cys Thr Leu Gln Ala Val Leu Ser Asp Asp Gly Met Gly Ile Gln
               260                 265                 270
   Ala Gly Glu Pro Asp Pro Pro Glu Glu Pro Leu Thr Ser Gln Ala Ser
           275                 280                 285
   Val Pro Pro His Gln Leu Arg Leu Gly Ser Leu His Pro His Thr Pro
       290                 295                 300
   Tyr His Ile Arg Val Ala Cys Thr Ser Ser Gln Gly Pro Ser Ser Trp
   305                 310                 315                 320
   Thr His Trp Leu Pro Val Glu Thr Pro Glu Gly Val Pro Leu Gly Pro
                   325                 330                 335
   Pro Glu Asn Ile Ser Ala Thr Arg Asn Gly Ser Gln Ala Phe Val His
               340                 345                 350
   Trp Gln Glu Pro Arg Ala Pro Leu Gln Gly Thr Leu Leu Gly Tyr Arg
           355                 360                 365
   Leu Ala Tyr Gln Gly Gln Asp Thr Pro Glu Val Leu Met Asp Ile Gly
       370                 375                 380
   Leu Arg Gln Glu Val Thr Leu Glu Leu Gln Gly Asp Gly Ser Val Ser
   385                 390                 395                 400
   Asn Leu Thr Val Cys Val Ala Ala Tyr Thr Ala Ala Gly Asp Gly Pro
                   405                 410                 415
   Trp Ser Leu Pro Val Pro Leu Glu Ala Trp Arg Pro Gly Gln Ala Gln
               420                 425                 430
   Pro Val His Gln Leu Val Lys Glu Pro Ser Thr Pro Ala Phe Ser Trp
           435                 440                 445
   Pro Trp Trp Tyr Val Leu Leu Gly Ala Val Val Ala Ala Ala Cys Val
       450                 455                 460
   Leu Ile Leu Ala Leu Phe Leu Val His Arg Arg Lys Lys Glu Thr Arg
   465                 470                 475                 480
   Tyr Gly Glu Val Phe Glu Pro Thr Val Glu Arg Gly Glu Leu Val Val
                   485                 490                 495
   Arg Tyr Arg Val Arg Lys Ser Tyr Ser Arg Arg Thr Thr Glu Ala Thr
               500                 505                 510
   Leu Asn Ser Leu Gly Ile Ser Glu Glu Leu Lys Glu Lys Leu Arg Asp
           515                 520                 525
   Val Met Val Asp Arg His Lys Val Ala Leu Gly Lys Thr Leu Gly Glu
       530                 535                 540
   Gly Glu Phe Gly Ala Val Met Glu Gly Gln Leu Asn Gln Asp Asp Ser
   545                 550                 555                 560
   Ile Leu Lys Val Ala Val Lys Thr Met Lys Ile Ala Ile Cys Thr Arg
                   565                 570                 575
   Ser Glu Leu Glu Asp Phe Leu Ser Glu Ala Val Cys Met Lys Glu Phe
               580                 585                 590
   Asp His Pro Asn Val Met Arg Leu Ile Gly Val Cys Phe Gln Gly Ser
           595                 600                 605
   Glu Arg Glu Ser Phe Pro Ala Pro Val Val Ile Leu Pro Phe Met Lys
       610                 615                 620
   His Gly Asp Leu His Ser Phe Leu Leu Tyr Ser Arg Leu Gly Asp Gln
   625                 630                 635                 640
   Pro Val Tyr Leu Pro Thr Gln Met Leu Val Lys Phe Met Ala Asp Ile
                   645                 650                 655
   Ala Ser Gly Met Glu Tyr Leu Ser Thr Lys Arg Phe Ile His Arg Asp
               660                 665                 670
   Leu Ala Ala Arg Asn Cys Met Leu Asn Glu Asn Met Ser Val Cys Val
           675                 680                 685
   Ala Asp Phe Gly Leu Ser Lys Lys Ile Tyr Asn Gly Asp Tyr Tyr Arg
       690                 695                 700
   Gln Gly Arg Ile Ala Lys Met Pro Val Lys Trp Ile Ala Ile Glu Ser
   705                 710                 715                 720
   Leu Ala Asp Arg Val Tyr Thr Ser Lys Ser Asp Val Trp Ser Phe Gly
                   725                 730                 735
   Val Thr Met Trp Glu Ile Ala Thr Arg Gly Gln Thr Pro Tyr Pro Gly
               740                 745                 750
   Val Glu Asn Ser Glu Ile Tyr Asp Tyr Leu Arg Gln Gly Asn Arg Leu
           755                 760                 765
   Lys Gln Pro Ala Asp Cys Leu Asp Gly Leu Tyr Ala Leu Met Ser Arg
       770                 775                 780
   Cys Trp Glu Leu Asn Pro Gln Asp Arg Pro Ser Phe Thr Glu Leu Arg
   785                 790                 795                 800
   Glu Asp Leu Glu Asn Thr Leu Lys Ala Leu Pro Pro Ala Gln Glu Pro
                   805                 810                 815
   Asp Glu Ile Leu Tyr Val Asn Met Asp Glu Gly Gly Gly Tyr Pro Glu
               820                 825                 830
   Pro Pro Gly Ala Ala Gly Gly Ala Asp Pro Pro Thr Gln Pro Asp Pro
           835                 840                 845
   Lys Asp Ser Cys Ser Cys Leu Thr Ala Ala Glu Ile Asp Arg Arg Ile
       850                 855                 860
   Ser Ala Phe Ile Glu Arg Lys Gln Ala Glu Ile Asn Glu Asn Asn Val
   865                 870                 875                 880
   Arg Glu Phe Cys Asn Val Ile Asp Cys Asn Gln Glu Asn Ser Cys Ala
                   885                 890                 895
   Arg Thr Asp Ala Ile Phe Thr Pro Tyr Pro Gly Phe Lys Ser His Val
               900                 905                 910
   Lys Val Ser Arg Val Val Asn Thr Tyr Gly Pro Gln Thr Arg Pro Glu
           915                 920                 925
   Gly Ile Pro Gly Ser Gly His Lys Pro Asn Ser Met Leu Arg Asp Cys
       930                 935                 940
   Gly Asn Gln Ala Val Glu Glu Arg Leu Gln Asn Ile Glu Ala His Leu
   945                 950                 955                 960
   Arg Leu Gln Thr Gly Gly Pro Val Pro Arg Asp Ile Tyr Gln Arg Ile
                   965                 970                 975
   Lys Lys Leu Glu Asp Lys Ile Leu Glu Leu Glu Gly Ile Ser Pro Glu
               980                 985                 990
   Tyr Phe Gln Ser Val Ser Phe Ser Gly Lys Arg Arg Lys Val Gln Pro
           995                 1000                1005
   Pro Gln Gln Asn Tyr Ser Leu Ala Glu Leu Asp Glu Lys Ile Ser Ala
      1010                 1015                1020
   Leu Lys Gln Ala Leu Leu Arg Lys Ser Arg Glu Ala Glu Ser Met Ala
   1025                1030                1035                1040
   Thr His His Leu Pro
   1045
<210> 48
   <211> 10
   <212> PRT
   <213> Artificial Sequence
<220>
   <223> Fused region of AXL-MBIPCIT fusion protein
<400> 48
   Leu Thr Ala Ala Glu Ile Asp Arg Arg Ile
   1 5 10
<210> 49
   <211> 2292
   <212> DNA
   <213> Artificial Sequence
<220>
   <223> CDS of APLP2 gene (NM_001642)
<400> 49
   atggcggcca ccgggaccgc ggccgccgca gccacgggca ggctcctgct tctgctgctg       60
   gtggggctca cggcgcctgc cttggcgctg gccggctaca tcgaggctct tgcagccaat      120
   gccggaacag gatttgctgt tgctgagcct caaatcgcaa tgttttgtgg gaagttaaat      180
   atgcatgtga acattcagac tgggaaatgg gaacctgatc caacaggcac caagagctgc      240
   tttgaaacaa aagaagaagt tcttcagtac tgtcaggaga tgtatccaga gctacagatc      300
   acaaatgtga tggaggcaaa ccagcgggtt agtattgaca actggtgccg gagggacaaa      360
   aagcaatgca agagtcgctt tgttacacct ttcaagtgtc tcgtgggtga atttgtaagt      420
   gatgtcctgc tagttccaga aaagtgccag tttttccaca aagagcggat ggaggtgtgt      480
   gagaatcacc agcactggca cacggtagtc aaagaggcat gtctgactca gggaatgacc      540
   ttatatagct acggcatgct gctcccatgt ggggtagacc agttccatgg cactgaatat      600
   gtgtgctgcc ctcagacaaa gattattgga tctgtgtcaa aagaagagga agaggaagat      660
   gaagaggaag aggaagagga agatgaagag gaagactatg atgtttataa aagtgaattt      720
   cctactgaag cagatctgga agacttcaca gaagcagctg tggatgagga tgatgaggat      780
   gaggaagaag gggaggaagt ggtggaggac cgagattact actatgacac cttcaaagga      840
   gatgactaca atgaggagaa tcctactgaa cccggcagcg acggcaccat gtcagacaag      900
   gaaattactc atgatgtcaa agctgtctgc tcccaggagg cgatgacggg gccctgccgg      960
   gccgtgatgc ctcgttggta cttcgacctc tccaagggaa agtgcgtgcg ctttatatat     1020
   ggtggctgcg gcggcaacag gaacaatttt gagtctgagg attattgtat ggctgtgtgt     1080
   aaagcgatga ttcctccaac tcctctgcca accaatgatg ttgatgtgta tttcgagacc 1140
   tctgcagatg ataatgagca tgctcgcttc cagaaggcta aggagcagct ggagattcgg 1200
   caccgcaacc gaatggacag ggtaaagaag gaatgggaag aggcagagct tcaagctaag 1260
   aacctcccca aagcagagag gcagactctg attcagcact tccaagccat ggttaaagct 1320
   ttagagaagg aagcagccag tgagaagcag cagctggtgg agacccacct ggcccgagtg 1380
   gaagctatgc tgaatgaccg ccgtcggatg gctctggaga actacctggc tgccttgcag 1440
   tctgacccgc cacggcctca tcgcattctc caggccttac ggcgttatgt ccgtgctgag 1500
   aacaaagatc gcttacatac catccgtcat taccagcatg tgttggctgt tgacccagaa 1560
   aaggcggccc agatgaaatc ccaggtgatg acacatctcc acgtgattga agaaaggagg 1620
   aaccaaagcc tctctctgct ctacaaagta ccttatgtag cccaagaaat tcaagaggaa 1680
   attgatgagc tccttcagga gcagcgtgca gatatggacc agttcactgc ctcaatctca 1740
   gagacccctg tggacgtccg ggtgagctct gaggagagtg aggagatccc accgttccac 1800
   cccttccacc ccttcccagc cctacctgag aacgaagaca ctcagccgga gttgtaccac 1860
   ccaatgaaaa aaggatctgg agtgggagag caggatgggg gactgatcgg tgccgaagag 1920
   aaagtgatta acagtaagaa taaagtggat gaaaacatgg tcattgacga gactctggat 1980
   gttaaggaaa tgattttcaa tgccgagaga gttggaggcc tcgaggaaga gcgggaatcc 2040
   gtgggcccac tgcgggagga cttcagtctg agtagcagtg ctctcattgg cctgctggtc 2100
   atcgcagtgg ccattgccac ggtcatcgtc atcagcctgg tgatgctgag gaagaggcag 2160
   tatggcacca tcagccacgg gatcgtggag gttgatccaa tgctcacccc agaagagcgt 2220
   cacctgaaca agatgcagaa ccatggctat gagaacccca cctacaaata cctggagcag 2280
   atgcagattt ag 2292
<210> 50
   <211> 1584
   <212> DNA
   <213> Artificial Sequence
<220>
   <223> APLP2 gene fragment
<400> 50
   atggcggcca ccgggaccgc ggccgccgca gccacgggca ggctcctgct tctgctgctg     60
   gtggggctca cggcgcctgc cttggcgctg gccggctaca tcgaggctct tgcagccaat    120
   gccggaacag gatttgctgt tgctgagcct caaatcgcaa tgttttgtgg gaagttaaat    180
   atgcatgtga acattcagac tgggaaatgg gaacctgatc caacaggcac caagagctgc    240
   tttgaaacaa aagaagaagt tcttcagtac tgtcaggaga tgtatccaga gctacagatc    300
   acaaatgtga tggaggcaaa ccagcgggtt agtattgaca actggtgccg gagggacaaa    360
   aagcaatgca agagtcgctt tgttacacct ttcaagtgtc tcgtgggtga atttgtaagt    420
   gatgtcctgc tagttccaga aaagtgccag tttttccaca aagagcggat ggaggtgtgt    480
   gagaatcacc agcactggca cacggtagtc aaagaggcat gtctgactca gggaatgacc    540
   ttatatagct acggcatgct gctcccatgt ggggtagacc agttccatgg cactgaatat    600
   gtgtgctgcc ctcagacaaa gattattgga tctgtgtcaa aagaagagga agaggaagat    660
   gaagaggaag aggaagagga agatgaagag gaagactatg atgtttataa aagtgaattt    720
   cctactgaag cagatctgga agacttcaca gaagcagctg tggatgagga tgatgaggat    780
   gaggaagaag gggaggaagt ggtggaggac cgagattact actatgacac cttcaaagga    840
   gatgactaca atgaggagaa tcctactgaa cccggcagcg acggcaccat gtcagacaag    900
   gaaattactc atgatgtcaa agctgtctgc tcccaggagg cgatgacggg gccctgccgg    960
   gccgtgatgc ctcgttggta cttcgacctc tccaagggaa agtgcgtgcg ctttatatat   1020
   ggtggctgcg gcggcaacag gaacaatttt gagtctgagg attattgtat ggctgtgtgt   1080
   aaagcgatga ttcctccaac tcctctgcca accaatgatg ttgatgtgta tttcgagacc   1140
   tctgcagatg ataatgagca tgctcgcttc cagaaggcta aggagcagct ggagattcgg   1200
   caccgcaacc gaatggacag ggtaaagaag gaatgggaag aggcagagct tcaagctaag   1260
   aacctcccca aagcagagag gcagactctg attcagcact tccaagccat ggttaaagct   1320
   ttagagaagg aagcagccag tgagaagcag cagctggtgg agacccacct ggcccgagtg   1380
   gaagctatgc tgaatgaccg ccgtcggatg gctctggaga actacctggc tgccttgcag   1440
   tctgacccgc cacggcctca tcgcattctc caggccttac ggcgttatgt ccgtgctgag   1500
   aacaaagatc gcttacatac catccgtcat taccagcatg tgttggctgt tgacccagaa   1560
   aaggcggccc agatgaaatc ccag                                          1584
<210> 51
   <211> 21
   <212> DNA
   <213> Artificial Sequence
<220>
   <223> Break-point of APLP2 gene fragment
<400> 51
   gcggcccaga tgaaatccca g 21
<210> 52
   <211> 763
   <212> PRT
   <213> Artificial Sequence
<220>
   <223> APLP2 protein
<400> 52
   Met Ala Ala Thr Gly Thr Ala Ala Ala Ala Ala Thr Gly Arg Leu Leu
    1               5                  10                  15
   Leu Leu Leu Leu Val Gly Leu Thr Ala Pro Ala Leu Ala Leu Ala Gly
               20                  25                  30
   Tyr Ile Glu Ala Leu Ala Ala Asn Ala Gly Thr Gly Phe Ala Val Ala
           35                  40                  45
   Glu Pro Gln Ile Ala Met Phe Cys Gly Lys Leu Asn Met His Val Asn
       50                  55                  60
   Ile Gln Thr Gly Lys Trp Glu Pro Asp Pro Thr Gly Thr Lys Ser Cys
   65                  70                  75                      80
   Phe Glu Thr Lys Glu Glu Val Leu Gln Tyr Cys Gln Glu Met Tyr Pro
                   85                  90                  95
   Glu Leu Gln Ile Thr Asn Val Met Glu Ala Asn Gln Arg Val Ser Ile
               100                 105                 110
   Asp Asn Trp Cys Arg Arg Asp Lys Lys Gln Cys Lys Ser Arg Phe Val
           115                 120                 125
   Thr Pro Phe Lys Cys Leu Val Gly Glu Phe Val Ser Asp Val Leu Leu
       130                 135                 140
   Val Pro Glu Lys Cys Gln Phe Phe His Lys Glu Arg Met Glu Val Cys
   145                 150                 155                 160
   Glu Asn His Gln His Trp His Thr Val Val Lys Glu Ala Cys Leu Thr
                   165                 170                 175
   Gln Gly Met Thr Leu Tyr Ser Tyr Gly Met Leu Leu Pro Cys Gly Val
               180                 185                 190
   Asp Gln Phe His Gly Thr Glu Tyr Val Cys Cys Pro Gln Thr Lys Ile
           195                 200                 205
   Ile Gly Ser Val Ser Lys Glu Glu Glu Glu Glu Asp Glu Glu Glu Glu
       210                 215                 220
   Glu Glu Glu Asp Glu Glu Glu Asp Tyr Asp Val Tyr Lys Ser Glu Phe
   225                 230                 235                 240
   Pro Thr Glu Ala Asp Leu Glu Asp Phe Thr Glu Ala Ala Val Asp Glu
                   245                 250                 255
   Asp Asp Glu Asp Glu Glu Glu Gly Glu Glu Val Val Glu Asp Arg Asp
               260                 265                 270
   Tyr Tyr Tyr Asp Thr Phe Lys Gly Asp Asp Tyr Asn Glu Glu Asn Pro
           275                 280                 285
   Thr Glu Pro Gly Ser Asp Gly Thr Met Ser Asp Lys Glu Ile Thr His
       290                 295                 300
   Asp Val Lys Ala Val Cys Ser Gln Glu Ala Met Thr Gly Pro Cys Arg
   305 310 315 320
   Ala Val Met Pro Arg Trp Tyr Phe Asp Leu Ser Lys Gly Lys Cys Val
   325 330 335
   Arg Phe Ile Tyr Gly Gly Cys Gly Gly Asn Arg Asn Asn Phe Glu Ser
   340 345 350
   Glu Asp Tyr Cys Met Ala Val Cys Lys Ala Met Ile Pro Pro Thr Pro
   355 360 365
   Leu Pro Thr Asn Asp Val Asp Val Tyr Phe Glu Thr Ser Ala Asp Asp
   370 375 380
   Asn Glu His Ala Arg Phe Gln Lys Ala Lys Glu Gln Leu Glu Ile Arg
   385 390 395 400
   His Arg Asn Arg Met Asp Arg Val Lys Lys Glu Trp Glu Glu Ala Glu
   405 410 415
   Leu Gln Ala Lys Asn Leu Pro Lys Ala Glu Arg Gln Thr Leu Ile Gln
   420 425 430
   His Phe Gln Ala Met Val Lys Ala Leu Glu Lys Glu Ala Ala Ser Glu
   435 440 445
   Lys Gln Gln Leu Val Glu Thr His Leu Ala Arg Val Glu Ala Met Leu
   450 455 460
   Asn Asp Arg Arg Arg Met Ala Leu Glu Asn Tyr Leu Ala Ala Leu Gln
   465 470 475 480
   Ser Asp Pro Pro Arg Pro His Arg Ile Leu Gln Ala Leu Arg Arg Tyr
   485 490 495
   Val Arg Ala Glu Asn Lys Asp Arg Leu His Thr Ile Arg His Tyr Gln
   500 505 510
   His Val Leu Ala Val Asp Pro Glu Lys Ala Ala Gln Met Lys Ser Gln
   515 520 525
   Val Met Thr His Leu His Val Ile Glu Glu Arg Arg Asn Gln Ser Leu
   530 535 540
   Ser Leu Leu Tyr Lys Val Pro Tyr Val Ala Gln Glu Ile Gln Glu Glu
   545 550 555 560
   Ile Asp Glu Leu Leu Gln Glu Gln Arg Ala Asp Met Asp Gln Phe Thr
   565 570 575
   Ala Ser Ile Ser Glu Thr Pro Val Asp Val Arg Val Ser Ser Glu Glu
   580 585 590
   Ser Glu Glu Ile Pro Pro Phe His Pro Phe His Pro Phe Pro Ala Leu
   595 600 605
   Pro Glu Asn Glu Asp Thr Gln Pro Glu Leu Tyr His Pro Met Lys Lys
   610 615 620
   Gly Ser Gly Val Gly Glu Gln Asp Gly Gly Leu Ile Gly Ala Glu Glu
   625 630 635 640
   Lys Val Ile Asn Ser Lys Asn Lys Val Asp Glu Asn Met Val Ile Asp
   645 650 655
   Glu Thr Leu Asp Val Lys Glu Met Ile Phe Asn Ala Glu Arg Val Gly
   660 665 670
   Gly Leu Glu Glu Glu Arg Glu Ser Val Gly Pro Leu Arg Glu Asp Phe
   675 680 685
   Ser Leu Ser Ser Ser Ala Leu Ile Gly Leu Leu Val Ile Ala Val Ala
   690 695 700
   Ile Ala Thr Val Ile Val Ile Ser Leu Val Met Leu Arg Lys Arg Gln
   705 710 715 720
   Tyr Gly Thr Ile Ser His Gly Ile Val Glu Val Asp Pro Met Leu Thr
   725 730 735
   Pro Glu Glu Arg His Leu Asn Lys Met Gln Asn His Gly Tyr Glu Asn
   740 745 750
   Pro Thr Tyr Lys Tyr Leu Glu Gln Met Gln Ile
   755 760
<210> 53
   <211> 528
   <212> PRT
   <213> Artificial Sequence
<220>
   <223> APLP2 protein fragment
<400> 53
   Met Ala Ala Thr Gly Thr Ala Ala Ala Ala Ala Thr Gly Arg Leu Leu
    1               5                  10                  15
   Leu Leu Leu Leu Val Gly Leu Thr Ala Pro Ala Leu Ala Leu Ala Gly
               20                  25                  30
   Tyr Ile Glu Ala Leu Ala Ala Asn Ala Gly Thr Gly Phe Ala Val Ala
           35                  40                  45
   Glu Pro Gln Ile Ala Met Phe Cys Gly Lys Leu Asn Met His Val Asn
       50                  55                  60
   Ile Gln Thr Gly Lys Trp Glu Pro Asp Pro Thr Gly Thr Lys Ser Cys
   65                  70                  75                      80
   Phe Glu Thr Lys Glu Glu Val Leu Gln Tyr Cys Gln Glu Met Tyr Pro
                   85                  90                  95
   Glu Leu Gln Ile Thr Asn Val Met Glu Ala Asn Gln Arg Val Ser Ile
               100                 105                 110
   Asp Asn Trp Cys Arg Arg Asp Lys Lys Gln Cys Lys Ser Arg Phe Val
           115                 120                 125
   Thr Pro Phe Lys Cys Leu Val Gly Glu Phe Val Ser Asp Val Leu Leu
       130                 135                 140
   Val Pro Glu Lys Cys Gln Phe Phe His Lys Glu Arg Met Glu Val Cys
   145                 150                 155                 160
   Glu Asn His Gln His Trp His Thr Val Val Lys Glu Ala Cys Leu Thr
                   165                 170                 175
   Gln Gly Met Thr Leu Tyr Ser Tyr Gly Met Leu Leu Pro Cys Gly Val
               180                 185                 190
   Asp Gln Phe His Gly Thr Glu Tyr Val Cys Cys Pro Gln Thr Lys Ile
           195                 200                 205
   Ile Gly Ser Val Ser Lys Glu Glu Glu Glu Glu Asp Glu Glu Glu Glu
       210                 215                 220
   Glu Glu Glu Asp Glu Glu Glu Asp Tyr Asp Val Tyr Lys Ser Glu Phe
   225                 230                 235                 240
   Pro Thr Glu Ala Asp Leu Glu Asp Phe Thr Glu Ala Ala Val Asp Glu
                   245                 250                 255
   Asp Asp Glu Asp Glu Glu Glu Gly Glu Glu Val Val Glu Asp Arg Asp
               260                 265                 270
   Tyr Tyr Tyr Asp Thr Phe Lys Gly Asp Asp Tyr Asn Glu Glu Asn Pro
           275                 280                 285
   Thr Glu Pro Gly Ser Asp Gly Thr Met Ser Asp Lys Glu Ile Thr His
       290                 295                 300
   Asp Val Lys Ala Val Cys Ser Gln Glu Ala Met Thr Gly Pro Cys Arg
   305                 310                 315                 320
   Ala Val Met Pro Arg Trp Tyr Phe Asp Leu Ser Lys Gly Lys Cys Val
                   325                 330                 335
   Arg Phe Ile Tyr Gly Gly Cys Gly Gly Asn Arg Asn Asn Phe Glu Ser
               340                 345                 350
   Glu Asp Tyr Cys Met Ala Val Cys Lys Ala Met Ile Pro Pro Thr Pro
           355                 360                 365
   Leu Pro Thr Asn Asp Val Asp Val Tyr Phe Glu Thr Ser Ala Asp Asp
       370                 375                 380
   Asn Glu His Ala Arg Phe Gln Lys Ala Lys Glu Gln Leu Glu Ile Arg
   385                 390                 395                 400
   His Arg Asn Arg Met Asp Arg Val Lys Lys Glu Trp Glu Glu Ala Glu
                   405                 410                 415
   Leu Gln Ala Lys Asn Leu Pro Lys Ala Glu Arg Gln Thr Leu Ile Gln
               420                 425                 430
   His Phe Gln Ala Met Val Lys Ala Leu Glu Lys Glu Ala Ala Ser Glu
           435                 440                 445
   Lys Gln Gln Leu Val Glu Thr His Leu Ala Arg Val Glu Ala Met Leu
       450                 455                 460
   Asn Asp Arg Arg Arg Met Ala Leu Glu Asn Tyr Leu Ala Ala Leu Gln
   465                 470                 475                 480
   Ser Asp Pro Pro Arg Pro His Arg Ile Leu Gln Ala Leu Arg Arg Tyr
   485 490 495
   Val Arg Ala Glu Asn Lys Asp Arg Leu His Thr Ile Arg His Tyr Gln
   500 505 510
   His Val Leu Ala Val Asp Pro Glu Lys Ala Ala Gln Met Lys Ser Gln
   515 520 525
<210> 54
   <211> 7
   <212> PRT
   <213> Artificial Sequence
<220>
   <223> Break-point of APLP2 protein fragment
<400> 54
   Ala Ala Gln Met Lys Ser Gln
   1 5
<210> 55
   <211> 735
   <212> DNA
   <213> Artificial Sequence
<220>
   <223> CDS of TNFSF11 gene (NM_033012)
<400> 55
   atggatccta atagaatatc agaagatggc actcactgca tttatagaat tttgagactc 60
   catgaaaatg cagattttca agacacaact ctggagagtc aagatacaaa attaatacct 120
   gattcatgta ggagaattaa acaggccttt caaggagctg tgcaaaagga attacaacat 180
   atcgttggat cacagcacat cagagcagag aaagcgatgg tggatggctc atggttagat 240
   ctggccaaga ggagcaagct tgaagctcag ccttttgctc atctcactat taatgccacc 300
   gacatcccat ctggttccca taaagtgagt ctgtcctctt ggtaccatga tcggggttgg 360
   gccaagatct ccaacatgac ttttagcaat ggaaaactaa tagttaatca ggatggcttt 420
   tattacctgt atgccaacat ttgctttcga catcatgaaa cttcaggaga cctagctaca 480
   gagtatcttc aactaatggt gtacgtcact aaaaccagca tcaaaatccc aagttctcat 540
   accctgatga aaggaggaag caccaagtat tggtcaggga attctgaatt ccatttttat 600
   tccataaacg ttggtggatt ttttaagtta cggtctggag aggaaatcag catcgaggtc 660
   tccaacccct ccttactgga tccggatcag gatgcaacat actttggggc ttttaaagtt 720
   cgagatatag attga 735
<210> 56
   <211> 567
   <212> DNA
   <213> Artificial Sequence
<220>
   <223> TNFSF11 gene fragment
<400> 56
   gaattacaac atatcgttgg atcacagcac atcagagcag agaaagcgat ggtggatggc      60
   tcatggttag atctggccaa gaggagcaag cttgaagctc agccttttgc tcatctcact     120
   attaatgcca ccgacatccc atctggttcc cataaagtga gtctgtcctc ttggtaccat     180
   gatcggggtt gggccaagat ctccaacatg acttttagca atggaaaact aatagttaat     240
   caggatggct tttattacct gtatgccaac atttgctttc gacatcatga aacttcagga     300
   gacctagcta cagagtatct tcaactaatg gtgtacgtca ctaaaaccag catcaaaatc     360
   ccaagttctc ataccctgat gaaaggagga agcaccaagt attggtcagg gaattctgaa     420
   ttccattttt attccataaa cgttggtgga ttttttaagt tacggtctgg agaggaaatc     480
   agcatcgagg tctccaaccc ctccttactg gatccggatc aggatgcaac atactttggg     540
   gcttttaaag ttcgagatat agattga                                         567
<210> 57
   <211> 21
   <212> DNA
   <213> Artificial Sequence
<220>
   <223> Break-point of TNFSF11 gene fragment
<400> 57
   gaattacaac atatcgttgg a 21
<210> 58
   <211> 244
   <212> PRT
   <213> Artificial Sequence
<220>
   <223> TNFSF11 protein
<400> 58
   Met Asp Pro Asn Arg Ile Ser Glu Asp Gly Thr His Cys Ile Tyr Arg
   1               5                   10                  15
   Ile Leu Arg Leu His Glu Asn Ala Asp Phe Gln Asp Thr Thr Leu Glu
               20                  25                  30
   Ser Gln Asp Thr Lys Leu Ile Pro Asp Ser Cys Arg Arg Ile Lys Gln
           35                  40                  45
   Ala Phe Gln Gly Ala Val Gln Lys Glu Leu Gln His Ile Val Gly Ser
           50                  55                  60
   Gln His Ile Arg Ala Glu Lys Ala Met Val Asp Gly Ser Trp Leu Asp
    65                  70                  75                  80
   Leu Ala Lys Arg Ser Lys Leu Glu Ala Gln Pro Phe Ala His Leu Thr
                    85                  90                  95
   Ile Asn Ala Thr Asp Ile Pro Ser Gly Ser His Lys Val Ser Leu Ser
               100                 105                 110
   Ser Trp Tyr His Asp Arg Gly Trp Ala Lys Ile Ser Asn Met Thr Phe
           115                 120                 125
   Ser Asn Gly Lys Leu Ile Val Asn Gln Asp Gly Phe Tyr Tyr Leu Tyr
       130                 135                 140
   Ala Asn Ile Cys Phe Arg His His Glu Thr Ser Gly Asp Leu Ala Thr
   145                 150                 155                 160
   Glu Tyr Leu Gln Leu Met Val Tyr Val Thr Lys Thr Ser Ile Lys Ile
                   165                 170                 175
   Pro Ser Ser His Thr Leu Met Lys Gly Gly Ser Thr Lys Tyr Trp Ser
               180                 185                 190
   Gly Asn Ser Glu Phe His Phe Tyr Ser Ile Asn Val Gly Gly Phe Phe
           195                 200                 205
   Lys Leu Arg Ser Gly Glu Glu Ile Ser Ile Glu Val Ser Asn Pro Ser
       210                 215                 220
   Leu Leu Asp Pro Asp Gln Asp Ala Thr Tyr Phe Gly Ala Phe Lys Val
   225                 230                 235                 240
   Arg Asp Ile Asp
<210> 59
   <211> 188
   <212> PRT
   <213> Artificial Sequence
<220>
   <223> TNFSF11 protein fragment
<400> 59
   Glu Leu Gln His Ile Val Gly Ser Gln His Ile Arg Ala Glu Lys Ala
   1               5                   10                  15
   Met Val Asp Gly Ser Trp Leu Asp Leu Ala Lys Arg Ser Lys Leu Glu
               20                  25                  30
   Ala Gln Pro Phe Ala His Leu Thr Ile Asn Ala Thr Asp Ile Pro Ser
           35                  40                  45
   Gly Ser His Lys Val Ser Leu Ser Ser Trp Tyr His Asp Arg Gly Trp
       50                  55                  60
   Ala Lys Ile Ser Asn Met Thr Phe Ser Asn Gly Lys Leu Ile Val Asn
   65                  70                  75                  80
   Gln Asp Gly Phe Tyr Tyr Leu Tyr Ala Asn Ile Cys Phe Arg His His
                   85                  90                  95
   Glu Thr Ser Gly Asp Leu Ala Thr Glu Tyr Leu Gln Leu Met Val Tyr
               100                 105                 110
   Val Thr Lys Thr Ser Ile Lys Ile Pro Ser Ser His Thr Leu Met Lys
           115                 120                 125
   Gly Gly Ser Thr Lys Tyr Trp Ser Gly Asn Ser Glu Phe His Phe Tyr
       130                 135                 140
   Ser Ile Asn Val Gly Gly Phe Phe Lys Leu Arg Ser Gly Glu Glu Ile
   145                 150                 155                 160
   Ser Ile Glu Val Ser Asn Pro Ser Leu Leu Asp Pro Asp Gln Asp Ala
                   165                 170                 175
   Thr Tyr Phe Gly Ala Phe Lys Val Arg Asp Ile Asp
               180                 185
<210> 60
   <211> 7
   <212> PRT
   <213> Artificial Sequence
<220>
   <223> Break-point of TNFSF11 protein fragment
<400> 60
   Glu Leu Gln His Ile Val Gly
   1               5
<210> 61
   <211> 2151
   <212> DNA
   <213> Artificial Sequence
<220>
   <223> APLP2-TNFSF11 fusion gene
<400> 61
   atggcggcca ccgggaccgc ggccgccgca gccacgggca ggctcctgct tctgctgctg      60
   gtggggctca cggcgcctgc cttggcgctg gccggctaca tcgaggctct tgcagccaat     120
   gccggaacag gatttgctgt tgctgagcct caaatcgcaa tgttttgtgg gaagttaaat     180
   atgcatgtga acattcagac tgggaaatgg gaacctgatc caacaggcac caagagctgc     240
   tttgaaacaa aagaagaagt tcttcagtac tgtcaggaga tgtatccaga gctacagatc     300
   acaaatgtga tggaggcaaa ccagcgggtt agtattgaca actggtgccg gagggacaaa     360
   aagcaatgca agagtcgctt tgttacacct ttcaagtgtc tcgtgggtga atttgtaagt     420
   gatgtcctgc tagttccaga aaagtgccag tttttccaca aagagcggat ggaggtgtgt     480
   gagaatcacc agcactggca cacggtagtc aaagaggcat gtctgactca gggaatgacc      540
   ttatatagct acggcatgct gctcccatgt ggggtagacc agttccatgg cactgaatat      600
   gtgtgctgcc ctcagacaaa gattattgga tctgtgtcaa aagaagagga agaggaagat      660
   gaagaggaag aggaagagga agatgaagag gaagactatg atgtttataa aagtgaattt      720
   cctactgaag cagatctgga agacttcaca gaagcagctg tggatgagga tgatgaggat      780
   gaggaagaag gggaggaagt ggtggaggac cgagattact actatgacac cttcaaagga      840
   gatgactaca atgaggagaa tcctactgaa cccggcagcg acggcaccat gtcagacaag      900
   gaaattactc atgatgtcaa agctgtctgc tcccaggagg cgatgacggg gccctgccgg      960
   gccgtgatgc ctcgttggta cttcgacctc tccaagggaa agtgcgtgcg ctttatatat     1020
   ggtggctgcg gcggcaacag gaacaatttt gagtctgagg attattgtat ggctgtgtgt     1080
   aaagcgatga ttcctccaac tcctctgcca accaatgatg ttgatgtgta tttcgagacc     1140
   tctgcagatg ataatgagca tgctcgcttc cagaaggcta aggagcagct ggagattcgg     1200
   caccgcaacc gaatggacag ggtaaagaag gaatgggaag aggcagagct tcaagctaag     1260
   aacctcccca aagcagagag gcagactctg attcagcact tccaagccat ggttaaagct     1320
   ttagagaagg aagcagccag tgagaagcag cagctggtgg agacccacct ggcccgagtg     1380
   gaagctatgc tgaatgaccg ccgtcggatg gctctggaga actacctggc tgccttgcag     1440
   tctgacccgc cacggcctca tcgcattctc caggccttac ggcgttatgt ccgtgctgag     1500
   aacaaagatc gcttacatac catccgtcat taccagcatg tgttggctgt tgacccagaa     1560
   aaggcggccc agatgaaatc ccaggaatta caacatatcg ttggatcaca gcacatcaga     1620
   gcagagaaag cgatggtgga tggctcatgg ttagatctgg ccaagaggag caagcttgaa     1680
   gctcagcctt ttgctcatct cactattaat gccaccgaca tcccatctgg ttcccataaa     1740
   gtgagtctgt cctcttggta ccatgatcgg ggttgggcca agatctccaa catgactttt     1800
   agcaatggaa aactaatagt taatcaggat ggcttttatt acctgtatgc caacatttgc     1860
   tttcgacatc atgaaacttc aggagaccta gctacagagt atcttcaact aatggtgtac     1920
   gtcactaaaa ccagcatcaa aatcccaagt tctcataccc tgatgaaagg aggaagcacc     1980
   aagtattggt cagggaattc tgaattccat ttttattcca taaacgttgg tggatttttt     2040
   aagttacggt ctggagagga aatcagcatc gaggtctcca acccctcctt actggatccg     2100
   gatcaggatg caacatactt tggggctttt aaagttcgag atatagattg a              2151
<210> 62
   <211> 42
   <212> DNA
   <213> Artificial Sequence
<220>
   <223> Fused region of APLP2-TNFSF11 fusion gene
<400> 62
   gcggcccaga tgaaatccca ggaattacaa catatcgttg ga 42
<210> 63
   <211> 716
   <212> PRT
   <213> Artificial Sequence
<220>
   <223> APLP2-TNFSF11 fusion protein
<400> 63
   Met Ala Ala Thr Gly Thr Ala Ala Ala Ala Ala Thr Gly Arg Leu Leu
   1               5                   10                  15
   Leu Leu Leu Leu Val Gly Leu Thr Ala Pro Ala Leu Ala Leu Ala Gly
               20                  25                  30
   Tyr Ile Glu Ala Leu Ala Ala Asn Ala Gly Thr Gly Phe Ala Val Ala
           35                  40                  45
   Glu Pro Gln Ile Ala Met Phe Cys Gly Lys Leu Asn Met His Val Asn
       50                  55                  60
   Ile Gln Thr Gly Lys Trp Glu Pro Asp Pro Thr Gly Thr Lys Ser Cys
   65                  70                  75                  80
   Phe Glu Thr Lys Glu Glu Val Leu Gln Tyr Cys Gln Glu Met Tyr Pro
                   85                  90                  95
   Glu Leu Gln Ile Thr Asn Val Met Glu Ala Asn Gln Arg Val Ser Ile
               100                 105                 110
   Asp Asn Trp Cys Arg Arg Asp Lys Lys Gln Cys Lys Ser Arg Phe Val
           115                 120                 125
   Thr Pro Phe Lys Cys Leu Val Gly Glu Phe Val Ser Asp Val Leu Leu
       130                 135                 140
   Val Pro Glu Lys Cys Gln Phe Phe His Lys Glu Arg Met Glu Val Cys
   145                 150                 155                 160
   Glu Asn His Gln His Trp His Thr Val Val Lys Glu Ala Cys Leu Thr
                   165                 170                 175
   Gln Gly Met Thr Leu Tyr Ser Tyr Gly Met Leu Leu Pro Cys Gly Val
               180                 185                 190
   Asp Gln Phe His Gly Thr Glu Tyr Val Cys Cys Pro Gln Thr Lys Ile
           195                 200                 205
   Ile Gly Ser Val Ser Lys Glu Glu Glu Glu Glu Asp Glu Glu Glu Glu
       210                 215                 220
   Glu Glu Glu Asp Glu Glu Glu Asp Tyr Asp Val Tyr Lys Ser Glu Phe
   225                 230                 235                 240
   Pro Thr Glu Ala Asp Leu Glu Asp Phe Thr Glu Ala Ala Val Asp Glu
   245 250 255
   Asp Asp Glu Asp Glu Glu Glu Gly Glu Glu Val Val Glu Asp Arg Asp
   260 265 270
   Tyr Tyr Tyr Asp Thr Phe Lys Gly Asp Asp Tyr Asn Glu Glu Asn Pro
   275 280 285
   Thr Glu Pro Gly Ser Asp Gly Thr Met Ser Asp Lys Glu Ile Thr His
   290 295 300
   Asp Val Lys Ala Val Cys Ser Gln Glu Ala Met Thr Gly Pro Cys Arg
   305 310 315 320
   Ala Val Met Pro Arg Trp Tyr Phe Asp Leu Ser Lys Gly Lys Cys Val
   325 330 335
   Arg Phe Ile Tyr Gly Gly Cys Gly Gly Asn Arg Asn Asn Phe Glu Ser
   340 345 350
   Glu Asp Tyr Cys Met Ala Val Cys Lys Ala Met Ile Pro Pro Thr Pro
   355 360 365
   Leu Pro Thr Asn Asp Val Asp Val Tyr Phe Glu Thr Ser Ala Asp Asp
   370 375 380
   Asn Glu His Ala Arg Phe Gln Lys Ala Lys Glu Gln Leu Glu Ile Arg
   385 390 395 400
   His Arg Asn Arg Met Asp Arg Val Lys Lys Glu Trp Glu Glu Ala Glu
   405 410 415
   Leu Gln Ala Lys Asn Leu Pro Lys Ala Glu Arg Gln Thr Leu Ile Gln
   420 425 430
   His Phe Gln Ala Met Val Lys Ala Leu Glu Lys Glu Ala Ala Ser Glu
   435 440 445
   Lys Gln Gln Leu Val Glu Thr His Leu Ala Arg Val Glu Ala Met Leu
   450 455 460
   Asn Asp Arg Arg Arg Met Ala Leu Glu Asn Tyr Leu Ala Ala Leu Gln
   465 470 475 480
   Ser Asp Pro Pro Arg Pro His Arg Ile Leu Gln Ala Leu Arg Arg Tyr
   485 490 495
   Val Arg Ala Glu Asn Lys Asp Arg Leu His Thr Ile Arg His Tyr Gln
   500 505 510
   His Val Leu Ala Val Asp Pro Glu Lys Ala Ala Gln Met Lys Ser Gln
   515 520 525
   Glu Leu Gln His Ile Val Gly Ser Gln His Ile Arg Ala Glu Lys Ala
   530 535 540
   Met Val Asp Gly Ser Trp Leu Asp Leu Ala Lys Arg Ser Lys Leu Glu
   545 550 555 560
   Ala Gln Pro Phe Ala His Leu Thr Ile Asn Ala Thr Asp Ile Pro Ser
   565 570 575
   Gly Ser His Lys Val Ser Leu Ser Ser Trp Tyr His Asp Arg Gly Trp
                   580                 585                 590
   Ala Lys Ile Ser Asn Met Thr Phe Ser Asn Gly Lys Leu Ile Val Asn
           595                 600                 605
   Gln Asp Gly Phe Tyr Tyr Leu Tyr Ala Asn Ile Cys Phe Arg His His
       610                 615                 620
   Glu Thr Ser Gly Asp Leu Ala Thr Glu Tyr Leu Gln Leu Met Val Tyr
   625                 630                 635                 640
   Val Thr Lys Thr Ser Ile Lys Ile Pro Ser Ser His Thr Leu Met Lys
                   645                 650                 655
   Gly Gly Ser Thr Lys Tyr Trp Ser Gly Asn Ser Glu Phe His Phe Tyr
               660                 665                 670
   Ser Ile Asn Val Gly Gly Phe Phe Lys Leu Arg Ser Gly Glu Glu Ile
           675                 680                 685
   Ser Ile Glu Val Ser Asn Pro Ser Leu Leu Asp Pro Asp Gln Asp Ala
       690                 695                 700
   Thr Tyr Phe Gly Ala Phe Lys Val Arg Asp Ile Asp
   705                 710                 715
<210> 64
   <211> 14
   <212> PRT
   <213> Artificial Sequence
<220>
   <223> Fused region of APLP2-TNFSF11 fusion protein
<400> 64
   Ala Ala Gln Met Lys Ser Gln Glu Leu Gln His Ile Val Gly
   1               5                   10
<210> 65
   <211> 2685
   <212> DNA
   <213> Artificial Sequence
<220>
   <223> CDS of MAP4K3 gene (NM_003618)
<400> 65
   atgaaccccg gcttcgattt gtcccgccgg aacccgcagg aggacttcga gctgattcag      60
   cgcatcggca gcggcaccta cggcgacgtc tacaaggcac ggaatgttaa cactggtgaa     120
   ttagcagcaa ttaaagtaat aaaattggaa ccaggagaag actttgcagt tgtgcagcaa     180
   gaaattatta tgatgaaaga ctgtaaacac ccaaatattg ttgcttattt tggaagctat     240
   ctcaggcgag ataagctttg gatttgcatg gagttttgtg gaggtggttc tttacaggat     300
   atttatcacg taactggacc tctgtcagaa ctgcaaattg catatgttag cagagaaaca     360
   ctgcagggat tatattatct tcacagtaaa ggaaaaatgc acagagatat aaagggagct     420
   aacattctat taacggataa tggtcatgtg aaattggctg attttggagt atctgcacag     480
   ataacagcta caattgccaa acggaagtct ttcattggca caccatattg gatggctcca     540
   gaagttgcag ctgttgagag gaaggggggt tacaatcaac tctgtgatct ctgggcagtg      600
   ggaatcactg ccatagaact tgcagagctt cagcctccta tgtttgactt acacccaatg     660
   agagcattat ttctaatgac aaaaagcaat tttcagcctc ctaaactaaa ggataaaatg     720
   aaatggtcaa atagttttca tcactttgtg aaaatggcac ttaccaaaaa tccgaaaaaa     780
   agacctactg ctgaaaaatt attacagcat ccttttgtaa cacaacattt gacacggtct     840
   ttggcaatcg agctgttgga taaagtaaat aatccagatc attccactta ccatgatttc     900
   gatgatgatg atcctgagcc tcttgttgct gtaccacata gaattcactc aacaagtaga     960
   aacgtgagag aagaaaaaac acgctcagag ataacctttg gccaagtgaa atttgatcca    1020
   cccttaagaa aggagacaga accacatcat gaacttcccg acagtgatgg ttttttggac   1080
   agttcagaag aaatatacta cactgcaaga tctaatctgg atctgcaact ggaatatgga    1140
   caaggacacc aaggtggtta ctttttaggt gcaaacaaga gtcttctcaa gtctgttgaa    1200
   gaagaattgc atcagcgagg acacgtcgca catttagaag atgatgaagg agatgatgat    1260
   gaatctaaac actcaactct gaaagcaaaa attccacctc ctttgccacc aaagcctaag    1320
   tctatcttca taccacagga aatgcattct actgaggatg aaaatcaagg aacaatcaag    1380
   agatgtccca tgtcagggag cccagcaaag ccatcccaag ttccacctag accaccacct    1440
   cccagattac ccccacacaa acctgttgcc ttaggaaatg gaatgagctc cttccagtta     1500
   aatggtgaac gagatggctc attatgtcaa caacagaatg aacatagagg cacaaacctt    1560
   tcaagaaaag aaaagaaaga tgtaccaaag cctattagta atggtcttcc tccaacacct    1620
   aaagtgcata tgggtgcatg tttttcaaaa gtttttaatg ggtgtccctt gaaaattcac    1680
   tgtgcatcat catggataaa cccagataca agagatcagt acttgatatt tggtgccgaa    1740
   gaagggattt ataccctcaa tcttaatgaa cttcatgaaa catcaatgga acagctattc    1800
   cctcgaaggt gtacatggtt gtatgtaatg aacaattgct tgctatcaat atctggtaaa    1860
   gcttctcagc tttattccca taatttacca gggctttttg attatgcaag acaaatgcaa   1920
   aagttacctg ttgctattcc agcacacaaa ctccctgaca gaatactgcc aaggaaattt    1980
   tctgtatcag caaaaatccc tgaaaccaaa tggtgccaga agtgttgtgt tgtaagaaat    2040
   ccttacacgg gccataaata cctatgtgga gcacttcaga ctagcattgt tctattagaa    2100
   tgggttgaac caatgcagaa atttatgtta attaagcaca tagattttcc tataccatgt    2160
   ccacttagaa tgtttgaaat gctggtagtt cctgaacagg agtacccttt agtttgtgtt    2220
   ggtgtcagta gaggtagaga cttcaaccaa gtggttcgat ttgagacggt caatccaaat    2280
   tctacctctt catggtttac agaatcagat accccacaga caaatgttac tcatgtaacc 2340
   caactggaga gagataccat ccttgtatgc ttggactgtt gtataaaaat agtaaatctc 2400
   caaggaagat taaaatctag caggaaattg tcatcagaac tcacctttga tttccagatt 2460
   gaatcaatag tgtgcctaca agacagtgtg ctagctttct ggaaacatgg aatgcaaggt 2520
   agaagtttta gatctaatga ggtaacacaa gaaatttcag atagcacaag aattttcagg 2580
   ctgcttggat ctgacagggt cgtggttttg gaaagtaggc caactgataa ccccacagca 2640
   aatagcaatt tgtacatcct ggcgggtcat gaaaacagtt actga 2685
<210> 66
   <211> 96
   <212> DNA
   <213> Artificial Sequence
<220>
   <223> MAP4K3 gene fragment
<400> 66
   atgaaccccg gcttcgattt gtcccgccgg aacccgcagg aggacttcga gctgattcag 60
   cgcatcggca gcggcaccta cggcgacgtc tacaag 96
<210> 67
   <211> 21
   <212> DNA
   <213> Artificial Sequence
<220>
   <223> Break-point of MAP4K3 gene fragment
<400> 67
   acctacggcg acgtctacaa g 21
<210> 68
   <211> 894
   <212> PRT
   <213> Artificial Sequence
<220>
   <223> MAP4K3 protein
<400> 68
   Met Asn Pro Gly Phe Asp Leu Ser Arg Arg Asn Pro Gln Glu Asp Phe
   1 5 10 15
   Glu Leu Ile Gln Arg Ile Gly Ser Gly Thr Tyr Gly Asp Val Tyr Lys
   20 25 30
   Ala Arg Asn Val Asn Thr Gly Glu Leu Ala Ala Ile Lys Val Ile Lys
   35 40 45
   Leu Glu Pro Gly Glu Asp Phe Ala Val Val Gln Gln Glu Ile Ile Met
   50 55 60
   Met Lys Asp Cys Lys His Pro Asn Ile Val Ala Tyr Phe Gly Ser Tyr
   65 70 75 80
   Leu Arg Arg Asp Lys Leu Trp Ile Cys Met Glu Phe Cys Gly Gly Gly
   85 90 95
   Ser Leu Gln Asp Ile Tyr His Val Thr Gly Pro Leu Ser Glu Leu Gln
   100 105 110
   Ile Ala Tyr Val Ser Arg Glu Thr Leu Gln Gly Leu Tyr Tyr Leu His
   115 120 125
   Ser Lys Gly Lys Met His Arg Asp Ile Lys Gly Ala Asn Ile Leu Leu
   130 135 140
   Thr Asp Asn Gly His Val Lys Leu Ala Asp Phe Gly Val Ser Ala Gln
   145 150 155 160
   Ile Thr Ala Thr Ile Ala Lys Arg Lys Ser Phe Ile Gly Thr Pro Tyr
   165 170 175
   Trp Met Ala Pro Glu Val Ala Ala Val Glu Arg Lys Gly Gly Tyr Asn
   180 185 190
   Gln Leu Cys Asp Leu Trp Ala Val Gly Ile Thr Ala Ile Glu Leu Ala
   195 200 205
   Glu Leu Gln Pro Pro Met Phe Asp Leu His Pro Met Arg Ala Leu Phe
   210 215 220
   Leu Met Thr Lys Ser Asn Phe Gln Pro Pro Lys Leu Lys Asp Lys Met
   225 230 235 240
   Lys Trp Ser Asn Ser Phe His His Phe Val Lys Met Ala Leu Thr Lys
   245 250 255
   Asn Pro Lys Lys Arg Pro Thr Ala Glu Lys Leu Leu Gln His Pro Phe
   260 265 270
   Val Thr Gln His Leu Thr Arg Ser Leu Ala Ile Glu Leu Leu Asp Lys
   275 280 285
   Val Asn Asn Pro Asp His Ser Thr Tyr His Asp Phe Asp Asp Asp Asp
   290 295 300
   Pro Glu Pro Leu Val Ala Val Pro His Arg Ile His Ser Thr Ser Arg
   305 310 315 320
   Asn Val Arg Glu Glu Lys Thr Arg Ser Glu Ile Thr Phe Gly Gln Val
   325 330 335
   Lys Phe Asp Pro Pro Leu Arg Lys Glu Thr Glu Pro His His Glu Leu
   340 345 350
   Pro Asp Ser Asp Gly Phe Leu Asp Ser Ser Glu Glu Ile Tyr Tyr Thr
   355 360 365
   Ala Arg Ser Asn Leu Asp Leu Gln Leu Glu Tyr Gly Gln Gly His Gln
   370 375 380
   Gly Gly Tyr Phe Leu Gly Ala Asn Lys Ser Leu Leu Lys Ser Val Glu
   385                 390                 395                 400
   Glu Glu Leu His Gln Arg Gly His Val Ala His Leu Glu Asp Asp Glu
                   405                 410                 415
   Gly Asp Asp Asp Glu Ser Lys His Ser Thr Leu Lys Ala Lys Ile Pro
               420                 425                 430
   Pro Pro Leu Pro Pro Lys Pro Lys Ser Ile Phe Ile Pro Gln Glu Met
           435                 440                 445
   His Ser Thr Glu Asp Glu Asn Gln Gly Thr Ile Lys Arg Cys Pro Met
       450                 455                 460
   Ser Gly Ser Pro Ala Lys Pro Ser Gln Val Pro Pro Arg Pro Pro Pro
   465                 470                 475                 480
   Pro Arg Leu Pro Pro His Lys Pro Val Ala Leu Gly Asn Gly Met Ser
                   485                 490                 495
   Ser Phe Gln Leu Asn Gly Glu Arg Asp Gly Ser Leu Cys Gln Gln Gln
               500                 505                 510
   Asn Glu His Arg Gly Thr Asn Leu Ser Arg Lys Glu Lys Lys Asp Val
           515                 520                 525
   Pro Lys Pro Ile Ser Asn Gly Leu Pro Pro Thr Pro Lys Val His Met
       530                 535                 540
   Gly Ala Cys Phe Ser Lys Val Phe Asn Gly Cys Pro Leu Lys Ile His
   545                 550                 555                 560
   Cys Ala Ser Ser Trp Ile Asn Pro Asp Thr Arg Asp Gln Tyr Leu Ile
                   565                 570                 575
   Phe Gly Ala Glu Glu Gly Ile Tyr Thr Leu Asn Leu Asn Glu Leu His
               580                 585                 590
   Glu Thr Ser Met Glu Gln Leu Phe Pro Arg Arg Cys Thr Trp Leu Tyr
           595                 600                 605
   Val Met Asn Asn Cys Leu Leu Ser Ile Ser Gly Lys Ala Ser Gln Leu
       610                 615                 620
   Tyr Ser His Asn Leu Pro Gly Leu Phe Asp Tyr Ala Arg Gln Met Gln
   625                 630                 635                 640
   Lys Leu Pro Val Ala Ile Pro Ala His Lys Leu Pro Asp Arg Ile Leu
                   645                 650                 655
   Pro Arg Lys Phe Ser Val Ser Ala Lys Ile Pro Glu Thr Lys Trp Cys
               660                 665                 670
   Gln Lys Cys Cys Val Val Arg Asn Pro Tyr Thr Gly His Lys Tyr Leu
           675                 680                 685
   Cys Gly Ala Leu Gln Thr Ser Ile Val Leu Leu Glu Trp Val Glu Pro
       690                 695                 700
   Met Gln Lys Phe Met Leu Ile Lys His Ile Asp Phe Pro Ile Pro Cys
   705                 710                 715                 720
   Pro Leu Arg Met Phe Glu Met Leu Val Val Pro Glu Gln Glu Tyr Pro
   725 730 735
   Leu Val Cys Val Gly Val Ser Arg Gly Arg Asp Phe Asn Gln Val Val
   740 745 750
   Arg Phe Glu Thr Val Asn Pro Asn Ser Thr Ser Ser Trp Phe Thr Glu
   755 760 765
   Ser Asp Thr Pro Gln Thr Asn Val Thr His Val Thr Gln Leu Glu Arg
   770 775 780
   Asp Thr Ile Leu Val Cys Leu Asp Cys Cys Ile Lys Ile Val Asn Leu
   785 790 795 800
   Gln Gly Arg Leu Lys Ser Ser Arg Lys Leu Ser Ser Glu Leu Thr Phe
   805 810 815
   Asp Phe Gln Ile Glu Ser Ile Val Cys Leu Gln Asp Ser Val Leu Ala
   820 825 830
   Phe Trp Lys His Gly Met Gln Gly Arg Ser Phe Arg Ser Asn Glu Val
   835 840 845
   Thr Gln Glu Ile Ser Asp Ser Thr Arg Ile Phe Arg Leu Leu Gly Ser
   850 855 860
   Asp Arg Val Val Val Leu Glu Ser Arg Pro Thr Asp Asn Pro Thr Ala
   865 870 875 880
   Asn Ser Asn Leu Tyr Ile Leu Ala Gly His Glu Asn Ser Tyr
   885 890
<210> 69
   <211> 32
   <212> PRT
   <213> Artificial Sequence
<220>
   <223> MAP4K3 protein fragment
<400> 69
   Met Asn Pro Gly Phe Asp Leu Ser Arg Arg Asn Pro Gln Glu Asp Phe
   1 5 10 15
   Glu Leu Ile Gln Arg Ile Gly Ser Gly Thr Tyr Gly Asp Val Tyr Lys
   20 25 30
<210> 70
   <211> 7
   <212> PRT
   <213> Artificial Sequence
<220>
   <223> Break-point of MAP4K3 protein fragment
<400> 70
   Thr Tyr Gly Asp Val Tyr Lys
   1 5
<210> 71
   <211> 2214
   <212> DNA
   <213> Artificial Sequence
<220>
   <223> CDS of PRKCE gene (NM_005400)
<400> 71
   atggtagtgt tcaatggcct tcttaagatc aaaatctgcg aggccgtgag cttgaagccc        60
   acagcctggt cgctgcgcca tgcggtggga ccccggccgc agactttcct tctcgacccc       120
   tacattgccc tcaatgtgga cgactcgcgc atcggccaaa cggccaccaa gcagaagacc       180
   aacagcccgg cctggcacga cgagttcgtc accgatgtgt gcaacggacg caagatcgag       240
   ctggctgtct ttcacgatgc ccccataggc tacgacgact tcgtggccaa ctgcaccatc       300
   cagtttgagg agctgctgca gaacgggagc cgccacttcg aggactggat tgatctggag       360
   ccagaaggaa gagtgtatgt gatcatcgat ctctcagggt cgtcgggtga agcccctaaa       420
   gacaatgaag agcgtgtgtt cagggaacgc atgcggccga ggaagcggca gggggccgtc       480
   aggcgcaggg tccatcaggt caacggccac aagttcatgg ccacctatct tcggcagccc       540
   acctactgct cccattgcag agacttcatc tggggtgtca taggaaagca gggataccag       600
   tgtcaagtct gcacctgcgt ggtccacaag cggtgccacg agctcataat cacaaagtgt       660
   gctgggttaa agaagcagga gacccccgac caggtgggct cccagcggtt cagcgtcaac       720
   atgccccaca agttcggtat ccacaactac aaggtcccta ccttctgcga tcactgtggg       780
   tccctgctct ggggactctt gcggcagggt ttgcagtgta aagtctgcaa aatgaatgtt       840
   caccgtcgat gtgagaccaa cgtggctccc aactgtggag tggatgccag aggaatcgcc       900
   aaagtactgg ccgacctggg cgttacccca gacaaaatca ccaacagcgg ccagagaagg       960
   aaaaagctca ttgctggtgc cgagtccccg cagcctgctt ctggaagctc accatctgag      1020
   gaagatcgat ccaagtcagc acccacctcc ccttgtgacc aggaaataaa agaacttgag      1080
   aacaacattc ggaaagcctt gtcatttgac aaccgaggag aggagcaccg ggcagcatcg      1140
   tctcctgatg gccagctgat gagccccggt gagaatggcg aagtccggca aggccaggcc      1200
   aagcgcctgg gcctggatga gttcaacttc atcaaggtgt tgggcaaagg cagctttggc      1260
   aaggtcatgt tggcagaact caagggcaaa gatgaagtat atgctgtgaa ggtcttaaag      1320
   aaggacgtca tccttcagga tgatgacgtg gactgcacaa tgacagagaa gaggattttg      1380
   gctctggcac ggaaacaccc gtaccttacc caactctact gctgcttcca gaccaaggac      1440
   cgcctctttt tcgtcatgga atatgtaaat ggtggagacc tcatgtttca gattcagcgc      1500
   tcccgaaaat tcgacgagcc tcgttcacgg ttctatgctg cagaggtcac atcggccctc        1560
   atgttcctcc accagcatgg agtcatctac agggatttga aactggacaa catccttctg        1620
   gatgcagaag gtcactgcaa gctggctgac ttcgggatgt gcaaggaagg gattctgaat        1680
   ggtgtgacga ccaccacgtt ctgtgggact cctgactaca tagctcctga gatcctgcag        1740
   gagttggagt atggcccctc cgtggactgg tgggccctgg gggtgctgat gtacgagatg        1800
   atggctggac agcctccctt tgaggccgac aatgaggacg acctatttga gtccatcctc        1860
   catgacgacg tgctgtaccc agtctggctc agcaaggagg ctgtcagcat cttgaaagct        1920
   ttcatgacga agaatcccca caagcgcctg ggctgtgtgg catcgcagaa tggcgaggac        1980
   gccatcaagc agcacccatt cttcaaagag attgactggg tgctcctgga gcagaagaag        2040
   atcaagccac ccttcaaacc acgcattaaa accaaaagag acgtcaataa ttttgaccaa        2100
   gactttaccc gggaagagcc ggtactcacc cttgtggacg aagcaattgt aaagcagatc        2160
   aaccaggagg aattcaaagg tttctcctac tttggtgaag acctgatgcc ctga              2214
<210> 72
   <211> 1866
   <212> DNA
   <213> Artificial Sequence
<220>
   <223> PRKCE gene fragment
<400> 72
   attgatctgg agccagaagg aagagtgtat gtgatcatcg atctctcagg gtcgtcgggt          60
   gaagccccta aagacaatga agagcgtgtg ttcagggaac gcatgcggcc gaggaagcgg         120
   cagggggccg tcaggcgcag ggtccatcag gtcaacggcc acaagttcat ggccacctat        180
   cttcggcagc ccacctactg ctcccattgc agagacttca tctggggtgt cataggaaag         240
   cagggatacc agtgtcaagt ctgcacctgc gtggtccaca agcggtgcca cgagctcata         300
   atcacaaagt gtgctgggtt aaagaagcag gagacccccg accaggtggg ctcccagcgg         360
   ttcagcgtca acatgcccca caagttcggt atccacaact acaaggtccc taccttctgc         420
   gatcactgtg ggtccctgct ctggggactc ttgcggcagg gtttgcagtg taaagtctgc         480
   aaaatgaatg ttcaccgtcg atgtgagacc aacgtggctc ccaactgtgg agtggatgcc         540
   agaggaatcg ccaaagtact ggccgacctg ggcgttaccc cagacaaaat caccaacagc         600
   ggccagagaa ggaaaaagct cattgctggt gccgagtccc cgcagcctgc ttctggaagc         660
   tcaccatctg aggaagatcg atccaagtca gcacccacct ccccttgtga ccaggaaata         720
   aaagaacttg agaacaacat tcggaaagcc ttgtcatttg acaaccgagg agaggagcac         780
   cgggcagcat cgtctcctga tggccagctg atgagccccg gtgagaatgg cgaagtccgg         840
   caaggccagg ccaagcgcct gggcctggat gagttcaact tcatcaaggt gttgggcaaa        900
   ggcagctttg gcaaggtcat gttggcagaa ctcaagggca aagatgaagt atatgctgtg        960
   aaggtcttaa agaaggacgt catccttcag gatgatgacg tggactgcac aatgacagag       1020
   aagaggattt tggctctggc acggaaacac ccgtacctta cccaactcta ctgctgcttc       1080
   cagaccaagg accgcctctt tttcgtcatg gaatatgtaa atggtggaga cctcatgttt       1140
   cagattcagc gctcccgaaa attcgacgag cctcgttcac ggttctatgc tgcagaggtc       1200
   acatcggccc tcatgttcct ccaccagcat ggagtcatct acagggattt gaaactggac       1260
   aacatccttc tggatgcaga aggtcactgc aagctggctg acttcgggat gtgcaaggaa       1320
   gggattctga atggtgtgac gaccaccacg ttctgtggga ctcctgacta catagctcct       1380
   gagatcctgc aggagttgga gtatggcccc tccgtggact ggtgggccct gggggtgctg       1440
   atgtacgaga tgatggctgg acagcctccc tttgaggccg acaatgagga cgacctattt       1500
   gagtccatcc tccatgacga cgtgctgtac ccagtctggc tcagcaagga ggctgtcagc       1560
   atcttgaaag ctttcatgac gaagaatccc cacaagcgcc tgggctgtgt ggcatcgcag       1620
   aatggcgagg acgccatcaa gcagcaccca ttcttcaaag agattgactg ggtgctcctg       1680
   gagcagaaga agatcaagcc acccttcaaa ccacgcatta aaaccaaaag agacgtcaat       1740
   aattttgacc aagactttac ccgggaagag ccggtactca cccttgtgga cgaagcaatt       1800
   gtaaagcaga tcaaccagga ggaattcaaa ggtttctcct actttggtga agacctgatg       1860
   ccctga                                                                  1866
<210> 73
   <211> 24
   <212> PRT
   <213> Artificial Sequence
<220>
   <223> Break-point of PRKCE gene fragment
<400> 73
   Ala Thr Thr Gly Ala Thr Cys Thr Gly Gly Ala Gly Cys Cys Ala Gly
   1               5                   10                  15
   Ala Ala Gly Gly Ala Ala Gly Ala
               20
<210> 74
   <211> 737
   <212> PRT
   <213> Artificial Sequence
<220>
   <223> PRKCE protein
<400> 74
<210> 75
   <211> 621
   <212> PRT
   <213> Artificial Sequence
<220>
   <223> PRKCE protein fragment
<400> 75
   Ile Asp Leu Glu Pro Glu Gly Arg Val Tyr Val Ile Ile Asp Leu Ser
   1               5                   10                  15
   Gly Ser Ser Gly Glu Ala Pro Lys Asp Asn Glu Glu Arg Val Phe Arg
               20                  25                  30
   Glu Arg Met Arg Pro Arg Lys Arg Gln Gly Ala Val Arg Arg Arg Val
           35                  40                  45
   His Gln Val Asn Gly His Lys Phe Met Ala Thr Tyr Leu Arg Gln Pro
       50                  55                  60
   Thr Tyr Cys Ser His Cys Arg Asp Phe Ile Trp Gly Val Ile Gly Lys
   65                  70                  75                  80
   Gln Gly Tyr Gln Cys Gln Val Cys Thr Cys Val Val His Lys Arg Cys
                   85                  90                  95
   His Glu Leu Ile Ile Thr Lys Cys Ala Gly Leu Lys Lys Gln Glu Thr
               100                 105                 110
   Pro Asp Gln Val Gly Ser Gln Arg Phe Ser Val Asn Met Pro His Lys
           115                 120                 125
   Phe Gly Ile His Asn Tyr Lys Val Pro Thr Phe Cys Asp His Cys Gly
       130                 135                 140
   Ser Leu Leu Trp Gly Leu Leu Arg Gln Gly Leu Gln Cys Lys Val Cys
   145                 150                 155                 160
   Lys Met Asn Val His Arg Arg Cys Glu Thr Asn Val Ala Pro Asn Cys
                   165                 170                 175
   Gly Val Asp Ala Arg Gly Ile Ala Lys Val Leu Ala Asp Leu Gly Val
               180                 185                 190
   Thr Pro Asp Lys Ile Thr Asn Ser Gly Gln Arg Arg Lys Lys Leu Ile
           195                 200                 205
   Ala Gly Ala Glu Ser Pro Gln Pro Ala Ser Gly Ser Ser Pro Ser Glu
       210                 215                 220
   Glu Asp Arg Ser Lys Ser Ala Pro Thr Ser Pro Cys Asp Gln Glu Ile
   225                 230                 235                 240
   Lys Glu Leu Glu Asn Asn Ile Arg Lys Ala Leu Ser Phe Asp Asn Arg
                   245                 250                 255
   Gly Glu Glu His Arg Ala Ala Ser Ser Pro Asp Gly Gln Leu Met Ser
               260                 265                 270
   Pro Gly Glu Asn Gly Glu Val Arg Gln Gly Gln Ala Lys Arg Leu Gly
           275                 280                 285
   Leu Asp Glu Phe Asn Phe Ile Lys Val Leu Gly Lys Gly Ser Phe Gly
       290                 295                 300
   Lys Val Met Leu Ala Glu Leu Lys Gly Lys Asp Glu Val Tyr Ala Val
   305                 310                 315                 320
   Lys Val Leu Lys Lys Asp Val Ile Leu Gln Asp Asp Asp Val Asp Cys
                   325                 330                 335
   Thr Met Thr Glu Lys Arg Ile Leu Ala Leu Ala Arg Lys His Pro Tyr
               340                 345                 350
   Leu Thr Gln Leu Tyr Cys Cys Phe Gln Thr Lys Asp Arg Leu Phe Phe
           355                 360                 365
   Val Met Glu Tyr Val Asn Gly Gly Asp Leu Met Phe Gln Ile Gln Arg
       370                 375                 380
   Ser Arg Lys Phe Asp Glu Pro Arg Ser Arg Phe Tyr Ala Ala Glu Val
   385                 390                 395                 400
   Thr Ser Ala Leu Met Phe Leu His Gln His Gly Val Ile Tyr Arg Asp
                   405                 410                 415
   Leu Lys Leu Asp Asn Ile Leu Leu Asp Ala Glu Gly His Cys Lys Leu
               420                 425                 430
   Ala Asp Phe Gly Met Cys Lys Glu Gly Ile Leu Asn Gly Val Thr Thr
           435                 440                 445
   Thr Thr Phe Cys Gly Thr Pro Asp Tyr Ile Ala Pro Glu Ile Leu Gln
       450                 455                 460
   Glu Leu Glu Tyr Gly Pro Ser Val Asp Trp Trp Ala Leu Gly Val Leu
   465                 470                 475                 480
   Met Tyr Glu Met Met Ala Gly Gln Pro Pro Phe Glu Ala Asp Asn Glu
                   485                 490                 495
   Asp Asp Leu Phe Glu Ser Ile Leu His Asp Asp Val Leu Tyr Pro Val
               500                 505                 510
   Trp Leu Ser Lys Glu Ala Val Ser Ile Leu Lys Ala Phe Met Thr Lys
           515                 520                 525
   Asn Pro His Lys Arg Leu Gly Cys Val Ala Ser Gln Asn Gly Glu Asp
   530 535 540
   Ala Ile Lys Gln His Pro Phe Phe Lys Glu Ile Asp Trp Val Leu Leu
   545 550 555 560
   Glu Gln Lys Lys Ile Lys Pro Pro Phe Lys Pro Arg Ile Lys Thr Lys
   565 570 575
   Arg Asp Val Asn Asn Phe Asp Gln Asp Phe Thr Arg Glu Glu Pro Val
   580 585 590
   Leu Thr Leu Val Asp Glu Ala Ile Val Lys Gln Ile Asn Gln Glu Glu
   595 600 605
   Phe Lys Gly Phe Ser Tyr Phe Gly Glu Asp Leu Met Pro
   610 615 620
<210> 76
   <211> 8
   <212> PRT
   <213> Artificial Sequence
<220>
   <223> Break-point of PRKCE protein fragment
<400> 76
   Ile Asp Leu Glu Pro Glu Gly Arg
   1 5
<210> 77
   <211> 1962
   <212> DNA
   <213> Artificial Sequence
<220>
   <223> MAP4K3-PRKCE fusion gene
<400> 77
   atgaaccccg gcttcgattt gtcccgccgg aacccgcagg aggacttcga gctgattcag 60
   cgcatcggca gcggcaccta cggcgacgtc tacaagattg atctggagcc agaaggaaga 120
   gtgtatgtga tcatcgatct ctcagggtcg tcgggtgaag cccctaaaga caatgaagag 180
   cgtgtgttca gggaacgcat gcggccgagg aagcggcagg gggccgtcag gcgcagggtc 240
   catcaggtca acggccacaa gttcatggcc acctatcttc ggcagcccac ctactgctcc 300
   cattgcagag acttcatctg gggtgtcata ggaaagcagg gataccagtg tcaagtctgc 360
   acctgcgtgg tccacaagcg gtgccacgag ctcataatca caaagtgtgc tgggttaaag 420
   aagcaggaga cccccgacca ggtgggctcc cagcggttca gcgtcaacat gccccacaag 480
   ttcggtatcc acaactacaa ggtccctacc ttctgcgatc actgtgggtc cctgctctgg 540
   ggactcttgc ggcagggttt gcagtgtaaa gtctgcaaaa tgaatgttca ccgtcgatgt 600
   gagaccaacg tggctcccaa ctgtggagtg gatgccagag gaatcgccaa agtactggcc 660
   gacctgggcg ttaccccaga caaaatcacc aacagcggcc agagaaggaa aaagctcatt 720
   gctggtgccg agtccccgca gcctgcttct ggaagctcac catctgagga agatcgatcc 780
   aagtcagcac ccacctcccc ttgtgaccag gaaataaaag aacttgagaa caacattcgg 840
   aaagccttgt catttgacaa ccgaggagag gagcaccggg cagcatcgtc tcctgatggc 900
   cagctgatga gccccggtga gaatggcgaa gtccggcaag gccaggccaa gcgcctgggc 960
   ctggatgagt tcaacttcat caaggtgttg ggcaaaggca gctttggcaa ggtcatgttg 1020
   gcagaactca agggcaaaga tgaagtatat gctgtgaagg tcttaaagaa ggacgtcatc 1080
   cttcaggatg atgacgtgga ctgcacaatg acagagaaga ggattttggc tctggcacgg 1140
   aaacacccgt accttaccca actctactgc tgcttccaga ccaaggaccg cctctttttc 1200
   gtcatggaat atgtaaatgg tggagacctc atgtttcaga ttcagcgctc ccgaaaattc 1260
   gacgagcctc gttcacggtt ctatgctgca gaggtcacat cggccctcat gttcctccac 1320
   cagcatggag tcatctacag ggatttgaaa ctggacaaca tccttctgga tgcagaaggt 1380
   cactgcaagc tggctgactt cgggatgtgc aaggaaggga ttctgaatgg tgtgacgacc 1440
   accacgttct gtgggactcc tgactacata gctcctgaga tcctgcagga gttggagtat 1500
   ggcccctccg tggactggtg ggccctgggg gtgctgatgt acgagatgat ggctggacag 1560
   cctccctttg aggccgacaa tgaggacgac ctatttgagt ccatcctcca tgacgacgtg 1620
   ctgtacccag tctggctcag caaggaggct gtcagcatct tgaaagcttt catgacgaag 1680
   aatccccaca agcgcctggg ctgtgtggca tcgcagaatg gcgaggacgc catcaagcag 1740
   cacccattct tcaaagagat tgactgggtg ctcctggagc agaagaagat caagccaccc 1800
   ttcaaaccac gcattaaaac caaaagagac gtcaataatt ttgaccaaga ctttacccgg 1860
   gaagagccgg tactcaccct tgtggacgaa gcaattgtaa agcagatcaa ccaggaggaa 1920
   ttcaaaggtt tctcctactt tggtgaagac ctgatgccct ga 1962
<210> 78
   <211> 45
   <212> DNA
   <213> Artificial Sequence
<220>
   <223> Fused region of MAP4K3-PRKCE fusion gene
<400> 78
   acctacggcg acgtctacaa gattgatctg gagccagaag gaaga 45
<210> 79
   <211> 653
   <212> PRT
   <213> Artificial Sequence
<220>
   <223> MAP4K3-PRKCE fusion protein
<400> 79
   Met Asn Pro Gly Phe Asp Leu Ser Arg Arg Asn Pro Gln Glu Asp Phe
   1               5                   10                  15
   Glu Leu Ile Gln Arg Ile Gly Ser Gly Thr Tyr Gly Asp Val Tyr Lys
               20                  25                  30
   Ile Asp Leu Glu Pro Glu Gly Arg Val Tyr Val Ile Ile Asp Leu Ser
           35                  40                  45
   Gly Ser Ser Gly Glu Ala Pro Lys Asp Asn Glu Glu Arg Val Phe Arg
       50                  55                  60
   Glu Arg Met Arg Pro Arg Lys Arg Gln Gly Ala Val Arg Arg Arg Val
   65                  70                  75                  80
   His Gln Val Asn Gly His Lys Phe Met Ala Thr Tyr Leu Arg Gln Pro
                   85                  90                  95
   Thr Tyr Cys Ser His Cys Arg Asp Phe Ile Trp Gly Val Ile Gly Lys
               100                 105                 110
   Gln Gly Tyr Gln Cys Gln Val Cys Thr Cys Val Val His Lys Arg Cys
           115                 120                 125
   His Glu Leu Ile Ile Thr Lys Cys Ala Gly Leu Lys Lys Gln Glu Thr
       130                 135                 140
   Pro Asp Gln Val Gly Ser Gln Arg Phe Ser Val Asn Met Pro His Lys
   145                 150                 155                 160
   Phe Gly Ile His Asn Tyr Lys Val Pro Thr Phe Cys Asp His Cys Gly
                   165                 170                 175
   Ser Leu Leu Trp Gly Leu Leu Arg Gln Gly Leu Gln Cys Lys Val Cys
               180                 185                 190
   Lys Met Asn Val His Arg Arg Cys Glu Thr Asn Val Ala Pro Asn Cys
           195                 200                 205
   Gly Val Asp Ala Arg Gly Ile Ala Lys Val Leu Ala Asp Leu Gly Val
       210                 215                 220
   Thr Pro Asp Lys Ile Thr Asn Ser Gly Gln Arg Arg Lys Lys Leu Ile
   225                 230                 235                 240
   Ala Gly Ala Glu Ser Pro Gln Pro Ala Ser Gly Ser Ser Pro Ser Glu
                   245                 250                 255
   Glu Asp Arg Ser Lys Ser Ala Pro Thr Ser Pro Cys Asp Gln Glu Ile
               260                 265                 270
   Lys Glu Leu Glu Asn Asn Ile Arg Lys Ala Leu Ser Phe Asp Asn Arg
           275                 280                 285
   Gly Glu Glu His Arg Ala Ala Ser Ser Pro Asp Gly Gln Leu Met Ser
       290                 295                 300
   Pro Gly Glu Asn Gly Glu Val Arg Gln Gly Gln Ala Lys Arg Leu Gly
   305                 310                 315                 320
   Leu Asp Glu Phe Asn Phe Ile Lys Val Leu Gly Lys Gly Ser Phe Gly
                   325                 330                 335
   Lys Val Met Leu Ala Glu Leu Lys Gly Lys Asp Glu Val Tyr Ala Val
               340                 345                 350
   Lys Val Leu Lys Lys Asp Val Ile Leu Gln Asp Asp Asp Val Asp Cys
           355                 360                 365
   Thr Met Thr Glu Lys Arg Ile Leu Ala Leu Ala Arg Lys His Pro Tyr
       370                 375                 380
   Leu Thr Gln Leu Tyr Cys Cys Phe Gln Thr Lys Asp Arg Leu Phe Phe
   385                 390                 395                 400
   Val Met Glu Tyr Val Asn Gly Gly Asp Leu Met Phe Gln Ile Gln Arg
                   405                 410                 415
   Ser Arg Lys Phe Asp Glu Pro Arg Ser Arg Phe Tyr Ala Ala Glu Val
               420                 425                 430
   Thr Ser Ala Leu Met Phe Leu His Gln His Gly Val Ile Tyr Arg Asp
           435                 440                 445
   Leu Lys Leu Asp Asn Ile Leu Leu Asp Ala Glu Gly His Cys Lys Leu
       450                 455                 460
   Ala Asp Phe Gly Met Cys Lys Glu Gly Ile Leu Asn Gly Val Thr Thr
   465                 470                 475                 480
   Thr Thr Phe Cys Gly Thr Pro Asp Tyr Ile Ala Pro Glu Ile Leu Gln
                   485                 490                 495
   Glu Leu Glu Tyr Gly Pro Ser Val Asp Trp Trp Ala Leu Gly Val Leu
               500                 505                 510
   Met Tyr Glu Met Met Ala Gly Gln Pro Pro Phe Glu Ala Asp Asn Glu
           515                 520                 525
   Asp Asp Leu Phe Glu Ser Ile Leu His Asp Asp Val Leu Tyr Pro Val
       530                 535                 540
   Trp Leu Ser Lys Glu Ala Val Ser Ile Leu Lys Ala Phe Met Thr Lys
   545                 550                 555                 560
   Asn Pro His Lys Arg Leu Gly Cys Val Ala Ser Gln Asn Gly Glu Asp
                   565                 570                 575
   Ala Ile Lys Gln His Pro Phe Phe Lys Glu Ile Asp Trp Val Leu Leu
               580                 585                 590
   Glu Gln Lys Lys Ile Lys Pro Pro Phe Lys Pro Arg Ile Lys Thr Lys
           595                 600                 605
   Arg Asp Val Asn Asn Phe Asp Gln Asp Phe Thr Arg Glu Glu Pro Val
       610                 615                 620
   Leu Thr Leu Val Asp Glu Ala Ile Val Lys Gln Ile Asn Gln Glu Glu
   625 630 635 640
   Phe Lys Gly Phe Ser Tyr Phe Gly Glu Asp Leu Met Pro
   645 650
<210> 80
   <211> 15
   <212> PRT
   <213> Artificial Sequence
<220>
   <223> Fused region of MAP4K3-PRKCE fusion protein
<400> 80
   Thr Tyr Gly Asp Val Tyr Lys Ile Asp Leu Glu Pro Glu Gly Arg
   1 5 10 15
<210> 81
   <211> 2788
   <212> DNA
   <213> Artificial Sequence
<220>
   <223> CDS of BCAS3 gene (NM_001099432)
<400> 81
   atgaatgaag ctatggctac agattcccca agaagaccca gtcgttgtac tggtggagtt      60
   gtggttcgcc cccaggctgt cacagagcag tcctacatgg aaagtgttgt gacttttctg     120
   caggatgttg tgccacaggc ttacagtgga acacctctaa cagaagaaaa ggagaaaata     180
   gtctgggtca gatttgaaaa tgcagattta aatgatacat caagaaatct ggaatttcat     240
   gaaatacata gtactgggaa tgaaccgcct ttgttgatta tgattggcta cagtgatgga     300
   atgcaggtct ggagcatccc tatcagtggc gaagcacaag agctcttctc tgttcgacat     360
   ggcccaattc gagcggctag aatcttgcct gctccacagt ttggtgctca aaaatgtgat     420
   aactttgctg aaaaaagacc cctccttggt gtttgtaaga gcattggatc ttctggcaca     480
   agcccaccgt actgttgtgt ggatctgtat tcacttcgta ctggggagat ggtcaagtcc     540
   attcaattta agacacctat ttatgatctc cattgcaata aacggatcct tgtcgtagtc     600
   ttgcaggaga aaattgctgc ctttgatagc tgtactttca cgaagaaatt ctttgttaca     660
   agctgctatc catgtccagg gccaaacatg aatcctattg ctcttgggag ccgctggctt     720
   gcttatgcag aaaacaagtt gattcgatgt catcagtccc gtggtggagc ctgtggagac     780
   aacattcagt cttatactgc cacagtcatt agtgctgcta aaacattgaa aagtggcctg     840
   acaatggtag ggaaagtggt gactcagctg acaggcacac tgccttcagg tgtgacagaa     900
   gatgatgttg ccatccacag taattcacgg cggagtcctt tggtcccagg catcatcaca     960
   gttattgaca ccgaaaccgt tggagagggc caggtgcttg tgagtgagga ttctgacagt 1020
   gatggcattg tggcccactt ccctgcccat gagaagccag tgtgctgcat ggcttttaat 1080
   acaagtggaa tgcttctagt cacaacagac acccttggcc atgactttca tgtcttccaa 1140
   attctgactc atccttggtc ctcatcacaa tgtgctgtcc accatctgta tactcttcac 1200
   aggggagaaa ctgaagccaa agtacaggac atctgcttca gccatgactg tcgctgggtt 1260
   gtggtcagta ctctccgggg tacttcccac gttttcccca tcaaccctta tggtggccag 1320
   ccttgtgttc gtacacatat gtcaccacga gtagtgaatc gcatgagccg tttccagaaa 1380
   agtgctggac tggaagagat tgaacaagaa ctgacgtcta agcaaggagg tcgctgtagc 1440
   cctgttccag gtctatcaag cagcccttct gggtcaccct tgcatgggaa actgaacagc 1500
   caagactcct ataacaattt taccaacaac aaccctggca accctcggct ctctcctctt 1560
   cccagcttga tggtagtgat gcctcttgca caaatcaagc agccaatgac attggggacc 1620
   atcaccaaac gaaccgggcc ttatctcttt ggagcggggt gtttttccat aaaagcccca 1680
   tgcaaagtta aacctcctcc acaaatttca cccagcaaat cgatgggcgg agaattttgt 1740
   gtggctgcta tcttcggaac atccaggtca tggtttgcaa ataatgcagg tctgaaaaga 1800
   gaaaaagatc agtccaaaca agttgtagtt gagtccctgt acattatcag ttgctatggc 1860
   accttagtgg aacacatgat ggagccgcga cccctcagca ctgcacccaa gattagtgac 1920
   gacacaccac tggaaatgat gacatcgcct cgagccagct ggactctggt tagaacccct 1980
   caatggaatg aattgcagcc accgtttaat gcaaaccacc ctctgctcct cgctgcagat 2040
   gcagtacagt attatcagtt cctgcttgct ggcctggttc cccctggaag tcctgggccc 2100
   attactcgac atgggtctta cgacagttta gcttctgacc atagtggaca ggaagatgaa 2160
   gaatggcttt cccaggttga aattgtaaca cacactggac cccatagacg tctgtggatg 2220
   ggtccacagt tccagttcaa aaccatccat ccctcaggcc aaaccacagt tatctcatcc 2280
   agttcatctg tgttgcagtc tcatggtccg agtgacacgc cacagcctct tttggatttt 2340
   gatacagatg atcttgatct caacagtctc aggatccagc cagtccgctc tgaccccgtc 2400
   agcatgccag ggtcatcccg tccagtctct gatcgaaggg gagtttccac agtgattgat 2460
   gctgcctcag ggtacctttg acaggagcgt gaccctgctg gaggtgtgcg ggagctggcc 2520
   tgagggcttc gggctgcggc acatgtcctc catggagcac acggaggagg gcctccggga 2580
   gcgacttgcc gacgccatgg ccgagtcacc tagccgggac gtcgtgggat ccggaacaga 2640
   acttcagcga gagggaagca tcgagactct gagtaacagc tcaggctcca ccagcggcag 2700
   cataccaaga aactttgatg gctaccgatc tccgctgccc accaatgaga gccagcccct 2760
   cagcctcttc ccgactggct tcccgtag 2788
<210> 82
   <211> 2470
   <212> DNA
   <213> Artificial Sequence
<220>
   <223> BCAS3 gene fragment
<400> 82
   atgaatgaag ctatggctac agattcccca agaagaccca gtcgttgtac tggtggagtt      60
   gtggttcgcc cccaggctgt cacagagcag tcctacatgg aaagtgttgt gacttttctg     120
   caggatgttg tgccacaggc ttacagtgga acacctctaa cagaagaaaa ggagaaaata     180
   gtctgggtca gatttgaaaa tgcagattta aatgatacat caagaaatct ggaatttcat     240
   gaaatacata gtactgggaa tgaaccgcct ttgttgatta tgattggcta cagtgatgga     300
   atgcaggtct ggagcatccc tatcagtggc gaagcacaag agctcttctc tgttcgacat     360
   ggcccaattc gagcggctag aatcttgcct gctccacagt ttggtgctca aaaatgtgat     420
   aactttgctg aaaaaagacc cctccttggt gtttgtaaga gcattggatc ttctggcaca     480
   agcccaccgt actgttgtgt ggatctgtat tcacttcgta ctggggagat ggtcaagtcc     540
   attcaattta agacacctat ttatgatctc cattgcaata aacggatcct tgtcgtagtc     600
   ttgcaggaga aaattgctgc ctttgatagc tgtactttca cgaagaaatt ctttgttaca     660
   agctgctatc catgtccagg gccaaacatg aatcctattg ctcttgggag ccgctggctt     720
   gcttatgcag aaaacaagtt gattcgatgt catcagtccc gtggtggagc ctgtggagac     780
   aacattcagt cttatactgc cacagtcatt agtgctgcta aaacattgaa aagtggcctg     840
   acaatggtag ggaaagtggt gactcagctg acaggcacac tgccttcagg tgtgacagaa     900
   gatgatgttg ccatccacag taattcacgg cggagtcctt tggtcccagg catcatcaca     960
   gttattgaca ccgaaaccgt tggagagggc caggtgcttg tgagtgagga ttctgacagt    1020
   gatggcattg tggcccactt ccctgcccat gagaagccag tgtgctgcat ggcttttaat    1080
   acaagtggaa tgcttctagt cacaacagac acccttggcc atgactttca tgtcttccaa    1140
   attctgactc atccttggtc ctcatcacaa tgtgctgtcc accatctgta tactcttcac    1200
   aggggagaaa ctgaagccaa agtacaggac atctgcttca gccatgactg tcgctgggtt    1260
   gtggtcagta ctctccgggg tacttcccac gttttcccca tcaaccctta tggtggccag    1320
   ccttgtgttc gtacacatat gtcaccacga gtagtgaatc gcatgagccg tttccagaaa    1380
   agtgctggac tggaagagat tgaacaagaa ctgacgtcta agcaaggagg tcgctgtagc    1440
   cctgttccag gtctatcaag cagcccttct gggtcaccct tgcatgggaa actgaacagc    1500
   caagactcct ataacaattt taccaacaac aaccctggca accctcggct ctctcctctt    1560
   cccagcttga tggtagtgat gcctcttgca caaatcaagc agccaatgac attggggacc 1620
   atcaccaaac gaaccgggcc ttatctcttt ggagcggggt gtttttccat aaaagcccca 1680
   tgcaaagtta aacctcctcc acaaatttca cccagcaaat cgatgggcgg agaattttgt 1740
   gtggctgcta tcttcggaac atccaggtca tggtttgcaa ataatgcagg tctgaaaaga 1800
   gaaaaagatc agtccaaaca agttgtagtt gagtccctgt acattatcag ttgctatggc 1860
   accttagtgg aacacatgat ggagccgcga cccctcagca ctgcacccaa gattagtgac 1920
   gacacaccac tggaaatgat gacatcgcct cgagccagct ggactctggt tagaacccct 1980
   caatggaatg aattgcagcc accgtttaat gcaaaccacc ctctgctcct cgctgcagat 2040
   gcagtacagt attatcagtt cctgcttgct ggcctggttc cccctggaag tcctgggccc 2100
   attactcgac atgggtctta cgacagttta gcttctgacc atagtggaca ggaagatgaa 2160
   gaatggcttt cccaggttga aattgtaaca cacactggac cccatagacg tctgtggatg 2220
   ggtccacagt tccagttcaa aaccatccat ccctcaggcc aaaccacagt tatctcatcc 2280
   agttcatctg tgttgcagtc tcatggtccg agtgacacgc cacagcctct tttggatttt 2340
   gatacagatg atcttgatct caacagtctc aggatccagc cagtccgctc tgaccccgtc 2400
   agcatgccag ggtcatcccg tccagtctct gatcgaaggg gagtttccac agtgattgat 2460
   gctgcctcag 2470
<210> 83
   <211> 22
   <212> DNA
   <213> Artificial Sequence
<220>
   <223> Break-point of BCAS3 gene fragment
<400> 83
   acagtgattg atgctgcctc ag 22
<210> 84
   <211> 928
   <212> PRT
   <213> Artificial Sequence
<220>
   <223> BCAS3 protein
<400> 84
   Met Asn Glu Ala Met Ala Thr Asp Ser Pro Arg Arg Pro Ser Arg Cys
    1               5                  10                  15
   Thr Gly Gly Val Val Val Arg Pro Gln Ala Val Thr Glu Gln Ser Tyr
               20                  25                  30
   Met Glu Ser Val Val Thr Phe Leu Gln Asp Val Val Pro Gln Ala Tyr
               35                  40                  45
   Ser Gly Thr Pro Leu Thr Glu Glu Lys Glu Lys Ile Val Trp Val Arg
       50                  55                  60
   Phe Glu Asn Ala Asp Leu Asn Asp Thr Ser Arg Asn Leu Glu Phe His
   65                  70                  75                  80
   Glu Ile His Ser Thr Gly Asn Glu Pro Pro Leu Leu Ile Met Ile Gly
                   85                  90                  95
   Tyr Ser Asp Gly Met Gln Val Trp Ser Ile Pro Ile Ser Gly Glu Ala
               100                 105                 110
   Gln Glu Leu Phe Ser Val Arg His Gly Pro Ile Arg Ala Ala Arg Ile
           115                 120                 125
   Leu Pro Ala Pro Gln Phe Gly Ala Gln Lys Cys Asp Asn Phe Ala Glu
       130                 135                 140
   Lys Arg Pro Leu Leu Gly Val Cys Lys Ser Ile Gly Ser Ser Gly Thr
   145                 150                 155                 160
   Ser Pro Pro Tyr Cys Cys Val Asp Leu Tyr Ser Leu Arg Thr Gly Glu
                   165                 170                 175
   Met Val Lys Ser Ile Gln Phe Lys Thr Pro Ile Tyr Asp Leu His Cys
               180                 185                 190
   Asn Lys Arg Ile Leu Val Val Val Leu Gln Glu Lys Ile Ala Ala Phe
           195                 200                 205
   Asp Ser Cys Thr Phe Thr Lys Lys Phe Phe Val Thr Ser Cys Tyr Pro
       210                 215                 220
   Cys Pro Gly Pro Asn Met Asn Pro Ile Ala Leu Gly Ser Arg Trp Leu
   225                 230                 235                 240
   Ala Tyr Ala Glu Asn Lys Leu Ile Arg Cys His Gln Ser Arg Gly Gly
                   245                 250                 255
   Ala Cys Gly Asp Asn Ile Gln Ser Tyr Thr Ala Thr Val Ile Ser Ala
               260                 265                 270
   Ala Lys Thr Leu Lys Ser Gly Leu Thr Met Val Gly Lys Val Val Thr
           275                 280                 285
   Gln Leu Thr Gly Thr Leu Pro Ser Gly Val Thr Glu Asp Asp Val Ala
       290                 295                 300
   Ile His Ser Asn Ser Arg Arg Ser Pro Leu Val Pro Gly Ile Ile Thr
   305                 310                 315                 320
   Val Ile Asp Thr Glu Thr Val Gly Glu Gly Gln Val Leu Val Ser Glu
                   325                 330                 335
   Asp Ser Asp Ser Asp Gly Ile Val Ala His Phe Pro Ala His Glu Lys
               340                 345                 350
   Pro Val Cys Cys Met Ala Phe Asn Thr Ser Gly Met Leu Leu Val Thr
           355                 360                 365
   Thr Asp Thr Leu Gly His Asp Phe His Val Phe Gln Ile Leu Thr His
       370                 375                 380
   Pro Trp Ser Ser Ser Gln Cys Ala Val His His Leu Tyr Thr Leu His
   385                 390                 395                 400
   Arg Gly Glu Thr Glu Ala Lys Val Gln Asp Ile Cys Phe Ser His Asp
                   405                 410                 415
   Cys Arg Trp Val Val Val Ser Thr Leu Arg Gly Thr Ser His Val Phe
               420                 425                 430
   Pro Ile Asn Pro Tyr Gly Gly Gln Pro Cys Val Arg Thr His Met Ser
           435                 440                 445
   Pro Arg Val Val Asn Arg Met Ser Arg Phe Gln Lys Ser Ala Gly Leu
       450                 455                 460
   Glu Glu Ile Glu Gln Glu Leu Thr Ser Lys Gln Gly Gly Arg Cys Ser
   465                 470                 475                 480
   Pro Val Pro Gly Leu Ser Ser Ser Pro Ser Gly Ser Pro Leu His Gly
                   485                 490                 495
   Lys Leu Asn Ser Gln Asp Ser Tyr Asn Asn Phe Thr Asn Asn Asn Pro
               500                 505                 510
   Gly Asn Pro Arg Leu Ser Pro Leu Pro Ser Leu Met Val Val Met Pro
           515                 520                 525
   Leu Ala Gln Ile Lys Gln Pro Met Thr Leu Gly Thr Ile Thr Lys Arg
       530                 535                 540
   Thr Gly Pro Tyr Leu Phe Gly Ala Gly Cys Phe Ser Ile Lys Ala Pro
   545                 550                 555                 560
   Cys Lys Val Lys Pro Pro Pro Gln Ile Ser Pro Ser Lys Ser Met Gly
                   565                 570                 575
   Gly Glu Phe Cys Val Ala Ala Ile Phe Gly Thr Ser Arg Ser Trp Phe
               580                 585                 590
   Ala Asn Asn Ala Gly Leu Lys Arg Glu Lys Asp Gln Ser Lys Gln Val
           595                 600                 605
   Val Val Glu Ser Leu Tyr Ile Ile Ser Cys Tyr Gly Thr Leu Val Glu
       610                 615                 620
   His Met Met Glu Pro Arg Pro Leu Ser Thr Ala Pro Lys Ile Ser Asp
   625                 630                 635                 640
   Asp Thr Pro Leu Glu Met Met Thr Ser Pro Arg Ala Ser Trp Thr Leu
                   645                 650                 655
   Val Arg Thr Pro Gln Trp Asn Glu Leu Gln Pro Pro Phe Asn Ala Asn
               660                 665                 670
   His Pro Leu Leu Leu Ala Ala Asp Ala Val Gln Tyr Tyr Gln Phe Leu
           675                 680                 685
   Leu Ala Gly Leu Val Pro Pro Gly Ser Pro Gly Pro Ile Thr Arg His
       690                 695                 700
   Gly Ser Tyr Asp Ser Leu Ala Ser Asp His Ser Gly Gln Glu Asp Glu
   705                 710                 715                 720
   Glu Trp Leu Ser Gln Val Glu Ile Val Thr His Thr Gly Pro His Arg
                   725                 730                 735
   Arg Leu Trp Met Gly Pro Gln Phe Gln Phe Lys Thr Ile His Pro Ser
               740                 745                 750
   Gly Gln Thr Thr Val Ile Ser Ser Ser Ser Ser Val Leu Gln Ser His
           755                 760                 765
   Gly Pro Ser Asp Thr Pro Gln Pro Leu Leu Asp Phe Asp Thr Asp Asp
       770                 775                 780
   Leu Asp Leu Asn Ser Leu Arg Ile Gln Pro Val Arg Ser Asp Pro Val
   785                 790                 795                 800
   Ser Met Pro Gly Ser Ser Arg Pro Val Ser Asp Arg Arg Gly Val Ser
                   805                 810                 815
   Thr Val Ile Asp Ala Ala Ser Gly Thr Phe Asp Arg Ser Val Thr Leu
               820                 825                 830
   Leu Glu Val Cys Gly Ser Trp Pro Glu Gly Phe Gly Leu Arg His Met
           835                 840                 845
   Ser Ser Met Glu His Thr Glu Glu Gly Leu Arg Glu Arg Leu Ala Asp
       850                 855                 860
   Ala Met Ala Glu Ser Pro Ser Arg Asp Val Val Gly Ser Gly Thr Glu
   865                 870                 875                 880
   Leu Gln Arg Glu Gly Ser Ile Glu Thr Leu Ser Asn Ser Ser Gly Ser
                   885                 890                 895
   Thr Ser Gly Ser Ile Pro Arg Asn Phe Asp Gly Tyr Arg Ser Pro Leu
               900                 905                 910
   Pro Thr Asn Glu Ser Gln Pro Leu Ser Leu Phe Pro Thr Gly Phe Pro
           915                 920                 925
<210> 85
   <211> 823
   <212> PRT
   <213> Artificial Sequence
<220>
   <223> BCAS3 protein fragment
<400> 85
   Met Asn Glu Ala Met Ala Thr Asp Ser Pro Arg Arg Pro Ser Arg Cys
   1               5                   10                  15
   Thr Gly Gly Val Val Val Arg Pro Gln Ala Val Thr Glu Gln Ser Tyr
               20                  25                  30
   Met Glu Ser Val Val Thr Phe Leu Gln Asp Val Val Pro Gln Ala Tyr
           35                  40                  45
   Ser Gly Thr Pro Leu Thr Glu Glu Lys Glu Lys Ile Val Trp Val Arg
       50                  55                  60
   Phe Glu Asn Ala Asp Leu Asn Asp Thr Ser Arg Asn Leu Glu Phe His
   65                  70                  75                  80
   Glu Ile His Ser Thr Gly Asn Glu Pro Pro Leu Leu Ile Met Ile Gly
                   85                  90                  95
   Tyr Ser Asp Gly Met Gln Val Trp Ser Ile Pro Ile Ser Gly Glu Ala
               100                 105                 110
   Gln Glu Leu Phe Ser Val Arg His Gly Pro Ile Arg Ala Ala Arg Ile
           115                 120                 125
   Leu Pro Ala Pro Gln Phe Gly Ala Gln Lys Cys Asp Asn Phe Ala Glu
       130                 135                 140
   Lys Arg Pro Leu Leu Gly Val Cys Lys Ser Ile Gly Ser Ser Gly Thr
   145                 150                 155                 160
   Ser Pro Pro Tyr Cys Cys Val Asp Leu Tyr Ser Leu Arg Thr Gly Glu
                   165                 170                 175
   Met Val Lys Ser Ile Gln Phe Lys Thr Pro Ile Tyr Asp Leu His Cys
               180                 185                 190
   Asn Lys Arg Ile Leu Val Val Val Leu Gln Glu Lys Ile Ala Ala Phe
           195                 200                 205
   Asp Ser Cys Thr Phe Thr Lys Lys Phe Phe Val Thr Ser Cys Tyr Pro
       210                 215                 220
   Cys Pro Gly Pro Asn Met Asn Pro Ile Ala Leu Gly Ser Arg Trp Leu
   225                 230                 235                 240
   Ala Tyr Ala Glu Asn Lys Leu Ile Arg Cys His Gln Ser Arg Gly Gly
                   245                 250                 255
   Ala Cys Gly Asp Asn Ile Gln Ser Tyr Thr Ala Thr Val Ile Ser Ala
               260                 265                 270
   Ala Lys Thr Leu Lys Ser Gly Leu Thr Met Val Gly Lys Val Val Thr
           275                 280                 285
   Gln Leu Thr Gly Thr Leu Pro Ser Gly Val Thr Glu Asp Asp Val Ala
       290                 295                 300
   Ile His Ser Asn Ser Arg Arg Ser Pro Leu Val Pro Gly Ile Ile Thr
   305                 310                 315                 320
   Val Ile Asp Thr Glu Thr Val Gly Glu Gly Gln Val Leu Val Ser Glu
                   325                 330                 335
   Asp Ser Asp Ser Asp Gly Ile Val Ala His Phe Pro Ala His Glu Lys
               340                 345                 350
   Pro Val Cys Cys Met Ala Phe Asn Thr Ser Gly Met Leu Leu Val Thr
           355                 360                 365
   Thr Asp Thr Leu Gly His Asp Phe His Val Phe Gln Ile Leu Thr His
       370                 375                 380
   Pro Trp Ser Ser Ser Gln Cys Ala Val His His Leu Tyr Thr Leu His
   385 390 395 400
   Arg Gly Glu Thr Glu Ala Lys Val Gln Asp Ile Cys Phe Ser His Asp
   405 410 415
   Cys Arg Trp Val Val Val Ser Thr Leu Arg Gly Thr Ser His Val Phe
   420 425 430
   Pro Ile Asn Pro Tyr Gly Gly Gln Pro Cys Val Arg Thr His Met Ser
   435 440 445
   Pro Arg Val Val Asn Arg Met Ser Arg Phe Gln Lys Ser Ala Gly Leu
   450 455 460
   Glu Glu Ile Glu Gln Glu Leu Thr Ser Lys Gln Gly Gly Arg Cys Ser
   465 470 475 480
   Pro Val Pro Gly Leu Ser Ser Ser Pro Ser Gly Ser Pro Leu His Gly
   485 490 495
   Lys Leu Asn Ser Gln Asp Ser Tyr Asn Asn Phe Thr Asn Asn Asn Pro
   500 505 510
   Gly Asn Pro Arg Leu Ser Pro Leu Pro Ser Leu Met Val Val Met Pro
   515 520 525
   Leu Ala Gln Ile Lys Gln Pro Met Thr Leu Gly Thr Ile Thr Lys Arg
   530 535 540
   Thr Gly Pro Tyr Leu Phe Gly Ala Gly Cys Phe Ser Ile Lys Ala Pro
   545 550 555 560
   Cys Lys Val Lys Pro Pro Pro Gln Ile Ser Pro Ser Lys Ser Met Gly
   565 570 575
   Gly Glu Phe Cys Val Ala Ala Ile Phe Gly Thr Ser Arg Ser Trp Phe
   580 585 590
   Ala Asn Asn Ala Gly Leu Lys Arg Glu Lys Asp Gln Ser Lys Gln Val
   595 600 605
   Val Val Glu Ser Leu Tyr Ile Ile Ser Cys Tyr Gly Thr Leu Val Glu
   610 615 620
   His Met Met Glu Pro Arg Pro Leu Ser Thr Ala Pro Lys Ile Ser Asp
   625 630 635 640
   Asp Thr Pro Leu Glu Met Met Thr Ser Pro Arg Ala Ser Trp Thr Leu
   645 650 655
   Val Arg Thr Pro Gln Trp Asn Glu Leu Gln Pro Pro Phe Asn Ala Asn
   660 665 670
   His Pro Leu Leu Leu Ala Ala Asp Ala Val Gln Tyr Tyr Gln Phe Leu
   675 680 685
   Leu Ala Gly Leu Val Pro Pro Gly Ser Pro Gly Pro Ile Thr Arg His
   690 695 700
   Gly Ser Tyr Asp Ser Leu Ala Ser Asp His Ser Gly Gln Glu Asp Glu
   705 710 715 720
   Glu Trp Leu Ser Gln Val Glu Ile Val Thr His Thr Gly Pro His Arg
                   725                 730                 735
   Arg Leu Trp Met Gly Pro Gln Phe Gln Phe Lys Thr Ile His Pro Ser
               740                 745                 750
   Gly Gln Thr Thr Val Ile Ser Ser Ser Ser Ser Val Leu Gln Ser His
           755                 760                 765
   Gly Pro Ser Asp Thr Pro Gln Pro Leu Leu Asp Phe Asp Thr Asp Asp
       770                 775                 780
   Leu Asp Leu Asn Ser Leu Arg Ile Gln Pro Val Arg Ser Asp Pro Val
   785                 790                 795                 800
   Ser Met Pro Gly Ser Ser Arg Pro Val Ser Asp Arg Arg Gly Val Ser
                   805                 810                 815
   Thr Val Ile Asp Ala Ala Ser
               820
<210> 86
   <211> 7
   <212> PRT
   <213> Artificial Sequence
<220>
   <223> Break-point of BCAS3 protein fragment
<400> 86
   Thr Val Ile Asp Ala Ala Ser
   1               5
<210> 87
   <211> 1883
   <212> DNA
   <213> Artificial Sequence
<220>
   <223> CDS of MAP3K3 gene (NM_002401)
<400> 87
   atggacacga acaggaggca ttgaactcaa tcatgaacga tctggtggcc ctccagatga        60
   accgacgtca ccggatgcct ggatatgaga ccatgaagaa caaagacaca ggtcactcaa       120
   ataggcagag tgacgtcaga atcaagttcg agcacaacgg ggagaggcga attatagcgt       180
   tcagccggcc tgtgaaatat gaagatgtgg agcacaaggt gacaacagta tttggacaac       240
   ctcttgatct acattacatg aacaatgagc tctccatcct gctgaaaaac caagatgatc       300
   ttgataaagc aattgacatt ttagatagaa gctcaagcat gaaaagcctt aggatattgc       360
   tgttgtccca ggacagaaac cataacagtt cctctcccca ctctggggtg tccagacagg       420
   tgcggatcaa ggcttcccag tccgcagggg atataaatac tatctaccag ccccccgagc       480
   ccagaagcag gcacctctct gtcagctccc agaaccctgg ccgaagctca cctccccctg       540
   gctatgttcc tgagcggcag cagcacattg cccggcaggg gtcctacacc agcatcaaca 600
   gtgaggggga gttcatccca gagaccagcg agcagtgcat gctggatccc ctgagcagtg 660
   cagaaaattc cttgtctgga agctgccaat ccttggacag gtcagcagac agcccatcct 720
   tccggaaatc acgaatgtcc cgtgcccaga gcttccctga caacagacag gaatactcag 780
   atcgggaaac tcagctttat gacaaagggg tcaaaggtgg aacctacccc cggcgctacc 840
   acgtgtctgt gcaccacaag gactacagtg atggcagaag aacatttccc cgaatacggc 900
   gtcatcaagg caacttgttc accctggtgc cctccagccg ctccctgagc acaaatggcg 960
   agaacatggg tctggctgtg caatacctgg acccccgtgg gcgcctgcgg agtgcggaca 1020
   gcgagaatgc cctctctgtg caggagagga atgtgccaac caagtctccc agtgccccca 1080
   tcaactggcg ccggggaaag ctcctgggcc agggtgcctt cggcagggtc tatttgtgct 1140
   atgacgtgga cacgggacgt gaacttgctt ccaagcaggt ccaatttgat ccagacagtc 1200
   ctgagacaag caaggaggtg agtgctctgg agtgcgagat ccagttgcta aagaacttgc 1260
   agcatgagcg catcgtgcag tactatggct gtctgcggga ccgcgctgag aagaccctga 1320
   ccatcttcat ggagtacatg ccagggggct cggtgaaaga ccagttgaag gcttacggtg 1380
   ctctgacaga gagcgtgacc cgaaagtaca cgcggcagat cctggagggc atgtcctacc 1440
   tgcacagcaa catgattgtt caccgggaca ttaagggagc caacatcctc cgagactctg 1500
   ctgggaatgt aaagctgggg gactttgggg ccagcaaacg cctgcagacg atctgtatgt 1560
   cggggacggg catgcgctcc gtcactggca caccctactg gatgagccct gaggtgatca 1620
   gcggcgaggg ctatggaagg aaagcagacg tgtggagcct gggctgcact gtggtggaga 1680
   tgctgacaga gaaaccaccg tgggcagagt atgaagctat ggccgccatc ttcaagattg 1740
   ccacccagcc caccaatcct cagctgccct cccacatctc tgaacatggc cgggacttcc 1800
   tgaggcgcat ttttgtggag gctcgccaga gaccttcagc tgaggagctg ctcacacacc 1860
   actttgcaca gctcatgtac tga 1883
<210> 88
   <211> 1877
   <212> DNA
   <213> Artificial Sequence
<220>
   <223> MAP3K3 gene fragment
<400> 88
   acgaacagga ggcattgaac tcaatcatga acgatctggt ggccctccag atgaaccgac        60
   gtcaccggat gcctggatat gagaccatga agaacaaaga cacaggtcac tcaaataggc       120
   agagtgacgt cagaatcaag ttcgagcaca acggggagag gcgaattata gcgttcagcc       180
   ggcctgtgaa atatgaagat gtggagcaca aggtgacaac agtatttgga caacctcttg 240
   atctacatta catgaacaat gagctctcca tcctgctgaa aaaccaagat gatcttgata 300
   aagcaattga cattttagat agaagctcaa gcatgaaaag ccttaggata ttgctgttgt 360
   cccaggacag aaaccataac agttcctctc cccactctgg ggtgtccaga caggtgcgga 420
   tcaaggcttc ccagtccgca ggggatataa atactatcta ccagcccccc gagcccagaa 480
   gcaggcacct ctctgtcagc tcccagaacc ctggccgaag ctcacctccc cctggctatg 540
   ttcctgagcg gcagcagcac attgcccggc aggggtccta caccagcatc aacagtgagg 600
   gggagttcat cccagagacc agcgagcagt gcatgctgga tcccctgagc agtgcagaaa 660
   attccttgtc tggaagctgc caatccttgg acaggtcagc agacagccca tccttccgga 720
   aatcacgaat gtcccgtgcc cagagcttcc ctgacaacag acaggaatac tcagatcggg 780
   aaactcagct ttatgacaaa ggggtcaaag gtggaaccta cccccggcgc taccacgtgt 840
   ctgtgcacca caaggactac agtgatggca gaagaacatt tccccgaata cggcgtcatc 900
   aaggcaactt gttcaccctg gtgccctcca gccgctccct gagcacaaat ggcgagaaca 960
   tgggtctggc tgtgcaatac ctggaccccc gtgggcgcct gcggagtgcg gacagcgaga 1020
   atgccctctc tgtgcaggag aggaatgtgc caaccaagtc tcccagtgcc cccatcaact 1080
   ggcgccgggg aaagctcctg ggccagggtg ccttcggcag ggtctatttg tgctatgacg 1140
   tggacacggg acgtgaactt gcttccaagc aggtccaatt tgatccagac agtcctgaga 1200
   caagcaagga ggtgagtgct ctggagtgcg agatccagtt gctaaagaac ttgcagcatg 1260
   agcgcatcgt gcagtactat ggctgtctgc gggaccgcgc tgagaagacc ctgaccatct 1320
   tcatggagta catgccaggg ggctcggtga aagaccagtt gaaggcttac ggtgctctga 1380
   cagagagcgt gacccgaaag tacacgcggc agatcctgga gggcatgtcc tacctgcaca 1440
   gcaacatgat tgttcaccgg gacattaagg gagccaacat cctccgagac tctgctggga 1500
   atgtaaagct gggggacttt ggggccagca aacgcctgca gacgatctgt atgtcgggga 1560
   cgggcatgcg ctccgtcact ggcacaccct actggatgag ccctgaggtg atcagcggcg 1620
   agggctatgg aaggaaagca gacgtgtgga gcctgggctg cactgtggtg gagatgctga 1680
   cagagaaacc accgtgggca gagtatgaag ctatggccgc catcttcaag attgccaccc 1740
   agcccaccaa tcctcagctg ccctcccaca tctctgaaca tggccgggac ttcctgaggc 1800
   gcatttttgt ggaggctcgc cagagacctt cagctgagga gctgctcaca caccactttg 1860
   cacagctcat gtactga 1877
<210> 89
   <211> 23
   <212> DNA
   <213> Artificial Sequence
<220>
   <223> Break-point of MAP3K3 gene fragment
<400> 89
   acgaacagga ggcattgaac tca 23
<210> 90
   <211> 626
   <212> PRT
   <213> Artificial Sequence
<220>
   <223> MAP3K3 protein
<400> 90
<210> 91
   <211> 624
   <212> PRT
   <213> Artificial Sequence
<220>
   <223> MAP3K3 protein fragment
<400> 91
   Glu Gln Glu Ala Leu Asn Ser Ile Met Asn Asp Leu Val Ala Leu Gln
    1               5                  10                  15
   Met Asn Arg Arg His Arg Met Pro Gly Tyr Glu Thr Met Lys Asn Lys
               20                  25                  30
   Asp Thr Gly His Ser Asn Arg Gln Ser Asp Val Arg Ile Lys Phe Glu
           35                  40                  45
   His Asn Gly Glu Arg Arg Ile Ile Ala Phe Ser Arg Pro Val Lys Tyr
       50                  55                  60
   Glu Asp Val Glu His Lys Val Thr Thr Val Phe Gly Gln Pro Leu Asp
   65                  70                  75                  80
   Leu His Tyr Met Asn Asn Glu Leu Ser Ile Leu Leu Lys Asn Gln Asp
                   85                  90                  95
   Asp Leu Asp Lys Ala Ile Asp Ile Leu Asp Arg Ser Ser Ser Met Lys
               100                 105                 110
   Ser Leu Arg Ile Leu Leu Leu Ser Gln Asp Arg Asn His Asn Ser Ser
           115                 120                 125
   Ser Pro His Ser Gly Val Ser Arg Gln Val Arg Ile Lys Ala Ser Gln
       130                 135                 140
   Ser Ala Gly Asp Ile Asn Thr Ile Tyr Gln Pro Pro Glu Pro Arg Ser
   145                 150                 155                 160
   Arg His Leu Ser Val Ser Ser Gln Asn Pro Gly Arg Ser Ser Pro Pro
                   165                 170                 175
   Pro Gly Tyr Val Pro Glu Arg Gln Gln His Ile Ala Arg Gln Gly Ser
               180                 185                 190
   Tyr Thr Ser Ile Asn Ser Glu Gly Glu Phe Ile Pro Glu Thr Ser Glu
           195                 200                 205
   Gln Cys Met Leu Asp Pro Leu Ser Ser Ala Glu Asn Ser Leu Ser Gly
       210                 215                 220
   Ser Cys Gln Ser Leu Asp Arg Ser Ala Asp Ser Pro Ser Phe Arg Lys
   225                 230                 235                 240
   Ser Arg Met Ser Arg Ala Gln Ser Phe Pro Asp Asn Arg Gln Glu Tyr
                   245                 250                 255
   Ser Asp Arg Glu Thr Gln Leu Tyr Asp Lys Gly Val Lys Gly Gly Thr
               260                 265                 270
   Tyr Pro Arg Arg Tyr His Val Ser Val His His Lys Asp Tyr Ser Asp
           275                 280                 285
   Gly Arg Arg Thr Phe Pro Arg Ile Arg Arg His Gln Gly Asn Leu Phe
       290                 295                 300
   Thr Leu Val Pro Ser Ser Arg Ser Leu Ser Thr Asn Gly Glu Asn Met
   305                 310                 315                 320
   Gly Leu Ala Val Gln Tyr Leu Asp Pro Arg Gly Arg Leu Arg Ser Ala
                   325                 330                 335
   Asp Ser Glu Asn Ala Leu Ser Val Gln Glu Arg Asn Val Pro Thr Lys
               340                 345                 350
   Ser Pro Ser Ala Pro Ile Asn Trp Arg Arg Gly Lys Leu Leu Gly Gln
           355                 360                 365
   Gly Ala Phe Gly Arg Val Tyr Leu Cys Tyr Asp Val Asp Thr Gly Arg
       370                 375                 380
   Glu Leu Ala Ser Lys Gln Val Gln Phe Asp Pro Asp Ser Pro Glu Thr
   385                 390                 395                 400
   Ser Lys Glu Val Ser Ala Leu Glu Cys Glu Ile Gln Leu Leu Lys Asn
                   405                 410                 415
   Leu Gln His Glu Arg Ile Val Gln Tyr Tyr Gly Cys Leu Arg Asp Arg
               420                 425                 430
   Ala Glu Lys Thr Leu Thr Ile Phe Met Glu Tyr Met Pro Gly Gly Ser
           435                 440                 445
   Val Lys Asp Gln Leu Lys Ala Tyr Gly Ala Leu Thr Glu Ser Val Thr
       450                 455                 460
   Arg Lys Tyr Thr Arg Gln Ile Leu Glu Gly Met Ser Tyr Leu His Ser
   465                 470                 475                 480
   Asn Met Ile Val His Arg Asp Ile Lys Gly Ala Asn Ile Leu Arg Asp
                   485                 490                 495
   Ser Ala Gly Asn Val Lys Leu Gly Asp Phe Gly Ala Ser Lys Arg Leu
               500                 505                 510
   Gln Thr Ile Cys Met Ser Gly Thr Gly Met Arg Ser Val Thr Gly Thr
           515                 520                 525
   Pro Tyr Trp Met Ser Pro Glu Val Ile Ser Gly Glu Gly Tyr Gly Arg
       530                 535                 540
   Lys Ala Asp Val Trp Ser Leu Gly Cys Thr Val Val Glu Met Leu Thr
   545 550 555 560
   Glu Lys Pro Pro Trp Ala Glu Tyr Glu Ala Met Ala Ala Ile Phe Lys
   565 570 575
   Ile Ala Thr Gln Pro Thr Asn Pro Gln Leu Pro Ser His Ile Ser Glu
   580 585 590
   His Gly Arg Asp Phe Leu Arg Arg Ile Phe Val Glu Ala Arg Gln Arg
   595 600 605
   Pro Ser Ala Glu Glu Leu Leu Thr His His Phe Ala Gln Leu Met Tyr
   610 615 620
<210> 92
   <211> 7
   <212> PRT
   <213> Artificial Sequence
<220>
   <223> Break-point of MAP3K3 protein fragment
<400> 92
   Glu Gln Glu Ala Leu Asn Ser
   1 5
<210> 93
   <211> 4347
   <212> DNA
   <213> Artificial Sequence
<220>
   <223> BCAS3-MAP3K3 fusion gene
<400> 93
   atgaatgaag ctatggctac agattcccca agaagaccca gtcgttgtac tggtggagtt 60
   gtggttcgcc cccaggctgt cacagagcag tcctacatgg aaagtgttgt gacttttctg 120
   caggatgttg tgccacaggc ttacagtgga acacctctaa cagaagaaaa ggagaaaata 180
   gtctgggtca gatttgaaaa tgcagattta aatgatacat caagaaatct ggaatttcat 240
   gaaatacata gtactgggaa tgaaccgcct ttgttgatta tgattggcta cagtgatgga 300
   atgcaggtct ggagcatccc tatcagtggc gaagcacaag agctcttctc tgttcgacat 360
   ggcccaattc gagcggctag aatcttgcct gctccacagt ttggtgctca aaaatgtgat 420
   aactttgctg aaaaaagacc cctccttggt gtttgtaaga gcattggatc ttctggcaca 480
   agcccaccgt actgttgtgt ggatctgtat tcacttcgta ctggggagat ggtcaagtcc 540
   attcaattta agacacctat ttatgatctc cattgcaata aacggatcct tgtcgtagtc 600
   ttgcaggaga aaattgctgc ctttgatagc tgtactttca cgaagaaatt ctttgttaca 660
   agctgctatc catgtccagg gccaaacatg aatcctattg ctcttgggag ccgctggctt 720
   gcttatgcag aaaacaagtt gattcgatgt catcagtccc gtggtggagc ctgtggagac 780
   aacattcagt cttatactgc cacagtcatt agtgctgcta aaacattgaa aagtggcctg 840
   acaatggtag ggaaagtggt gactcagctg acaggcacac tgccttcagg tgtgacagaa 900
   gatgatgttg ccatccacag taattcacgg cggagtcctt tggtcccagg catcatcaca 960
   gttattgaca ccgaaaccgt tggagagggc caggtgcttg tgagtgagga ttctgacagt 1020
   gatggcattg tggcccactt ccctgcccat gagaagccag tgtgctgcat ggcttttaat 1080
   acaagtggaa tgcttctagt cacaacagac acccttggcc atgactttca tgtcttccaa 1140
   attctgactc atccttggtc ctcatcacaa tgtgctgtcc accatctgta tactcttcac 1200
   aggggagaaa ctgaagccaa agtacaggac atctgcttca gccatgactg tcgctgggtt 1260
   gtggtcagta ctctccgggg tacttcccac gttttcccca tcaaccctta tggtggccag 1320
   ccttgtgttc gtacacatat gtcaccacga gtagtgaatc gcatgagccg tttccagaaa 1380
   agtgctggac tggaagagat tgaacaagaa ctgacgtcta agcaaggagg tcgctgtagc 1440
   cctgttccag gtctatcaag cagcccttct gggtcaccct tgcatgggaa actgaacagc 1500
   caagactcct ataacaattt taccaacaac aaccctggca accctcggct ctctcctctt 1560
   cccagcttga tggtagtgat gcctcttgca caaatcaagc agccaatgac attggggacc 1620
   atcaccaaac gaaccgggcc ttatctcttt ggagcggggt gtttttccat aaaagcccca 1680
   tgcaaagtta aacctcctcc acaaatttca cccagcaaat cgatgggcgg agaattttgt 1740
   gtggctgcta tcttcggaac atccaggtca tggtttgcaa ataatgcagg tctgaaaaga 1800
   gaaaaagatc agtccaaaca agttgtagtt gagtccctgt acattatcag ttgctatggc 1860
   accttagtgg aacacatgat ggagccgcga cccctcagca ctgcacccaa gattagtgac 1920
   gacacaccac tggaaatgat gacatcgcct cgagccagct ggactctggt tagaacccct 1980
   caatggaatg aattgcagcc accgtttaat gcaaaccacc ctctgctcct cgctgcagat 2040
   gcagtacagt attatcagtt cctgcttgct ggcctggttc cccctggaag tcctgggccc 2100
   attactcgac atgggtctta cgacagttta gcttctgacc atagtggaca ggaagatgaa 2160
   gaatggcttt cccaggttga aattgtaaca cacactggac cccatagacg tctgtggatg 2220
   ggtccacagt tccagttcaa aaccatccat ccctcaggcc aaaccacagt tatctcatcc 2280
   agttcatctg tgttgcagtc tcatggtccg agtgacacgc cacagcctct tttggatttt 2340
   gatacagatg atcttgatct caacagtctc aggatccagc cagtccgctc tgaccccgtc 2400
   agcatgccag ggtcatcccg tccagtctct gatcgaaggg gagtttccac agtgattgat 2460
   gctgcctcag acgaacagga ggcattgaac tcaatcatga acgatctggt ggccctccag 2520
   atgaaccgac gtcaccggat gcctggatat gagaccatga agaacaaaga cacaggtcac 2580
   tcaaataggc agagtgacgt cagaatcaag ttcgagcaca acggggagag gcgaattata 2640
   gcgttcagcc ggcctgtgaa atatgaagat gtggagcaca aggtgacaac agtatttgga 2700
   caacctcttg atctacatta catgaacaat gagctctcca tcctgctgaa aaaccaagat 2760
   gatcttgata aagcaattga cattttagat agaagctcaa gcatgaaaag ccttaggata 2820
   ttgctgttgt cccaggacag aaaccataac agttcctctc cccactctgg ggtgtccaga 2880
   caggtgcgga tcaaggcttc ccagtccgca ggggatataa atactatcta ccagcccccc 2940
   gagcccagaa gcaggcacct ctctgtcagc tcccagaacc ctggccgaag ctcacctccc 3000
   cctggctatg ttcctgagcg gcagcagcac attgcccggc aggggtccta caccagcatc 3060
   aacagtgagg gggagttcat cccagagacc agcgagcagt gcatgctgga tcccctgagc 3120
   agtgcagaaa attccttgtc tggaagctgc caatccttgg acaggtcagc agacagccca 3180
   tccttccgga aatcacgaat gtcccgtgcc cagagcttcc ctgacaacag acaggaatac 3240
   tcagatcggg aaactcagct ttatgacaaa ggggtcaaag gtggaaccta cccccggcgc 3300
   taccacgtgt ctgtgcacca caaggactac agtgatggca gaagaacatt tccccgaata 3360
   cggcgtcatc aaggcaactt gttcaccctg gtgccctcca gccgctccct gagcacaaat 3420
   ggcgagaaca tgggtctggc tgtgcaatac ctggaccccc gtgggcgcct gcggagtgcg 3480
   gacagcgaga atgccctctc tgtgcaggag aggaatgtgc caaccaagtc tcccagtgcc 3540
   cccatcaact ggcgccgggg aaagctcctg ggccagggtg ccttcggcag ggtctatttg 3600
   tgctatgacg tggacacggg acgtgaactt gcttccaagc aggtccaatt tgatccagac 3660
   agtcctgaga caagcaagga ggtgagtgct ctggagtgcg agatccagtt gctaaagaac 3720
   ttgcagcatg agcgcatcgt gcagtactat ggctgtctgc gggaccgcgc tgagaagacc 3780
   ctgaccatct tcatggagta catgccaggg ggctcggtga aagaccagtt gaaggcttac 3840
   ggtgctctga cagagagcgt gacccgaaag tacacgcggc agatcctgga gggcatgtcc 3900
   tacctgcaca gcaacatgat tgttcaccgg gacattaagg gagccaacat cctccgagac 3960
   tctgctggga atgtaaagct gggggacttt ggggccagca aacgcctgca gacgatctgt 4020
   atgtcgggga cgggcatgcg ctccgtcact ggcacaccct actggatgag ccctgaggtg 4080
   atcagcggcg agggctatgg aaggaaagca gacgtgtgga gcctgggctg cactgtggtg 4140
   gagatgctga cagagaaacc accgtgggca gagtatgaag ctatggccgc catcttcaag 4200
   attgccaccc agcccaccaa tcctcagctg ccctcccaca tctctgaaca tggccgggac 4260
   ttcctgaggc gcatttttgt ggaggctcgc cagagacctt cagctgagga gctgctcaca 4320
   caccactttg cacagctcat gtactga 4347
<210> 94
   <211> 45
   <212> DNA
   <213> Artificial Sequence
<220>
   <223> Fused region of BCAS3-MAP3K3 fusion gene
<400> 94
   acagtgattg atgctgcctc agacgaacag gaggcattga actca 45
<210> 95
   <211> 1448
   <212> PRT
   <213> Artificial Sequence
<220>
   <223> BCAS3-MAP3K3 fusion protein
<400> 95
   Met Asn Glu Ala Met Ala Thr Asp Ser Pro Arg Arg Pro Ser Arg Cys
   1               5                   10                  15
   Thr Gly Gly Val Val Val Arg Pro Gln Ala Val Thr Glu Gln Ser Tyr
               20                  25                  30
   Met Glu Ser Val Val Thr Phe Leu Gln Asp Val Val Pro Gln Ala Tyr
           35                  40                  45
   Ser Gly Thr Pro Leu Thr Glu Glu Lys Glu Lys Ile Val Trp Val Arg
       50                  55                  60
   Phe Glu Asn Ala Asp Leu Asn Asp Thr Ser Arg Asn Leu Glu Phe His
   65                  70                  75                  80
   Glu Ile His Ser Thr Gly Asn Glu Pro Pro Leu Leu Ile Met Ile Gly
                   85                  90                  95
   Tyr Ser Asp Gly Met Gln Val Trp Ser Ile Pro Ile Ser Gly Glu Ala
               100                 105                 110
   Gln Glu Leu Phe Ser Val Arg His Gly Pro Ile Arg Ala Ala Arg Ile
           115                 120                 125
   Leu Pro Ala Pro Gln Phe Gly Ala Gln Lys Cys Asp Asn Phe Ala Glu
       130                 135                 140
   Lys Arg Pro Leu Leu Gly Val Cys Lys Ser Ile Gly Ser Ser Gly Thr
   145                 150                 155                 160
   Ser Pro Pro Tyr Cys Cys Val Asp Leu Tyr Ser Leu Arg Thr Gly Glu
                   165                 170                 175
   Met Val Lys Ser Ile Gln Phe Lys Thr Pro Ile Tyr Asp Leu His Cys
               180                 185                 190
   Asn Lys Arg Ile Leu Val Val Val Leu Gln Glu Lys Ile Ala Ala Phe
           195                 200                 205
   Asp Ser Cys Thr Phe Thr Lys Lys Phe Phe Val Thr Ser Cys Tyr Pro
       210                 215                 220
   Cys Pro Gly Pro Asn Met Asn Pro Ile Ala Leu Gly Ser Arg Trp Leu
   225                 230                 235                 240
   Ala Tyr Ala Glu Asn Lys Leu Ile Arg Cys His Gln Ser Arg Gly Gly
                   245                 250                 255
   Ala Cys Gly Asp Asn Ile Gln Ser Tyr Thr Ala Thr Val Ile Ser Ala
               260                 265                 270
   Ala Lys Thr Leu Lys Ser Gly Leu Thr Met Val Gly Lys Val Val Thr
           275                 280                 285
   Gln Leu Thr Gly Thr Leu Pro Ser Gly Val Thr Glu Asp Asp Val Ala
       290                 295                 300
   Ile His Ser Asn Ser Arg Arg Ser Pro Leu Val Pro Gly Ile Ile Thr
   305                 310                 315                 320
   Val Ile Asp Thr Glu Thr Val Gly Glu Gly Gln Val Leu Val Ser Glu
                   325                 330                 335
   Asp Ser Asp Ser Asp Gly Ile Val Ala His Phe Pro Ala His Glu Lys
               340                 345                 350
   Pro Val Cys Cys Met Ala Phe Asn Thr Ser Gly Met Leu Leu Val Thr
           355                 360                 365
   Thr Asp Thr Leu Gly His Asp Phe His Val Phe Gln Ile Leu Thr His
       370                 375                 380
   Pro Trp Ser Ser Ser Gln Cys Ala Val His His Leu Tyr Thr Leu His
   385                 390                 395                 400
   Arg Gly Glu Thr Glu Ala Lys Val Gln Asp Ile Cys Phe Ser His Asp
                   405                 410                 415
   Cys Arg Trp Val Val Val Ser Thr Leu Arg Gly Thr Ser His Val Phe
               420                 425                 430
   Pro Ile Asn Pro Tyr Gly Gly Gln Pro Cys Val Arg Thr His Met Ser
           435                 440                 445
   Pro Arg Val Val Asn Arg Met Ser Arg Phe Gln Lys Ser Ala Gly Leu
       450                 455                 460
   Glu Glu Ile Glu Gln Glu Leu Thr Ser Lys Gln Gly Gly Arg Cys Ser
   465                 470                 475                 480
   Pro Val Pro Gly Leu Ser Ser Ser Pro Ser Gly Ser Pro Leu His Gly
                   485                 490                 495
   Lys Leu Asn Ser Gln Asp Ser Tyr Asn Asn Phe Thr Asn Asn Asn Pro
               500                 505                 510
   Gly Asn Pro Arg Leu Ser Pro Leu Pro Ser Leu Met Val Val Met Pro
           515                 520                 525
   Leu Ala Gln Ile Lys Gln Pro Met Thr Leu Gly Thr Ile Thr Lys Arg
       530                 535                 540
   Thr Gly Pro Tyr Leu Phe Gly Ala Gly Cys Phe Ser Ile Lys Ala Pro
   545 550 555 560
   Cys Lys Val Lys Pro Pro Pro Gln Ile Ser Pro Ser Lys Ser Met Gly
   565 570 575
   Gly Glu Phe Cys Val Ala Ala Ile Phe Gly Thr Ser Arg Ser Trp Phe
   580 585 590
   Ala Asn Asn Ala Gly Leu Lys Arg Glu Lys Asp Gln Ser Lys Gln Val
   595 600 605
   Val Val Glu Ser Leu Tyr Ile Ile Ser Cys Tyr Gly Thr Leu Val Glu
   610 615 620
   His Met Met Glu Pro Arg Pro Leu Ser Thr Ala Pro Lys Ile Ser Asp
   625 630 635 640
   Asp Thr Pro Leu Glu Met Met Thr Ser Pro Arg Ala Ser Trp Thr Leu
   645 650 655
   Val Arg Thr Pro Gln Trp Asn Glu Leu Gln Pro Pro Phe Asn Ala Asn
   660 665 670
   His Pro Leu Leu Leu Ala Ala Asp Ala Val Gln Tyr Tyr Gln Phe Leu
   675 680 685
   Leu Ala Gly Leu Val Pro Pro Gly Ser Pro Gly Pro Ile Thr Arg His
   690 695 700
   Gly Ser Tyr Asp Ser Leu Ala Ser Asp His Ser Gly Gln Glu Asp Glu
   705 710 715 720
   Glu Trp Leu Ser Gln Val Glu Ile Val Thr His Thr Gly Pro His Arg
   725 730 735
   Arg Leu Trp Met Gly Pro Gln Phe Gln Phe Lys Thr Ile His Pro Ser
   740 745 750
   Gly Gln Thr Thr Val Ile Ser Ser Ser Ser Ser Val Leu Gln Ser His
   755 760 765
   Gly Pro Ser Asp Thr Pro Gln Pro Leu Leu Asp Phe Asp Thr Asp Asp
   770 775 780
   Leu Asp Leu Asn Ser Leu Arg Ile Gln Pro Val Arg Ser Asp Pro Val
   785 790 795 800
   Ser Met Pro Gly Ser Ser Arg Pro Val Ser Asp Arg Arg Gly Val Ser
   805 810 815
   Thr Val Ile Asp Ala Ala Ser Asp Glu Gln Glu Ala Leu Asn Ser Ile
   820 825 830
   Met Asn Asp Leu Val Ala Leu Gln Met Asn Arg Arg His Arg Met Pro
   835 840 845
   Gly Tyr Glu Thr Met Lys Asn Lys Asp Thr Gly His Ser Asn Arg Gln
   850 855 860
   Ser Asp Val Arg Ile Lys Phe Glu His Asn Gly Glu Arg Arg Ile Ile
   865 870 875 880
   Ala Phe Ser Arg Pro Val Lys Tyr Glu Asp Val Glu His Lys Val Thr
                   885                 890                 895
   Thr Val Phe Gly Gln Pro Leu Asp Leu His Tyr Met Asn Asn Glu Leu
               900                 905                 910
   Ser Ile Leu Leu Lys Asn Gln Asp Asp Leu Asp Lys Ala Ile Asp Ile
           915                 920                 925
   Leu Asp Arg Ser Ser Ser Met Lys Ser Leu Arg Ile Leu Leu Leu Ser
       930                 935                 940
   Gln Asp Arg Asn His Asn Ser Ser Ser Pro His Ser Gly Val Ser Arg
   945                 950                 955                 960
   Gln Val Arg Ile Lys Ala Ser Gln Ser Ala Gly Asp Ile Asn Thr Ile
                   965                 970                 975
   Tyr Gln Pro Pro Glu Pro Arg Ser Arg His Leu Ser Val Ser Ser Gln
               980                 985                 990
   Asn Pro Gly Arg Ser Ser Pro Pro Pro Gly Tyr Val Pro Glu Arg Gln
           995                 1000                1005
   Gln His Ile Ala Arg Gln Gly Ser Tyr Thr Ser Ile Asn Ser Glu Gly
      1010                1015                1020
   Glu Phe Ile Pro Glu Thr Ser Glu Gln Cys Met Leu Asp Pro Leu Ser
   1025                1030                1035                1040
   Ser Ala Glu Asn Ser Leu Ser Gly Ser Cys Gln Ser Leu Asp Arg Ser
                  1045                1050                1055
   Ala Asp Ser Pro Ser Phe Arg Lys Ser Arg Met Ser Arg Ala Gln Ser
              1060                1065                1070
   Phe Pro Asp Asn Arg Gln Glu Tyr Ser Asp Arg Glu Thr Gln Leu Tyr
          1075                1080                1085
   Asp Lys Gly Val Lys Gly Gly Thr Tyr Pro Arg Arg Tyr His Val Ser
      1090                1095                1100
   Val His His Lys Asp Tyr Ser Asp Gly Arg Arg Thr Phe Pro Arg Ile
   1105                1110                1115                1120
   Arg Arg His Gln Gly Asn Leu Phe Thr Leu Val Pro Ser Ser Arg Ser
                  1125                1130                1135
   Leu Ser Thr Asn Gly Glu Asn Met Gly Leu Ala Val Gln Tyr Leu Asp
              1140                1145                1150
   Pro Arg Gly Arg Leu Arg Ser Ala Asp Ser Glu Asn Ala Leu Ser Val
          1155                1160                1165
   Gln Glu Arg Asn Val Pro Thr Lys Ser Pro Ser Ala Pro Ile Asn Trp
      1170                1175                1180
   Arg Arg Gly Lys Leu Leu Gly Gln Gly Ala Phe Gly Arg Val Tyr Leu
   1185                1190                1195                1200
   Cys Tyr Asp Val Asp Thr Gly Arg Glu Leu Ala Ser Lys Gln Val Gln
                  1205                1210                1215
   Phe Asp Pro Asp Ser Pro Glu Thr Ser Lys Glu Val Ser Ala Leu Glu
               1220                1225                1230
   Cys Glu Ile Gln Leu Leu Lys Asn Leu Gln His Glu Arg Ile Val Gln
           1235                1240                1245
   Tyr Tyr Gly Cys Leu Arg Asp Arg Ala Glu Lys Thr Leu Thr Ile Phe
       1250                1255                1260
   Met Glu Tyr Met Pro Gly Gly Ser Val Lys Asp Gln Leu Lys Ala Tyr
   1265                1270                1275                1280
   Gly Ala Leu Thr Glu Ser Val Thr Arg Lys Tyr Thr Arg Gln Ile Leu
                   1285                1290                1295
   Glu Gly Met Ser Tyr Leu His Ser Asn Met Ile Val His Arg Asp Ile
               1300                1305                1310
   Lys Gly Ala Asn Ile Leu Arg Asp Ser Ala Gly Asn Val Lys Leu Gly
           1315                1320                1325
   Asp Phe Gly Ala Ser Lys Arg Leu Gln Thr Ile Cys Met Ser Gly Thr
       1330                1335                1340
   Gly Met Arg Ser Val Thr Gly Thr Pro Tyr Trp Met Ser Pro Glu Val
   1345                1350                1355                1360
   Ile Ser Gly Glu Gly Tyr Gly Arg Lys Ala Asp Val Trp Ser Leu Gly
                   1365                1370                1375
   Cys Thr Val Val Glu Met Leu Thr Glu Lys Pro Pro Trp Ala Glu Tyr
               1380                1385                1390
   Glu Ala Met Ala Ala Ile Phe Lys Ile Ala Thr Gln Pro Thr Asn Pro
           1395                1400                1405
   Gln Leu Pro Ser His Ile Ser Glu His Gly Arg Asp Phe Leu Arg Arg
       1410                1415                1420
   Ile Phe Val Glu Ala Arg Gln Arg Pro Ser Ala Glu Glu Leu Leu Thr
   1425                1430                1435                1440
   His His Phe Ala Gln Leu Met Tyr
                   1445
<210> 96
   <211> 15
   <212> PRT
   <213> Artificial Sequence
<220>
   <223> Fused region of BCAS3-MAP3K3 fusion protein
<400> 96
   Thr Val Ile Asp Ala Ala Ser Asp Glu Gln Glu Ala Leu Asn Ser
   1               5                   10                  15
<210> 97
   <211> 567
   <212> DNA
   <213> Artificial Sequence
<220>
   <223> CDS of KRAS gene (NM_004985)
<400> 97
   atgactgaat ataaacttgt ggtagttgga gctggtggcg taggcaagag tgccttgacg     60
   atacagctaa ttcagaatca ttttgtggac gaatatgatc caacaataga ggattcctac    120
   aggaagcaag tagtaattga tggagaaacc tgtctcttgg atattctcga cacagcaggt    180
   caagaggagt acagtgcaat gagggaccag tacatgagga ctggggaggg ctttctttgt    240
   gtatttgcca taaataatac taaatcattt gaagatattc accattatag agaacaaatt    300
   aaaagagtta aggactctga agatgtacct atggtcctag taggaaataa atgtgatttg    360
   ccttctagaa cagtagacac aaaacaggct caggacttag caagaagtta tggaattcct    420
   tttattgaaa catcagcaaa gacaagacag ggtgttgatg atgccttcta tacattagtt    480
   cgagaaattc gaaaacataa agaaaagatg agcaaagatg gtaaaaagaa gaaaaagaag    540
   tcaaagacaa agtgtgtaat tatgtaa                                        567
<210> 98
   <211> 450
   <212> DNA
   <213> Artificial Sequence
<220>
   <223> KRAS gene fragment
<400> 98
   atgactgaat ataaacttgt ggtagttgga gctggtggcg taggcaagag tgccttgacg     60
   atacagctaa ttcagaatca ttttgtggac gaatatgatc caacaataga ggattcctac    120
   aggaagcaag tagtaattga tggagaaacc tgtctcttgg atattctcga cacagcaggt    180
   caagaggagt acagtgcaat gagggaccag tacatgagga ctggggaggg ctttctttgt    240
   gtatttgcca taaataatac taaatcattt gaagatattc accattatag agaacaaatt    300
   aaaagagtta aggactctga agatgtacct atggtcctag taggaaataa atgtgatttg    360
   ccttctagaa cagtagacac aaaacaggct caggacttag caagaagtta tggaattcct    420
   tttattgaaa catcagcaaa gacaagacag                                     450
<210> 99
   <211> 21
   <212> DNA
   <213> Artificial Sequence
<220>
   <223> Break-point of KRAS gene fragment
<400> 99
   acatcagcaa agacaagaca g 21
<210> 100
   <211> 188
   <212> PRT
   <213> Artificial Sequence
<220>
   <223> KRAS protein
<400> 100
<210> 101
   <211> 150
   <212> PRT
   <213> Artificial Sequence
<220>
   <223> KRAS protein fragment
<400> 101
   Met Thr Glu Tyr Lys Leu Val Val Val Gly Ala Gly Gly Val Gly Lys
    1               5                  10                  15
   Ser Ala Leu Thr Ile Gln Leu Ile Gln Asn His Phe Val Asp Glu Tyr
               20                  25                  30
   Asp Pro Thr Ile Glu Asp Ser Tyr Arg Lys Gln Val Val Ile Asp Gly
           35                  40                  45
   Glu Thr Cys Leu Leu Asp Ile Leu Asp Thr Ala Gly Gln Glu Glu Tyr
       50                  55                  60
   Ser Ala Met Arg Asp Gln Tyr Met Arg Thr Gly Glu Gly Phe Leu Cys
   65                  70                  75                  80
   Val Phe Ala Ile Asn Asn Thr Lys Ser Phe Glu Asp Ile His His Tyr
                   85                  90                  95
   Arg Glu Gln Ile Lys Arg Val Lys Asp Ser Glu Asp Val Pro Met Val
               100                 105                 110
   Leu Val Gly Asn Lys Cys Asp Leu Pro Ser Arg Thr Val Asp Thr Lys
           115                 120                 125
   Gln Ala Gln Asp Leu Ala Arg Ser Tyr Gly Ile Pro Phe Ile Glu Thr
       130                 135                 140
   Ser Ala Lys Thr Arg Gln
   145                 150
<210> 102
   <211> 7
   <212> PRT
   <213> Artificial Sequence
<220>
   <223> Break-point of KRAS protein fragment
<400> 102
   Thr Ser Ala Lys Thr Arg Gln
   1               5
<210> 103
   <211> 2142
   <212> DNA
   <213> Artificial Sequence
<220>
   <223> CDS of CDH13 gene (NM_001257)
<400> 103
   atgcagccga gaactccgct cgttctgtgc gttctcctgt cccaggtgct gctgctaaca      60
   tctgcagaag atttggactg cactcctgga tttcagcaga aagtgttcca tatcaatcag     120
   ccagctgaat tcattgagga ccagtcaatt ctaaacttga ccttcagtga ctgtaaggga     180
   aacgacaagc tacgctatga ggtctcgagc ccatacttca aggtgaacag cgatggcggc     240
   ttagttgctc tgagaaacat aactgcagtg ggcaaaactc tgttcgtcca tgcacggacc     300
   ccccatgcgg aagatatggc agaactcgtg attgtcgggg ggaaagacat ccagggctcc 360
   ttgcaggata tatttaaatt tgcaagaact tctcctgtcc caagacaaaa gaggtccatt 420
   gtggtatctc ccattttaat tccagagaat cagagacagc ctttcccaag agatgttggc 480
   aaggtagtcg atagtgacag gccagaaagg tccaagttcc ggctcactgg aaagggagtg 540
   gatcaagagc ctaaaggaat tttcagaatc aatgagaaca cagggagcgt ctccgtgaca 600
   cggaccttgg acagagaagt aatcgctgtt tatcaactat ttgtggagac cactgatgtc 660
   aatggcaaaa ctctcgaggg gccggtgcct ctggaagtca ttgtgattga tcagaatgac 720
   aaccgaccga tctttcggga aggcccctac atcggccacg tcatggaagg gtcacccaca 780
   ggcaccacag tgatgcggat gacagccttt gatgcagatg acccagccac cgataatgcc 840
   ctcctgcggt ataatatccg tcagcagacg cctgacaagc catctcccaa catgttctac 900
   atcgatcctg agaaaggaga cattgtcact gttgtgtcac ctgcgctgct ggaccgagag 960
   actctggaaa atcccaagta tgaactgatc atcgaggctc aagatatggc tggactggat 1020
   gttggattaa caggcacggc cacagccacg atcatgatcg atgacaaaaa tgatcactca 1080
   ccaaaattca ccaagaaaga gtttcaagcc acagtcgagg aaggagctgt gggagttatt 1140
   gtcaatttga cagttgaaga taaggatgac cccaccacag gtgcatggag ggctgcctac 1200
   accatcatca acggaaaccc cgggcagagc tttgaaatcc acaccaaccc tcaaaccaac 1260
   gaagggatgc tttctgttgt caaaccattg gactatgaaa tttctgcctt ccacaccctg 1320
   ctgatcaaag tggaaaatga agacccactc gtacccgacg tctcctacgg ccccagctcc 1380
   acagccaccg tccacatcac tgtcctggat gtcaacgagg gcccagtctt ctacccagac 1440
   cccatgatgg tgaccaggca ggaggacctc tctgtgggca gcgtgctgct gacagtgaat 1500
   gccacggacc ccgactccct gcagcatcaa accatcaggt attctgttta caaggaccca 1560
   gcaggttggc tgaatattaa ccccatcaat gggactgttg acaccacagc tgtgctggac 1620
   cgtgagtccc catttgtcga caacagcgtg tacactgctc tcttcctggc aattgacagt 1680
   ggcaaccctc ccgctacggg cactgggact ttgctgataa ccctggagga cgtgaatgac 1740
   aatgccccgt tcatttaccc cacagtagct gaagtctgtg atgatgccaa aaacctcagt 1800
   gtagtcattt tgggagcatc agataaggat cttcacccga atacagatcc tttcaaattt 1860
   gaaatccaca aacaagctgt tcctgataaa gtctggaaga tctccaagat caacaataca 1920
   cacgccctgg taagccttct tcaaaatctg aacaaagcaa actacaacct gcccatcatg 1980
   gtgacagatt cagggaaacc acccatgacg aatatcacag atctcagggt acaagtgtgc 2040
   tcctgcagga attccaaagt ggactgcaac gcggcagggg ccctgcgctt cagcctgccc 2100
   tcagtcctgc tcctcagcct cttcagctta gcttgtctgt ga 2142
<210> 104
   <211> 1776
   <212> DNA
   <213> Artificial Sequence
<220>
   <223> CDH13 gene fragment
<400> 104
   gatatattta aatttgcaag aacttctcct gtcccaagac aaaagaggtc cattgtggta      60
   tctcccattt taattccaga gaatcagaga cagcctttcc caagagatgt tggcaaggta     120
   gtcgatagtg acaggccaga aaggtccaag ttccggctca ctggaaaggg agtggatcaa     180
   gagcctaaag gaattttcag aatcaatgag aacacaggga gcgtctccgt gacacggacc     240
   ttggacagag aagtaatcgc tgtttatcaa ctatttgtgg agaccactga tgtcaatggc     300
   aaaactctcg aggggccggt gcctctggaa gtcattgtga ttgatcagaa tgacaaccga     360
   ccgatctttc gggaaggccc ctacatcggc cacgtcatgg aagggtcacc cacaggcacc     420
   acagtgatgc ggatgacagc ctttgatgca gatgacccag ccaccgataa tgccctcctg     480
   cggtataata tccgtcagca gacgcctgac aagccatctc ccaacatgtt ctacatcgat     540
   cctgagaaag gagacattgt cactgttgtg tcacctgcgc tgctggaccg agagactctg     600
   gaaaatccca agtatgaact gatcatcgag gctcaagata tggctggact ggatgttgga     660
   ttaacaggca cggccacagc cacgatcatg atcgatgaca aaaatgatca ctcaccaaaa     720
   ttcaccaaga aagagtttca agccacagtc gaggaaggag ctgtgggagt tattgtcaat     780
   ttgacagttg aagataagga tgaccccacc acaggtgcat ggagggctgc ctacaccatc     840
   atcaacggaa accccgggca gagctttgaa atccacacca accctcaaac caacgaaggg     900
   atgctttctg ttgtcaaacc attggactat gaaatttctg ccttccacac cctgctgatc     960
   aaagtggaaa atgaagaccc actcgtaccc gacgtctcct acggccccag ctccacagcc    1020
   accgtccaca tcactgtcct ggatgtcaac gagggcccag tcttctaccc agaccccatg    1080
   atggtgacca ggcaggagga cctctctgtg ggcagcgtgc tgctgacagt gaatgccacg    1140
   gaccccgact ccctgcagca tcaaaccatc aggtattctg tttacaagga cccagcaggt    1200
   tggctgaata ttaaccccat caatgggact gttgacacca cagctgtgct ggaccgtgag    1260
   tccccatttg tcgacaacag cgtgtacact gctctcttcc tggcaattga cagtggcaac    1320
   cctcccgcta cgggcactgg gactttgctg ataaccctgg aggacgtgaa tgacaatgcc    1380
   ccgttcattt accccacagt agctgaagtc tgtgatgatg ccaaaaacct cagtgtagtc    1440
   attttgggag catcagataa ggatcttcac ccgaatacag atcctttcaa atttgaaatc    1500
   cacaaacaag ctgttcctga taaagtctgg aagatctcca agatcaacaa tacacacgcc    1560
   ctggtaagcc ttcttcaaaa tctgaacaaa gcaaactaca acctgcccat catggtgaca        1620
   gattcaggga aaccacccat gacgaatatc acagatctca gggtacaagt gtgctcctgc        1680
   aggaattcca aagtggactg caacgcggca ggggccctgc gcttcagcct gccctcagtc        1740
   ctgctcctca gcctcttcag cttagcttgt ctgtga                                  1776
<210> 105
   <211> 21
   <212> DNA
   <213> Artificial Sequence
<220>
   <223> Break-point of CDH13 gene fragment
<400> 105
   gatatattta aatttgcaag a 21
<210> 106
   <211> 713
   <212> PRT
   <213> Artificial Sequence
<220>
   <223> CDH13 protein
<400> 106
   Met Gln Pro Arg Thr Pro Leu Val Leu Cys Val Leu Leu Ser Gln Val
   1               5                   10                  15
   Leu Leu Leu Thr Ser Ala Glu Asp Leu Asp Cys Thr Pro Gly Phe Gln
               20                  25                  30
   Gln Lys Val Phe His Ile Asn Gln Pro Ala Glu Phe Ile Glu Asp Gln
           35                  40                  45
   Ser Ile Leu Asn Leu Thr Phe Ser Asp Cys Lys Gly Asn Asp Lys Leu
       50                  55                  60
   Arg Tyr Glu Val Ser Ser Pro Tyr Phe Lys Val Asn Ser Asp Gly Gly
   65                  70                  75                  80
   Leu Val Ala Leu Arg Asn Ile Thr Ala Val Gly Lys Thr Leu Phe Val
                   85                  90                  95
   His Ala Arg Thr Pro His Ala Glu Asp Met Ala Glu Leu Val Ile Val
               100                 105                 110
   Gly Gly Lys Asp Ile Gln Gly Ser Leu Gln Asp Ile Phe Lys Phe Ala
           115                 120                 125
   Arg Thr Ser Pro Val Pro Arg Gln Lys Arg Ser Ile Val Val Ser Pro
       130                 135                 140
   Ile Leu Ile Pro Glu Asn Gln Arg Gln Pro Phe Pro Arg Asp Val Gly
   145                 150                 155                 160
   Lys Val Val Asp Ser Asp Arg Pro Glu Arg Ser Lys Phe Arg Leu Thr
   165 170 175
   Gly Lys Gly Val Asp Gln Glu Pro Lys Gly Ile Phe Arg Ile Asn Glu
   180 185 190
   Asn Thr Gly Ser Val Ser Val Thr Arg Thr Leu Asp Arg Glu Val Ile
   195 200 205
   Ala Val Tyr Gln Leu Phe Val Glu Thr Thr Asp Val Asn Gly Lys Thr
   210 215 220
   Leu Glu Gly Pro Val Pro Leu Glu Val Ile Val Ile Asp Gln Asn Asp
   225 230 235 240
   Asn Arg Pro Ile Phe Arg Glu Gly Pro Tyr Ile Gly His Val Met Glu
   245 250 255
   Gly Ser Pro Thr Gly Thr Thr Val Met Arg Met Thr Ala Phe Asp Ala
   260 265 270
   Asp Asp Pro Ala Thr Asp Asn Ala Leu Leu Arg Tyr Asn Ile Arg Gln
   275 280 285
   Gln Thr Pro Asp Lys Pro Ser Pro Asn Met Phe Tyr Ile Asp Pro Glu
   290 295 300
   Lys Gly Asp Ile Val Thr Val Val Ser Pro Ala Leu Leu Asp Arg Glu
   305 310 315 320
   Thr Leu Glu Asn Pro Lys Tyr Glu Leu Ile Ile Glu Ala Gln Asp Met
   325 330 335
   Ala Gly Leu Asp Val Gly Leu Thr Gly Thr Ala Thr Ala Thr Ile Met
   340 345 350
   Ile Asp Asp Lys Asn Asp His Ser Pro Lys Phe Thr Lys Lys Glu Phe
   355 360 365
   Gln Ala Thr Val Glu Glu Gly Ala Val Gly Val Ile Val Asn Leu Thr
   370 375 380
   Val Glu Asp Lys Asp Asp Pro Thr Thr Gly Ala Trp Arg Ala Ala Tyr
   385 390 395 400
   Thr Ile Ile Asn Gly Asn Pro Gly Gln Ser Phe Glu Ile His Thr Asn
   405 410 415
   Pro Gln Thr Asn Glu Gly Met Leu Ser Val Val Lys Pro Leu Asp Tyr
   420 425 430
   Glu Ile Ser Ala Phe His Thr Leu Leu Ile Lys Val Glu Asn Glu Asp
   435 440 445
   Pro Leu Val Pro Asp Val Ser Tyr Gly Pro Ser Ser Thr Ala Thr Val
   450 455 460
   His Ile Thr Val Leu Asp Val Asn Glu Gly Pro Val Phe Tyr Pro Asp
   465 470 475 480
   Pro Met Met Val Thr Arg Gln Glu Asp Leu Ser Val Gly Ser Val Leu
   485 490 495
   Leu Thr Val Asn Ala Thr Asp Pro Asp Ser Leu Gln His Gln Thr Ile
   500 505 510
   Arg Tyr Ser Val Tyr Lys Asp Pro Ala Gly Trp Leu Asn Ile Asn Pro
   515 520 525
   Ile Asn Gly Thr Val Asp Thr Thr Ala Val Leu Asp Arg Glu Ser Pro
   530 535 540
   Phe Val Asp Asn Ser Val Tyr Thr Ala Leu Phe Leu Ala Ile Asp Ser
   545 550 555 560
   Gly Asn Pro Pro Ala Thr Gly Thr Gly Thr Leu Leu Ile Thr Leu Glu
   565 570 575
   Asp Val Asn Asp Asn Ala Pro Phe Ile Tyr Pro Thr Val Ala Glu Val
   580 585 590
   Cys Asp Asp Ala Lys Asn Leu Ser Val Val Ile Leu Gly Ala Ser Asp
   595 600 605
   Lys Asp Leu His Pro Asn Thr Asp Pro Phe Lys Phe Glu Ile His Lys
   610 615 620
   Gln Ala Val Pro Asp Lys Val Trp Lys Ile Ser Lys Ile Asn Asn Thr
   625 630 635 640
   His Ala Leu Val Ser Leu Leu Gln Asn Leu Asn Lys Ala Asn Tyr Asn
   645 650 655
   Leu Pro Ile Met Val Thr Asp Ser Gly Lys Pro Pro Met Thr Asn Ile
   660 665 670
   Thr Asp Leu Arg Val Gln Val Cys Ser Cys Arg Asn Ser Lys Val Asp
   675 680 685
   Cys Asn Ala Ala Gly Ala Leu Arg Phe Ser Leu Pro Ser Val Leu Leu
   690 695 700
   Leu Ser Leu Phe Ser Leu Ala Cys Leu
   705 710
<210> 107
   <211> 591
   <212> PRT
   <213> Artificial Sequence
<220>
   <223> CDH13 protein fragment
<400> 107
   Asp Ile Phe Lys Phe Ala Arg Thr Ser Pro Val Pro Arg Gln Lys Arg
   1               5                   10                  15
   Ser Ile Val Val Ser Pro Ile Leu Ile Pro Glu Asn Gln Arg Gln Pro
               20                  25                  30
   Phe Pro Arg Asp Val Gly Lys Val Val Asp Ser Asp Arg Pro Glu Arg
           35                  40                  45
   Ser Lys Phe Arg Leu Thr Gly Lys Gly Val Asp Gln Glu Pro Lys Gly
       50                  55                  60
   Ile Phe Arg Ile Asn Glu Asn Thr Gly Ser Val Ser Val Thr Arg Thr
    65                  70                  75                  80
   Leu Asp Arg Glu Val Ile Ala Val Tyr Gln Leu Phe Val Glu Thr Thr
                    85                  90                  95
   Asp Val Asn Gly Lys Thr Leu Glu Gly Pro Val Pro Leu Glu Val Ile
               100                 105                 110
   Val Ile Asp Gln Asn Asp Asn Arg Pro Ile Phe Arg Glu Gly Pro Tyr
           115                 120                 125
   Ile Gly His Val Met Glu Gly Ser Pro Thr Gly Thr Thr Val Met Arg
       130                 135                 140
   Met Thr Ala Phe Asp Ala Asp Asp Pro Ala Thr Asp Asn Ala Leu Leu
   145                 150                 155                 160
   Arg Tyr Asn Ile Arg Gln Gln Thr Pro Asp Lys Pro Ser Pro Asn Met
                   165                 170                 175
   Phe Tyr Ile Asp Pro Glu Lys Gly Asp Ile Val Thr Val Val Ser Pro
               180                 185                 190
   Ala Leu Leu Asp Arg Glu Thr Leu Glu Asn Pro Lys Tyr Glu Leu Ile
           195                 200                 205
   Ile Glu Ala Gln Asp Met Ala Gly Leu Asp Val Gly Leu Thr Gly Thr
       210                 215                 220
   Ala Thr Ala Thr Ile Met Ile Asp Asp Lys Asn Asp His Ser Pro Lys
   225                 230                 235                 240
   Phe Thr Lys Lys Glu Phe Gln Ala Thr Val Glu Glu Gly Ala Val Gly
                   245                 250                 255
   Val Ile Val Asn Leu Thr Val Glu Asp Lys Asp Asp Pro Thr Thr Gly
               260                 265                 270
   Ala Trp Arg Ala Ala Tyr Thr Ile Ile Asn Gly Asn Pro Gly Gln Ser
           275                 280                 285
   Phe Glu Ile His Thr Asn Pro Gln Thr Asn Glu Gly Met Leu Ser Val
       290                 295                 300
   Val Lys Pro Leu Asp Tyr Glu Ile Ser Ala Phe His Thr Leu Leu Ile
   305                 310                 315                 320
   Lys Val Glu Asn Glu Asp Pro Leu Val Pro Asp Val Ser Tyr Gly Pro
                   325                 330                 335
   Ser Ser Thr Ala Thr Val His Ile Thr Val Leu Asp Val Asn Glu Gly
               340                 345                 350
   Pro Val Phe Tyr Pro Asp Pro Met Met Val Thr Arg Gln Glu Asp Leu
           355                 360                 365
   Ser Val Gly Ser Val Leu Leu Thr Val Asn Ala Thr Asp Pro Asp Ser
       370                 375                 380
   Leu Gln His Gln Thr Ile Arg Tyr Ser Val Tyr Lys Asp Pro Ala Gly
   385                 390                 395                 400
   Trp Leu Asn Ile Asn Pro Ile Asn Gly Thr Val Asp Thr Thr Ala Val
   405 410 415
   Leu Asp Arg Glu Ser Pro Phe Val Asp Asn Ser Val Tyr Thr Ala Leu
   420 425 430
   Phe Leu Ala Ile Asp Ser Gly Asn Pro Pro Ala Thr Gly Thr Gly Thr
   435 440 445
   Leu Leu Ile Thr Leu Glu Asp Val Asn Asp Asn Ala Pro Phe Ile Tyr
   450 455 460
   Pro Thr Val Ala Glu Val Cys Asp Asp Ala Lys Asn Leu Ser Val Val
   465 470 475 480
   Ile Leu Gly Ala Ser Asp Lys Asp Leu His Pro Asn Thr Asp Pro Phe
   485 490 495
   Lys Phe Glu Ile His Lys Gln Ala Val Pro Asp Lys Val Trp Lys Ile
   500 505 510
   Ser Lys Ile Asn Asn Thr His Ala Leu Val Ser Leu Leu Gln Asn Leu
   515 520 525
   Asn Lys Ala Asn Tyr Asn Leu Pro Ile Met Val Thr Asp Ser Gly Lys
   530 535 540
   Pro Pro Met Thr Asn Ile Thr Asp Leu Arg Val Gln Val Cys Ser Cys
   545 550 555 560
   Arg Asn Ser Lys Val Asp Cys Asn Ala Ala Gly Ala Leu Arg Phe Ser
   565 570 575
   Leu Pro Ser Val Leu Leu Leu Ser Leu Phe Ser Leu Ala Cys Leu
   580 585 590
<210> 108
   <211> 7
   <212> PRT
   <213> Artificial Sequence
<220>
   <223> Break-point of CDH13 protein fragment
<400> 108
   Asp Ile Phe Lys Phe Ala Arg
   1 5
<210> 109
   <211> 2226
   <212> DNA
   <213> Artificial Sequence
<220>
   <223> KRAS-CDH13 fusion gene
<400> 109
   atgactgaat ataaacttgt ggtagttgga gctggtggcg taggcaagag tgccttgacg        60
   atacagctaa ttcagaatca ttttgtggac gaatatgatc caacaataga ggattcctac       120
   aggaagcaag tagtaattga tggagaaacc tgtctcttgg atattctcga cacagcaggt       180
   caagaggagt acagtgcaat gagggaccag tacatgagga ctggggaggg ctttctttgt       240
   gtatttgcca taaataatac taaatcattt gaagatattc accattatag agaacaaatt       300
   aaaagagtta aggactctga agatgtacct atggtcctag taggaaataa atgtgatttg       360
   ccttctagaa cagtagacac aaaacaggct caggacttag caagaagtta tggaattcct       420
   tttattgaaa catcagcaaa gacaagacag gatatattta aatttgcaag aacttctcct       480
   gtcccaagac aaaagaggtc cattgtggta tctcccattt taattccaga gaatcagaga       540
   cagcctttcc caagagatgt tggcaaggta gtcgatagtg acaggccaga aaggtccaag       600
   ttccggctca ctggaaaggg agtggatcaa gagcctaaag gaattttcag aatcaatgag       660
   aacacaggga gcgtctccgt gacacggacc ttggacagag aagtaatcgc tgtttatcaa       720
   ctatttgtgg agaccactga tgtcaatggc aaaactctcg aggggccggt gcctctggaa       780
   gtcattgtga ttgatcagaa tgacaaccga ccgatctttc gggaaggccc ctacatcggc       840
   cacgtcatgg aagggtcacc cacaggcacc acagtgatgc ggatgacagc ctttgatgca       900
   gatgacccag ccaccgataa tgccctcctg cggtataata tccgtcagca gacgcctgac       960
   aagccatctc ccaacatgtt ctacatcgat cctgagaaag gagacattgt cactgttgtg      1020
   tcacctgcgc tgctggaccg agagactctg gaaaatccca agtatgaact gatcatcgag      1080
   gctcaagata tggctggact ggatgttgga ttaacaggca cggccacagc cacgatcatg      1140
   atcgatgaca aaaatgatca ctcaccaaaa ttcaccaaga aagagtttca agccacagtc      1200
   gaggaaggag ctgtgggagt tattgtcaat ttgacagttg aagataagga tgaccccacc      1260
   acaggtgcat ggagggctgc ctacaccatc atcaacggaa accccgggca gagctttgaa      1320
   atccacacca accctcaaac caacgaaggg atgctttctg ttgtcaaacc attggactat      1380
   gaaatttctg ccttccacac cctgctgatc aaagtggaaa atgaagaccc actcgtaccc      1440
   gacgtctcct acggccccag ctccacagcc accgtccaca tcactgtcct ggatgtcaac      1500
   gagggcccag tcttctaccc agaccccatg atggtgacca ggcaggagga cctctctgtg      1560
   ggcagcgtgc tgctgacagt gaatgccacg gaccccgact ccctgcagca tcaaaccatc      1620
   aggtattctg tttacaagga cccagcaggt tggctgaata ttaaccccat caatgggact      1680
   gttgacacca cagctgtgct ggaccgtgag tccccatttg tcgacaacag cgtgtacact      1740
   gctctcttcc tggcaattga cagtggcaac cctcccgcta cgggcactgg gactttgctg      1800
   ataaccctgg aggacgtgaa tgacaatgcc ccgttcattt accccacagt agctgaagtc      1860
   tgtgatgatg ccaaaaacct cagtgtagtc attttgggag catcagataa ggatcttcac 1920
   ccgaatacag atcctttcaa atttgaaatc cacaaacaag ctgttcctga taaagtctgg 1980
   aagatctcca agatcaacaa tacacacgcc ctggtaagcc ttcttcaaaa tctgaacaaa 2040
   gcaaactaca acctgcccat catggtgaca gattcaggga aaccacccat gacgaatatc 2100
   acagatctca gggtacaagt gtgctcctgc aggaattcca aagtggactg caacgcggca 2160
   ggggccctgc gcttcagcct gccctcagtc ctgctcctca gcctcttcag cttagcttgt 2220
   ctgtga 2226
<210> 110
   <211> 42
   <212> DNA
   <213> Artificial Sequence
<220>
   <223> Fused region of KRAS-CDH13 fusion gene
<400> 110
   acatcagcaa agacaagaca ggatatattt aaatttgcaa ga 42
<210> 111
   <211> 741
   <212> PRT
   <213> Artificial Sequence
<220>
   <223> KRAS-CDH13 fusion protein
<400> 111
   Met Thr Glu Tyr Lys Leu Val Val Val Gly Ala Gly Gly Val Gly Lys
   1               5                   10                  15
   Ser Ala Leu Thr Ile Gln Leu Ile Gln Asn His Phe Val Asp Glu Tyr
               20                  25                  30
   Asp Pro Thr Ile Glu Asp Ser Tyr Arg Lys Gln Val Val Ile Asp Gly
           35                  40                  45
   Glu Thr Cys Leu Leu Asp Ile Leu Asp Thr Ala Gly Gln Glu Glu Tyr
       50                  55                  60
   Ser Ala Met Arg Asp Gln Tyr Met Arg Thr Gly Glu Gly Phe Leu Cys
   65                  70                  75                  80
   Val Phe Ala Ile Asn Asn Thr Lys Ser Phe Glu Asp Ile His His Tyr
                   85                  90                  95
   Arg Glu Gln Ile Lys Arg Val Lys Asp Ser Glu Asp Val Pro Met Val
               100                 105                 110
   Leu Val Gly Asn Lys Cys Asp Leu Pro Ser Arg Thr Val Asp Thr Lys
           115                 120                 125
   Gln Ala Gln Asp Leu Ala Arg Ser Tyr Gly Ile Pro Phe Ile Glu Thr
           130                 135                 140
   Ser Ala Lys Thr Arg Gln Asp Ile Phe Lys Phe Ala Arg Thr Ser Pro
   145                 150                 155                 160
   Val Pro Arg Gln Lys Arg Ser Ile Val Val Ser Pro Ile Leu Ile Pro
                   165                 170                 175
   Glu Asn Gln Arg Gln Pro Phe Pro Arg Asp Val Gly Lys Val Val Asp
               180                 185                 190
   Ser Asp Arg Pro Glu Arg Ser Lys Phe Arg Leu Thr Gly Lys Gly Val
           195                 200                 205
   Asp Gln Glu Pro Lys Gly Ile Phe Arg Ile Asn Glu Asn Thr Gly Ser
       210                 215                 220
   Val Ser Val Thr Arg Thr Leu Asp Arg Glu Val Ile Ala Val Tyr Gln
   225                 230                 235                 240
   Leu Phe Val Glu Thr Thr Asp Val Asn Gly Lys Thr Leu Glu Gly Pro
                   245                 250                 255
   Val Pro Leu Glu Val Ile Val Ile Asp Gln Asn Asp Asn Arg Pro Ile
               260                 265                 270
   Phe Arg Glu Gly Pro Tyr Ile Gly His Val Met Glu Gly Ser Pro Thr
           275                 280                 285
   Gly Thr Thr Val Met Arg Met Thr Ala Phe Asp Ala Asp Asp Pro Ala
       290                 295                 300
   Thr Asp Asn Ala Leu Leu Arg Tyr Asn Ile Arg Gln Gln Thr Pro Asp
   305                 310                 315                 320
   Lys Pro Ser Pro Asn Met Phe Tyr Ile Asp Pro Glu Lys Gly Asp Ile
                   325                 330                 335
   Val Thr Val Val Ser Pro Ala Leu Leu Asp Arg Glu Thr Leu Glu Asn
               340                 345                 350
   Pro Lys Tyr Glu Leu Ile Ile Glu Ala Gln Asp Met Ala Gly Leu Asp
           355                 360                 365
   Val Gly Leu Thr Gly Thr Ala Thr Ala Thr Ile Met Ile Asp Asp Lys
       370                 375                 380
   Asn Asp His Ser Pro Lys Phe Thr Lys Lys Glu Phe Gln Ala Thr Val
   385                 390                 395                 400
   Glu Glu Gly Ala Val Gly Val Ile Val Asn Leu Thr Val Glu Asp Lys
                   405                 410                 415
   Asp Asp Pro Thr Thr Gly Ala Trp Arg Ala Ala Tyr Thr Ile Ile Asn
               420                 425                 430
   Gly Asn Pro Gly Gln Ser Phe Glu Ile His Thr Asn Pro Gln Thr Asn
           435                 440                 445
   Glu Gly Met Leu Ser Val Val Lys Pro Leu Asp Tyr Glu Ile Ser Ala
       450                 455                 460
   Phe His Thr Leu Leu Ile Lys Val Glu Asn Glu Asp Pro Leu Val Pro
   465                 470                 475                 480
   Asp Val Ser Tyr Gly Pro Ser Ser Thr Ala Thr Val His Ile Thr Val
                   485                 490                 495
   Leu Asp Val Asn Glu Gly Pro Val Phe Tyr Pro Asp Pro Met Met Val
               500                 505                 510
   Thr Arg Gln Glu Asp Leu Ser Val Gly Ser Val Leu Leu Thr Val Asn
           515                 520                 525
   Ala Thr Asp Pro Asp Ser Leu Gln His Gln Thr Ile Arg Tyr Ser Val
       530                 535                 540
   Tyr Lys Asp Pro Ala Gly Trp Leu Asn Ile Asn Pro Ile Asn Gly Thr
   545                 550                 555                 560
   Val Asp Thr Thr Ala Val Leu Asp Arg Glu Ser Pro Phe Val Asp Asn
                   565                 570                 575
   Ser Val Tyr Thr Ala Leu Phe Leu Ala Ile Asp Ser Gly Asn Pro Pro
               580                 585                 590
   Ala Thr Gly Thr Gly Thr Leu Leu Ile Thr Leu Glu Asp Val Asn Asp
           595                 600                 605
   Asn Ala Pro Phe Ile Tyr Pro Thr Val Ala Glu Val Cys Asp Asp Ala
       610                 615                 620
   Lys Asn Leu Ser Val Val Ile Leu Gly Ala Ser Asp Lys Asp Leu His
   625                 630                 635                 640
   Pro Asn Thr Asp Pro Phe Lys Phe Glu Ile His Lys Gln Ala Val Pro
                   645                 650                 655
   Asp Lys Val Trp Lys Ile Ser Lys Ile Asn Asn Thr His Ala Leu Val
               660                 665                 670
   Ser Leu Leu Gln Asn Leu Asn Lys Ala Asn Tyr Asn Leu Pro Ile Met
           675                 680                 685
   Val Thr Asp Ser Gly Lys Pro Pro Met Thr Asn Ile Thr Asp Leu Arg
       690                 695                 700
   Val Gln Val Cys Ser Cys Arg Asn Ser Lys Val Asp Cys Asn Ala Ala
   705                 710                 715                 720
   Gly Ala Leu Arg Phe Ser Leu Pro Ser Val Leu Leu Leu Ser Leu Phe
                   725                 730                 735
   Ser Leu Ala Cys Leu
               740
<210> 112
   <211> 14
   <212> PRT
   <213> Artificial Sequence
<220>
   <223> Fused region of KRAS-CDH13 fusion protein
<400> 112
   Thr Ser Ala Lys Thr Arg Gln Asp Ile Phe Lys Phe Ala Arg
    1               5                  10
<210> 113
   <211> 4101
   <212> DNA
   <213> Artificial Sequence
<220>
   <223> CDS of ZFYVE9 gene (NM_007324)
<400> 113
   atggagaatt acttccaagc agaagcttac aacctggaca aggtgttaga tgaatttgaa      60
   caaaacgaag atgaaacagt ttcttctact ttattggata caaagtggaa taagattcta     120
   gatccccctt ctcaccggct gtcatttaac cctactttgg ccagtgtgaa tgaatctgca     180
   gtttctaatg agtcacaacc acaactgaaa gtcttctccc tggctcattc agctcccctg     240
   accacagagg aagaggatca ctgtgctaat ggacaggact gtaatctaaa tccagagatt     300
   gccacaatgt ggattgatga aaatgctgtt gcagaagacc agttaattaa gagaaactat     360
   agttgggatg atcaatgcag tgctgttgaa gtgggagaga agaaatgtgg aaacctggct     420
   tgtctgccag atgagaagaa tgttcttgtt gtagccgtca tgcataactg tgataaaagg     480
   acattacaaa acgatttaca ggattgtaat aattataata gtcaatccct tatggatgct     540
   tttagctgtt cactggataa tgaaaacaga caaactgatc aatttagttt tagtataaat     600
   gagtccactg aaaaagatat gaattcagag aaacaaatgg atccattgaa tagaccgaaa     660
   acagagggga gatctgttaa ccatctgtgt cctacttcat ctgatagtct agccagtgtc     720
   tgttcccctt cacaattaaa ggatgacgga agtataggta gagacccctc catgtctgcg     780
   attacaagtt taacggttga ttcagtaatc tcatcccagg gaacagatgg atgtcctgct     840
   gttaaaaagc aagagaacta tataccagat gaggacctca ctggcaaaat cagctctcct     900
   aggacagatc tagggagtcc aaattccttt tcccacatga gtgaggggat tttgatgaaa     960
   aaagagccag cagaggagag caccactgaa gaatccctcc ggtctggttt acctttgctt    1020
   ctcaaaccag acatgcctaa tgggtctgga aggaataatg actgtgaacg gtgttcagat    1080
   tgccttgtgc ctaatgaagt tagggctgat gaaaatgaag gttatgaaca tgaagaaact    1140
   cttggcacta cagaattcct taatatgaca gagcatttct ctgaatctca ggacatgact    1200
   aattggaagt tgactaaact aaatgagatg aatgatagcc aagtaaacga agaaaaggaa    1260
   aagtttctac agattagtca gcctgaggac actaatggtg atagtggagg acagtgtgtt    1320
   ggattggcag atgcaggtct agatttaaaa ggaacttgca ttagtgaaag tgaagaatgt    1380
   gatttctcca ctgttataga cacaccagca gcaaattatc tatctaatgg ttgtgattcc    1440
   tatggaatgc aagacccagg tgtttctttt gttccaaaga ctttaccctc caaagaagat 1500
   tcagtaacag aagaaaaaga aatagaggaa agcaagtcag aatgctactc aaatatttat 1560
   gaacagagag gaaatgaggc cacagaaggg agtggactac ttttaaacag cactggtgac 1620
   ctaatgaaga aaaattattt acataatttc tgtagtcaag ttccatcagt gcttgggcaa 1680
   tcttccccca aggtagtagc aagcctgcca tctatcagtg ttccttttgg tggtgcaaga 1740
   cccaagcaac cttctaatct taaacttcaa attccaaagc cattatcaga ccatttacaa 1800
   aatgactttc ctgcaaacag tggaaataat actaaaaata aaaatgatat tcttgggaaa 1860
   gcaaaattag gggaaaactc agcaaccaat gtatgcagtc catctttggg aaacatctct 1920
   aatgtcgata caaatgggga acatttagaa agttatgagg ctgagatctc cactagacca 1980
   tgccttgcat tagctccaga tagcccagat aatgatctca gagctggtca gtttggaatt 2040
   tctgccagaa agccattcac cactctgggt gaggtggctc cagtatgggt accggattct 2100
   caggctccaa attgcatgaa atgtgaagcc aggtttacat tcaccaaaag gaggcatcac 2160
   tgcagagcat gtgggaaggt tttctgtgct tcctgctgta gcctgaaatg taaactgtta 2220
   tacatggaca gaaaggaagc tagagtgtgt gtaatctgcc attcagtgct aatgaatgtg 2280
   gctcagccca gagagcagag gcgagtttgg tttgctgatg ggatcttgcc caatggagaa 2340
   gttgctgatg cagccaaatt aacaatgaat ggaacttcct ctgcaggaac cctggctgtg 2400
   tcacacgacc cagtcaagcc agtaactacc agtcctctac cagcagagac ggatatttgt 2460
   ctattctctg ggagtataac tcaggttgga agtcctgttg gaagtgcaat gaatcttatt 2520
   cctgaagatg gccttcctcc cattctcatc tccactggtg taaaaggaga ctatgctgtg 2580
   gaagagaaac catcacagat ttcagtaatg cagcagttgg aggatggtgg ccctgaccca 2640
   cttgtatttg ttttaaatgc aaatttgttg tcaatggtta aaattgtaaa ttatgtgaac 2700
   aggaagtgct ggtgtttcac aaccaaggga atgcatgcag tgggtcagtc tgagatagtc 2760
   attcttctac agtgtttacc ggatgaaaag tgtttgccaa aggatatctt taatcacttt 2820
   gtgcagcttt atcgggatgc tctggcaggg aatgtggtga gcaacttggg acattccttc 2880
   ttcagtcaaa gtttccttgg cagtaaagaa catggtggat tcttatatgt gacatctacc 2940
   taccagtcac tgcaagacct agtactccca accccacctt acttgtttgg gattcttatc 3000
   cagaaatggg aaactccttg ggctaaagta tttcctatcc gtctgatgtt gagacttgga 3060
   gctgaatatc gactttatcc atgcccacta ttcagtgtca gatttcggaa gccattgttt 3120
   ggagagacgg ggcataccat catgaatctt cttgcagact tcagaaatta ccagtatacc 3180
   ttgccagtag ttcaaggttt ggtggttgat atggaagttc ggaaaactag catcaaaatt 3240
   cccagcaaca gatacaatga gatgatgaaa gccatgaaca agtccaatga gcatgtcctg 3300
   gcaggaggtg cctgcttcaa tgaaaaggca gactctcatc ttgtgtgtgt acagaatgat 3360
   gatggaaact atcagaccca ggctatcagt attcacaatc agcccagaaa agtgactggt 3420
   gccagtttct ttgtgttcag tggcgctctg aaatcctctt ctggatacct tgccaagtcc 3480
   agtattgtgg aagatggtgt tatggtccag attactgcag agaacatgga ttccttgagg 3540
   caggcactgc gagagatgaa ggacttcacc atcacctgtg ggaaggcgga cgcggaggaa 3600
   ccccaggagc acatccacat ccagtgggtg gatgatgaca agaacgttag caagggtgtc 3660
   gtaagtccta tagatgggaa gtccatggag actataacaa atgtgaagat attccatgga 3720
   tcagaatata aagcaaatgg aaaagtaatc agatggacag aggtgttttt cctagaaaac 3780
   gatgaccagc acaattgcct cagtgatcct gcagatcaca gtagattgac tgagcatgtt 3840
   gccaaagctt tttgccttgc tctctgtcct cacctgaaac ttctgaagga agatggaatg 3900
   accaaactgg gactacgtgt gacacttgac tcagatcagg ttggctatca agcagggagc 3960
   aatggccagc cccttccctc gcagtacatg aatgatctgg acagcgcctt ggtgccggtg 4020
   atccatggag gggcctgcca gcttagtgag ggccccgttg tcatggaact catcttttat 4080
   attctggaaa acatcgtata a 4101
<210> 114
   <211> 3656
   <212> DNA
   <213> Artificial Sequence
<220>
   <223> ZFYVE9 gene fragment
<400> 114
   atggagaatt acttccaagc agaagcttac aacctggaca aggtgttaga tgaatttgaa      60
   caaaacgaag atgaaacagt ttcttctact ttattggata caaagtggaa taagattcta     120
   gatcccccct ctcaccggct gtcatttaac cctactttgg ccagtgtgaa tgaatctgca    180
   gtttctaatg agtcacaacc acaactgaaa gtcttctccc tggctcattc agctcccctg     240
   accacagagg aagaggatca ctgtgctaat ggacaggact gtaatctaaa tccagagatt     300
   gccacaatgt ggattgatga aaatgctgtt gcagaagacc agttaattaa gagaaactat     360
   agttgggatg atcaatgcag tgctgttgaa gtgggagaga agaaatgtgg aaacctggct     420
   tgtctgccag atgagaagaa tgttcttgtt gtagccgtca tgcataactg tgataaaagg     480
   acattacaaa acgatttaca ggattgtaat aattataata gtcaatccct tatggatgct     540
   tttagctgtt cactggataa tgaaaacaga caaactgatc aatttagttt tagtataaat     600
   gagtccactg aaaaagatat gaattcagag aaacaaatgg atccattgaa tagaccgaaa     660
   acagagggga gatctgttaa ccatctgtgt cctacttcat ctgatagtct agccagtgtc    720
   tgttcccctt cacaattaaa ggatgacgga agtataggta gagacccctc catgtctgcg     780
   attacaagtt taacggttga ttcagtaatc tcatcccagg gaacagatgg atgtcctgct 840
   gttaaaaagc aagagaacta tataccagat gaggacctca ctggcaaaat cagctctcct 900
   aggacagatc tagggagtcc aaattccttt tcccacatga gtgaggggat tttgatgaaa 960
   aaagagccag cagaggagag caccactgaa gaatccctcc ggtctggttt acctttgctt 1020
   ctcaaaccag acatgcctaa tgggtctgga aggaataatg actgtgaacg gtgttcagat 1080
   tgccttgtgc ctaatgaagt tagggctgat gaaaatgaag gttatgaaca tgaagaaact 1140
   cttggcacta cagaattcct taatatgaca gagcatttct ctgaatctca ggacatgact 1200
   aattggaagt tgactaaact aaatgagatg aatgatagcc aagtaaacga agaaaaggaa 1260
   aagtttctac agattagtca gcctgaggac actaatggtg atagtggagg acagtgtgtt 1320
   ggattggcag atgcaggtct agatttaaaa ggaacttgca ttagtgaaag tgaagaatgt 1380
   gatttctcca ctgttataga cacaccagca gcaaattatc tatctaatgg ttgtgattcc 1440
   tatggaatgc aagacccagg tgtttctttt gttccaaaga ctttaccctc caaagaagat 1500
   tcagtaacag aagaaaaaga aatagaggaa agcaagtcag aatgctactc aaatatttat 1560
   gaacagagag gaaatgaggc cacagaaggg agtggactac ttttaaacag cactggtgac 1620
   ctaatgaaga aaaattattt acataatttc tgtagtcaag ttccatcagt gcttgggcaa 1680
   tcttccccca aggtagtagc aagcctgcca tctatcagtg ttccttttgg tggtgcaaga 1740
   cccaagcaac cttctaatct taaacttcaa attccaaagc cattatcaga ccatttacaa 1800
   aatgactttc ctgcaaacag tggaaataat actaaaaata aaaatgatat tcttgggaaa 1860
   gcaaaattag ggaaaaactc agcaaccaat gtatgcagtc catctttggg aaacatctct 1920
   aatgtcgata caaatgggga acatttagaa agttatgagg ctgagatctc cactagacca 1980
   tgccttgcat tagctccaga tagcccagat aatgatctca gagctggtca gtttggaatt 2040
   tctgccagaa agccattcac cactctgggt gaggtggctc cagtatgggt accggattct 2100
   caggctccaa attgcatgaa atgtgaagcc aggtttacat tcaccaaaag gaggcatcac 2160
   tgcagagcat gtgggaaggt tttctgtgct tcctgctgta gcctgaaatg taaactgtta 2220
   tacatggaca gaaaggaagc tagagtgtgt gtaatctgcc attcagtgct aatgaatgtg 2280
   gctcagccca gagagcagag gcgagtttgg tttgctgatg ggatcttgcc caatggagaa 2340
   gttgctgatg cagccaaatt aacaatgaat ggaacttcct ctgcaggaac cctggctgtg 2400
   tcacacgacc cagtcaagcc agtaactacc agtcctctac cagcagagac ggatatttgt 2460
   ctattctctg ggagtataac tcaggttgga agtcctgttg gaagtgcaat gaatcttatt 2520
   cctgaagatg gccttcctcc cattctcatc tccactggtg taaaaggaga ctatgctgtg 2580
   gaagagaaac catcacagat ttcagtaatg cagcagttgg aggatggtgg ccctgaccca 2640
   cttgtatttg ttttaaatgc aaatttgttg tcaatggtta aaattgtaaa ttatgtgaac 2700
   aggaagtgct ggtgtttcac aaccaaggga atgcatgcag tgggtcagtc tgagatagtc 2760
   attcttctac agtgtttacc ggatgaaaag tgtttgccaa aggatatctt taatcacttt 2820
   gtgcagcttt atcgggatgc tctggcaggg aatgtggtga gcaacttggg acattccttc 2880
   ttcagtcaaa gtttccttgg cagtaaagaa catggtggat tcttatatgt gacatctacc 2940
   taccagtcac tgcaagacct agtactccca accccacctt acttgtttgg gattcttatc 3000
   cagaaatggg aaactccttg ggctaaagta tttcctatcc gtctgatgtt gagacttgga 3060
   gctgaatatc gactttatcc atgcccacta ttcagtgtca gatttcggaa gccattgttt 3120
   ggagagacgg ggcataccat catgaatctt cttgcagact tcagaaatta ccagtatacc 3180
   ttgccagtag ttcaaggttt ggtggttgat atggaagttc ggaaaactag catcaaaatt 3240
   cccagcaaca gatacaatga gatgatgaaa gccatgaaca agtccaatga gcatgtcctg 3300
   gcaggaggtg cctgcttcaa tgaaaaggca gactctcatc ttgtgtgtgt acagaatgat 3360
   gatggaaact atcagaccca ggctatcagt attcacaatc agcccagaaa agtgactggt 3420
   gccagtttct ttgtgttcag tggcgctctg aaatcctctt ctggatacct tgccaagtcc 3480
   agtattgtgg aagatggtgt tatggtccag attactgcag agaacatgga ttccttgagg 3540
   caggcactgc gagagatgaa ggacttcacc atcacctgtg ggaaggcgga cgcggaggaa 3600
   ccccaggagc acatccacat ccagtgggtg gatgatgaca agaacgttag caaggg 3656
<210> 115
   <211> 20
   <212> DNA
   <213> Artificial Sequence
<220>
   <223> Break-point of ZFYVE9 gene fragment
<400> 115
   gacaagaacg ttagcaaggg 20
<210> 116
   <211> 1366
   <212> PRT
   <213> Artificial Sequence
<220>
   <223> ZFYVE9 protein
<400> 116
   Met Glu Asn Tyr Phe Gln Ala Glu Ala Tyr Asn Leu Asp Lys Val Leu
    1               5                  10                  15
   Asp Glu Phe Glu Gln Asn Glu Asp Glu Thr Val Ser Ser Thr Leu Leu
               20                  25                  30
   Asp Thr Lys Trp Asn Lys Ile Leu Asp Pro Pro Ser His Arg Leu Ser
           35                  40                  45
   Phe Asn Pro Thr Leu Ala Ser Val Asn Glu Ser Ala Val Ser Asn Glu
       50                  55                  60
   Ser Gln Pro Gln Leu Lys Val Phe Ser Leu Ala His Ser Ala Pro Leu
   65                  70                  75                  80
   Thr Thr Glu Glu Glu Asp His Cys Ala Asn Gly Gln Asp Cys Asn Leu
                   85                  90                  95
   Asn Pro Glu Ile Ala Thr Met Trp Ile Asp Glu Asn Ala Val Ala Glu
               100                 105                 110
   Asp Gln Leu Ile Lys Arg Asn Tyr Ser Trp Asp Asp Gln Cys Ser Ala
           115                 120                 125
   Val Glu Val Gly Glu Lys Lys Cys Gly Asn Leu Ala Cys Leu Pro Asp
       130                 135                 140
   Glu Lys Asn Val Leu Val Val Ala Val Met His Asn Cys Asp Lys Arg
   145                 150                 155                 160
   Thr Leu Gln Asn Asp Leu Gln Asp Cys Asn Asn Tyr Asn Ser Gln Ser
                   165                 170                 175
   Leu Met Asp Ala Phe Ser Cys Ser Leu Asp Asn Glu Asn Arg Gln Thr
               180                 185                 190
   Asp Gln Phe Ser Phe Ser Ile Asn Glu Ser Thr Glu Lys Asp Met Asn
           195                 200                 205
   Ser Glu Lys Gln Met Asp Pro Leu Asn Arg Pro Lys Thr Glu Gly Arg
       210                 215                 220
   Ser Val Asn His Leu Cys Pro Thr Ser Ser Asp Ser Leu Ala Ser Val
   225                 230                 235                 240
   Cys Ser Pro Ser Gln Leu Lys Asp Asp Gly Ser Ile Gly Arg Asp Pro
                   245                 250                 255
   Ser Met Ser Ala Ile Thr Ser Leu Thr Val Asp Ser Val Ile Ser Ser
               260                 265                 270
   Gln Gly Thr Asp Gly Cys Pro Ala Val Lys Lys Gln Glu Asn Tyr Ile
           275                 280                 285
   Pro Asp Glu Asp Leu Thr Gly Lys Ile Ser Ser Pro Arg Thr Asp Leu
       290                 295                 300
   Gly Ser Pro Asn Ser Phe Ser His Met Ser Glu Gly Ile Leu Met Lys
   305                 310                 315                 320
   Lys Glu Pro Ala Glu Glu Ser Thr Thr Glu Glu Ser Leu Arg Ser Gly
                   325                 330                 335
   Leu Pro Leu Leu Leu Lys Pro Asp Met Pro Asn Gly Ser Gly Arg Asn
               340                 345                 350
   Asn Asp Cys Glu Arg Cys Ser Asp Cys Leu Val Pro Asn Glu Val Arg
           355                 360                 365
   Ala Asp Glu Asn Glu Gly Tyr Glu His Glu Glu Thr Leu Gly Thr Thr
       370                 375                 380
   Glu Phe Leu Asn Met Thr Glu His Phe Ser Glu Ser Gln Asp Met Thr
   385                 390                 395                 400
   Asn Trp Lys Leu Thr Lys Leu Asn Glu Met Asn Asp Ser Gln Val Asn
                   405                 410                 415
   Glu Glu Lys Glu Lys Phe Leu Gln Ile Ser Gln Pro Glu Asp Thr Asn
               420                 425                 430
   Gly Asp Ser Gly Gly Gln Cys Val Gly Leu Ala Asp Ala Gly Leu Asp
           435                 440                 445
   Leu Lys Gly Thr Cys Ile Ser Glu Ser Glu Glu Cys Asp Phe Ser Thr
       450                 455                 460
   Val Ile Asp Thr Pro Ala Ala Asn Tyr Leu Ser Asn Gly Cys Asp Ser
   465                 470                 475                 480
   Tyr Gly Met Gln Asp Pro Gly Val Ser Phe Val Pro Lys Thr Leu Pro
                   485                 490                 495
   Ser Lys Glu Asp Ser Val Thr Glu Glu Lys Glu Ile Glu Glu Ser Lys
               500                 505                 510
   Ser Glu Cys Tyr Ser Asn Ile Tyr Glu Gln Arg Gly Asn Glu Ala Thr
           515                 520                 525
   Glu Gly Ser Gly Leu Leu Leu Asn Ser Thr Gly Asp Leu Met Lys Lys
       530                 535                 540
   Asn Tyr Leu His Asn Phe Cys Ser Gln Val Pro Ser Val Leu Gly Gln
   545                 550                 555                 560
   Ser Ser Pro Lys Val Val Ala Ser Leu Pro Ser Ile Ser Val Pro Phe
                   565                 570                 575
   Gly Gly Ala Arg Pro Lys Gln Pro Ser Asn Leu Lys Leu Gln Ile Pro
               580                 585                 590
   Lys Pro Leu Ser Asp His Leu Gln Asn Asp Phe Pro Ala Asn Ser Gly
           595                 600                 605
   Asn Asn Thr Lys Asn Lys Asn Asp Ile Leu Gly Lys Ala Lys Leu Gly
       610                 615                 620
   Glu Asn Ser Ala Thr Asn Val Cys Ser Pro Ser Leu Gly Asn Ile Ser
   625                 630                 635                 640
   Asn Val Asp Thr Asn Gly Glu His Leu Glu Ser Tyr Glu Ala Glu Ile
                   645                 650                 655
   Ser Thr Arg Pro Cys Leu Ala Leu Ala Pro Asp Ser Pro Asp Asn Asp
               660                 665                 670
   Leu Arg Ala Gly Gln Phe Gly Ile Ser Ala Arg Lys Pro Phe Thr Thr
           675                 680                 685
   Leu Gly Glu Val Ala Pro Val Trp Val Pro Asp Ser Gln Ala Pro Asn
       690                 695                 700
   Cys Met Lys Cys Glu Ala Arg Phe Thr Phe Thr Lys Arg Arg His His
   705                 710                 715                 720
   Cys Arg Ala Cys Gly Lys Val Phe Cys Ala Ser Cys Cys Ser Leu Lys
                   725                 730                 735
   Cys Lys Leu Leu Tyr Met Asp Arg Lys Glu Ala Arg Val Cys Val Ile
               740                 745                 750
   Cys His Ser Val Leu Met Asn Val Ala Gln Pro Arg Glu Gln Arg Arg
           755                 760                 765
   Val Trp Phe Ala Asp Gly Ile Leu Pro Asn Gly Glu Val Ala Asp Ala
       770                 775                 780
   Ala Lys Leu Thr Met Asn Gly Thr Ser Ser Ala Gly Thr Leu Ala Val
   785                 790                 795                 800
   Ser His Asp Pro Val Lys Pro Val Thr Thr Ser Pro Leu Pro Ala Glu
                   805                 810                 815
   Thr Asp Ile Cys Leu Phe Ser Gly Ser Ile Thr Gln Val Gly Ser Pro
               820                 825                 830
   Val Gly Ser Ala Met Asn Leu Ile Pro Glu Asp Gly Leu Pro Pro Ile
           835                 840                 845
   Leu Ile Ser Thr Gly Val Lys Gly Asp Tyr Ala Val Glu Glu Lys Pro
       850                 855                 860
   Ser Gln Ile Ser Val Met Gln Gln Leu Glu Asp Gly Gly Pro Asp Pro
   865                 870                 875                 880
   Leu Val Phe Val Leu Asn Ala Asn Leu Leu Ser Met Val Lys Ile Val
                   885                 890                 895
   Asn Tyr Val Asn Arg Lys Cys Trp Cys Phe Thr Thr Lys Gly Met His
               900                 905                 910
   Ala Val Gly Gln Ser Glu Ile Val Ile Leu Leu Gln Cys Leu Pro Asp
           915                 920                 925
   Glu Lys Cys Leu Pro Lys Asp Ile Phe Asn His Phe Val Gln Leu Tyr
       930                 935                 940
   Arg Asp Ala Leu Ala Gly Asn Val Val Ser Asn Leu Gly His Ser Phe
   945                 950                 955                 960
   Phe Ser Gln Ser Phe Leu Gly Ser Lys Glu His Gly Gly Phe Leu Tyr
                   965                 970                 975
   Val Thr Ser Thr Tyr Gln Ser Leu Gln Asp Leu Val Leu Pro Thr Pro
               980                 985                 990
   Pro Tyr Leu Phe Gly Ile Leu Ile Gln Lys Trp Glu Thr Pro Trp Ala
           995                 1000                1005
   Lys Val Phe Pro Ile Arg Leu Met Leu Arg Leu Gly Ala Glu Tyr Arg
      1010                 1015                1020
   Leu Tyr Pro Cys Pro Leu Phe Ser Val Arg Phe Arg Lys Pro Leu Phe
   1025                1030                1035                1040
   Gly Glu Thr Gly His Thr Ile Met Asn Leu Leu Ala Asp Phe Arg Asn
   1045 1050 1055
   Tyr Gln Tyr Thr Leu Pro Val Val Gln Gly Leu Val Val Asp Met Glu
   1060 1065 1070
   Val Arg Lys Thr Ser Ile Lys Ile Pro Ser Asn Arg Tyr Asn Glu Met
   1075 1080 1085
   Met Lys Ala Met Asn Lys Ser Asn Glu His Val Leu Ala Gly Gly Ala
   1090 1095 1100
   Cys Phe Asn Glu Lys Ala Asp Ser His Leu Val Cys Val Gln Asn Asp
   1105 1110 1115 1120
   Asp Gly Asn Tyr Gln Thr Gln Ala Ile Ser Ile His Asn Gln Pro Arg
   1125 1130 1135
   Lys Val Thr Gly Ala Ser Phe Phe Val Phe Ser Gly Ala Leu Lys Ser
   1140 1145 1150
   Ser Ser Gly Tyr Leu Ala Lys Ser Ser Ile Val Glu Asp Gly Val Met
   1155 1160 1165
   Val Gln Ile Thr Ala Glu Asn Met Asp Ser Leu Arg Gln Ala Leu Arg
   1170 1175 1180
   Glu Met Lys Asp Phe Thr Ile Thr Cys Gly Lys Ala Asp Ala Glu Glu
   1185 1190 1195 1200
   Pro Gln Glu His Ile His Ile Gln Trp Val Asp Asp Asp Lys Asn Val
   1205 1210 1215
   Ser Lys Gly Val Val Ser Pro Ile Asp Gly Lys Ser Met Glu Thr Ile
   1220 1225 1230
   Thr Asn Val Lys Ile Phe His Gly Ser Glu Tyr Lys Ala Asn Gly Lys
   1235 1240 1245
   Val Ile Arg Trp Thr Glu Val Phe Phe Leu Glu Asn Asp Asp Gln His
   1250 1255 1260
   Asn Cys Leu Ser Asp Pro Ala Asp His Ser Arg Leu Thr Glu His Val
   1265 1270 1275 1280
   Ala Lys Ala Phe Cys Leu Ala Leu Cys Pro His Leu Lys Leu Leu Lys
   1285 1290 1295
   Glu Asp Gly Met Thr Lys Leu Gly Leu Arg Val Thr Leu Asp Ser Asp
   1300 1305 1310
   Gln Val Gly Tyr Gln Ala Gly Ser Asn Gly Gln Pro Leu Pro Ser Gln
   1315 1320 1325
   Tyr Met Asn Asp Leu Asp Ser Ala Leu Val Pro Val Ile His Gly Gly
   1330 1335 1340
   Ala Cys Gln Leu Ser Glu Gly Pro Val Val Met Glu Leu Ile Phe Tyr
   1345 1350 1355 1360
   Ile Leu Glu Asn Ile Val
   1365
<210> 117
   <211> 1218
   <212> PRT
   <213> Artificial Sequence
<220>
   <223> ZFYVE9 protein fragment
<400> 117
   Met Glu Asn Tyr Phe Gln Ala Glu Ala Tyr Asn Leu Asp Lys Val Leu
   1               5                   10                  15
   Asp Glu Phe Glu Gln Asn Glu Asp Glu Thr Val Ser Ser Thr Leu Leu
               20                  25                  30
   Asp Thr Lys Trp Asn Lys Ile Leu Asp Pro Pro Ser His Arg Leu Ser
           35                  40                  45
   Phe Asn Pro Thr Leu Ala Ser Val Asn Glu Ser Ala Val Ser Asn Glu
       50                  55                  60
   Ser Gln Pro Gln Leu Lys Val Phe Ser Leu Ala His Ser Ala Pro Leu
   65                  70                  75                  80
   Thr Thr Glu Glu Glu Asp His Cys Ala Asn Gly Gln Asp Cys Asn Leu
                   85                  90                  95
   Asn Pro Glu Ile Ala Thr Met Trp Ile Asp Glu Asn Ala Val Ala Glu
               100                 105                 110
   Asp Gln Leu Ile Lys Arg Asn Tyr Ser Trp Asp Asp Gln Cys Ser Ala
           115                 120                 125
   Val Glu Val Gly Glu Lys Lys Cys Gly Asn Leu Ala Cys Leu Pro Asp
       130                 135                 140
   Glu Lys Asn Val Leu Val Val Ala Val Met His Asn Cys Asp Lys Arg
   145                 150                 155                 160
   Thr Leu Gln Asn Asp Leu Gln Asp Cys Asn Asn Tyr Asn Ser Gln Ser
                   165                 170                 175
   Leu Met Asp Ala Phe Ser Cys Ser Leu Asp Asn Glu Asn Arg Gln Thr
               180                 185                 190
   Asp Gln Phe Ser Phe Ser Ile Asn Glu Ser Thr Glu Lys Asp Met Asn
           195                 200                 205
   Ser Glu Lys Gln Met Asp Pro Leu Asn Arg Pro Lys Thr Glu Gly Arg
       210                 215                 220
   Ser Val Asn His Leu Cys Pro Thr Ser Ser Asp Ser Leu Ala Ser Val
   225                 230                 235                 240
   Cys Ser Pro Ser Gln Leu Lys Asp Asp Gly Ser Ile Gly Arg Asp Pro
                   245                 250                 255
   Ser Met Ser Ala Ile Thr Ser Leu Thr Val Asp Ser Val Ile Ser Ser
               260                 265                 270
   Gln Gly Thr Asp Gly Cys Pro Ala Val Lys Lys Gln Glu Asn Tyr Ile
           275                 280                 285
   Pro Asp Glu Asp Leu Thr Gly Lys Ile Ser Ser Pro Arg Thr Asp Leu
       290                 295                 300
   Gly Ser Pro Asn Ser Phe Ser His Met Ser Glu Gly Ile Leu Met Lys
   305                 310                 315                 320
   Lys Glu Pro Ala Glu Glu Ser Thr Thr Glu Glu Ser Leu Arg Ser Gly
                   325                 330                 335
   Leu Pro Leu Leu Leu Lys Pro Asp Met Pro Asn Gly Ser Gly Arg Asn
               340                 345                 350
   Asn Asp Cys Glu Arg Cys Ser Asp Cys Leu Val Pro Asn Glu Val Arg
           355                 360                 365
   Ala Asp Glu Asn Glu Gly Tyr Glu His Glu Glu Thr Leu Gly Thr Thr
       370                 375                 380
   Glu Phe Leu Asn Met Thr Glu His Phe Ser Glu Ser Gln Asp Met Thr
   385                 390                 395                 400
   Asn Trp Lys Leu Thr Lys Leu Asn Glu Met Asn Asp Ser Gln Val Asn
                   405                 410                 415
   Glu Glu Lys Glu Lys Phe Leu Gln Ile Ser Gln Pro Glu Asp Thr Asn
               420                 425                 430
   Gly Asp Ser Gly Gly Gln Cys Val Gly Leu Ala Asp Ala Gly Leu Asp
           435                 440                 445
   Leu Lys Gly Thr Cys Ile Ser Glu Ser Glu Glu Cys Asp Phe Ser Thr
       450                 455                 460
   Val Ile Asp Thr Pro Ala Ala Asn Tyr Leu Ser Asn Gly Cys Asp Ser
   465                 470                 475                 480
   Tyr Gly Met Gln Asp Pro Gly Val Ser Phe Val Pro Lys Thr Leu Pro
                   485                 490                 495
   Ser Lys Glu Asp Ser Val Thr Glu Glu Lys Glu Ile Glu Glu Ser Lys
               500                 505                 510
   Ser Glu Cys Tyr Ser Asn Ile Tyr Glu Gln Arg Gly Asn Glu Ala Thr
           515                 520                 525
   Glu Gly Ser Gly Leu Leu Leu Asn Ser Thr Gly Asp Leu Met Lys Lys
       530                 535                 540
   Asn Tyr Leu His Asn Phe Cys Ser Gln Val Pro Ser Val Leu Gly Gln
   545                 550                 555                 560
   Ser Ser Pro Lys Val Val Ala Ser Leu Pro Ser Ile Ser Val Pro Phe
                   565                 570                 575
   Gly Gly Ala Arg Pro Lys Gln Pro Ser Asn Leu Lys Leu Gln Ile Pro
               580                 585                 590
   Lys Pro Leu Ser Asp His Leu Gln Asn Asp Phe Pro Ala Asn Ser Gly
           595                 600                 605
   Asn Asn Thr Lys Asn Lys Asn Asp Ile Leu Gly Lys Ala Lys Leu Gly
   610 615 620
   Glu Asn Ser Ala Thr Asn Val Cys Ser Pro Ser Leu Gly Asn Ile Ser
   625 630 635 640
   Asn Val Asp Thr Asn Gly Glu His Leu Glu Ser Tyr Glu Ala Glu Ile
   645 650 655
   Ser Thr Arg Pro Cys Leu Ala Leu Ala Pro Asp Ser Pro Asp Asn Asp
   660 665 670
   Leu Arg Ala Gly Gln Phe Gly Ile Ser Ala Arg Lys Pro Phe Thr Thr
   675 680 685
   Leu Gly Glu Val Ala Pro Val Trp Val Pro Asp Ser Gln Ala Pro Asn
   690 695 700
   Cys Met Lys Cys Glu Ala Arg Phe Thr Phe Thr Lys Arg Arg His His
   705 710 715 720
   Cys Arg Ala Cys Gly Lys Val Phe Cys Ala Ser Cys Cys Ser Leu Lys
   725 730 735
   Cys Lys Leu Leu Tyr Met Asp Arg Lys Glu Ala Arg Val Cys Val Ile
   740 745 750
   Cys His Ser Val Leu Met Asn Val Ala Gln Pro Arg Glu Gln Arg Arg
   755 760 765
   Val Trp Phe Ala Asp Gly Ile Leu Pro Asn Gly Glu Val Ala Asp Ala
   770 775 780
   Ala Lys Leu Thr Met Asn Gly Thr Ser Ser Ala Gly Thr Leu Ala Val
   785 790 795 800
   Ser His Asp Pro Val Lys Pro Val Thr Thr Ser Pro Leu Pro Ala Glu
   805 810 815
   Thr Asp Ile Cys Leu Phe Ser Gly Ser Ile Thr Gln Val Gly Ser Pro
   820 825 830
   Val Gly Ser Ala Met Asn Leu Ile Pro Glu Asp Gly Leu Pro Pro Ile
   835 840 845
   Leu Ile Ser Thr Gly Val Lys Gly Asp Tyr Ala Val Glu Glu Lys Pro
   850 855 860
   Ser Gln Ile Ser Val Met Gln Gln Leu Glu Asp Gly Gly Pro Asp Pro
   865 870 875 880
   Leu Val Phe Val Leu Asn Ala Asn Leu Leu Ser Met Val Lys Ile Val
   885 890 895
   Asn Tyr Val Asn Arg Lys Cys Trp Cys Phe Thr Thr Lys Gly Met His
   900 905 910
   Ala Val Gly Gln Ser Glu Ile Val Ile Leu Leu Gln Cys Leu Pro Asp
   915 920 925
   Glu Lys Cys Leu Pro Lys Asp Ile Phe Asn His Phe Val Gln Leu Tyr
   930 935 940
   Arg Asp Ala Leu Ala Gly Asn Val Val Ser Asn Leu Gly His Ser Phe
   945                 950                 955                 960
   Phe Ser Gln Ser Phe Leu Gly Ser Lys Glu His Gly Gly Phe Leu Tyr
                   965                 970                 975
   Val Thr Ser Thr Tyr Gln Ser Leu Gln Asp Leu Val Leu Pro Thr Pro
               980                 985                 990
   Pro Tyr Leu Phe Gly Ile Leu Ile Gln Lys Trp Glu Thr Pro Trp Ala
           995                 1000                1005
   Lys Val Phe Pro Ile Arg Leu Met Leu Arg Leu Gly Ala Glu Tyr Arg
      1010                1015                1020
   Leu Tyr Pro Cys Pro Leu Phe Ser Val Arg Phe Arg Lys Pro Leu Phe
   1025                1030                1035                1040
   Gly Glu Thr Gly His Thr Ile Met Asn Leu Leu Ala Asp Phe Arg Asn
                  1045                1050                1055
   Tyr Gln Tyr Thr Leu Pro Val Val Gln Gly Leu Val Val Asp Met Glu
              1060                1065                1070
   Val Arg Lys Thr Ser Ile Lys Ile Pro Ser Asn Arg Tyr Asn Glu Met
          1075                1080                1085
   Met Lys Ala Met Asn Lys Ser Asn Glu His Val Leu Ala Gly Gly Ala
      1090                1095                1100
   Cys Phe Asn Glu Lys Ala Asp Ser His Leu Val Cys Val Gln Asn Asp
   1105                1110                1115                1120
   Asp Gly Asn Tyr Gln Thr Gln Ala Ile Ser Ile His Asn Gln Pro Arg
                  1125                1130                1135
   Lys Val Thr Gly Ala Ser Phe Phe Val Phe Ser Gly Ala Leu Lys Ser
              1140                1145                1150
   Ser Ser Gly Tyr Leu Ala Lys Ser Ser Ile Val Glu Asp Gly Val Met
          1155                1160                1165
   Val Gln Ile Thr Ala Glu Asn Met Asp Ser Leu Arg Gln Ala Leu Arg
      1170                1175                1180
   Glu Met Lys Asp Phe Thr Ile Thr Cys Gly Lys Ala Asp Ala Glu Glu
   1185                1190                1195                1200
   Pro Gln Glu His Ile His Ile Gln Trp Val Asp Asp Asp Lys Asn Val
                  1205                1210                1215
   Ser Lys
<210> 118
   <211> 6
   <212> PRT
   <213> Artificial Sequence
<220>
   <223> Break-point of ZFYVE9 protein fragment
<400> 118
   Asp Lys Asn Val Ser Lys
   1 5
<210> 119
   <211> 493
   <212> DNA
   <213> Artificial Sequence
<220>
   <223> CGA gene (NM_000735)
<400> 119
   acactctgct ggtataaaag caggtgagga cttcattaac tgcagttact gagaactcat      60
   aagacgaagc taaaatccct cttcggatcc acagtcaacc gccctgaaca catcctgcaa     120
   aaagcccaga gaaaggagcg ccatggatta ctacagaaaa tatgcagcta tctttctggt     180
   cacattgtcg gtgtttctgc atgttctcca ttccgctcct gatgtgcagg attgcccaga     240
   atgcacgcta caggaaaacc cattcttctc ccagccgggt gccccaatac ttcagtgcat     300
   gggctgctgc ttctctagag catatcccac tccactaagg tccaagaaga cgatgttggt     360
   ccaaaagaac gtcacctcag agtccacttg ctgtgtagct aaatcatata acagggtcac     420
   agtaatgggg ggtttcaaag tggagaacca cacggcgtgc cactgcagta cttgttatta     480
   tcacaaatct taa                                                        493
<210> 120
   <211> 358
   <212> DNA
   <213> Artificial Sequence
<220>
   <223> CGA gene fragment
<400> 120
   gagcgccatg gattactaca gaaaatatgc agctatcttt ctggtcacat tgtcggtgtt      60
   tctgcatgtt ctccattccg ctcctgatgt gcaggattgc ccagaatgca cgctacagga     120
   aaacccattc ttctcccagc cgggtgcccc aatacttcag tgcatgggct gctgcttctc     180
   tagagcatat cccactccac taaggtccaa gaagacgatg ttggtccaaa agaacgtcac     240
   ctcagagtcc acttgctgtg tagctaaatc atataacagg gtcacagtaa tggggggttt     300
   caaagtggag aaccacacgg cgtgccactg cagtacttgt tattatcaca aatcttaa       358
<210> 121
   <211> 7
   <212> DNA
   <213> Artificial Sequence
<220>
   <223> Break-point of CGA gene fragment
<400> 121
   gagcgcc 7
<210> 122
   <211> 116
   <212> PRT
   <213> Artificial Sequence
<220>
   <223> CGA protein
<400> 122
   Met Asp Tyr Tyr Arg Lys Tyr Ala Ala Ile Phe Leu Val Thr Leu Ser
   1               5                   10                  15
   Val Phe Leu His Val Leu His Ser Ala Pro Asp Val Gln Asp Cys Pro
               20                  25                  30
   Glu Cys Thr Leu Gln Glu Asn Pro Phe Phe Ser Gln Pro Gly Ala Pro
           35                  40                  45
   Ile Leu Gln Cys Met Gly Cys Cys Phe Ser Arg Ala Tyr Pro Thr Pro
       50                  55                  60
   Leu Arg Ser Lys Lys Thr Met Leu Val Gln Lys Asn Val Thr Ser Glu
   65                  70                  75                  80
   Ser Thr Cys Cys Val Ala Lys Ser Tyr Asn Arg Val Thr Val Met Gly
                   85                  90                  95
   Gly Phe Lys Val Glu Asn His Thr Ala Cys His Cys Ser Thr Cys Tyr
               100                 105                 110
   Tyr His Lys Ser
           115
<210> 123
   <211> 116
   <212> PRT
   <213> Artificial Sequence
<220>
   <223> CGA protein fragment
<400> 123
   Met Asp Tyr Tyr Arg Lys Tyr Ala Ala Ile Phe Leu Val Thr Leu Ser
   1               5                   10                  15
   Val Phe Leu His Val Leu His Ser Ala Pro Asp Val Gln Asp Cys Pro
               20                  25                  30
   Glu Cys Thr Leu Gln Glu Asn Pro Phe Phe Ser Gln Pro Gly Ala Pro
           35                  40                  45
   Ile Leu Gln Cys Met Gly Cys Cys Phe Ser Arg Ala Tyr Pro Thr Pro
       50                  55                  60
   Leu Arg Ser Lys Lys Thr Met Leu Val Gln Lys Asn Val Thr Ser Glu
   65                  70                  75                  80
   Ser Thr Cys Cys Val Ala Lys Ser Tyr Asn Arg Val Thr Val Met Gly
                   85                  90                  95
   Gly Phe Lys Val Glu Asn His Thr Ala Cys His Cys Ser Thr Cys Tyr
               100                 105                 110
   Tyr His Lys Ser
           115
<210> 124
   <211> 4014
   <212> DNA
   <213> Artificial Sequence
<220>
   <223> ZFYVE9-CGA fusion gene
<400> 124
   atggagaatt acttccaagc agaagcttac aacctggaca aggtgttaga tgaatttgaa         60
   caaaacgaag atgaaacagt ttcttctact ttattggata caaagtggaa taagattcta        120
   gatccccctt ctcaccggct gtcatttaac cctactttgg ccagtgtgaa tgaatctgca        180
   gtttctaatg agtcacaacc acaactgaaa gtcttctccc tggctcattc agctcccctg        240
   accacagagg aagaggatca ctgtgctaat ggacaggact gtaatctaaa tccagagatt        300
   gccacaatgt ggattgatga aaatgctgtt gcagaagacc agttaattaa gagaaactat        360
   agttgggatg atcaatgcag tgctgttgaa gtgggagaga agaaatgtgg aaacctggct        420
   tgtctgccag atgagaagaa tgttcttgtt gtagccgtca tgcataactg tgataaaagg        480
   acattacaaa acgatttaca ggattgtaat aattataata gtcaatccct tatggatgct        540
   tttagctgtt cactggataa tgaaaacaga caaactgatc aatttagttt tagtataaat        600
   gagtccactg aaaaagatat gaattcagag aaacaaatgg atccattgaa tagaccgaaa        660
   acagagggga gatctgttaa ccatctgtgt cctacttcat ctgatagtct agccagtgtc        720
   tgttcccctt cacaattaaa ggatgacgga agtataggta gagacccctc catgtctgcg        780
   attacaagtt taacggttga ttcagtaatc tcatcccagg gaacagatgg atgtcctgct        840
   gttaaaaagc aagagaacta tataccagat gaggacctca ctggcaaaat cagctctcct        900
   aggacagatc tagggagtcc aaattccttt tcccacatga gtgaggggat tttgatgaaa        960
   aaagagccag cagaggagag caccactgaa gaatccctcc ggtctggttt acctttgctt       1020
   ctcaaaccag acatgcctaa tgggtctgga aggaataatg actgtgaacg gtgttcagat       1080
   tgccttgtgc ctaatgaagt tagggctgat gaaaatgaag gttatgaaca tgaagaaact       1140
   cttggcacta cagaattcct taatatgaca gagcatttct ctgaatctca ggacatgact 1200
   aattggaagt tgactaaact aaatgagatg aatgatagcc aagtaaacga agaaaaggaa 1260
   aagtttctac agattagtca gcctgaggac actaatggtg atagtggagg acagtgtgtt 1320
   ggattggcag atgcaggtct agatttaaaa ggaacttgca ttagtgaaag tgaagaatgt 1380
   gatttctcca ctgttataga cacaccagca gcaaattatc tatctaatgg ttgtgattcc 1440
   tatggaatgc aagacccagg tgtttctttt gttccaaaga ctttaccctc caaagaagat 1500
   tcagtaacag aagaaaaaga aatagaggaa agcaagtcag aatgctactc aaatatttat 1560
   gaacagagag gaaatgaggc cacagaaggg agtggactac ttttaaacag cactggtgac 1620
   ctaatgaaga aaaattattt acataatttc tgtagtcaag ttccatcagt gcttgggcaa 1680
   tcttccccca aggtagtagc aagcctgcca tctatcagtg ttccttttgg tggtgcaaga 1740
   cccaagcaac cttctaatct taaacttcaa attccaaagc cattatcaga ccatttacaa 1800
   aatgactttc ctgcaaacag tggaaataat actaaaaata aaaatgatat tcttgggaaa 1860
   gcaaaattag gggaaaactc agcaaccaat gtatgcagtc catctttggg aaacatctct 1920
   aatgtcgata caaatgggga acatttagaa agttatgagg ctgagatctc cactagacca 1980
   tgccttgcat tagctccaga tagcccagat aatgatctca gagctggtca gtttggaatt 2040
   tctgccagaa agccattcac cactctgggt gaggtggctc cagtatgggt accggattct 2100
   caggctccaa attgcatgaa atgtgaagcc aggtttacat tcaccaaaag gaggcatcac 2160
   tgcagagcat gtgggaaggt tttctgtgct tcctgctgta gcctgaaatg taaactgtta 2220
   tacatggaca gaaaggaagc tagagtgtgt gtaatctgcc attcagtgct aatgaatgtg 2280
   gctcagccca gagagcagag gcgagtttgg tttgctgatg ggatcttgcc caatggagaa 2340
   gttgctgatg cagccaaatt aacaatgaat ggaacttcct ctgcaggaac cctggctgtg 2400
   tcacacgacc cagtcaagcc agtaactacc agtcctctac cagcagagac ggatatttgt 2460
   ctattctctg ggagtataac tcaggttgga agtcctgttg gaagtgcaat gaatcttatt 2520
   cctgaagatg gccttcctcc cattctcatc tccactggtg taaaaggaga ctatgctgtg 2580
   gaagagaaac catcacagat ttcagtaatg cagcagttgg aggatggtgg ccctgaccca 2640
   cttgtatttg ttttaaatgc aaatttgttg tcaatggtta aaattgtaaa ttatgtgaac 2700
   aggaagtgct ggtgtttcac aaccaaggga atgcatgcag tgggtcagtc tgagatagtc 2760
   attcttctac agtgtttacc ggatgaaaag tgtttgccaa aggatatctt taatcacttt 2820
   gtgcagcttt atcgggatgc tctggcaggg aatgtggtga gcaacttggg acattccttc 2880
   ttcagtcaaa gtttccttgg cagtaaagaa catggtggat tcttatatgt gacatctacc 2940
   taccagtcac tgcaagacct agtactccca accccacctt acttgtttgg gattcttatc 3000
   cagaaatggg aaactccttg ggctaaagta tttcctatcc gtctgatgtt gagacttgga 3060
   gctgaatatc gactttatcc atgcccacta ttcagtgtca gatttcggaa gccattgttt 3120
   ggagagacgg ggcataccat catgaatctt cttgcagact tcagaaatta ccagtatacc 3180
   ttgccagtag ttcaaggttt ggtggttgat atggaagttc ggaaaactag catcaaaatt 3240
   cccagcaaca gatacaatga gatgatgaaa gccatgaaca agtccaatga gcatgtcctg 3300
   gcaggaggtg cctgcttcaa tgaaaaggca gactctcatc ttgtgtgtgt acagaatgat 3360
   gatggaaact atcagaccca ggctatcagt attcacaatc agcccagaaa agtgactggt 3420
   gccagtttct ttgtgttcag tggcgctctg aaatcctctt ctggatacct tgccaagtcc 3480
   agtattgtgg aagatggtgt tatggtccag attactgcag agaacatgga ttccttgagg 3540
   caggcactgc gagagatgaa ggacttcacc atcacctgtg ggaaggcgga cgcggaggaa 3600
   ccccaggagc acatccacat ccagtgggtg gatgatgaca agaacgttag caaggggagc 3660
   gccatggatt actacagaaa atatgcagct atctttctgg tcacattgtc ggtgtttctg 3720
   catgttctcc attccgctcc tgatgtgcag gattgcccag aatgcacgct acaggaaaac 3780
   ccattcttct cccagccggg tgccccaata cttcagtgca tgggctgctg cttctctaga 3840
   gcatatccca ctccactaag gtccaagaag acgatgttgg tccaaaagaa cgtcacctca 3900
   gagtccactt gctgtgtagc taaatcatat aacagggtca cagtaatggg gggtttcaaa 3960
   gtggagaacc acacggcgtg ccactgcagt acttgttatt atcacaaatc ttaa 4014
<210> 125
   <211> 27
   <212> DNA
   <213> Artificial Sequence
<220>
   <223> Fused region of ZFYVE9-CGA fusion gene
<400> 125
   gacaagaacg ttagcaaggg gagcgcc 27
<210> 126
   <211> 1337
   <212> PRT
   <213> Artificial Sequence
<220>
   <223> ZFYVE9-CGA fusion protein
<400> 126
   Met Glu Asn Tyr Phe Gln Ala Glu Ala Tyr Asn Leu Asp Lys Val Leu
   1 5 10 15
   Asp Glu Phe Glu Gln Asn Glu Asp Glu Thr Val Ser Ser Thr Leu Leu
   20 25 30
   Asp Thr Lys Trp Asn Lys Ile Leu Asp Pro Pro Ser His Arg Leu Ser
           35                  40                  45
   Phe Asn Pro Thr Leu Ala Ser Val Asn Glu Ser Ala Val Ser Asn Glu
       50                  55                  60
   Ser Gln Pro Gln Leu Lys Val Phe Ser Leu Ala His Ser Ala Pro Leu
   65                  70                  75                  80
   Thr Thr Glu Glu Glu Asp His Cys Ala Asn Gly Gln Asp Cys Asn Leu
                   85                  90                  95
   Asn Pro Glu Ile Ala Thr Met Trp Ile Asp Glu Asn Ala Val Ala Glu
               100                 105                 110
   Asp Gln Leu Ile Lys Arg Asn Tyr Ser Trp Asp Asp Gln Cys Ser Ala
           115                 120                 125
   Val Glu Val Gly Glu Lys Lys Cys Gly Asn Leu Ala Cys Leu Pro Asp
       130                 135                 140
   Glu Lys Asn Val Leu Val Val Ala Val Met His Asn Cys Asp Lys Arg
   145                 150                 155                 160
   Thr Leu Gln Asn Asp Leu Gln Asp Cys Asn Asn Tyr Asn Ser Gln Ser
                   165                 170                 175
   Leu Met Asp Ala Phe Ser Cys Ser Leu Asp Asn Glu Asn Arg Gln Thr
               180                 185                 190
   Asp Gln Phe Ser Phe Ser Ile Asn Glu Ser Thr Glu Lys Asp Met Asn
           195                 200                 205
   Ser Glu Lys Gln Met Asp Pro Leu Asn Arg Pro Lys Thr Glu Gly Arg
       210                 215                 220
   Ser Val Asn His Leu Cys Pro Thr Ser Ser Asp Ser Leu Ala Ser Val
   225                 230                 235                 240
   Cys Ser Pro Ser Gln Leu Lys Asp Asp Gly Ser Ile Gly Arg Asp Pro
                   245                 250                 255
   Ser Met Ser Ala Ile Thr Ser Leu Thr Val Asp Ser Val Ile Ser Ser
               260                 265                 270
   Gln Gly Thr Asp Gly Cys Pro Ala Val Lys Lys Gln Glu Asn Tyr Ile
           275                 280                 285
   Pro Asp Glu Asp Leu Thr Gly Lys Ile Ser Ser Pro Arg Thr Asp Leu
       290                 295                 300
   Gly Ser Pro Asn Ser Phe Ser His Met Ser Glu Gly Ile Leu Met Lys
   305                 310                 315                 320
   Lys Glu Pro Ala Glu Glu Ser Thr Thr Glu Glu Ser Leu Arg Ser Gly
                   325                 330                 335
   Leu Pro Leu Leu Leu Lys Pro Asp Met Pro Asn Gly Ser Gly Arg Asn
               340                 345                 350
   Asn Asp Cys Glu Arg Cys Ser Asp Cys Leu Val Pro Asn Glu Val Arg
           355                 360                 365
   Ala Asp Glu Asn Glu Gly Tyr Glu His Glu Glu Thr Leu Gly Thr Thr
       370                 375                 380
   Glu Phe Leu Asn Met Thr Glu His Phe Ser Glu Ser Gln Asp Met Thr
   385                 390                 395                 400
   Asn Trp Lys Leu Thr Lys Leu Asn Glu Met Asn Asp Ser Gln Val Asn
                   405                 410                 415
   Glu Glu Lys Glu Lys Phe Leu Gln Ile Ser Gln Pro Glu Asp Thr Asn
               420                 425                 430
   Gly Asp Ser Gly Gly Gln Cys Val Gly Leu Ala Asp Ala Gly Leu Asp
           435                 440                 445
   Leu Lys Gly Thr Cys Ile Ser Glu Ser Glu Glu Cys Asp Phe Ser Thr
       450                 455                 460
   Val Ile Asp Thr Pro Ala Ala Asn Tyr Leu Ser Asn Gly Cys Asp Ser
   465                 470                 475                 480
   Tyr Gly Met Gln Asp Pro Gly Val Ser Phe Val Pro Lys Thr Leu Pro
                   485                 490                 495
   Ser Lys Glu Asp Ser Val Thr Glu Glu Lys Glu Ile Glu Glu Ser Lys
               500                 505                 510
   Ser Glu Cys Tyr Ser Asn Ile Tyr Glu Gln Arg Gly Asn Glu Ala Thr
           515                 520                 525
   Glu Gly Ser Gly Leu Leu Leu Asn Ser Thr Gly Asp Leu Met Lys Lys
       530                 535                 540
   Asn Tyr Leu His Asn Phe Cys Ser Gln Val Pro Ser Val Leu Gly Gln
   545                 550                 555                 560
   Ser Ser Pro Lys Val Val Ala Ser Leu Pro Ser Ile Ser Val Pro Phe
                   565                 570                 575
   Gly Gly Ala Arg Pro Lys Gln Pro Ser Asn Leu Lys Leu Gln Ile Pro
               580                 585                 590
   Lys Pro Leu Ser Asp His Leu Gln Asn Asp Phe Pro Ala Asn Ser Gly
           595                 600                 605
   Asn Asn Thr Lys Asn Lys Asn Asp Ile Leu Gly Lys Ala Lys Leu Gly
       610                 615                 620
   Glu Asn Ser Ala Thr Asn Val Cys Ser Pro Ser Leu Gly Asn Ile Ser
   625                 630                 635                 640
   Asn Val Asp Thr Asn Gly Glu His Leu Glu Ser Tyr Glu Ala Glu Ile
                   645                 650                 655
   Ser Thr Arg Pro Cys Leu Ala Leu Ala Pro Asp Ser Pro Asp Asn Asp
               660                 665                 670
   Leu Arg Ala Gly Gln Phe Gly Ile Ser Ala Arg Lys Pro Phe Thr Thr
           675                 680                 685
   Leu Gly Glu Val Ala Pro Val Trp Val Pro Asp Ser Gln Ala Pro Asn
       690                 695                 700
   Cys Met Lys Cys Glu Ala Arg Phe Thr Phe Thr Lys Arg Arg His His
   705                 710                 715                 720
   Cys Arg Ala Cys Gly Lys Val Phe Cys Ala Ser Cys Cys Ser Leu Lys
                   725                 730                 735
   Cys Lys Leu Leu Tyr Met Asp Arg Lys Glu Ala Arg Val Cys Val Ile
               740                 745                 750
   Cys His Ser Val Leu Met Asn Val Ala Gln Pro Arg Glu Gln Arg Arg
           755                 760                 765
   Val Trp Phe Ala Asp Gly Ile Leu Pro Asn Gly Glu Val Ala Asp Ala
       770                 775                 780
   Ala Lys Leu Thr Met Asn Gly Thr Ser Ser Ala Gly Thr Leu Ala Val
   785                 790                 795                 800
   Ser His Asp Pro Val Lys Pro Val Thr Thr Ser Pro Leu Pro Ala Glu
                   805                 810                 815
   Thr Asp Ile Cys Leu Phe Ser Gly Ser Ile Thr Gln Val Gly Ser Pro
               820                 825                 830
   Val Gly Ser Ala Met Asn Leu Ile Pro Glu Asp Gly Leu Pro Pro Ile
           835                 840                 845
   Leu Ile Ser Thr Gly Val Lys Gly Asp Tyr Ala Val Glu Glu Lys Pro
       850                 855                 860
   Ser Gln Ile Ser Val Met Gln Gln Leu Glu Asp Gly Gly Pro Asp Pro
   865                 870                 875                 880
   Leu Val Phe Val Leu Asn Ala Asn Leu Leu Ser Met Val Lys Ile Val
                   885                 890                 895
   Asn Tyr Val Asn Arg Lys Cys Trp Cys Phe Thr Thr Lys Gly Met His
               900                 905                 910
   Ala Val Gly Gln Ser Glu Ile Val Ile Leu Leu Gln Cys Leu Pro Asp
           915                 920                 925
   Glu Lys Cys Leu Pro Lys Asp Ile Phe Asn His Phe Val Gln Leu Tyr
       930                 935                 940
   Arg Asp Ala Leu Ala Gly Asn Val Val Ser Asn Leu Gly His Ser Phe
   945                 950                 955                 960
   Phe Ser Gln Ser Phe Leu Gly Ser Lys Glu His Gly Gly Phe Leu Tyr
                   965                 970                 975
   Val Thr Ser Thr Tyr Gln Ser Leu Gln Asp Leu Val Leu Pro Thr Pro
               980                 985                 990
   Pro Tyr Leu Phe Gly Ile Leu Ile Gln Lys Trp Glu Thr Pro Trp Ala
           995                 1000                1005
   Lys Val Phe Pro Ile Arg Leu Met Leu Arg Leu Gly Ala Glu Tyr Arg
      1010                1015                1020
   Leu Tyr Pro Cys Pro Leu Phe Ser Val Arg Phe Arg Lys Pro Leu Phe
   1025                1030                1035                1040
   Gly Glu Thr Gly His Thr Ile Met Asn Leu Leu Ala Asp Phe Arg Asn
   1045 1050 1055
   Tyr Gln Tyr Thr Leu Pro Val Val Gln Gly Leu Val Val Asp Met Glu
   1060 1065 1070
   Val Arg Lys Thr Ser Ile Lys Ile Pro Ser Asn Arg Tyr Asn Glu Met
   1075 1080 1085
   Met Lys Ala Met Asn Lys Ser Asn Glu His Val Leu Ala Gly Gly Ala
   1090 1095 1100
   Cys Phe Asn Glu Lys Ala Asp Ser His Leu Val Cys Val Gln Asn Asp
   1105 1110 1115 1120
   Asp Gly Asn Tyr Gln Thr Gln Ala Ile Ser Ile His Asn Gln Pro Arg
   1125 1130 1135
   Lys Val Thr Gly Ala Ser Phe Phe Val Phe Ser Gly Ala Leu Lys Ser
   1140 1145 1150
   Ser Ser Gly Tyr Leu Ala Lys Ser Ser Ile Val Glu Asp Gly Val Met
   1155 1160 1165
   Val Gln Ile Thr Ala Glu Asn Met Asp Ser Leu Arg Gln Ala Leu Arg
   1170 1175 1180
   Glu Met Lys Asp Phe Thr Ile Thr Cys Gly Lys Ala Asp Ala Glu Glu
   1185 1190 1195 1200
   Pro Gln Glu His Ile His Ile Gln Trp Val Asp Asp Asp Lys Asn Val
   1205 1210 1215
   Ser Lys Gly Ser Ala Met Asp Tyr Tyr Arg Lys Tyr Ala Ala Ile Phe
   1220 1225 1230
   Leu Val Thr Leu Ser Val Phe Leu His Val Leu His Ser Ala Pro Asp
   1235 1240 1245
   Val Gln Asp Cys Pro Glu Cys Thr Leu Gln Glu Asn Pro Phe Phe Ser
   1250 1255 1260
   Gln Pro Gly Ala Pro Ile Leu Gln Cys Met Gly Cys Cys Phe Ser Arg
   1265 1270 1275 1280
   Ala Tyr Pro Thr Pro Leu Arg Ser Lys Lys Thr Met Leu Val Gln Lys
   1285 1290 1295
   Asn Val Thr Ser Glu Ser Thr Cys Cys Val Ala Lys Ser Tyr Asn Arg
   1300 1305 1310
   Val Thr Val Met Gly Gly Phe Lys Val Glu Asn His Thr Ala Cys His
   1315 1320 1325
   Cys Ser Thr Cys Tyr Tyr His Lys Ser
   1330 1335
<210> 127
   <211> 9
   <212> PRT
   <213> Artificial Sequence
<220>
   <223> Fused region of ZFYVE9-CGA fusion protein
<400> 127
   Asp Lys Asn Val Ser Lys Gly Ser Ala
   1               5
<210> 128
   <211> 3909
   <212> DNA
   <213> Artificial Sequence
<220>
   <223> CDS of ERBB2IP gene (NM_001006600)
<400> 128
   atgactacaa aacgaagttt gtttgtgcgg ttggtaccat gtcgctgtct acgaggggaa      60
   gaggagactg tcactactct tgattattct cattgcagct tagaacaagt tccgaaagag     120
   atttttactt ttgaaaaaac cttggaggaa ctctatttag atgctaatca gattgaagag     180
   cttccaaagc aactttttaa ctgtcagtct ttacacaaac tgagtttgcc agacaatgat     240
   ttaacaacgt taccagcatc cattgcaaac cttattaatc tcagggaact ggatgtcagc     300
   aagaatggaa tacaggagtt tccagaaaat ataaaaaatt gtaaagtttt gacaattgtg     360
   gaggccagtg taaaccctat ttccaagctc cctgatggat tttctcagct gttaaaccta     420
   acccagttgt atctgaatga tgcttttctt gagttcttgc cagcaaattt tggcagatta     480
   actaaactcc aaatattaga gcttagagaa aaccagttaa aaatgttgcc taaaactatg     540
   aatagactga cccagctgga aagactggat ttgggaagta acgaattcac ggaagtgcct     600
   gaagtacttg agcaactaag tggattgaaa gagttttgga tggatgctaa tagactgact     660
   tttattccag ggtttattgg tagtttgaaa cagctcacat atttggatgt ttctaaaaat     720
   aatattgaaa tggttgaaga aggaatttca acatgtgaaa accttcaaga cctcctatta     780
   tcaagcaatt cacttcagca gcttcctgag actattggtt cgttgaagaa tataacaacg     840
   cttaaaatag atgaaaacca gttaatgtat ctgccagact ctataggagg gttaatatca     900
   gtagaagaac tggattgtag tttcaatgaa gttgaagctt tgccttcatc tattgggcag     960
   cttactaact taagaacttt tgctgctgat cataattact tacagcagtt gcccccagag    1020
   attggaagct ggaaaaatat aactgtgctg tttctccatt ccaataaact tgagacactt    1080
   ccagaggaaa tgggtgatat gcaaaaatta aaagtcatta atttaagtga taatagatta    1140
   aagaatttac cctttagctt tacaaagcta cagcaattga cagctatgtg gctctcagat    1200
   aatcagtcca aacccctgat acctcttcaa aaagaaactg attcagagac ccagaaaatg    1260
   gtgcttacca actacatgtt ccctcaacag ccaaggactg aggatgttat gtttatatca    1320
   gataatgaaa gttttaaccc ttcattgtgg gaggaacaga ggaaacagcg ggctcaagtt 1380
   gcatttgaat gtgatgaaga caaagatgaa agggaggcac ctcccaggga gggaaattta 1440
   aaaagatatc caacaccata cccagatgag cttaagaata tggtcaaaac tgttcaaacc 1500
   attgtacata gattaaaaga tgaagagacc aatgaagact caggaagaga tttgaaacca 1560
   catgaagatc aacaagatat aaataaagat gtgggtgtga agacctcaga aagtactact 1620
   acagtaaaaa gcaaagttga tgaaagagaa aaatatatga taggaaactc tgtacagaag 1680
   atcagtgaac ctgaagctga gattagtcct gggagtttac cagtgactgc aaatatgaaa 1740
   gcctctgaga acttgaagca tattgttaac catgatgatg tttttgagga atctgaagaa 1800
   ctttcttctg atgaagagat gaaaatggcg gagatgcgac caccattaat tgaaacctct 1860
   attaaccagc caaaagtcgt agcacttagt aataacaaaa aagatgatac aaaggaaaca 1920
   gattctttat cagatgaagt tacacacaat agcaatcaga ataacagcaa ttgttcttct 1980
   ccatctcgga tgtctgattc agtttctctt aatactgata gtagtcaaga cacctcactc 2040
   tgctctccag tgaaacaaac tcatattgat attaattcca aaatcaggca agaagatgaa 2100
   aattttaaca gccttttaca aaatggagat attttaaaca gttcaacaga ggaaaagttc 2160
   aaagctcatg ataaaaaaga ttttaactta cctgaatatg atttgaatgt tgaagagcga 2220
   ttagttctaa ttgagaaaag tgttgactca acagccacag ctgatgacac tcacaaatta 2280
   gatcatatca atatgaatct taataaactt ataactaatg atacatttca accagagatc 2340
   atggaaagat caaaaacaca ggatattgtg cttggaacaa gctttttaag cattaattct 2400
   aaagaggaaa ctgagcactt ggaaaatgga aacaagtatc ctaatttgga atccgtaaat 2460
   aaggtaaatg gacattctga ggaaacttcc cagtctccta ataggactga accacatgac 2520
   agtgattgtt ctgttgactt aggtatttcc aaaagcactg aagatctctc ccctcagaaa 2580
   agtggtccag ttggatctgt tgtgaaatct catagcataa ctaatatgga gattggaggg 2640
   ctaaaaatct atgatattct tagtgataat ggacctcagc agccaagtac aaccgttaaa 2700
   atcacatctg ctgttgatgg aaaaaatata gtcaggagca agtctgccac actgttgtat 2760
   gatcaaccat tgcaggtatt tactggttct tcctcatctt ctgatttaat atcaggaaca 2820
   aaggcaattt tcaagtttga ttcaaatcat aatcccgaag agccaaatat aataagaggc 2880
   cccacaagtg gcccacaatc tgcacctcaa atatatggtc ctccacagta taatatccaa 2940
   tacagtagca gtgctgcagt caaagacact ttgtggcact ccaaacaaaa tccccaaata 3000
   gaccatgcca gttttcctcc tcagctcctt cctagatcag agagcacaga aaatcaaagt 3060
   tatgctaaac attctgccaa tatgaatttc tctaatcata acaatgttcg agctaatact 3120
   gcataccatt tacatcagag acttggccca gcaagacatg gggaaatgtg ggccatctca 3180
   ccaaacgacc gacttattcc tgcagtaact cgaagtacaa tccagcgaca aagtagtgtg 3240
   tcctccacag cctctgtaaa tcttggtgat ccaggctcta caaggcgggc tcagattcct    3300
   gaaggagatt atttatcata cagagagttc cactcagcgg gaagaactcc tccaatgatg    3360
   ccaggatcac agagacccct ttctgcacga acatacagca tagatggtcc aaatgcatca    3420
   agacctcaga gtgctcgacc ctctattaat gaaataccag agagaactat gtcagttagt    3480
   gatttcaatt attcacggac tagtccttca aaaagaccaa atgcaagggt tggttctgag    3540
   cattctttat tagatcctcc aggaaaaagt aaagttcctc gtgactggag agaacaagta    3600
   cttcgacata ttgaagccaa aaagttagaa aagattcgag tgagggttga aaaggatcca    3660
   gaacttggat ttagcatatc aggtggtgtc gggggtagag gaaacccatt cagacctgat    3720
   gatgatggta tatttgtaac aagggtacaa cctgaaggac cagcatcaaa attactgcag    3780
   ccaggtgata aaattattca ggctaatggc tacagtttta taaatattga acatggacaa    3840
   gcagtgtcct tgctaaaaac tttccagaat acagttgaac tcatcattgt acgagaagtt    3900
   tcctcataa                                                            3909
<210> 129
   <211> 3801
   <212> DNA
   <213> Artificial Sequence
<220>
   <223> ERBB2IP gene fragment
<400> 129
   atgactacaa aacgaagttt gtttgtgcgg ttggtaccat gtcgctgtct acgaggggaa      60
   gaggagactg tcactactct tgattattct cattgcagct tagaacaagt tccgaaagag     120
   atttttactt ttgaaaaaac cttggaggaa ctctatttag atgctaatca gattgaagag     180
   cttccaaagc aactttttaa ctgtcagtct ttacacaaac tgagtttgcc agacaatgat     240
   ttaacaacgt taccagcatc cattgcaaac cttattaatc tcagggaact ggatgtcagc     300
   aagaatggaa tacaggagtt tccagaaaat ataaaaaatt gtaaagtttt gacaattgtg     360
   gaggccagtg taaaccctat ttccaagctc cctgatggat tttctcagct  gttaaaccta     420
   acccagttgt atctgaatga tgcttttctt gagttcttgc cagcaaattt tggcagatta     480
   actaaactcc aaatattaga gcttagagaa aaccagttaa aaatgttgcc taaaactatg     540
   aatagactga cccagctgga aagactggat ttgggaagta acgaattcac ggaagtgcct     600
   gaagtacttg agcaactaag tggattgaaa gagttttgga tggatgctaa tagactgact     660
   tttattccag ggtttattgg tagtttgaaa cagctcacat atttggatgt ttctaaaaat     720
   aatattgaaa tggttgaaga aggaatttca acatgtgaaa accttcaaga cctcctatta     780
   tcaagcaatt cacttcagca gcttcctgag actattggtt cgttgaagaa tataacaacg     840
   cttaaaatag atgaaaacca gttaatgtat ctgccagact ctataggagg gttaatatca 900
   gtagaagaac tggattgtag tttcaatgaa gttgaagctt tgccttcatc tattgggcag 960
   cttactaact taagaacttt tgctgctgat cataattact tacagcagtt gcccccagag 1020
   attggaagct ggaaaaatat aactgtgctg tttctccatt ccaataaact tgagacactt 1080
   ccagaggaaa tgggtgatat gcaaaaatta aaagtcatta atttaagtga taatagatta 1140
   aagaatttac cctttagctt tacaaagcta cagcaattga cagctatgtg gctctcagat 1200
   aatcagtcca aacccctgat acctcttcaa aaagaaactg attcagagac ccagaaaatg 1260
   gtgcttacca actacatgtt ccctcaacag ccaaggactg aggatgttat gtttatatca 1320
   gataatgaaa gttttaaccc ttcattgtgg gaggaacaga ggaaacagcg ggctcaagtt 1380
   gcatttgaat gtgatgaaga caaagatgaa agggaggcac ctcccaggga gggaaattta 1440
   aaaagatatc caacaccata cccagatgag cttaagaata tggtcaaaac tgttcaaacc 1500
   attgtacata gattaaaaga tgaagagacc aatgaagact caggaagaga tttgaaacca 1560
   catgaagatc aacaagatat aaataaagat gtgggtgtga agacctcaga aagtactact 1620
   acagtaaaaa gcaaagttga tgaaagagaa aaatatatga taggaaactc tgtacagaag 1680
   atcagtgaac ctgaagctga gattagtcct gggagtttac cagtgactgc aaatatgaaa 1740
   gcctctgaga acttgaagca tattgttaac catgatgatg tttttgagga atctgaagaa 1800
   ctttcttctg atgaagagat gaaaatggcg gagatgcgac caccattaat tgaaacctct 1860
   attaaccagc caaaagtcgt agcacttagt aataacaaaa aagatgatac aaaggaaaca 1920
   gattctttat cagatgaagt tacacacaat agcaatcaga ataacagcaa ttgttcttct 1980
   ccatctcgga tgtctgattc agtttctctt aatactgata gtagtcaaga cacctcactc 2040
   tgctctccag tgaaacaaac tcatattgat attaattcca aaatcaggca agaagatgaa 2100
   aattttaaca gccttttaca aaatggagat attttaaaca gttcaacaga ggaaaagttc 2160
   aaagctcatg ataaaaaaga ttttaactta cctgaatatg atttgaatgt tgaagagcga 2220
   ttagttctaa ttgagaaaag tgttgactca acagccacag ctgatgacac tcacaaatta 2280
   gatcatatca atatgaatct taataaactt ataactaatg atacatttca accagagatc 2340
   atggaaagat caaaaacaca ggatattgtg cttggaacaa gctttttaag cattaattct 2400
   aaagaggaaa ctgagcactt ggaaaatgga aacaagtatc ctaatttgga atccgtaaat 2460
   aaggtaaatg gacattctga ggaaacttcc cagtctccta ataggactga accacatgac 2520
   agtgattgtt ctgttgactt aggtatttcc aaaagcactg aagatctctc ccctcagaaa 2580
   agtggtccag ttggatctgt tgtgaaatct catagcataa ctaatatgga gattggaggg 2640
   ctaaaaatct atgatattct tagtgataat ggacctcagc agccaagtac aaccgttaaa 2700
   atcacatctg ctgttgatgg aaaaaatata gtcaggagca agtctgccac actgttgtat      2760
   gatcaaccat tgcaggtatt tactggttct tcctcatctt ctgatttaat atcaggaaca      2820
   aaggcaattt tcaagtttga ttcaaatcat aatcccgaag agccaaatat aataagaggc      2880
   cccacaagtg gcccacaatc tgcacctcaa atatatggtc ctccacagta taatatccaa      2940
   tacagtagca gtgctgcagt caaagacact ttgtggcact ccaaacaaaa tccccaaata      3000
   gaccatgcca gttttcctcc tcagctcctt cctagatcag agagcacaga aaatcaaagt      3060
   tatgctaaac attctgccaa tatgaatttc tctaatcata acaatgttcg agctaatact      3120
   gcataccatt tacatcagag acttggccca gcaagacatg ggaaatgtgg ggccatctca      3180
   ccaaacgacc gacttattcc tgcagtaact cgaagtacaa tccagcgaca aagtagtgtg      3240
   tcctccacag cctctgtaaa tcttggtgat ccaggctcta caaggcgggc tcagattcct      3300
   gaaggagatt atttatcata cagagagttc cactcagcgg gaagaactcc tccaatgatg      3360
   ccaggatcac agagacccct ttctgcacga acatacagca tagatggtcc aaatgcatca      3420
   agacctcaga gtgctcgacc ctctattaat gaaataccag agagaactat gtcagttagt      3480
   gatttcaatt attcacggac tagtccttca aaaagaccaa atgcaagggt tggttctgag      3540
   cattctttat tagatcctcc aggaaaaagt aaagttcctc gtgactggag agaacaagta      3600
   cttcgacata ttgaagccaa aaagttagaa aagattcgag tgagggttga aaaggatcca      3660
   gaacttggat ttagcatatc aggtggtgtc gggggtagag gaaacccatt cagacctgat      3720
   gatgatggta tatttgtaac aagggtacaa cctgaaggac cagcatcaaa attactgcag      3780
   ccaggtgata aaattattca g                                                3801
<210> 130
   <211> 24
   <212> DNA
   <213> Artificial Sequence
<220>
   <223> Break-point of ERBB2IP gene fragment
<400> 130
   cagccaggtg ataaaattat tcag 24
<210> 131
   <211> 1302
   <212> PRT
   <213> Artificial Sequence
<220>
   <223> ERBB2IP protein
<400> 131
   Met Thr Thr Lys Arg Ser Leu Phe Val Arg Leu Val Pro Cys Arg Cys
   1               5                   10                  15
   Leu Arg Gly Glu Glu Glu Thr Val Thr Thr Leu Asp Tyr Ser His Cys
               20                  25                  30
   Ser Leu Glu Gln Val Pro Lys Glu Ile Phe Thr Phe Glu Lys Thr Leu
           35                  40                  45
   Glu Glu Leu Tyr Leu Asp Ala Asn Gln Ile Glu Glu Leu Pro Lys Gln
       50                  55                  60
   Leu Phe Asn Cys Gln Ser Leu His Lys Leu Ser Leu Pro Asp Asn Asp
   65                  70                  75                  80
   Leu Thr Thr Leu Pro Ala Ser Ile Ala Asn Leu Ile Asn Leu Arg Glu
                   85                  90                  95
   Leu Asp Val Ser Lys Asn Gly Ile Gln Glu Phe Pro Glu Asn Ile Lys
               100                 105                 110
   Asn Cys Lys Val Leu Thr Ile Val Glu Ala Ser Val Asn Pro Ile Ser
           115                 120                 125
   Lys Leu Pro Asp Gly Phe Ser Gln Leu Leu Asn Leu Thr Gln Leu Tyr
       130                 135                 140
   Leu Asn Asp Ala Phe Leu Glu Phe Leu Pro Ala Asn Phe Gly Arg Leu
   145                 150                 155                 160
   Thr Lys Leu Gln Ile Leu Glu Leu Arg Glu Asn Gln Leu Lys Met Leu
                   165                 170                 175
   Pro Lys Thr Met Asn Arg Leu Thr Gln Leu Glu Arg Leu Asp Leu Gly
               180                 185                 190
   Ser Asn Glu Phe Thr Glu Val Pro Glu Val Leu Glu Gln Leu Ser Gly
           195                 200                 205
   Leu Lys Glu Phe Trp Met Asp Ala Asn Arg Leu Thr Phe Ile Pro Gly
       210                 215                 220
   Phe Ile Gly Ser Leu Lys Gln Leu Thr Tyr Leu Asp Val Ser Lys Asn
   225                 230                 235                 240
   Asn Ile Glu Met Val Glu Glu Gly Ile Ser Thr Cys Glu Asn Leu Gln
                   245                 250                 255
   Asp Leu Leu Leu Ser Ser Asn Ser Leu Gln Gln Leu Pro Glu Thr Ile
               260                 265                 270
   Gly Ser Leu Lys Asn Ile Thr Thr Leu Lys Ile Asp Glu Asn Gln Leu
           275                 280                 285
   Met Tyr Leu Pro Asp Ser Ile Gly Gly Leu Ile Ser Val Glu Glu Leu
       290                 295                 300
   Asp Cys Ser Phe Asn Glu Val Glu Ala Leu Pro Ser Ser Ile Gly Gln
   305                 310                 315                 320
   Leu Thr Asn Leu Arg Thr Phe Ala Ala Asp His Asn Tyr Leu Gln Gln
                   325                 330                 335
   Leu Pro Pro Glu Ile Gly Ser Trp Lys Asn Ile Thr Val Leu Phe Leu
   340 345 350
   His Ser Asn Lys Leu Glu Thr Leu Pro Glu Glu Met Gly Asp Met Gln
   355 360 365
   Lys Leu Lys Val Ile Asn Leu Ser Asp Asn Arg Leu Lys Asn Leu Pro
   370 375 380
   Phe Ser Phe Thr Lys Leu Gln Gln Leu Thr Ala Met Trp Leu Ser Asp
   385 390 395 400
   Asn Gln Ser Lys Pro Leu Ile Pro Leu Gln Lys Glu Thr Asp Ser Glu
   405 410 415
   Thr Gln Lys Met Val Leu Thr Asn Tyr Met Phe Pro Gln Gln Pro Arg
   420 425 430
   Thr Glu Asp Val Met Phe Ile Ser Asp Asn Glu Ser Phe Asn Pro Ser
   435 440 445
   Leu Trp Glu Glu Gln Arg Lys Gln Arg Ala Gln Val Ala Phe Glu Cys
   450 455 460
   Asp Glu Asp Lys Asp Glu Arg Glu Ala Pro Pro Arg Glu Gly Asn Leu
   465 470 475 480
   Lys Arg Tyr Pro Thr Pro Tyr Pro Asp Glu Leu Lys Asn Met Val Lys
   485 490 495
   Thr Val Gln Thr Ile Val His Arg Leu Lys Asp Glu Glu Thr Asn Glu
   500 505 510
   Asp Ser Gly Arg Asp Leu Lys Pro His Glu Asp Gln Gln Asp Ile Asn
   515 520 525
   Lys Asp Val Gly Val Lys Thr Ser Glu Ser Thr Thr Thr Val Lys Ser
   530 535 540
   Lys Val Asp Glu Arg Glu Lys Tyr Met Ile Gly Asn Ser Val Gln Lys
   545 550 555 560
   Ile Ser Glu Pro Glu Ala Glu Ile Ser Pro Gly Ser Leu Pro Val Thr
   565 570 575
   Ala Asn Met Lys Ala Ser Glu Asn Leu Lys His Ile Val Asn His Asp
   580 585 590
   Asp Val Phe Glu Glu Ser Glu Glu Leu Ser Ser Asp Glu Glu Met Lys
   595 600 605
   Met Ala Glu Met Arg Pro Pro Leu Ile Glu Thr Ser Ile Asn Gln Pro
   610 615 620
   Lys Val Val Ala Leu Ser Asn Asn Lys Lys Asp Asp Thr Lys Glu Thr
   625 630 635 640
   Asp Ser Leu Ser Asp Glu Val Thr His Asn Ser Asn Gln Asn Asn Ser
   645 650 655
   Asn Cys Ser Ser Pro Ser Arg Met Ser Asp Ser Val Ser Leu Asn Thr
   660 665 670
   Asp Ser Ser Gln Asp Thr Ser Leu Cys Ser Pro Val Lys Gln Thr His
           675                 680                 685
   Ile Asp Ile Asn Ser Lys Ile Arg Gln Glu Asp Glu Asn Phe Asn Ser
       690                 695                 700
   Leu Leu Gln Asn Gly Asp Ile Leu Asn Ser Ser Thr Glu Glu Lys Phe
   705                 710                 715                 720
   Lys Ala His Asp Lys Lys Asp Phe Asn Leu Pro Glu Tyr Asp Leu Asn
                   725                 730                 735
   Val Glu Glu Arg Leu Val Leu Ile Glu Lys Ser Val Asp Ser Thr Ala
               740                 745                 750
   Thr Ala Asp Asp Thr His Lys Leu Asp His Ile Asn Met Asn Leu Asn
           755                 760                 765
   Lys Leu Ile Thr Asn Asp Thr Phe Gln Pro Glu Ile Met Glu Arg Ser
       770                 775                 780
   Lys Thr Gln Asp Ile Val Leu Gly Thr Ser Phe Leu Ser Ile Asn Ser
   785                 790                 795                 800
   Lys Glu Glu Thr Glu His Leu Glu Asn Gly Asn Lys Tyr Pro Asn Leu
                   805                 810                 815
   Glu Ser Val Asn Lys Val Asn Gly His Ser Glu Glu Thr Ser Gln Ser
               820                 825                 830
   Pro Asn Arg Thr Glu Pro His Asp Ser Asp Cys Ser Val Asp Leu Gly
           835                 840                 845
   Ile Ser Lys Ser Thr Glu Asp Leu Ser Pro Gln Lys Ser Gly Pro Val
       850                 855                 860
   Gly Ser Val Val Lys Ser His Ser Ile Thr Asn Met Glu Ile Gly Gly
   865                 870                 875                 880
   Leu Lys Ile Tyr Asp Ile Leu Ser Asp Asn Gly Pro Gln Gln Pro Ser
                   885                 890                 895
   Thr Thr Val Lys Ile Thr Ser Ala Val Asp Gly Lys Asn Ile Val Arg
               900                 905                 910
   Ser Lys Ser Ala Thr Leu Leu Tyr Asp Gln Pro Leu Gln Val Phe Thr
           915                 920                 925
   Gly Ser Ser Ser Ser Ser Asp Leu Ile Ser Gly Thr Lys Ala Ile Phe
       930                 935                 940
   Lys Phe Asp Ser Asn His Asn Pro Glu Glu Pro Asn Ile Ile Arg Gly
   945                 950                 955                 960
   Pro Thr Ser Gly Pro Gln Ser Ala Pro Gln Ile Tyr Gly Pro Pro Gln
                   965                 970                 975
   Tyr Asn Ile Gln Tyr Ser Ser Ser Ala Ala Val Lys Asp Thr Leu Trp
               980                 985                 990
   His Ser Lys Gln Asn Pro Gln Ile Asp His Ala Ser Phe Pro Pro Gln
           995                 1000                1005
   Leu Leu Pro Arg Ser Glu Ser Thr Glu Asn Gln Ser Tyr Ala Lys His
       1010                1015                1020
   Ser Ala Asn Met Asn Phe Ser Asn His Asn Asn Val Arg Ala Asn Thr
   1025                1030                1035                1040
   Ala Tyr His Leu His Gln Arg Leu Gly Pro Ala Arg His Gly Glu Met
                   1045                1050                1055
   Trp Ala Ile Ser Pro Asn Asp Arg Leu Ile Pro Ala Val Thr Arg Ser
               1060                1065                1070
   Thr Ile Gln Arg Gln Ser Ser Val Ser Ser Thr Ala Ser Val Asn Leu
           1075                1080                1085
   Gly Asp Pro Gly Ser Thr Arg Arg Ala Gln Ile Pro Glu Gly Asp Tyr
       1090                1095                1100
   Leu Ser Tyr Arg Glu Phe His Ser Ala Gly Arg Thr Pro Pro Met Met
   1105                1110                1115                1120
   Pro Gly Ser Gln Arg Pro Leu Ser Ala Arg Thr Tyr Ser Ile Asp Gly
                   1125                1130                1135
   Pro Asn Ala Ser Arg Pro Gln Ser Ala Arg Pro Ser Ile Asn Glu Ile
               1140                1145                1150
   Pro Glu Arg Thr Met Ser Val Ser Asp Phe Asn Tyr Ser Arg Thr Ser
           1155                1160                1165
   Pro Ser Lys Arg Pro Asn Ala Arg Val Gly Ser Glu His Ser Leu Leu
       1170                1175                1180
   Asp Pro Pro Gly Lys Ser Lys Val Pro Arg Asp Trp Arg Glu Gln Val
   1185                1190                1195                1200
   Leu Arg His Ile Glu Ala Lys Lys Leu Glu Lys Ile Arg Val Arg Val
                   1205                1210                1215
   Glu Lys Asp Pro Glu Leu Gly Phe Ser Ile Ser Gly Gly Val Gly Gly
               1220                1225                1230
   Arg Gly Asn Pro Phe Arg Pro Asp Asp Asp Gly Ile Phe Val Thr Arg
           1235                1240                1245
   Val Gln Pro Glu Gly Pro Ala Ser Lys Leu Leu Gln Pro Gly Asp Lys
       1250                1255                1260
   Ile Ile Gln Ala Asn Gly Tyr Ser Phe Ile Asn Ile Glu His Gly Gln
   1265                1270                1275                1280
   Ala Val Ser Leu Leu Lys Thr Phe Gln Asn Thr Val Glu Leu Ile Ile
                   1285                1290                1295
   Val Arg Glu Val Ser Ser
               1300
<210> 132
   <211> 1267
   <212> PRT
   <213> Artificial Sequence
<220>
   <223> ERBB2IP protein fragment
<400> 132
   Met Thr Thr Lys Arg Ser Leu Phe Val Arg Leu Val Pro Cys Arg Cys
   1               5                   10                  15
   Leu Arg Gly Glu Glu Glu Thr Val Thr Thr Leu Asp Tyr Ser His Cys
               20                  25                  30
   Ser Leu Glu Gln Val Pro Lys Glu Ile Phe Thr Phe Glu Lys Thr Leu
           35                  40                  45
   Glu Glu Leu Tyr Leu Asp Ala Asn Gln Ile Glu Glu Leu Pro Lys Gln
       50                  55                  60
   Leu Phe Asn Cys Gln Ser Leu His Lys Leu Ser Leu Pro Asp Asn Asp
   65                  70                  75                  80
   Leu Thr Thr Leu Pro Ala Ser Ile Ala Asn Leu Ile Asn Leu Arg Glu
                   85                  90                  95
   Leu Asp Val Ser Lys Asn Gly Ile Gln Glu Phe Pro Glu Asn Ile Lys
               100                 105                 110
   Asn Cys Lys Val Leu Thr Ile Val Glu Ala Ser Val Asn Pro Ile Ser
           115                 120                 125
   Lys Leu Pro Asp Gly Phe Ser Gln Leu Leu Asn Leu Thr Gln Leu Tyr
       130                 135                 140
   Leu Asn Asp Ala Phe Leu Glu Phe Leu Pro Ala Asn Phe Gly Arg Leu
   145                 150                 155                 160
   Thr Lys Leu Gln Ile Leu Glu Leu Arg Glu Asn Gln Leu Lys Met Leu
                   165                 170                 175
   Pro Lys Thr Met Asn Arg Leu Thr Gln Leu Glu Arg Leu Asp Leu Gly
               180                 185                 190
   Ser Asn Glu Phe Thr Glu Val Pro Glu Val Leu Glu Gln Leu Ser Gly
           195                 200                 205
   Leu Lys Glu Phe Trp Met Asp Ala Asn Arg Leu Thr Phe Ile Pro Gly
       210                 215                 220
   Phe Ile Gly Ser Leu Lys Gln Leu Thr Tyr Leu Asp Val Ser Lys Asn
   225                 230                 235                 240
   Asn Ile Glu Met Val Glu Glu Gly Ile Ser Thr Cys Glu Asn Leu Gln
                   245                 250                 255
   Asp Leu Leu Leu Ser Ser Asn Ser Leu Gln Gln Leu Pro Glu Thr Ile
               260                 265                 270
   Gly Ser Leu Lys Asn Ile Thr Thr Leu Lys Ile Asp Glu Asn Gln Leu
           275                 280                 285
   Met Tyr Leu Pro Asp Ser Ile Gly Gly Leu Ile Ser Val Glu Glu Leu
       290                 295                 300
   Asp Cys Ser Phe Asn Glu Val Glu Ala Leu Pro Ser Ser Ile Gly Gln
   305                 310                 315                 320
   Leu Thr Asn Leu Arg Thr Phe Ala Ala Asp His Asn Tyr Leu Gln Gln
                   325                 330                 335
   Leu Pro Pro Glu Ile Gly Ser Trp Lys Asn Ile Thr Val Leu Phe Leu
               340                 345                 350
   His Ser Asn Lys Leu Glu Thr Leu Pro Glu Glu Met Gly Asp Met Gln
           355                 360                 365
   Lys Leu Lys Val Ile Asn Leu Ser Asp Asn Arg Leu Lys Asn Leu Pro
       370                 375                 380
   Phe Ser Phe Thr Lys Leu Gln Gln Leu Thr Ala Met Trp Leu Ser Asp
   385                 390                 395                 400
   Asn Gln Ser Lys Pro Leu Ile Pro Leu Gln Lys Glu Thr Asp Ser Glu
                   405                 410                 415
   Thr Gln Lys Met Val Leu Thr Asn Tyr Met Phe Pro Gln Gln Pro Arg
               420                 425                 430
   Thr Glu Asp Val Met Phe Ile Ser Asp Asn Glu Ser Phe Asn Pro Ser
           435                 440                 445
   Leu Trp Glu Glu Gln Arg Lys Gln Arg Ala Gln Val Ala Phe Glu Cys
       450                 455                 460
   Asp Glu Asp Lys Asp Glu Arg Glu Ala Pro Pro Arg Glu Gly Asn Leu
   465                 470                 475                 480
   Lys Arg Tyr Pro Thr Pro Tyr Pro Asp Glu Leu Lys Asn Met Val Lys
                   485                 490                 495
   Thr Val Gln Thr Ile Val His Arg Leu Lys Asp Glu Glu Thr Asn Glu
               500                 505                 510
   Asp Ser Gly Arg Asp Leu Lys Pro His Glu Asp Gln Gln Asp Ile Asn
           515                 520                 525
   Lys Asp Val Gly Val Lys Thr Ser Glu Ser Thr Thr Thr Val Lys Ser
       530                 535                 540
   Lys Val Asp Glu Arg Glu Lys Tyr Met Ile Gly Asn Ser Val Gln Lys
   545                 550                 555                 560
   Ile Ser Glu Pro Glu Ala Glu Ile Ser Pro Gly Ser Leu Pro Val Thr
                   565                 570                 575
   Ala Asn Met Lys Ala Ser Glu Asn Leu Lys His Ile Val Asn His Asp
               580                 585                 590
   Asp Val Phe Glu Glu Ser Glu Glu Leu Ser Ser Asp Glu Glu Met Lys
           595                 600                 605
   Met Ala Glu Met Arg Pro Pro Leu Ile Glu Thr Ser Ile Asn Gln Pro
       610                 615                 620
   Lys Val Val Ala Leu Ser Asn Asn Lys Lys Asp Asp Thr Lys Glu Thr
   625                 630                 635                 640
   Asp Ser Leu Ser Asp Glu Val Thr His Asn Ser Asn Gln Asn Asn Ser
                   645                 650                 655
   Asn Cys Ser Ser Pro Ser Arg Met Ser Asp Ser Val Ser Leu Asn Thr
               660                 665                 670
   Asp Ser Ser Gln Asp Thr Ser Leu Cys Ser Pro Val Lys Gln Thr His
           675                 680                 685
   Ile Asp Ile Asn Ser Lys Ile Arg Gln Glu Asp Glu Asn Phe Asn Ser
       690                 695                 700
   Leu Leu Gln Asn Gly Asp Ile Leu Asn Ser Ser Thr Glu Glu Lys Phe
   705                 710                 715                 720
   Lys Ala His Asp Lys Lys Asp Phe Asn Leu Pro Glu Tyr Asp Leu Asn
                   725                 730                 735
   Val Glu Glu Arg Leu Val Leu Ile Glu Lys Ser Val Asp Ser Thr Ala
               740                 745                 750
   Thr Ala Asp Asp Thr His Lys Leu Asp His Ile Asn Met Asn Leu Asn
           755                 760                 765
   Lys Leu Ile Thr Asn Asp Thr Phe Gln Pro Glu Ile Met Glu Arg Ser
       770                 775                 780
   Lys Thr Gln Asp Ile Val Leu Gly Thr Ser Phe Leu Ser Ile Asn Ser
   785                 790                 795                 800
   Lys Glu Glu Thr Glu His Leu Glu Asn Gly Asn Lys Tyr Pro Asn Leu
                   805                 810                 815
   Glu Ser Val Asn Lys Val Asn Gly His Ser Glu Glu Thr Ser Gln Ser
               820                 825                 830
   Pro Asn Arg Thr Glu Pro His Asp Ser Asp Cys Ser Val Asp Leu Gly
           835                 840                 845
   Ile Ser Lys Ser Thr Glu Asp Leu Ser Pro Gln Lys Ser Gly Pro Val
       850                 855                 860
   Gly Ser Val Val Lys Ser His Ser Ile Thr Asn Met Glu Ile Gly Gly
   865                 870                 875                 880
   Leu Lys Ile Tyr Asp Ile Leu Ser Asp Asn Gly Pro Gln Gln Pro Ser
                   885                 890                 895
   Thr Thr Val Lys Ile Thr Ser Ala Val Asp Gly Lys Asn Ile Val Arg
               900                 905                 910
   Ser Lys Ser Ala Thr Leu Leu Tyr Asp Gln Pro Leu Gln Val Phe Thr
           915                 920                 925
   Gly Ser Ser Ser Ser Ser Asp Leu Ile Ser Gly Thr Lys Ala Ile Phe
       930                 935                 940
   Lys Phe Asp Ser Asn His Asn Pro Glu Glu Pro Asn Ile Ile Arg Gly
   945                 950                 955                 960
   Pro Thr Ser Gly Pro Gln Ser Ala Pro Gln Ile Tyr Gly Pro Pro Gln
                   965                 970                 975
   Tyr Asn Ile Gln Tyr Ser Ser Ser Ala Ala Val Lys Asp Thr Leu Trp
               980                 985                 990
   His Ser Lys Gln Asn Pro Gln Ile Asp His Ala Ser Phe Pro Pro Gln
   995 1000 1005
   Leu Leu Pro Arg Ser Glu Ser Thr Glu Asn Gln Ser Tyr Ala Lys His
   1010 1015 1020
   Ser Ala Asn Met Asn Phe Ser Asn His Asn Asn Val Arg Ala Asn Thr
   1025 1030 1035 1040
   Ala Tyr His Leu His Gln Arg Leu Gly Pro Ala Arg His Gly Glu Met
   1045 1050 1055
   Trp Ala Ile Ser Pro Asn Asp Arg Leu Ile Pro Ala Val Thr Arg Ser
   1060 1065 1070
   Thr Ile Gln Arg Gln Ser Ser Val Ser Ser Thr Ala Ser Val Asn Leu
   1075 1080 1085
   Gly Asp Pro Gly Ser Thr Arg Arg Ala Gln Ile Pro Glu Gly Asp Tyr
   1090 1095 1100
   Leu Ser Tyr Arg Glu Phe His Ser Ala Gly Arg Thr Pro Pro Met Met
   1105 1110 1115 1120
   Pro Gly Ser Gln Arg Pro Leu Ser Ala Arg Thr Tyr Ser Ile Asp Gly
   1125 1130 1135
   Pro Asn Ala Ser Arg Pro Gln Ser Ala Arg Pro Ser Ile Asn Glu Ile
   1140 1145 1150
   Pro Glu Arg Thr Met Ser Val Ser Asp Phe Asn Tyr Ser Arg Thr Ser
   1155 1160 1165
   Pro Ser Lys Arg Pro Asn Ala Arg Val Gly Ser Glu His Ser Leu Leu
   1170 1175 1180
   Asp Pro Pro Gly Lys Ser Lys Val Pro Arg Asp Trp Arg Glu Gln Val
   1185 1190 1195 1200
   Leu Arg His Ile Glu Ala Lys Lys Leu Glu Lys Ile Arg Val Arg Val
   1205 1210 1215
   Glu Lys Asp Pro Glu Leu Gly Phe Ser Ile Ser Gly Gly Val Gly Gly
   1220 1225 1230
   Arg Gly Asn Pro Phe Arg Pro Asp Asp Asp Gly Ile Phe Val Thr Arg
   1235 1240 1245
   Val Gln Pro Glu Gly Pro Ala Ser Lys Leu Leu Gln Pro Gly Asp Lys
   1250 1255 1260
   Ile Ile Gln
   1265
<210> 133
   <211> 8
   <212> PRT
   <213> Artificial Sequence
<220>
   <223> Break-point of ERBB2IP protein fragment
<400> 133
   Gln Pro Gly Asp Lys Ile Ile Gln
   1 5
<210> 134
   <211> 7872
   <212> DNA
   <213> Artificial Sequence
<220>
   <223> CDS of MAST4 gene (NM_001164664)
<400> 134
   atgggggaga aagtttcgga ggcgccagag ccggtgcccc gcggctgcag tggccacggc      60
   agccggactc cagcctctgc gctggtcgcc gcgtcctctc cgggtgcttc ctcggccgag     120
   tcctcctcgg gctcagaaac tctgtcggag gaaggggagc ccggcggctt ctccagagag     180
   catcagccgc cgccgccgcc gccgttggga ggcaccctgg gcgcccgggc gcccgccgcg     240
   tgggctccgg caagcgtgct gctggagcgc ggagtccttg cgctgccgcc gccgcttccc     300
   ggaggagctg tgccgcccgc gccccggggc agcagcgcgt cccaggagga gcaggacgag     360
   gagcttgacc acatattatc ccctccaccc atgccgtttc ggaaatgcag caacccagat     420
   gtggcttctg gccctggaaa atcactgaag tataaaagac agctgagtga ggatggaaga     480
   cagctaaggc gagggagcct gggaggagcc ctgactggga ggtaccttct tccaaacccg     540
   gtggcgggac aggcctggcc ggcctctgca gagacgtcca acctcgtgcg catgcgcagc     600
   caggccctgg gccagtcggc gccctcgctc accgccagcc tgaaggagct gagtctcccc     660
   agaagaggaa gtttttgccg aacaagcaac cggaaaagct taataggcaa tgggcagtca     720
   ccagcattgc ctcgaccaca ctcacctctc tctgctcatg caggaaatag ccctcaagat     780
   agtccaagaa atttctcccc cagtgcctca gcccattttt catttgcacg gaggactgat     840
   ggacgccgct ggtcgttggc ttctctccct tcctctggct atgggacaaa cacacccagc     900
   tctacggtct cttcatcctg ttcctcccag gagaagttgc atcagttacc ataccaacca     960
   acaccagacg agttacactt cttatcaaaa catttctgta ccaccgaaag catcgccact    1020
   gagaacagat gcaggaacac gccgatgcgc ccccgttccc gaagtctgag ccctggacgt    1080
   tctcccgcct gctgtgacca tgaaataatt atgatgaacc atgtctacaa agaaaggttc    1140
   ccaaaggcta cagctcagat ggaagaacgt ctaaaggaaa ttatcaccag ctactctcct    1200
   gacaacgttc tacccttagc agatggagtg cttagtttca ctcaccacca gattattgaa    1260
   ctggctcgag attgcttgga taaatcccac cagggcctca tcacctcacg atacttcctt    1320
   gaattacagc acaaattaga taagttgcta caggaggctc atgatcgttc agaaagtgga    1380
   gaattggcat ttattaaaca actagttcga aagatcctaa ttgttattgc ccgccctgct 1440
   cggttattag agtgcctgga atttgatccg gaagaatttt actacctatt ggaagcagca 1500
   gaaggccatg ccaaagaagg acagggtatt aaaaccgaca ttcccaggta catcattagc 1560
   caactgggac tcaataagga tcccttggaa gaaatggctc atttgggaaa ctacgatagt 1620
   gggacagcag aaacaccaga aacagatgaa tcagtgagta gctctaatgc ctccctgaaa 1680
   cttcgaagga aacctcggga aagtgatttt gaaacgatta aattgattag caatggagcc 1740
   tatggggcag tctactttgt tcggcataaa gaatcccggc agaggtttgc catgaagaag 1800
   attaataaac agaacctcat ccttcgaaac cagatccagc aggcctttgt ggagcgggat 1860
   atcctgactt ttgcagaaaa cccctttgtt gtcagcatgt attgctcctt tgaaacaagg 1920
   cgccacttgt gcatggtcat ggaatatgtg gaagggggag actgtgctac tttaatgaaa 1980
   aacatgggtc ctctccctgt tgatatggcc agaatgtact ttgctgagac ggtcttggcc 2040
   ttggaatatt tacataatta tggaattgta cacagggatt tgaaaccaga caacttgttg 2100
   gttacctcca tggggcacat aaagctgaca gattttggat tatctaaggt gggactaatg 2160
   agcatgacta ccaaccttta cgagggtcat attgagaagg atgctagaga gttcctggat 2220
   aaacaggtct gtggcacacc tgaatacatt gcaccagaag tgattctgag gcagggttat 2280
   ggaaagccgg tggactggtg ggccatgggg attatcctct atgaatttct ggttggatgc 2340
   gtgccattct ttggggatac tccagaggag ctatttggac aagtcatcag tgatgagatc 2400
   aactggcctg agaaggatga ggcaccccca cctgatgccc aggatctgat taccttactc 2460
   ctcaggcaga atcccctgga gaggctggga acaggtggtg catatgaagt caaacagcat 2520
   cgattcttcc gttctttaga ctggaacagt ttgctgagac agaaggcaga atttattccc 2580
   caactggaat ctgaggatga cacaagttat tttgatactc ggtctgagaa gtatcatcat 2640
   atggaaacgg aggaagaaga tgacacaaat gatgaagact ttaatgtgga aataaggcag 2700
   ttttcttcat gttcacacag gttttcaaaa gttttcagca gtatagatcg aatcactcag 2760
   aattcagcag aagagaagga agactctgtg gacaaaacca aaagcaccac cttgccatcc 2820
   acagaaacac tgagctggag ttcagaatat tctgaaatgc aacagctatc aacatccaac 2880
   tcttcagata ctgaaagcaa cagacataaa ctcagttctg gcctacttcc caaactggct 2940
   atttcaacag agggagagca agatgaagct gcctcctgcc ctggagaccc ccatgaggag 3000
   ccaggaaagc cagcccttcc tcctgaagag tgtgcccagg aggagcctga ggtcaccacc 3060
   ccagccagca ccatcagcag ctccaccctg tcagttggca gtttttcaga gcacttggat 3120
   cagataaatg gacgaagcga gtgtgtggac agtacagata attcctcaaa gccatccagt 3180
   gaacccgctt ctcacatggc tcggcagcga ttagaaagca cagaaaaaaa gaaaatctcg 3240
   gggaaagtca caaagtccct ctctgccagt gctctttccc tcatgatccc aggagatatg 3300
   tttgctgttt cccctctggg aagtccaatg tctccccatt ccctgtcctc ggacccttct 3360
   tcttcacgag attcctctcc cagccgagat tcctcagcag cttctgccag tccacatcag 3420
   ccgattgtga tccacagttc ggggaagaac tacggcttta ccatccgagc catccgggtg 3480
   tatgtgggag acagtgacat ctatacagtg caccatatcg tctggaatgt agaagaagga 3540
   agtccggcat gccaggcagg actgaaggct ggagatctta tcactcacat caatggagaa 3600
   ccagtgcatg gacttgtcca cacagaagtt atagaactcc tactgaagag tgggaataag 3660
   gtgtcaatca ctactacccc atttgaaaac acatcaatca aaactggacc agccaggaga 3720
   aacagctata agagccggat ggtgaggcgg agcaagaaat ccaagaagaa agaaagtctc 3780
   gaaaggagga gatctctttt caaaaagcta gccaagcagc cttctccttt actccacacc 3840
   agccgaagtt tctcctgctt gaacagatcc ctgtcatcgg gtgagagcct cccaggttcc 3900
   cccactcata gcttgtctcc ccggtctcca acaccaagct accgctccac ccctgacttc 3960
   ccatctggta ctaattcctc ccagagcagc tcccctagtt ctagtgcccc caattcccca 4020
   gcagggtccg ggcacatccg gcccagcact ctccacggtc ttgcacccaa actcggcggg 4080
   cagcggtacc ggtccggaag gcgaaagtcc gccggcaaca tcccactgtc cccgctggcc 4140
   cggacgccct ctccaacccc gcaacccacc tccccgcagc ggtcaccatc ccctcttctg 4200
   ggacactcac tgggcaattc caagatcgcg caagcctttc ccagcaagat gcactccccg 4260
   cccaccatcg tcagacacat cgtgaggccc aagagtgcgg agccccccag gtccccgctg 4320
   ctcaagcgcg tgcagtccga ggagaagctg tcgccctctt acggcagtga caagaagcac 4380
   ctgtgctccc gcaagcacag cctggaggtg acccaagagg aggtgcagcg ggagcagtcc 4440
   cagcgggagg cgccgctgca gagcctggat gagaacgtgt gcgacgtgcc gccgctcagc 4500
   cgcgcccggc cagtggagca aggctgcctg aaacgcccag tctcccggaa ggtgggccgc 4560
   caggagtctg tggacgacct ggaccgcgac aagctgaagg ccaaggtggt ggtgaagaaa 4620
   gcagacggct tcccagagaa acaggaatcc caccagaaat cccatggacc cgggagtgat 4680
   ttggaaaact ttgctctgtt taagctggaa gagagagaga agaaagtcta tccgaaggct 4740
   gtggaaaggt caagtacttt tgaaaacaaa gcgtctatgc aggaggcgcc accgctgggc 4800
   agcctgctga aggatgctct tcacaagcag gccagcgtgc gcgccagcga gggtgcgatg 4860
   tcggatggcc gggtgcctgc ggagcaccgc cagggtggcg gggacttcag acgggccccc 4920
   gctcctggca ccctccagga tggtctctgc cactccctcg acaggggcat ctctgggaag 4980
   ggggaaggca cggagaagtc ctcccaggcc aaggagcttc tccgatgtga aaagttagac 5040
   agcaagctgg ccaacatcga ttacctccga aagaaaatgt cacttgagga caaagaggac 5100
   aacctctgcc ctgtgctgaa gcccaagatg acagctggct cccacgaatg cctgccaggg 5160
   aacccagtcc gacccacggg tgggcagcag gagcccccgc cggcttctga gagccgagct 5220
   tttgtcagca gcacccatgc agctcagatg agtgccgtct cttttgttcc cctcaaggcc 5280
   ttaacaggcc gggtggacag tggaacggag aagcctggct tggttgctcc tgagtcccct 5340
   gttaggaaga gcccctccga gtataagctg gaaggtaggt ctgtctcatg cctgaagccg 5400
   atcgagggca ctctggacat tgctctcctg tccggacctc aggcctccaa gacagaactg 5460
   ccttccccag agtctgcaca gagccccagc ccaagtggtg acgtgagggc ctctgtgcca 5520
   ccagttctcc ccagcagcag tgggaaaaag aacgatacca ccagtgcaag agagctttct 5580
   ccttccagct taaagatgaa taaatcctac ctgctggagc cttggttcct gccccccagc 5640
   cgaggtctcc agaattcacc agcagtttcc ctgcctgacc cagagttcaa gagggacagg 5700
   aaaggtcccc atcctactgc caggagccct ggaacagtca tggaaagcaa tccccaacag 5760
   agagagggca gctcccctaa acaccaagac cacaccactg accccaagct tctgacctgc 5820
   ctggggcaga acctccacag ccctgacctg gccaggccac gctgcccgct cccacctgaa 5880
   gcttccccct caagggagaa gccaggcctg agggaatcgt ctgaaagagg ccctcccaca 5940
   gccagaagcg agcgctctgc tgcgagggct gacacatgca gagagccctc catggaactg 6000
   tgctttccag aaactgcgaa aaccagtgac aactccaaaa atctcctctc tgtgggaagg 6060
   acccacccag atttctatac acagacccag gccatggaga aagcatgggc gccgggtggg 6120
   aaaacgaacc acaaagatgg cccaggtgag gcgaggcccc cgcccagaga caactcctct 6180
   ctgcactcag ctggaattcc ctgtgagaag gagctgggca aggtgaggcg tggcgtggaa 6240
   cccaagcccg aagcgcttct tgccaggcgg tctctgcagc cacctggaat tgagagtgag 6300
   aagagtgaaa agctctccag tttcccatct ttgcagaaag atggtgccaa ggaacctgaa 6360
   aggaaggagc agcctctaca aaggcatccc agcagcatcc ctccgccccc tctgacggcc 6420
   aaagacctgt ccagcccggc tgccaggcag cattgcagtt ccccaagcca cgcttctggc 6480
   agagagccgg gggccaagcc cagcactgca gagcccagct cgagccccca ggaccctccc 6540
   aagcctgttg ctgcgcacag tgaaagcagc agccacaagc cccggcctgg ccctgacccg 6600
   ggccctccaa agactaagca ccccgaccgg tccctctcct ctcagaaacc aagtgtcggg 6660
   gccacaaagg gcaaagagcc tgccactcag tccctcggtg gctctagcag agaggggaag 6720
   ggccacagta agagtgggcc ggatgtgttt cctgctaccc caggctccca gaacaaagcc 6780
   agcgatggga ttggccaggg agaaggtggg ccctctgtcc cactgcacac tgacagggct 6840
   cctctagacg ccaagccaca acccaccagt ggtgggcggc ccctggaggt gctggagaag 6900
   cctgtgcatt tgccaaggcc gggacaccca gggcctagtg agccagcgga ccagaaactg 6960
   tccgctgttg gtgaaaagca aaccctgtct ccaaagcacc ccaaaccatc cactgtgaaa 7020
   gattgcccca ccctgtgcaa acagacagac aacagacaga cagacaaaag cccgagtcag 7080
   ccggccgcca acaccgacag aagggcggaa gggaagaaat gcactgaagc actttatgct 7140
   ccagcagagg gcgacaagct cgaggccggc ctttcctttg tgcatagcga gaaccggttg 7200
   aaaggcgcgg agcggccagc cgcgggggtg gggaagggct tccctgaggc cagagggaaa 7260
   gggcccggtc cccagaagcc accgacggag gcagacaagc ccaatggcat gaaacggtcc 7320
   ccctcagcca ctgggcagag ttctttccga tccacggccc tcccggaaaa gtctctgagc 7380
   tgctcctcca gcttccctga aaccagggcc ggagttagag aggcctctgc agccagcagc 7440
   gacacctctt ctgccaaggc cgccgggggc atgctggagc ttccagcccc cagcaacagg 7500
   gaccatagga aggctcagcc tgccggggag ggccgaaccc acatgacaaa gagtgactcc 7560
   ctgccctcct tccgggtctc caccctgcct ctggagtcac accaccccga cccaaacacc 7620
   atgggcgggg ccagccaccg ggacagggct ctctcggtga ctgccaccgt aggggaaacc 7680
   aaagggaagg accctgcccc agcccagcct cccccagcta ggaaacagaa cgtgggcaga 7740
   gacgtgacca agccatcccc agccccaaac actgaccgcc ccatctctct ttctaatgag 7800
   aaggactttg tggtacggca gaggcggggg aaagagagtt tgcgtagcag ccctcacaaa 7860
   aaggccttgt aa 7872
<210> 135
   <211> 6726
   <212> DNA
   <213> Artificial Sequence
<220>
   <223> MAST4 gene fragment
<400> 135
   gctacagctc agatggaaga acgtctaaag gaaattatca ccagctactc tcctgacaac      60
   gttctaccct tagcagatgg agtgcttagt ttcactcacc accagattat tgaactggct     120
   cgagattgct tggataaatc ccaccagggc ctcatcacct cacgatactt ccttgaatta     180
   cagcacaaat tagataagtt gctacaggag gctcatgatc gttcagaaag tggagaattg     240
   gcatttatta aacaactagt tcgaaagatc ctaattgtta ttgcccgccc tgctcggtta     300
   ttagagtgcc tggaatttga tccggaagaa ttttactacc tattggaagc agcagaaggc     360
   catgccaaag aaggacaggg tattaaaacc gacattccca ggtacatcat tagccaactg     420
   ggactcaata aggatccctt ggaagaaatg gctcatttgg gaaactacga tagtgggaca     480
   gcagaaacac cagaaacaga tgaatcagtg agtagctcta atgcctccct gaaacttcga     540
   aggaaacctc gggaaagtga ttttgaaacg attaaattga ttagcaatgg agcctatggg     600
   gcagtctact ttgttcggca taaagaatcc cggcagaggt ttgccatgaa gaagattaat     660
   aaacagaacc tcatccttcg aaaccagatc cagcaggcct ttgtggagcg ggatatcctg     720
   acttttgcag aaaacccctt tgttgtcagc atgtattgct cctttgaaac aaggcgccac 780
   ttgtgcatgg tcatggaata tgtggaaggg ggagactgtg ctactttaat gaaaaacatg 840
   ggtcctctcc ctgttgatat ggccagaatg tactttgctg agacggtctt ggccttggaa 900
   tatttacata attatggaat tgtacacagg gatttgaaac cagacaactt gttggttacc 960
   tccatggggc acataaagct gacagatttt ggattatcta aggtgggact aatgagcatg 1020
   actaccaacc tttacgaggg tcatattgag aaggatgcta gagagttcct ggataaacag 1080
   gtctgtggca cacctgaata cattgcacca gaagtgattc tgaggcaggg ttatggaaag 1140
   ccggtggact ggtgggccat ggggattatc ctctatgaat ttctggttgg atgcgtgcca 1200
   ttctttgggg atactccaga ggagctattt ggacaagtca tcagtgatga gatcaactgg 1260
   cctgagaagg atgaggcacc cccacctgat gcccaggatc tgattacctt actcctcagg 1320
   cagaatcccc tggagaggct gggaacaggt ggtgcatatg aagtcaaaca gcatcgattc 1380
   ttccgttctt tagactggaa cagtttgctg agacagaagg cagaatttat tccccaactg 1440
   gaatctgagg atgacacaag ttattttgat actcggtctg agaagtatca tcatatggaa 1500
   acggaggaag aagatgacac aaatgatgaa gactttaatg tggaaataag gcagttttct 1560
   tcatgttcac acaggttttc aaaagttttc agcagtatag atcgaatcac tcagaattca 1620
   gcagaagaga aggaagactc tgtggacaaa accaaaagca ccaccttgcc atccacagaa 1680
   acactgagct ggagttcaga atattctgaa atgcaacagc tatcaacatc caactcttca 1740
   gatactgaaa gcaacagaca taaactcagt tctggcctac ttcccaaact ggctatttca 1800
   acagagggag agcaagatga agctgcctcc tgccctggag acccccatga ggagccagga 1860
   aagccagccc ttcctcctga agagtgtgcc caggaggagc ctgaggtcac caccccagcc 1920
   agcaccatca gcagctccac cctgtcagtt ggcagttttt cagagcactt ggatcagata 1980
   aatggacgaa gcgagtgtgt ggacagtaca gataattcct caaagccatc cagtgaaccc 2040
   gcttctcaca tggctcggca gcgattagaa agcacagaaa aaaagaaaat ctcggggaaa 2100
   gtcacaaagt ccctctctgc cagtgctctt tccctcatga tcccaggaga tatgtttgct 2160
   gtttcccctc tgggaagtcc aatgtctccc cattccctgt cctcggaccc ttcttcttca 2220
   cgagattcct ctcccagccg agattcctca gcagcttctg ccagtccaca tcagccgatt 2280
   gtgatccaca gttcggggaa gaactacggc tttaccatcc gagccatccg ggtgtatgtg 2340
   ggagacagtg acatctatac agtgcaccat atcgtctgga atgtagaaga aggaagtccg 2400
   gcatgccagg caggactgaa ggctggagat cttatcactc acatcaatgg agaaccagtg 2460
   catggacttg tccacacaga agttatagaa ctcctactga agagtgggaa taaggtgtca 2520
   atcactacta ccccatttga aaacacatca atcaaaactg gaccagccag gagaaacagc 2580
   tataagagcc ggatggtgag gcggagcaag aaatccaaga agaaagaaag tctcgaaagg 2640
   aggagatctc ttttcaaaaa gctagccaag cagccttctc ctttactcca caccagccga 2700
   agtttctcct gcttgaacag atccctgtca tcgggtgaga gcctcccagg ttcccccact 2760
   catagcttgt ctccccggtc tccaacacca agctaccgct ccacccctga cttcccatct 2820
   ggtactaatt cctcccagag cagctcccct agttctagtg cccccaattc cccagcaggg 2880
   tccgggcaca tccggcccag cactctccac ggtcttgcac ccaaactcgg cgggcagcgg 2940
   taccggtccg gaaggcgaaa gtccgccggc aacatcccac tgtccccgct ggcccggacg 3000
   ccctctccaa ccccgcaacc cacctccccg cagcggtcac catcccctct tctgggacac 3060
   tcactgggca attccaagat cgcgcaagcc tttcccagca agatgcactc cccgcccacc 3120
   atcgtcagac acatcgtgag gcccaagagt gcggagcccc ccaggtcccc gctgctcaag 3180
   cgcgtgcagt ccgaggagaa gctgtcgccc tcttacggca gtgacaagaa gcacctgtgc 3240
   tcccgcaagc acagcctgga ggtgacccaa gaggaggtgc agcgggagca gtcccagcgg 3300
   gaggcgccgc tgcagagcct ggatgagaac gtgtgcgacg tgccgccgct cagccgcgcc 3360
   cggccagtgg agcaaggctg cctgaaacgc ccagtctccc ggaaggtggg ccgccaggag 3420
   tctgtggacg acctggaccg cgacaagctg aaggccaagg tggtggtgaa gaaagcagac 3480
   ggcttcccag agaaacagga atcccaccag aaatcccatg gacccgggag tgatttggaa 3540
   aactttgctc tgtttaagct ggaagagaga gagaagaaag tctatccgaa ggctgtggaa 3600
   aggtcaagta cttttgaaaa caaagcgtct atgcaggagg cgccaccgct gggcagcctg 3660
   ctgaaggatg ctcttcacaa gcaggccagc gtgcgcgcca gcgagggtgc gatgtcggat 3720
   ggccgggtgc ctgcggagca ccgccagggt ggcggggact tcagacgggc ccccgctcct 3780
   ggcaccctcc aggatggtct ctgccactcc ctcgacaggg gcatctctgg gaagggggaa 3840
   ggcacggaga agtcctccca ggccaaggag cttctccgat gtgaaaagtt agacagcaag 3900
   ctggccaaca tcgattacct ccgaaagaaa atgtcacttg aggacaaaga ggacaacctc 3960
   tgccctgtgc tgaagcccaa gatgacagct ggctcccacg aatgcctgcc agggaaccca 4020
   gtccgaccca cgggtgggca gcaggagccc ccgccggctt ctgagagccg agcttttgtc 4080
   agcagcaccc atgcagctca gatgagtgcc gtctcttttg ttcccctcaa ggccttaaca 4140
   ggccgggtgg acagtggaac ggagaagcct ggcttggttg ctcctgagtc ccctgttagg 4200
   aagagcccct ccgagtataa gctggaaggt aggtctgtct catgcctgaa gccgatcgag 4260
   ggcactctgg acattgctct cctgtccgga cctcaggcct ccaagacaga actgccttcc 4320
   ccagagtctg cacagagccc cagcccaagt ggtgacgtga gggcctctgt gccaccagtt 4380
   ctccccagca gcagtgggaa aaagaacgat accaccagtg caagagagct ttctccttcc 4440
   agcttaaaga tgaataaatc ctacctgctg gagccttggt tcctgccccc cagccgaggt 4500
   ctccagaatt caccagcagt ttccctgcct gacccagagt tcaagaggga caggaaaggt 4560
   ccccatccta ctgccaggag ccctggaaca gtcatggaaa gcaatcccca acagagagag 4620
   ggcagctccc ctaaacacca agaccacacc actgacccca agcttctgac ctgcctgggg 4680
   cagaacctcc acagccctga cctggccagg ccacgctgcc cgctcccacc tgaagcttcc 4740
   ccctcaaggg agaagccagg cctgagggaa tcgtctgaaa gaggccctcc cacagccaga 4800
   agcgagcgct ctgctgcgag ggctgacaca tgcagagagc cctccatgga actgtgcttt 4860
   ccagaaactg cgaaaaccag tgacaactcc aaaaatctcc tctctgtggg aaggacccac 4920
   ccagatttct atacacagac ccaggccatg gagaaagcat gggcgccggg tgggaaaacg 4980
   aaccacaaag atggcccagg tgaggcgagg cccccgccca gagacaactc ctctctgcac 5040
   tcagctggaa ttccctgtga gaaggagctg ggcaaggtga ggcgtggcgt ggaacccaag 5100
   cccgaagcgc ttcttgccag gcggtctctg cagccacctg gaattgagag tgagaagagt 5160
   gaaaagctct ccagtttccc atctttgcag aaagatggtg ccaaggaacc tgaaaggaag 5220
   gagcagcctc tacaaaggca tcccagcagc atccctccgc cccctctgac ggccaaagac 5280
   ctgtccagcc cggctgccag gcagcattgc agttccccaa gccacgcttc tggcagagag 5340
   ccgggggcca agcccagcac tgcagagccc agctcgagcc cccaggaccc tcccaagcct 5400
   gttgctgcgc acagtgaaag cagcagccac aagccccggc ctggccctga cccgggccct 5460
   ccaaagacta agcaccccga ccggtccctc tcctctcaga aaccaagtgt cggggccaca 5520
   aagggcaaag agcctgccac tcagtccctc ggtggctcta gcagagaggg gaagggccac 5580
   agtaagagtg ggccggatgt gtttcctgct accccaggct cccagaacaa agccagcgat 5640
   gggattggcc agggagaagg tgggccctct gtcccactgc acactgacag ggctcctcta 5700
   gacgccaagc cacaacccac cagtggtggg cggcccctgg aggtgctgga gaagcctgtg 5760
   catttgccaa ggccgggaca cccagggcct agtgagccag cggaccagaa actgtccgct 5820
   gttggtgaaa agcaaaccct gtctccaaag caccccaaac catccactgt gaaagattgc 5880
   cccaccctgt gcaaacagac agacaacaga cagacagaca aaagcccgag tcagccggcc 5940
   gccaacaccg acagaagggc ggaagggaag aaatgcactg aagcacttta tgctccagca 6000
   gagggcgaca agctcgaggc cggcctttcc tttgtgcata gcgagaaccg gttgaaaggc 6060
   gcggagcggc cagccgcggg ggtggggaag ggcttccctg aggccagagg gaaagggccc 6120
   ggtccccaga agccaccgac ggaggcagac aagcccaatg gcatgaaacg gtccccctca 6180
   gccactgggc agagttcttt ccgatccacg gccctcccgg aaaagtctct gagctgctcc 6240
   tccagcttcc ctgaaaccag ggccggagtt agagaggcct ctgcagccag cagcgacacc 6300
   tcttctgcca aggccgccgg gggcatgctg gagcttccag cccccagcaa cagggaccat 6360
   aggaaggctc agcctgccgg ggagggccga acccacatga caaagagtga ctccctgccc        6420
   tccttccggg tctccaccct gcctctggag tcacaccacc ccgacccaaa caccatgggc        6480
   ggggccagcc accgggacag ggctctctcg gtgactgcca ccgtagggga aaccaaaggg        6540
   aaggaccctg ccccagccca gcctccccca gctaggaaac agaacgtggg cagagacgtg        6600
   accaagccat ccccagcccc aaacactgac cgccccatct ctctttctaa tgagaaggac        6660
   tttgtggtac ggcagaggcg ggggaaagag agtttgcgta gcagccctca caaaaaggcc         6720
   ttgtaa                                                                   6726
<210> 136
   <211> 24
   <212> DNA
   <213> Artificial Sequence
<220>
   <223> Break-point of MAST4 gene fragment
<400> 136
   gctacagctc agatggaaga acgt 24
<210> 137
   <211> 2623
   <212> PRT
   <213> Artificial Sequence
<220>
   <223> MAST4 protein
<400> 137
   Met Gly Glu Lys Val Ser Glu Ala Pro Glu Pro Val Pro Arg Gly Cys
    1               5                  10                  15
   Ser Gly His Gly Ser Arg Thr Pro Ala Ser Ala Leu Val Ala Ala Ser
               20                  25                  30
   Ser Pro Gly Ala Ser Ser Ala Glu Ser Ser Ser Gly Ser Glu Thr Leu
           35                  40                  45
   Ser Glu Glu Gly Glu Pro Gly Gly Phe Ser Arg Glu His Gln Pro Pro
       50                  55                  60
   Pro Pro Pro Pro Leu Gly Gly Thr Leu Gly Ala Arg Ala Pro Ala Ala
   65                  70                  75                  80
   Trp Ala Pro Ala Ser Val Leu Leu Glu Arg Gly Val Leu Ala Leu Pro
                   85                  90                  95
   Pro Pro Leu Pro Gly Gly Ala Val Pro Pro Ala Pro Arg Gly Ser Ser
               100                 105                 110
   Ala Ser Gln Glu Glu Gln Asp Glu Glu Leu Asp His Ile Leu Ser Pro
           115                 120                 125
   Pro Pro Met Pro Phe Arg Lys Cys Ser Asn Pro Asp Val Ala Ser Gly
       130                 135                 140
   Pro Gly Lys Ser Leu Lys Tyr Lys Arg Gln Leu Ser Glu Asp Gly Arg
   145                 150                 155                 160
   Gln Leu Arg Arg Gly Ser Leu Gly Gly Ala Leu Thr Gly Arg Tyr Leu
                   165                 170                 175
   Leu Pro Asn Pro Val Ala Gly Gln Ala Trp Pro Ala Ser Ala Glu Thr
               180                 185                 190
   Ser Asn Leu Val Arg Met Arg Ser Gln Ala Leu Gly Gln Ser Ala Pro
           195                 200                 205
   Ser Leu Thr Ala Ser Leu Lys Glu Leu Ser Leu Pro Arg Arg Gly Ser
       210                 215                 220
   Phe Cys Arg Thr Ser Asn Arg Lys Ser Leu Ile Gly Asn Gly Gln Ser
   225                 230                 235                 240
   Pro Ala Leu Pro Arg Pro His Ser Pro Leu Ser Ala His Ala Gly Asn
                   245                 250                 255
   Ser Pro Gln Asp Ser Pro Arg Asn Phe Ser Pro Ser Ala Ser Ala His
               260                 265                 270
   Phe Ser Phe Ala Arg Arg Thr Asp Gly Arg Arg Trp Ser Leu Ala Ser
           275                 280                 285
   Leu Pro Ser Ser Gly Tyr Gly Thr Asn Thr Pro Ser Ser Thr Val Ser
       290                 295                 300
   Ser Ser Cys Ser Ser Gln Glu Lys Leu His Gln Leu Pro Tyr Gln Pro
   305                 310                 315                 320
   Thr Pro Asp Glu Leu His Phe Leu Ser Lys His Phe Cys Thr Thr Glu
                   325                 330                 335
   Ser Ile Ala Thr Glu Asn Arg Cys Arg Asn Thr Pro Met Arg Pro Arg
               340                 345                 350
   Ser Arg Ser Leu Ser Pro Gly Arg Ser Pro Ala Cys Cys Asp His Glu
           355                 360                 365
   Ile Ile Met Met Asn His Val Tyr Lys Glu Arg Phe Pro Lys Ala Thr
       370                 375                 380
   Ala Gln Met Glu Glu Arg Leu Lys Glu Ile Ile Thr Ser Tyr Ser Pro
   385                 390                 395                 400
   Asp Asn Val Leu Pro Leu Ala Asp Gly Val Leu Ser Phe Thr His His
                   405                 410                 415
   Gln Ile Ile Glu Leu Ala Arg Asp Cys Leu Asp Lys Ser His Gln Gly
               420                 425                 430
   Leu Ile Thr Ser Arg Tyr Phe Leu Glu Leu Gln His Lys Leu Asp Lys
           435                 440                 445
   Leu Leu Gln Glu Ala His Asp Arg Ser Glu Ser Gly Glu Leu Ala Phe
       450                 455                 460
   Ile Lys Gln Leu Val Arg Lys Ile Leu Ile Val Ile Ala Arg Pro Ala
   465 470 475 480
   Arg Leu Leu Glu Cys Leu Glu Phe Asp Pro Glu Glu Phe Tyr Tyr Leu
   485 490 495
   Leu Glu Ala Ala Glu Gly His Ala Lys Glu Gly Gln Gly Ile Lys Thr
   500 505 510
   Asp Ile Pro Arg Tyr Ile Ile Ser Gln Leu Gly Leu Asn Lys Asp Pro
   515 520 525
   Leu Glu Glu Met Ala His Leu Gly Asn Tyr Asp Ser Gly Thr Ala Glu
   530 535 540
   Thr Pro Glu Thr Asp Glu Ser Val Ser Ser Ser Asn Ala Ser Leu Lys
   545 550 555 560
   Leu Arg Arg Lys Pro Arg Glu Ser Asp Phe Glu Thr Ile Lys Leu Ile
   565 570 575
   Ser Asn Gly Ala Tyr Gly Ala Val Tyr Phe Val Arg His Lys Glu Ser
   580 585 590
   Arg Gln Arg Phe Ala Met Lys Lys Ile Asn Lys Gln Asn Leu Ile Leu
   595 600 605
   Arg Asn Gln Ile Gln Gln Ala Phe Val Glu Arg Asp Ile Leu Thr Phe
   610 615 620
   Ala Glu Asn Pro Phe Val Val Ser Met Tyr Cys Ser Phe Glu Thr Arg
   625 630 635 640
   Arg His Leu Cys Met Val Met Glu Tyr Val Glu Gly Gly Asp Cys Ala
   645 650 655
   Thr Leu Met Lys Asn Met Gly Pro Leu Pro Val Asp Met Ala Arg Met
   660 665 670
   Tyr Phe Ala Glu Thr Val Leu Ala Leu Glu Tyr Leu His Asn Tyr Gly
   675 680 685
   Ile Val His Arg Asp Leu Lys Pro Asp Asn Leu Leu Val Thr Ser Met
   690 695 700
   Gly His Ile Lys Leu Thr Asp Phe Gly Leu Ser Lys Val Gly Leu Met
   705 710 715 720
   Ser Met Thr Thr Asn Leu Tyr Glu Gly His Ile Glu Lys Asp Ala Arg
   725 730 735
   Glu Phe Leu Asp Lys Gln Val Cys Gly Thr Pro Glu Tyr Ile Ala Pro
   740 745 750
   Glu Val Ile Leu Arg Gln Gly Tyr Gly Lys Pro Val Asp Trp Trp Ala
   755 760 765
   Met Gly Ile Ile Leu Tyr Glu Phe Leu Val Gly Cys Val Pro Phe Phe
   770 775 780
   Gly Asp Thr Pro Glu Glu Leu Phe Gly Gln Val Ile Ser Asp Glu Ile
   785 790 795 800
   Asn Trp Pro Glu Lys Asp Glu Ala Pro Pro Pro Asp Ala Gln Asp Leu
   805 810 815
   Ile Thr Leu Leu Leu Arg Gln Asn Pro Leu Glu Arg Leu Gly Thr Gly
   820 825 830
   Gly Ala Tyr Glu Val Lys Gln His Arg Phe Phe Arg Ser Leu Asp Trp
   835 840 845
   Asn Ser Leu Leu Arg Gln Lys Ala Glu Phe Ile Pro Gln Leu Glu Ser
   850 855 860
   Glu Asp Asp Thr Ser Tyr Phe Asp Thr Arg Ser Glu Lys Tyr His His
   865 870 875 880
   Met Glu Thr Glu Glu Glu Asp Asp Thr Asn Asp Glu Asp Phe Asn Val
   885 890 895
   Glu Ile Arg Gln Phe Ser Ser Cys Ser His Arg Phe Ser Lys Val Phe
   900 905 910
   Ser Ser Ile Asp Arg Ile Thr Gln Asn Ser Ala Glu Glu Lys Glu Asp
   915 920 925
   Ser Val Asp Lys Thr Lys Ser Thr Thr Leu Pro Ser Thr Glu Thr Leu
   930 935 940
   Ser Trp Ser Ser Glu Tyr Ser Glu Met Gln Gln Leu Ser Thr Ser Asn
   945 950 955 960
   Ser Ser Asp Thr Glu Ser Asn Arg His Lys Leu Ser Ser Gly Leu Leu
   965 970 975
   Pro Lys Leu Ala Ile Ser Thr Glu Gly Glu Gln Asp Glu Ala Ala Ser
   980 985 990
   Cys Pro Gly Asp Pro His Glu Glu Pro Gly Lys Pro Ala Leu Pro Pro
   995 1000 1005
   Glu Glu Cys Ala Gln Glu Glu Pro Glu Val Thr Thr Pro Ala Ser Thr
   1010 1015 1020
   Ile Ser Ser Ser Thr Leu Ser Val Gly Ser Phe Ser Glu His Leu Asp
   1025 1030 1035 1040
   Gln Ile Asn Gly Arg Ser Glu Cys Val Asp Ser Thr Asp Asn Ser Ser
   1045 1050 1055
   Lys Pro Ser Ser Glu Pro Ala Ser His Met Ala Arg Gln Arg Leu Glu
   1060 1065 1070
   Ser Thr Glu Lys Lys Lys Ile Ser Gly Lys Val Thr Lys Ser Leu Ser
   1075 1080 1085
   Ala Ser Ala Leu Ser Leu Met Ile Pro Gly Asp Met Phe Ala Val Ser
   1090 1095 1100
   Pro Leu Gly Ser Pro Met Ser Pro His Ser Leu Ser Ser Asp Pro Ser
   1105 1110 1115 1120
   Ser Ser Arg Asp Ser Ser Pro Ser Arg Asp Ser Ser Ala Ala Ser Ala
   1125 1130 1135
   Ser Pro His Gln Pro Ile Val Ile His Ser Ser Gly Lys Asn Tyr Gly
   1140 1145 1150
   Phe Thr Ile Arg Ala Ile Arg Val Tyr Val Gly Asp Ser Asp Ile Tyr
   1155 1160 1165
   Thr Val His His Ile Val Trp Asn Val Glu Glu Gly Ser Pro Ala Cys
   1170 1175 1180
   Gln Ala Gly Leu Lys Ala Gly Asp Leu Ile Thr His Ile Asn Gly Glu
   1185 1190 1195 1200
   Pro Val His Gly Leu Val His Thr Glu Val Ile Glu Leu Leu Leu Lys
   1205 1210 1215
   Ser Gly Asn Lys Val Ser Ile Thr Thr Thr Pro Phe Glu Asn Thr Ser
   1220 1225 1230
   Ile Lys Thr Gly Pro Ala Arg Arg Asn Ser Tyr Lys Ser Arg Met Val
   1235 1240 1245
   Arg Arg Ser Lys Lys Ser Lys Lys Lys Glu Ser Leu Glu Arg Arg Arg
   1250 1255 1260
   Ser Leu Phe Lys Lys Leu Ala Lys Gln Pro Ser Pro Leu Leu His Thr
   1265 1270 1275 1280
   Ser Arg Ser Phe Ser Cys Leu Asn Arg Ser Leu Ser Ser Gly Glu Ser
   1285 1290 1295
   Leu Pro Gly Ser Pro Thr His Ser Leu Ser Pro Arg Ser Pro Thr Pro
   1300 1305 1310
   Ser Tyr Arg Ser Thr Pro Asp Phe Pro Ser Gly Thr Asn Ser Ser Gln
   1315 1320 1325
   Ser Ser Ser Pro Ser Ser Ser Ala Pro Asn Ser Pro Ala Gly Ser Gly
   1330 1335 1340
   His Ile Arg Pro Ser Thr Leu His Gly Leu Ala Pro Lys Leu Gly Gly
   1345 1350 1355 1360
   Gln Arg Tyr Arg Ser Gly Arg Arg Lys Ser Ala Gly Asn Ile Pro Leu
   1365 1370 1375
   Ser Pro Leu Ala Arg Thr Pro Ser Pro Thr Pro Gln Pro Thr Ser Pro
   1380 1385 1390
   Gln Arg Ser Pro Ser Pro Leu Leu Gly His Ser Leu Gly Asn Ser Lys
   1395 1400 1405
   Ile Ala Gln Ala Phe Pro Ser Lys Met His Ser Pro Pro Thr Ile Val
   1410 1415 1420
   Arg His Ile Val Arg Pro Lys Ser Ala Glu Pro Pro Arg Ser Pro Leu
   1425 1430 1435 1440
   Leu Lys Arg Val Gln Ser Glu Glu Lys Leu Ser Pro Ser Tyr Gly Ser
   1445 1450 1455
   Asp Lys Lys His Leu Cys Ser Arg Lys His Ser Leu Glu Val Thr Gln
   1460 1465 1470
   Glu Glu Val Gln Arg Glu Gln Ser Gln Arg Glu Ala Pro Leu Gln Ser
   1475 1480 1485
   Leu Asp Glu Asn Val Cys Asp Val Pro Pro Leu Ser Arg Ala Arg Pro
   1490 1495 1500
   Val Glu Gln Gly Cys Leu Lys Arg Pro Val Ser Arg Lys Val Gly Arg
   1505 1510 1515 1520
   Gln Glu Ser Val Asp Asp Leu Asp Arg Asp Lys Leu Lys Ala Lys Val
   1525 1530 1535
   Val Val Lys Lys Ala Asp Gly Phe Pro Glu Lys Gln Glu Ser His Gln
   1540 1545 1550
   Lys Ser His Gly Pro Gly Ser Asp Leu Glu Asn Phe Ala Leu Phe Lys
   1555 1560 1565
   Leu Glu Glu Arg Glu Lys Lys Val Tyr Pro Lys Ala Val Glu Arg Ser
   1570 1575 1580
   Ser Thr Phe Glu Asn Lys Ala Ser Met Gln Glu Ala Pro Pro Leu Gly
   1585 1590 1595 1600
   Ser Leu Leu Lys Asp Ala Leu His Lys Gln Ala Ser Val Arg Ala Ser
   1605 1610 1615
   Glu Gly Ala Met Ser Asp Gly Arg Val Pro Ala Glu His Arg Gln Gly
   1620 1625 1630
   Gly Gly Asp Phe Arg Arg Ala Pro Ala Pro Gly Thr Leu Gln Asp Gly
   1635 1640 1645
   Leu Cys His Ser Leu Asp Arg Gly Ile Ser Gly Lys Gly Glu Gly Thr
   1650 1655 1660
   Glu Lys Ser Ser Gln Ala Lys Glu Leu Leu Arg Cys Glu Lys Leu Asp
   1665 1670 1675 1680
   Ser Lys Leu Ala Asn Ile Asp Tyr Leu Arg Lys Lys Met Ser Leu Glu
   1685 1690 1695
   Asp Lys Glu Asp Asn Leu Cys Pro Val Leu Lys Pro Lys Met Thr Ala
   1700 1705 1710
   Gly Ser His Glu Cys Leu Pro Gly Asn Pro Val Arg Pro Thr Gly Gly
   1715 1720 1725
   Gln Gln Glu Pro Pro Pro Ala Ser Glu Ser Arg Ala Phe Val Ser Ser
   1730 1735 1740
   Thr His Ala Ala Gln Met Ser Ala Val Ser Phe Val Pro Leu Lys Ala
   1745 1750 1755 1760
   Leu Thr Gly Arg Val Asp Ser Gly Thr Glu Lys Pro Gly Leu Val Ala
   1765 1770 1775
   Pro Glu Ser Pro Val Arg Lys Ser Pro Ser Glu Tyr Lys Leu Glu Gly
   1780 1785 1790
   Arg Ser Val Ser Cys Leu Lys Pro Ile Glu Gly Thr Leu Asp Ile Ala
   1795 1800 1805
   Leu Leu Ser Gly Pro Gln Ala Ser Lys Thr Glu Leu Pro Ser Pro Glu
       1810                1815                1820
   Ser Ala Gln Ser Pro Ser Pro Ser Gly Asp Val Arg Ala Ser Val Pro
   1825                1830                1835                1840
   Pro Val Leu Pro Ser Ser Ser Gly Lys Lys Asn Asp Thr Thr Ser Ala
                   1845                1850                1855
   Arg Glu Leu Ser Pro Ser Ser Leu Lys Met Asn Lys Ser Tyr Leu Leu
               1860                1865                1870
   Glu Pro Trp Phe Leu Pro Pro Ser Arg Gly Leu Gln Asn Ser Pro Ala
           1875                1880                1885
   Val Ser Leu Pro Asp Pro Glu Phe Lys Arg Asp Arg Lys Gly Pro His
       1890                1895                1900
   Pro Thr Ala Arg Ser Pro Gly Thr Val Met Glu Ser Asn Pro Gln Gln
   1905                1910                1915                1920
   Arg Glu Gly Ser Ser Pro Lys His Gln Asp His Thr Thr Asp Pro Lys
                   1925                1930                1935
   Leu Leu Thr Cys Leu Gly Gln Asn Leu His Ser Pro Asp Leu Ala Arg
               1940                1945                1950
   Pro Arg Cys Pro Leu Pro Pro Glu Ala Ser Pro Ser Arg Glu Lys Pro
           1955                1960                1965
   Gly Leu Arg Glu Ser Ser Glu Arg Gly Pro Pro Thr Ala Arg Ser Glu
       1970                1975                1980
   Arg Ser Ala Ala Arg Ala Asp Thr Cys Arg Glu Pro Ser Met Glu Leu
   1985                1990                1995                2000
   Cys Phe Pro Glu Thr Ala Lys Thr Ser Asp Asn Ser Lys Asn Leu Leu
                   2005                2010                2015
   Ser Val Gly Arg Thr His Pro Asp Phe Tyr Thr Gln Thr Gln Ala Met
               2020                2025                2030
   Glu Lys Ala Trp Ala Pro Gly Gly Lys Thr Asn His Lys Asp Gly Pro
           2035                2040                2045
   Gly Glu Ala Arg Pro Pro Pro Arg Asp Asn Ser Ser Leu His Ser Ala
       2050                2055                2060
   Gly Ile Pro Cys Glu Lys Glu Leu Gly Lys Val Arg Arg Gly Val Glu
   2065                2070                2075                2080
   Pro Lys Pro Glu Ala Leu Leu Ala Arg Arg Ser Leu Gln Pro Pro Gly
                   2085                2090                2095
   Ile Glu Ser Glu Lys Ser Glu Lys Leu Ser Ser Phe Pro Ser Leu Gln
               2100                2105                2110
   Lys Asp Gly Ala Lys Glu Pro Glu Arg Lys Glu Gln Pro Leu Gln Arg
           2115                2120                2125
   His Pro Ser Ser Ile Pro Pro Pro Pro Leu Thr Ala Lys Asp Leu Ser
       2130                2135                2140
   Ser Pro Ala Ala Arg Gln His Cys Ser Ser Pro Ser His Ala Ser Gly
   2145 2150 2155 2160
   Arg Glu Pro Gly Ala Lys Pro Ser Thr Ala Glu Pro Ser Ser Ser Pro
   2165 2170 2175
   Gln Asp Pro Pro Lys Pro Val Ala Ala His Ser Glu Ser Ser Ser His
   2180 2185 2190
   Lys Pro Arg Pro Gly Pro Asp Pro Gly Pro Pro Lys Thr Lys His Pro
   2195 2200 2205
   Asp Arg Ser Leu Ser Ser Gln Lys Pro Ser Val Gly Ala Thr Lys Gly
   2210 2215 2220
   Lys Glu Pro Ala Thr Gln Ser Leu Gly Gly Ser Ser Arg Glu Gly Lys
   2225 2230 2235 2240
   Gly His Ser Lys Ser Gly Pro Asp Val Phe Pro Ala Thr Pro Gly Ser
   2245 2250 2255
   Gln Asn Lys Ala Ser Asp Gly Ile Gly Gln Gly Glu Gly Gly Pro Ser
   2260 2265 2270
   Val Pro Leu His Thr Asp Arg Ala Pro Leu Asp Ala Lys Pro Gln Pro
   2275 2280 2285
   Thr Ser Gly Gly Arg Pro Leu Glu Val Leu Glu Lys Pro Val His Leu
   2290 2295 2300
   Pro Arg Pro Gly His Pro Gly Pro Ser Glu Pro Ala Asp Gln Lys Leu
   2305 2310 2315 2320
   Ser Ala Val Gly Glu Lys Gln Thr Leu Ser Pro Lys His Pro Lys Pro
   2325 2330 2335
   Ser Thr Val Lys Asp Cys Pro Thr Leu Cys Lys Gln Thr Asp Asn Arg
   2340 2345 2350
   Gln Thr Asp Lys Ser Pro Ser Gln Pro Ala Ala Asn Thr Asp Arg Arg
   2355 2360 2365
   Ala Glu Gly Lys Lys Cys Thr Glu Ala Leu Tyr Ala Pro Ala Glu Gly
   2370 2375 2380
   Asp Lys Leu Glu Ala Gly Leu Ser Phe Val His Ser Glu Asn Arg Leu
   2385 2390 2395 2400
   Lys Gly Ala Glu Arg Pro Ala Ala Gly Val Gly Lys Gly Phe Pro Glu
   2405 2410 2415
   Ala Arg Gly Lys Gly Pro Gly Pro Gln Lys Pro Pro Thr Glu Ala Asp
   2420 2425 2430
   Lys Pro Asn Gly Met Lys Arg Ser Pro Ser Ala Thr Gly Gln Ser Ser
   2435 2440 2445
   Phe Arg Ser Thr Ala Leu Pro Glu Lys Ser Leu Ser Cys Ser Ser Ser
   2450 2455 2460
   Phe Pro Glu Thr Arg Ala Gly Val Arg Glu Ala Ser Ala Ala Ser Ser
   2465 2470 2475 2480
   Asp Thr Ser Ser Ala Lys Ala Ala Gly Gly Met Leu Glu Leu Pro Ala
   2485 2490 2495
   Pro Ser Asn Arg Asp His Arg Lys Ala Gln Pro Ala Gly Glu Gly Arg
   2500 2505 2510
   Thr His Met Thr Lys Ser Asp Ser Leu Pro Ser Phe Arg Val Ser Thr
   2515 2520 2525
   Leu Pro Leu Glu Ser His His Pro Asp Pro Asn Thr Met Gly Gly Ala
   2530 2535 2540
   Ser His Arg Asp Arg Ala Leu Ser Val Thr Ala Thr Val Gly Glu Thr
   2545 2550 2555 2560
   Lys Gly Lys Asp Pro Ala Pro Ala Gln Pro Pro Pro Ala Arg Lys Gln
   2565 2570 2575
   Asn Val Gly Arg Asp Val Thr Lys Pro Ser Pro Ala Pro Asn Thr Asp
   2580 2585 2590
   Arg Pro Ile Ser Leu Ser Asn Glu Lys Asp Phe Val Val Arg Gln Arg
   2595 2600 2605
   Arg Gly Lys Glu Ser Leu Arg Ser Ser Pro His Lys Lys Ala Leu
   2610 2615 2620
<210> 138
   <211> 2241
   <212> PRT
   <213> Artificial Sequence
<220>
   <223> MAST4 protein fragment
<400> 138
   Ala Thr Ala Gln Met Glu Glu Arg Leu Lys Glu Ile Ile Thr Ser Tyr
   1 5 10 15
   Ser Pro Asp Asn Val Leu Pro Leu Ala Asp Gly Val Leu Ser Phe Thr
   20 25 30
   His His Gln Ile Ile Glu Leu Ala Arg Asp Cys Leu Asp Lys Ser His
   35 40 45
   Gln Gly Leu Ile Thr Ser Arg Tyr Phe Leu Glu Leu Gln His Lys Leu
   50 55 60
   Asp Lys Leu Leu Gln Glu Ala His Asp Arg Ser Glu Ser Gly Glu Leu
   65 70 75 80
   Ala Phe Ile Lys Gln Leu Val Arg Lys Ile Leu Ile Val Ile Ala Arg
   85 90 95
   Pro Ala Arg Leu Leu Glu Cys Leu Glu Phe Asp Pro Glu Glu Phe Tyr
   100 105 110
   Tyr Leu Leu Glu Ala Ala Glu Gly His Ala Lys Glu Gly Gln Gly Ile
   115 120 125
   Lys Thr Asp Ile Pro Arg Tyr Ile Ile Ser Gln Leu Gly Leu Asn Lys
   130 135 140
   Asp Pro Leu Glu Glu Met Ala His Leu Gly Asn Tyr Asp Ser Gly Thr
   145 150 155 160
   Ala Glu Thr Pro Glu Thr Asp Glu Ser Val Ser Ser Ser Asn Ala Ser
   165 170 175
   Leu Lys Leu Arg Arg Lys Pro Arg Glu Ser Asp Phe Glu Thr Ile Lys
   180 185 190
   Leu Ile Ser Asn Gly Ala Tyr Gly Ala Val Tyr Phe Val Arg His Lys
   195 200 205
   Glu Ser Arg Gln Arg Phe Ala Met Lys Lys Ile Asn Lys Gln Asn Leu
   210 215 220
   Ile Leu Arg Asn Gln Ile Gln Gln Ala Phe Val Glu Arg Asp Ile Leu
   225 230 235 240
   Thr Phe Ala Glu Asn Pro Phe Val Val Ser Met Tyr Cys Ser Phe Glu
   245 250 255
   Thr Arg Arg His Leu Cys Met Val Met Glu Tyr Val Glu Gly Gly Asp
   260 265 270
   Cys Ala Thr Leu Met Lys Asn Met Gly Pro Leu Pro Val Asp Met Ala
   275 280 285
   Arg Met Tyr Phe Ala Glu Thr Val Leu Ala Leu Glu Tyr Leu His Asn
   290 295 300
   Tyr Gly Ile Val His Arg Asp Leu Lys Pro Asp Asn Leu Leu Val Thr
   305 310 315 320
   Ser Met Gly His Ile Lys Leu Thr Asp Phe Gly Leu Ser Lys Val Gly
   325 330 335
   Leu Met Ser Met Thr Thr Asn Leu Tyr Glu Gly His Ile Glu Lys Asp
   340 345 350
   Ala Arg Glu Phe Leu Asp Lys Gln Val Cys Gly Thr Pro Glu Tyr Ile
   355 360 365
   Ala Pro Glu Val Ile Leu Arg Gln Gly Tyr Gly Lys Pro Val Asp Trp
   370 375 380
   Trp Ala Met Gly Ile Ile Leu Tyr Glu Phe Leu Val Gly Cys Val Pro
   385 390 395 400
   Phe Phe Gly Asp Thr Pro Glu Glu Leu Phe Gly Gln Val Ile Ser Asp
   405 410 415
   Glu Ile Asn Trp Pro Glu Lys Asp Glu Ala Pro Pro Pro Asp Ala Gln
   420 425 430
   Asp Leu Ile Thr Leu Leu Leu Arg Gln Asn Pro Leu Glu Arg Leu Gly
   435 440 445
   Thr Gly Gly Ala Tyr Glu Val Lys Gln His Arg Phe Phe Arg Ser Leu
   450 455 460
   Asp Trp Asn Ser Leu Leu Arg Gln Lys Ala Glu Phe Ile Pro Gln Leu
   465                 470                 475                 480
   Glu Ser Glu Asp Asp Thr Ser Tyr Phe Asp Thr Arg Ser Glu Lys Tyr
                   485                 490                 495
   His His Met Glu Thr Glu Glu Glu Asp Asp Thr Asn Asp Glu Asp Phe
               500                 505                 510
   Asn Val Glu Ile Arg Gln Phe Ser Ser Cys Ser His Arg Phe Ser Lys
           515                 520                 525
   Val Phe Ser Ser Ile Asp Arg Ile Thr Gln Asn Ser Ala Glu Glu Lys
       530                 535                 540
   Glu Asp Ser Val Asp Lys Thr Lys Ser Thr Thr Leu Pro Ser Thr Glu
   545                 550                 555                 560
   Thr Leu Ser Trp Ser Ser Glu Tyr Ser Glu Met Gln Gln Leu Ser Thr
                   565                 570                 575
   Ser Asn Ser Ser Asp Thr Glu Ser Asn Arg His Lys Leu Ser Ser Gly
               580                 585                 590
   Leu Leu Pro Lys Leu Ala Ile Ser Thr Glu Gly Glu Gln Asp Glu Ala
           595                 600                 605
   Ala Ser Cys Pro Gly Asp Pro His Glu Glu Pro Gly Lys Pro Ala Leu
       610                 615                 620
   Pro Pro Glu Glu Cys Ala Gln Glu Glu Pro Glu Val Thr Thr Pro Ala
   625                 630                 635                 640
   Ser Thr Ile Ser Ser Ser Thr Leu Ser Val Gly Ser Phe Ser Glu His
                   645                 650                 655
   Leu Asp Gln Ile Asn Gly Arg Ser Glu Cys Val Asp Ser Thr Asp Asn
               660                 665                 670
   Ser Ser Lys Pro Ser Ser Glu Pro Ala Ser His Met Ala Arg Gln Arg
           675                 680                 685
   Leu Glu Ser Thr Glu Lys Lys Lys Ile Ser Gly Lys Val Thr Lys Ser
       690                 695                 700
   Leu Ser Ala Ser Ala Leu Ser Leu Met Ile Pro Gly Asp Met Phe Ala
   705                 710                 715                 720
   Val Ser Pro Leu Gly Ser Pro Met Ser Pro His Ser Leu Ser Ser Asp
                   725                 730                 735
   Pro Ser Ser Ser Arg Asp Ser Ser Pro Ser Arg Asp Ser Ser Ala Ala
               740                 745                 750
   Ser Ala Ser Pro His Gln Pro Ile Val Ile His Ser Ser Gly Lys Asn
           755                 760                 765
   Tyr Gly Phe Thr Ile Arg Ala Ile Arg Val Tyr Val Gly Asp Ser Asp
       770                 775                 780
   Ile Tyr Thr Val His His Ile Val Trp Asn Val Glu Glu Gly Ser Pro
   785                 790                 795                 800
   Ala Cys Gln Ala Gly Leu Lys Ala Gly Asp Leu Ile Thr His Ile Asn
                   805                 810                 815
   Gly Glu Pro Val His Gly Leu Val His Thr Glu Val Ile Glu Leu Leu
               820                 825                 830
   Leu Lys Ser Gly Asn Lys Val Ser Ile Thr Thr Thr Pro Phe Glu Asn
           835                 840                 845
   Thr Ser Ile Lys Thr Gly Pro Ala Arg Arg Asn Ser Tyr Lys Ser Arg
       850                 855                 860
   Met Val Arg Arg Ser Lys Lys Ser Lys Lys Lys Glu Ser Leu Glu Arg
   865                 870                 875                 880
   Arg Arg Ser Leu Phe Lys Lys Leu Ala Lys Gln Pro Ser Pro Leu Leu
                   885                 890                 895
   His Thr Ser Arg Ser Phe Ser Cys Leu Asn Arg Ser Leu Ser Ser Gly
               900                 905                 910
   Glu Ser Leu Pro Gly Ser Pro Thr His Ser Leu Ser Pro Arg Ser Pro
           915                 920                 925
   Thr Pro Ser Tyr Arg Ser Thr Pro Asp Phe Pro Ser Gly Thr Asn Ser
       930                 935                 940
   Ser Gln Ser Ser Ser Pro Ser Ser Ser Ala Pro Asn Ser Pro Ala Gly
   945                 950                 955                 960
   Ser Gly His Ile Arg Pro Ser Thr Leu His Gly Leu Ala Pro Lys Leu
                   965                 970                 975
   Gly Gly Gln Arg Tyr Arg Ser Gly Arg Arg Lys Ser Ala Gly Asn Ile
               980                 985                 990
   Pro Leu Ser Pro Leu Ala Arg Thr Pro Ser Pro Thr Pro Gln Pro Thr
           995                 1000                1005
   Ser Pro Gln Arg Ser Pro Ser Pro Leu Leu Gly His Ser Leu Gly Asn
      1010                1015                1020
   Ser Lys Ile Ala Gln Ala Phe Pro Ser Lys Met His Ser Pro Pro Thr
   1025               1030                1035                1040
   Ile Val Arg His Ile Val Arg Pro Lys Ser Ala Glu Pro Pro Arg Ser
                  1045                1050                1055
   Pro Leu Leu Lys Arg Val Gln Ser Glu Glu Lys Leu Ser Pro Ser Tyr
              1060                1065                1070
   Gly Ser Asp Lys Lys His Leu Cys Ser Arg Lys His Ser Leu Glu Val
          1075                1080                1085
   Thr Gln Glu Glu Val Gln Arg Glu Gln Ser Gln Arg Glu Ala Pro Leu
      1090                1095                1100
   Gln Ser Leu Asp Glu Asn Val Cys Asp Val Pro Pro Leu Ser Arg Ala
   1105               1110                1115                1120
   Arg Pro Val Glu Gln Gly Cys Leu Lys Arg Pro Val Ser Arg Lys Val
                  1125                1130                1135
   Gly Arg Gln Glu Ser Val Asp Asp Leu Asp Arg Asp Lys Leu Lys Ala
   1140 1145 1150
   Lys Val Val Val Lys Lys Ala Asp Gly Phe Pro Glu Lys Gln Glu Ser
   1155 1160 1165
   His Gln Lys Ser His Gly Pro Gly Ser Asp Leu Glu Asn Phe Ala Leu
   1170 1175 1180
   Phe Lys Leu Glu Glu Arg Glu Lys Lys Val Tyr Pro Lys Ala Val Glu
   1185 1190 1195 1200
   Arg Ser Ser Thr Phe Glu Asn Lys Ala Ser Met Gln Glu Ala Pro Pro
   1205 1210 1215
   Leu Gly Ser Leu Leu Lys Asp Ala Leu His Lys Gln Ala Ser Val Arg
   1220 1225 1230
   Ala Ser Glu Gly Ala Met Ser Asp Gly Arg Val Pro Ala Glu His Arg
   1235 1240 1245
   Gln Gly Gly Gly Asp Phe Arg Arg Ala Pro Ala Pro Gly Thr Leu Gln
   1250 1255 1260
   Asp Gly Leu Cys His Ser Leu Asp Arg Gly Ile Ser Gly Lys Gly Glu
   1265 1270 1275 1280
   Gly Thr Glu Lys Ser Ser Gln Ala Lys Glu Leu Leu Arg Cys Glu Lys
   1285 1290 1295
   Leu Asp Ser Lys Leu Ala Asn Ile Asp Tyr Leu Arg Lys Lys Met Ser
   1300 1305 1310
   Leu Glu Asp Lys Glu Asp Asn Leu Cys Pro Val Leu Lys Pro Lys Met
   1315 1320 1325
   Thr Ala Gly Ser His Glu Cys Leu Pro Gly Asn Pro Val Arg Pro Thr
   1330 1335 1340
   Gly Gly Gln Gln Glu Pro Pro Pro Ala Ser Glu Ser Arg Ala Phe Val
   1345 1350 1355 1360
   Ser Ser Thr His Ala Ala Gln Met Ser Ala Val Ser Phe Val Pro Leu
   1365 1370 1375
   Lys Ala Leu Thr Gly Arg Val Asp Ser Gly Thr Glu Lys Pro Gly Leu
   1380 1385 1390
   Val Ala Pro Glu Ser Pro Val Arg Lys Ser Pro Ser Glu Tyr Lys Leu
   1395 1400 1405
   Glu Gly Arg Ser Val Ser Cys Leu Lys Pro Ile Glu Gly Thr Leu Asp
   1410 1415 1420
   Ile Ala Leu Leu Ser Gly Pro Gln Ala Ser Lys Thr Glu Leu Pro Ser
   1425 1430 1435 1440
   Pro Glu Ser Ala Gln Ser Pro Ser Pro Ser Gly Asp Val Arg Ala Ser
   1445 1450 1455
   Val Pro Pro Val Leu Pro Ser Ser Ser Gly Lys Lys Asn Asp Thr Thr
   1460 1465 1470
   Ser Ala Arg Glu Leu Ser Pro Ser Ser Leu Lys Met Asn Lys Ser Tyr
           1475                1480                1485
   Leu Leu Glu Pro Trp Phe Leu Pro Pro Ser Arg Gly Leu Gln Asn Ser
       1490                1495                1500
   Pro Ala Val Ser Leu Pro Asp Pro Glu Phe Lys Arg Asp Arg Lys Gly
   1505                1510                1515                1520
   Pro His Pro Thr Ala Arg Ser Pro Gly Thr Val Met Glu Ser Asn Pro
                   1525                1530                1535
   Gln Gln Arg Glu Gly Ser Ser Pro Lys His Gln Asp His Thr Thr Asp
               1540                1545                1550
   Pro Lys Leu Leu Thr Cys Leu Gly Gln Asn Leu His Ser Pro Asp Leu
           1555                1560                1565
   Ala Arg Pro Arg Cys Pro Leu Pro Pro Glu Ala Ser Pro Ser Arg Glu
       1570                1575                1580
   Lys Pro Gly Leu Arg Glu Ser Ser Glu Arg Gly Pro Pro Thr Ala Arg
   1585                1590                1595                1600
   Ser Glu Arg Ser Ala Ala Arg Ala Asp Thr Cys Arg Glu Pro Ser Met
                   1605                1610                1615
   Glu Leu Cys Phe Pro Glu Thr Ala Lys Thr Ser Asp Asn Ser Lys Asn
               1620                1625                1630
   Leu Leu Ser Val Gly Arg Thr His Pro Asp Phe Tyr Thr Gln Thr Gln
           1635                1640                1645
   Ala Met Glu Lys Ala Trp Ala Pro Gly Gly Lys Thr Asn His Lys Asp
       1650                1655                1660
   Gly Pro Gly Glu Ala Arg Pro Pro Pro Arg Asp Asn Ser Ser Leu His
   1665                1670                1675                1680
   Ser Ala Gly Ile Pro Cys Glu Lys Glu Leu Gly Lys Val Arg Arg Gly
                   1685                1690                1695
   Val Glu Pro Lys Pro Glu Ala Leu Leu Ala Arg Arg Ser Leu Gln Pro
               1700                1705                1710
   Pro Gly Ile Glu Ser Glu Lys Ser Glu Lys Leu Ser Ser Phe Pro Ser
           1715                1720                1725
   Leu Gln Lys Asp Gly Ala Lys Glu Pro Glu Arg Lys Glu Gln Pro Leu
       1730                1735                1740
   Gln Arg His Pro Ser Ser Ile Pro Pro Pro Pro Leu Thr Ala Lys Asp
   1745                1750                1755                1760
   Leu Ser Ser Pro Ala Ala Arg Gln His Cys Ser Ser Pro Ser His Ala
                   1765                1770                1775
   Ser Gly Arg Glu Pro Gly Ala Lys Pro Ser Thr Ala Glu Pro Ser Ser
               1780                1785                1790
   Ser Pro Gln Asp Pro Pro Lys Pro Val Ala Ala His Ser Glu Ser Ser
           1795                1800                1805
   Ser His Lys Pro Arg Pro Gly Pro Asp Pro Gly Pro Pro Lys Thr Lys
   1810 1815 1820
   His Pro Asp Arg Ser Leu Ser Ser Gln Lys Pro Ser Val Gly Ala Thr
   1825 1830 1835 1840
   Lys Gly Lys Glu Pro Ala Thr Gln Ser Leu Gly Gly Ser Ser Arg Glu
   1845 1850 1855
   Gly Lys Gly His Ser Lys Ser Gly Pro Asp Val Phe Pro Ala Thr Pro
   1860 1865 1870
   Gly Ser Gln Asn Lys Ala Ser Asp Gly Ile Gly Gln Gly Glu Gly Gly
   1875 1880 1885
   Pro Ser Val Pro Leu His Thr Asp Arg Ala Pro Leu Asp Ala Lys Pro
   1890 1895 1900
   Gln Pro Thr Ser Gly Gly Arg Pro Leu Glu Val Leu Glu Lys Pro Val
   1905 1910 1915 1920
   His Leu Pro Arg Pro Gly His Pro Gly Pro Ser Glu Pro Ala Asp Gln
   1925 1930 1935
   Lys Leu Ser Ala Val Gly Glu Lys Gln Thr Leu Ser Pro Lys His Pro
   1940 1945 1950
   Lys Pro Ser Thr Val Lys Asp Cys Pro Thr Leu Cys Lys Gln Thr Asp
   1955 1960 1965
   Asn Arg Gln Thr Asp Lys Ser Pro Ser Gln Pro Ala Ala Asn Thr Asp
   1970 1975 1980
   Arg Arg Ala Glu Gly Lys Lys Cys Thr Glu Ala Leu Tyr Ala Pro Ala
   1985 1990 1995 2000
   Glu Gly Asp Lys Leu Glu Ala Gly Leu Ser Phe Val His Ser Glu Asn
   2005 2010 2015
   Arg Leu Lys Gly Ala Glu Arg Pro Ala Ala Gly Val Gly Lys Gly Phe
   2020 2025 2030
   Pro Glu Ala Arg Gly Lys Gly Pro Gly Pro Gln Lys Pro Pro Thr Glu
   2035 2040 2045
   Ala Asp Lys Pro Asn Gly Met Lys Arg Ser Pro Ser Ala Thr Gly Gln
   2050 2055 2060
   Ser Ser Phe Arg Ser Thr Ala Leu Pro Glu Lys Ser Leu Ser Cys Ser
   2065 2070 2075 2080
   Ser Ser Phe Pro Glu Thr Arg Ala Gly Val Arg Glu Ala Ser Ala Ala
   2085 2090 2095
   Ser Ser Asp Thr Ser Ser Ala Lys Ala Ala Gly Gly Met Leu Glu Leu
   2100 2105 2110
   Pro Ala Pro Ser Asn Arg Asp His Arg Lys Ala Gln Pro Ala Gly Glu
   2115 2120 2125
   Gly Arg Thr His Met Thr Lys Ser Asp Ser Leu Pro Ser Phe Arg Val
   2130 2135 2140
   Ser Thr Leu Pro Leu Glu Ser His His Pro Asp Pro Asn Thr Met Gly
   2145 2150 2155 2160
   Gly Ala Ser His Arg Asp Arg Ala Leu Ser Val Thr Ala Thr Val Gly
   2165 2170 2175
   Glu Thr Lys Gly Lys Asp Pro Ala Pro Ala Gln Pro Pro Pro Ala Arg
   2180 2185 2190
   Lys Gln Asn Val Gly Arg Asp Val Thr Lys Pro Ser Pro Ala Pro Asn
   2195 2200 2205
   Thr Asp Arg Pro Ile Ser Leu Ser Asn Glu Lys Asp Phe Val Val Arg
   2210 2215 2220
   Gln Arg Arg Gly Lys Glu Ser Leu Arg Ser Ser Pro His Lys Lys Ala
   2225 2230 2235 2240
   Leu
<210> 139
   <211> 8
   <212> PRT
   <213> Artificial Sequence
<220>
   <223> Break-point of MAST4 protein fragment
<400> 139
   Ala Thr Ala Gln Met Glu Glu Arg
   1 5
<210> 140
   <211> 10527
   <212> DNA
   <213> Artificial Sequence
<220>
   <223> ERBB2IP-MAST4 fusion gene
<400> 140
   atgactacaa aacgaagttt gtttgtgcgg ttggtaccat gtcgctgtct acgaggggaa        60
   gaggagactg tcactactct tgattattct cattgcagct tagaacaagt tccgaaagag       120
   atttttactt ttgaaaaaac cttggaggaa ctctatttag atgctaatca gattgaagag       180
   cttccaaagc aactttttaa ctgtcagtct ttacacaaac tgagtttgcc agacaatgat       240
   ttaacaacgt taccagcatc cattgcaaac cttattaatc tcagggaact ggatgtcagc       300
   aagaatggaa tacaggagtt tccagaaaat ataaaaaatt gtaaagtttt gacaattgtg       360
   gaggccagtg taaaccctat ttccaagctc cctgatggat tttctcagct gttaaaccta       420
   acccagttgt atctgaatga tgcttttctt gagttcttgc cagcaaattt tggcagatta       480
   actaaactcc aaatattaga gcttagagaa aaccagttaa aaatgttgcc taaaactatg       540
   aatagactga cccagctgga aagactggat ttgggaagta acgaattcac ggaagtgcct 600
   gaagtacttg agcaactaag tggattgaaa gagttttgga tggatgctaa tagactgact 660
   tttattccag ggtttattgg tagtttgaaa cagctcacat atttggatgt ttctaaaaat 720
   aatattgaaa tggttgaaga aggaatttca acatgtgaaa accttcaaga cctcctatta 780
   tcaagcaatt cacttcagca gcttcctgag actattggtt cgttgaagaa tataacaacg 840
   cttaaaatag atgaaaacca gttaatgtat ctgccagact ctataggagg gttaatatca 900
   gtagaagaac tggattgtag tttcaatgaa gttgaagctt tgccttcatc tattgggcag 960
   cttactaact taagaacttt tgctgctgat cataattact tacagcagtt gcccccagag 1020
   attggaagct ggaaaaatat aactgtgctg tttctccatt ccaataaact tgagacactt 1080
   ccagaggaaa tgggtgatat gcaaaaatta aaagtcatta atttaagtga taatagatta 1140
   aagaatttac cctttagctt tacaaagcta cagcaattga cagctatgtg gctctcagat 1200
   aatcagtcca aacccctgat acctcttcaa aaagaaactg attcagagac ccagaaaatg 1260
   gtgcttacca actacatgtt ccctcaacag ccaaggactg aggatgttat gtttatatca 1320
   gataatgaaa gttttaaccc ttcattgtgg gaggaacaga ggaaacagcg ggctcaagtt 1380
   gcatttgaat gtgatgaaga caaagatgaa agggaggcac ctcccaggga gggaaattta 1440
   aaaagatatc caacaccata cccagatgag cttaagaata tggtcaaaac tgttcaaacc 1500
   attgtacata gattaaaaga tgaagagacc aatgaagact caggaagaga tttgaaacca 1560
   catgaagatc aacaagatat aaataaagat gtgggtgtga agacctcaga aagtactact 1620
   acagtaaaaa gcaaagttga tgaaagagaa aaatatatga taggaaactc tgtacagaag 1680
   atcagtgaac ctgaagctga gattagtcct gggagtttac cagtgactgc aaatatgaaa 1740
   gcctctgaga acttgaagca tattgttaac catgatgatg tttttgagga atctgaagaa 1800
   ctttcttctg atgaagagat gaaaatggcg gagatgcgac caccattaat tgaaacctct 1860
   attaaccagc caaaagtcgt agcacttagt aataacaaaa aagatgatac aaaggaaaca 1920
   gattctttat cagatgaagt tacacacaat agcaatcaga ataacagcaa ttgttcttct 1980
   ccatctcgga tgtctgattc agtttctctt aatactgata gtagtcaaga cacctcactc 2040
   tgctctccag tgaaacaaac tcatattgat attaattcca aaatcaggca agaagatgaa 2100
   aattttaaca gccttttaca aaatggagat attttaaaca gttcaacaga ggaaaagttc 2160
   aaagctcatg ataaaaaaga ttttaactta cctgaatatg atttgaatgt tgaagagcga 2220
   ttagttctaa ttgagaaaag tgttgactca acagccacag ctgatgacac tcacaaatta 2280
   gatcatatca atatgaatct taataaactt ataactaatg atacatttca accagagatc 2340
   atggaaagat caaaaacaca ggatattgtg cttggaacaa gctttttaag cattaattct 2400
   aaagaggaaa ctgagcactt ggaaaatgga aacaagtatc ctaatttgga atccgtaaat 2460
   aaggtaaatg gacattctga ggaaacttcc cagtctccta ataggactga accacatgac 2520
   agtgattgtt ctgttgactt aggtatttcc aaaagcactg aagatctctc ccctcagaaa 2580
   agtggtccag ttggatctgt tgtgaaatct catagcataa ctaatatgga gattggaggg 2640
   ctaaaaatct atgatattct tagtgataat ggacctcagc agccaagtac aaccgttaaa 2700
   atcacatctg ctgttgatgg aaaaaatata gtcaggagca agtctgccac actgttgtat 2760
   gatcaaccat tgcaggtatt tactggttct tcctcatctt ctgatttaat atcaggaaca 2820
   aaggcaattt tcaagtttga ttcaaatcat aatcccgaag agccaaatat aataagaggc 2880
   cccacaagtg gcccacaatc tgcacctcaa atatatggtc ctccacagta taatatccaa 2940
   tacagtagca gtgctgcagt caaagacact ttgtggcact ccaaacaaaa tccccaaata 3000
   gaccatgcca gttttcctcc tcagctcctt cctagatcag agagcacaga aaatcaaagt 3060
   tatgctaaac attctgccaa tatgaatttc tctaatcata acaatgttcg agctaatact 3120
   gcataccatt tacatcagag acttggccca gcaagacatg gggaaatgtg ggccatctca 3180
   ccaaacgacc gacttattcc tgcagtaact cgaagtacaa tccagcgaca aagtagtgtg 3240
   tcctccacag cctctgtaaa tcttggtgat ccaggctcta caaggcgggc tcagattcct 3300
   gaaggagatt atttatcata cagagagttc cactcagcgg gaagaactcc tccaatgatg 3360
   ccaggatcac agagacccct ttctgcacga acatacagca tagatggtcc aaatgcatca 3420
   agacctcaga gtgctcgacc ctctattaat gaaataccag agagaactat gtcagttagt 3480
   gatttcaatt attcacggac tagtccttca aaaagaccaa atgcaagggt tggttctgag 3540
   cattctttat tagatcctcc aggaaaaagt aaagttcctc gtgactggag agaacaagta 3600
   cttcgacata ttgaagccaa aaagttagaa aagattcgag tgagggttga aaaggatcca 3660
   gaacttggat ttagcatatc aggtggtgtc gggggtagag gaaacccatt cagacctgat 3720
   gatgatggta tatttgtaac aagggtacaa cctgaaggac cagcatcaaa attactgcag 3780
   ccaggtgata aaattattca ggctacagct cagatggaag aacgtctaaa ggaaattatc 3840
   accagctact ctcctgacaa cgttctaccc ttagcagatg gagtgcttag tttcactcac 3900
   caccagatta ttgaactggc tcgagattgc ttggataaat cccaccaggg cctcatcacc 3960
   tcacgatact tccttgaatt acagcacaaa ttagataagt tgctacagga ggctcatgat 4020
   cgttcagaaa gtggagaatt ggcatttatt aaacaactag ttcgaaagat cctaattgtt 4080
   attgcccgcc ctgctcggtt attagagtgc ctggaatttg atccggaaga attttactac 4140
   ctattggaag cagcagaagg ccatgccaaa gaaggacagg gtattaaaac cgacattccc 4200
   aggtacatca ttagccaact gggactcaat aaggatccct tggaagaaat ggctcatttg 4260
   ggaaactacg atagtgggac agcagaaaca ccagaaacag atgaatcagt gagtagctct 4320
   aatgcctccc tgaaacttcg aaggaaacct cgggaaagtg attttgaaac gattaaattg 4380
   attagcaatg gagcctatgg ggcagtctac tttgttcggc ataaagaatc ccggcagagg 4440
   tttgccatga agaagattaa taaacagaac ctcatccttc gaaaccagat ccagcaggcc 4500
   tttgtggagc gggatatcct gacttttgca gaaaacccct ttgttgtcag catgtattgc 4560
   tcctttgaaa caaggcgcca cttgtgcatg gtcatggaat atgtggaagg gggagactgt 4620
   gctactttaa tgaaaaacat gggtcctctc cctgttgata tggccagaat gtactttgct 4680
   gagacggtct tggccttgga atatttacat aattatggaa ttgtacacag ggatttgaaa 4740
   ccagacaact tgttggttac ctccatgggg cacataaagc tgacagattt tggattatct 4800
   aaggtgggac taatgagcat gactaccaac ctttacgagg gtcatattga gaaggatgct 4860
   agagagttcc tggataaaca ggtctgtggc acacctgaat acattgcacc agaagtgatt 4920
   ctgaggcagg gttatggaaa gccggtggac tggtgggcca tggggattat cctctatgaa 4980
   tttctggttg gatgcgtgcc attctttggg gatactccag aggagctatt tggacaagtc 5040
   atcagtgatg agatcaactg gcctgagaag gatgaggcac ccccacctga tgcccaggat 5100
   ctgattacct tactcctcag gcagaatccc ctggagaggc tgggaacagg tggtgcatat 5160
   gaagtcaaac agcatcgatt cttccgttct ttagactgga acagtttgct gagacagaag 5220
   gcagaattta ttccccaact ggaatctgag gatgacacaa gttattttga tactcggtct 5280
   gagaagtatc atcatatgga aacggaggaa gaagatgaca caaatgatga agactttaat 5340
   gtggaaataa ggcagttttc ttcatgttca cacaggtttt caaaagtttt cagcagtata 5400
   gatcgaatca ctcagaattc agcagaagag aaggaagact ctgtggacaa aaccaaaagc 5460
   accaccttgc catccacaga aacactgagc tggagttcag aatattctga aatgcaacag 5520
   ctatcaacat ccaactcttc agatactgaa agcaacagac ataaactcag ttctggccta 5580
   cttcccaaac tggctatttc aacagaggga gagcaagatg aagctgcctc ctgccctgga 5640
   gacccccatg aggagccagg aaagccagcc cttcctcctg aagagtgtgc ccaggaggag 5700
   cctgaggtca ccaccccagc cagcaccatc agcagctcca ccctgtcagt tggcagtttt 5760
   tcagagcact tggatcagat aaatggacga agcgagtgtg tggacagtac agataattcc 5820
   tcaaagccat ccagtgaacc cgcttctcac atggctcggc agcgattaga aagcacagaa 5880
   aaaaagaaaa tctcggggaa agtcacaaag tccctctctg ccagtgctct ttccctcatg 5940
   atcccaggag atatgtttgc tgtttcccct ctgggaagtc caatgtctcc ccattccctg 6000
   tcctcggacc cttcttcttc acgagattcc tctcccagcc gagattcctc agcagcttct 6060
   gccagtccac atcagccgat tgtgatccac agttcgggga agaactacgg ctttaccatc 6120
   cgagccatcc gggtgtatgt gggagacagt gacatctata cagtgcacca tatcgtctgg 6180
   aatgtagaag aaggaagtcc ggcatgccag gcaggactga aggctggaga tcttatcact 6240
   cacatcaatg gagaaccagt gcatggactt gtccacacag aagttataga actcctactg 6300
   aagagtggga ataaggtgtc aatcactact accccatttg aaaacacatc aatcaaaact 6360
   ggaccagcca ggagaaacag ctataagagc cggatggtga ggcggagcaa gaaatccaag 6420
   aagaaagaaa gtctcgaaag gaggagatct cttttcaaaa agctagccaa gcagccttct 6480
   cctttactcc acaccagccg aagtttctcc tgcttgaaca gatccctgtc atcgggtgag 6540
   agcctcccag gttcccccac tcatagcttg tctccccggt ctccaacacc aagctaccgc 6600
   tccacccctg acttcccatc tggtactaat tcctcccaga gcagctcccc tagttctagt 6660
   gcccccaatt ccccagcagg gtccgggcac atccggccca gcactctcca cggtcttgca 6720
   cccaaactcg gcgggcagcg gtaccggtcc ggaaggcgaa agtccgccgg caacatccca 6780
   ctgtccccgc tggcccggac gccctctcca accccgcaac ccacctcccc gcagcggtca 6840
   ccatcccctc ttctgggaca ctcactgggc aattccaaga tcgcgcaagc ctttcccagc 6900
   aagatgcact ccccgcccac catcgtcaga cacatcgtga ggcccaagag tgcggagccc 6960
   cccaggtccc cgctgctcaa gcgcgtgcag tccgaggaga agctgtcgcc ctcttacggc 7020
   agtgacaaga agcacctgtg ctcccgcaag cacagcctgg aggtgaccca agaggaggtg 7080
   cagcgggagc agtcccagcg ggaggcgccg ctgcagagcc tggatgagaa cgtgtgcgac 7140
   gtgccgccgc tcagccgcgc ccggccagtg gagcaaggct gcctgaaacg cccagtctcc 7200
   cggaaggtgg gccgccagga gtctgtggac gacctggacc gcgacaagct gaaggccaag 7260
   gtggtggtga agaaagcaga cggcttccca gagaaacagg aatcccacca gaaatcccat 7320
   ggacccggga gtgatttgga aaactttgct ctgtttaagc tggaagagag agagaagaaa 7380
   gtctatccga aggctgtgga aaggtcaagt acttttgaaa acaaagcgtc tatgcaggag 7440
   gcgccaccgc tgggcagcct gctgaaggat gctcttcaca agcaggccag cgtgcgcgcc 7500
   agcgagggtg cgatgtcgga tggccgggtg cctgcggagc accgccaggg tggcggggac 7560
   ttcagacggg cccccgctcc tggcaccctc caggatggtc tctgccactc cctcgacagg 7620
   ggcatctctg ggaaggggga aggcacggag aagtcctccc aggccaagga gcttctccga 7680
   tgtgaaaagt tagacagcaa gctggccaac atcgattacc tccgaaagaa aatgtcactt 7740
   gaggacaaag aggacaacct ctgccctgtg ctgaagccca agatgacagc tggctcccac 7800
   gaatgcctgc cagggaaccc agtccgaccc acgggtgggc agcaggagcc cccgccggct 7860
   tctgagagcc gagcttttgt cagcagcacc catgcagctc agatgagtgc cgtctctttt 7920
   gttcccctca aggccttaac aggccgggtg gacagtggaa cggagaagcc tggcttggtt 7980
   gctcctgagt cccctgttag gaagagcccc tccgagtata agctggaagg taggtctgtc 8040
   tcatgcctga agccgatcga gggcactctg gacattgctc tcctgtccgg acctcaggcc 8100
   tccaagacag aactgccttc cccagagtct gcacagagcc ccagcccaag tggtgacgtg 8160
   agggcctctg tgccaccagt tctccccagc agcagtggga aaaagaacga taccaccagt 8220
   gcaagagagc tttctccttc cagcttaaag atgaataaat cctacctgct ggagccttgg 8280
   ttcctgcccc ccagccgagg tctccagaat tcaccagcag tttccctgcc tgacccagag 8340
   ttcaagaggg acaggaaagg tccccatcct actgccagga gccctggaac agtcatggaa 8400
   agcaatcccc aacagagaga gggcagctcc cctaaacacc aagaccacac cactgacccc 8460
   aagcttctga cctgcctggg gcagaacctc cacagccctg acctggccag gccacgctgc 8520
   ccgctcccac ctgaagcttc cccctcaagg gagaagccag gcctgaggga atcgtctgaa 8580
   agaggccctc ccacagccag aagcgagcgc tctgctgcga gggctgacac atgcagagag 8640
   ccctccatgg aactgtgctt tccagaaact gcgaaaacca gtgacaactc caaaaatctc 8700
   ctctctgtgg gaaggaccca cccagatttc tatacacaga cccaggccat ggagaaagca 8760
   tgggcgccgg gtgggaaaac gaaccacaaa gatggcccag gtgaggcgag gcccccgccc 8820
   agagacaact cctctctgca ctcagctgga attccctgtg agaaggagct gggcaaggtg 8880
   aggcgtggcg tggaacccaa gcccgaagcg cttcttgcca ggcggtctct gcagccacct 8940
   ggaattgaga gtgagaagag tgaaaagctc tccagtttcc catctttgca gaaagatggt 9000
   gccaaggaac ctgaaaggaa ggagcagcct ctacaaaggc atcccagcag catccctccg 9060
   ccccctctga cggccaaaga cctgtccagc ccggctgcca ggcagcattg cagttcccca 9120
   agccacgctt ctggcagaga gccgggggcc aagcccagca ctgcagagcc cagctcgagc 9180
   ccccaggacc ctcccaagcc tgttgctgcg cacagtgaaa gcagcagcca caagccccgg 9240
   cctggccctg acccgggccc tccaaagact aagcaccccg accggtccct ctcctctcag 9300
   aaaccaagtg tcggggccac aaagggcaaa gagcctgcca ctcagtccct cggtggctct 9360
   agcagagagg ggaagggcca cagtaagagt gggccggatg tgtttcctgc taccccaggc 9420
   tcccagaaca aagccagcga tgggattggc cagggagaag gtgggccctc tgtcccactg 9480
   cacactgaca gggctcctct agacgccaag ccacaaccca ccagtggtgg gcggcccctg 9540
   gaggtgctgg agaagcctgt gcatttgcca aggccgggac acccagggcc tagtgagcca 9600
   gcggaccaga aactgtccgc tgttggtgaa aagcaaaccc tgtctccaaa gcaccccaaa 9660
   ccatccactg tgaaagattg ccccaccctg tgcaaacaga cagacaacag acagacagac 9720
   aaaagcccga gtcagccggc cgccaacacc gacagaaggg cggaagggaa gaaatgcact 9780
   gaagcacttt atgctccagc agagggcgac aagctcgagg ccggcctttc ctttgtgcat 9840
   agcgagaacc ggttgaaagg cgcggagcgg ccagccgcgg gggtggggaa gggcttccct 9900
   gaggccagag ggaaagggcc cggtccccag aagccaccga cggaggcaga caagcccaat 9960
   ggcatgaaac ggtccccctc agccactggg cagagttctt tccgatccac ggccctcccg 10020
   gaaaagtctc tgagctgctc ctccagcttc cctgaaacca gggccggagt tagagaggcc 10080
   tctgcagcca gcagcgacac ctcttctgcc aaggccgccg ggggcatgct ggagcttcca 10140
   gcccccagca acagggacca taggaaggct cagcctgccg gggagggccg aacccacatg 10200
   acaaagagtg actccctgcc ctccttccgg gtctccaccc tgcctctgga gtcacaccac 10260
   cccgacccaa acaccatggg cggggccagc caccgggaca gggctctctc ggtgactgcc 10320
   accgtagggg aaaccaaagg gaaggaccct gccccagccc agcctccccc agctaggaaa 10380
   cagaacgtgg gcagagacgt gaccaagcca tccccagccc caaacactga ccgccccatc 10440
   tctctttcta atgagaagga ctttgtggta cggcagaggc gggggaaaga gagtttgcgt 10500
   agcagccctc acaaaaaggc cttgtaa 10527
<210> 141
   <211> 48
   <212> DNA
   <213> Artificial Sequence
<220>
   <223> Fused region of ERBB2IP-MAST4 fusion gene
<400> 141
   cagccaggtg ataaaattat tcaggctaca gctcagatgg aagaacgt 48
<210> 142
   <211> 3508
   <212> PRT
   <213> Artificial Sequence
<220>
   <223> ERBB2IP-MAST4 fusion protein
<400> 142
   Met Thr Thr Lys Arg Ser Leu Phe Val Arg Leu Val Pro Cys Arg Cys
   1 5 10 15
   Leu Arg Gly Glu Glu Glu Thr Val Thr Thr Leu Asp Tyr Ser His Cys
   20 25 30
   Ser Leu Glu Gln Val Pro Lys Glu Ile Phe Thr Phe Glu Lys Thr Leu
   35 40 45
   Glu Glu Leu Tyr Leu Asp Ala Asn Gln Ile Glu Glu Leu Pro Lys Gln
   50 55 60
   Leu Phe Asn Cys Gln Ser Leu His Lys Leu Ser Leu Pro Asp Asn Asp
   65 70 75 80
   Leu Thr Thr Leu Pro Ala Ser Ile Ala Asn Leu Ile Asn Leu Arg Glu
   85 90 95
   Leu Asp Val Ser Lys Asn Gly Ile Gln Glu Phe Pro Glu Asn Ile Lys
               100                 105                 110
   Asn Cys Lys Val Leu Thr Ile Val Glu Ala Ser Val Asn Pro Ile Ser
           115                 120                 125
   Lys Leu Pro Asp Gly Phe Ser Gln Leu Leu Asn Leu Thr Gln Leu Tyr
       130                 135                 140
   Leu Asn Asp Ala Phe Leu Glu Phe Leu Pro Ala Asn Phe Gly Arg Leu
   145                 150                 155                 160
   Thr Lys Leu Gln Ile Leu Glu Leu Arg Glu Asn Gln Leu Lys Met Leu
                   165                 170                 175
   Pro Lys Thr Met Asn Arg Leu Thr Gln Leu Glu Arg Leu Asp Leu Gly
               180                 185                 190
   Ser Asn Glu Phe Thr Glu Val Pro Glu Val Leu Glu Gln Leu Ser Gly
           195                 200                 205
   Leu Lys Glu Phe Trp Met Asp Ala Asn Arg Leu Thr Phe Ile Pro Gly
       210                 215                 220
   Phe Ile Gly Ser Leu Lys Gln Leu Thr Tyr Leu Asp Val Ser Lys Asn
   225                 230                 235                 240
   Asn Ile Glu Met Val Glu Glu Gly Ile Ser Thr Cys Glu Asn Leu Gln
                   245                 250                 255
   Asp Leu Leu Leu Ser Ser Asn Ser Leu Gln Gln Leu Pro Glu Thr Ile
               260                 265                 270
   Gly Ser Leu Lys Asn Ile Thr Thr Leu Lys Ile Asp Glu Asn Gln Leu
           275                 280                 285
   Met Tyr Leu Pro Asp Ser Ile Gly Gly Leu Ile Ser Val Glu Glu Leu
       290                 295                 300
   Asp Cys Ser Phe Asn Glu Val Glu Ala Leu Pro Ser Ser Ile Gly Gln
   305                 310                 315                 320
   Leu Thr Asn Leu Arg Thr Phe Ala Ala Asp His Asn Tyr Leu Gln Gln
                   325                 330                 335
   Leu Pro Pro Glu Ile Gly Ser Trp Lys Asn Ile Thr Val Leu Phe Leu
               340                 345                 350
   His Ser Asn Lys Leu Glu Thr Leu Pro Glu Glu Met Gly Asp Met Gln
           355                 360                 365
   Lys Leu Lys Val Ile Asn Leu Ser Asp Asn Arg Leu Lys Asn Leu Pro
       370                 375                 380
   Phe Ser Phe Thr Lys Leu Gln Gln Leu Thr Ala Met Trp Leu Ser Asp
   385                 390                 395                 400
   Asn Gln Ser Lys Pro Leu Ile Pro Leu Gln Lys Glu Thr Asp Ser Glu
                   405                 410                 415
   Thr Gln Lys Met Val Leu Thr Asn Tyr Met Phe Pro Gln Gln Pro Arg
               420                 425                 430
   Thr Glu Asp Val Met Phe Ile Ser Asp Asn Glu Ser Phe Asn Pro Ser
   435 440 445
   Leu Trp Glu Glu Gln Arg Lys Gln Arg Ala Gln Val Ala Phe Glu Cys
   450 455 460
   Asp Glu Asp Lys Asp Glu Arg Glu Ala Pro Pro Arg Glu Gly Asn Leu
   465 470 475 480
   Lys Arg Tyr Pro Thr Pro Tyr Pro Asp Glu Leu Lys Asn Met Val Lys
   485 490 495
   Thr Val Gln Thr Ile Val His Arg Leu Lys Asp Glu Glu Thr Asn Glu
   500 505 510
   Asp Ser Gly Arg Asp Leu Lys Pro His Glu Asp Gln Gln Asp Ile Asn
   515 520 525
   Lys Asp Val Gly Val Lys Thr Ser Glu Ser Thr Thr Thr Val Lys Ser
   530 535 540
   Lys Val Asp Glu Arg Glu Lys Tyr Met Ile Gly Asn Ser Val Gln Lys
   545 550 555 560
   Ile Ser Glu Pro Glu Ala Glu Ile Ser Pro Gly Ser Leu Pro Val Thr
   565 570 575
   Ala Asn Met Lys Ala Ser Glu Asn Leu Lys His Ile Val Asn His Asp
   580 585 590
   Asp Val Phe Glu Glu Ser Glu Glu Leu Ser Ser Asp Glu Glu Met Lys
   595 600 605
   Met Ala Glu Met Arg Pro Pro Leu Ile Glu Thr Ser Ile Asn Gln Pro
   610 615 620
   Lys Val Val Ala Leu Ser Asn Asn Lys Lys Asp Asp Thr Lys Glu Thr
   625 630 635 640
   Asp Ser Leu Ser Asp Glu Val Thr His Asn Ser Asn Gln Asn Asn Ser
   645 650 655
   Asn Cys Ser Ser Pro Ser Arg Met Ser Asp Ser Val Ser Leu Asn Thr
   660 665 670
   Asp Ser Ser Gln Asp Thr Ser Leu Cys Ser Pro Val Lys Gln Thr His
   675 680 685
   Ile Asp Ile Asn Ser Lys Ile Arg Gln Glu Asp Glu Asn Phe Asn Ser
   690 695 700
   Leu Leu Gln Asn Gly Asp Ile Leu Asn Ser Ser Thr Glu Glu Lys Phe
   705 710 715 720
   Lys Ala His Asp Lys Lys Asp Phe Asn Leu Pro Glu Tyr Asp Leu Asn
   725 730 735
   Val Glu Glu Arg Leu Val Leu Ile Glu Lys Ser Val Asp Ser Thr Ala
   740 745 750
   Thr Ala Asp Asp Thr His Lys Leu Asp His Ile Asn Met Asn Leu Asn
   755 760 765
   Lys Leu Ile Thr Asn Asp Thr Phe Gln Pro Glu Ile Met Glu Arg Ser
   770 775 780
   Lys Thr Gln Asp Ile Val Leu Gly Thr Ser Phe Leu Ser Ile Asn Ser
   785 790 795 800
   Lys Glu Glu Thr Glu His Leu Glu Asn Gly Asn Lys Tyr Pro Asn Leu
   805 810 815
   Glu Ser Val Asn Lys Val Asn Gly His Ser Glu Glu Thr Ser Gln Ser
   820 825 830
   Pro Asn Arg Thr Glu Pro His Asp Ser Asp Cys Ser Val Asp Leu Gly
   835 840 845
   Ile Ser Lys Ser Thr Glu Asp Leu Ser Pro Gln Lys Ser Gly Pro Val
   850 855 860
   Gly Ser Val Val Lys Ser His Ser Ile Thr Asn Met Glu Ile Gly Gly
   865 870 875 880
   Leu Lys Ile Tyr Asp Ile Leu Ser Asp Asn Gly Pro Gln Gln Pro Ser
   885 890 895
   Thr Thr Val Lys Ile Thr Ser Ala Val Asp Gly Lys Asn Ile Val Arg
   900 905 910
   Ser Lys Ser Ala Thr Leu Leu Tyr Asp Gln Pro Leu Gln Val Phe Thr
   915 920 925
   Gly Ser Ser Ser Ser Ser Asp Leu Ile Ser Gly Thr Lys Ala Ile Phe
   930 935 940
   Lys Phe Asp Ser Asn His Asn Pro Glu Glu Pro Asn Ile Ile Arg Gly
   945 950 955 960
   Pro Thr Ser Gly Pro Gln Ser Ala Pro Gln Ile Tyr Gly Pro Pro Gln
   965 970 975
   Tyr Asn Ile Gln Tyr Ser Ser Ser Ala Ala Val Lys Asp Thr Leu Trp
   980 985 990
   His Ser Lys Gln Asn Pro Gln Ile Asp His Ala Ser Phe Pro Pro Gln
   995 1000 1005
   Leu Leu Pro Arg Ser Glu Ser Thr Glu Asn Gln Ser Tyr Ala Lys His
   1010 1015 1020
   Ser Ala Asn Met Asn Phe Ser Asn His Asn Asn Val Arg Ala Asn Thr
   1025 1030 1035 1040
   Ala Tyr His Leu His Gln Arg Leu Gly Pro Ala Arg His Gly Glu Met
   1045 1050 1055
   Trp Ala Ile Ser Pro Asn Asp Arg Leu Ile Pro Ala Val Thr Arg Ser
   1060 1065 1070
   Thr Ile Gln Arg Gln Ser Ser Val Ser Ser Thr Ala Ser Val Asn Leu
   1075 1080 1085
   Gly Asp Pro Gly Ser Thr Arg Arg Ala Gln Ile Pro Glu Gly Asp Tyr
   1090 1095 1100
   Leu Ser Tyr Arg Glu Phe His Ser Ala Gly Arg Thr Pro Pro Met Met
   1105 1110 1115 1120
   Pro Gly Ser Gln Arg Pro Leu Ser Ala Arg Thr Tyr Ser Ile Asp Gly
   1125 1130 1135
   Pro Asn Ala Ser Arg Pro Gln Ser Ala Arg Pro Ser Ile Asn Glu Ile
   1140 1145 1150
   Pro Glu Arg Thr Met Ser Val Ser Asp Phe Asn Tyr Ser Arg Thr Ser
   1155 1160 1165
   Pro Ser Lys Arg Pro Asn Ala Arg Val Gly Ser Glu His Ser Leu Leu
   1170 1175 1180
   Asp Pro Pro Gly Lys Ser Lys Val Pro Arg Asp Trp Arg Glu Gln Val
   1185 1190 1195 1200
   Leu Arg His Ile Glu Ala Lys Lys Leu Glu Lys Ile Arg Val Arg Val
   1205 1210 1215
   Glu Lys Asp Pro Glu Leu Gly Phe Ser Ile Ser Gly Gly Val Gly Gly
   1220 1225 1230
   Arg Gly Asn Pro Phe Arg Pro Asp Asp Asp Gly Ile Phe Val Thr Arg
   1235 1240 1245
   Val Gln Pro Glu Gly Pro Ala Ser Lys Leu Leu Gln Pro Gly Asp Lys
   1250 1255 1260
   Ile Ile Gln Ala Thr Ala Gln Met Glu Glu Arg Leu Lys Glu Ile Ile
   1265 1270 1275 1280
   Thr Ser Tyr Ser Pro Asp Asn Val Leu Pro Leu Ala Asp Gly Val Leu
   1285 1290 1295
   Ser Phe Thr His His Gln Ile Ile Glu Leu Ala Arg Asp Cys Leu Asp
   1300 1305 1310
   Lys Ser His Gln Gly Leu Ile Thr Ser Arg Tyr Phe Leu Glu Leu Gln
   1315 1320 1325
   His Lys Leu Asp Lys Leu Leu Gln Glu Ala His Asp Arg Ser Glu Ser
   1330 1335 1340
   Gly Glu Leu Ala Phe Ile Lys Gln Leu Val Arg Lys Ile Leu Ile Val
   1345 1350 1355 1360
   Ile Ala Arg Pro Ala Arg Leu Leu Glu Cys Leu Glu Phe Asp Pro Glu
   1365 1370 1375
   Glu Phe Tyr Tyr Leu Leu Glu Ala Ala Glu Gly His Ala Lys Glu Gly
   1380 1385 1390
   Gln Gly Ile Lys Thr Asp Ile Pro Arg Tyr Ile Ile Ser Gln Leu Gly
   1395 1400 1405
   Leu Asn Lys Asp Pro Leu Glu Glu Met Ala His Leu Gly Asn Tyr Asp
   1410 1415 1420
   Ser Gly Thr Ala Glu Thr Pro Glu Thr Asp Glu Ser Val Ser Ser Ser
   1425 1430 1435 1440
   Asn Ala Ser Leu Lys Leu Arg Arg Lys Pro Arg Glu Ser Asp Phe Glu
   1445 1450 1455
   Thr Ile Lys Leu Ile Ser Asn Gly Ala Tyr Gly Ala Val Tyr Phe Val
   1460 1465 1470
   Arg His Lys Glu Ser Arg Gln Arg Phe Ala Met Lys Lys Ile Asn Lys
   1475 1480 1485
   Gln Asn Leu Ile Leu Arg Asn Gln Ile Gln Gln Ala Phe Val Glu Arg
   1490 1495 1500
   Asp Ile Leu Thr Phe Ala Glu Asn Pro Phe Val Val Ser Met Tyr Cys
   1505 1510 1515 1520
   Ser Phe Glu Thr Arg Arg His Leu Cys Met Val Met Glu Tyr Val Glu
   1525 1530 1535
   Gly Gly Asp Cys Ala Thr Leu Met Lys Asn Met Gly Pro Leu Pro Val
   1540 1545 1550
   Asp Met Ala Arg Met Tyr Phe Ala Glu Thr Val Leu Ala Leu Glu Tyr
   1555 1560 1565
   Leu His Asn Tyr Gly Ile Val His Arg Asp Leu Lys Pro Asp Asn Leu
   1570 1575 1580
   Leu Val Thr Ser Met Gly His Ile Lys Leu Thr Asp Phe Gly Leu Ser
   1585 1590 1595 1600
   Lys Val Gly Leu Met Ser Met Thr Thr Asn Leu Tyr Glu Gly His Ile
   1605 1610 1615
   Glu Lys Asp Ala Arg Glu Phe Leu Asp Lys Gln Val Cys Gly Thr Pro
   1620 1625 1630
   Glu Tyr Ile Ala Pro Glu Val Ile Leu Arg Gln Gly Tyr Gly Lys Pro
   1635 1640 1645
   Val Asp Trp Trp Ala Met Gly Ile Ile Leu Tyr Glu Phe Leu Val Gly
   1650 1655 1660
   Cys Val Pro Phe Phe Gly Asp Thr Pro Glu Glu Leu Phe Gly Gln Val
   1665 1670 1675 1680
   Ile Ser Asp Glu Ile Asn Trp Pro Glu Lys Asp Glu Ala Pro Pro Pro
   1685 1690 1695
   Asp Ala Gln Asp Leu Ile Thr Leu Leu Leu Arg Gln Asn Pro Leu Glu
   1700 1705 1710
   Arg Leu Gly Thr Gly Gly Ala Tyr Glu Val Lys Gln His Arg Phe Phe
   1715 1720 1725
   Arg Ser Leu Asp Trp Asn Ser Leu Leu Arg Gln Lys Ala Glu Phe Ile
   1730 1735 1740
   Pro Gln Leu Glu Ser Glu Asp Asp Thr Ser Tyr Phe Asp Thr Arg Ser
   1745 1750 1755 1760
   Glu Lys Tyr His His Met Glu Thr Glu Glu Glu Asp Asp Thr Asn Asp
   1765 1770 1775
   Glu Asp Phe Asn Val Glu Ile Arg Gln Phe Ser Ser Cys Ser His Arg
   1780 1785 1790
   Phe Ser Lys Val Phe Ser Ser Ile Asp Arg Ile Thr Gln Asn Ser Ala
   1795 1800 1805
   Glu Glu Lys Glu Asp Ser Val Asp Lys Thr Lys Ser Thr Thr Leu Pro
   1810 1815 1820
   Ser Thr Glu Thr Leu Ser Trp Ser Ser Glu Tyr Ser Glu Met Gln Gln
   1825 1830 1835 1840
   Leu Ser Thr Ser Asn Ser Ser Asp Thr Glu Ser Asn Arg His Lys Leu
   1845 1850 1855
   Ser Ser Gly Leu Leu Pro Lys Leu Ala Ile Ser Thr Glu Gly Glu Gln
   1860 1865 1870
   Asp Glu Ala Ala Ser Cys Pro Gly Asp Pro His Glu Glu Pro Gly Lys
   1875 1880 1885
   Pro Ala Leu Pro Pro Glu Glu Cys Ala Gln Glu Glu Pro Glu Val Thr
   1890 1895 1900
   Thr Pro Ala Ser Thr Ile Ser Ser Ser Thr Leu Ser Val Gly Ser Phe
   1905 1910 1915 1920
   Ser Glu His Leu Asp Gln Ile Asn Gly Arg Ser Glu Cys Val Asp Ser
   1925 1930 1935
   Thr Asp Asn Ser Ser Lys Pro Ser Ser Glu Pro Ala Ser His Met Ala
   1940 1945 1950
   Arg Gln Arg Leu Glu Ser Thr Glu Lys Lys Lys Ile Ser Gly Lys Val
   1955 1960 1965
   Thr Lys Ser Leu Ser Ala Ser Ala Leu Ser Leu Met Ile Pro Gly Asp
   1970 1975 1980
   Met Phe Ala Val Ser Pro Leu Gly Ser Pro Met Ser Pro His Ser Leu
   1985 1990 1995 2000
   Ser Ser Asp Pro Ser Ser Ser Arg Asp Ser Ser Pro Ser Arg Asp Ser
   2005 2010 2015
   Ser Ala Ala Ser Ala Ser Pro His Gln Pro Ile Val Ile His Ser Ser
   2020 2025 2030
   Gly Lys Asn Tyr Gly Phe Thr Ile Arg Ala Ile Arg Val Tyr Val Gly
   2035 2040 2045
   Asp Ser Asp Ile Tyr Thr Val His His Ile Val Trp Asn Val Glu Glu
   2050 2055 2060
   Gly Ser Pro Ala Cys Gln Ala Gly Leu Lys Ala Gly Asp Leu Ile Thr
   2065 2070 2075 2080
   His Ile Asn Gly Glu Pro Val His Gly Leu Val His Thr Glu Val Ile
   2085 2090 2095
   Glu Leu Leu Leu Lys Ser Gly Asn Lys Val Ser Ile Thr Thr Thr Pro
   2100 2105 2110
   Phe Glu Asn Thr Ser Ile Lys Thr Gly Pro Ala Arg Arg Asn Ser Tyr
           2115                2120                2125
   Lys Ser Arg Met Val Arg Arg Ser Lys Lys Ser Lys Lys Lys Glu Ser
       2130                2135                2140
   Leu Glu Arg Arg Arg Ser Leu Phe Lys Lys Leu Ala Lys Gln Pro Ser
   2145                2150                2155                2160
   Pro Leu Leu His Thr Ser Arg Ser Phe Ser Cys Leu Asn Arg Ser Leu
                   2165                2170                2175
   Ser Ser Gly Glu Ser Leu Pro Gly Ser Pro Thr His Ser Leu Ser Pro
               2180                2185                2190
   Arg Ser Pro Thr Pro Ser Tyr Arg Ser Thr Pro Asp Phe Pro Ser Gly
           2195                2200                2205
   Thr Asn Ser Ser Gln Ser Ser Ser Pro Ser Ser Ser Ala Pro Asn Ser
       2210                2215                2220
   Pro Ala Gly Ser Gly His Ile Arg Pro Ser Thr Leu His Gly Leu Ala
   2225                2230                2235                2240
   Pro Lys Leu Gly Gly Gln Arg Tyr Arg Ser Gly Arg Arg Lys Ser Ala
                   2245                2250                2255
   Gly Asn Ile Pro Leu Ser Pro Leu Ala Arg Thr Pro Ser Pro Thr Pro
               2260                2265                2270
   Gln Pro Thr Ser Pro Gln Arg Ser Pro Ser Pro Leu Leu Gly His Ser
           2275                2280                2285
   Leu Gly Asn Ser Lys Ile Ala Gln Ala Phe Pro Ser Lys Met His Ser
       2290                2295                2300
   Pro Pro Thr Ile Val Arg His Ile Val Arg Pro Lys Ser Ala Glu Pro
   2305                2310                2315                2320
   Pro Arg Ser Pro Leu Leu Lys Arg Val Gln Ser Glu Glu Lys Leu Ser
                   2325                2330                2335
   Pro Ser Tyr Gly Ser Asp Lys Lys His Leu Cys Ser Arg Lys His Ser
               2340                2345                2350
   Leu Glu Val Thr Gln Glu Glu Val Gln Arg Glu Gln Ser Gln Arg Glu
           2355                2360                2365
   Ala Pro Leu Gln Ser Leu Asp Glu Asn Val Cys Asp Val Pro Pro Leu
       2370                2375                2380
   Ser Arg Ala Arg Pro Val Glu Gln Gly Cys Leu Lys Arg Pro Val Ser
   2385                2390                2395                2400
   Arg Lys Val Gly Arg Gln Glu Ser Val Asp Asp Leu Asp Arg Asp Lys
                   2405                2410                2415
   Leu Lys Ala Lys Val Val Val Lys Lys Ala Asp Gly Phe Pro Glu Lys
               2420                2425                2430
   Gln Glu Ser His Gln Lys Ser His Gly Pro Gly Ser Asp Leu Glu Asn
           2435                2440                2445
   Phe Ala Leu Phe Lys Leu Glu Glu Arg Glu Lys Lys Val Tyr Pro Lys
   2450 2455 2460
   Ala Val Glu Arg Ser Ser Thr Phe Glu Asn Lys Ala Ser Met Gln Glu
   2465 2470 2475 2480
   Ala Pro Pro Leu Gly Ser Leu Leu Lys Asp Ala Leu His Lys Gln Ala
   2485 2490 2495
   Ser Val Arg Ala Ser Glu Gly Ala Met Ser Asp Gly Arg Val Pro Ala
   2500 2505 2510
   Glu His Arg Gln Gly Gly Gly Asp Phe Arg Arg Ala Pro Ala Pro Gly
   2515 2520 2525
   Thr Leu Gln Asp Gly Leu Cys His Ser Leu Asp Arg Gly Ile Ser Gly
   2530 2535 2540
   Lys Gly Glu Gly Thr Glu Lys Ser Ser Gln Ala Lys Glu Leu Leu Arg
   2545 2550 2555 2560
   Cys Glu Lys Leu Asp Ser Lys Leu Ala Asn Ile Asp Tyr Leu Arg Lys
   2565 2570 2575
   Lys Met Ser Leu Glu Asp Lys Glu Asp Asn Leu Cys Pro Val Leu Lys
   2580 2585 2590
   Pro Lys Met Thr Ala Gly Ser His Glu Cys Leu Pro Gly Asn Pro Val
   2595 2600 2605
   Arg Pro Thr Gly Gly Gln Gln Glu Pro Pro Pro Ala Ser Glu Ser Arg
   2610 2615 2620
   Ala Phe Val Ser Ser Thr His Ala Ala Gln Met Ser Ala Val Ser Phe
   2625 2630 2635 2640
   Val Pro Leu Lys Ala Leu Thr Gly Arg Val Asp Ser Gly Thr Glu Lys
   2645 2650 2655
   Pro Gly Leu Val Ala Pro Glu Ser Pro Val Arg Lys Ser Pro Ser Glu
   2660 2665 2670
   Tyr Lys Leu Glu Gly Arg Ser Val Ser Cys Leu Lys Pro Ile Glu Gly
   2675 2680 2685
   Thr Leu Asp Ile Ala Leu Leu Ser Gly Pro Gln Ala Ser Lys Thr Glu
   2690 2695 2700
   Leu Pro Ser Pro Glu Ser Ala Gln Ser Pro Ser Pro Ser Gly Asp Val
   2705 2710 2715 2720
   Arg Ala Ser Val Pro Pro Val Leu Pro Ser Ser Ser Gly Lys Lys Asn
   2725 2730 2735
   Asp Thr Thr Ser Ala Arg Glu Leu Ser Pro Ser Ser Leu Lys Met Asn
   2740 2745 2750
   Lys Ser Tyr Leu Leu Glu Pro Trp Phe Leu Pro Pro Ser Arg Gly Leu
   2755 2760 2765
   Gln Asn Ser Pro Ala Val Ser Leu Pro Asp Pro Glu Phe Lys Arg Asp
   2770 2775 2780
   Arg Lys Gly Pro His Pro Thr Ala Arg Ser Pro Gly Thr Val Met Glu
   2785 2790 2795 2800
   Ser Asn Pro Gln Gln Arg Glu Gly Ser Ser Pro Lys His Gln Asp His
   2805 2810 2815
   Thr Thr Asp Pro Lys Leu Leu Thr Cys Leu Gly Gln Asn Leu His Ser
   2820 2825 2830
   Pro Asp Leu Ala Arg Pro Arg Cys Pro Leu Pro Pro Glu Ala Ser Pro
   2835 2840 2845
   Ser Arg Glu Lys Pro Gly Leu Arg Glu Ser Ser Glu Arg Gly Pro Pro
   2850 2855 2860
   Thr Ala Arg Ser Glu Arg Ser Ala Ala Arg Ala Asp Thr Cys Arg Glu
   2865 2870 2875 2880
   Pro Ser Met Glu Leu Cys Phe Pro Glu Thr Ala Lys Thr Ser Asp Asn
   2885 2890 2895
   Ser Lys Asn Leu Leu Ser Val Gly Arg Thr His Pro Asp Phe Tyr Thr
   2900 2905 2910
   Gln Thr Gln Ala Met Glu Lys Ala Trp Ala Pro Gly Gly Lys Thr Asn
   2915 2920 2925
   His Lys Asp Gly Pro Gly Glu Ala Arg Pro Pro Pro Arg Asp Asn Ser
   2930 2935 2940
   Ser Leu His Ser Ala Gly Ile Pro Cys Glu Lys Glu Leu Gly Lys Val
   2945 2950 2955 2960
   Arg Arg Gly Val Glu Pro Lys Pro Glu Ala Leu Leu Ala Arg Arg Ser
   2965 2970 2975
   Leu Gln Pro Pro Gly Ile Glu Ser Glu Lys Ser Glu Lys Leu Ser Ser
   2980 2985 2990
   Phe Pro Ser Leu Gln Lys Asp Gly Ala Lys Glu Pro Glu Arg Lys Glu
   2995 3000 3005
   Gln Pro Leu Gln Arg His Pro Ser Ser Ile Pro Pro Pro Pro Leu Thr
   3010 3015 3020
   Ala Lys Asp Leu Ser Ser Pro Ala Ala Arg Gln His Cys Ser Ser Pro
   3025 3030 3035 3040
   Ser His Ala Ser Gly Arg Glu Pro Gly Ala Lys Pro Ser Thr Ala Glu
   3045 3050 3055
   Pro Ser Ser Ser Pro Gln Asp Pro Pro Lys Pro Val Ala Ala His Ser
   3060 3065 3070
   Glu Ser Ser Ser His Lys Pro Arg Pro Gly Pro Asp Pro Gly Pro Pro
   3075 3080 3085
   Lys Thr Lys His Pro Asp Arg Ser Leu Ser Ser Gln Lys Pro Ser Val
   3090 3095 3100
   Gly Ala Thr Lys Gly Lys Glu Pro Ala Thr Gln Ser Leu Gly Gly Ser
   3105 3110 3115 3120
   Ser Arg Glu Gly Lys Gly His Ser Lys Ser Gly Pro Asp Val Phe Pro
   3125 3130 3135
   Ala Thr Pro Gly Ser Gln Asn Lys Ala Ser Asp Gly Ile Gly Gln Gly
   3140 3145 3150
   Glu Gly Gly Pro Ser Val Pro Leu His Thr Asp Arg Ala Pro Leu Asp
   3155 3160 3165
   Ala Lys Pro Gln Pro Thr Ser Gly Gly Arg Pro Leu Glu Val Leu Glu
   3170 3175 3180
   Lys Pro Val His Leu Pro Arg Pro Gly His Pro Gly Pro Ser Glu Pro
   3185 3190 3195 3200
   Ala Asp Gln Lys Leu Ser Ala Val Gly Glu Lys Gln Thr Leu Ser Pro
   3205 3210 3215
   Lys His Pro Lys Pro Ser Thr Val Lys Asp Cys Pro Thr Leu Cys Lys
   3220 3225 3230
   Gln Thr Asp Asn Arg Gln Thr Asp Lys Ser Pro Ser Gln Pro Ala Ala
   3235 3240 3245
   Asn Thr Asp Arg Arg Ala Glu Gly Lys Lys Cys Thr Glu Ala Leu Tyr
   3250 3255 3260
   Ala Pro Ala Glu Gly Asp Lys Leu Glu Ala Gly Leu Ser Phe Val His
   3265 3270 3275 3280
   Ser Glu Asn Arg Leu Lys Gly Ala Glu Arg Pro Ala Ala Gly Val Gly
   3285 3290 3295
   Lys Gly Phe Pro Glu Ala Arg Gly Lys Gly Pro Gly Pro Gln Lys Pro
   3300 3305 3310
   Pro Thr Glu Ala Asp Lys Pro Asn Gly Met Lys Arg Ser Pro Ser Ala
   3315 3320 3325
   Thr Gly Gln Ser Ser Phe Arg Ser Thr Ala Leu Pro Glu Lys Ser Leu
   3330 3335 3340
   Ser Cys Ser Ser Ser Phe Pro Glu Thr Arg Ala Gly Val Arg Glu Ala
   3345 3350 3355 3360
   Ser Ala Ala Ser Ser Asp Thr Ser Ser Ala Lys Ala Ala Gly Gly Met
   3365 3370 3375
   Leu Glu Leu Pro Ala Pro Ser Asn Arg Asp His Arg Lys Ala Gln Pro
   3380 3385 3390
   Ala Gly Glu Gly Arg Thr His Met Thr Lys Ser Asp Ser Leu Pro Ser
   3395 3400 3405
   Phe Arg Val Ser Thr Leu Pro Leu Glu Ser His His Pro Asp Pro Asn
   3410 3415 3420
   Thr Met Gly Gly Ala Ser His Arg Asp Arg Ala Leu Ser Val Thr Ala
   3425 3430 3435 3440
   Thr Val Gly Glu Thr Lys Gly Lys Asp Pro Ala Pro Ala Gln Pro Pro
   3445 3450 3455
   Pro Ala Arg Lys Gln Asn Val Gly Arg Asp Val Thr Lys Pro Ser Pro
   3460 3465 3470
   Ala Pro Asn Thr Asp Arg Pro Ile Ser Leu Ser Asn Glu Lys Asp Phe
   3475 3480 3485
   Val Val Arg Gln Arg Arg Gly Lys Glu Ser Leu Arg Ser Ser Pro His
   3490 3495 3500
   Lys Lys Ala Leu
   3505
<210> 143
   <211> 16
   <212> PRT
   <213> Artificial Sequence
<220>
   <223> Fused region of ERBB2IP-MAST4 fusion protein
<400> 143
   Gln Pro Gly Asp Lys Ile Ile Gln Ala Thr Ala Gln Met Glu Glu Arg
   1 5 10 15
<210> 144
   <211> 530
   <212> DNA
   <213> Artificial Sequence
<220>
   <223> CDS of TPD52L1 gene (NM_001003395)
<400> 144
   atgctctctg aggaggaaaa ggaagagtta aaagcagagt tagttcagct agaagacgaa 60
   attacaacac tacgacaagt tttgtcagcg aaagaaaggc atctagttga gataaaacaa 120
   aaactcggca tgaacctgat gaatgaatta aaacagaact tcagcaaaag ctggcatgac 180
   atgcagacta ccactgccta caagaaaaca catgaaaccc tgagtcacgc agggcaaaag 240
   gcaactgcag ctttcagcaa cgttggaacg gccatcagca agaagttcgg agacatgaga 300
   gttactccat tcgccattcc ataagtatgc ctgctatgag gaattctcct actttcaaat 360
   catttgagga gagggttgag acaactgtca caagcctcaa gacgaaagta ggcggtacga 420
   accctaatgg aggcagtttt gaggaggtcc tcagctccac ggcccatgcc agtgcccaga 480
   gcttggcagg aggctcccgg cggaccaagg aggaggagct gcagtgctaa 530
<210> 145
   <211> 299
   <212> DNA
   <213> Artificial Sequence
<220>
   <223> TPD52L1 gene fragment
<400> 145
   atgctctctg aggaggaaaa ggaagagtta aaagcagagt tagttcagct agaagacgaa        60
   attacaacac tacgacaagt tttgtcagcg aaagaaaggc atctagttga gataaaacaa       120
   aaactcggca tgaacctgat gaatgaatta aaacagaact tcagcaaaag ctggcatgac       180
   atgcagacta ccactgccta caagaaaaca catgaaaccc tgagtcacgc agggcaaaag       240
   gcaactgcag ctttcagcaa cgttggaacg gccatcagca agaagttcgg agacatgag        299
<210> 146
   <211> 25
   <212> DNA
   <213> Artificial Sequence
<220>
   <223> Break-point of TPD52L1 gene fragment
<400> 146
   tcagcaagaa gttcggagac atgag 25
<210> 147
   <211> 175
   <212> PRT
   <213> Artificial Sequence
<220>
   <223> TPD52L1 protein
<400> 147
   Met Leu Ser Glu Glu Glu Lys Glu Glu Leu Lys Ala Glu Leu Val Gln
    1               5                  10                  15
   Leu Glu Asp Glu Ile Thr Thr Leu Arg Gln Val Leu Ser Ala Lys Glu
               20                  25                  30
   Arg His Leu Val Glu Ile Lys Gln Lys Leu Gly Met Asn Leu Met Asn
           35                  40                  45
   Glu Leu Lys Gln Asn Phe Ser Lys Ser Trp His Asp Met Gln Thr Thr
       50                  55                  60
   Thr Ala Tyr Lys Lys Thr His Glu Thr Leu Ser His Ala Gly Gln Lys
   65                  70                  75                  80
   Ala Thr Ala Ala Phe Ser Asn Val Gly Thr Ala Ile Ser Lys Lys Phe
                   85                  90                  95
   Gly Asp Met Ser Tyr Ser Ile Arg His Ser Ile Ser Met Pro Ala Met
               100                 105                 110
   Arg Asn Ser Pro Thr Phe Lys Ser Phe Glu Glu Arg Val Glu Thr Thr
           115                 120                 125
   Val Thr Ser Leu Lys Thr Lys Val Gly Gly Thr Asn Pro Asn Gly Gly
       130                 135                 140
   Ser Phe Glu Glu Val Leu Ser Ser Thr Ala His Ala Ser Ala Gln Ser
   145                 150                 155                 160
   Leu Ala Gly Gly Ser Arg Arg Thr Lys Glu Glu Glu Leu Gln Cys
                   165                 170                 175
<210> 148
   <211> 99
   <212> PRT
   <213> Artificial Sequence
<220>
   <223> TPD52L1 protein fragment
<400> 148
   Met Leu Ser Glu Glu Glu Lys Glu Glu Leu Lys Ala Glu Leu Val Gln
   1               5                   10                  15
   Leu Glu Asp Glu Ile Thr Thr Leu Arg Gln Val Leu Ser Ala Lys Glu
               20                  25                  30
   Arg His Leu Val Glu Ile Lys Gln Lys Leu Gly Met Asn Leu Met Asn
           35                  40                  45
   Glu Leu Lys Gln Asn Phe Ser Lys Ser Trp His Asp Met Gln Thr Thr
       50                  55                  60
   Thr Ala Tyr Lys Lys Thr His Glu Thr Leu Ser His Ala Gly Gln Lys
   65                  70                  75                  80
   Ala Thr Ala Ala Phe Ser Asn Val Gly Thr Ala Ile Ser Lys Lys Phe
                   85                  90                  95
   Gly Asp Met
<210> 149
   <211> 7
   <212> PRT
   <213> Artificial Sequence
<220>
   <223> Break-point of TPD52L1 protein fragment
<400> 149
   Ser Lys Lys Phe Gly Asp Met
   1               5
<210> 150
   <211> 1393
   <212> DNA
   <213> Artificial Sequence
<220>
   <223> CDS of TRMT11 gene
<400> 150
   atggcgctgt cgtgtaccct taacaggtat ctgctcctca tggcgcagga gcatctggag      60
   ttccgcctgc cggaaataaa gtctttgctt ttgctttttg gaggtcagtt tgccagcagt      120
   caagaaactt atggaaagtc accattttgg attcttagca ttccctctga agatattgca     180
   agaaatttga tgaaacggac agtgtgtgcc aagtctatat ttgaactatg gggtcatgga     240
   caatctcctg aggagctgta cagttctctt aaaaactacc ctgtggagaa gatggttcca     300
   tttctacatt cggactctac atataaaata aagattcaca cttttaataa gacattgaca     360
   caagaagaga aaatcaagcg aatagatgca cttgaatttc tgccatttga aggaaaagtg     420
   aatttaaaga aaccgcaaca tgtattttct gttttggagg attatggttt agacccaaac     480
   tgcatccctg agaatccaca taatatttat tttggtagat ggattgcaga tggacagaga     540
   gagcttattg agtcatacag tgtcaaaaag agacacttta ttggaaatac aagtatggat     600
   gctggtttgt cattcattat ggctaaccat ggaaaagtga aagaaaatga tattgtcttt     660
   gatccatttg ttggaacagg tggcctgctg atagcatgtg ctcattttgg tgcatatgtg     720
   tatgggacag acatagacta caacacagtt catggcttgg gaaaggctac taggaaaaac     780
   cagaagtgga gaggaccaga tgaaaacatt agggccaatc ttcgtcaata tggtttagag     840
   aagtattacc ttgatgtcct ggtttcagat gcatctaaac cttcctggag gaagggcaca     900
   tattttgatg caatcattac tgatcctcca tatggtatca gagaatctac aagaagaaca     960
   ggttcacaga aggagatacc aaaggggata gaaaaatggg aaaaatgtcc agaaagccat    1020
   gttcctgttt ccttgagtta tcatctgagt gatatgtttc ttgacctgtt aaacttcgca    1080
   gctgagaccc tcgttttagg tggaagacta gtctattggt taccggtgta tacgccagaa    1140
   atacactgaa gagatggtgc cttggcaccc ttgcctggaa ctcgttagca actgcgagca    1200
   gaagctttcc agtcacacat caaggcgctt gatcacaatg gaaaaggtga agaaatttga    1260
   gaatcgggac cagtattcac atctgctaag tgatcatttt ctgccatacc aaggtcataa    1320
   ttccttccgt gagaaatatt ttagtggggt aacaaaaaga attgccaagg aagaaaaatc    1380
   cacccaggaa tga                                                       1393
<210> 151
   <211> 253
   <212> DNA
   <213> Artificial Sequence
<220>
   <223> TRMT11 gene fragment
<400> 151
   atacactgaa gagatggtgc cttggcaccc ttgcctggaa ctcgttagca actgcgagca 60
<210> 152
   <211> 22
   <212> DNA
   <213> Artificial Sequence
<220>
   <223> Break-point of TRMT11 gene fragment
<400> 152
   atacactgaa gagatggtgc ct 22
<210> 153
   <211> 463
   <212> PRT
   <213> Artificial Sequence
<220>
   <223> TRMT11 protein
<400> 153
   Met Ala Leu Ser Cys Thr Leu Asn Arg Tyr Leu Leu Leu Met Ala Gln
   1               5                   10                  15
   Glu His Leu Glu Phe Arg Leu Pro Glu Ile Lys Ser Leu Leu Leu Leu
               20                  25                  30
   Phe Gly Gly Gln Phe Ala Ser Ser Gln Glu Thr Tyr Gly Lys Ser Pro
           35                  40                  45
   Phe Trp Ile Leu Ser Ile Pro Ser Glu Asp Ile Ala Arg Asn Leu Met
       50                  55                  60
   Lys Arg Thr Val Cys Ala Lys Ser Ile Phe Glu Leu Trp Gly His Gly
   65                  70                  75                  80
   Gln Ser Pro Glu Glu Leu Tyr Ser Ser Leu Lys Asn Tyr Pro Val Glu
                   85                  90                  95
   Lys Met Val Pro Phe Leu His Ser Asp Ser Thr Tyr Lys Ile Lys Ile
               100                 105                 110
   His Thr Phe Asn Lys Thr Leu Thr Gln Glu Glu Lys Ile Lys Arg Ile
           115                 120                 125
   Asp Ala Leu Glu Phe Leu Pro Phe Glu Gly Lys Val Asn Leu Lys Lys
       130                 135                 140
   Pro Gln His Val Phe Ser Val Leu Glu Asp Tyr Gly Leu Asp Pro Asn
   145                 150                 155                 160
   Cys Ile Pro Glu Asn Pro His Asn Ile Tyr Phe Gly Arg Trp Ile Ala
   165 170 175
   Asp Gly Gln Arg Glu Leu Ile Glu Ser Tyr Ser Val Lys Lys Arg His
   180 185 190
   Phe Ile Gly Asn Thr Ser Met Asp Ala Gly Leu Ser Phe Ile Met Ala
   195 200 205
   Asn His Gly Lys Val Lys Glu Asn Asp Ile Val Phe Asp Pro Phe Val
   210 215 220
   Gly Thr Gly Gly Leu Leu Ile Ala Cys Ala His Phe Gly Ala Tyr Val
   225 230 235 240
   Tyr Gly Thr Asp Ile Asp Tyr Asn Thr Val His Gly Leu Gly Lys Ala
   245 250 255
   Thr Arg Lys Asn Gln Lys Trp Arg Gly Pro Asp Glu Asn Ile Arg Ala
   260 265 270
   Asn Leu Arg Gln Tyr Gly Leu Glu Lys Tyr Tyr Leu Asp Val Leu Val
   275 280 285
   Ser Asp Ala Ser Lys Pro Ser Trp Arg Lys Gly Thr Tyr Phe Asp Ala
   290 295 300
   Ile Ile Thr Asp Pro Pro Tyr Gly Ile Arg Glu Ser Thr Arg Arg Thr
   305 310 315 320
   Gly Ser Gln Lys Glu Ile Pro Lys Gly Ile Glu Lys Trp Glu Lys Cys
   325 330 335
   Pro Glu Ser His Val Pro Val Ser Leu Ser Tyr His Leu Ser Asp Met
   340 345 350
   Phe Leu Asp Leu Leu Asn Phe Ala Ala Glu Thr Leu Val Leu Gly Gly
   355 360 365
   Arg Leu Val Tyr Trp Leu Pro Val Tyr Thr Pro Glu Tyr Thr Glu Glu
   370 375 380
   Met Val Pro Trp His Pro Cys Leu Glu Leu Val Ser Asn Cys Glu Gln
   385 390 395 400
   Lys Leu Ser Ser His Thr Ser Arg Arg Leu Ile Thr Met Glu Lys Val
   405 410 415
   Lys Lys Phe Glu Asn Arg Asp Gln Tyr Ser His Leu Leu Ser Asp His
   420 425 430
   Phe Leu Pro Tyr Gln Gly His Asn Ser Phe Arg Glu Lys Tyr Phe Ser
   435 440 445
   Gly Val Thr Lys Arg Ile Ala Lys Glu Glu Lys Ser Thr Gln Glu
   450 455 460
<210> 154
   <211> 83
   <212> PRT
   <213> Artificial Sequence
<220>
   <223> TRMT11 protein fragment
<400> 154
   Tyr Thr Glu Glu Met Val Pro Trp His Pro Cys Leu Glu Leu Val Ser
   1 5 10 15
   Asn Cys Glu Gln Lys Leu Ser Ser His Thr Ser Arg Arg Leu Ile Thr
   20 25 30
   Met Glu Lys Val Lys Lys Phe Glu Asn Arg Asp Gln Tyr Ser His Leu
   35 40 45
   Leu Ser Asp His Phe Leu Pro Tyr Gln Gly His Asn Ser Phe Arg Glu
   50 55 60
   Lys Tyr Phe Ser Gly Val Thr Lys Arg Ile Ala Lys Glu Glu Lys Ser
   65 70 75 80
   Thr Gln Glu
<210> 155
   <211> 7
   <212> PRT
   <213> Artificial Sequence
<220>
   <223> Break-point of TRMT11 protein fragment
<400> 155
   Tyr Thr Glu Glu Met Val Pro
   1 5
<210> 156
   <211> 552
   <212> DNA
   <213> Artificial Sequence
<220>
   <223> TPD52L1-TRMT11 fusion gene
<400> 156
   atgctctctg aggaggaaaa ggaagagtta aaagcagagt tagttcagct agaagacgaa 60
   attacaacac tacgacaagt tttgtcagcg aaagaaaggc atctagttga gataaaacaa 120
   aaactcggca tgaacctgat gaatgaatta aaacagaact tcagcaaaag ctggcatgac 180
   atgcagacta ccactgccta caagaaaaca catgaaaccc tgagtcacgc agggcaaaag 240
   gcaactgcag ctttcagcaa cgttggaacg gccatcagca agaagttcgg agacatgaga 300
   tacactgaag agatggtgcc ttggcaccct tgcctggaac tcgttagcaa ctgcgagcag 360
   aagctttcca gtcacacatc aaggcgcttg atcacaatgg aaaaggtgaa gaaatttgag 420
   aatcgggacc agtattcaca tctgctaagt gatcattttc tgccatacca aggtcataat 480
   tccttccgtg agaaatattt tagtggggta acaaaaagaa ttgccaagga agaaaaatcc 540
   acccaggaat ga 552
<210> 157
   <211> 45
   <212> DNA
   <213> Artificial Sequence
<220>
   <223> Fused region of TPD52L1-TRMT11 fusion gene
<400> 157
   agcaagaagt tcggagacat gagatacact gaagagatgg tgcct 45
<210> 158
   <211> 183
   <212> PRT
   <213> Artificial Sequence
<220>
   <223> TPD52L1-TRMT11 fusion protein
<400> 158
   Met Leu Ser Glu Glu Glu Lys Glu Glu Leu Lys Ala Glu Leu Val Gln
   1 5 10 15
   Leu Glu Asp Glu Ile Thr Thr Leu Arg Gln Val Leu Ser Ala Lys Glu
   20 25 30
   Arg His Leu Val Glu Ile Lys Gln Lys Leu Gly Met Asn Leu Met Asn
   35 40 45
   Glu Leu Lys Gln Asn Phe Ser Lys Ser Trp His Asp Met Gln Thr Thr
   50 55 60
   Thr Ala Tyr Lys Lys Thr His Glu Thr Leu Ser His Ala Gly Gln Lys
   65 70 75 80
   Ala Thr Ala Ala Phe Ser Asn Val Gly Thr Ala Ile Ser Lys Lys Phe
   85 90 95
   Gly Asp Met Arg Tyr Thr Glu Glu Met Val Pro Trp His Pro Cys Leu
   100 105 110
   Glu Leu Val Ser Asn Cys Glu Gln Lys Leu Ser Ser His Thr Ser Arg
   115 120 125
   Arg Leu Ile Thr Met Glu Lys Val Lys Lys Phe Glu Asn Arg Asp Gln
   130 135 140
   Tyr Ser His Leu Leu Ser Asp His Phe Leu Pro Tyr Gln Gly His Asn
   145 150 155 160
   Ser Phe Arg Glu Lys Tyr Phe Ser Gly Val Thr Lys Arg Ile Ala Lys
   165 170 175
   Glu Glu Lys Ser Thr Gln Glu
   180
<210> 159
   <211> 15
   <212> PRT
   <213> Artificial Sequence
<220>
   <223> Fused region of TPD52L1-TRMT11 fusion protein
<400> 159
   Ser Lys Lys Phe Gly Asp Met Arg Tyr Thr Glu Glu Met Val Pro
   1 5 10 15
<210> 160
   <211> 1656
   <212> DNA
   <213> Artificial Sequence
<220>
   <223> CDS of TXNRD1 gene (NM_003330)
<400> 160
   atgtcatgtg aggacggtcg ggccctggaa ggaacgctct cggaattggc cgcggaaacc      60
   gatctgcccg ttgtgtttgt gaaacagaga aagataggcg gccatggtcc aaccttgaag     120
   gcttatcagg agggcagact tcaaaagcta ctaaaaatga acggccctga agatcttccc     180
   aagtcctatg actatgacct tatcatcatt ggaggtggct caggaggtct ggcagctgct     240
   aaggaggcag cccaatatgg caagaaggtg atggtcctgg actttgtcac tcccacccct     300
   cttggaacta gatggggtct cggaggaaca tgtgtgaatg tgggttgcat acctaaaaaa     360
   ctgatgcatc aagcagcttt gttaggacaa gccctgcaag actctcgaaa ttatggatgg     420
   aaagtcgagg agacagttaa gcatgattgg gacagaatga tagaagctgt acagaatcac     480
   attggctctt tgaattgggg ctaccgagta gctctgcggg agaaaaaagt cgtctatgag     540
   aatgcttatg ggcaatttat tggtcctcac aggattaagg caacaaataa taaaggcaaa     600
   gaaaaaattt attcagcaga gagatttctc attgccactg gtgaaagacc acgttacttg     660
   ggcatccctg gtgacaaaga atactgcatc agcagtgatg atcttttctc cttgccttac     720
   tgcccgggta agaccctggt tgttggagca tcctatgtcg ctttggagtg cgctggattt     780
   cttgctggta ttggtttaga cgtcactgtt atggttaggt ccattcttct tagaggattt     840
   gaccaggaca tggccaacaa aattggtgaa cacatggaag aacatggcat caagtttata     900
   agacagttcg taccaattaa agttgaacaa attgaagcag ggacaccagg ccgactcaga     960
   gtagtagctc agtccaccaa tagtgaggaa atcattgaag gagaatataa tacggtgatg    1020
   ctggcaatag gaagagatgc ttgcacaaga aaaattggct tagaaaccgt aggggtgaag    1080
   ataaatgaaa agactggaaa aatacctgtc acagatgaag aacagaccaa tgtgccttac    1140
   atctatgcca ttggcgatat attggaggat aaggtggagc tcaccccagt tgcaatccag    1200
   gcaggaagat tgctggctca gaggctctat gcaggttcca ctgtcaagtg tgactatgaa    1260
   aatgttccaa ccactgtatt tactcctttg gaatatggtg cttgtggcct ttctgaggag    1320
   aaagctgtgg agaagtttgg ggaagaaaat attgaggttt accatagtta cttttggcca    1380
   ttggaatgga cgattccgtc aagagataac aacaaatgtt atgcaaaaat aatctgtaat    1440
   actaaagaca atgaacgtgt tgtgggcttt cacgtactgg gtccaaatgc tggagaagtt    1500
   acacaaggct ttgcagctgc gctcaaatgt ggactgacca aaaagcagct ggacagcaca    1560
   attggaatcc accctgtctg tgcagaggta ttcacaacat tgtctgtgac caagcgctct    1620
   ggggcaagca tcctccaggc tggctgctga ggttaa                              1656
<210> 161
   <211> 1587
   <212> DNA
   <213> Artificial Sequence
<220>
   <223> TXNRD1 gene fragment
<400> 161
   atgtcatgtg aggacggtcg ggccctggaa ggaacgctct cggaattggc cgcggaaacc      60
   gatctgcccg ttgtgtttgt gaaacagaga aagataggcg gccatggtcc aaccttgaag     120
   gcttatcagg agggcagact tcaaaagcta ctaaaaatga acggccctga agatcttccc     180
   aagtcctatg actatgacct tatcatcatt ggaggtggct caggaggtct ggcagctgct     240
   aaggaggcag cccaatatgg caagaaggtg atggtcctgg actttgtcac tcccacccct     300
   cttggaacta gatggggtct cggaggaaca tgtgtgaatg tgggttgcat acctaaaaaa     360
   ctgatgcatc aagcagcttt gttaggacaa gccctgcaag actctcgaaa ttatggatgg     420
   aaagtcgagg agacagttaa gcatgattgg gacagaatga tagaagctgt acagaatcac     480
   attggctctt tgaattgggg ctaccgagta gctctgcggg agaaaaaagt cgtctatgag     540
   aatgcttatg ggcaatttat tggtcctcac aggattaagg caacaaataa taaaggcaaa     600
   gaaaaaattt attcagcaga gagatttctc attgccactg gtgaaagacc acgttacttg     660
   ggcatccctg gtgacaaaga atactgcatc agcagtgatg atcttttctc cttgccttac     720
   tgcccgggta agaccctggt tgttggagca tcctatgtcg ctttggagtg cgctggattt     780
   cttgctggta ttggtttaga cgtcactgtt atggttaggt ccattcttct tagaggattt     840
   gaccaggaca tggccaacaa aattggtgaa cacatggaag aacatggcat caagtttata     900
   agacagttcg taccaattaa agttgaacaa attgaagcag ggacaccagg ccgactcaga     960
   gtagtagctc agtccaccaa tagtgaggaa atcattgaag gagaatataa tacggtgatg    1020
   ctggcaatag gaagagatgc ttgcacaaga aaaattggct tagaaaccgt aggggtgaag    1080
   ataaatgaaa agactggaaa aatacctgtc acagatgaag aacagaccaa tgtgccttac    1140
   atctatgcca ttggcgatat attggaggat aaggtggagc tcaccccagt tgcaatccag    1200
   gcaggaagat tgctggctca gaggctctat gcaggttcca ctgtcaagtg tgactatgaa    1260
   aatgttccaa ccactgtatt tactcctttg gaatatggtg cttgtggcct ttctgaggag    1320
   aaagctgtgg agaagtttgg ggaagaaaat attgaggttt accatagtta cttttggcca    1380
   ttggaatgga cgattccgtc aagagataac aacaaatgtt atgcaaaaat aatctgtaat    1440
   actaaagaca atgaacgtgt tgtgggcttt cacgtactgg gtccaaatgc tggagaagtt    1500
   acacaaggct ttgcagctgc gctcaaatgt ggactgacca aaaagcagct ggacagcaca    1560
   attggaatcc accctgtctg tgcagag                                         1587
<210> 162
   <211> 22
   <212> DNA
   <213> Artificial Sequence
<220>
   <223> Break-point of TXNRD1 gene fragment
<400> 162
   aatccaccct gtctgtgcag ag 22
<210> 163
   <211> 549
   <212> PRT
   <213> Artificial Sequence
<220>
   <223> TXNRD1 protein wherein "U" and "G" are additionally added at C-terminus
<400> 163
   Met Ser Cys Glu Asp Gly Arg Ala Leu Glu Gly Thr Leu Ser Glu Leu
   1               5                   10                  15
   Ala Ala Glu Thr Asp Leu Pro Val Val Phe Val Lys Gln Arg Lys Ile
               20                  25                  30
   Gly Gly His Gly Pro Thr Leu Lys Ala Tyr Gln Glu Gly Arg Leu Gln
           35                  40                  45
   Lys Leu Leu Lys Met Asn Gly Pro Glu Asp Leu Pro Lys Ser Tyr Asp
       50                  55                  60
   Tyr Asp Leu Ile Ile Ile Gly Gly Gly Ser Gly Gly Leu Ala Ala Ala
   65                  70                  75                  80
   Lys Glu Ala Ala Gln Tyr Gly Lys Lys Val Met Val Leu Asp Phe Val
   85 90 95
   Thr Pro Thr Pro Leu Gly Thr Arg Trp Gly Leu Gly Gly Thr Cys Val
   100 105 110
   Asn Val Gly Cys Ile Pro Lys Lys Leu Met His Gln Ala Ala Leu Leu
   115 120 125
   Gly Gln Ala Leu Gln Asp Ser Arg Asn Tyr Gly Trp Lys Val Glu Glu
   130 135 140
   Thr Val Lys His Asp Trp Asp Arg Met Ile Glu Ala Val Gln Asn His
   145 150 155 160
   Ile Gly Ser Leu Asn Trp Gly Tyr Arg Val Ala Leu Arg Glu Lys Lys
   165 170 175
   Val Val Tyr Glu Asn Ala Tyr Gly Gln Phe Ile Gly Pro His Arg Ile
   180 185 190
   Lys Ala Thr Asn Asn Lys Gly Lys Glu Lys Ile Tyr Ser Ala Glu Arg
   195 200 205
   Phe Leu Ile Ala Thr Gly Glu Arg Pro Arg Tyr Leu Gly Ile Pro Gly
   210 215 220
   Asp Lys Glu Tyr Cys Ile Ser Ser Asp Asp Leu Phe Ser Leu Pro Tyr
   225 230 235 240
   Cys Pro Gly Lys Thr Leu Val Val Gly Ala Ser Tyr Val Ala Leu Glu
   245 250 255
   Cys Ala Gly Phe Leu Ala Gly Ile Gly Leu Asp Val Thr Val Met Val
   260 265 270
   Arg Ser Ile Leu Leu Arg Gly Phe Asp Gln Asp Met Ala Asn Lys Ile
   275 280 285
   Gly Glu His Met Glu Glu His Gly Ile Lys Phe Ile Arg Gln Phe Val
   290 295 300
   Pro Ile Lys Val Glu Gln Ile Glu Ala Gly Thr Pro Gly Arg Leu Arg
   305 310 315 320
   Val Val Ala Gln Ser Thr Asn Ser Glu Glu Ile Ile Glu Gly Glu Tyr
   325 330 335
   Asn Thr Val Met Leu Ala Ile Gly Arg Asp Ala Cys Thr Arg Lys Ile
   340 345 350
   Gly Leu Glu Thr Val Gly Val Lys Ile Asn Glu Lys Thr Gly Lys Ile
   355 360 365
   Pro Val Thr Asp Glu Glu Gln Thr Asn Val Pro Tyr Ile Tyr Ala Ile
   370 375 380
   Gly Asp Ile Leu Glu Asp Lys Val Glu Leu Thr Pro Val Ala Ile Gln
   385 390 395 400
   Ala Gly Arg Leu Leu Ala Gln Arg Leu Tyr Ala Gly Ser Thr Val Lys
   405 410 415
   Cys Asp Tyr Glu Asn Val Pro Thr Thr Val Phe Thr Pro Leu Glu Tyr
   420 425 430
   Gly Ala Cys Gly Leu Ser Glu Glu Lys Ala Val Glu Lys Phe Gly Glu
   435 440 445
   Glu Asn Ile Glu Val Tyr His Ser Tyr Phe Trp Pro Leu Glu Trp Thr
   450 455 460
   Ile Pro Ser Arg Asp Asn Asn Lys Cys Tyr Ala Lys Ile Ile Cys Asn
   465 470 475 480
   Thr Lys Asp Asn Glu Arg Val Val Gly Phe His Val Leu Gly Pro Asn
   485 490 495
   Ala Gly Glu Val Thr Gln Gly Phe Ala Ala Ala Leu Lys Cys Gly Leu
   500 505 510
   Thr Lys Lys Gln Leu Asp Ser Thr Ile Gly Ile His Pro Val Cys Ala
   515 520 525
   Glu Val Phe Thr Thr Leu Ser Val Thr Lys Arg Ser Gly Ala Ser Ile
   530 535 540
   Leu Gln Ala Gly Cys
   545
<210> 164
   <211> 529
   <212> PRT
   <213> Artificial Sequence
<220>
   <223> TXNRD1 protein fragment
<400> 164
   Met Ser Cys Glu Asp Gly Arg Ala Leu Glu Gly Thr Leu Ser Glu Leu
    1               5                  10                  15
   Ala Ala Glu Thr Asp Leu Pro Val Val Phe Val Lys Gln Arg Lys Ile
               20                  25                  30
   Gly Gly His Gly Pro Thr Leu Lys Ala Tyr Gln Glu Gly Arg Leu Gln
           35                  40                  45
   Lys Leu Leu Lys Met Asn Gly Pro Glu Asp Leu Pro Lys Ser Tyr Asp
       50                  55                  60
   Tyr Asp Leu Ile Ile Ile Gly Gly Gly Ser Gly Gly Leu Ala Ala Ala
   65                  70                  75                  80
   Lys Glu Ala Ala Gln Tyr Gly Lys Lys Val Met Val Leu Asp Phe Val
                   85                  90                  95
   Thr Pro Thr Pro Leu Gly Thr Arg Trp Gly Leu Gly Gly Thr Cys Val
               100                 105                 110
   Asn Val Gly Cys Ile Pro Lys Lys Leu Met His Gln Ala Ala Leu Leu
           115                 120                 125
   Gly Gln Ala Leu Gln Asp Ser Arg Asn Tyr Gly Trp Lys Val Glu Glu
   130 135 140
   Thr Val Lys His Asp Trp Asp Arg Met Ile Glu Ala Val Gln Asn His
   145 150 155 160
   Ile Gly Ser Leu Asn Trp Gly Tyr Arg Val Ala Leu Arg Glu Lys Lys
   165 170 175
   Val Val Tyr Glu Asn Ala Tyr Gly Gln Phe Ile Gly Pro His Arg Ile
   180 185 190
   Lys Ala Thr Asn Asn Lys Gly Lys Glu Lys Ile Tyr Ser Ala Glu Arg
   195 200 205
   Phe Leu Ile Ala Thr Gly Glu Arg Pro Arg Tyr Leu Gly Ile Pro Gly
   210 215 220
   Asp Lys Glu Tyr Cys Ile Ser Ser Asp Asp Leu Phe Ser Leu Pro Tyr
   225 230 235 240
   Cys Pro Gly Lys Thr Leu Val Val Gly Ala Ser Tyr Val Ala Leu Glu
   245 250 255
   Cys Ala Gly Phe Leu Ala Gly Ile Gly Leu Asp Val Thr Val Met Val
   260 265 270
   Arg Ser Ile Leu Leu Arg Gly Phe Asp Gln Asp Met Ala Asn Lys Ile
   275 280 285
   Gly Glu His Met Glu Glu His Gly Ile Lys Phe Ile Arg Gln Phe Val
   290 295 300
   Pro Ile Lys Val Glu Gln Ile Glu Ala Gly Thr Pro Gly Arg Leu Arg
   305 310 315 320
   Val Val Ala Gln Ser Thr Asn Ser Glu Glu Ile Ile Glu Gly Glu Tyr
   325 330 335
   Asn Thr Val Met Leu Ala Ile Gly Arg Asp Ala Cys Thr Arg Lys Ile
   340 345 350
   Gly Leu Glu Thr Val Gly Val Lys Ile Asn Glu Lys Thr Gly Lys Ile
   355 360 365
   Pro Val Thr Asp Glu Glu Gln Thr Asn Val Pro Tyr Ile Tyr Ala Ile
   370 375 380
   Gly Asp Ile Leu Glu Asp Lys Val Glu Leu Thr Pro Val Ala Ile Gln
   385 390 395 400
   Ala Gly Arg Leu Leu Ala Gln Arg Leu Tyr Ala Gly Ser Thr Val Lys
   405 410 415
   Cys Asp Tyr Glu Asn Val Pro Thr Thr Val Phe Thr Pro Leu Glu Tyr
   420 425 430
   Gly Ala Cys Gly Leu Ser Glu Glu Lys Ala Val Glu Lys Phe Gly Glu
   435 440 445
   Glu Asn Ile Glu Val Tyr His Ser Tyr Phe Trp Pro Leu Glu Trp Thr
   450 455 460
   Ile Pro Ser Arg Asp Asn Asn Lys Cys Tyr Ala Lys Ile Ile Cys Asn
   465                 470                 475                 480
   Thr Lys Asp Asn Glu Arg Val Val Gly Phe His Val Leu Gly Pro Asn
                   485                 490                 495
   Ala Gly Glu Val Thr Gln Gly Phe Ala Ala Ala Leu Lys Cys Gly Leu
               500                 505                 510
   Thr Lys Lys Gln Leu Asp Ser Thr Ile Gly Ile His Pro Val Cys Ala
           515                 520                 525
   Glu
<210> 165
   <211> 7
   <212> PRT
   <213> Artificial Sequence
<220>
   <223> Break-point of TXNRD1 protein fragment
<400> 165
   Ile His Pro Val Cys Ala Glu
   1               5
<210> 166
   <211> 2625
   <212> DNA
   <213> Artificial Sequence
<220>
   <223> CDS of GPR133 gene (NM_198827)
<400> 166
   atggaaaagc tgctgcggct gtgctgctgg tactcctggc tgctgctatt ttattacaac      60
   tttcaggtgc gtggcgtcta ctccagatcg caggaccatc caggatttca ggtgttggcg     120
   tctgcttccc attactggcc actggagaat gtggatggga tccatgaact tcaggataca     180
   actggagata ttgtggaagg gaaggtcaac aaaggcattt acctgaaaga ggaaaaggga     240
   gtcacgcttc tctattacgg caggtacaac agctcctgca tcagcaagcc agagcagtgt     300
   ggccctgaag gggtcacgtt ttcttttttc tggaagacac aaggagaaca gtctagacca     360
   atcccttctg cgtatggggg acaggtcatc tccaatgggt tcaaagtctg ctccagcggt     420
   ggcagaggct ctgtggagct gtatacgcgg gacaattcca tgacatggga ggcctccttc     480
   agccccccag gcccctattg gactcatgtc ctatttacat ggaaatccaa ggagggcctg     540
   aaagtctacg tcaacgggac cctgagcacc tctgatccga gtggaaaagt gtctcgtgac     600
   tatggagagt ccaacgtcaa cctcgtgata gggtctgagc aggaccaggc caagtgttat     660
   gagaacggtg ctttcgatga gttcatcatc tgggagcggg ctctgactcc ggatgagatc     720
   gccatgtact tcactgctgc cattggaaag catgctttat tgtcttcaac gctgccaagc 780
   ctcttcatga catccacagc aagccccgtg atgcccacag atgcctacca tcccatcata 840
   accaacctga cagaagagag aaaaaccttc caaagtcccg gagtgatact gagttacctc 900
   caaaatgtat ccctcagctt acccagtaag tccctctcgg agcagacagc cttgaatctc 960
   accaagacct tcttaaaagc cgtgggagag atccttctac tgcctggttg gattgctctg 1020
   tcagaggaca gcgccgtggt actgagtctc atcgacacta ttgacaccgt catgggccat 1080
   gtatcctcca acctgcacgg cagcacgccc caggtcaccg tggagggctc ctctgccatg 1140
   gcagagtttt ccgtggccaa aatcctgccc aagaccgtga attcctccca ttaccgcttc 1200
   ccggcccacg ggcagagctt catccagatc ccccacgagg ccttccacag gcacgcctgg 1260
   agcaccgtcg tgggtctgct gtaccacagc atgcactact acctgaacaa catctggccc 1320
   gcccacacca agatcgcgga ggccatgcat caccaggact gcctgctgtt cgccaccagc 1380
   cacctgattt ccctggaggt gtccccacca cccaccctgt ctcagaacct gtcgggctct 1440
   ccactcatta cggtccacct caagcacaga ttgacacgta agcagcacag tgaggccacc 1500
   aacagcagca accgagtctt cgtgtactgc gccttcctgg acttcagctc cggagaaggg 1560
   gtctggtcga accacggctg tgcgctcacg agaggaaacc tcacctactc cgtctgccgc 1620
   tgcactcacc tcaccaactt tgccatcctc atgcaggtgg tcccgctgga gcttgcacgc 1680
   ggacaccagg tggcgctgtc gtctatcagc tatgtgggct gctccctctc cgtgctctgc 1740
   ctggtggcca cgctggtcac cttcgccgtg ctgtcctccg tgagcaccat ccggaaccag 1800
   cgctaccaca tccacgccaa cctgtccttc gccgtgctgg tggcccaggt cctgctgctc 1860
   attagtttcc gcctcgagcc gggcacgacc ccctgccaag tgatggccgt gctcctacac 1920
   tacttcttcc tgagtgcctt cgcatggatg ctggtggagg ggctgcacct ctacagcatg 1980
   gtgatcaagg tctttgggtc ggaggacagc aagcaccgtt actactatgg gatgggatgg 2040
   ggttttcctc ttctgatctg catcatttca ctgtcatttg ccatggacag ttacggaaca 2100
   agcaacaatt gctggctgtc gttggcgagt ggcgccatct gggcctttgt agcccctgcc 2160
   ctgtttgtca tcgtggtcaa cattggcatc ctcatcgctg tgaccagagt catctcacag 2220
   atcagcgccg acaactacaa gatccatgga gaccccagtg ccttcaagtt gacagccaag 2280
   gcagtggccg tgctgctgcc catcctgggt acctcgtggg tctttggcgt gcttgctgtc 2340
   aacggttgtg ctgtggtttt ccagtacatg tttgccacgc tcaactccct gcagggactg 2400
   ttcatattcc tctttcattg tctcctgaat tcagaggtga gagccgcctt caagcacaaa 2460
   accaaggtct ggtcgctcac gagcagctct gcccgcacct ccaacgcgaa gcccttccac 2520
   tcggacctca tgaatgggac ccggccaggc atggcctcca ccaagctcag cccttgggac 2580
   aagagcagcc actctgccca ccgcgtcgac ctgtcagccg tgtga 2625
<210> 167
   <211> 1152
   <212> DNA
   <213> Artificial Sequence
<220>
   <223> GPR133 gene fragment
<400> 167
   acacgtaagc agcacagtga ggccaccaac agcagcaacc gagtcttcgt gtactgcgcc      60
   ttcctggact tcagctccgg agaaggggtc tggtcgaacc acggctgtgc gctcacgaga     120
   ggaaacctca cctactccgt ctgccgctgc actcacctca ccaactttgc catcctcatg     180
   caggtggtcc cgctggagct tgcacgcgga caccaggtgg cgctgtcgtc tatcagctat     240
   gtgggctgct ccctctccgt gctctgcctg gtggccacgc tggtcacctt cgccgtgctg     300
   tcctccgtga gcaccatccg gaaccagcgc taccacatcc acgccaacct gtccttcgcc     360
   gtgctggtgg cccaggtcct gctgctcatt agtttccgcc tcgagccggg cacgaccccc     420
   tgccaagtga tggccgtgct cctacactac ttcttcctga gtgccttcgc atggatgctg     480
   gtggaggggc tgcacctcta cagcatggtg atcaaggtct ttgggtcgga ggacagcaag     540
   caccgttact actatgggat gggatggggt tttcctcttc tgatctgcat catttcactg     600
   tcatttgcca tggacagtta cggaacaagc aacaattgct ggctgtcgtt ggcgagtggc     660
   gccatctggg cctttgtagc ccctgccctg tttgtcatcg tggtcaacat tggcatcctc     720
   atcgctgtga ccagagtcat ctcacagatc agcgccgaca actacaagat ccatggagac     780
   cccagtgcct tcaagttgac agccaaggca gtggccgtgc tgctgcccat cctgggtacc     840
   tcgtgggtct ttggcgtgct tgctgtcaac ggttgtgctg tggttttcca gtacatgttt     900
   gccacgctca actccctgca gggactgttc atattcctct ttcattgtct cctgaattca     960
   gaggtgagag ccgccttcaa gcacaaaacc aaggtctggt cgctcacgag cagctctgcc    1020
   cgcacctcca acgcgaagcc cttccactcg gacctcatga atgggacccg gccaggcatg    1080
   gcctccacca agctcagccc ttgggacaag agcagccact ctgcccaccg cgtcgacctg    1140
   tcagccgtgt ga                                                        1152
<210> 168
   <211> 15
   <212> DNA
   <213> Artificial Sequence
<220>
   <223> Break-point of GPR133 gene fragment
<400> 168
   acacgtaagc agcac 15
<210> 169
   <211> 874
   <212> PRT
   <213> Artificial Sequence
<220>
   <223> GPR133 protein
<400> 169
   Met Glu Lys Leu Leu Arg Leu Cys Cys Trp Tyr Ser Trp Leu Leu Leu
   1               5                   10                  15
   Phe Tyr Tyr Asn Phe Gln Val Arg Gly Val Tyr Ser Arg Ser Gln Asp
               20                  25                  30
   His Pro Gly Phe Gln Val Leu Ala Ser Ala Ser His Tyr Trp Pro Leu
           35                  40                  45
   Glu Asn Val Asp Gly Ile His Glu Leu Gln Asp Thr Thr Gly Asp Ile
       50                  55                  60
   Val Glu Gly Lys Val Asn Lys Gly Ile Tyr Leu Lys Glu Glu Lys Gly
   65                  70                  75                  80
   Val Thr Leu Leu Tyr Tyr Gly Arg Tyr Asn Ser Ser Cys Ile Ser Lys
                   85                  90                  95
   Pro Glu Gln Cys Gly Pro Glu Gly Val Thr Phe Ser Phe Phe Trp Lys
               100                 105                 110
   Thr Gln Gly Glu Gln Ser Arg Pro Ile Pro Ser Ala Tyr Gly Gly Gln
           115                 120                 125
   Val Ile Ser Asn Gly Phe Lys Val Cys Ser Ser Gly Gly Arg Gly Ser
       130                 135                 140
   Val Glu Leu Tyr Thr Arg Asp Asn Ser Met Thr Trp Glu Ala Ser Phe
   145                 150                 155                 160
   Ser Pro Pro Gly Pro Tyr Trp Thr His Val Leu Phe Thr Trp Lys Ser
                   165                 170                 175
   Lys Glu Gly Leu Lys Val Tyr Val Asn Gly Thr Leu Ser Thr Ser Asp
               180                 185                 190
   Pro Ser Gly Lys Val Ser Arg Asp Tyr Gly Glu Ser Asn Val Asn Leu
           195                 200                 205
   Val Ile Gly Ser Glu Gln Asp Gln Ala Lys Cys Tyr Glu Asn Gly Ala
       210                 215                 220
   Phe Asp Glu Phe Ile Ile Trp Glu Arg Ala Leu Thr Pro Asp Glu Ile
   225                 230                 235                 240
   Ala Met Tyr Phe Thr Ala Ala Ile Gly Lys His Ala Leu Leu Ser Ser
                   245                 250                 255
   Thr Leu Pro Ser Leu Phe Met Thr Ser Thr Ala Ser Pro Val Met Pro
               260                 265                 270
   Thr Asp Ala Tyr His Pro Ile Ile Thr Asn Leu Thr Glu Glu Arg Lys
   275 280 285
   Thr Phe Gln Ser Pro Gly Val Ile Leu Ser Tyr Leu Gln Asn Val Ser
   290 295 300
   Leu Ser Leu Pro Ser Lys Ser Leu Ser Glu Gln Thr Ala Leu Asn Leu
   305 310 315 320
   Thr Lys Thr Phe Leu Lys Ala Val Gly Glu Ile Leu Leu Leu Pro Gly
   325 330 335
   Trp Ile Ala Leu Ser Glu Asp Ser Ala Val Val Leu Ser Leu Ile Asp
   340 345 350
   Thr Ile Asp Thr Val Met Gly His Val Ser Ser Asn Leu His Gly Ser
   355 360 365
   Thr Pro Gln Val Thr Val Glu Gly Ser Ser Ala Met Ala Glu Phe Ser
   370 375 380
   Val Ala Lys Ile Leu Pro Lys Thr Val Asn Ser Ser His Tyr Arg Phe
   385 390 395 400
   Pro Ala His Gly Gln Ser Phe Ile Gln Ile Pro His Glu Ala Phe His
   405 410 415
   Arg His Ala Trp Ser Thr Val Val Gly Leu Leu Tyr His Ser Met His
   420 425 430
   Tyr Tyr Leu Asn Asn Ile Trp Pro Ala His Thr Lys Ile Ala Glu Ala
   435 440 445
   Met His His Gln Asp Cys Leu Leu Phe Ala Thr Ser His Leu Ile Ser
   450 455 460
   Leu Glu Val Ser Pro Pro Pro Thr Leu Ser Gln Asn Leu Ser Gly Ser
   465 470 475 480
   Pro Leu Ile Thr Val His Leu Lys His Arg Leu Thr Arg Lys Gln His
   485 490 495
   Ser Glu Ala Thr Asn Ser Ser Asn Arg Val Phe Val Tyr Cys Ala Phe
   500 505 510
   Leu Asp Phe Ser Ser Gly Glu Gly Val Trp Ser Asn His Gly Cys Ala
   515 520 525
   Leu Thr Arg Gly Asn Leu Thr Tyr Ser Val Cys Arg Cys Thr His Leu
   530 535 540
   Thr Asn Phe Ala Ile Leu Met Gln Val Val Pro Leu Glu Leu Ala Arg
   545 550 555 560
   Gly His Gln Val Ala Leu Ser Ser Ile Ser Tyr Val Gly Cys Ser Leu
   565 570 575
   Ser Val Leu Cys Leu Val Ala Thr Leu Val Thr Phe Ala Val Leu Ser
   580 585 590
   Ser Val Ser Thr Ile Arg Asn Gln Arg Tyr His Ile His Ala Asn Leu
   595 600 605
   Ser Phe Ala Val Leu Val Ala Gln Val Leu Leu Leu Ile Ser Phe Arg
       610                 615                 620
   Leu Glu Pro Gly Thr Thr Pro Cys Gln Val Met Ala Val Leu Leu His
   625                 630                 635                 640
   Tyr Phe Phe Leu Ser Ala Phe Ala Trp Met Leu Val Glu Gly Leu His
                   645                 650                 655
   Leu Tyr Ser Met Val Ile Lys Val Phe Gly Ser Glu Asp Ser Lys His
               660                 665                 670
   Arg Tyr Tyr Tyr Gly Met Gly Trp Gly Phe Pro Leu Leu Ile Cys Ile
           675                 680                 685
   Ile Ser Leu Ser Phe Ala Met Asp Ser Tyr Gly Thr Ser Asn Asn Cys
       690                 695                 700
   Trp Leu Ser Leu Ala Ser Gly Ala Ile Trp Ala Phe Val Ala Pro Ala
   705                 710                 715                 720
   Leu Phe Val Ile Val Val Asn Ile Gly Ile Leu Ile Ala Val Thr Arg
                   725                 730                 735
   Val Ile Ser Gln Ile Ser Ala Asp Asn Tyr Lys Ile His Gly Asp Pro
               740                 745                 750
   Ser Ala Phe Lys Leu Thr Ala Lys Ala Val Ala Val Leu Leu Pro Ile
           755                 760                 765
   Leu Gly Thr Ser Trp Val Phe Gly Val Leu Ala Val Asn Gly Cys Ala
       770                 775                 780
   Val Val Phe Gln Tyr Met Phe Ala Thr Leu Asn Ser Leu Gln Gly Leu
   785                 790                 795                 800
   Phe Ile Phe Leu Phe His Cys Leu Leu Asn Ser Glu Val Arg Ala Ala
                   805                 810                 815
   Phe Lys His Lys Thr Lys Val Trp Ser Leu Thr Ser Ser Ser Ala Arg
               820                 825                 830
   Thr Ser Asn Ala Lys Pro Phe His Ser Asp Leu Met Asn Gly Thr Arg
           835                 840                 845
   Pro Gly Met Ala Ser Thr Lys Leu Ser Pro Trp Asp Lys Ser Ser His
       850                 855                 860
   Ser Ala His Arg Val Asp Leu Ser Ala Val
   865                 870
<210> 170
   <211> 383
   <212> PRT
   <213> Artificial Sequence
<220>
   <223> GPR133 protein fragment
<400> 170
   Thr Arg Lys Gln His Ser Glu Ala Thr Asn Ser Ser Asn Arg Val Phe
    1               5                  10                  15
   Val Tyr Cys Ala Phe Leu Asp Phe Ser Ser Gly Glu Gly Val Trp Ser
               20                  25                  30
   Asn His Gly Cys Ala Leu Thr Arg Gly Asn Leu Thr Tyr Ser Val Cys
           35                  40                  45
   Arg Cys Thr His Leu Thr Asn Phe Ala Ile Leu Met Gln Val Val Pro
       50                  55                  60
   Leu Glu Leu Ala Arg Gly His Gln Val Ala Leu Ser Ser Ile Ser Tyr
   65                  70                  75                  80
   Val Gly Cys Ser Leu Ser Val Leu Cys Leu Val Ala Thr Leu Val Thr
                   85                  90                  95
   Phe Ala Val Leu Ser Ser Val Ser Thr Ile Arg Asn Gln Arg Tyr His
               100                 105                 110
   Ile His Ala Asn Leu Ser Phe Ala Val Leu Val Ala Gln Val Leu Leu
           115                 120                 125
   Leu Ile Ser Phe Arg Leu Glu Pro Gly Thr Thr Pro Cys Gln Val Met
       130                 135                 140
   Ala Val Leu Leu His Tyr Phe Phe Leu Ser Ala Phe Ala Trp Met Leu
   145                 150                 155                 160
   Val Glu Gly Leu His Leu Tyr Ser Met Val Ile Lys Val Phe Gly Ser
                   165                 170                 175
   Glu Asp Ser Lys His Arg Tyr Tyr Tyr Gly Met Gly Trp Gly Phe Pro
               180                 185                 190
   Leu Leu Ile Cys Ile Ile Ser Leu Ser Phe Ala Met Asp Ser Tyr Gly
           195                 200                 205
   Thr Ser Asn Asn Cys Trp Leu Ser Leu Ala Ser Gly Ala Ile Trp Ala
       210                 215                 220
   Phe Val Ala Pro Ala Leu Phe Val Ile Val Val Asn Ile Gly Ile Leu
   225                 230                 235                 240
   Ile Ala Val Thr Arg Val Ile Ser Gln Ile Ser Ala Asp Asn Tyr Lys
                   245                 250                 255
   Ile His Gly Asp Pro Ser Ala Phe Lys Leu Thr Ala Lys Ala Val Ala
               260                 265                 270
   Val Leu Leu Pro Ile Leu Gly Thr Ser Trp Val Phe Gly Val Leu Ala
           275                 280                 285
   Val Asn Gly Cys Ala Val Val Phe Gln Tyr Met Phe Ala Thr Leu Asn
       290                 295                 300
   Ser Leu Gln Gly Leu Phe Ile Phe Leu Phe His Cys Leu Leu Asn Ser
   305                 310                 315                 320
   Glu Val Arg Ala Ala Phe Lys His Lys Thr Lys Val Trp Ser Leu Thr
                   325                 330                 335
   Ser Ser Ser Ala Arg Thr Ser Asn Ala Lys Pro Phe His Ser Asp Leu
   340 345 350
   Met Asn Gly Thr Arg Pro Gly Met Ala Ser Thr Lys Leu Ser Pro Trp
   355 360 365
   Asp Lys Ser Ser His Ser Ala His Arg Val Asp Leu Ser Ala Val
   370 375 380
<210> 171
   <211> 6
   <212> PRT
   <213> Artificial Sequence
<220>
   <223> Break-point of GPR133 protein fragment
<400> 171
   Thr Arg Lys Gln His Ser
   1 5
<210> 172
   <211> 2739
   <212> DNA
   <213> Artificial Sequence
<220>
   <223> TXNRD1-GPR133 fusion gene
<400> 172
   atgtcatgtg aggacggtcg ggccctggaa ggaacgctct cggaattggc cgcggaaacc 60
   gatctgcccg ttgtgtttgt gaaacagaga aagataggcg gccatggtcc aaccttgaag 120
   gcttatcagg agggcagact tcaaaagcta ctaaaaatga acggccctga agatcttccc 180
   aagtcctatg actatgacct tatcatcatt ggaggtggct caggaggtct ggcagctgct 240
   aaggaggcag cccaatatgg caagaaggtg atggtcctgg actttgtcac tcccacccct 300
   cttggaacta gatggggtct cggaggaaca tgtgtgaatg tgggttgcat acctaaaaaa 360
   ctgatgcatc aagcagcttt gttaggacaa gccctgcaag actctcgaaa ttatggatgg 420
   aaagtcgagg agacagttaa gcatgattgg gacagaatga tagaagctgt acagaatcac 480
   attggctctt tgaattgggg ctaccgagta gctctgcggg agaaaaaagt cgtctatgag 540
   aatgcttatg ggcaatttat tggtcctcac aggattaagg caacaaataa taaaggcaaa 600
   gaaaaaattt attcagcaga gagatttctc attgccactg gtgaaagacc acgttacttg 660
   ggcatccctg gtgacaaaga atactgcatc agcagtgatg atcttttctc cttgccttac 720
   tgcccgggta agaccctggt tgttggagca tcctatgtcg ctttggagtg cgctggattt 780
   cttgctggta ttggtttaga cgtcactgtt atggttaggt ccattcttct tagaggattt 840
   gaccaggaca tggccaacaa aattggtgaa cacatggaag aacatggcat caagtttata 900
   agacagttcg taccaattaa agttgaacaa attgaagcag ggacaccagg ccgactcaga 960
   gtagtagctc agtccaccaa tagtgaggaa atcattgaag gagaatataa tacggtgatg 1020
   ctggcaatag gaagagatgc ttgcacaaga aaaattggct tagaaaccgt aggggtgaag 1080
   ataaatgaaa agactggaaa aatacctgtc acagatgaag aacagaccaa tgtgccttac 1140
   atctatgcca ttggcgatat attggaggat aaggtggagc tcaccccagt tgcaatccag 1200
   gcaggaagat tgctggctca gaggctctat gcaggttcca ctgtcaagtg tgactatgaa 1260
   aatgttccaa ccactgtatt tactcctttg gaatatggtg cttgtggcct ttctgaggag 1320
   aaagctgtgg agaagtttgg ggaagaaaat attgaggttt accatagtta cttttggcca 1380
   ttggaatgga cgattccgtc aagagataac aacaaatgtt atgcaaaaat aatctgtaat 1440
   actaaagaca atgaacgtgt tgtgggcttt cacgtactgg gtccaaatgc tggagaagtt 1500
   acacaaggct ttgcagctgc gctcaaatgt ggactgacca aaaagcagct ggacagcaca 1560
   attggaatcc accctgtctg tgcagagaca cgtaagcagc acagtgaggc caccaacagc 1620
   agcaaccgag tcttcgtgta ctgcgccttc ctggacttca gctccggaga aggggtctgg 1680
   tcgaaccacg gctgtgcgct cacgagagga aacctcacct actccgtctg ccgctgcact 1740
   cacctcacca actttgccat cctcatgcag gtggtcccgc tggagcttgc acgcggacac 1800
   caggtggcgc tgtcgtctat cagctatgtg ggctgctccc tctccgtgct ctgcctggtg 1860
   gccacgctgg tcaccttcgc cgtgctgtcc tccgtgagca ccatccggaa ccagcgctac 1920
   cacatccacg ccaacctgtc cttcgccgtg ctggtggccc aggtcctgct gctcattagt 1980
   ttccgcctcg agccgggcac gaccccctgc caagtgatgg ccgtgctcct acactacttc 2040
   ttcctgagtg ccttcgcatg gatgctggtg gaggggctgc acctctacag catggtgatc 2100
   aaggtctttg ggtcggagga cagcaagcac cgttactact atgggatggg atggggtttt 2160
   cctcttctga tctgcatcat ttcactgtca tttgccatgg acagttacgg aacaagcaac 2220
   aattgctggc tgtcgttggc gagtggcgcc atctgggcct ttgtagcccc tgccctgttt 2280
   gtcatcgtgg tcaacattgg catcctcatc gctgtgacca gagtcatctc acagatcagc 2340
   gccgacaact acaagatcca tggagacccc agtgccttca agttgacagc caaggcagtg 2400
   gccgtgctgc tgcccatcct gggtacctcg tgggtctttg gcgtgcttgc tgtcaacggt 2460
   tgtgctgtgg ttttccagta catgtttgcc acgctcaact ccctgcaggg actgttcata 2520
   ttcctctttc attgtctcct gaattcagag gtgagagccg ccttcaagca caaaaccaag 2580
   gtctggtcgc tcacgagcag ctctgcccgc acctccaacg cgaagccctt ccactcggac 2640
   ctcatgaatg ggacccggcc aggcatggcc tccaccaagc tcagcccttg ggacaagagc 2700
   agccactctg cccaccgcgt cgacctgtca gccgtgtga 2739
<210> 173
   <211> 36
   <212> DNA
   <213> Artificial Sequence
<220>
   <223> Fused region of TXNRD1-GPR133 fusion gene
<400> 173
   atccaccctg tctgtgcaga gacacgtaag cagcac 36
<210> 174
   <211> 912
   <212> PRT
   <213> Artificial Sequence
<220>
   <223> TXNRD1-GPR133 fusion protein
<400> 174
   Met Ser Cys Glu Asp Gly Arg Ala Leu Glu Gly Thr Leu Ser Glu Leu
   1               5                   10                  15
   Ala Ala Glu Thr Asp Leu Pro Val Val Phe Val Lys Gln Arg Lys Ile
               20                  25                  30
   Gly Gly His Gly Pro Thr Leu Lys Ala Tyr Gln Glu Gly Arg Leu Gln
           35                  40                  45
   Lys Leu Leu Lys Met Asn Gly Pro Glu Asp Leu Pro Lys Ser Tyr Asp
       50                  55                  60
   Tyr Asp Leu Ile Ile Ile Gly Gly Gly Ser Gly Gly Leu Ala Ala Ala
   65                  70                  75                  80
   Lys Glu Ala Ala Gln Tyr Gly Lys Lys Val Met Val Leu Asp Phe Val
                   85                  90                  95
   Thr Pro Thr Pro Leu Gly Thr Arg Trp Gly Leu Gly Gly Thr Cys Val
               100                 105                 110
   Asn Val Gly Cys Ile Pro Lys Lys Leu Met His Gln Ala Ala Leu Leu
           115                 120                 125
   Gly Gln Ala Leu Gln Asp Ser Arg Asn Tyr Gly Trp Lys Val Glu Glu
       130                 135                 140
   Thr Val Lys His Asp Trp Asp Arg Met Ile Glu Ala Val Gln Asn His
   145                 150                 155                 160
   Ile Gly Ser Leu Asn Trp Gly Tyr Arg Val Ala Leu Arg Glu Lys Lys
                   165                 170                 175
   Val Val Tyr Glu Asn Ala Tyr Gly Gln Phe Ile Gly Pro His Arg Ile
               180                 185                 190
   Lys Ala Thr Asn Asn Lys Gly Lys Glu Lys Ile Tyr Ser Ala Glu Arg
           195                 200                 205
   Phe Leu Ile Ala Thr Gly Glu Arg Pro Arg Tyr Leu Gly Ile Pro Gly
       210                 215                 220
   Asp Lys Glu Tyr Cys Ile Ser Ser Asp Asp Leu Phe Ser Leu Pro Tyr
   225                 230                 235                 240
   Cys Pro Gly Lys Thr Leu Val Val Gly Ala Ser Tyr Val Ala Leu Glu
                   245                 250                 255
   Cys Ala Gly Phe Leu Ala Gly Ile Gly Leu Asp Val Thr Val Met Val
               260                 265                 270
   Arg Ser Ile Leu Leu Arg Gly Phe Asp Gln Asp Met Ala Asn Lys Ile
           275                 280                 285
   Gly Glu His Met Glu Glu His Gly Ile Lys Phe Ile Arg Gln Phe Val
       290                 295                 300
   Pro Ile Lys Val Glu Gln Ile Glu Ala Gly Thr Pro Gly Arg Leu Arg
   305                 310                 315                 320
   Val Val Ala Gln Ser Thr Asn Ser Glu Glu Ile Ile Glu Gly Glu Tyr
                   325                 330                 335
   Asn Thr Val Met Leu Ala Ile Gly Arg Asp Ala Cys Thr Arg Lys Ile
               340                 345                 350
   Gly Leu Glu Thr Val Gly Val Lys Ile Asn Glu Lys Thr Gly Lys Ile
           355                 360                 365
   Pro Val Thr Asp Glu Glu Gln Thr Asn Val Pro Tyr Ile Tyr Ala Ile
       370                 375                 380
   Gly Asp Ile Leu Glu Asp Lys Val Glu Leu Thr Pro Val Ala Ile Gln
   385                 390                 395                 400
   Ala Gly Arg Leu Leu Ala Gln Arg Leu Tyr Ala Gly Ser Thr Val Lys
                   405                 410                 415
   Cys Asp Tyr Glu Asn Val Pro Thr Thr Val Phe Thr Pro Leu Glu Tyr
               420                 425                 430
   Gly Ala Cys Gly Leu Ser Glu Glu Lys Ala Val Glu Lys Phe Gly Glu
           435                 440                 445
   Glu Asn Ile Glu Val Tyr His Ser Tyr Phe Trp Pro Leu Glu Trp Thr
       450                 455                 460
   Ile Pro Ser Arg Asp Asn Asn Lys Cys Tyr Ala Lys Ile Ile Cys Asn
   465                 470                 475                 480
   Thr Lys Asp Asn Glu Arg Val Val Gly Phe His Val Leu Gly Pro Asn
                   485                 490                 495
   Ala Gly Glu Val Thr Gln Gly Phe Ala Ala Ala Leu Lys Cys Gly Leu
               500                 505                 510
   Thr Lys Lys Gln Leu Asp Ser Thr Ile Gly Ile His Pro Val Cys Ala
           515                 520                 525
   Glu Thr Arg Lys Gln His Ser Glu Ala Thr Asn Ser Ser Asn Arg Val
       530                 535                 540
   Phe Val Tyr Cys Ala Phe Leu Asp Phe Ser Ser Gly Glu Gly Val Trp
   545                 550                 555                 560
   Ser Asn His Gly Cys Ala Leu Thr Arg Gly Asn Leu Thr Tyr Ser Val
                   565                 570                 575
   Cys Arg Cys Thr His Leu Thr Asn Phe Ala Ile Leu Met Gln Val Val
               580                 585                 590
   Pro Leu Glu Leu Ala Arg Gly His Gln Val Ala Leu Ser Ser Ile Ser
           595                 600                 605
   Tyr Val Gly Cys Ser Leu Ser Val Leu Cys Leu Val Ala Thr Leu Val
       610                 615                 620
   Thr Phe Ala Val Leu Ser Ser Val Ser Thr Ile Arg Asn Gln Arg Tyr
   625                 630                 635                 640
   His Ile His Ala Asn Leu Ser Phe Ala Val Leu Val Ala Gln Val Leu
                   645                 650                 655
   Leu Leu Ile Ser Phe Arg Leu Glu Pro Gly Thr Thr Pro Cys Gln Val
               660                 665                 670
   Met Ala Val Leu Leu His Tyr Phe Phe Leu Ser Ala Phe Ala Trp Met
           675                 680                 685
   Leu Val Glu Gly Leu His Leu Tyr Ser Met Val Ile Lys Val Phe Gly
       690                 695                 700
   Ser Glu Asp Ser Lys His Arg Tyr Tyr Tyr Gly Met Gly Trp Gly Phe
   705                 710                 715                 720
   Pro Leu Leu Ile Cys Ile Ile Ser Leu Ser Phe Ala Met Asp Ser Tyr
                   725                 730                 735
   Gly Thr Ser Asn Asn Cys Trp Leu Ser Leu Ala Ser Gly Ala Ile Trp
               740                 745                 750
   Ala Phe Val Ala Pro Ala Leu Phe Val Ile Val Val Asn Ile Gly Ile
           755                 760                 765
   Leu Ile Ala Val Thr Arg Val Ile Ser Gln Ile Ser Ala Asp Asn Tyr
       770                 775                 780
   Lys Ile His Gly Asp Pro Ser Ala Phe Lys Leu Thr Ala Lys Ala Val
   785                 790                 795                 800
   Ala Val Leu Leu Pro Ile Leu Gly Thr Ser Trp Val Phe Gly Val Leu
                   805                 810                 815
   Ala Val Asn Gly Cys Ala Val Val Phe Gln Tyr Met Phe Ala Thr Leu
               820                 825                 830
   Asn Ser Leu Gln Gly Leu Phe Ile Phe Leu Phe His Cys Leu Leu Asn
           835                 840                 845
   Ser Glu Val Arg Ala Ala Phe Lys His Lys Thr Lys Val Trp Ser Leu
       850                 855                 860
   Thr Ser Ser Ser Ala Arg Thr Ser Asn Ala Lys Pro Phe His Ser Asp
   865 870 875 880
   Leu Met Asn Gly Thr Arg Pro Gly Met Ala Ser Thr Lys Leu Ser Pro
   885 890 895
   Trp Asp Lys Ser Ser His Ser Ala His Arg Val Asp Leu Ser Ala Val
   900 905 910
<210> 175
   <211> 13
   <212> PRT
   <213> Artificial Sequence
<220>
   <223> Fused region of TXNRD1-GPR133 fusion protein
<400> 175
   Ile His Pro Val Cys Ala Glu Thr Arg Lys Gln His Ser
   1 5 10
<210> 176
   <211> 266
   <212> DNA
   <213> Artificial Sequence
<220>
   <223> SCAF11 gene (NM_004719) fragment
<400> 176
   ggaggggagg ggagggagga ggctagacaa ggcgggggaa gggggagtag cggtggctta 60
   agccgcgcgg agcagcgcaa cctgggtcgc tccctgcttc gccgccgcct ccggaccgag 120
   ccagcggagt cagtgtccta gagaccctgt aacaccacaa agcggacgaa ggagtccatg 180
   ttggggaact tggcagcgga gtgactggga cctgggaacc tactgtgggg ccgcggccgg 240
   accgagcgcc tcgacctcgg tctgag 266
<210> 177
   <211> 11
   <212> DNA
   <213> Artificial Sequence
<220>
   <223> Break-point of SCAF11 gene fragment
<400> 177
   ctcggtctga g 11
<210> 178
   <211> 3282
   <212> DNA
   <213> Artificial Sequence
<220>
   <223> PDGFRA gene (NM_006206) fragment
<400> 178
   tttcccagag ctatggggac ttcccatccg gcgttcctgg tcttaggctg tcttctcaca      60
   gggctgagcc taatcctctg ccagctttca ttaccctcta tccttccaaa tgaaaatgaa     120
   aaggttgtgc agctgaattc atccttttct ctgagatgct ttggggagag tgaagtgagc     180
   tggcagtacc ccatgtctga agaagagagc tccgatgtgg aaatcagaaa tgaagaaaac     240
   aacagcggcc tttttgtgac ggtcttggaa gtgagcagtg cctcggcggc ccacacaggg     300
   ttgtacactt gctattacaa ccacactcag acagaagaga atgagcttga aggcaggcac     360
   atttacatct atgtgccaga cccagatgta gcctttgtac ctctaggaat gacggattat     420
   ttagtcatcg tggaggatga tgattctgcc attatacctt gtcgcacaac tgatcccgag     480
   actcctgtaa ccttacacaa cagtgagggg gtggtacctg cctcctacga cagcagacag     540
   ggctttaatg ggaccttcac tgtagggccc tatatctgtg aggccaccgt caaaggaaag     600
   aagttccaga ccatcccatt taatgtttat gctttaaaag caacatcaga gctggatcta     660
   gaaatggaag ctcttaaaac cgtgtataag tcaggggaaa cgattgtggt cacctgtgct     720
   gtttttaaca atgaggtggt tgaccttcaa tggacttacc ctggagaagt gaaaggcaaa     780
   ggcatcacaa tgctggaaga aatcaaagtc ccatccatca aattggtgta cactttgacg     840
   gtccccgagg ccacggtgaa agacagtgga gattacgaat gtgctgcccg ccaggctacc     900
   agggaggtca aagaaatgaa gaaagtcact atttctgtcc atgagaaagg tttcattgaa     960
   atcaaaccca ccttcagcca gttggaagct gtcaacctgc atgaagtcaa acattttgtt    1020
   gtagaggtgc gggcctaccc acctcccagg atatcctggc tgaaaaacaa tctgactctg    1080
   attgaaaatc tcactgagat caccactgat gtggaaaaga ttcaggaaat aaggtatcga    1140
   agcaaattaa agctgatccg tgctaaggaa gaagacagtg gccattatac tattgtagct    1200
   caaaatgaag atgctgtgaa gagctatact tttgaactgt taactcaagt tccttcatcc    1260
   attctggact tggtcgatga tcaccatggc tcaactgggg gacagacggt gaggtgcaca    1320
   gctgaaggca cgccgcttcc tgatattgag tggatgatat gcaaagatat taagaaatgt    1380
   aataatgaaa cttcctggac tattttggcc aacaatgtct caaacatcat cacggagatc    1440
   cactcccgag acaggagtac cgtggagggc cgtgtgactt tcgccaaagt ggaggagacc    1500
   atcgccgtgc gatgcctggc taagaatctc cttggagctg agaaccgaga gctgaagctg    1560
   gtggctccca ccctgcgttc tgaactcacg gtggctgctg cagtcctggt gctgttggtg    1620
   attgtgatca tctcacttat tgtcctggtt gtcatttgga aacagaaacc gaggtatgaa    1680
   attcgctgga gggtcattga atcaatcagc ccagatggac atgaatatat ttatgtggac    1740
   ccgatgcagc tgccttatga ctcaagatgg gagtttccaa gagatggact agtgcttggt 1800
   cgggtcttgg ggtctggagc gtttgggaag gtggttgaag gaacagccta tggattaagc 1860
   cggtcccaac ctgtcatgaa agttgcagtg aagatgctaa aacccacggc cagatccagt 1920
   gaaaaacaag ctctcatgtc tgaactgaag ataatgactc acctggggcc acatttgaac 1980
   attgtaaact tgctgggagc ctgcaccaag tcaggcccca tttacatcat cacagagtat 2040
   tgcttctatg gagatttggt caactatttg cataagaata gggatagctt cctgagccac 2100
   cacccagaga agccaaagaa agagctggat atctttggat tgaaccctgc tgatgaaagc 2160
   acacggagct atgttatttt atcttttgaa aacaatggtg actacatgga catgaagcag 2220
   gctgatacta cacagtatgt ccccatgcta gaaaggaaag aggtttctaa atattccgac 2280
   atccagagat cactctatga tcgtccagcc tcatataaga agaaatctat gttagactca 2340
   gaagtcaaaa acctcctttc agatgataac tcagaaggcc ttactttatt ggatttgttg 2400
   agcttcacct atcaagttgc ccgaggaatg gagtttttgg cttcaaaaaa ttgtgtccac 2460
   cgtgatctgg ctgctcgcaa cgtcctcctg gcacaaggaa aaattgtgaa gatctgtgac 2520
   tttggcctgg ccagagacat catgcatgat tcgaactatg tgtcgaaagg cagtaccttt 2580
   ctgcccgtga agtggatggc tcctgagagc atctttgaca acctctacac cacactgagt 2640
   gatgtctggt cttatggcat tctgctctgg gagatctttt cccttggtgg caccccttac 2700
   cccggcatga tggtggattc tactttctac aataagatca agagtgggta ccggatggcc 2760
   aagcctgacc acgctaccag tgaagtctac gagatcatgg tgaaatgctg gaacagtgag 2820
   ccggagaaga gaccctcctt ttaccacctg agtgagattg tggagaatct gctgcctgga 2880
   caatataaaa agagttatga aaaaattcac ctggacttcc tgaagagtga ccatcctgct 2940
   gtggcacgca tgcgtgtgga ctcagacaat gcatacattg gtgtcaccta caaaaacgag 3000
   gaagacaagc tgaaggactg ggagggtggt ctggatgagc agagactgag cgctgacagt 3060
   ggctacatca ttcctctgcc tgacattgac cctgtccctg aggaggagga cctgggcaag 3120
   aggaacagac acagctcgca gacctctgaa gagagtgcca ttgagacggg ttccagcagt 3180
   tccaccttca tcaagagaga ggacgagacc attgaagaca tcgacatgat ggatgacatc 3240
   ggcatagact cttcagacct ggtggaagac agcttcctgt aa 3282
<210> 179
   <211> 12
   <212> DNA
   <213> Artificial Sequence
<220>
   <223> Break-point of PDGFRA gene fragment
<400> 179
   tttcccagag ct 12
<210> 180
   <211> 3548
   <212> DNA
   <213> Artificial Sequence
<220>
   <223> SCAF11-PDGFRA fusion gene
<400> 180
   ggaggggagg ggagggagga ggctagacaa ggcgggggaa gggggagtag cggtggctta      60
   agccgcgcgg agcagcgcaa cctgggtcgc tccctgcttc gccgccgcct ccggaccgag     120
   ccagcggagt cagtgtccta gagaccctgt aacaccacaa agcggacgaa ggagtccatg     180
   ttggggaact tggcagcgga gtgactggga cctgggaacc tactgtgggg ccgcggccgg     240
   accgagcgcc tcgacctcgg tctgagtttc ccagagctat ggggacttcc catccggcgt     300
   tcctggtctt aggctgtctt ctcacagggc tgagcctaat cctctgccag ctttcattac     360
   cctctatcct tccaaatgaa aatgaaaagg ttgtgcagct gaattcatcc ttttctctga     420
   gatgctttgg ggagagtgaa gtgagctggc agtaccccat gtctgaagaa gagagctccg     480
   atgtggaaat cagaaatgaa gaaaacaaca gcggcctttt tgtgacggtc ttggaagtga     540
   gcagtgcctc ggcggcccac acagggttgt acacttgcta ttacaaccac actcagacag     600
   aagagaatga gcttgaaggc aggcacattt acatctatgt gccagaccca gatgtagcct     660
   ttgtacctct aggaatgacg gattatttag tcatcgtgga ggatgatgat tctgccatta     720
   taccttgtcg cacaactgat cccgagactc ctgtaacctt acacaacagt gagggggtgg      780
   tacctgcctc ctacgacagc agacagggct ttaatgggac cttcactgta gggccctata     840
   tctgtgaggc caccgtcaaa ggaaagaagt tccagaccat cccatttaat gtttatgctt     900
   taaaagcaac atcagagctg gatctagaaa tggaagctct taaaaccgtg tataagtcag     960
   gggaaacgat tgtggtcacc tgtgctgttt ttaacaatga ggtggttgac cttcaatgga    1020
   cttaccctgg agaagtgaaa ggcaaaggca tcacaatgct ggaagaaatc aaagtcccat    1080
   ccatcaaatt ggtgtacact ttgacggtcc ccgaggccac ggtgaaagac agtggagatt    1140
   acgaatgtgc tgcccgccag gctaccaggg aggtcaaaga aatgaagaaa gtcactattt    1200
   ctgtccatga gaaaggtttc attgaaatca aacccacctt cagccagttg gaagctgtca    1260
   acctgcatga agtcaaacat tttgttgtag aggtgcgggc ctacccacct cccaggatat    1320
   cctggctgaa aaacaatctg actctgattg aaaatctcac tgagatcacc actgatgtgg    1380
   aaaagattca ggaaataagg tatcgaagca aattaaagct gatccgtgct aaggaagaag    1440
   acagtggcca ttatactatt gtagctcaaa atgaagatgc tgtgaagagc tatacttttg     1500
   aactgttaac tcaagttcct tcatccattc tggacttggt cgatgatcac catggctcaa 1560
   ctgggggaca gacggtgagg tgcacagctg aaggcacgcc gcttcctgat attgagtgga 1620
   tgatatgcaa agatattaag aaatgtaata atgaaacttc ctggactatt ttggccaaca 1680
   atgtctcaaa catcatcacg gagatccact cccgagacag gagtaccgtg gagggccgtg 1740
   tgactttcgc caaagtggag gagaccatcg ccgtgcgatg cctggctaag aatctccttg 1800
   gagctgagaa ccgagagctg aagctggtgg ctcccaccct gcgttctgaa ctcacggtgg 1860
   ctgctgcagt cctggtgctg ttggtgattg tgatcatctc acttattgtc ctggttgtca 1920
   tttggaaaca gaaaccgagg tatgaaattc gctggagggt cattgaatca atcagcccag 1980
   atggacatga atatatttat gtggacccga tgcagctgcc ttatgactca agatgggagt 2040
   ttccaagaga tggactagtg cttggtcggg tcttggggtc tggagcgttt gggaaggtgg 2100
   ttgaaggaac agcctatgga ttaagccggt cccaacctgt catgaaagtt gcagtgaaga 2160
   tgctaaaacc cacggccaga tccagtgaaa aacaagctct catgtctgaa ctgaagataa 2220
   tgactcacct ggggccacat ttgaacattg taaacttgct gggagcctgc accaagtcag 2280
   gccccattta catcatcaca gagtattgct tctatggaga tttggtcaac tatttgcata 2340
   agaataggga tagcttcctg agccaccacc cagagaagcc aaagaaagag ctggatatct 2400
   ttggattgaa ccctgctgat gaaagcacac ggagctatgt tattttatct tttgaaaaca 2460
   atggtgacta catggacatg aagcaggctg atactacaca gtatgtcccc atgctagaaa 2520
   ggaaagaggt ttctaaatat tccgacatcc agagatcact ctatgatcgt ccagcctcat 2580
   ataagaagaa atctatgtta gactcagaag tcaaaaacct cctttcagat gataactcag 2640
   aaggccttac tttattggat ttgttgagct tcacctatca agttgcccga ggaatggagt 2700
   ttttggcttc aaaaaattgt gtccaccgtg atctggctgc tcgcaacgtc ctcctggcac 2760
   aaggaaaaat tgtgaagatc tgtgactttg gcctggccag agacatcatg catgattcga 2820
   actatgtgtc gaaaggcagt acctttctgc ccgtgaagtg gatggctcct gagagcatct 2880
   ttgacaacct ctacaccaca ctgagtgatg tctggtctta tggcattctg ctctgggaga 2940
   tcttttccct tggtggcacc ccttaccccg gcatgatggt ggattctact ttctacaata 3000
   agatcaagag tgggtaccgg atggccaagc ctgaccacgc taccagtgaa gtctacgaga 3060
   tcatggtgaa atgctggaac agtgagccgg agaagagacc ctccttttac cacctgagtg 3120
   agattgtgga gaatctgctg cctggacaat ataaaaagag ttatgaaaaa attcacctgg 3180
   acttcctgaa gagtgaccat cctgctgtgg cacgcatgcg tgtggactca gacaatgcat 3240
   acattggtgt cacctacaaa aacgaggaag acaagctgaa ggactgggag ggtggtctgg 3300
   atgagcagag actgagcgct gacagtggct acatcattcc tctgcctgac attgaccctg 3360
   tccctgagga ggaggacctg ggcaagagga acagacacag ctcgcagacc tctgaagaga 3420
   gtgccattga gacgggttcc agcagttcca ccttcatcaa gagagaggac gagaccattg 3480
   aagacatcga catgatggat gacatcggca tagactcttc agacctggtg gaagacagct 3540
   tcctgtaa 3548
<210> 181
   <211> 22
   <212> DNA
   <213> Artificial Sequence
<220>
   <223> Fused region of SCAF11-PDGFRA fusion gene
<400> 181
   ctcggtctga gtttcccaga gc 22
<210> 182
   <211> 22
   <212> DNA
   <213> Artificial Sequence
<220>
   <223> Forward Primer of CCDC6-ROS1
<400> 182
   cctgcaggaa aaattagacc ag 22
<210> 183
   <211> 22
   <212> DNA
   <213> Artificial Sequence
<220>
   <223> Reverse Primer of CCDC6-ROS1
<400> 183
   agctcagcca actctttgtc tt 22
<210> 184
   <211> 22
   <212> DNA
   <213> Artificial Sequence
<220>
   <223> Forward Primer of SCAF11-PDGFRA
<400> 184
   cagcggagtc agtgtcctag ag 22
<210> 185
   <211> 22
   <212> DNA
   <213> Artificial Sequence
<220>
   <223> Reverse Primer of SCAF11-PDGFRA
<400> 185
   tgagaagaca gcctaagacc ag 22
<210> 186
   <211> 22
   <212> DNA
   <213> Artificial Sequence
<220>
   <223> Forward Primer of FGFR2-CIT
<400> 186
   acatgatgat gagggactgt tg 22
<210> 187
   <211> 22
   <212> DNA
   <213> Artificial Sequence
<220>
   <223> Reverse Primer of FGFR2-CIT
<400> 187
   acagctgtta cgaagagcat ca 22
<210> 188
   <211> 22
   <212> DNA
   <213> Artificial Sequence
<220>
   <223> Forward Primer of AXL-MBIP
<400> 188
   gcctgacgaa atcctctatg tc 22
<210> 189
   <211> 22
   <212> DNA
   <213> Artificial Sequence
<220>
   <223> Reverse Primer of AXL-MBIP
<400> 189
   caaaattccc tgacgttgtt tt 22
<210> 190
   <211> 22
   <212> DNA
   <213> Artificial Sequence
<220>
   <223> Forward Primer of APLP2-TNFSF11
<400> 190
   tgctgagaac aaagatcgct ta 22
<210> 191
   <211> 22
   <212> DNA
   <213> Artificial Sequence
<220>
   <223> Reverse Primer of APLP2-TNFSF11
<400> 191
   tgtcggtggc attaatagtg ag 22
<210> 192
   <211> 21
   <212> DNA
   <213> Artificial Sequence
<220>
   <223> Forward Primer of MAP4K3-PRKCE
<400> 192
   aggaggactt cgagctgatt c 21
<210> 193
   <211> 21
   <212> DNA
   <213> Artificial Sequence
<220>
   <223> Reverse Primer of MAP4K3-PRKCE
<400> 193
   acgaccctga gagatcgatg a 21
<210> 194
   <211> 20
   <212> DNA
   <213> Artificial Sequence
<220>
   <223> Forward Primer of BCAS3-MAP3K3
<400> 194
   catcccgtcc agtctctgat 20
<210> 195
   <211> 22
   <212> DNA
   <213> Artificial Sequence
<220>
   <223> Reverse Primer of BCAS3-MAP3K3
<400> 195
   ctgcctattt gagtgacctg tg 22
<210> 196
   <211> 23
   <212> DNA
   <213> Artificial Sequence
<220>
   <223> Forward Primer of KRAS-CDH13
<400> 196
   ggaaataaat gtgatttgcc ttc 23
<210> 197
   <211> 22
   <212> DNA
   <213> Artificial Sequence
<220>
   <223> Reverse Primer of KRAS-CDH13
<400> 197
   aaggctgtct ctgattctct gg 22
<210> 198
   <211> 22
   <212> DNA
   <213> Artificial Sequence
<220>
   <223> Forward Primer of ZFYVE9-CGA
<400> 198
   actgcagaga acatggattc ct 22
<210> 199
   <211> 22
   <212> DNA
   <213> Artificial Sequence
<220>
   <223> Reverse Primer of ZFYVE9-CGA
<400> 199
   gaatggagaa catgcagaaa ca 22
<210> 200
   <211> 22
   <212> DNA
   <213> Artificial Sequence
<220>
   <223> Forward Primer of ERBB2IP-MAST4
<400> 200
   aacaagggta caacctgaag ga 22
<210> 201
   <211> 22
   <212> DNA
   <213> Artificial Sequence
<220>
   <223> Reverse Primer of ERBB2IP-MAST4
<400> 201
   tcaaggaagt atcgtgaggt ga 22
<210> 202
   <211> 23
   <212> DNA
   <213> Artificial Sequence
<220>
   <223> Forward Primer of TPD52L1-TRMT11
<400> 202
   gaaaacacat gaaaccctga gtc 23
<210> 203
   <211> 22
   <212> DNA
   <213> Artificial Sequence
<220>
   <223> Reverse Primer of TPD52L1-TRMT11
<400> 203
   atgtgtgact ggaaagcttc tg 22
<210> 204
   <211> 22
   <212> DNA
   <213> Artificial Sequence
<220>
   <223> Forward Primer of TXNRD1-GPR133
<400> 204
   tccaaatgct ggagaagtta ca 22
<210> 205
   <211> 22
   <212> DNA
   <213> Artificial Sequence
<220>
   <223> Reverse Primer of TXNRD1-GPR133
<400> 205
   agtacacgaa gactcggttg ct 22

## Description

### [Technical Field]

The present invention relates to a fusion protein comprising AXL receptor tyrosine kinase (AXL protein) and MAP3K12 binding inhibitory protein 1 (MBIP protein), and a composition for use in diagnosing a solid cancer, the solid cancer being a lung cancer.

### [Background Art]

Lung cancer is the most common cancer in humans, as well as the leading cause of cancer-related death worldwide. Although diagnosis at an early stage is increasing with the introduction of low-dose computerized tomography screening, lung cancer is still a devastating disease which has a very poor prognosis. Lung cancer has been classified based on histopathologic findings: adenocarcinoma is the most common type, which frequently occurs in never- or light-smokers and female patients. In the past decade, adenocarcinoma has been at the center of lung cancer research: our understanding of this disease has advanced in every aspect including pathology, molecular biology, genetics, radiology, and clinical therapeutics.

In particular, advances in the understanding of the major genetic alterations and signaling pathways involved have suggested a reclassification of lung adenocarcinoma based on underlying genetic changes. Cancer cells with these genetic alterations, called "driver mutations", have survival and growth advantages over cells without these changes. Driver mutations occur in genes that encode signaling proteins such as kinases; this can generate a constitutively active survival signal, which initiates and maintains tumorigenesis. Currently, around ten driver mutations are known in lung adenocarcinoma, and several drugs targeting these alterations have reported remarkable outcomes in recent trials. For example, gefitinib, an epidermal growth factor receptor (*EGFR*) tyrosine kinase inhibitor, yielded a response rate of about 70% and progression-free survival of 10 months in patients with an *EGFR-*activating mutation, proving superiority over platinum-based doublet chemotherapy in two recent large phase III clinical trials. Crizotinib, a dual inhibitor of anaplastic lymphoma kinase (*ALK*) and *MET* tyrosine kinase, has shown efficacy in a recent phase I trial of lung cancer patients harboring *ALK* fusion. From these pivotal studies, treatment strategies of lung adenocarcinoma have shifted from a histology-based approach to driver mutation-directed therapies. More recently, two new transforming fusion genes containing protein tyrosine kinase (*KIF58-RET* and *ROS1* fusions) were also identified. Nevertheless, the molecular drivers of about 40% of lung adenocarcinomas still have not been known. Interestingly, the frequencies of some driver mutations have been shown to be significantly different between ethnic groups, and therefore comprehensive genetic studies are necessary to find new druggable targets and targeted treatments of lung cancer.

EP 1 897 940 describes a composition for detecting, determining, or predicting the presence and/or metastasis of esophageal cancer, which composition comprises one or more antibodies against the markers for the metastasis of esophageal cancer, i.e., polypeptides encoded by the AXL, C6orf54, ZBTB11, TNFRSF14, NSUN5, SPEN, LTBP3, SYNGR1, ARL3, SLC13A1, RALGDS, ADD3, MAP3K12, AVPI1, GIMAP6, FLJ11259, C3AR1, PCGF2, PDE6D, PLCG2, GPR148, ARF6, NISCH, GLYAT, IGHM, FBXO38, SLC12A1, PGDS, CD48, IMPA2, HSPA6, EIF3S9, ZNF659, RAB6C, NOL1, DAB2, EBI3, PRSS3, GLB1, SAMSN1, AQP3, CAPZA2, B4GALT2, ARHGEF3, POGK, PRAF1, and HPGD genes.

### [Disclosure]

### [Technical Problem]

It is conformed that the presence of a fusion protein, wherein a part of AXL (AXL receptor tyrosine kinase) and a certain fusion partner, namely a part of MBIP (MAP3K12 binding inhibitory protein 1) are fused, is observed specifically in a human solid tumor, for example a lung cancer, to complete the description.

Accordingly, in an aspect of the present invention, an AXL-MBIP fusion protein is provided, which comprises a fragment of AXL receptor tyrosine kinase (AXL) protein at N-terminal part and a fragment of MAP3K12 binding inhibitory protein 1 (MBIP) protein at C-terminal part, which are linked to each other, wherein the fragment of AXL protein comprises an amino acid sequence encoded by a nucleotide sequence from the 1^{st} exon to the 244^{th} nucleotide of exon 20 of NM_021913 or NM_001699, and the fragment of MBIP protein comprises an amino acid sequence encoded by a nucleotide sequence from the exon 4 to the last exon of NM_016586 or NM_001144891.

According to another aspect of the present invention, a composition for diagnosing a cancer is provided, comprising at least one selected from the group consisting of materials interacting with the fusion protein of any one of claims 1 to 3, and a material interacting with a fusion gene encoding the fusion protein or mRNA corresponding to the fusion gene, wherein the material interacting with the fusion protein is selected from the group consisting of antibodies and aptamers, which bind to the fusion protein, and wherein the material interacting with the fusion gene encoding the fusion protein or mRNA corresponding to the fusion gene is at least one selected from the group consisting of: primer pairs, wherein each primer of a primer pair is capable of hybridizing with 1) a nucleotide sequence consisting of 20 to 100 consecutive nucleotides adjacent to one or the other end of a polynucleotide fragment or mRNA fragment corresponding to the polynucleotide fragment, wherein the polynucleotide fragment consists of 50 to 250 consecutive nucleotides within the fusion gene, comprising a fusion region of the fusion gene, or 2) a complementary nucleotide sequence thereto, and antisense oligonucleotides, probes, and aptamers, which are in 5 to 100 bp length and capable of hybridizing with a fusion region of the fusion gene or mRNA region corresponding to the fusion region, wherein the fusion region comprises the nucleotide sequence of SEQ ID NO: 46.

### [Technical Solution]

To develop a therapy that properly individualized to an individual cancer patient, it is important to understand characteristics of the cancer. As an effective means to understand an overall genetic basis of cancer, such as gene expression, point mutation, gene fusion, alternative splicing, and the like, a large scale parallel RNA sequencing can be illustrated.

A study was conducted for a lung cancer which is one of representative solid cancers. In this study, genetic alterations in 200 fresh surgical specimens of lung adenocarcinoma were broadly surveyed, and 87 of these were analyzed by transciptome sequencing combined with whole-exome sequencing (n=76), and transcriptome sequencing (n=77) for matched adjacent normal tissue samples. Transcriptome sequencing is an adequate method for detecting driver mutations in cancers, since not only somatic point mutations but also aberrant RNA variants, such as fusion genes and alternative splicing, can be examined. Though advances in genomic technologies have enabled genome-wide analyses of cancers, this description is the first large-scale study of lung adenocarcinoma using RNA sequencing.

In particular, transcriptome analyses of 87 surgical specimens of lung adenocarcinoma in Koreans were conducted combined with exome and RNA sequencing of 77 adjacent-normal tissues. Gene expression profiles showed very strong perturbation in cancer tissues taken from smokers. In addition, we identified somatic mutations in transforming genes, such as *EGFR, KRAS, NRAS*, *BRAF*, *PIK3CA, MET and CTNNB1*. The frequency of *EGFR* mutations was extremely high (∼60%) in Korean patients, which can be explained by a dominant haplotype of *EGFR* in Asians. The inventors identified 30 chimeric transcripts including *ALK, RET, ROS1* and other new tyrosine kinase genes (*FGFR2*, *PDGFRA* and *AXL*), which are highly likely to be driver mutations in lung cancer.

As the result of the study, it is confirmed that a fusion gene, wherein two genes positioned on the same chromosome or different chromosomes are fused, which is a mutation that is specifically found in cancer tissue in a surgical specimen of a patient suffered with a cancer (e.g., non-small cell lung cancer (NSCLC)), but not found in normal tissue around in the same tissue with the surgical specimen, and/or a fusion protein generated by the expression of the fusion gene, is present.

Fusion genes specifically present in cancer tissue (e.g., lung cancer rissue) are summarized in Table 1:

**[Table 1]**

| | **Donor Gene** | **Acceptor Gene** | **Chromosome (Donor;Acceptor)** | **Distance (Mb)** |
|---|---|---|---|---|
| 1 | *CCDC6* | *ROS1* | chr10(q21.2);chr6(q22.1) | Interchromosomal |
| 2 | *SCAF11* | *PDGFRA* | chr12(q12);chr4(q12) | Interchromosomal |
| 3 | *FGFR2* | *CIT* | chr10(q26.13);chr12(q24.2 3) | Interchromosomal |
| 4 | *AXL* | *MBIP* | chr19(q13.2);chr14(q13.3) | Interchromosomal |
| 5 | *APLP2* | *TNFSF11* | chr11(q24.3);chr13(q14.11 ) | Interchromosomal |
| 6 | *MAP4K* 3 | *PRKCE* | chr2(p22.1);chr2(p21) | 6.215 |
| 7 | *BCAS3* | *MAP3K3* | chr17(q23.2); chr17(q23.3) | 2.23 |
| 8 | *KRAS* | *CDH13* | chr12(p12.1);chr16(q23.3) | Interchromosomal |
| 9 | *ZFYVE9* | *CGA* | chr1(p32.3);chr6(q14.3) | Interchromosomal |
| 10 | *ERBB2I P* | *MAST4* | chr5(q12.3);chr5(q12.3) | 0.515 |
| 11 | *TPD52L 1* | *TRMT11* | chr6(q22.31); chr6(q22.32) | 0.723 |
| 12 | *TXNRD 1* | *GPR133* | chr12(q23.3);chr12(q24.33 ) | 26.694 |

The fusion genes shown in Table 1 are present specifically in a cancer tissue, and thus, fusion gens and/or fusion proteins encoded thereby may be useful as a biomarker for diagnosing a cancer or a target for treating cancer.

According to an aspect of the present invention, an AXL-MBIP fusion protein is provided, comprising a fragment of AXL receptor tyrosine kinase (AXL) at N-terminal part and a fragment of MAP3K12 binding inhibitory protein 1 (MBIP) protein at C-terminal part, which are linked to each other.

Further described herein are fusion proteins selected from the group consisting of:
CCDC6-ROS1 fusion protein comprising CCDC6 protein or a fragment thereof and ROS1 protein or a fragment, which are fused to each other;
FGFR2-CIT fusion protein comprising FGFR2 protein or a fragment thereof and CIT protein or a fragment thereof, which are fused to each other;
APLP2-TNFSF11 fusion protein comprising APLP2 protein or a fragment thereof and TNFSF11 protein or a fragment thereof, which are fused to each other;
MAP4K3-PRKCE fusion protein comprising MAP4K3 protein or a fragment thereof and PRKCE protein or a fragment thereof, which are fused to each other;
BCAS3-MAP3K3 fusion protein comprising BCAS3 protein or a fragment thereof and MAP3K3 protein or a fragment thereof, which are fused to each other;
KRAS-CDH 13 fusion protein comprising KRAS protein or a fragment thereof and CDH13 protein or a fragment thereof, which are fused to each other;
ZFYVE9-CGA fusion protein comprising ZFYVE9 protein or a fragment thereof and CGA protein or a fragment thereof, which are fused to each other;
ERBB2IP-MAST4 fusion protein comprising ERBB2IP protein or a fragment thereof and MAST4 protein or a fragment thereof, which are fused to each other;
TPD52L1-TRMT11 fusion protein comprising TPD52L1 protein or a fragment thereof and TRMT11 protein or a fragment thereof, which are fused to each other; and
TXNRD1-GPR133 fusion protein comprising TXNRD1 protein or a fragment thereof and GPR133 protein or a fragment thereof, which are fused to each other.

Further described herein is a fusion gene (or polynucleotide molecule), which encodes the fusion protein.

The fusion protein and/or a fusion gene encoding the fusion protein may be useful as a diagnosing biomarker of cancer.

In this description, break points (or fusion region) of protein fragments as fusion partners are described on the base of exons of genes encoding the protein fragments. In the gene encoding the protein fragment, the cleaved site of the gene will not affect the produced protein fragment, if the cleaved site is within an intron between (1) an exon encoding the most N-terminal region of the protein fragment (in case that the protein fragment is a C-terminal fusion partner) or the most C-terminal region of the protein fragment (in case that the protein fragment is a N-terminal fusion partner) and (2) an exon that is removed during production of the protein fragment. Therefore, the cleavage site of a gene for producing the protein fragment may be any site within the intron between the exons (1) and (2) as described above.

CCDC6 gene, which encodes coiled-coil domain containing 6 (CCDC6) protein, may be one from human. Human CCDC6 gene positions in human chromosome 10(q21.2), and CCDC6 protein encoded thereby comprises 474 amino acids in total. CCDC6 protein or a fragment of CCDC6 protein may be an N-terminal fusion partner of CCDC6-ROS1 fusion protein, which is positioned at N-terminal part of the CCDC6-ROS1 fusion protein. For example, CCDC6 gene may comprise the nucleotide sequence of GenBank accession no. NM_005436, and CCDC6 protein may comprise the amino acid sequence encoded by the nucleotide sequence of the CCDC6 gene.

A fragment of CCDC6 protein may comprise an amino acid sequence encoded by a nucleotide sequence from the 1^{st} exon (exon 1) to the 5^{th} exon (exon 5) (based on the position on chromosome 10 ((-) strand), corresponding to the nucleotide region of positions 61572393-61572553) of the CCDC6 gene, and the last nucleotide (C) at the 3' end of exon 5 may be one that does not participate in constituting a codon. For example, a gene encoding a fragment of CCDC6 protein and a fragment of CCDC6 protein encoded by the gene are exemplified in Tables 2 and 3:

**[Table 2]**

| Gene encoding a fragment of CCDC6 protein | | | | | |
|---|---|---|---|---|---|
| CCDC6 gene (Accession No.) | CCDC6 protein coding region-CDS | CCDC6 protein fragment coding region: based on exon | CCDC6 protein fragment coding region: based on cDNA | Position of beak-point on chromosome | nucleotide sequence of break-point region |
| NM_005436 | 233-1657 (1425bp) (SEQ ID NO: 1) | Region from exon 1 to exon 5 | 233∼107 9 (846bp +1 nt(c); 847bp in total) (SEQ ID NO: 2) | chr10:[61572 393 (3' end of exon 5) | |

**[Table 3]**

| Fragment of CCDC6 protein | | |
|---|---|---|
| Full size(a.a) of CCDC6 protein | CCDC6 protein fragment region | Amino acid sequence of breakpoint region |
| 474aa (SEQ ID NO: 4) | aa 1∼282+1nt(c) (amino acid sequence: SEQ ID NO: 5) | AAQLQ+1nt(c) (amino acid sequence: SEQ ID NO: 6) |

ROS1 gene, which encodes receptor tyrosine kinase 1 (ROS1) protein, may be one from human. Human ROS1 gene positions in human chromosome 6(q22.1), and ROS1 protein encoded thereby comprises 2347 amino acids in total. ROS1 protein or a fragment of ROS1 protein may be a C-terminal fusion partner of CCDC6-ROS1 fusion protein, which is positioned at C-terminal part of the CCDC6-ROS1 fusion protein. For example, ROS1 gene may comprise the nucleotide sequence of GenBank accession no. NM_002944, and ROS1 protein may comprise the amino acid sequence encoded by the nucleotide sequence of the ROS1 gene.

A fragment of ROS1 protein may comprise an amino acid sequence encoded by a nucleotide sequence from the 35^{th} exon (exon 35) (based on the position on chromosome 6 ((-) strand), corresponding to the nucleotide region of positions 117642422-117642557) to the last exon of the ROS1 gene. The first two nucleotides (TC) starting from the 5' end of exon 35 may not participate in constituting a codon, and when the ROS1 protein fragment is fused with CCDC6 protein fragment as described above, the first two nucleotides (TC) of the 5' end of the nucleotide sequence encoding the ROS1 protein fragment may be linked to the last nucleotide (C) at the 3' end of the nucleotide sequence encoding the CCDC6 protein fragment to constitute a codon (CTC) thereby encoding an amino acid (L). For example, a gene encoding a fragment of ROS1 protein and a fragment of ROS1 protein encoded by the gene are exemplified in Tables 4 and 5:

**[Table 4]**

| Gene encoding a fragment of ROS1 protein | | | | | |
|---|---|---|---|---|---|
| ROS1gene (Accession No.) | ROS1 protein coding region-CDS | ROS1 protein fragment coding region: based on exon | ROS1 protein fragment coding region: based on cDNA | Position of beak point on chromosome | nucleotide sequence of break-point region |
| NM_002944 | 200∼7243 (7044bp) (SEQ ID NO: 7) | Region from exon 35 to the last exon | 5841∼7243 (2nt(tc) +1401 bp; 1403bp in total) (SEQ ID NO: 8) | chr6:1176425 57] (5' end of exon 35) | |

**[Table 5]**

| Fragment of ROS1 protein | | |
|---|---|---|
| Full size(a.a) of ROS1 protein | ROS1 protein fragment region | Amino acid sequence of breakpoint region |
| 2347aa (SEQ ID NO: 10) | aa 1882∼2347 2nt(tc)+ aa 1882∼2347 (466aa) (amino acid sequence: SEQ ID NO: 11) | 2nt(tc)+WHRRL (amino acid sequence: SEQ ID NO: 12) |

A fusion gene (CCDC6-ROS1 fusion gene) encoding a CCDC6-ROS1 fusion protein comprising CCDC6 protein or a fragment thereof and ROS1 protein or a fragment thereof, which are fused to each other, may comprise a polynucleotide molecule encoding CCDC6 protein or a fragment thereof at 5' terminal part and a polynucleotide molecule encoding ROS1 protein or a fragment thereof at 3' terminal part of the fusion gene. For example, the CCDC6-ROS1 fusion gene may comprise a nucleotide sequence from the 1^{st} exon (exon 1) to exon 5 of NM_005436 at 5' terminal part and a nucleotide sequence from exon 35 to the last exon of NM_002944 at 3' terminal part, wherein the two nucleotide sequences are fused to each other. In a concrete embodiment described herein, the CCDC6-ROS1 fusion gene may a fusion gene (SEQ ID NO: 13; fusion region: SEQ ID NO: 14), wherein the nucleotide sequence (SEQ ID NO: 2) of positions 233 to 1079 of NM_005436 at 5' terminal part and the nucleotide sequence (SEQ ID NO: 8) of positions 5841 to 7243 of NM_002944 at 3' terminal part are linked to each other.

A CCDC6-ROS1 fusion protein may comprise CCDC6 protein or a fragment thereof at N-terminal part and ROS1 protein or a fragment thereof at C-terminal part of the fusion protein, which are linked to each other. For example, the CCDC6-ROS1 fusion protein may comprise an amino acid sequence encoded by a fusion gene that comprises a nucleotide sequence from the 1^{st} exon (exon 1) to exon 5 of NM_005436 at 5' terminal part and a nucleotide sequence from exon 35 to the last exon of NM_002944 at 3' terminal part, wherein the two nucleotide sequences are fused to each other. In a concrete embodiment described herein, the CCDC6-ROS1 fusion protein may be a polypeptide molecule comprising the amino acid sequence (SEQ ID NO: 15; fusion region: SEQ ID NO: 16) that is encoded by the nucleotide sequence of SEQ ID NO: 13, or an amino acid sequence having a sequence homology of at least 90%, at least 95%, or at least 99% to the amino acid sequence of SEQ ID NO: 15.

FGFR2 gene, which encodes fibroblast growth factor receptor 2 (FGFR2) protein, may be one from human. Human FGFR2 gene positions in human chromosome 10(q26.13), and FGFR2 protein may be encoded by the FGFR2 gene. FGFR2 protein or a fragment thereof may be an N-terminal fusion partner of FGFR2-CIT fusion protein, which is positioned at N-terminal part of the FGFR2-CIT fusion protein. For example, FGFR2 gene may comprise a nucleotide sequence of GenBank accession no. NM_001144914, NM_001144916, NM_001144915, NM_001144917, NM_001144918, NM_022970, NM_000141, NM_001144913, or NM_001144919, and FGFR2 protein may comprise an amino acid sequence encoded by the nucleotide sequence of the FGFR2 gene.

A fragment of FGFR2 protein may comprise an amino acid sequence encoded by a nucleotide sequence from the 1^{st} exon (exon 1) to the 19^{th} exon (exon 19) (based on the position on chromosome 10 ((-) strand), corresponding to the nucleotide region of positions 123243212-123243317) of the nucleotide sequence of FGFR2 gene. For example, genes encoding a fragment of FGFR2 protein and fragments of FGFR2 protein encoded by the gene are exemplified in Tables 6 and 7:

**[Table 6]**

| Gene encoding a fragment of FGFR2 protein | | | | | |
|---|---|---|---|---|---|
| FGFR2 gene (Accession No.) | FGFR2 protein coding region-CDS | FGFR2 protein fragment coding region: based on exon | FGFR2 protein fragment coding region: based on cDNA | Position of beak point on chromoso me | nucleotide sequence of break-point region |
| NM_001144 914 | 151∼2280 (2130bp) (SEQ ID NO: 17) | Region from exon 1 to exon 19 | 151∼2115 (1965bp) (SEQ ID NO: 18) | chr10:[123 243212 (3' end of exon 19) | ctcactctcacaaccaatgag (SEQ ID NO: 19) |
| NM_001144 916 | 442∼2562 (2121bp) | | 442∼2397 (1956bp) | | |
| NM_001144 915 | 320∼2443 (2124bp) | | 320∼2353 (2034bp) | | |
| NM_001144 917 | 648∼2765 (2118bp) | | 648∼2600 (1953bp) | | |
| NM_001144 918 | 648∼2762 (2115bp) | | 648∼2597 (1950bp) | | |
| NM_022970 | 648∼3116 (2469bp) | | 648∼2951 (2304bp) | | |
| NM_000141 | 648∼3113 (2466bp) | | 648∼2948 (2301bp) | | |
| NM_001144 913 | 151∼2460 (1813bp) | | 151∼2454 (2304bp) | | |
| NM_001144 919 | 648∼2690 (2043bp) | | 648∼2648 (2001 bp) | | |

**[Table 7]**

| Fragment of FGFR2 protein | | | |
|---|---|---|---|
| FGFR2 gene (Accession No.) | Full size(a.a) of FGFR2 protein | FGFR2 protein fragment region | Amino acid sequence of breakpoint region |
| NM_001144914 | 709aa (SEQ ID NO: 20) | aa 1∼655 (SEQ ID NO: 21) | LTLTTNE (SEQ ID NO: 22) |
| NM_001144916 | 706aa | aa 1∼652 | |
| NM_001144915 | 707aa | aa 1∼678 | |
| NM_001144917 | 705aa | aa 1∼651 | |
| NM_001144918 | 704aa | aa 1∼650 | |
| NM_022970 | 822aa | aa 1∼768 | |
| NM_000141 | 821aa | aa 1∼767 | |
| NM_001144913 | 769aa | aa 1∼768 | |
| NM_001144919 | 680aa | aa 1∼679 | |

CIT gene, which encodes CIT [citron (rho-interacting, serine/threonine kinase 21)] protein, may be one from human. Human CIT gene positions in human chromosome 12(q24.23), and CIT protein encoded thereby comprises 2027 amino acids in total. CIT protein or a fragment of CIT protein may be a C-terminal fusion partner of FGFR2-CIT fusion protein, which is positioned at C-terminal part of the FGFR2-CIT fusion protein. For example, CIT gene may comprise the nucleotide sequence of GenBank accession no. NM_007174, and CIT protein may comprise the amino acid sequence encoded by the nucleotide sequence of the CIT gene.

A fragment of CIT protein may comprise an amino acid sequence encoded by a nucleotide sequence from the 24^{th} exon (exon 24) (based on the position on chromosome 12 ((-) strand), corresponding to the nucleotide region of positions 120180216-12018026) to the last exon of the CIT gene. For example, a gene encoding a fragment of CIT protein and a fragment of CIT protein encoded by the gene are exemplified in Tables 8 and 9:

**[Table 8]**

| Gene encoding a fragment of CIT protein | | | | | |
|---|---|---|---|---|---|
| CIT gene (Accession No.) | CIT protein coding region-CDS | CIT protein fragment coding region: based on exon | CIT protein fragment coding region: based on cDNA | Position of beak point on chromosom e | nucleotide sequence of break-point region |
| NM_007174 | 57-6140 (6084bp) (SEQ ID NO: 23) | region from exon 24 to the last exon | 2835-6140 (3306bp) (SEQ ID NO: 24) | chr12:12018 0269] (5' end of exon 24) | gcacatagagatg aaatccag (SEQ ID NO: 25) |

**[Table 9]**

| Fragment of CIT protein | | |
|---|---|---|
| Full size(a.a) of CIT protein | CIT protein fragment region | Amino acid sequence of breakpoint region |
| 2027aa (SEQ ID NO: 26) | aa 927∼2027 (1101 aa) (SEQ ID NO: 27) | AHRDEIQ (SEQ ID NO: 28) |

A fusion gene (FGFR2-CIT fusion gene) encoding a FGFR2-CIT fusion protein comprising FGFR2 protein or a fragment thereof and CIT protein or a fragment thereof, which are fused to each other, may comprise a polynucleotide molecule encoding FGFR2 protein or a fragment thereof at 5' terminal part and a polynucleotide molecule encoding CIT protein or a fragment thereof at 3' terminal part of the fusion gene. For example, the FGFR2-CIT fusion gene may comprise a nucleotide sequence from the 1^{st} exon (exon 1) to exon 19 of NM_001144914, NM_001144916, NM_001144915, NM_001144917, NM_001144918, NM_022970, NM_000141, NM_001144913, or NM_001144919 at 5' terminal part and a nucleotide sequence from exon 24 to the last exon of NM_007174 at 3' terminal part, wherein the two nucleotide sequences are fused to each other. In a concrete embodiment described herein, the FGFR2-CIT fusion gene may a fusion gene (SEQ ID NO: 29; fusion region: SEQ ID NO: 30), wherein the nucleotide sequence (SEQ ID NO: 18) of positions 151 to 2115 of NM_001144914 at 5' terminal part and the nucleotide sequence (SEQ ID NO: 24) of positions 2835 to 6140 of NM_007174 at 3' terminal part are linked to each other.

A FGFR2-CIT fusion protein may comprise FGFR2 protein or a fragment thereof at N-terminal part and CIT protein or a fragment thereof at C-terminal part of the fusion protein, which are linked to each other. For example, the FGFR2-CIT fusion protein may comprise an amino acid sequence encoded by a fusion gene that comprises a nucleotide sequence from the 1^{st} exon (exon 1) to exon 19 of NM_001144914, NM_001144916, NM_001144915, NM_001144917, NM_001144918, NM_022970, NM_000141, NM_001144913, or NM_001144919 at 5' terminal part and a nucleotide sequence from exon 24 to the last exon of NM_007174 at 3' terminal part, wherein the two nucleotide sequences are fused to each other. In a concrete embodiment described herein, the FGFR2-CIT fusion protein may be a polypeptide molecule comprising the amino acid sequence (SEQ ID NO: 31; fusion region: SEQ ID NO: 32) that is encoded by the nucleotide sequence of SEQ ID NO: 29, or an amino acid sequence having a sequence homology of at least 90%, at least 95%, or at least 99% to the amino acid sequence of SEQ ID NO: 31.

In accordance with the present invention, the AXL gene, which encodes AXL receptor tyrosine kinase (AXL) protein, is one from human. Human AXL gene positions in human chromosome 19(q13.2) of, and AXL protein is one encoded by the AXL gene. According to the present invention, a fragment of AXL protein is an N-terminal fusion partner of AXL-MBIP fusion protein, which is positioned at N-terminal part of the AXL-MBIP fusion protein. The AXL gene comprises a nucleotide sequence of GenBank accession no. NM_021913 or NM_001699, and the AXL protein comprises an amino acid sequence encoded by said nucleotide sequence of the AXL gene.

The fragment of AXL protein comprises an amino acid sequence encoded by a nucleotide sequence from the 1^{st} exon (exon 1) to the 244^{th} nucleotide of the 20^{th} exon (exon 20) (based on the position on chromosome 19 ((+) strand), corresponding to the nucleotide region of positions 41765458-41767670) of the AXL gene, the AXL gene comprising a nucleotide sequence of GenBank accession no. NM_021913 or NM_001699. Genes encoding a fragment of AXL protein and fragments of AXL protein encoded by the gene are exemplified in Tables 10 and 11:

**[Table 10]**

| Gene encoding a fragment of AXL protein | | | | | |
|---|---|---|---|---|---|
| AXL gene (Accession No.) | AXL protein coding region-CDS | AXL protein fragment coding region: based on exon | AXL protein fragment coding region: based on cDNA | Position of beak point on chromosome | nucleotide sequence of break-point region |
| NM_021913 | 191∼2875 (2685bp) (SEQ ID NO: 33) | Region from exon 1 to the 244^{th} nucleotide of exon 20 | 191∼2767 (2577bp) (SEQ ID NO: 34) | chr19:4176570 1] 3' position of the 244^{th} nucleotide of exon 20 | ctcactgcggct gag (SEQ ID NO: 35) |
| NM_001699 | 191∼2848 (2658bp) | | 191∼2740 (2550bp) | | |

**[Table 11]**

| Fragment of AXL protein | | | |
|---|---|---|---|
| AXL gene (Accession No.) | Full size(a.a) of AXL protein | AXL protein fragment region | Amino acid sequence of breakpoint region |
| NM_021913 | 894aa (SEQ ID NO: 36) | aa 1∼859 (SEQ ID NO: 37) | LTAAE (SEQ ID NO: 38) |
| NM_001699 | 885aa | aa 1∼850 | |

In accordance with the present invention, the MBIP gene, which encodes MAP3K12 binding inhibitory protein 1 (MBIP) protein, is one from human. Human MBIP gene positions in human chromosome 14(q13.3), and MBIP protein is encoded by the MBIP gene. According to the present invention, a fragment of MBIP protein is a C-terminal fusion partner of AXL-MBIP fusion protein, which is positioned at C-terminal part of the AXL-MBIP fusion protein. The MBIP gene comprises the nucleotide sequence of GenBank accession no. NM_016586 or NM_001144891, and the MBIP protein comprises the amino acid sequence encoded by said nucleotide sequence of the MBIP gene.

The fragment of MBIP protein comprises an amino acid sequence encoded by a nucleotide sequence from the 4^{th} exon (exon 4) (based on the position on chromosome 14 ((-) strand), corresponding to the nucleotide region of positions 36783718-36783814) to the last exon of the MBIP gene, the MBIP gene comprising a nucleotide sequence of GenBank accession no. NM_016586 or NM_001144891. Genes encoding a fragment of MBIP protein and fragments of MBIP protein encoded by the gene are exemplified in Tables 12 and 13:

**[Table 12]**

| Gene encoding a fragment of MBIP protein | | | | | |
|---|---|---|---|---|---|
| MBIP gene (Accession No.) | MBIP protein coding region-CDS | MBIP protein fragment coding region: based on exon | MBIP protein fragment coding region: based on cDNA | Position of beak point on chromosome | nucleotide sequence of break-point region |
| NM_016586 | 89∼1123 (1035bp) (SEQ ID NO: 39) | Region from exon 4 to the last exon | 563∼1123 (561 bp) (SEQ ID NO: 40) | chr14:367838 14] (5' end of exon 4) | attgacagacgaa ta (SEQ ID NO: 41) |
| NM_001144 891 | 89∼1120 (1032bp) | | 563∼1120 (558bp) | | |

**[Table 13]**

| Fragment of MBIP protein | | | |
|---|---|---|---|
| MBIP gene (Accession No.) | Full size(a.a) of MBIP protein | MBIP protein fragment region | Amino acid sequence of breakpoint region |
| NM_016586 | 344aa (SEQ ID NO: 42) | aa 159∼344 (186aa) (SEQ ID NO: 43) | IDRRI (SEQ ID NO: 44) |
| NM_00114489 1 | 343aa | aa 159∼343 (185aa) | |

Further described herein is a fusion gene (AXL-MBIP fusion gene) encoding a AXL-MBIP fusion protein comprising AXL protein or a fragment thereof and MBIP protein or a fragment thereof which are fused to each other. Said fusion gene may comprise a polynucleotide molecule encoding AXL protein or a fragment thereof at 5' terminal part and a polynucleotide molecule encoding MBIP protein or a fragment thereof at 3' terminal part of the fusion gene. For example, the AXL-MBIP fusion gene may comprise a nucleotide sequence from the 1^{st} exon (exon 1) to the 244^{th} nucleotide of exon 20 of NM_021913 or NM_001699 at 5' terminal part and a nucleotide sequence from exon 4 to the last exon of NM_016586 or NM_001144891 at 3' terminal part, wherein the two nucleotide sequences are fused to each other. In a concrete embodiment of the present invention, the AXL-MBIP fusion gene may a fusion gene (SEQ ID NO: 45; fusion region: SEQ ID NO: 46), wherein the nucleotide sequence (SEQ ID NO: 34) of positions 191 to 2767 of NM_021913 at 5' terminal part and the nucleotide sequence (SEQ ID NO: 40) of positions 563 to 1123 of NM_016586 at 3' terminal part are linked to each other.

According to the present invention, an AXL-MBIP fusion protein comprises a fragment of AXL protein at N-terminal part and a fragment of MBIP protein at C-terminal part of the fusion protein, which are linked to each other. The AXL-MBIP fusion protein comprises an amino acid sequence encoded by a fusion gene that comprises a nucleotide sequence from the 1^{st} exon (exon 1) to the 244^{th} nucleotide of exon 20 of NM_021913 or NM_001699 at 5' terminal part and a nucleotide sequence from exon 4 to the last exon of NM_016586 or NM_001144891 at 3' terminal part, wherein the two nucleotide sequences are fused to each other. In a concrete embodiment of the present invention, the AXL-MBIP fusion protein may be a polypeptide molecule comprising the amino acid sequence (SEQ ID NO: 47; fusion region: SEQ ID NO: 48) that is encoded by the nucleotide sequence of SEQ ID NO: 45, or an amino acid sequence having a sequence homology of at least 90%, at least 95%, or at least 99% to the amino acid sequence of SEQ ID NO: 47.

APLP2 gene, which encodes amyloid beta (A4) precursor-like protein 2 (APLP2 protein), may be one from human. Human APLP2 gene positions in human chromosome 11 (q24.3), and APLP2 protein may be encoded by the APLP2 gene. APLP2 protein or a fragment thereof may be an N-terminal fusion partner of APLP2-TNFSF11 fusion protein, which is positioned at N-terminal part of the APLP2-TNFSF11 fusion protein. For example, APLP2 gene may comprise a nucleotide sequence of GenBank accession no. NM_001642, NM_001142276, NM_001142278, NM_001142277, NR_024516, or NR_024515, and APLP2 protein may comprise an amino acid sequence encoded by the nucleotide sequence of the APLP2 gene.

A fragment of APLP2 protein may comprise an amino acid sequence encoded by a nucleotide sequence from the 1^{st} exon (exon 1) to the 12^{th} exon (exon 12) (based on the position on chromosome 11 ((+) strand), corresponding to the nucleotide region of positions 129999933-130000061) of the nucleotide sequence of the APLP2 gene. For example, genes encoding a fragment of APLP2 protein and fragments of APLP2 protein encoded by the gene are exemplified in Tables 14 and 15:

**[Table 14]**

| Gene encoding a fragment of APLP2 protein | | | | | |
|---|---|---|---|---|---|
| APLP2 gene (Accession No.) | APLP2 protein coding region-CDS | APLP2 protein fragment coding region: based on exon | APLP2 protein fragment coding region: based on cDNA | Position of beak point on chromosome | nucleotide sequence of break-point region |
| NM_001642 | 158∼2449 (2292bp) (SEQ ID NO: 49) | Region from exon 1 to exon 12 | 158∼1741 (1584bp) (SEQ ID NO: 50) | chr11:130000 061] (3' end of exon 12) | gcggcccagatg aaatcccag (SEQ ID NO: 51) |
| NM_001142276 | 158∼2413 (2256bp) | | 158∼1741 (1584bp) | | |
| NM_001142278 | 158∼1726 (1569bp) | | 158∼1054 (896bp) | | |
| NM_001142277 | 158∼2245 (2088bp) | | 158∼1573 (1416bp) | | |

**[Table 15]**

| Fragment of APLP2 protein | | | |
|---|---|---|---|
| APLP2gene (Accession No.) | Full size(a.a) of APLP2 protein | APLP2 protein fragment region | Amino acid sequence of breakpoint region |
| NM_001642 | 763aa (SEQ ID NO: 52) | aa 1∼528 (SEQ ID NO: 53) | AAQMKSQ (SEQ ID NO: 54) |
| NM_001142276 | 751aa | aa 1∼528 | |
| NM_001142278 | 522aa | aa 1∼299 | |
| NM_001142277 | 695aa | aa 1∼472 | |

TNFSF11 gene, which encodes tumor necrosis factor (ligand) superfamily, member 11 (TNFSF11) protein, may be one from human. Human TNFSF11 gene positions in human chromosome 13(q14.11), and TNFSF11 protein may be encoded by the TNFSF11 gene. TNFSF11 protein or a fragment of TNFSF11 protein may be a C-terminal fusion partner of APLP2-TNFSF11 fusion protein, which is positioned at C-terminal part of the APLP2-TNFSF11 fusion protein. For example, TNFSF11 gene may comprise the nucleotide sequence of GenBank accession no. NM_033012 or NM_003701, and TNFSF11 protein may comprise the amino acid sequence encoded by the nucleotide sequence of the TNFSF11 gene.

A fragment of TNFSF11 protein may comprise an amino acid sequence encoded by a nucleotide sequence from the 6^{th} exon (exon 6) (based on the position on chromosome 13 ((+) strand), corresponding to the nucleotide region of positions 43174888-43174933) to the last exon of the TNFSF11 gene. For example, genes encoding a fragment of TNFSF11 protein and fragments of TNFSF11 protein encoded by the gene are exemplified in Tables 16 and 17:

**[Table 16]**

| Gene encoding a fragment of TNFSF11 protein | | | | | |
|---|---|---|---|---|---|
| TNFSF11gene (Accession No.) | TNFSF11 protein coding region-CDS | TNFSF11 protein fragment coding region: based on exon | TNFSF11 protein fragment coding region: based on cDNA | Position of beak point on chromosome | nucleotide sequence of break-point region |
| NM_033012 | 530∼1264 (735bp) (SEQ ID NO: 55) | Region from exon 6 to the last exon | 698∼1264 (567bp) (SEQ ID NO: 56) | chr13:[43174 888 (5' end of exon 6) | |
| NM_003701 | 150∼1122 (973bp) | | 537∼2198 (1662bp) | | |

**[Table 17]**

| Fragment of TNFSF11 protein | | | |
|---|---|---|---|
| TNFSF11gene (Accession No.) | Full size(a.a) of TNFSF11 protein | TNFSF11 protein fragment region | Amino acid sequence of breakpoint region |
| NM_033012 | 244aa (SEQ ID NO: 58) | aa 57∼244 (188aa) (SEQ ID NO: 59) | ELQHIVG (SEQ ID NO: 60) |
| NM_003701 | 315aa | aa 130∼315 (186aa) | |

A fusion gene (APLP2-TNFSF11 fusion gene) encoding a APLP2-TNFSF11 fusion protein comprising APLP2 protein or a fragment thereof and TNFSF11 protein or a fragment thereof, which are fused to each other, may comprise a polynucleotide molecule encoding APLP2 protein or a fragment thereof at 5' terminal part and a polynucleotide molecule encoding TNFSF11 protein or a fragment thereof at 3' terminal part of the fusion gene. For example, the APLP2-TNFSF11 fusion gene may comprise a nucleotide sequence from the 1^{st} exon (exon 1) to exon 12 of NM_001642, NM_001142276, NM_001142278, NM_001142277, NR_024516, or NR_024515 at 5' terminal part and a nucleotide sequence from exon 6 to the last exon of NM_033012 or NM_003701 at 3' terminal part, wherein the two nucleotide sequences are fused to each other. In a concrete embodiment described herein, the APLP2-TNFSF11 fusion gene may a fusion gene (SEQ ID NO: 61; fusion region: SEQ ID NO: 62), wherein the nucleotide sequence (SEQ ID NO: 50) of positions 158 to 1741 of NM_001642 at 5' terminal part and the nucleotide sequence (SEQ ID NO: 56) of positions 698 to 1264 of NM_033012 at 3' terminal part are linked to each other.

An APLP2-TNFSF11 fusion protein may comprise APLP2 protein or a fragment thereof at N-terminal part and TNFSF11 protein or a fragment thereof at C-terminal part of the fusion protein, which are linked to each other. For example, the APLP2-TNFSF11 fusion protein may comprise an amino acid sequence encoded by a fusion gene that comprises a nucleotide sequence from the 1^{st} exon (exon 1) to exon 12 of NM_001642, NM_001142276, NM_001142278, NM_001142277, NR_024516, or NR_024515 at 5' terminal part and a nucleotide sequence from exon 6 to the last exon of NM_033012 or NM_003701 at 3' terminal part, wherein the two nucleotide sequences are fused to each other. In a concrete embodiment described herein, the APLP2-TNFSF11 fusion protein may be a polypeptide molecule comprising the amino acid sequence (SEQ ID NO: 63; fusion region: SEQ ID NO: 64) that is encoded by the nucleotide sequence of SEQ ID NO: 61, or an amino acid sequence having a sequence homology of at least 90%, at least 95%, or at least 99% to the amino acid sequence of SEQ ID NO: 63.

MAP4K3 gene, which encodes mitogen-activated protein kinase kinase kinase kinase3 (MAP4K3) protein, may be one from human. Human MAP4K3 gene positions in human chromosome 2(p22.1), and MAP4K3 protein encoded thereby comprises 894 amino acids in total. MAP4K3 protein or a fragment of MAP4K3 protein may be an N-terminal fusion partner of MAP4K3-PRKCE fusion protein, which is positioned at N-terminal part of the MAP4K3-PRKCE fusion protein. For example, MAP4K3 gene may comprise the nucleotide sequence of GenBank accession no. NM_003618, and MAP4K3 protein may comprise the amino acid sequence encoded by the nucleotide sequence of the MAP4K3 gene.

A fragment of MAP4K3 protein may comprise an amino acid sequence encoded by a nucleotide sequence of the 1^{st} exon (exon 1) (based on the position on chromosome 2 ((-) strand), corresponding to the nucleotide region of positions 39664033-39664219) of the MAP4K3 gene. For example, a gene encoding a fragment of MAP4K3 protein and the fragment of MAP4K3 protein encoded by the gene are exemplified in Tables 18 and 19:

**[Table 18]**

| Gene encoding a fragment of MAP4K3 protein | | | | | |
|---|---|---|---|---|---|
| MAP4K3 gene (Accession No.) | MAP4K3 protein coding region-CDS | MAP4K3 protein fragment coding region: based on exon | MAP4K3 protein fragment coding region: based on cDNA | Position of beak point on chromosome | nucleotide sequence of break-point region |
| NM_003618 | 326∼301 0 (2685bp) (SEQ ID NO: 65) | exon 1 region | 326∼21 (96bp) (SEQ ID NO: 66) | chr2:[396640 33 (3' end of exon 1) | |

**[Table 19]**

| Fragment of MAP4K3 protein | | |
|---|---|---|
| Full size(a.a) of MAP4K3 protein | MAP4K3 protein fragment region | Amino acid sequence of breakpoint region |
| 894aa (SEQ ID NO: 68) | aa 1∼32 (SEQ ID NO: 69) | TYGDVYK (SEQ ID NO: 70) |

PRKCE gene, which encodes "protein kinase C, epsilon" (PRKCE) protein, may be one from human. Human ROS1 gene positions in human chromosome 2(p21), and PRKCE protein encoded thereby comprises 737 amino acids in total. PRKCE protein or a fragment of PRKCE protein may be a C-terminal fusion partner of MAP4K3-PRKCE fusion protein, which is positioned at C-terminal part of the MAP4K3-PRKCE fusion protein. For example, PRKCE gene may comprise the nucleotide sequence of GenBank accession no. NM_005400, and PRKCE protein may comprise the amino acid sequence encoded by the nucleotide sequence of the PRKCE gene.

A fragment of PRKCE protein may comprise an amino acid sequence encoded by a nucleotide sequence from the 2^{nd} exon (exon 2) (based on the position on chromosome 2 ((+) strand), corresponding to the nucleotide region of positions 46070139-46070202) to the last exon of the PRKCE gene. For example, a gene encoding a fragment of PRKCE protein and the fragment of PRKCE protein encoded by the gene are exemplified in Tables 20 and 21:

**[Table 20]**

| Gene encoding a fragment of PRKCE protein | | | | | |
|---|---|---|---|---|---|
| PRKCE gene (Accession No.) | PRKCE protein coding region-CDS | PRKCE protein fragment coding region: based on exon | PRKCE protein fragment coding region: based on cDNA | Position of beak point on chromosome | nucleotide sequence of break-point region |
| NM_005400 | 198∼2411 (2214bp) (SEQ ID NO: 71) | Region from exon 2 to the last exon | 546∼2411 (1866bp) (SEQ ID NO: 72 | chr2:[46070139 (5' end of exon 2) | |

**[Table 21]**

| Fragment of PRKCE protein | | |
|---|---|---|
| Full size(a.a) of PRKCE protein | PRKCE protein fragment region | Amino acid sequence of breakpoint region |
| 737aa (SEQ ID NO: 74) | aa 117∼737 (621aa) (SEQ ID NO: 75) | IDLEPEGR (SEQ ID NO: 76) |

A fusion gene (MAP4K3-PRKCE fusion gene) encoding a MAP4K3-PRKCE fusion protein comprising MAP4K3 protein or a fragment thereof and PRKCE protein or a fragment thereof, which are fused to each other, may comprise a polynucleotide molecule encoding MAP4K3 protein or a fragment thereof at 5' terminal part and a polynucleotide molecule encoding PRKCE protein or a fragment thereof at 3' terminal part of the fusion gene. For example, the MAP4K3-PRKCE fusion gene may comprise a nucleotide sequence of the 1^{st} exon (exon 1) of NM_003618 at 5' terminal part and a nucleotide sequence from exon 2 to the last exon of NM_005400 at 3' terminal part, wherein the two nucleotide sequences are fused to each other. In a concrete embodiment described herein, the MAP4K3-PRKCE fusion gene may a fusion gene (SEQ ID NO: 77; fusion region: SEQ ID NO: 78), wherein the nucleotide sequence (SEQ ID NO: 66) of positions 326 to 421 of NM_003618 at 5' terminal part and the nucleotide sequence (SEQ ID NO: 72) of positions 546 to 2411 of NM_005400 at 3' terminal part are linked to each other.

A MAP4K3-PRKCE fusion protein may comprise MAP4K3 protein or a fragment thereof at N-terminal part and PRKCE protein or a fragment thereof at C-terminal part of the fusion protein, which are linked to each other. For example, the MAP4K3-PRKCE fusion protein may comprise an amino acid sequence encoded by a fusion gene that comprises a nucleotide sequence of the 1^{st} exon (exon 1) of NM_003618 at 5' terminal part and a nucleotide sequence from exon 2 to the last exon of NM_005400 at 3' terminal part, wherein the two nucleotide sequences are fused to each other. In a concrete embodiment described herein, the MAP4K3-PRKCE fusion protein may be a polypeptide molecule comprising the amino acid sequence (SEQ ID NO: 79; fusion region: SEQ ID NO: 80) that is encoded by the nucleotide sequence of SEQ ID NO: 77, or an amino acid sequence having a sequence homology of at least 90%, at least 95%, or at least 99% to the amino acid sequence of SEQ ID NO: 79.

BCAS3 gene, which encodes breast carcinoma amplified sequence 3 (BCAS3) protein, may be one from human. Human BCAS3 gene positions in human chromosome 17(q23.2), and BCAS3 protein may be a protein encoded by the BCAS3 gene. BCAS3 protein or a fragment of BCAS3 protein may be an N-terminal fusion partner of BCAS3-MAP3K3 fusion protein, which is positioned at N-terminal part of the BCAS3-MAP3K3 fusion protein. For example, BCAS3 gene may comprise the nucleotide sequence of GenBank accession no. NM_017679 or NM_001099432, and BCAS3 protein may comprise the amino acid sequence encoded by the nucleotide sequence of the BCAS3 gene.

A fragment of BCAS3 protein may comprise an amino acid sequence encoded by a nucleotide sequence from the 1^{st} exon (exon 1) to the 23^{th} exon (exon 23) (based on the position on chromosome 17 ((+) strand), corresponding to the nucleotide region of positions 59161828-59161925) of the BCAS3 gene, and the last nucleotide (G) at the 3' end of exon 23 may be one that does not participate in constituting a codon. For example, genes encoding a fragment of BCAS3 protein and fragments of BCAS3 protein encoded by the gene are exemplified in Tables 22 and 23:

**[Table 22]**

| Gene encoding a fragment of BCAS3 protein | | | | | |
|---|---|---|---|---|---|
| BCAS3 gene (Accession No.) | BCAS3 protein coding region-CDS | BCAS3 protein fragment coding region: based on exon | BCAS3 protein fragment coding region: based on cDNA | Position of beak point on chromosome | nucleotide sequence of break-point region |
| NM_001099432 | 110∼2896 (2787bp) (SEQ ID NO: 81) | Region from exon 1 to exon 23 | 110∼2579 (2469bp +1 nt(c); 2470bp in total) (SEQ ID NO: 82) | chr17:59161 925] (3' end of exon 23) | |
| NM_017679 | 110∼285 1 (2742bp) | | 110∼2534 (2454bp) | | |

**[Table 23]**

| Fragment of BCAS3 protein | | | |
|---|---|---|---|
| BCAS3gene (Accession No.) | Full size(a.a) of BCAS3 protein | BCAS3 protein fragment region | Amino acid sequence of breakpoint region |
| NM_001099432 | 928aa (SEQ ID NO: 84) | aa 1∼823+1nt(g) (amino acid sequence: SEQ ID NO: 85) | TVIDAAS+1nt(g) (amino acid sequence: SEQ ID NO: 86) |
| NM_017679 | 913aa | aa 1∼808 | |

MAP3K3 gene, which encodes mitogen activated protein kinase kinase kinase3 (MAP3K3) protein, may be one from human. Human MAP3K3 gene positions in human chromosome 17(q23.3), and MAP3K3 protein may be one encoded by the MAP3K3 gene. MAP3K3 protein or a fragment of MAP3K3 protein may be a C-terminal fusion partner of BCAS3-MAP3K3 fusion protein, which is positioned at C-terminal part of the BCAS3-MAP3K3 fusion protein. For example, MAP3K3 gene may comprise the nucleotide sequence of GenBank accession no. NM_002401 or NM_203351, and MAP3K3 protein may comprise the amino acid sequence encoded by the nucleotide sequence of the MAP3K3 gene.

A fragment of MAP3K3 protein may comprise an amino acid sequence encoded by a nucleotide sequence from the 2^{nd} exon (exon 2) (based on the position on chromosome 17 ((+) strand), corresponding to the nucleotide region of positions 61710041-61710162) to the last exon of the MAP3K3 gene. The first two nucleotides (AC) starting from the 5' end of exon 35 may not participate in constituting a codon, and when the MAP3K3 protein fragment is fused with BCAS3 protein fragment as described above, the first two nucleotides (AC) of the 5' end of the nucleotide sequence encoding the MAP3K3 protein fragment may be linked to the last nucleotide (G) at the 3' end of the nucleotide sequence encoding the BCAS3 protein fragment to constitute a codon (GAC) thereby encoding an amino acid (D). For example, genes encoding a fragment of MAP3K3 protein and fragments of MAP3K3 protein encoded by the gene are exemplified in Tables 24 and 25:

**[Table 24]**

| Gene encoding a fragment of MAP3K3protein | | | | | |
|---|---|---|---|---|---|
| MAP3K3 gene (Accession No.) | MAP3K3 protein coding region-CDS | MAP3K3 protein fragment coding region: based on exon | MAP3K3 protein fragment coding region: based on cDNA | Position of beak point on chromosome | nucleotide sequence of break-point region |
| NM_002401 | 320-2200 (1881 bp) (SEQ ID NO: 87) | Region from exon 2 to the last exon | 324-2200 (2nt(ac) +1875bp; 1877bp in total) (SEQ ID NO: 88) | chr17:61710041] (5' end of exon 2) | acgaacaggag gcattgaactca (SEQ ID NO: 89) |
| NM_203351 | 320∼2293 (1974bp) | | 324∼2293 (1970bp) | | |

**[Table 25]**

| Fragment of MAP3K3 protein | | | |
|---|---|---|---|
| MAP3K3 gene (Accession No.) | Full size(a.a) of MAP3K3 protein | MAP3K3 protein fragment region | Amino acid sequence of breakpoint region |
| NM_002401 | 626aa (SEQ ID NO: 90) | aa 3∼626 (2nt(ac)+ aa 3∼626; 624aa in total) (amino acid sequence: SEQ ID NO: 91) | 2nt(ac)+EQEALNS (amino acid sequence: SEQ ID NO: 92) |
| NM_203351 | 657aa | aa 3∼655 | |

A fusion gene (BCAS3-MAP3K3 fusion gene) encoding a BCAS3-MAP3K3 fusion protein comprising BCAS3 protein or a fragment thereof and MAP3K3 protein or a fragment thereof, which are fused to each other, may comprise a polynucleotide molecule encoding BCAS3 protein or a fragment thereof at 5' terminal part and a polynucleotide molecule encoding MAP3K3 protein or a fragment thereof at 3' terminal part of the fusion gene. For example, the BCAS3-MAP3K3 fusion gene may comprise a nucleotide sequence from the 1^{st} exon (exon 1) to exon 23 of NM_017679 or NM_001099432 at 5' terminal part and a nucleotide sequence from exon 2 to the last exon of NM_002401 or NM_203351 at 3' terminal part, wherein the two nucleotide sequences are fused to each other. In a concrete embodiment described herein, the BCAS3-MAP3K3 fusion gene may a fusion gene (SEQ ID NO: 93; fusion region: SEQ ID NO: 94), wherein the nucleotide sequence (SEQ ID NO: 82) of positions 110 to 2579 of NM_001099432 at 5' terminal part and the nucleotide sequence (SEQ ID NO: 88) of positions 324 to 2200 of NM_002401 at 3' terminal part are linked to each other.

A BCAS3-MAP3K3 fusion protein may comprise BCAS3 protein or a fragment thereof at N-terminal part and MAP3K3 protein or a fragment thereof at C-terminal part of the fusion protein, which are linked to each other. For example, the BCAS3-MAP3K3 fusion protein may comprise an amino acid sequence encoded by a fusion gene that comprises a nucleotide sequence from the 1^{st} exon (exon 1) to exon 23 of NM_017679 or NM_001099432 at 5' terminal part and a nucleotide sequence from exon 2 to the last exon of NM_002401 or NM_203351 at 3' terminal part, wherein the two nucleotide sequences are fused to each other. In a concrete embodiment described herein, the BCAS3-MAP3K3 fusion protein may be a polypeptide molecule comprising the amino acid sequence (SEQ ID NO: 95; fusion region: SEQ ID NO: 96) that is encoded by the nucleotide sequence of SEQ ID NO: 93, or an amino acid sequence having a sequence homology of at least 90%, at least 95%, or at least 99% to the amino acid sequence of SEQ ID NO: 95.

KRAS gene, which encodes Ki-ras2 Kirsten rat sarcoma viral oncogene homolog (KRAS) protein, may be one from human. Human KRAS gene positions in human chromosome 12(p12.1), and KRAS protein may be encoded by the KRAS gene. KRAS protein or a fragment thereof may be an N-terminal fusion partner of KRAS-CDH13 fusion protein, which is positioned at N-terminal part of the KRAS-CDH13 fusion protein. For example, KRAS gene may comprise a nucleotide sequence of GenBank accession no. NM_004985 or NM_033360, and KRAS protein may comprise an amino acid sequence encoded by nucleotide sequence of the KRAS gene.

A fragment of KRAS protein may comprise an amino acid sequence encoded by a nucleotide sequence from the 1^{st} exon (exon 1) to the 4^{th} exon (exon 4) (based on the position on chromosome 12 ((-) strand), corresponding to the nucleotide region of positions 25378548-25378707) of the nucleotide sequence of KRAS gene. For example, genes encoding a fragment of KRAS protein and fragments of KRAS protein encoded by the gene are exemplified in Tables 26 and 27:

**[Table 26]**

| Gene encoding a fragment of KRAS protein | | | | | |
|---|---|---|---|---|---|
| KRAS gene (Accession No.) | KRAS protein coding region-CDS | KRAS protein fragment coding region: based on exon | KRAS protein fragment coding region: based on cDNA | Position of beak point on chromosome | nucleotide sequence of break-point region |
| NM_004985 | 182∼748 (567bp) (SEQ ID NO: 97) | Region from exon 1 to exon 4 | 182∼631 (450bp) (SEQ ID NO: 98) | chr12:[25378 548 (3' end of exon 4) | |
| NM_033360 | 182∼751 (570bp) | | 182∼631 (450bp) | | |

**[Table 27]**

| Fragment of KRAS protein | | | |
|---|---|---|---|
| KRAS gene (Accession No.) | Full size(a.a) of KRAS protein | KRAS protein fragment region | Amino acid sequence of breakpoint region |
| NM_004985 | 188aa (SEQ ID NO: 100) | aa 1∼150 (SEQ ID NO: 101) | TSAKTRQ (SEQ ID NO: 102) |
| NM_033360 | 189aa | aa 1∼150 | |

CDH13 gene, which encodes CDH13 (cadherin 13, H-cadherin) protein, may be one from human. Human CDH13 gene positions in human chromosome 16(q23.3), and CDH13 protein may be one encoded by the CDH13 gene. CDH13 protein or a fragment of CDH13 protein may be a C-terminal fusion partner of KRAS-CDH13 fusion protein, which is positioned at C-terminal part of the KRAS-CDH13 fusion protein. For example, CDH13 gene may comprise the nucleotide sequence of GenBank accession no. NM_001257, and CDH13 protein may comprise the amino acid sequence encoded by the nucleotide sequence of the CDH13 gene.

A fragment of CDH13 protein may comprise an amino acid sequence encoded by a nucleotide sequence from the 5^{th} exon (exon 5) (based on the position on chromosome 16 ((+) strand), corresponding to the nucleotide region of positions 83158990-83159106) to the last exon of the CDH13 gene. For example, a gene encoding a fragment of CDH13 protein and the fragment of CDH13 protein encoded by the gene are exemplified in Tables 28 and 29:

**[Table 28]**

| Gene encoding a fragment of CDH13 protein | | | | | |
|---|---|---|---|---|---|
| CDH13 gene (Accession No.) | CDH13 protein coding region-CDS | CDH13 protein fragment coding region: based on exon | CDH13 protein fragment coding region: based on cDNA | Position of beak point on chromosome | nucleotide sequence of break-point region |
| NM_001257 | 300∼2441 (2142bp) (SEQ ID NO: 103) | Region from exon 5 to the last exon | 666∼2441 (1776bp) (SEQ ID NO: 104) | chr16:[83158990 (5' end of exon 5) | |

**[Table 29]**

| Fragment of CDH13 protein | | | |
|---|---|---|---|
| CDH13 gene (Accession No.) | Full size(a.a) of CDH13 protein | CDH13 protein fragment region | Amino acid sequence of breakpoint region |
| NM_001257 | 713aa (SEQ ID NO: 106) | aa 123∼713 (591aa) (SEQ ID NO: 107) | DIFKFAR (SEQ ID NO: 108) |

A fusion gene (KRAS-CDH13 fusion gene) encoding a KRAS-CDH13 fusion protein comprising KRAS protein or a fragment thereof and CDH13 protein or a fragment thereof, which are fused to each other, may comprise a polynucleotide molecule encoding KRAS protein or a fragment thereof at 5' terminal part and a polynucleotide molecule encoding CDH13 protein or a fragment thereof at 3' terminal part of the fusion gene. For example, the KRAS-CDH13 fusion gene may comprise a nucleotide sequence from the 1^{st} exon (exon 1) to exon 4 of NM_004985 or NM_033360 at 5' terminal part and a nucleotide sequence from exon 5 to the last exon of NM_001257 at 3' terminal part, wherein the two nucleotide sequences are fused to each other. In a concrete embodiment described herein, the KRAS-CDH13 fusion gene may a fusion gene (SEQ ID NO: 109; fusion region: SEQ ID NO: 110), wherein the nucleotide sequence (SEQ ID NO: 98) of positions 182 to 631 of NM_004985 at 5' terminal part and the nucleotide sequence (SEQ ID NO: 104) of positions 666 to 2441 of NM_001257 at 3' terminal part are linked to each other.

A KRAS-CDH13 fusion protein may comprise KRAS protein or a fragment thereof at N-terminal part and CDH13 protein or a fragment thereof at C-terminal part of the fusion protein, which are linked to each other. For example, the KRAS-CDH13 fusion protein may comprise an amino acid sequence encoded by a fusion gene that comprises a nucleotide sequence from the 1^{st} exon (exon 1) to exon 4 of NM_004985 or NM_033360 at 5' terminal part and a nucleotide sequence from exon 5 to the last exon of NM_001257 at 3' terminal part, wherein the two nucleotide sequences are fused to each other. In a concrete embodiment described herein, the KRAS-CDH13 fusion protein may be a polypeptide molecule comprising the amino acid sequence (SEQ ID NO: 111; fusion region: SEQ ID NO: 112) that is encoded by the nucleotide sequence of SEQ ID NO: 109, or an amino acid sequence having a sequence homology of at least 90%, at least 95%, or at least 99% to the amino acid sequence of SEQ ID NO: 111.

ZFYVE9 gene, which encodes ZFYVE9 (zinc finger, FYVE domain containing 9) protein, may be one from human. Human ZFYVE9 gene positions in human chromosome 1(p32.3), and ZFYVE9 protein may be one encoded by the ZFYVE9 gene. ZFYVE9 protein or a fragment thereof may be an N-terminal fusion partner of ZFYVE9-CGA fusion protein, which is positioned at N-terminal part of the ZFYVE9-CGA fusion protein. For example, ZFYVE9 gene may comprise a nucleotide sequence of GenBank accession no. NM_007324 or NM_004799, and ZFYVE9 protein may comprise an amino acid sequence encoded by the nucleotide sequence of the ZFYVE9 gene.

A fragment of ZFYVE9 protein may comprise an amino acid sequence encoded by a nucleotide sequence from the 1^{st} exon (exon 1) to the 16^{th} exon (exon 16) (based on the position on chromosome 1 ((+) strand), corresponding to the nucleotide region of positions 52803444-52803606) of the nucleotide sequence of ZFYVE9 gene, and the last two nucleotides (GG) at the 3' end of exon 16 may not participate in constituting a codon. For example, genes encoding a fragment of ZFYVE9 protein and fragments of ZFYVE9 protein encoded by the gene are exemplified in Tables 30 and 31:

**[Table 30]**

| Gene encoding a fragment of ZFYVE9 protein | | | | | |
|---|---|---|---|---|---|
| ZFYVE9 gene (Accession No.) | ZFYVE9 protein coding region-CDS | ZFYVE9 protein fragment coding region: based on exon | ZFYVE9 protein fragment coding region: based on cDNA | Position of beak point on chromosome | nucleotide sequence of break-point region |
| NM_007324 | 173∼4273 (4101 bp) (SEQ ID NO: 113) | Region from exon 1 to exon 16 | 173∼3828 (3654bp +2nt(gg); 3656bp in total) (SEQ ID NO:114) | chr1:52803606] (3' end of exon16) | |
| NM_004799 | 173∼4450 (4278bp) | | 173∼4005 (3833bp) | | |

**[Table 31]**

| Fragment ZFYVE9 protein | | | |
|---|---|---|---|
| ZFYVE9gene (Accession No.) | Full size(a.a) of ZFYVE9 protein | ZFYVE9 protein fragment region | Amino acid sequence of breakpoint region |
| NM_007324116) | 1366aa (SEQ ID NO: 116) | aa 1∼1218+2nt(gg) (amino acid sequence: SEQ ID NO: 117) | DKNVSK+2nt(gg) (amino acid sequence: SEQ ID NO: 118) |
| NM_004799 | 1425aa | aa 1∼1277+2nt | |

CGA gene, which encodes CGA (glycoprotein hormones, alpha polypeptide) protein, may be one from human. Human CGA gene positions in human chromosome 6(q14.3), and CGA protein may be one encoded by the CGA gene. CGA protein or a fragment of CGA protein may be a C-terminal fusion partner of ZFYVE9-CGA fusion protein, which is positioned at C-terminal part of the ZFYVE9-CGA fusion protein. For example, CGA gene may comprise the nucleotide sequence of GenBank accession no. NM_000735, and CGA protein may comprise the amino acid sequence encoded by the nucleotide sequence of the CGA gene.

A fragment of CGA protein may comprise an amino acid sequence encoded by a nucleotide sequence from the 2^{nd} exon (exon 2) (based on the position on chromosome 6 ((-) strand), corresponding to the nucleotide region of positions 87797831-87797925) to the last exon of the CGA gene. The first nucleotide (G) of the 5' end of exon 2 may not participate in constituting a codon, and when the CGA protein fragment is fused with ZFYVE9 protein fragment as described above, the first nucleotide (G) of the 5' end of the nucleotide sequence encoding the CGA protein fragment may be linked to the last two nucleotides (GG) of the 3' end of the nucleotide sequence encoding the ZFYVE9 protein fragment to constitute a codon (GGG) thereby encoding an amino acid (G). For example, a gene encoding a fragment of CGA protein and the fragment of CGA protein encoded by the gene are exemplified in Tables 32 and 33:

**[Table 32]**

| Gene encoding a fragment of CGA protein | | | | | |
|---|---|---|---|---|---|
| CGA gene (Accession No.) | CGA protein coding region-CDS | CGA protein fragment coding region: based on exon | CGA protein fragment coding region: based on cDNA | Position of beak point on chromosome | nucleotide sequence of break-point region |
| NM_000735 | 143∼493 (351 bp) (SEQ ID NO: 119) | Region from exon2 to the last exon | 5UTR+143∼449 (358bp) (SEQ ID NO: 120) | Chr6:8779792 5](5' end exon2) | gagcgcc(121) |

**[Table 33]**

| Fragment of CGA protein | | |
|---|---|---|
| Full size(a.a)CGA protein | CGA protein fragment region | Breakpoint region amino acid sequence |
| 116aa (SEQ ID NO: 122) | 116aa (SEQ ID NO: 123) | Breakpoint occurs in UTR |

A fusion gene (ZFYVE9-CGA fusion gene) encoding a ZFYVE9-CGA fusion protein comprising ZFYVE9 protein or a fragment thereof and CGA protein or a fragment thereof, which are fused to each other, may comprise a polynucleotide molecule encoding ZFYVE9 protein or a fragment thereof at 5' terminal part and a polynucleotide molecule encoding CGA protein or a fragment thereof at 3' terminal part of the fusion gene. For example, the ZFYVE9-CGA fusion gene may comprise a nucleotide sequence from the 1^{st} exon (exon 1) to exon 16 of NM_007324 or NM_004799 at 5' terminal part and a nucleotide sequence from exon 2 including 5URT (7bp) to the last exon of NM_000735 at 3' terminal part, wherein the two nucleotide sequences are fused to each other. In a concrete embodiment described herein, the ZFYVE9-CGA fusion gene may a fusion gene (SEQ ID NO: 124; fusion region: SEQ ID NO: 125), wherein the nucleotide sequence (SEQ ID NO: 114) of positions 173 to 3828 of NM_007324 at 5' terminal part and the nucleotide sequence (SEQ ID NO: 120) of positions 136 to 493 of NM_000735 at 3' terminal part are linked to each other.

A ZFYVE9-CGA fusion protein may comprise ZFYVE9 protein or a fragment thereof at N-terminal part and CGA protein or a fragment thereof at C-terminal part of the fusion protein, which are linked to each other. For example, the ZFYVE9-CGA fusion protein may comprise an amino acid sequence encoded by a fusion gene that comprises a nucleotide sequence from the 1^{st} exon (exon 1) to exon 16 of NM_007324 or NM_004799 at 5' terminal part and a nucleotide sequence from exon 2 including 5URT (7bp) to the last exon of NM_000735 at 3' terminal part, wherein the two nucleotide sequences are fused to each other. In a concrete embodiment described herein, the ZFYVE9-CGA fusion protein may be a polypeptide molecule comprising the amino acid sequence (SEQ ID NO: 126; fusion region: SEQ ID NO: 127) that is encoded by the nucleotide sequence of SEQ ID NO: 124, or an amino acid sequence having a sequence homology of at least 90%, at least 95%, or at least 99% to the amino acid sequence of SEQ ID NO: 126.

ERBB2IP gene, which encodes erbb2 interacting protein (ERBB2IP protein), may be one from human. Human ERBB2IP gene positions in human chromosome 5(q12.3), and ERBB2IP protein may be encoded by the ERBB2IP gene. ERBB2IP protein or a fragment thereof may be an N-terminal fusion partner of ERBB2IP-MAST4 fusion protein, which is positioned at N-terminal part of the ERBB2IP-MAST4 fusion protein. For example, ERBB2IP gene may comprise a nucleotide sequence of GenBank accession no. NM_018695 or NM_001006600, and ERBB2IP protein may comprise an amino acid sequence encoded by the nucleotide sequence of the ERBB2IP gene.

A fragment of ERBB2IP protein may comprise an amino acid sequence encoded by a nucleotide sequence from the 1^{st} exon (exon 1) to the 26^{th} exon (exon 26) (based on the position on chromosome 5 ((-) strand), corresponding to the nucleotide region of positions 65372703-65372777) of the nucleotide sequence of ERBB2IP gene. For example, genes encoding a fragment of ERBB2IP protein and fragments of ERBB2IP protein encoded by the gene are exemplified in Tables 34 and 35:

**[Table 34]**

| Gene encoding a fragment of ERBB2IP protein | | | | | |
|---|---|---|---|---|---|
| ERBB2IP gene (Accession No.) | ERBB2IP protein coding region-CDS | ERBB2IP protein fragment coding region: based on exon | ERBB2IP protein fragment coding region: based on cDNA | Position of beak point on chromosome | nucleotide sequence of break-point region |
| NM_001006600 | 311∼4219 (3909bp) (SEQ ID NO: 128) | Region from exon 1 to exon 26 | 311∼4111 (3801bp) (SEQ ID NO: 129) | chr5:65372777] (3' end of exon 26) | |
| NM_018695 | 311∼4426 (4116bp) | | 311∼4318 (4008bp) | | |

**[Table 35]**

| Fragment of ERBB2IP protein | | | |
|---|---|---|---|
| ERBB2IP gene (Accession No.) | ERBB2IP protein 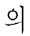 Full size(a.a) | ERBB2IP protein fragment region | Amino acid sequence of breakpoint region |
| NM_001006600 | 1302aa (SEQ ID NO: 131) | aa 1∼1267 (SEQ ID NO: 132) | QPGDKIIQ (SEQ ID NO: 133) |
| NM_018695 | 1371aa | aa 1∼1336 | |

MAST4 gene, which encodes microtubule associated serine/threonine kinase family member4 (MAST4) protein, may be one from human. Human MAST4 gene positions in human chromosome 5(q12.3), and MAST4 protein may be one encoded by the MAST4 gene. MAST4 protein or a fragment of MAST4 protein may be a C-terminal fusion partner of ERBB2IP-MAST4 fusion protein, which is positioned at C-terminal part of the ERBB2IP-MAST4 fusion protein. For example, MAST4 gene may comprise the nucleotide sequence of GenBank accession no. NM_001164664 or NM_015183, and MAST4 protein may comprise the amino acid sequence encoded by the nucleotide sequence of the MAST4 gene.

A fragment of CIT protein may comprise an amino acid sequence encoded by a nucleotide sequence from the 13^{th} exon (exon 13) (based on the position on chromosome 5 ((+) strand), corresponding to the nucleotide region of positions 66400194-66400403) to the last exon of the MAST4 gene. For example, genes encoding a fragment of CIT protein and fragments of CIT protein encoded by the gene are exemplified in Tables 36 and 37:

**[Table 36]**

| Gene encoding a fragment of MAST4 protein | | | | | |
|---|---|---|---|---|---|
| MAST4 gene (Accession No.) | MAST4 protein coding region-CDS | MAST4 protein fragment coding region: based on exon | MAST4 protein fragment coding region: based on cDNA | Position of beak point on chromosome | nucleotide sequence of break-point region |
| NM_001164664 | 309∼8180 (7872bp) (SEQ ID NO: 134) | Region from exon 13 to the last exon | 1455∼8180 (6726bp) (sequence 135) | chr5:[66400194 (5' end of exon 13) | |
| NM_015183 | 69∼7373bp (7065bp) | | 648∼7373 (6726bp) | | |

**[Table 37]**

| Fragment of MAST4 protein | | | |
|---|---|---|---|
| MAST4 gene (Accession No.) | Full size(a.a) of MAST4 protein | MAST4 protein fragment region | Amino acid sequence of breakpoint region |
| NM_001164664 | 2623aa (SEQ ID NO: 137) | aa 383∼2623 (2241aa) (SEQ ID NO: 138) | ATAQMEER (SEQ ID NO: 139) |
| NM_015183 | 2623aa | aa 383∼2623 (2241aa) | |

A fusion gene (ERBB2IP-MAST4 fusion gene) encoding a ERBB2IP-MAST4 fusion protein comprising ERBB2IP protein or a fragment thereof and MAST4 protein or a fragment thereof, which are fused to each other, may comprise a polynucleotide molecule encoding ERBB2IP protein or a fragment thereof at 5' terminal part and a polynucleotide molecule encoding MAST4 protein or a fragment thereof at 3' terminal part of the fusion gene. For example, the ERBB2IP-MAST4 fusion gene may comprise a nucleotide sequence from the 1^{st} exon (exon 1) to exon 26 of NM_018695 or NM_001006600 at 5' terminal part and a nucleotide sequence from exon 13 to the last exon of NM_001164664 or NM_015183 at 3' terminal part, wherein the two nucleotide sequences are fused to each other. In a concrete embodiment described herein, the ERBB2IP-MAST4 fusion gene may a fusion gene (SEQ ID NO: 140; fusion region: SEQ ID NO: 141), wherein the nucleotide sequence (SEQ ID NO: 129) of positions 311 to 4111 of NM_001006600 at 5' terminal part and the nucleotide sequence (SEQ ID NO: 135) of positions 1455 to 8180 of NM_001164664 at 3' terminal part are linked to each other.

An ERBB2IP-MAST4 fusion protein may comprise ERBB2IP protein or a fragment thereof at N-terminal part and MAST4 protein or a fragment thereof at C-terminal part of the fusion protein, which are linked to each other. For example, the ERBB2IP-MAST4 fusion protein may comprise an amino acid sequence encoded by a fusion gene that comprises a nucleotide sequence from the 1^{st} exon (exon 1) to exon 26 of NM_018695 or NM_001006600 at 5' terminal part and a nucleotide sequence from exon 13 to the last exon of NM_001164664 or NM_015183 at 3' terminal part, wherein the two nucleotide sequences are fused to each other. In a concrete embodiment described herein, the ERBB2IP-MAST4 fusion protein may be a polypeptide molecule comprising the amino acid sequence (SEQ ID NO: 142; fusion region: SEQ ID NO: 143) that is encoded by the nucleotide sequence of SEQ ID NO: 140, or an amino acid sequence having a sequence homology of at least 90%, at least 95%, or at least 99% to the amino acid sequence of SEQ ID NO: 142.

TPD52L1 gene, which encodes tumor protein D52-like1 (TPD52L1) protein, may be one from human. Human TPD52L1 gene positions in human chromosome 6(q22.31), and TPD52L1 protein mat be one encoded by the TPD52L1 gene. TPD52L1 protein or a fragment of TPD52L1 protein may be an N-terminal fusion partner of TPD52L1-TRMT11 fusion protein, which is positioned at N-terminal part of the TPD52L1-TRMT11 fusion protein. For example, TPD52L1 gene may comprise the nucleotide sequence of GenBank accession no. NM_003287, NM_001003396, NM_001003397, or NM_001003395, and TPD52L1 protein may comprise the amino acid sequence encoded by the nucleotide sequence of the TPD52L1 gene.

A fragment of TPD52L1 protein may comprise an amino acid sequence encoded by a nucleotide sequence from the 1^{st} exon (exon 1) to the 5^{th} exon (exon 5) (based on the position on chromosome 6 ((+) strand), corresponding to the nucleotide region of positions 125569428-125569529) of the TPD52L gene, and the last two nucleotide (AG) at the 3' end of exon 5 may not participate in constituting a codon. For example, genes encoding a fragment of TPD52L1 protein and fragments of TPD52L1 protein encoded by the gene are exemplified in Tables 38 and 39:

**[Table 38]**

| Gene encoding a fragment of TPD52L1 protein | | | | | |
|---|---|---|---|---|---|
| TPD52L1 gene (Accession No.) | TPD52L1 protein coding region-CDS | TPD52L1 protein fragment coding region: based on exon | TPD52L1 protein fragment coding region: based on cDNA | Position of beak point on chromosome | nucleotide sequence of break-point region |
| NM_001003395 | 328∼855 (528bp) (SEQ ID NO: 144) | Region from exon 1 to exon 5 | 328∼626 (297bp +2nt(c); 299bp in total) (SEQ ID NO: 145) | chr6:125569529] (3' end of exon 5) | |
| NM_001003396 | 220∼654 (435bp) | | 220∼605 (386bp) | | |
| NM_001003397 | 220∼615 (396bp) | | 220∼605 (386bp) | | |
| NM_003287 | 220∼834 (615bp) | | 220∼605 (386bp) | | |

**[Table 39]**

| Fragment of TPD52L1 protein | | | |
|---|---|---|---|
| TPD52L1gene (Accession No.) | Full size(a.a) of TPD52L1 protein | TPD52L1 protein fragment region | Amino acid sequence of breakpoint region |
| NM_001003395 | 175aa (SEQ ID NO: 147) | aa 1∼99+2nt(ag) (amino acid sequence: SEQ ID NO: 148) | SKKFGDM+2nt(ag) (amino acid sequence: SEQ ID NO: 149) |
| NM_001003396 | 144aa | aa 1∼128 | |
| NM_001003397 | 131aa | aa 1∼128 | |
| NM_003287 | 204aa | aa 1∼128 | |

TRMT11 gene, which encodes tRNA methyl transferase11 homolog (TRMT11) protein, may be one from human. Human TRMT11 gene positions in human chromosome 6(q22.32), and TRMT11 protein may be one encoded thereby the TRMT11 gene. TRMT11 protein or a fragment of TRMT11 protein may be a C-terminal fusion partner of TPD52L1-TRMT11 fusion protein, which is positioned at C-terminal part of the TPD52L1-TRMT11 fusion protein. For example, TRMT11 gene may comprise the nucleotide sequence of GenBank accession no. NM_001031712, and TRMT11 protein may comprise the amino acid sequence encoded by the nucleotide sequence of the TRMT11 gene.

A fragment of TRMT11 protein may comprise an amino acid sequence encoded by a nucleotide sequence from the 12^{th} exon (exon 12) (based on the position on chromosome 6 ((+) strand), corresponding to the nucleotide region of positions 126342306-126342426) to the last exon of the TRMT11 gene. The first nucleotide (A) at the 5' end of exon 12 may not participate in constituting a codon, and when the TRMT11 protein fragment is fused with TPD52L1 protein fragment as described above, the first nucleotide (A) of the 5' end of the nucleotide sequence encoding the TRMT11 protein fragment may be linked to the last nucleotide (AG) at the 3' end of the nucleotide sequence encoding the TPD52L1 protein fragment to constitute a codon (AGA) thereby encoding an amino acid (R). For example, a gene encoding a fragment of TRMT11 protein and a fragment of TRMT11 protein encoded by the gene are exemplified in Tables 40 and 41:

**[Table 40]**

| Gene encoding a fragment of TRMT11 protein | | | | | |
|---|---|---|---|---|---|
| TRMT11 gene (Accession No.) | TRMT11 protein coding region-CDS | TRMT11 protein fragment coding region: based on exon | TRMT11 protein fragment coding region: based on cDNA | Position of beak point on chromosome | nucleotide sequence of break-point region |
| NM_001031712 | 122∼1513 (1392bp) (SEQ ID NO: 150) | Region from exon 12 to the last exon | 1261∼1513 (1 nt(a) +252bp; 1877bpin total) (SEQ ID NO: 151) | chr6:[126342306 (5' end of exon 12) | |

**[Table 41]**

| Fragment of TRMT11 protein | | | |
|---|---|---|---|
| TRMT11 gene (Accession No.) | Full size(a.a) of TRMT11 protein | TRMT11 protein fragment region | Amino acid sequence of breakpoint region |
| NM_001031712 | 463aa (SEQ ID NO: 153) | 1nt(a)+ aa 381∼463 (83aa in total) (amino acid sequence: SEQ ID NO: 154) | 1 nt(a)+ YTEEMVP (amino acid sequence: SEQ ID NO: 155) |

A fusion gene (TPD52L1-TRMT11 fusion gene) encoding a TPD52L1-TRMT11 fusion protein comprising TPD52L1 protein or a fragment thereof and TRMT11 protein or a fragment thereof, which are fused to each other, may comprise a polynucleotide molecule encoding TPD52L1 protein or a fragment thereof at 5' terminal part and a polynucleotide molecule encoding TRMT11 protein or a fragment thereof at 3' terminal part of the fusion gene. For example, the TPD52L1-TRMT11 fusion gene may comprise a nucleotide sequence from the 1^{st} exon (exon 1) to exon 5 of NM_003287, NM_001003396, NM_001003397, or NM_001003395 at 5' terminal part and a nucleotide sequence from exon 12 to the last exon of NM_001031712 at 3' terminal part, wherein the two nucleotide sequences are fused to each other. In a concrete embodiment described herein, the TPD52L1-TRMT11 fusion gene may a fusion gene (SEQ ID NO: 156; fusion region: SEQ ID NO: 157), wherein the nucleotide sequence (SEQ ID NO: 145) of positions 328 to 626 of NM_001003395 at 5' terminal part and the nucleotide sequence (SEQ ID NO: 151) of positions 1261 to 1513 of NM_001031712 at 3' terminal part are linked to each other.

A TPD52L1-TRMT11 fusion protein may comprise TPD52L1 protein or a fragment thereof at N-terminal part and TRMT11 protein or a fragment thereof at C-terminal part of the fusion protein, which are linked to each other. For example, the TPD52L1-TRMT11 fusion protein may comprise an amino acid sequence encoded by a fusion gene that comprises a nucleotide sequence from the 1^{st} exon (exon 1) to exon 5 of NM_003287, NM_001003396, NM_001003397, or NM_001003395 at 5' terminal part and a nucleotide sequence from exon 12 to the last exon of NM_001031712 at 3' terminal part, wherein the two nucleotide sequences are fused to each other. In a concrete embodiment described herein, the TPD52L1-TRMT11 fusion protein may be a polypeptide molecule comprising the amino acid sequence (SEQ ID NO: 158; fusion region: SEQ ID NO: 159) that is encoded by the nucleotide sequence of SEQ ID NO: 156, or an amino acid sequence having a sequence homology of at least 90%, at least 95%, or at least 99% to the amino acid sequence of SEQ ID NO: 158.

TXNRD1 gene, which encodes thioredoxin reductase1 (TXNRD1) protein, may be one from human. Human TXNRD1 gene positions in human chromosome 12(q23.3), and TXNRD1 protein may be one encoded by the TXNRD1 gene. TXNRD1 protein or a fragment thereof may be an N-terminal fusion partner of TXNRD1-GPR133 fusion protein, which is positioned at N-terminal part of the TXNRD1-GPR133 fusion protein. For example, TXNRD1 gene may comprise a nucleotide sequence of GenBank accession no. NM_003330, NM_001093771, NM_182729, NM_182743, or NM_182742, and TXNRD1 protein may comprise an amino acid sequence encoded by the nucleotide sequence of the TXNRD1 gene.

A fragment of TXNRD1 protein may comprise an amino acid sequence encoded by a nucleotide sequence from the 1^{st} exon (exon 1) to the 17^{th} exon (exon 17) (based on the position on chromosome 12 ((+) strand), corresponding to the nucleotide region of positions 104732917-104733051) of the TXNRD1 gene as described above. For example, genes encoding a fragment of TXNRD1 protein and fragments of TXNRD1 protein encoded by the gene are exemplified in Tables 42 and 43:

**[Table 42]**

| Gene encoding a fragment of TXNRD1 protein | | | | | |
|---|---|---|---|---|---|
| TXNRD1 gene (Accession No.) | TXNRD1 protein coding region-CDS | TXNRD1 protein fragment coding region: based on exon | TXNRD1 protein fragment coding region: based on cDNA | Position of beak point on chromosome | nucleotide sequence of break-point region |
| | | | | | |
| NM_003330 | 656∼2311 (1656bp) (SEQ ID NO: 160) | Region from exon 1 to exon 17 | 656∼2242 (1587bp) (SEQ ID NO: 161) | chr12:104733051] (3' end of exon 17) | |
| NM_0010937 71 | 25∼1974 (1950bp) | | 258∼1905 (1881bp) | | |
| NM_182729 | 527∼2074 (1548bp) | | 527∼2005 (1479bp) | | |
| NM_182743 | 465∼1964 (1500bp) | | 465∼1895 (1431 bp) | | |
| NM_182742 | 702∼2201 (1500bp) | | 702∼2132 (1431 bp) | | |

**[Table 43]**

| Fragment of TXNRD1 protein | | | |
|---|---|---|---|
| TXNRD1 gene (Accession No.) | Full size(a.a) of ERBB21P protein | ERBB2IP protein fragment region | Amino acid sequence of breakpoint region |
| NM_003330 | 551aa (SEQ ID NO: 163) | aa 1∼529 (SEQ ID NO: 164) | IHPVCAE (SEQ ID NO: 165) |
| NM_001093771 | 649aa | aa 1∼627 | |
| NM_182729 | 499aa | aa 1∼477 | |
| NM_182743 | 499aa | aa 1∼477 | |
| NM_182742 | 499aa | aa 1∼477 | |

GPR133 gene, which encodes G protein-coupled receptor133 (GPR133) protein, may be one from human. Human GPR133 gene positions in human chromosome 12(q24.33), and GPR133 protein may be encoded by the GPR133 gene. GPR133 protein or a fragment of GPR133 protein may be a C-terminal fusion partner of TXNRD1-GPR133 fusion protein, which is positioned at C-terminal part of the TXNRD1-GPR133 fusion protein. For example, GPR133 gene may comprise the nucleotide sequence of GenBank accession no. NM_198827, and GPR133 protein may comprise the amino acid sequence encoded by the nucleotide sequence of the GPR133 gene.

A fragment of GPR133 protein may comprise an amino acid sequence encoded by a nucleotide sequence from the 14^{th} exon (exon 14) (based on the position on chromosome 12 ((+) strand), corresponding to the nucleotide region of positions 131561346-131561419) to the last exon of the GPR133 gene. For example, genes encoding a fragment of GPR133 protein and fragments of GPR133 protein encoded by the gene are exemplified in Tables 44 and 45:

**[Table 44]**

| Gene encoding a fragment of GPR133protein | | | | | |
|---|---|---|---|---|---|
| GPR133 gene (Accession No.) | GPR133 protein coding region-CDS | GPR133 protein fragment coding region: based on exon | GPR133 protein fragment coding region: based on cDNA | Position of beak point on chromosome | nucleotide sequence of break-point region |
| NM_198827 | 560∼3184 (2625bp) (SEQ ID NO: 166) | Region from exon 14 to the last exon | 2033∼318 4 (11526bp) (sequence 167) | chr12:[13156 1346 (5' end of exon 14) | acacgtaagcagcac (SEQ ID NO: 168) |

**[Table 45]**

| Fragment of GPR133 protein | | | |
|---|---|---|---|
| GPR133 gene (Accession No.) | Full size(a.a) of GPR133 protein | GPR133 protein fragment region | Amino acid sequence of breakpoint region |
| NM_198827 | 874aa (SEQ ID NO: 169) | aa 492∼874 (383aa) (SEQ ID NO: 170) | TRKQHS (SEQ ID NO: 171) |

A fusion gene (TXNRD1-GPR133 fusion gene) encoding a TXNRD1-GPR133 fusion protein comprising TXNRD1 protein or a fragment thereof and GPR133 protein or a fragment thereof, which are fused to each other, may comprise a polynucleotide molecule encoding TXNRD1 protein or a fragment thereof at 5' terminal part and a polynucleotide molecule encoding GPR133 protein or a fragment thereof at 3' terminal part of the fusion gene. For example, the TXNRD1-GPR133 fusion gene may comprise a nucleotide sequence from the 1^{st} exon (exon 1) to exon 17 of NM_003330, NM_001093771, NM_182729, NM_182743, or NM_182742 at 5' terminal part and a nucleotide sequence from exon 14 to the last exon of NM_198827 at 3' terminal part, wherein the two nucleotide sequences are fused to each other. In a concrete embodiment described herein, the TXNRD1-GPR133 fusion gene may a fusion gene (SEQ ID NO: 172; fusion region: SEQ ID NO: 173), wherein the nucleotide sequence (SEQ ID NO: 161) of positions 656 to 2242 of NM_003330 at 5' terminal part and the nucleotide sequence (SEQ ID NO: 167) of positions 2033 to 3184 of NM_198827 at 3' terminal part are linked to each other.

A TXNRD1-GPR133 fusion protein may comprise TXNRD1 protein or a fragment thereof at N-terminal part and GPR133 protein or a fragment thereof at C-terminal part of the fusion protein, which are linked to each other. For example, the TXNRD1-GPR133 fusion protein may comprise an amino acid sequence encoded by a fusion gene that comprises a nucleotide sequence from the 1^{st} exon (exon 1) to exon 17 of NM_003330, NM_001093771, NM_182729, NM_182743, or NM_182742 at 5' terminal part and a nucleotide sequence from exon 14 to the last exon of NM_198827 at 3' terminal part, wherein the two nucleotide sequences are fused to each other. In a concrete embodiment described herein, the TXNRD1-GPR133 fusion protein may be a polypeptide molecule comprising the amino acid sequence (SEQ ID NO: 174; fusion region: SEQ ID NO: 175) that is encoded by the nucleotide sequence of SEQ ID NO: 172, or an amino acid sequence having a sequence homology of at least 90%, at least 95%, or at least 99% to the amino acid sequence of SEQ ID NO: 174.

As used herein, the terms "first exon" and "last exon" may respectively refer to the firstly located exon and the lastly located exon of the nucleotide sequence of given accession number regardless of the number of exon, and the number of exon may be given from the sequence information of NCBI.

In case of a fusion gene comprising a 5UTR region of SCAF11 gene and PDGFRA gene or a fragment of PDGFRA gene, which are linked to each other, it is confirmed that the expression level of the PDGFRA gene or a fragment thereof is considerably increased, and such increase is observed specifically in a cancer patient. Therefore, another embodiment described herein provides a polynucleotide molecule (SCAF11-PDGFRA fusion gene) comprising a 5UTR region of SCAF11 gene and PDGFRA gene or a fragment of PDGFRA gene, which are fused to each other, and a used of the polynucleotide molecule as a marker for diagnosing a cancer.

SCAF11 gene, which encodes SCAF11 (SR-related CTD-associated factor 11) protein, may be from human. Human SCAF11 gene is positioned in human chromosome 12(q12). 5UTR region of SCAF11 gene is a 5' terminal fusion partner of SCAF11-PDGFRA fusion gene, which is positioned at 5' terminal part of SCAF11-PDGFRA fusion gene. For example, SCAF11 gene may comprise a nucleotide sequence of GenBank accession no. NM_004719, and the 5UTR region of SCAF11 gene is a polynucleotide molecule comprising a nucleotide sequence from positions 1 to 266 of NM_004719, which corresponds to exon 1 of NM_004719 (position on chromosome((-) strand)-chr12:46384136-46384401; breakpoint on chromosome-chr12:[46384136; SEQ ID NO: 176; fusion region-SEQ ID NO: 177).

PDGFRA gene, which encodes PDGFRA (platelet-derived growth factor receptor, alpha polypeptide) protein, may be from human. Human PDGFRA gene is positioned in human chromosome 4(q12). PDGFRA gene or a fragment of PDGFRA gene is a 3' fusion partner of SCAF11-PDGFRA fusion gene, which is positioned at 3' terminal part of SCAF11-PDGFRA fusion gene. For example, PDGFRA gene may comprise a nucleotide sequence of GenBank accession no. NM_006206, and a fragment of PDGFRA gene may comprise CDS region of NM_006206 (nucleotide sequence from positions 332 to 3601 of NM_006206; 3270 bp in total), wherein the CDS region may further comprise 5UTR region of 12 bp of NM_006206 (nucleotide sequence from positions 120 to 331 of NM_006206) at 5' end, which is expressed as a gene fragment from exon 2 (position on chromosome ((+) strand)-chr4:55124924-55124984; breakpoint on chromosome-chr12:120180269]) to the last exon of NM_006206. In a concrete embodiment described herein, the fragment of PDGFRA gene may be a polynucleotide molecule comprising the nucleotide sequence of SEQ ID NO: 178 (fusion region-SEQ ID NO: 179).

For example, the SCAF11-PDGFRA fusion gene may comprise the nucleotide sequence of SEQ ID NO: 180 (fusion region: SEQ ID NO: 181).

Unless stated otherwise, all the nucleotide sequences which are determined by sequencing the DNA molecules described herein may be sequenced using any automatic DNA sequencer (e.g., Model 373 provided by Applied Biosystems, Inc.), and all the amino acid sequence of polypeptide molecules encoded by the determined nucleotide sequences may be sequenced using any automatic peptide sequencer. Such nucleotide sequences determined by an automatic approach may include partial errors compared to real exact sequences. For example, the automatically determined nucleotide sequences of DNA molecules may usually have sequence similarity (homology) of at least 90%, at least 95%, at least 99%, or at least 99.9% to real exact sequences of the DNA molecules. The nucleotide sequences may include a nucleotide insertion or deletion compared to real exact sequences, wherein such nucleotide insertion or deletion may cause a frame shift during translation of the nucleotide sequences, thereby leading to encoding amino acid sequences which are different from that encoded by real exact nucleotide sequences.

The presence or expression of the fusion protein and/or fusion gene may be specifically detected in a patient suffering from solid cancer, such as lung cancer, in particular non-small cell lung cancer (NSCLC) such as lung adenocarcinoma, and thus, the fusion protein and/or a fusion gene encoding the fusion protein and/or SCAF11-PDGFRA fusion gene may be used as a marker for diagnosing solid cancer, such as lung cancer, in particular non-small cell lung cancer (NSCLC) such as lung adenocarcinoma.

Further described herein is an embodiment providing a pharmaceutical composition for diagnosing a cancer, the composition comprising a material (molecule) interacting (e.g., binding) with the fusion protein as described above, a fusion gene encoding the fusion protein, SCAF11-PDGFRA fusion gene, and/or transcript (e.g., mRNA) corresponding to the fusion gene.

The material interacting with the fusion protein is used for detecting whether the fusion protein is expressed (or present), and may be selected from the group consisting of an antibody, aptamer and the like, which specially biding to the fusion protein or a fusion region (break point) of the fusion protein.

In addition, the material interacting with a fusion gene encoding the fusion protein, SCAF11-PDGFRA fusion gene, or mRNA corresponding to the fusion gene may be a nucleic acid molecule capable of hybridizing with the fusion gene or mRNA. For example, the nucleic acid molecule may be at least one selected from the group consisting of an antisense oligonucleotide (e.g., siRNA, microRNA, etc.), a probe (e.g., 5 to 100 bp, 5 to 50bp, 5 to 30bp, or 5 to 25bp), an aptamer, and the like, which are capable of hybridizing specifically with the fusion gene, a fusion region of the fusion gene, or mRNA molecule (expression transcript) corresponding to the fusion gene or the fusion region in a biological sample. In another embodiment described herein, the nucleic acid molecule capable of hybridizing with a fusion gene encoding the fusion protein, SCAF11-PDGFRA fusion gene, or mRNA molecule corresponding to the fusion gene may be a pair of primers, each of which is in 20 to 100bp or 25 to 50bp length, and capable of hybridizing with a nucleotide sequence having 20 to 100 or 25 to 50 consecutive nucleotides or a complementary nucleotide sequence thereof, that is adjacent to each of both ends of a polynucleotide fragment having 50 to 250 or 100 to 200 consecutive nucleotides of a fusion gene, the polynucleotide fragment including a fusion region of the fusion gene, thereby being capable of amplifying the polynucleotide fragment. The term "capable of hybridizing with a subject nucleotide sequence or gene" may refer to having sequence complementarity of 100% (i.e., completely complementary), at least 80% (e.g., 80-100%), or at least 90% (e.g., 90-100%) to the subject nucleotide sequence to be detected or mRNA corresponding thereto, thereby specifically binding to the nucleotide sequence or mRNA. For example, pairs of primers capable of hybridizing with each fusion gene are exemplified in Table 46.

**[Table 46]**

| fusion gene | Fusion region | Forward Primer Sequence | Reverse Primer Sequence |
|---|---|---|---|
| CCDC6-ROS1 | SEQ ID NO: 14 | | |
| SCAF11-PDGFRA | SEQ ID NO: 181 | | |
| FGFR2-CIT | SEQ ID NO: 30 | | |
| AXL-MBIP | SEQ ID NO: 46 | | |
| APLP2-TNFSF11 | SEQ ID NO: 62 | | |
| MAP4K3 -PRKCE | SEQ ID NO: 78 | | |
| BCAS3-MAP3K3 | SEQ ID NO: 94 | CATCCCGTCCAGTCTCTGAT (SEQ ID NO: 194) | |
| KRAS-CDH13 | SEQ ID NO: 110 | | |
| ZFYVE9-CGA | SEQ ID NO: 125 | | |
| ERBB2IP-MAST4 | SEQ ID NO: 141 | | |
| TPD52L1-TRMT11 | SEQ ID NO: 157 | | |
| TXNRD1-GPR133 | SEQ ID NO: 173 | | |

In a concrete embodiment described herein, a material interacting with the fusion protein or fusion gene or mRNA may be used together with or by being attached by at least one labelling substance selected from the group consisting of free radicals, radioisotopes, fluorescent dyes, chromogenic substrates, enzymes, bacteriophages, coenzymes, and the like.

Further described herein is another embodiment providing a method of providing information for diagnosing a cancer, the method comprising detecting a fusion protein, a fusion gene encoding the fusion protein, SCAF11-PDGFRA fusion gene, and/or mRNA corresponding to the fusion gene in a biological sample obtained from a subject.

The step of detecting a fusion protein, a fusion gene, and/or mRNA in a biological sample may further comprising i) contacting (treating or adding) a material interacting with at least one selected from the group consisting of the fusion protein, a fusion gene encoding the fusion protein, SCAF11-PDGFRA fusion gene, and mRNA corresponding to the fusion gene to a biological sample obtained from a subject to allow a reaction; and ii) detecting a reaction product obtained from step i). The method may further comprise a step of providing a biological sample, prior to step i), and the step of providing a biological sample may comprise a step of obtained (or separating) a biological sample from a subject. In step i), the interacting material may be at least one selected from the group consisting of compounds, antibodies, and aptamers, which are capable of specifically binding to the entirety or a part (e.g., a fusion region) of the fusion protein, and compounds and nucleic acid molecules (e.g., primers, probes, aptamers, etc.), which are capable of binding to the entirety or a part (e.g., a fusion region) of the fusion gene and/or mRNA. As described above, the interacting material may be used together with or by being attached by at least one labelling substance selected from the group consisting of free radicals, radioisotopes, fluorescent dyes, chromogenic substrates, enzymes, bacteriophages, coenzymes, and the like. In step ii), the reaction product obtained from step i) may be a complex generated by an interaction (binding) between the interacting material and at least on selected from the group consisting of the fusion protein, fusion gene, and mRNA. If the reaction product is detected in the detecting step, it can be determined that the fusion protein, fusion gene, and/or mRNA is present in the biological sample.

In the method of providing information for diagnosing a cancer, if it is determined that the fusion protein, fusion gene, and/or mRNA is present in the biological sample, the subject from whom the biological sample is obtained may be determined as a patient of a cancer (solid cancer), for example non-small cell lung cancer (NSCLC), in particular, lung adenocarcinoma.

The detection of the fusion protein, fusion gene, and/or mRNA may be conducted by any means general used in detecting a protein or gene.

For example, the detection of a fusion protein may be conducted by any general assay detecting an interaction (e.g., formation of a complex) between the fusion protein and a material (e.g., an antibody, an aptamer, or a compound) interacting with the fusion protein using such interacting material (e.g., an antibody, an aptamer, or a compound). The general assay may be selected from the group consisting of immunochromatography, immunohistochemical staining, enzyme linked immunosorbent assay (ELISA), radioimmunoassay (RIA), enzyme immunoassay (EIA), florescence immunoassay (FIA), luminescence immunoassay (LIA), western blotting, FACS, and the like.

For detecting a protein expression, any general immune analysis assay may be conducted. A useful immune analysis assay may be an alloimmune analysis assay or a heteroimmune analysis assay. In alloimmune assay, the immune reaction may often involve an agent specific to a fusion protein to be detected (e.g., a fusion protein specific antibody), a labeled analyte, and/or a biological sample to be analyzed. The signal generated from the label may be directly or indirectly modified with a binding of the antibody to the labeled analyte. Not only detection of the immune reaction but also quantitation of the immune reaction may be performed in a homogenous solution. An immunochemical label useful in this analysis may be at least one selected from the group consisting of free radicals, radioisotopes, fluorescent dyes, chromogenic substrates, enzymes, bacteriophages, coenzymes, and the like.

Antibodies, which are useful in performing methods described herein, may be attached to a solid support (e.g., well, bead, plate, or slide, which is made of a substance such as latex or polystyrene) that is suitable for diagnosing or analyzing, by known methods such as precipitation. Antibodies or other agents binding to a fusion protein may be labelled by a detectable label such as a radioactive substance (e.g., ³⁵S, ¹²⁵I, ¹³¹I, etc.), an enzyme (e.g., horseradish peroxidase, alkaline phosphatase, etc.), and a fluorescent substance (e.g., fluorescein, etc.), by known methods.

A cell-based analysis such as flow cytometry (FC), immunohistochemical (IHC) analysis, or immunofluorescence (IF) analysis may be suitably used in the methods described herein, since such analysis formats can be clinically suitable, allow in vivo detecting an expression of kinase polypeptide in the fusion protein, and avoid a risk of an artificial change in an activity causing operation of cells obtained from a tumor sample, for example. Therefore, in a concrete embodiment, the methods described herein may be performed by using an analysis format such as a cell-based analysis such as flow cytometry (FC); immunohistochemical (IHC) analysis; or immunofluorescence (IF) analysis.

The flow cytometry (FC) may be employed for determining the expression of a kinase polypeptide in the fusion protein in a mammal tumor which is one before, during, or after a treatment of a targeted drug for inhibiting an activity of the kinase polypeptide in the fusion protein. For example, a tumor cell obtained from a bone marrow sample may be analyzed by a flow cytometry for expression and/or activation of the fusion protein as well as makers for identifying cancer cell type.

The immunohistochemical (IHC) staining may be employed for determining the expression and/or activation of a kinase in the fusion protein in a mammal cancer (e.g., a solid cancer such as NSCLC) which is one before, during, or after a treatment of a targeted drug for inhibiting an activity of the kinase in the fusion protein.

The immunofluorescence (IF) analysis may be employed for determining the expression and/or activation of a kinase polypeptide in the fusion protein in a mammal cancer which is one before, during, or after a treatment of a targeted drug for inhibiting an activity of the kinase in the fusion protein.

In addition, other protocols such as enzyme linked immunosorbent assay (ELISA), radioimmunoassay (RIA), fluorescence activated cell sorter (FACS), and the like are known to the relevant field, and methods of diagnosing the altered or abnormal level of expression of the fusion protein may also employed.

AQUA peptides for detecting/quantifying the fusion protein expressed in a biological sample including cells obtained from a tumor may be used and prepared in a standard AQUA analysis. Therefore, in a concrete embodiment, a fusion protein specific reagent may comprise an isotope-labelled phosphopeptide (AQUA peptide) present in a peptide sequence corresponding to a peptide sequence including the fusion protein or fusion region (breakpoint) as described above.

In addition, a detection of the fusion gene and/or mRNA corresponding thereto may be performed by any general detecting method using an interacting material that interacts with the fusion gene or mRNA, for example an nucleic acid molecule (e.g., a probe, an aptamer, or a primer, etc.) capable of hybridizing with the fusion gene or mRNA, and for example, the detection may be performed by polymerase chain reaction (PCR), fluorescent in situ hybridization (FISH), UV spectrometry, chromatography, Warburg-Christian method, or Schmidt-Thannhauser-Schneider method, but not be limited thereto. In a concrete embodiment, the detection of the fusion gene and/or mRNA corresponding thereto may be performed by a combination technique of whole-transcriptome (RNA) or whole-genome (DNA) sequencing through a massively parallel sequencing technique. The interacting material (reagent specific to the gene or mRMA) for use in the detection of nucleic acid molecule may be a siRNA, an oligonucleotide or a DNA probe, which is capable of directly hybridizing with a fused- or truncated-polypeptide expressing transcript in a biological sample and detecting the same.

The subject may be a mammal such as a primate including human, monkey, and the like, a rodent including mouse, rat, and the like, and for example, the subject may be human.

The biological sample may be one selected from a cell (e.g., a lung cell), a tissue (e.g., a lung tissue), body fluid (e.g., blood), and the like. For example, a biological sample may be obtained from a mammal who has a cancer (solid or non-solid cancer) characterized by an expression of the fusion protein. In a particular embodiment, the mammal may be human, for example, human who is a subject of a treatment for a cancer such as NSCLC. The human subject may be a patient who is currently or scheduled to be under a medical treatment using an inhibitor of a kinase in a fusion protein to be detected. For example, the biological sample may be one comprising a cell or a tissue, which is obtained (or separated) from a cancer of mammal, or an extract thereof.

The fusion protein specifically expressed in a cancer patient may be used as a target for cancer treatment.

Therefore, described herein is also another embodiment that provides a pharmaceutical composition for treating and/or preventing a cancer, the composition comprising at least one selected from the group consisting of an inhibitor of the fusion protein, an inhibitor of SCAF11-PDGFRA fusion gene, an inhibitor of a fusion gene encoding the fusion protein, and an inhibitor of mRNA corresponding to the fusion gene, as an active ingredient.

Further described herein is another embodiment providing a method of treating and/or preventing a cancer, the method comprising administering a pharmaceutically effective amount of at least one selected from the group consisting of an inhibitor of the fusion protein, an inhibitor of SCAF11-PDGFRA fusion gene, an inhibitor of a fusion gene encoding the fusion protein, and an inhibitor of mRNA corresponding to the fusion gene, to a subject in need of treating and/or preventing a cancer.

Further described herein is another embodiment providing a use of a composition in treating and/or preventing a cancer, the composition comprising at least one selected from the group consisting of an inhibitor of the fusion protein, an inhibitor of SCAF11-PDGFRA fusion gene, an inhibitor of a fusion gene encoding the fusion protein, and an inhibitor of mRNA corresponding to the fusion gene.

The subject may be a mammal including a primate such as human or monkey, a rodent such as mouse or rat, and the like, and for example, the subject may be human.

The inhibitor may be administered orally or parenterally. The parenteral administration may include intravenous injection, subcutaneous injection, muscular injection, intraperitoneal injection, endothelial administration, local administration, intranasal administration, intrapulmonary administration, and rectal administration.

As used herein, the term "pharmaceutically effective amount" refers to an amount of the active ingredient capable of exhibiting meaningful effect, and may be properly determined in a variety of ways, depending on factors such as formulation methods, administration methods, age of patients, body weight, gender, pathologic conditions, diets, administration time, administration route, excretion speed, reaction sensitivity, and the like.

The inhibitor of the fusion protein may be any material capable of binding to the fusion protein and removing or decreasing a function thereof, and for example, at least one selected from the group consisting of antibodies and aptamers against the fusion protein, general kinase inhibitors (the fusion gene encoding a polypeptide including tyrosine kinase may be CCDC6-ROS1, SCAF11-PDGFRA, FGFR2-CIT, AXL-MBIP, MAP4K3-PRKCE, BCAS3-MAP3K3, or ERBB2IP-MAST4), signal transduction inhibitors, and the like. The inhibitor of a fusion gene encoding the fusion protein or mRNA corresponding thereto may be any material capable of binding the DNA or RNA molecule and preventing its expression into the fusion protein, and for example, at least one selected from the group consisting of siRNAs, shRNAs, micro RNA, aptamers, and the like, which specifically bind to the DNA or RNA molecule.

A cancer, which is applicable to the composition for diagnosing a cancer is a solid cancer, the solid cancer being lung cancer, particularly small cell lung cancer (SCLC) or non-small cell lung cancer (NSCLC), such as lung adenocarcinoma, squamous cell lung carcinoma, or large cell lung carcinoma, and for example, lung adenocarcinoma.

Further described herein is another embodiment that provides a method for screening an anticancer agent using the fusion protein.

For example, the method for screening may comprise:
treating (or contacting) a cell expressing the fusion protein or SCAF11-PDGFRA fusion gene with a candidate compound; and
measuring the expression level of the fusion protein and/or the fusion gene in the cell,
wherein if the expression level of the fusion protein and/or the fusion gene in the cell treated with the candidate compound is decreased compared to that of a cell before or without the treatment of the candidate compound, the candidate compound can be determined as an anticancer agent. The expression level of the fusion protein may be measured by measuring the level (amount or concentration) of fusion protein or a fusion gene encoding the fusion protein or mRNA corresponding to the fusion gene.

The method for screening an anticancer agent may further comprise, prior to the step of treating a candidate compound, a step of measuring the expression level of the fusion protein and/or fusion gene in the cell before the treatment of a candidate compound, wherein if the expression level of the fusion protein and/or the fusion gene in the cell treated with the candidate compound (i.e., after the treatment of the candidate compound) is decreased compared to that of the cell before the treatment of the candidate compound, the candidate compound can be determined as an anticancer agent. Alternatively, the method for screening an anticancer agent may comprise steps of providing cells that expresses the fusion protein and/or the fusion gene; treating (or contacting) a part of the cells with the candidate compound; and measuring the expression level of the fusion protein and/or the fusion gene in the part of the cells treated with the candidate compound and the rest part of the cells that is not treated with the candidate compound, wherein if the expression level of the fusion protein and/or the fusion gene in the part of the cells treated with the candidate compound is decreased compared to the part of the cells that is not treated with the candidate compound, the candidate compound can be determined as an anticancer agent.

The cell used in the method for screening may be a cancer cell wherein at least one of the fusion proteins or fusion genes is expressed and/or activated (e.g., a cancer cell on which the screened anticancer agent is desired to exhibit anticancer effect), or an extract or culture thereof, which is extracted or cultured by any general method.

The cell expressing the fusion protein and/or the fusion gene may be a cancer cell as described above, and in particular, a solid cancer cell, for example, a lung cancer cell such as a lung adenocarcinoma cell.

The measurement of the expression level of the fusion protein may be performed by any general protein assay. For example, the assay may be selected from immunochromatography), immunohistochemical staining, enzyme linked immunosorbent assay (ELISA), radioimmunoassay (RIA), enzyme immunoassay (EIA), fluorescence immunoassay (FIA), luminescence immunoassay (LIA), western blotting, FACS, and the like, but not be limited thereto. The level of the fusion gene or mRNA may be measured by any general gene quantifying assay. For example, the assay may be selected from PCR, FISH, UV spectroscopy, chromatography, Warburg-Christian method, Schmidt-Thannhauser-Schneider method, and the like, but not be limited thereto.

The candidate compound may be any natural or artificially synthetic compound. For example, the candidate compound may be at least one selected from the group consisting of small molecular compound, DNA, RNA, protein, and the like.

For determining whether or not the candidate compound inhibits progress of a cancer characterized by the fusion protein, a biological sample may be one comprising cells obtained from a mammal xenograft model. The xenograft may be a human cancer cell expressing the fusion protein, and a recipient of the xenograft may be a small mammal such as a mouse. In determining the expression or present of the fusion protein in a biological sample comprising cells obtained from mammal cancer (tumor), a cell in which the fusion protein is not expressed may be used as a control sample. The control sample may comprise a normal cell of a tumor occurring tissue, in which the fusion protein is not expressed (e.g., a normal cell around tumor) or a cancer cell from a cancer that does not expressing the fusion protein.

The cancer to be treated with the anticancer agent developed by the method for screening is as described above.

### [Advantageous Effects]

The fusion protein according to the present invention and/or DNA or mRNA molecule encoding the fusion protein as described herein, which are expressed specifically in lung cancer such as lung adenocarcinoma, may be useful as not only a marker for diagnosing a lung cancer but also a target marker for treating lung cancer.

### [Mode for Invention]

Hereafter, the present invention will be described in detail by examples.

The following examples are intended merely to illustrate the invention and are not construed to restrict the invention.

### Example 1: Preparation of cancer samples

200 surgical specimens of primary lung adenocarcinoma were collected from patients who underwent lobectomy at Seoul National University Hospital (n=164; from 2010 to September 2011) and Seoul St. Mary's Hospital (n=36; samples deposited in its tissue bank from 2009 to 2011). Twenty patients from inventor's previous report (Ju YS et al. Genome Res. 2012 22:436-445) were included in this cohort. For each patient, diagnosis, gender, cancer stage and smoking status were recorded. Among the 200 cancer patients, the proportions of females and never-smokers were 54.5% (n=109) and 58.0% (n=116) respectively.

Using method previously reported (Ju YS et al. Genome Res. 2012 22:436-445), a screening genetic tests was performed for three well-known driver mutations in a subset of the 200 lung adenocarcinoma tissues (exon 18-21 of *EGFR* was tested by PCR and Sanger sequencing (n=164); exon 2 of *KRAS* was tested by PCR and Sanger sequencing (n=37); *EML4-ALK* fusion genes was by fluorescent in-situ hybridization⁹ (FISH; n=163)). Of the 200 cancer tissues, 110 tissues were positive for driver mutations in one of *EGFR* (n=99), *KRAS* (n=6) and *EML4-ALK* (n=7). These mutations were virtually exclusive with each other, except for two samples (1 *EGFR*⁺*KRAS*⁺ and 1 *EGFR*⁺*EML4-ALK*⁺)*.* Driver mutations in the remaining 90 samples were unknown.

These 90 samples were targeted for RNA sequencing. Excluding 3 samples which did not pass the RNA quality control, mRNA sequences were obtained from 87 lung adenocarcinomas. Thereafter, transcriptome (n=77) and whole-exome (n=76) sequencing of adjacent normal lung tissues was performed for comparison between cancer and normal tissues. All these sequencing experiments were done as previously described in "Ju YS et al. Genome Res. 2012 22:436-445".

14,038,673,860 paired-end 101 bp-long reads were generated from RNA sequencing of 164 samples (87 cancer and 77 corresponding normal tissues). On average, the RNA sequencing throughputs were 9.77 and 7.38 Gbp for cancer and normal tissues respectively. In the whole-exome sequencing of 76 normal tissues, 32.96 x read depth was obtained per paired-normal tissue for on-target regions.

### Example 2: Fusion gene analysis

Thereafter, detecting fusion genes was focused since several transforming fusion genes were recently detected in lung adenocarcinoma as driver mutations. Using the gene fusion program (GFP) described in "Ju YS et al. Genome Res. 2012 22:436-445", 45 in-frame fusion transcripts were identified from the 87 cancer tissues.

For detecting a fusion gene using transcriptome sequencing, the sequences of 101 bp fragment of both end of cDNA fragment of 300bp was identified using next generation sequencing (Ju YS et al. Genome Res. 2012 22:436-445), and then, discordant sequences (discordant reads) having both ends consists of different gene sequences from each other were searched. In addition, exon-spanning sequences (exon-spanning reads), wherein one end sequence is generated from breakpoint of fusion gene, whereby the exon-spanning sequence comprises combination of two different gene sequences from each other, were searched. All the discordant sequences and exon-spanning sequences suggest the presence of fusion gene. Gene pairs comprising both of discordant sequence and exon-spanning sequence were chosen as final candidate of fusion gene, and among them, in-frame fusion genes, in which original codon frame of gene based on original amino acid sequence is maintained, were finally selected as fusion genes for lung cancer.

Genes fused in the above 45 in-frame fusion transcripts, chromosomes where each gene locates, and distances between the genes are summarized in Table 47:

**[Table 47]**

| Index | Donor Gene | Acceptor Gene | Chromosome (Donor; Acceptor) | Distance (Mb) |
|---|---|---|---|---|
| 1 | *EML4* | *ALK* | chr2;chr2 | 12.252 |
| 2 | *KIF5B* | *RET* | chr10;chr10 | 11.227 |
| 2 | *KIF5B* | *RET* | chr10;chr10 | 11.227 |
| 2 | *KIF5B* | *RET* | chr10;chr10 | 11.227 |
| 2 | *KIF5B* | *RET* | chr10;chr10 | 11.227 |
| 3 | *CD74* | *ROS1* | chr5;chr6 | Interchromosomal |
| 4 | *SLC34A2* | *ROS1* | chr4;chr6 | Interchromosomal |
| 5 | *CCDC6* | *ROS1* | chr10;chr6 | Interchromosomal |
| 6 | *SCAF11* | *PDGFRA* | chr12;chr4 | Interchromosomal |
| 7 | *FGFR2* | *C*/*T* | chr10;chr12 | Interchromosomal |
| 8 | *AXL* | *MBIP* | chr19;chr14 | Interchromosomal |
| 9 | *APLP2* | *TNFSF11* | chr11;chr13 | Interchromosomal |
| 10 | *MAP4K3* | *PRKCE* | chr2;chr2 | 6.215 |
| 11 | *BCAS3* | *MAP3K3* | chr17;chr17 | 2.23 |
| 12 | *KRAS* | *CDH13* | chr12;chr16 | Interchromosomal |
| 13 | *ZFYVE9* | *CGA* | chr1;chr6 | Interchromosomal |
| 14 | *ERBB2IP* | *MAST4* | chr5;chr5 | 0.515 |
| 15 | *TPD52L1* | *TRMT11* | chr6;chr6 | 0.723 |
| 16 | *TXNRD1* | *GPR133* | chr12;chr12 | 26.694 |
| 17 | *SRSF4* | *SNRNP40* | chr1;chr1 | 2.224 |
| 18 | *EDA* | *MID1* | chrX;chrX | 57.984 |
| 19 | *HYOU1* | *C11orf93* | chr11;chr11 | 7.736 |
| 20 | *SLC16A7* | *MUCL1* | chr12;chr12 | 4.831 |
| 21 | *MIER2* | *ITGB1BP3* | chr19;chr19 | 3.588 |
| 22 | *RBM14* | *FGF3* | chr11;chr11 | 3.211 |
| 23 | *UBR4* | *ATP13A2* | chr1;chr1 | 2.063 |
| 24 | *TTC19* | *ATPAF2* | chr17;chr17 | 1.989 |
| 25 | *IGSF3* | *MAN1A2* | chr1;chr1 | 0.7 |
| 26 | *XAF1* | *FAM64A* | chr17;chr17 | 0.305 |
| 27 | *IL6ST* | *KDM1B* | chr5;chr6 | Interchromosomal |
| 28 | *UBE2E1* | *ASCC3* | chr3;chr6 | Interchromosomal |
| 29 | *XRCC1* | *MAL* | chr19;chr2 | Interchromosomal |
| 30 | *BRWD1* | *CCDC46* | chr21;chr17 | Interchromosomal |
| 31 | *SPTLC3* | *MAOA* | chr20;chrX | Interchromosomal |
| 32 | *UTRN* | *OS9* | chr6;chr12 | Interchromosomal |
| 33 | *LOC100306951* | *NUP93* | chr17;chr16 | Interchromosomal |
| 34 | *MGAT5* | *HNMT* | chr2;chr2 | 3.515 |
| 35 | *MAP3K3* | *PECAM1* | chr17;chr17 | 0.623 |
| 36 | *CMBL* | *C8orf38* | chr5;chr8 | Interchromosomal |
| 37 | *ITGB1BP3* | *DNM2* | chr19;chr19 | 6.886 |
| 38 | *LSM14A* | *SIPA1L3* | chr19;chr19 | 3.677 |
| 39 | *RAB21* | *FRS2* | chr12;chr12 | 2.175 |
| 40 | *ARHGEF16* | *TCTEX1D4* | chr1;chr1 | 41.874 |
| 41 | *MMP14* | *H19* | chr14;chr11 | Interchromosomal |
| 42 | *H19* | *CALR* | chr11;chr19 | Interchromosomal |
| 43 | *SFTPB* | *DPYSL2* | chr2;chr8 | Interchromosomal |
| 44 | *SFTPA2* | *SFTPB* | chr10;chr2 | Interchromosomal |
| 45 | *FTL* | *SFTPA2* | chr19;chr10 | Interchromosomal |

Breakpoints in donor regions of the above 45 fusion transcripts (3'-terminal breakpoints based on transcripts), amino acid sequences near breakpoints, and exons where breakpoints are positioned were summarized according to each gene (including mutants) in Table 48:

**[Table 48]**

| Index | Donor Breakpoint (RNA) | Donor protein sequence near breakpoint | Donor exon number |
|---|---|---|---|
| 1 | chr2:42522656] | TPGKGPK+1nt | EML4(NM_001145076,+strand),exon12(chr2:42522521-42522656;EML4(NM_019063,+strand),exon 13(chr2:42522521-42522656; |
| 2 | chr10:[32317356 | NNDVK | KIF5B(NM_004521,-strand),exon15(chr10:32317356-32317499; |
| 2 | chr10:[32306980 | KVHKQ | KIF5B(NM_004521,-strand),exon23(chr10:32306980-32307084; |
| 2 | chr10:[32317356 | NNDVK | KIF5B(NM_004521,-strand),exon15(chr10:32317356-32317499; |
| 2 | chr10:[32317356 | NNDVK | KIF5B(NM_004521,-strand),exon15(chr10:32317356-32317499; |
| 3 | chr5:[149784243 | DAPPK+1nt | CD74(NM_004355,-strand),exon6(chr5:149784243-149784330;CD74(NM_001025159,-strand),exon6(chr5:149784243-149784330; |
| 4 | chr4:25678324] | SREAQ+1nt | SLC34A2(NM_006424,+strand),exon13(chr 4:25677757-25680366;SLC34A2(NM_001177998,+stran d),exon13(chr4:25677757-25680366;SLC34A2(NM_001177999,+stran d),exon13(chr4:25677757-25680366; |
| 5 | chr10:[61572393 | AAQLQ+1nt | CCDC6(NM_005436,-strand),exon5(chr10:61572393-61572553; |
| 6 | chr12:[46384136 | 5UTR | SRSF2IP(NM_004719,-strand),exon1(chr12:46384136-46384401; |
| 7 | chr10:[123243212 | LTLTTNE | FGFR2(NM_001144914,-strand),exon14(chr10:123243212-123243317;FGFR2(NM_001144916,-strand),exon14(chr10:123243212-123243317;FGFR2(NM_001144915,-strand),exon16(chr10:123243212-123243317;FGFR2(NM_001144917,-strand),exon15(chr10:123243212-123243317;FGFR2(NM_001144918,-strand),exon15(chr10:123243212-123243317;FGFR2(NM_022970,-strand),exon17(chr10:123243212-123243317;FGFR2(NM_000141,-strand),exon17(chr10:123243212-123243317;FGFR2(NM_001144913,-strand),exon16(chr10:123243212-123243317;FGFR2(NM_001144919,-strand),exon16(chr10:123243212-123243317; |
| 8 | chr19:41765701] | LTAAE | AXL(NM_021913,+strand),exon20(chr19:41765458-41767670;AXL(NM_001699,+strand),exon19(chr19:41765458-41767670; |
| 9 | chr11:130000061] | AAQMKSQ | APLP2(NM_001642,+strand),exon11(chr11: 129999933-130000061;APLP2(NM_001142276,+strand) ,exon11(chr11:129999933-130000061;APLP2(NM_001142278,+strand) ,exon7(chr11:129999933-130000061;APLP2(NM_001142277,+strand) ,exon10(chr11:129999933-130000061;APLP2(NR_024516,+strand),ex on8(chr11:129999933-130000061;APLP2(NR_024515,+strand),ex on8(chr11:129999933-130000061; |
| 10 | chr2:[39664033 | TYGDVYK | MAP4K3(NM_003618,-strand),exon1(chr2:39664033-39664219; |
| 11 | chr17:59161925] | TVIDAAS+1 nt | BCAS3(NM_017679,+strand),exon22(chr17:59161828-59161925;BCAS3(NM_001099432,+strand), exon23(chr17:59161828-59161925; |
| 12 | chr12:[25378548 | TSAKTRQ | KRAS(NM_004985,-strand),exon4(chr12:25378548-25378707;KRAS(NM_033360,-strand),exon4(chr12:25378548-25378707; |
| 13 | chr1:52803606] | DKNVSK+2 nt | ZFYVE9(NM_007324,+strand),exon15(chr1: 52803444-52803606;ZFYVE9(NM_004799,+strand),ex on16(chr1:52803444-52803606; |
| 14 | chr5:65372777] | QPGDKIIQ | ERBB2IP(NM_018695,+strand),exon24(chr5:65372703-65372777;ERBB2IP(NM_001006600,+stran d),exon23(chr5:65372703-65372777; |
| 15 | chr6:125569529] | SKKFGDM+ 2nt | TPD52L1(NM_003287,+strand),exon4(chr6: 125569428-125569529;TPD52L1(NM_001003396,+stra nd),exon4(chr6:125569428-125569529;TPD52L1(NM_001003397,+stra nd),exon4(chr6:125569428-125569529;TPD52L1(NM_001003395,+stra nd),exon4(chr6:125569428-125569529; |
| 16 | chr12:104733051] | IHPVCAE | TXNRD1(NM_001093771,+strand),exon16(chr12:104732917-104733051;TXNRD1(NM_003330,+strand), exon14(chr12:104732917-104733051;TXNRD1(NM_182729,+strand), exon14(chr12:104732917-104733051;TXNRD1(NM_182743,+strand), exon13(chr12:104732917-104733051;TXNRD1(NM_182742,+strand), exon13(chr12:104732917-104733051; |
| 17 | chr1:[29485886 | SRCSWQD LK | SRSF4(NM_005626,-strand),exon3(chr1:29485886-29485998; |
| 18 | chrX:68836548] | DSQDGHQ | EDA(NM_001005610,+strand),exon1(chrX:6 8835911-68836548;EDA(NM_001005613,+strand),ex on1(chrX:68835911-68836548;EDA(NM_001399,+strand),exon1 (chrX:68835911-68836548;EDA(NM_001005609,+strand),ex on1(chrX:68835911-68836548;EDA(NM_001005612,+strand),ex on1(chrX:68835911-68836548; |
| 19 | chr11:[118921747 | SGVLSLDR | HYOU1(NM_006389,-strand),exon14(chr11:118921747-118921885;HYOU1(NM_001130991,-strand),exon14(chr11:118921747-118921885; |
| 20 | chr12:60098799] | LAVMYAG+ 1 nt | SLC16A7(NM_004731,+strand),exon2(chr12:60098553-60098799; |
| 21 | chr19:[325635 | LNRHCEK+ 1 nt | MIER2(NM_017550,-strand),exon7(chr19:325635-325704; |
| 22 | chr11:66384528] | IECDVVK+1 nt | RBM14(NM_006328,+strand),exon1(chr11:66384053-66384528; |
| 23 | chr1:[19523635 | LSCLYA+1nt | UBR4(NM_020765,-strand),exon8(chr1:19523635-19523759; |
| 24 | chr17:15930016] | AAVLMHR+ 1 nt | TTC19(NM_017775,+strand),exon9(chr17:15929854-15930016; |
| 25 | chr1:117156387] | VVNVQPT+ 1 nt | IGSF3(NM_001542,-strand),exon4(chr1:117156387-117156797;IGSF3(NM_001007237,-strand),exon4(chr1:117156387-117156797; |
| 26 | chr17:6663920] | EQAQLGK+ 1 nt | XAF1(NM_017523,+strand),exon4(chr17:66 63725-6663920;XAF1(NM_199139,+strand),exon3(chr17:6663725-6663920; |
| 27 | chr5:[55290612 | 5UTR | IL6ST(NM_175767,-strand),exon1(chr5:55290612-55290821;IL6ST(NM_002184,-strand),exon1(chr5:55290612-55290821;IL6ST(NM_001190981,-strand),exon1(chr5:55290612-55290821; |
| 28 | chr3:23847579] | 5UTR | UBE2E1(NM_003341,+strand),exon1(chr3:2 3847439-23847579;UBE2E1 (NM_182666,+strand),ex on1(chr3:23847439-23847579; |
| 29 | chr19:[44079062 | TISVVLQ | XRCC1(NM_006297,-strand),exon2(chr19:44079062-44079154; |
| 30 | chr21:[40604103 | WRKMDLR | BRWD1(NM_018963,-strand),exon25(chr21:40604103-40604210;BRWD1(NM_033656,-strand),exon25(chr21:40604103-40604210; |
| 31 | chr20:13074224] | IRIFKHN+1 nt | SPTLC3(NM_018327,+strand),exon6(chr20: 13074131-13074224; |
| 32 | chr6:144820563 ] | LDTEISWA K | UTRN(NM_007124,+strand),exon33(chr6:14 4820393-144820563; |
| 33 | chr17:1420387] | 5UTR | LOC100306951(NR_028514,+strand),exon1 (chr17:1420213-1420387; |
| 34 | chr2:135028121] | LEKINVA+1 nt | MGAT5(NM_002410,+strand),exon2(chr2:13 5027957-135028121; |
| 35 | chr17:61723434] | EHNGER+2 nt | MAP3K3(NM_002401,+strand),exon3(chr17:61723394-61723434;MAP3K3(NM_203351,+strand),ex on4(chr17:61723394-61723434; |
| 36 | chr5:[10307737 | 5UTR | CMBL(NM_138809,-strand),exon1(chr5:10307737-10308168; |
| 37 | chr19:3942267] | ASQQDS+2 nt | ITGB1BP3(NM_170678,+strand),exon8(chr19:3942081-3942412; |
| 38 | chr19:34663668] | NSTVALAK +1 nt | LSM14A(NM_015578,+strand),exon1(chr19:34663352-34663668;LSM14A(NM_001114093,+strand) ,exon1(chr19:34663352-34663668; |
| 39 | chr12:72176438] | LFLDLCK+1 nt | RAB21(NM_014999,+strand),exon6(chr12:72176350-72176438; |
| 40 | chr1:3392626] | QLDFSKVK | ARHGEF16(NM_014448,+strand),exon10(chr1:3392534-3392626; |
| 41 | chr14:23316426] | 3UTR | MMP14(NM_004995,+strand),exon10(chr14:23314917-23316802; |
| 42 | chr11:[2018179 | noncoding | H19(NR_002196,-strand),exon1(chr11:2017748-2019065; |
| 43 | chr2:[85885042 | 3UTR | SFTPB(NM_000542,-strand),exon12(chr2:85884441-85886805;SFTPB(NM_198843,-strand),exon12(chr2:85884441-85885978; |
| 44 | chr10:[81319068 | GDPGPP+1 nt | SFTPA2(NM_001098668,-strand),exon3(chr10:81319068-81319262; |
| 45 | chr19:49468806] | NYSTDVE | FTL(NM_000146,+strand),exon1(chr19:49468566-49468866; |

Breakpoints in acceptor regions of the above 45 fusion transcripts (5'-terminal breakpoints based on transcripts), amino acid sequences near breakpoints, and exons where breakpoints are positioned were summarized according to each gene (including mutants) in Table 49:

**[Table 49]**

| Index | Acceptor Breakpoint (RNA) | Acceptor protein sequence near breakpoint | Acceptor exon number |
|---|---|---|---|
| 1 | chr2:29446394] | 2nt+YRRKH QE | ALK(NM_004304,-strand),exon20(chr2:29446208-29446394; |
| 2 | chr10:[43612032 | EDPKWEF | RET(NM_020630,+strand),exon12(chr10:43 612032-43612179;RET(NM_020975,+strand),exon12(chr10:43612032-43612179; |
| 2 | chr10:[43612032 | EDPKWEF | RET(NM_020630,+strand),exon12(chr10:43 612032-43612179;RET(NM_020975,+strand),exon12(chr10:43612032-43612179; |
| 2 | chr10:[43612032 | EDPKWEF | RET(NM_020630,+strand),exon12(chr10:43 612032-43612179;RET(NM_020975,+strand),exon12(chr10:43612032-43612179; |
| 2 | chr10:[43612032 | EDPKWEF | RET(NM_020630,+strand),exon12(chr10:43 612032-43612179;RET(NM_020975,+strand),exon12(chr10:43612032-43612179; |
| 3 | chr6:117645578] | 2nt+DFWIP | ROS1(NM_002944,-strand),exon34(chr6:117645495-117645578; |
| 4 | chr6:117650609] | 2nt+GVPNK | ROS1(NM_002944,-strand),exon32(chr6:117650492-117650609; |
| 5 | chr6:117642557] | 2nt+WHRRL | ROS1(NM_002944,-strand),exon35(chr6:117642422-117642557; |
| 6 | chr4:[55124924 | 5UTR, in-frame | PDGFRA(NM_006206,+strand),exon2(chr4: 55124924-55124984; |
| 7 | chr12:120180269] | AHRDEIQ | CIT(NM_007174,-strand),exon23(chr12:120180216-120180269; |
| 8 | chr14:36783814] | IDRRI | MBIP(NM_016586,-strand),exon4(chr14:36783718-36783814;MBIP(NM_001144891,-strand),exon4(chr14:36783718-36783814; |
| 9 | chr13:[43174888 | ELQHIVG | TNFSF11(NM_033012,+strand),exon5(chr13:43174888-43174933;TNFSF11(NM_003701,+strand),e xon3(chr13:43174888-43174933; |
| 10 | chr2:[46070139 | IDLEPEGR | PRKCE(NM_005400,+strand),exon2(chr2:46070139-46070202; |
| 11 | chr17:61710041] | 2nt+EQEAL NS | MAP3K3(NM_002401,+strand),exon2(chr17:61710041-61710162;MAP3K3(NM_203351,+strand),ex on2(chr17:61710041-61710162; |
| 12 | chr16:[83158990 | DIFKFAR | CDH13(NM_001257,+strand),exon4(chr16:8 3158990-83159106; |
| 13 | chr6:87797925] | 5UTR, in-frame | CGA(NM_000735,-strand),exon2(chr6:87797831-87797925; |
| 14 | chr5:[66400194 | ATAQMEER | MAST4(NM_001164664,+strand),exon10(ch r5:66400194-66400403;MAST4(NM_015183,+strand),exo n9(chr5:66400194-66400403; |
| 15 | chr6:[126342306 | 1nt+YTEEM VP | TRMT11(NM_001031712,+strand),exon12(chr6:126342306-126342426; |
| 16 | chr12:[131561346 | TRKQHS | GPR133(NM_198827,+strand),exon14(chr12:131561346-131561419; |
| 17 | chr1:31744346] | VWDLRQN | SNRNP40(NM_004814,-strand),exon6(chr1:31744226-31744346; |
| 18 | chrX:10463731] | VNASRQE | MID1(NM_001193277,-strand),exon4(chrX:10463624-10463731;MID1(NM_000381,-strand),exon4(chrX:10463624-10463731;MID1(NM_033289,-strand),exon4(chrX:10463624-10463731;MID1(NM_001098624,-strand),exon4(chrX:10463624-10463731;MID1(NM_033290,-strand),exon4(chrX:10463624-10463731;MID1(NM_001193278,-strand),exon4(chrX:10463624-10463731;MID1(NM_001193279,-strand),exon3(chrX:10463624-10463731;MID1(NM_001193280,-strand),exon3(chrX:10463624-10463731; |
| 19 | chr11:[111175653 | 5 UTR | C11orf93(NM_001136105,+strand),exon3(ch r11:111175653-111175707; |
| 20 | chr12:[55248900 | 2nt+NPTTA APAD | MUCL1(NM_058173,+strand),exon2(chr12:5 5248900-55248941; |
| 21 | chr19:[3942081 | 2nt+YLDGM KS | ITGB1BP3(NM_170678,+strand),exon8(chr1 9:3942081-3942412; |
| 22 | chr11:69631191] | 2nt+ILEITAV | FGF3(NM_005247,-strand),exon2(chr11:69631088-69631191; |
| 23 | chr1:17332273] | 2nt+SSPLV G | ATP13A2(NM_001141973,-strand),exon2(chr1:17332179-17332273;ATP13A2(NM_022089,-strand),exon2(chr1:17332179-17332273;ATP13A2(NM_001141974,-strand),exon2(chr1:17332179-17332273; |
| 24 | chr17:17931973] | 2nt+RKRFY QN | ATPAF2(NM_145691,-strand),exon2(chr17:17931929-17931973; |
| 25 | chr1:[118035769 | 2nt+HTSVG GLGD | MAN1A2(NM_006699,+strand),exon9(chr1:118035769-118035884; |
| 26 | chr17:[6348396 | 5UTR, in-frame | FAM64A(NM_001195228,+strand),exon2(chr17:6348396-6348724;FAM64A(NM_019013,+strand),exo n2(chr17:6348396-6348724; |
| 27 | chr6:[18215238 | KKHSVLM | KDM1B(NM_153042,+strand),exon16(chr6:18215238-18215360; |
| 28 | chr6:100966018] | AMLDVAAN | ASCC3(NM_006828,-strand),exon38(chr6:100965867-100966018; |
| 29 | chr2:[95713704 | IFGGLVW | MAL(NM_022438,+strand),exon2(chr2:9571 3704-95713871;MAL(NM_002371,+strand),exon2 (chr2:95713704-95713871; |
| 30 | chr17:63685336] | VLQDELE | CCDC46(NM_001037325,-strand),exon4(chr17:63685247-63685336;CCDC46(NM_145036,-strand),exon24(chr17:63685247-63685336; |
| 31 | chrX:[43542761 | 2nt+LSAAK LL | MAOA(NM_000240,+strand),exon2(chrX:43542761-43542855; |
| 32 | chr12:[58109543 | FLCDEGA | OS9(NM_006812,+strand),exon6(chr12:58109543-58109753;OS9(NM_001017956,+strand),ex on6(chr12:58109543-58109753;OS9(NM_001017957,+strand),ex on6(chr12:58109543-58109753;OS9(NM_001017958,+strand),ex on6(chr12:58109543-58109753; |
| 33 | chr16:[56870513 | PGVIDKF | NUP93(NM_014669,+strand),exon17(chr16:56870513-56870629; |
| 34 | chr2:[138758488 | 2nt+EIDLQI L | HNMT(NM_006895,+strand),exon3(chr2:138758488-138758595; |
| 35 | chr17:62401205] | unidentified | PECAM1(NM_000442,-strand),exon1(chr17:62399864-62401205; |
| 36 | chr8:[96044223 | 5UTR | C8orf38(NM_152416,+strand),exon2(chr8:96044223-96044322; |
| 37 | chr19:[10870414 | 1nt+DFLPR GS | DNM2(NM_001190716,+strand),exon2(chr1 9:10870414-10870487;DNM2(NM_004945,+strand),exon 2(chr19:10870414-10870487;DNM2(NM_001005361,+strand),e xon2(chr19:10870414-10870487;DNM2(NM_001005362,+strand),e xon2(chr19:10870414-10870487;DNM2(NM_001005360,+strand),e xon2(chr19:10870414-10870487; |
| 38 | chr19:[38519729 | 5UTR | SIPA1L3(NM_015073,+strand),exon2(chr19: 38519729-38519796; |
| 39 | chr12:[69924645 | 5UTR | FRS2(NM_006654,+strand),exon2(chr12:69 924645-69924740;FRS2(NM_001042555,+strand),e xon3(chr12:69924645-69924740; |
| 40 | chr1:45272510] | 5UTR | TCTEX1D4(NM_001013632,-strand),exon1(chr1:45272456-45272957; |
| 41 | chr11:2018689] | noncoding | H19(NR_002196,-strand),exon1(chr11:2017748-2019065; |
| 42 | chr19:[13054527 | AAEKQMK | CALR(NM_004343,+strand),exon9(chr19:13054527-13055304; |
| 43 | chr8:[26501052 | 1nt+SPPLS PD | DPYSL2(NM_001386,+strand),exon9(chr8:26500955-26501111; |
| 44 | chr2:85885494] | 3UTR | SFTPB(NM_000542,-strand),exon12(chr2:85884441-85886805;SFTPB(NM_198843,-strand),exon12(chr2:85884441-85885978; |
| 45 | chr10:81316285] | 3UTR | SFTPA2(NM_001098668,-strand),exon6(chr10:81315609-81317341; |

Among them, 22 fusion genes (48.9%) were generated by intra-chromosomal fusions. Using PCR amplification and Sanger sequencing of cDNAs, 30 fusion gens were selected and out of them, 29 fusion genes (numbers 1-29) were validated (see Table 50).

**[Table 50]**

| | Donor Gene | Acceptor Gene | Forward Primer Name | Forward Primer Sequence | Reverse Primer Name | Reverse Primer Sequence |
|---|---|---|---|---|---|---|
| 1 | KIF5B | RET | GF1_KIF5B:RET_F | | GF1_KIF5B:RET_ R | |
| 2 | KRAS | CDH13 | GF2_KRAS:CDH13_F | | GF2_KRAS:CDH13_R | |
| 3 | APLP2 | TNFSF11 | GF3_APL | | GF3_APL | |
| | | | P2:TNFSF11_F | | P2:TNFSF11_R | |
| 4 | ZFYVE 9 | CGA | GF4_ZFYVE9:CGA_ F | | GF4_ZFYVE9:CGA _R | |
| 5 | CCDC 6 | ROS1 | GF5_CCDC6:ROS1_ F | | GF5_CCDC6:ROS1_R | |
| 6 | FGFR 2 | CIT | GF6_FGFR2:CIT_F | | GF6_FGFR2:CIT_ R | |
| 7 | AXL | MBIP | GF7_AXL:MBIP_F | | GF7_AXL:MBIP_R | |
| 8 | SCAF11 | PDGFRA | GF8_SCAF11:PDGFRA_F | | GF8_SCAF11:PDGFRA_R | |
| 9 | CD74 | ROS1 | GF9_CD74:ROS1_F | | GF9_CD74:ROS1_R | |
| 10 | SLC34 A2 | ROS1 | GF10_SLC34A2:ROS1_F | | GF10_SLC34A2:R OS1_R | |
| 11 | TXNR D1 | GPR133 | GF11_TXNRD1:GPR133_F | | GF11_TXNRD1:G PR133_R | |
| 12 | EML4 | ALK | GF12_EML4:ALK_F | | GF12_EML4:ALK _R | |
| 13 | HYOU 1 | C11orf93 | GF13_HYOU1:C11orf93_F | | GF13_HYOU1:C11orf93_R | |
| 14 | MAP4 K3 | PRKCE | GF14_MAP4K3:PRKCE_F | | GF14_MAP4K3:PRKCE_R | |
| 15 | RBM1 4 | FGF3 | GF15_RBM14:FGF3_F | | GF15_RBM14:FGF3_R | |
| 16 | BCAS | MAP3K3 | GF16_BC | | GF16_B | |
| | 3 | | AS3:MAP3 K3_F | | CAS3:MA P3K3_R | |
| 17 | SRSF4 | SNRNP40 | GF17_SRSF4:SNRNP40_F | | GF17_S RSF4:SN RNP40_ R | |
| 18 | UBR4 | ATP13A2 | GF18_UBR4:ATP13A2_F | | GF18_U BR4:ATP 13A2_R | |
| 19 | TTC19 | ATPAF2 | GF19_TTC19:ATPAF2_F | | GF19_TT C19:ATP AF2_R | |
| 20 | TPD52L1 | TRMT11 | GF20_TPD52L1:TR MT11_F | | GF20_TP D52L1:T RMT11_ R | |
| 21 | IGSF3 | MAN1A2 | GF21_IGSF3:MAN1A2_F | | GF21_IG SF3:MAN 1A2_R | |
| 22 | ERBB2IP | MAST4 | GF22_ERBB2IP:MAST4_F | | GF22_ERBB2IP:MAST4_R | |
| 23 | XAF1 | FAM64A | GF23_XAF1:FAM64A_F | | GF23_XAF1:FAM64A_R | |
| 24 | MIER2 | ITGB1BP3 | GF24_MIER2:ITGB1BP3_F | | GF24_MIER2:ITGB1BP3_R | |
| 25 | SLC16A7 | MUCL1 | GF25_SLC16A7:MUCL1_F | | GF25_SLC16A7:MUCL1_R | |
| 26 | ITGB1BP3 | DNM2 | GF26_ITGB1BP3:DNM2_F | | GF26_ITGB1BP3:DNM2_R | |
| 27 | ARHGEF16 | TCTEX1D4 | GF27_ARHGEF16:TCTEX1D4 _F | | GF27_ARHGEF16:TCTEX 1D4_R | |
| 28 | CMBL | C8orf38 | GF29_CMBL:C8orf3 8_F | | GF29_CMBL:C8orf38_R | |
| 29 | EDA | MID1 | GF30_ED A:MID1_F | | GF30_EDA:MID1_R | |
| 30 | H19 | CALR | GF28_H19:CALR_F | | GF28_H19:CALR_R | |

### Example 3: Fusion gene validation

The matters observed in Example 2 were validated using Sanger sequencing and PCR amplification for genome DNA and cDNA.

Using RNeasy mini kit (Qiagen), RNAs were extracted from tumor samples. DNAs were extracted using DNeasy tissue kit (Qiagen). For performing RT-PCR, the first strand cDNA was synthesized from 2.5mg of total RNA using SuperScript TM III first-strand synthesis system (Invitrogen) including oligo(dT)20, and then amplified using each primer pair corresponding to each fusion gene (see Table 50). Gene amplification was performed by PCR using each primer pair and Taq DNA polymerase high fidelity (Invitrogen).

PCR reaction was performed under the following conditions: at 95 °C for 10 min, at 95 °C for 30sec - at 62 °C for 30sec - at 72 °C for 30 sec (30 cycles), and finally at 72 °C for 10 min. Primers used in PCR for detecting genome deletion and Sanger sequencing were as follows: 5'-AACAAGGGTACAACCTGAAGGA-3' and 5'-TCAAGGAAGTATCGTGAGGTGA-3'. Primers used for fusion transcripts are as follows: 5'-AACAAGGGTACAACCTGAAGGA-3' and 5'-TCAAGGAAGTATCGTGAGGTGA-3'. All Sanger sequencing tests were performed according to the manual of Macrogen Inc. (http://www.macrogen.com).

### Example 5: Test for inhibition of solid cancer cell growth by inhibitor against fusion protein

To confirm whether or not the suggested fusion proteins participate in promoting growth and survival of cell lines or tumors that express the fusion protein, the cell lines were treated with an inhibitor of kinase in each fusion protein or the other domain therein.

Cells expressing the fusion protein were counted, and determination of cell growth inhibition was performed by CellTiter 96 AQueous one solution cell proliferation assay (Promega) according to manufacturer's manual. In brief, 1000 to 5000 cells were seeded on flat bottom 96 well plate, and cultured in 10% FBS supplemented complete medium. 24 hours after, the medium was replaced by 100 µl of 10% FBS supplemented complete medium containing various concentrations of the inhibitor of each of the fusion genes, and further culture was performed for 72 hours. The concentration of each inhibitor was applied to triplicate wells containing cells. When the culture was finished, 20 µl of CellTiter 96 AQueous one solution was added to each well, and the plate was cultured for 1-4 hours. Absorbance at 490nm was read using microplate reader. The cell proliferation inhibition can be expressed by average ± SD value of the absorbance read from the inhibitor-treated cells compared to inhibitor non-treated cells. Such analysis was repeated at least three times. From these analyses, it can be confirmed that the fusion protein promotes the growth and survival of a subset of human NSCLC in which such fusion protein is expressed, and such tumor cells can be inhibited by inhibition of activity of kinase or other domain in the fusion protein using a targeted inhibitor.

### Example 6: Test for promoting growth and survival of transformed mammal cell line by the fusion protein

NIH 3T3 cells were transformed by a structure including cDNA encoding each of the fusion proteins to express the fusion protein, and the transformed cells were used for confirming whether or not the expression of the fusion proteins can make normal cells to be transformed into cancer phenotype cells. In brief, the cells were maintained in RPMI-1640 medium (Invitrogen) supplemented with 10% fetal bovine serum (FBS; Sigma) and 1.0 ng/ml IL-3 (R&D Systems). A retrovirus supernatant was generated and transfected by generally known methods. NIH3T3 cells were subjected to transduction with retrovirus supernatant including pMXs-puro/fusion protein expression vector, and selected for puromycin(2ug/ml). Thereafter, abilities of transformed cells grown in soft agar were measured. By such measurement, it can be confirmed that an expression of the fusion protein can lead to transformation of NIH3T3 cells, and promote viability and growth of the cells in soft agar, whereas an inhibition of the expression of the fusion cell may lead to decreased viability and increased apoptosis of the cells.
## Claims

1. An AXL-MBIP fusion protein, comprising a fragment of AXL receptor tyrosine kinase (AXL) protein at N-terminal part and a fragment of MAP3K12 binding inhibitory protein 1 (MBIP) protein at C-terminal part, which are linked to each other, wherein
the fragment of AXL protein comprises an amino acid sequence encoded by a nucleotide sequence from the 1^{st} exon to the 244^{th} nucleotide of exon 20 of NM_021913 or NM_001699, and
the fragment of MBIP protein comprises an amino acid sequence encoded by a nucleotide sequence from the exon 4 to the last exon of NM_016586 or NM_001144891.

2. The fusion protein of claim 1, which is encoded by a fusion gene comprising a nucleotide sequence of positions 191 to 2767 of NM_021913 at 5' terminal part and a nucleotide sequence of positions 563 to 1123 of NM_016586 at 3' terminal part, which are linked to each other.

3. The fusion protein of claim 2, which comprises the amino acid sequence of SEQ ID NO: 47.

4. A fusion gene encoding the fusion protein of any one of claims 1 to 3.

5. The fusion gene of claim 4, which comprises the nucleotide sequence of SEQ ID NO: 45.

6. The fusion protein of any one of claims 1 to 3, a fusion gene encoding the fusion protein or mRNA corresponding to the fusion gene, for use as a marker for diagnosing a cancer.

7. A composition comprising at least one selected from the group consisting of materials interacting with the fusion protein of any one of claims 1 to 3, and a material interacting with a fusion gene encoding the fusion protein or mRNA corresponding to the fusion gene, for use in diagnosing a solid cancer, the solid cancer being a lung cancer,
wherein the material interacting with the fusion protein is selected from the group consisting of antibodies and aptamers, which bind to a fusion region of SEQ ID NO: 48 of the fusion protein, and
wherein the material interacting with the fusion gene encoding the fusion protein or mRNA corresponding to the fusion gene is at least one selected from the group consisting of:
primer pairs, wherein each primer of a primer pair is capable of hybridizing with 1) a nucleotide sequence consisting of 20 to 100 consecutive nucleotides adjacent to one or the other end of a polynucleotide fragment or mRNA fragment corresponding to the polynucleotide fragment, wherein the polynucleotide fragment consists of 50 to 250 consecutive nucleotides within the fusion gene, comprising a fusion region of the fusion gene, or 2) a complementary nucleotide sequence thereto, and
antisense oligonucleotides, probes, and aptamers, which are in 5 to 100 bp length and capable of hybridizing with a fusion region of the fusion gene or mRNA region corresponding to the fusion region,
wherein the fusion region comprises the nucleotide sequence of SEQ ID NO: 46.

8. The composition for use of claim 7, wherein the primer pair comprises SEQ ID NO: 188 and SEQ ID NO: 189.

9. The composition for use of claim 7 or 8, wherein the solid cancer is a non-small cell lung cancer (NSCLC).

10. The composition for use of any one of claims 7 to 9, further comprising at least one material interacting with at least one selected from the group consisting of:
CCDC6-ROS1fusion protein comprising CCDC6 protein or a fragment thereof and ROS1 protein or a fragment thereof, which are fused to each other;
FGFR2-CIT fusion protein comprising FGFR protein or a fragment thereof and CIT protein or a fragment thereof, which are fused to each other;
APLP2-TNFSF11 fusion protein comprising APLP2 protein or a fragment thereof and TNFSF11 protein or a fragment thereof, which are fused to each other;
MAP4K3-PRKCE fusion protein comprising MAP4K3 protein or a fragment thereof and PRKCE protein or a fragment thereof, which are fused to each other;
BCAS3-MAP3K3 fusion protein comprising BCAS3 protein or a fragment thereof and MAP3K3 protein or a fragment thereof, which are fused to each other;
KRAS-CDH13 fusion protein comprising KRAS protein or a fragment thereof and CDH13 protein or a fragment thereof, which are fused to each other;
ZFYVE9-CGA fusion protein comprising ZFYVE9 protein or a fragment thereof and CGA protein or a fragment thereof, which are fused to each other;
ERBB2IP-MAST4 fusion protein comprising ERBB2IP protein or a fragment thereof and MAST4 protein or a fragment thereof, which are fused to each other;
TPD52L1-TRMT11 fusion protein comprising TPD52L1 protein or a fragment thereof and TRMT11 protein or a fragment thereof, which are fused to each other;
TXNRD1-GPR133 fusion protein comprising TXNRD1 protein or a fragment thereof and GPR133 protein or a fragment thereof, which are fused to each other;
fusion genes encoding the fusion protein; and
SCAF11-PDGFRA fusion gene comprising 5UTR region of SCAF11 consisting of exon 1 of NM_004719 and a nucleotide sequence from exon 2 to the last exon of NM_006206, which are fused to each other,
wherein the material interacting is at least one selected from the group consisting of:
a primer pair of SEQ ID NO: 182 and SEQ ID NO: 183;
a primer pair of SEQ ID NO: 184 and SEQ ID NO: 185;
a primer pair of SEQ ID NO:186 and SEQ ID NO: 187;
a primer pair of SEQ ID NO: 190 and SEQ ID NO: 191;
a primer pair of SEQ ID NO: 192 and SEQ ID NO: 193;
a primer pair of SEQ ID NO: 194 and SEQ ID NO: 195;
a primer pair of SEQ ID NO: 196 and SEQ ID NO: 197;
a primer pair of SEQ ID NO: 198 and SEQ ID NO: 199;
a primer pair of SEQ ID NO: 200 and SEQ ID NO: 201;
a primer pair of SEQ ID NO: 202 and SEQ ID NO: 203; and
a primer pair of SEQ ID NO: 204 and SEQ ID NO: 205.

## Patentansprüche

1. AXL-MBIP-Fusionsprotein, umfassend ein Fragment eines AXL-Rezeptor-Tyrosinkinase (AXL)-Proteins am N-terminalen Teil und ein Fragment eines MAP3K12-bindungshemmendes Protein 1 (MAP3K12 binding inhibitory protein 1, MBIP)-Proteins am C-terminalen Teil, welche miteinander verbunden sind, wobei
das Fragment des AXL-Proteins eine Aminosäuresequenz, kodiert von einer Nukleotidsequenz vom ersten Exon bis zum 244sten Nukleotid von Exon 20 von NM_021913 oder NM_001699, umfasst, und
das Fragment des MBIP-Proteins eine Aminosäuresequenz, kodiert von einer Nukleotidsequenz von Exon 4 bis zum letzten Exon von NM_016586 oder NM_001144891, umfasst.

2. Fusionsprotein nach Anspruch 1, welches von einem Fusionsgen, umfassend eine Nukleotidsequenz von Positionen 191 bis 2767 von NM_021913 am 5'-terminalen Teil und eine Nukleotidsequenz von Positionen 563 bis 1123 von NM_016586 am 3'-terminalen Teil, welche miteinander verbunden sind, kodiert wird.

3. Fusionsprotein nach Anspruch 2, welches die Aminosäuresequenz nach SEQ ID NO: 47 umfasst.

4. Fusionsgen, welches das Fusionsprotein nach einem der Ansprüche 1 bis 3 kodiert.

5. Fusionsgen nach Anspruch 4, welches die Nukleotidsequenz nach SEQ ID NO: 45 umfasst.

6. Fusionsprotein nach einem der Ansprüche 1 bis 3, Fusionsgen, welches das Fusionsprotein kodiert, oder mRNA, die dem Fusionsgen entspricht, zur Verwendung als Marker zum Diagnostizieren einer Krebserkrankung.

7. Zusammensetzung, umfassend mindestens ein Material, das ausgewählt ist aus der Gruppe, bestehend aus Materialien, die mit dem Fusionsprotein nach einem der Ansprüche 1 bis 3 interagieren, und einem Material, das mit einem Fusionsgen, welches das Fusionsprotein kodiert, oder mRNA, die dem Fusionsgen entspricht, interagiert, zur Verwendung beim Diagnostizieren eines soliden Krebses, wobei der solide Krebs ein Lungenkrebs ist,
wobei das mit dem Fusionsprotein interagierende Material ausgewählt ist aus der Gruppe, bestehend aus Antikörpern und Aptameren, welche an eine Fusionsregion nach SEQ ID NO: 48 des Fusionsproteins binden, und
wobei das mit dem Fusionsgen, welches das Fusionsprotein kodiert, oder mRNA, die dem Fusionsgen entspricht, interagierende Material mindestens eines ist, das ausgewählt ist aus der Gruppe, bestehend aus:
Primer-Paaren, wobei jeder Primer eines Primer-Paares fähig ist zum Hybridisieren mit 1) einer Nukleotidsequenz, bestehend aus 20 bis 100 konsekutiven Nukleotiden, angrenzend an ein oder das andere Ende eines Polynukleotidfragments oder mRNA, die dem Polynukleotidfragment entspricht, wobei das Polynukleotidfragment aus 50 bis 250 konsekutiven Nukleotiden innerhalb des Fusionsgens, umfassend eine Fusionsregion des Fusionsgens, besteht, oder 2) einer dazu komplementären Nukleotidsequenz, und
antisense-Oligonukleotiden, Sonden und Aptameren, welche 5 bis 100 bp lang und fähig sind, mit einer Fusionsregion des Fusionsgens oder mRNA, die der Fusionsregion entspricht, zu hybridisieren,
wobei die Fusionsregion die Nukleotide-Sequenz nach SEQ ID NO: 46 umfasst.

8. Zusammensetzung zur Verwendung nach Anspruch 7, wobei das Primer-Paar SEQ ID NO: 188 und SEQ ID NO: 189 umfasst.

9. Zusammensetzung zur Verwendung nach Anspruch 7 oder 8, wobei der solide Krebs ein nicht-kleinzelliger Lungenkrebs (non-small cell lung cancer, NSCLC) ist.

10. Zusammensetzung zur Verwendung nach einem der Ansprüche 7 bis 9, weiterhin umfassend mindestens ein Material, das mit mindestens einem Material interagiert, das ausgewählt ist aus der Gruppe, bestehend aus:
CCDC6-ROS1-Fusionsprotein, umfassend CCDC6-Protein oder ein Fragment davon und ROS1-Protein oder ein Fragment davon, welche miteinander fusioniert sind;
FGFR2-CIT-Fusionsprotein, umfassend FGFR-Protein oder ein Fragment davon und CIT-Protein oder ein Fragment davon, welche miteinander fusioniert sind;
APLP2-TNFSF11-Fusionsprotein, umfassend APLP2-Protein oder ein Fragment davon und TNFSF11-Protein oder ein Fragment davon, welche miteinander fusioniert sind;
MAP4K3-PRKCE-Fusionsprotein, umfassend MAP4K3-Protein oder ein Fragment davon und PRKCE-Protein oder ein Fragment davon, welche miteinander fusioniert sind;
BCAS3-MAP3K3-Fusionsprotein, umfassend BCAS3-Protein oder ein Fragment davon und MAP3K3-Protein oder ein Fragment davon, welche miteinander fusioniert sind;
KRAS-CDH13-Fusionsprotein, umfassend KRAS-Protein oder ein Fragment davon und CDH13-Protein oder ein Fragment davon, welche miteinander fusioniert sind;
ZFYVE9-CGA-Fusionsprotein, umfassend ZFYVE9-Protein oder ein Fragment davon und CGA-Protein oder ein Fragment davon, welche miteinander fusioniert sind;
ERBB2IP-MAST4-Fusionsprotein, umfassend ERBB2IP-Protein oder ein Fragment davon und MAST4-Protein oder ein Fragment davon, welche miteinander fusioniert sind;
TPD52L1-TRMT11-Fusionsprotein, umfassend TPD52L1-Protein oder ein Fragment davon und TRMT11-Protein oder ein Fragment davon, welche miteinander fusioniert sind;
TXNRD1-GPR133-Fusionsprotein, umfassend TXNRD1-Protein oder ein Fragment davon und GPR133-Protein oder ein Fragment davon, welche miteinander fusioniert sind;
Fusionsgenen, welche das Fusionsprotein kodieren; und
SCAF11-PDGFRA-Fusionsgen, umfassend eine 5UTR-Region von SCAF11,
bestehend aus Exon 1 von NM_004719 und einer Nukleotidsequenz von Exon 2 bis zum letzten Exon von NM_006206, welche miteinander fusioniert sind,
wobei das interagierende Material mindestens eines ist, das ausgewählt ist aus der Gruppe, bestehend aus:
einem Primer-Paar nach SEQ ID NO: 182 und SEQ ID NO: 183;
einem Primer-Paar nach SEQ ID NO: 184 und SEQ ID NO: 185;
einem Primer-Paar nach SEQ ID NO:186 und SEQ ID NO: 187;
einem Primer-Paar nach SEQ ID NO: 190 und SEQ ID NO: 191;
einem Primer-Paar nach SEQ ID NO: 192 und SEQ ID NO: 193;
einem Primer-Paar nach SEQ ID NO: 194 und SEQ ID NO: 195;
einem Primer-Paar nach SEQ ID NO: 196 und SEQ ID NO: 197;
einem Primer-Paar nach SEQ ID NO: 198 und SEQ ID NO: 199;
einem Primer-Paar nach SEQ ID NO: 200 und SEQ ID NO: 201;
einem Primer-Paar nach SEQ ID NO: 202 und SEQ ID NO: 203; und
einem Primer-Paar nach SEQ ID NO: 204 und SEQ ID NO: 205.

## Revendications

1. Protéine de fusion AXL-MBIP, comprenant un fragment de la protéine récepteur tyrosine kinase AXL (AXL) à la partie N-terminale et un fragment de la protéine 1 inhibitrice de la liaison de MAP3K13 (MBIP) à la partie C-terminale, qui sont liés l'un à l'autre, dans laquelle
le fragment de la protéine AXL comprend une séquence d'acides aminés codée par une séquence nucléotidique du 1^{er} exon au 244^{ème} nucléotide de l'exon 20 de NM_021913 ou de NM_001699, et
le fragment de la protéine MBIP comprend une séquence d'acides aminés codée par une séquence nucléotidique de l'exon 4 au dernier exon de NM_016586 ou de NM_001144891.

2. Protéine de fusion selon la revendication 1, qui est codée par un gène de fusion comprenant une séquence nucléotidique des positions 191 à 2767 de NM_021913 à la partie terminale en 5' et une séquence nucléotidique des positions 563 à 1123 de NM_016586 a la partie terminale en 3', qui sont liées l'une à l'autre.

3. Protéine de fusion selon la revendication 2, qui comprend la séquence d'acides aminés de SEQ ID NO : 47.

4. Gène de fusion codant pour la protéine de fusion selon l'une quelconque des revendications 1 à 3.

5. Gène de fusion selon la revendication 4, qui comprend la séquence nucléotidique de SEQ ID NO : 45.

6. Protéine de fusion selon l'une quelconque des revendications 1 à 3, gène de fusion codant pour la protéine de fusion ou ARNm correspondant au gène de fusion, pour une utilisation en tant que marqueur pour diagnostiquer un cancer.

7. Composition comprenant au moins l'un choisi dans le groupe constitué de matériaux interagissant avec la protéine de fusion selon l'une quelconque des revendications 1 à 3, et d'un matériau interagissant avec un gène de fusion codant pour la protéine de fusion ou l'ARNm correspondant au gène de fusion, pour une utilisation dans le diagnostic d'un cancer solide, le cancer solide étant un cancer du poumon,
dans laquelle le matériau interagissant avec la protéine de fusion est choisi dans le groupe constitué d'anticorps et d'aptamères, qui se lient à une région de fusion de SEQ ID NO : 48 de la protéine de fusion, et
dans laquelle le matériau interagissant avec le gène de fusion codant pour la protéine de fusion ou l'ARNm correspondant gène de fusion est au moins l'un choisi dans le groupe constitué :
de paires d'amorces, dans laquelle chaque amorce d'une paire d'amorces est capable de s'hybrider avec 1) une séquence nucléotidique constituée de 20 à 100 nucléotides consécutifs adjacents à l'une ou l'autre extrémité d'un fragment de polynucléotide ou d'un fragment d'ARNm correspondant au fragment de polynucléotide, dans laquelle le fragment de polynucléotide est constitué de 50 à 250 nucléotides consécutifs au sein du gène de fusion, comprenant une région de fusion du gène de fusion, ou 2) une séquence nucléotidique qui y est complémentaire, et
d'oligonucléotides antisens, de sondes, et d'aptamères, qui ont une longueur de 5 à 100 pb et sont capables de s'hybrider avec une région de fusion du gène de fusion ou à la région de l'ARNm correspondant à la région de fusion,
dans laquelle la région de fusion comprend la séquence nucléotidique de SEQ ID NO : 46.

8. Composition pour une utilisation selon la revendication 7, dans laquelle la paire d'amorces comprend SEQ ID NO : 188 et SEQ ID NO : 189.

9. Composition pour une utilisation selon la revendication 7 ou 8, dans laquelle le cancer solide est un cancer du poumon non à petites cellules (NSCLC).

10. Composition pour une utilisation selon l'une quelconque des revendications 7 à 9, comprenant en outre au moins un matériau interagissant avec au moins l'un choisi dans le groupe constitué de :
la protéine de fusion CCDC6-ROS1 comprenant la protéine CCDC6 ou l'un de ses fragments et la protéine ROS1 ou l'un de ses fragments, qui sont fusionnées l'une à l'autre ;
la protéine de fusion FGFR2-CIT comprenant la protéine FGFR ou l'un de ses fragments et la protéine CIT ou l'un de ses fragments, qui sont fusionnées l'une à l'autre ;
la protéine de fusion APLP2-TNFSF11 comprenant la protéine APLP2 ou l'un de ses fragments et la protéine TNFSF11 ou l'un de ses fragments, qui sont fusionnées l'une à l'autre ;
la protéine de fusion MAP4K3-PRKCE comprenant la protéine MAP4K3 ou l'un de ses fragments et la protéine PRKCE ou l'un de ses fragments, qui sont fusionnées l'une à l'autre ;
la protéine de fusion BCAS3-MAP3K3 comprenant la protéine BCAS3 ou l'un de ses fragments et la protéine MAP3K3 ou l'un de ses fragments, qui sont fusionnées l'une à l'autre ;
la protéine de fusion KRAS-CDH13 comprenant la protéine KRAS ou l'un de ses fragments et la protéine CDH13 ou l'un de ses fragments, qui sont fusionnées l'une à l'autre ;
la protéine de fusion ZFYVE9-CGA comprenant la protéine ZFYVE9 ou l'un de ses fragments et la protéine CGA ou l'un de ses fragments, qui sont fusionnées l'une à l'autre ;
la protéine de fusion ERBB2IP-MAST4 comprenant la protéine ERBB2IP ou l'un de ses fragments et la protéine MAST4 ou l'un de ses fragments, qui sont fusionnées l'une à l'autre ;
la protéine de fusion TPD52L1-TRMT11 comprenant la protéine TPD52L1 ou l'un de ses fragments et la protéine TRMT11 ou l'un de ses fragments, qui sont fusionnées l'une à l'autre ;
la protéine de fusion TXNRD1-GPR133 comprenant la protéine TXNRD1 ou l'un de ses fragments et la protéine GPR133 ou l'un de ses fragments, qui sont fusionnées l'une à l'autre ;
des gènes de fusion codant pour la protéine de fusion ; et
le gène de fusion SCAF11-PDGFRA comprenant la région 5UTR de SCAF11 constituée de l'exon 1 de NM_004719 et une séquence nucléotidique de l'exon 2 au dernier exon de NM_006206, qui sont fusionnées l'une à l'autre,
dans laquelle le matériau interagissant est au moins l'une choisie dans le groupe constitué de :
une paire d'amorces de SEQ ID NO : 182 et SEQ ID NO : 183 ;
une paire d'amorces de SEQ ID NO : 184 et SEQ ID NO : 185 ;
une paire d'amorces de SEQ ID NO : 186 et SEQ ID NO : 187 ;
une paire d'amorces de SEQ ID NO : 190 et SEQ ID NO : 191 ;
une paire d'amorces de SEQ ID NO : 192 et SEQ ID NO : 193 ;
une paire d'amorces de SEQ ID NO : 194 et SEQ ID NO : 195 ;
une paire d'amorces de SEQ ID NO : 196 et SEQ ID NO : 197 ;
une paire d'amorces de SEQ ID NO : 198 et SEQ ID NO : 199 ;
une paire d'amorces de SEQ ID NO : 200 et SEQ ID NO : 201 ;
une paire d'amorces de SEQ ID NO : 202 et SEQ ID NO : 203 ; et
une paire d'amorces de SEQ ID NO : 204 et SEQ ID NO : 205.
